# EUROPEAN PATENT SPECIFICATION

(11) **EP 4 139 876 B1**
(45) Date of publication and mention of the grant of the patent: **28.01.2026**
(21) Application number: 21792953.8
(22) Date of filing: 26.04.2021
(51) Int. Cl.: G06Q 50/00

(54) **GUEST TRACKING AND ACCESS CONTROL USING HEALTH METRICS**
GASTVERFOLGUNG UND ZUGANGSKONTROLLE UNTER VERWENDUNG VON GESUNDHEITSMETRIKEN
SUIVI D'INVITÉ ET CONTRÔLE D'ACCÈS À L'AIDE DE MESURES DE SANTÉ

(30) Priority: 24.04.2020 US 202063015079 P; 01.05.2020 US 202063018674 P; 13.05.2020 US 202063024180 P; 05.06.2020 US 202063035037 P; 06.07.2020 US 202063048388 P; 23.07.2020 US 202063055463 P; 19.09.2020 US 202063080693 P
(43) Date of publication of application: 01.03.2023
(73) Proprietor: Aman, James, Celebration, FL 34747 (US)
(72) Inventor: CHEESMAN, Jeffrey, Upper Saddle River, NJ 07458 (US); AMAN, James, Celebration, FL 34747 (US)
(74) Representative: Meissner Bolte Nürnberg
(86) International application number: PCT/US2021/029186
(87) International publication number: WO 2021/217139

(56) References cited:
- US-A1- 2013 072 295
- US-A1- 2013 173 300
- US-A1- 2013 179 188
- US-A1- 2017 289 341
- US-A1- 2019 272 695
- US-A1- 2020 050 745
- US-A1- 2020 082 377

## Description

### CROSS-REFERENCE TO RELATED APPLICATIONS

This application claims the benefit of U.S. Provisional Application No. 63/015,079 entitled GUEST TRACKING AND ACCESS CONTROL USING HEALTH METRICS filed on April 24, 2020. This application claims the benefit of U.S. Provisional Application No. 63/018,674 entitled GUEST TRACKING AND ACCESS CONTROL USING HEALTH METRICS filed on May 1st, 2020. This application claims the benefit of U.S. Provisional Application No. 63/024,180 entitled GUEST TRACKING AND ACCESS CONTROL USING HEALTH METRICS filed on May 13th, 2020. This application claims the benefit of U.S. Provisional Application No. 63/035,037 entitled GUEST TRACKING AND ACCESS CONTROL USING HEALTH METRICS filed on June 5th, 2020. This application claims the benefit of U.S. Provisional Application No. 63/048,388 entitled GUEST TRACKING AND ACCESS CONTROL USING HEALTH METRICS filed on July 6th, 2020. This application claims the benefit of U.S. Provisional Application No. 63/055,463 entitled GUEST TRACKING AND ACCESS CONTROL USING HEALTH METRICS filed on July 23rd, 2020. This application claims the benefit of U.S. Provisional Application No. 63/080,693 entitled GUEST TRACKING AND ACCESS CONTROL USING HEALTH METRICS filed on September 19th, 2020. U.S.

### FIELD OF INVENTION

The present invention relates to an entity access control system that combines the use of a smart-ticket and a health regiment, where the health regiment defines a set of health metrics to be confirmed for the ticket holder by using health measurement devices or services prior to attempting to gain access to a premises controlled by the entity, where the health measurement devices are any one of or any combination of self-operated or other-operated, and where for each measurement recorded in the regiment the identity of the ticket holder is confirmed, thereby confirming that the recorded measurement is of the ticket holder.

### BACKGROUND OF THE INVENTION

Amongst other teachings, U.S. Patent No. 10,719,134 entitled INTERACTIVE OBJECT TRACKING MIRROR-DISPLAY AND ENTERTAINMENT SYSTEM filed on May 9, 2018 discloses an "interactive display" "apparatus and methods" for example to be used at "theme parks such as Universal Studios and Disney World" that "provide immersive environments for their guest's enjoyment," including "park-wide games." Amongst other teachings, the application discloses a "guest tracking system" that provided guest information including "current and historical locations" for use at least in part by an "interactive gaming system" with interfaces distributed throughout the "entity."

Amongst other teachings, U.S. Patent No. 10,861,267 entitled THEME PARK GAMIFICATION, GUEST TRACKING AND ACCESS CONTROL SYSTEM filed on August 4th, 2018 discloses a "smart-ticket" for use by a person visiting an entity premises referred to as "ZONE4." The smart-ticket provided many uses including providing for "self-serve access" to ZONE4. The smart-ticket was shown to support "guest tracking," an "interactive gaming system" and a "lost guest services platform." An exemplary smart-ticket can be formed by the combination of a mobile device (such as a smartphone) using a special smart-ticket "app," where the mobile device and app are the smart-ticket. Smart-tickets can also work with traditional printed tickets or tickets including printed electronic circuits, referred to in the reference art as "electronic tickets." Wearables are shown for acting as electronic tickets to carry electronically detectable information communicated by the mobile device and app including the "right-to-access" as determined, received and / or confirmed by the mobile device and app.

In a preferable embodiment, a person uses their mobile device to first download the special app that includes a unique app ID for associating with the unique ID of their mobile device, thus forming the basis for a smart-ticket. The app ID is preferably encrypted and remains concealed from the person. The person would then use the mobile device and app to purchase or otherwise acquire a right-to-access / ticket for a premise, where the "ticket number" or "ticket ID" is uniquely associated with their App ID, after which preferably while "at-home" in "ZONE1" the person registers / pre-registers one or more personal biometrics for association with the app ID and ticket ID. Personal biometrics at least include their face or fingerprint, but can include any biometric sufficient for substantially differentiating the person from another person.

Ultimately, the person travels to a premise ZONE4 (from ZONE1 through ZONE2), but before gaining access they must first enter a confined ZONE3 adjoining ZONE2 and ZONE4 through a self-serve access point. Within the confined ZONE3 the person is required to confirm their right-to-access by entering one or more biometrics sufficiently matching the biometrics registered to the ticket. Upon successful conformation, the mobile device and app updates the status of the right-to-access and optionally provides this status update to the electronic ticket, if an electronic ticket (or wearable equivalent) is being used. Once confirmed, the person uses a self-serve access point to exit ZONE3 and validly enter ZONE4.

It is shown that using a smart-ticket provides a means for allowing individuals to privately register and self-confirm their right-to-access a premise such that personal information is never shared with the premise.

Amongst other teachings, U.S. Patent No. 10,974,135 entitled INTERACTIVE GAME THEATER WITH SECRET MESSAGE IMAGING SYSTEM filed on September 27, 2018 discloses "managing visitor flow" "to avoid excessive wait times." The teachings referred to the "smart-ticket," "guest tracking system" and "interactive gaming system."

Amongst other teachings, U.S. Patent No. 10,688,378 entitled PHYSICAL-VIRTUAL GAME BOARD AND CONTENT DELIVERY SYSTEM filed on July 4, 2017 and U.S. Patent No. 10,857,450 entitled PHYSICAL-VIRTUAL GAME BOARD AND CONTENT DELIVERY PLATFORM filed on August 9, 2018 discloses a "physical-virtual gaming system" that at least in part uses input from a "global environment eco-system" and a "local environment eco-system" maintained by "entity" such as a theme park to alter the experience provided by the physical-virtual gaming system.

In brief summary intended to be introductory but not limiting, and without touching upon or reviewing all of the many teachings herein provided, the present invention teaches further adaptations to the incorporated referenced art especially for supporting a novel mutual health assurance system. The mutual health assurance system provides an entity with means for ensuring that persons wishing to enter a premise have sufficiently complied with a specified health regiment. The system further provides a person wishing to enter a premises information regarding the health regiment compliance of persons already present inside the premises. Persons are shown to be of any kind including visitors or workers, where visitors are for example provided with private controlled health-verified access to the premises while workers are for example provided non-private controlled health-verified access to the entity premises.

Both private and non-private access are shown to include means for verifying a person's identity as matching the registered identity associated with rights to enter a premises (including for example a ticket or work pass) and means for verifying that the identified person has sufficiently complied with a specified health regiment. Private access means allow for not sharing personal information regarding the person with the entity including for example a name, a fingerprint or a facial image, or any specific health measurement data, whereas non-private access may require the sharing of personal information.

The mutual health assurance system is shown to include a smart-ticket as taught in the reference art that has been further adapted to support the objects and advantages of the present invention including the ability to receive information regarding one or more health regiments, preferably provided by any of the entity or a public health organization. Further adaptations describe communications and exchanges of information between the further adapted smart-ticket and one or more novel health measurement devices for determining one or more health measurements of the smart-ticket holder in relation to a health regiment. Health measurement devices are shown to be either self-operated with ID confirmation or other-operated with ID confirmation. These devices provide measurements of personal biometrics, for example but not limited to temperature, pulse rate, blood oxygen levels, body motion, retinal scan data, and sleep patterns, as well as environmental measurements such as temperature, humidity, lighting, etc.

Many variations of health measurement devices are taught, including wearable devices with a clasp lock for verifying the open or closed state of the wearable with respect to a body part (such as the wrist) of a person. Variations also include means for confirming that the measurement(s) being taken are actually of the person who is the registered ticket holder. In some examples, these confirming means include light emitting apparatus such as an LED included as a part of the health measurement device that emits confirming signals that are sensed preferably by a camera on the smart-ticket, where the same captured images on the smart-ticket are then useable to simultaneously confirm the identity of the ticket holder and the identity of the health measurement device (and thereby confirm that the health measurement does belong to the ticket holder). Health measurement devices are shown as wired or wireless, where wired variations can draw power and communicate with a smart-ticket through the wired connection, offering advantages such as a reduction in the manufacturing complexity and cost of the device.

In another variation, a traditional "no-touch" health measurement device is further adapted to include new camera means or additional processing capabilities to existing camera means to first image a person for ID confirmation (such as via facial or retinal scanning) after which the device is directed to a body part for taking at least one health measurement, where during the directing step additional images of the confirmed person are captured and image analysis is used to show that the same person's body has remained in view of the camera without interference so as to serve as a confirmation that a final measurement (for example of the forehead, ear or foot) is in fact being made of the same identified person.

A "touch" health measurement device is shown for taking personal temperature that is anticipated to be of a minimal cost and to be "finger-worn" (preferably the index finger), where the power and communications to the device are provided by a wired connection to the device. This device also includes an LED for emitting ID confirming signals. A variation of the touch temperature device is taught that is further adapted to measure one or more electrical properties of the skin, such as resistance and capacitance. It is shown that the by measuring one or more of these electrical properties it is possible to provide some validation that the contact surface of the temperature sensor or the skin has not been altered to affect a normal measurement. This variation "electrodermal-thermometer" is also shown to include a second piece (for example worn on the thumb) for first or additionally bringing into contact with the temperature sensor (for example worn on the index-finger). This additional "validator" piece is passive and includes a surface for measurement that has pre-known electrical properties. These pre-known electrical properties can then be measured by the electrodermal-thermometer to confirm that the measured values are within an expected tolerance. It is preferable to additionally measure the electrical properties of the person's skin, were the combination of electrical property measurements in comparison to expected values serves to assure the proper functioning of the temperature sensor.

Other variations are in the form of health measurement and / or person id confirmation kiosks preferably provided by the entity near premises access control points. Health measurements are responsive at least in part to one or more health regiment(s) and can be dynamically varied and even updated over time to adapt to special circumstances, for example where a person might otherwise fail to be within a required health measurement range due to some other health condition or medication, and where for example the person may optionally seek other measurements obtained at a health-care provider as qualified substitute measurements or even seek a waiver from an appropriate organization such as the entity or a public health organization.

A novel wearable smart-ticket is taught comprising certain features of the further adapted smart-ticket and certain features of self-operated health measurement devices. The smart-ticket and wearable smart-ticket are shown to be capable of creating a virtually 100% "contact list" of persons coming within a certain proximity of other persons while being within a mutual health assurance premises, using and further adapting a technology referred to as "contact tracing."

Health regiments are shown to be enforceable based at least upon location and time information including with respect to a scheduled or unscheduled visit to a premises, where the regiment can be enforced before arriving at the premises, during time spent at the premises, or after leaving the premises.

The present invention also provides alternative teachings of the mutual health assurance system including a health-verified guarded checkpoint system and a health-verified appointment (or reservation) system. Each alternative system comprises a mobile device and app capable of working with health measurement devices and following a health regiment including the personal ID verification of each health measurement. The health-verified guarded checkpoint system addresses use cases where a formal ticket is not typically required nor even an appointment or reservation, for example at a store or public place of gathering such as a mall area or park. This alternative system provides for one or more persons confirming to a guard at the premises that they are using a validated app for monitoring and verifying their health status, and that they have been successfully following and passing a prescribed health regiment.

In another variation, an appointment system addresses use cases where a formal ticket is not typically required but some pre-authorized access rights are helpful, and where access to a premises is typically for the purposes of receiving a service, for example visiting a doctor, a hair salon, restaurant, etc. In some cases, where for example the appointment is with a doctor, the system provides for the sharing of personal health measurements, and otherwise the appointment system also provides for automatically providing a feedback health verification token to an appointment scheduling module. This feedback for example confirms that a person is passing the required health regiment and therefore can keep their appointment at least from a health status perspective. When the appointment system determines for example that a person is failing a health regiment, it is also possible to automatically reschedule the appointment or reservation, but to otherwise notify the service provider.

The appointment system is shown to be extensible into any number of "private meeting networks" ranging from a formal dating service to an open-to-all meetup service or a private arrangement between parents for their children to have a playdate. In this sense, a third-party agent or automatic service is acting as an "honest broker" to arrange health-verified meetings between any two or more individuals for any reason. The scheduling software is not limited to running on a remote server but can for example be deployed onto an organizer's smartphone, where the organizer acts as the broker. In other variations, each individual exchanges data with other individuals more in the fashion of a ring network. The honest broker system allows for the sharing of one or more agreed upon health regiments and the sharing of the current status of each possible meeting participant with respect to the shared health regiment(s).

Other types of private networks include sports teams and clubs that may assemble at a different premise at different times, where the sports teams and clubs as the "honest broker" establish their own regiments and rules that must be passed by all team / club members prior to each group participation. Still yet other types of private networks are a health club where different people at different times aggregate or meet at the same place, where the members agree to follow a strict health regiment and the club operator is the "honest broker," and where at any time any member showing up at the club must prove sufficient, authenticated compliance with the regiment. Many uses and variations are possible, all of which add the layer of health assurance as taught herein to any type of meeting.

The present invention also describes the use of "authenticated health test kits," or more particularly teaches how to collect traditional biometric health data samples that are authenticated to uniquely belong to, or "be from" the person 1, where the results of testing on the health data samples (determined at a later time) are then and therefore also authenticated via association with the authenticated person. Two major use cases are discussed, that of "at-home" versus using "a service provider." Many at-home tests kits are known in the art for collecting biometric samples such as saliva, mucus, blood, urine, etc. While the at-home tests are convenient for the person giving the samples, there is no current way to ensure that the samples are indeed coming from the person. Furthermore, the person's anonymity is not protected in that they must send in their samples for testing along with some form of (personal) contact information. Some health samples can only be taken at a service provider, or are at least also taken at a service provider, were the person shows up at the provider such as a clinic or doctor's office, identifies themselves and then requests a health test service (such as a "blood workup"). The person may be asked for some form of government ID as a means of verifying their identity, were in other cases this is not done or deemed necessary. In either case, and once again, the person's anonymity is not protected.

Well it is known that there are anonymous clinics that do not require the person to be identified, the present invention further teaches how to provide authentication of the person to be associated with the health data samples in an anonymous manner, while the person at home or at a service provider provides biometric samples. The anonymous manner includes providing a preferably encrypted "authentication token" whereby the person's health-app ID (also smart-ticket ID since the smart-ticket is monitoring a health regiment), or an access rights ID (such as a ticket ID issued by a premises such as a theme park or cruise ship), or a personal alias ID is included in the token and essentially stays associated with the health samples and ultimately with the resulting measurements, which then can only be associated with that person's health regiment data. The present invention thus teaches not only health measurement devices for self-use or to be operated by a healthcare agent, where the health measurement devices directly create measurement results verified to belong to a given person, the present invention also teaches kits or apparatus for self-use or to be operated by a healthcare agent, where the kits or apparatus collect one or more health samples verified to belong to a given person, and then where later in the process the health samples are tested to become measurement results, these measurement results are therefore pre-verified to belong to the given person.

Regarding at least the at-home test kits, two examples are shown for a swab that collects both saliva and mucus and a sample strip for collecting blood after pricking a finger. Both kits include modifications for including either or both of an electronic tag or visible markings on the health sampling device (e.g., the swab of the sample strip). The processes described for the use of these two exemplary kits show a step of validating that health sampling device while substantially also validating the person, where both the person and the sampling device are imaged substantially simultaneously by the camera being used by the device and app that are a smart-ticket with health regiment app. A variation fingerprint temperature device was also shown that attaches to the device (such as a smartphone) with app and allows a person to provide their fingerprint in view of the smartphone while simultaneously providing their image, thus increasing veracity of their authentication.

There are many and varied health measurement devices and home test kits available in the marketplace, where the devices and kits are typically produced and sold by different manufacturers, where these devices and kits may be used to collect "digital health metrics," and where the digital health metrics are not authenticated (i.e., ensured to be "of," "from," or otherwise with respect) to the person using the device on themselves. Also, the various digital health metrics are not typically shared or aggregated across disparate devices or kit testing labs forming a unique "digital health picture" of a person, and means are not provided for using this digital health picture in relation to a health regiment established to verify a person's current health state.

In addition to providing means for authenticating the home test kits, the present invention also provides means for authenticating other and varied health measurement devices currently available in the marketplace. For example, the finger-worn device described herein that is authenticated for use via a process conducted with the mobile device and app (also serving as a smart-ticket), is configured to include a reader for electronically reading / writing an electronically readable tag. The preferred reader is an NFC reader typically built into a mobile device such as a smartphone, such that the preferred electronically readable tag is an NFC tag. The person attaches the NFC tag onto the "third-party" health measurement device, where the attachment serves to permanently associate a unique ID with the third-party device, and where the unique ID is then ultimately detectable by the mobile device and app for example using the finger-worn NFC reader.

Example third-party devices include a "continuous glucose monitoring" patch typically worn on the arm of the person, a cough-detection patch typically worn on the neck of the person, and a breath analyzer typically held by the person as they breath into a mouthpiece on the breath analyzer. It is shown that the mobile device and app monitoring the health regiment being followed by the person then has a data log of when each third-party device was authenticated such that data collected preferably electronically from these devices and determined after the logged date/time of authentication can be considered authenticated and useful for the purposes of validating a person's health status.

The present invention further distinguishes spatial body patterns, such as facial features or fingerprints, that are useful for uniquely identifying a person as being "inter-identification" means. Temporal body patterns, such as a heartbeat or breathing pattern are referred to as "intra-identification" means and shown to be useful for correlating a health device dataset being captured by a device that does not otherwise provide for inter-identification. It is shown that the mobile device and app which is capable of inter-identification (by at least doing facial recognition) can be configured to also be capable of intra-identification. In one embodiment, the mobile device and app are configured to use an imaging technology referred to a "rPPG" (remote photoplethysmography) for visually determining a temporal body pattern / intra-identification means such a heartbeat pattern, where the heartbeat pattern is at least discernible as small color fluctuations in the face of the person. In another embodiment, the finger-worn device is configured to use a pulse sensor for determining the temporal body pattern / intra-identification means such a heartbeat pattern, where then the finger-worn device can also be configured to capture a fingerprint / spatial body pattern / inter-identification means, and / or to perform visual LED confirmation with the mobile device and app, thereby being associated with the mobile device and apps facial recognition / spatial body pattern / inter-identification means.

It is then shown that once the mobile device and app, either acting on its own or in combination with for example the finger-worn device, has determined a concurrent combination of both inter-identification and intra-identification, it may then use the intra-identification means to correlate with substantially a real-time matching temporal pattern (such as the heartbeat) being detected by another health device. Exemplary other devices are shown that can determine intra-identification but in some configurations not capable of determining inter-identification, thus the other devices are insufficient when used alone for determining authenticated health measurements for use in an authenticated regiment being followed by the person.

One of these other devices is shown to be a chest band equipped with sensors such as MEM microphones for capturing audible sounds emanating from the person's chest, where the audible sounds are usable for determining the intra-identification means of a heartbeat as well as other valuable metrics including lung / breathing patterns and cough patterns. Another exemplary device shown is a "smart scale" either already comprising or further adapted to include pulse detecting means sufficient for determining the intra-identification means of a heartbeat. Using correlated intra-identification, the mobile device and app is then able to authenticate that any data collected from the correlated devices is "of," "from," or otherwise with respect to the person using the device on themselves, thus being a health metric available for use in an authenticated regiment.

The present invention teaches how the registered person's personal biometrics associated with the wealth of on-going authenticated health measurements, themselves associated with regiments, regiment rules, and authenticating health devices for collecting the measurements, are all maintained as encrypted and private data on the mobile device and app / smart-ticket. This encrypted "local health database" of information is usable by the person for providing "proof-of-health" to any premise prior to entering the premise. The "proof-of-health" may be used with or without also needing to provide a "right-to-access" the premise. It is also shown how for example "rolling snapshots" of select personal health metrics can be anonymized via association with a group code such as a zip code or "health study ID," where these snapshots represent metrics over a useful period of time such as fourteen days and are securely uploaded to a "centralized anonymous health database" of health information relating to a population.

The central database is shown to be analyzed for example using machine learning or AI (ML/AI) for detecting patterns in the population's rolling health metrics. Means are provided for broadcasting public messages to the population from the central database, where the messages are based at least in part on the detected patterns and include instructions to be provided to certain person(s) whose personal health data indicate a correlation to the centrally determined pattern, where the correlation is shown to be detectable on each person's private mobile device and app by for example providing a trained ML data model in the public broadcast message that is privately applied to all individuals in the population and serves to select out only the correlated persons. Public messages were also shown to include "regiment rule changes" / updates that can be applied for example to any correlated person, for example increasing or decreasing the number and type of health measurements included with a given regiment, where for example an increase can include seeing a doctor or healthcare professional or getting measurements through an authorized health kit provider.

The "local health database" is shown to optionally include personal contact tracing data using traditional methods such as logging each anonymous contact as determined using for example wireless communication such as Bluetooth signal analysis implemented on the mobile device and app / smart-ticket. This traditional contract tracing is referred to as "quantified" tracing, where then it is shown that each quantified contact can then also be "qualified" using at least the personal health status information known to the person's own mobile device and app regarding the person, but then preferably also using personal health status information known to by the contact's own mobile device and app regarding the contact. Quantified and qualified contact tracing is shown to greatly decrease the number of detected / logged contacts that would be necessary to "decrypt" based upon notification of possible infected contacts, thus saving considerable time and energy / power to be expended by the mobile device and app.

It was shown that current "quantitative only" contact tracing technologies and methods are proving to be ineffective at least because of their insufficient "population uptake," where uptake refers to the number of people who have, have enabled and are therefore able to participate in contact tracing. The predominant technology for enabling contact tracing is shown to be the smartphone, which is also considered by many to create an "equity" concern with respect to their cost of the smartphone and otherwise shown to be not owned or used by a significant portion of the population at least including children.

The present invention teaches an "active face mask" that includes electronics for performing "active mask functions" (AM functions) including pairing and communicating with a mobile device and app and communicating with a mask tracking system. Active masks are shown to include various sensor configurations for determining at least a "proper fit" and preferably also the presence of a "proper filter insert," where the combination verifies that the active mask is able to perform the expected filtration of infectious disease "airborne particulates." **It** is shown that many premises such as office buildings, campuses, healthcare facilities, public transportation vehicles such as airplanes, buses and subway cars, theme parks, stadiums, public parks, schools and universities, military bases, etc. are currently enforcing "mask rules," whereby all persons within the facility must be wearing masks essentially properly fitted at all times.

It is shown that by using active masks as taught herein, personal compliance to these mask rules can now be determined substantially on a real-time basis and shared for example with the mask tracking system such that the premise is provided means for enforcing the mask rules. It is shown that the masks are usable for implementing contact tracing, where the contact tracing is both quantified and qualified using health data determined and available on companion mobile device and apps and health data such as "proper fit and filtration" determined by the active mask. It is also shown that since masks are required within a given premise, that effectively the premise can implement 100% quantified and qualified contact tracing which is a significant advantage over the current state-of-the-art. The active masks are anticipated to be substantially less expensive than a smartphone thereby diminishing any "equity" concern.

Parks, stadiums, public parks, schools and universities, military bases, etc. are currently enforcing "mask rules," whereby all persons within the facility must be wearing masks essentially properly fitted at all times.

It is shown that by using active masks as taught herein, personal compliance to these mask rules can now be determined substantially on a real-time basis and shared for example with the mask tracking system such that the premise is provided means for enforcing the mask rules. It is shown that the masks are usable for implementing contact tracing, where the contact tracing is both quantified and qualified using health data determined and available on companion mobile device and apps and health data such as "proper fit and filtration" determined by the active mask. It is also shown that since masks are required within a given premise, that effectively the premise can implement 100% quantified and qualified contact tracing which is a significant advantage over the current state-of-the-art. The active masks are anticipated to be substantially less expensive than a smartphone thereby diminishing any "equity" concern.

Active masks paired with companion mobile devices and apps joined into a "private meeting network" (not associated per se with a given premise) are shown to provide a powerful tool for allowing those in the private network to enforce and track their own mask related policies, where for example a private meeting group is a network of families with children, a "meetup group," or an association conference meeting. Active masks, as a wearable, where also shown to be useable as a smart-ticket, where for example the mobile device and app transfers the person's "right-to-access" proof data to the active mask, along with "proof-of-health" certification, the combination of which may then be used by the active mask alone (i.e. even when the mobile device an app are not present with the person), to gain authorized access to a premise.

Data determined by the active mask in combination with other health metrics and mask tracking system data was shown to be useful for providing premise "gamification," whereby persons are substantially engaged with a game and rewarded by their tracked healthy behaviors including following a regiment and wearing their mask properly during their time at the premise, as well as minimizing contacts and maximizing social distancing. It was shown that by forming a "private network" such as a family and friends, it was possible for the mask tracking system to differentiate close contacts within the private network as being different from close contacts outside the private network, where the gamification rules are focused on minimizing "outside" close contacts.

The present invention also teaches a further adaptation to the original smart-ticket as taught in the reference art. The alternate method employs the prior "zones" including a "confirmation zone," and then adds a means for ensuring confirmation of right-to-access based upon the time sequence of data captured and known to the system. It is shown that the information known to the premise during the entire ticketing and self-access process does not include any personal information about a ticketed person entering and being at the premise.

Additional background can also be found in the following cited art:
US 11,416, 588 B2 (KIM KOKEUN [KR] ET AL), entitled MOBILE TERMINAL WITH ENHANCED SECURITY BY HAVING EXPLICIT AUTHENTICATION AND IMPLICIT AUTHENTICATION, and filed on August 8th, 2019, discloses a memory configured to store a registered user behavior patte rn for performing an implicit authentication; a gyro sensor; a camera; an acceleration sensor; an output unit; a controller configured to:
   perform explicit authentication based on authentication information; and
   based on a determination that the explicit authentication is successful, collect user behavior pattern for performing the implicit authentication including a first behavior item indicating a state in which a user holds the mobile terminal collected by the gyro sensor, a second behavior item indicating that a gaze direction of the user is directed toward a front surface of a display unit included in the output unit collected by the camera, a third behavior item including a state in which a user walks collected by the acceleration sensor, and a fourth behavior item including a touch input speed of a keyboard included on the display unit;
   determine whether the implicit authentication is successful, wherein the implicit authentication is determined to be successful based on newly collected first behavior item, newly collected second behavior item, newly collected third behavior item, and newly collected fourth behavior item respectively matching a pre-enrolled first behavior item, a pre-enrolled second behavior item, a pre-enrolled third behavior item, and a pre-enrolled fourth behavior pattern item from the stored registered user behavior pattern;
   maintain an authentication state based on a determination that the implicit authentication is successful;
   release the authentication state based on a determination that the implicit authentication has failed; and
   cause the output unit to output a notification indicating that the authentication state has been released after the authentication state is released.
US 10,997,578 B2 (SONG HYEWON [KR] ET AL), entitled MOBILE TERMINAL AND CONTROL METHOD THEREOF, and filed on August 18th, 2015 discloses a mobile terminal, comprising:
   a touch screen configured to sense a preset user input in a power-off state;
   a Near Field Communication (NFC) antenna configured to receive a settlement request signal from an external terminal in the power-off state;
   a Near Field Communication integrated circuit (NFC IC); an energy storage configured to store energy; and
   a controller operably coupled to the touch screen and the NFC antenna, and configured to:
      cause the NFC IC to store token data of a default card 5 associated with settlement information in a program memory region of the NFC IC;
      cause the NFC antenna to receive a radio frequency (RF) field energy from the external terminal in the power-off state;
      cause the energy storage to store the received RF field energy in the power-off state;
      determine whether a power amount of the stored RF field energy is more than a threshold value for entering an emergency settlement mode;
      cause the mobile terminal to enter the emergency settlement mode by using the stored RF field energy when a value of the power amount corresponding to the stored RF field energy is more than the threshold value in response to authentication of user information received via the touch screen in the power-off state, wherein only some functions among all functions available for the mobile terminal are performable by using power acquired from the stored RF field energy in the emergency settlement mode;
      cause the NFC antenna to transmit the settlement information corresponding to the settlement request signal to the external terminal while no settlement application program is activated in the emergency settlement mode,
      wherein the token data stored in the program memory region of the NFC IC is used at a time of an emergency settlement in the emergency settlement mode;
      access the settlement information stored in a universal subscriber identity module (USIM) when the mobile terminal is turned on in the emergency settlement mode; and
      cause the touch screen to display usage history associated with the settlement information by executing a settlement application program when the mobile terminal is turned on in the emergency settlement mode,
      wherein the controller is further configured to supply the power to the NFC IC and the USIM based on the received RF field energy stored in the energy storage.
US 10,528,913 B1 (ELWHA LLC), entitled EVIDENCE-BASED HEALTHCARE INFORMATION MANAGEMENT PROTOCOLS, and filed on December 5th, 2012 discloses a healthcare information management system comprising: a server device in communication with at least a first mobile device local to a first individual and a second device, the server device implementing one or more instructions that program the server device for at least:
   detecting, via at least one application running on the first mobile device, at least one indication that the first mobile device has operatively coupled with at least one stationary wireless node having at least one known location;
   retrieving data from the first mobile device associated with the first individual, the data including at least one indication whether the first individual is com- pliant with a health regimen, the retrieving initiated at least partly responsive to the at least one indication that the first mobile device has operatively coupled with at least one stationary wireless node having at least one known location;
   modifying at least one medical record associated with the first individual based at least partly on the retrieved indication whether the first individual is compliant with a health regimen;
   alerting at least one health regimen provider when the 55 at least one medical record indicates that the first individual is non-compliant with the health regimen;
   determining from the second device that a second individual is available to participate in an electronic intercommunication, the second individual associated with the at least one health regimen provider; and
   transmitting an electronic intercommunication between the first individual and the second individual based at least partly on the indication whether the first individual is compliant with the health regimen and based at least partly on the determination that the second individual is available to participate in the electronic intercommunication.
US 10,706,673 B2 (ALDERUCCI et al.), entitled BIOMETRIC ACCESS DATA ENCRYPTION, and filed on September 14th, 2012 discloses a method comprising:
   receiving by at least one computer processor at least one item of identity verification data from a gaming device, wherein the received at least one item of identity verification data is encrypted;
   comparing by at least one computer processor the at least one item of encrypted identity verification data received and at least one item of encrypted identity verification data obtained and stored previously, wherein the at least one item of encrypted identity verification data received and the at least one item of encrypted identity verification data obtained and stored previously are compared in their encrypted form;
   enabling by at least one computer processor at least one service on the gaming device based on a match between the encrypted identity verification data, the at least one service comprising a game;
   displaying by at least one computer processor an interface screen on the gaming device comprising graphic objects associated with the game and at least one selectable element for a user of the gaming device to submit a command during play of the game;
   obtaining by at least one computer processor user continuity data from the gaming device, the user continuity data comprising behavioral data or proficiency data;
   comparing by at least one computer processor the user continuity data to prior user continuity data, wherein the user continuity data comprises a rate at which the user navigates menu items on the interface screen of the gaming device;
   determining by at least one computer processor that the obtained user continuity data is not within a predetermined level of confidence of the prior user continuity data; and
   based on the determination that the user continuity data is not within the pre -detennined level of confidence of the prior user continuity data, triggering by at least one computer processor the display of a prompt on the gaming device, the prompt requesting additional identity verification data from the user, wherein the additional identity verification data is different than the user continuity data.
US 10,785,365 B2 (DIGIMARC CORPORATION), entitled INTUITIVE COMPUTING METHODS AND SYSTEMS, and filed on June 12th, 2017 discloses a method employing a device equipped with a processor, a display, a camera and a microphone, the camera capturing imagery depicting plural items in a user's physical environment, the method comprising the acts:
   capturing first speech of the user, with the device microphone;
   the device processor detecting that the captured first speech includes a cueing expression, and in response to detection of the cueing expression, the device switch- ing from a lower activity state to a heightened alert state, in the heightened alert state the device perform- ing functionality including:
      capturing second user speech with the device microphone; sending data corresponding to the second user speech to a recognition module, and receiving recognized second speech data in return, the recognized second user speech indicating one of said plural items depicted in the captured imagery as of particular user interest;
      based on one or more descriptors included in the recognized second speech data, determining a first of said plural depicted items as being of likely user interest;
      presenting a marking on the device display, at a location indicating said first item;
      capturing third user speech with the device microphone, the captured third user speech being different than the second user speech;
      sending data corresponding to the third user speech to the recognition module, and receiving recognized third speech data in return, the recognized third speech data again indicating one of said plural items as of particular user interest;
      based on one or more descriptors included in the recognized third speech data, determining that a second, different one of said plural depicted items is of greater interest to the user than the first item;
      moving said marking on the device display to a location indicating said second item; and taking an action based on the second item, said action including presenting information related to the second item to the user;
      wherein the device is not on heightened alert all the time, but is cued into activation from a lower activity state by the cueing expression, thereby bounding the device's processing efforts, and the descriptors in the recognized second and third speech data iteratively guide the device in identifying which of the plural items in the user's physical environment is of user interest, thereby further bounding the device's processing efforts.
US 10,475,142 B2 (ELWHA LLC), entitled EVIDENCE-BASED HEALTHCARE INFORMATION MANAGEMENT PROTOCOLS, and filed on December 5th, 2012 discloses a healthcare information management system comprising:
   at least one network device including one or more electronic devices including at least:
   at least one camera configured for capturing at least one image;
   at least one display device configured at least for displaying the at least one image;
   circuitry configured for obtaining at least one user input selecting a specific portion of the at least one image displayed on the at least one display device for depicting at least a portion of one person of one or more persons present in the at least one image;
   circuitry configured for recognizing the one person of the one or more persons via at least automatic facial recognition applied to the at least one image;
   circuitry configured for obtaining medical data about the one person of the one or more persons responsive to the at least one user input including at least triggering acquisition of one or more images associated with the one person, evaluating the one or 55 more images in relation to the at least one health regimen, and deriving at least one performance metric indicative of compliance of the one person with at least one health regimen;
   circuitry configured for detecting, via at least one sensor, whether the one person is within a predetermined vicinity of at least one dispenser;
   circuitry configured for authorizing or declining to authorize at least one dispensation of at least one of a drug or a treatment based at least partly on the at 65 least one performance metric indicative of compliance of the one person with at least one health regimen and the detection that the one person is within the predetermined vicinity of the at least one dispenser; and
   circuitry configured for facilitating a dispensing, via the at least one dispenser, the at least one of the drug or the treatment responsive to authorization of the at least one dispensation.
US 2021/0264710 A1 (UNIVERSAL CITY STUDIOS LLC), entitled QUEUE MANAGEMENT SYSTEM AND METHOD, and filed on May 10th, 2021 discloses a queue management system, comprising:
   a detection system configured to output an entry signal in response to detection of a portable identification feature of a guest traversing an entrance into an amusement park, the amusement park comprising a plurality of attractions therein; and
   a data server system comprising one or more processors configured to:
      receive an attraction list having guest selections of two or more attractions of the plurality of attractions;
      receive the entry signal indicating that the guest is traversing the entrance into the amusement park;
      determine, in response to receiving the entry signal, operational status data for the two or more attractions of the attraction list;
      generate, in response to receiving the operational status data, a proposed itinerary for the guest based at least on the attraction list and the operational status data; and
      provide the proposed itinerary to the guest.

Given the state-of-the art in device electronics, device apps, health sensors, environment sensors, GPS, LPS, access control systems, kiosks, computer and communication systems and other arts as will be recognized within the present specification, it is now possible to implement a beneficial mutual health assurance system based upon the novel teachings herein provide.

### BRIEF DESCRIPTION OF THE SEVERAL VIEWS OF THE DRAWING

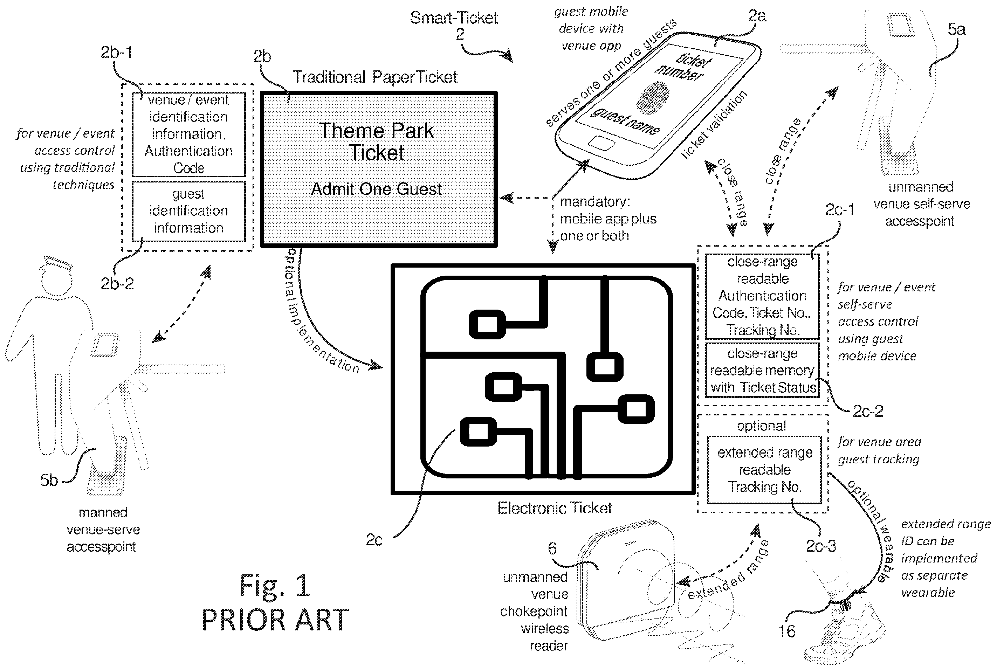

(PRIOR ART) Fig. 1 is a pictorial-component diagram showing a smart-ticket 2 and its scanners including unmanned venue self-serve access point 5a and unmanned venue chokepoint wireless reader 6. Smart-ticket 2 comprises at least guest cell-phone and venue app 2a combined with either or both traditional paper ticket 2b or electronic ticket 2c. Paper ticket 2b comprises venue / event identification information 2b-1 and guest identification information 2b-2. Electronic ticket 2c comprises close-range readable authentication code, ticket number and tracking number 2c-1, close-range readable memory with ticket status 2c-2 and optional extended range readable tracking number 2c-3, where optional tracking number 2c-3 can alternatively be implemented in a wearable, such as anklet 16. Also shown is manned venue serve access point 5b for at least using the smart-ticket 2 to permit venue access.

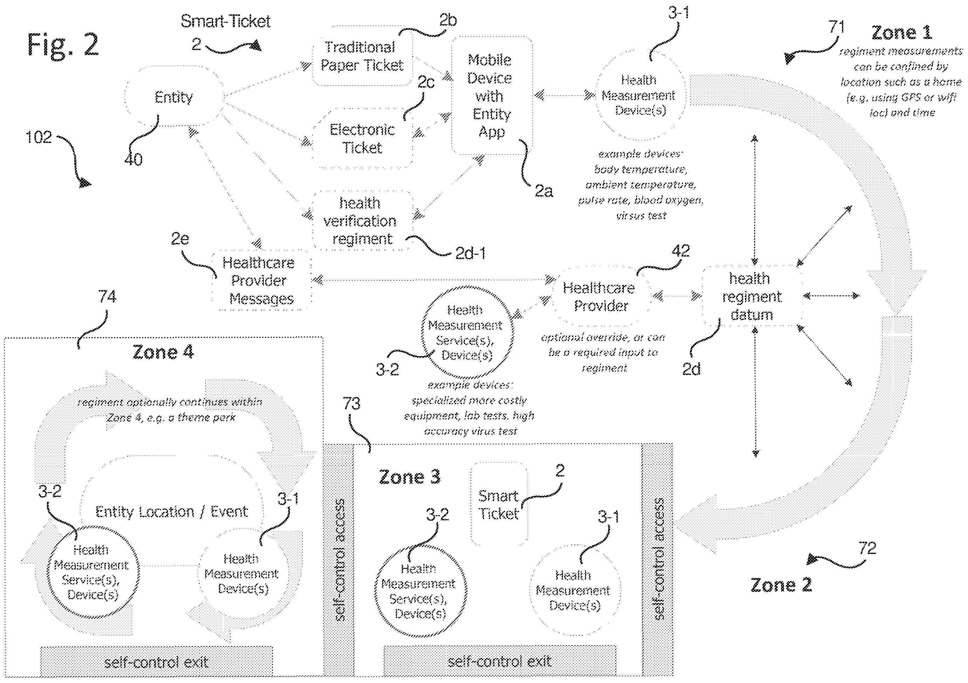

Fig. 2 there is shown a block diagram of mutual assurance system 102 comprising the PRIOR ART smart-ticket 2 including mobile device with entity app 2a, traditional paper ticket 2b and electronic ticket 2c, all as preferably issued by entity 40 owning or otherwise in control of access onto a premises ZONE4 74. Smart-ticket 2 is further adapted to receive one or more health verification regiments 2d-1 and to interact with one or more self-operated health measurement devices 3-1 or other-operated health measurement service(s), device(s) 3-2 for determining health regiment datum 2d responsive to a regiment 2-d1, where the measurements are taken in any of ZONE1 71 substantially away from the premises ZONE4 74, open ZONE2 72 in between ZONE1 71 and ZONE3 73, and enclosed ZONE73 in between open ZONE2 72 and enclosed premises ZONE4 74. Entity 40 optionally communicates with a healthcare provider 42 through healthcare provider messages 2e.

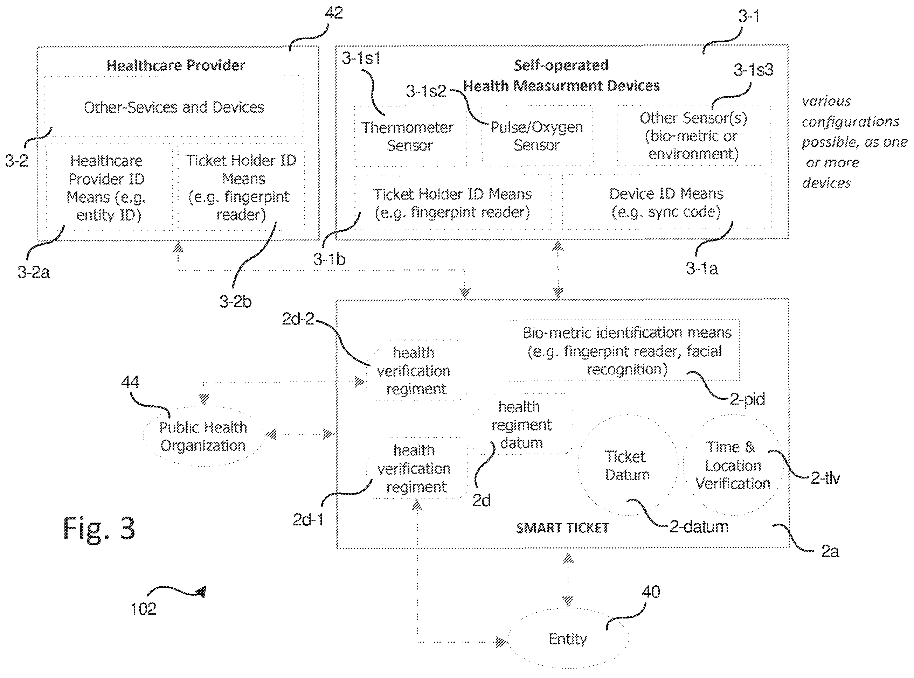

Fig. 3 is a block diagram of mutual assurance system 102 depicting key components of smart-ticket 2, self-operated health measurement devices 3-1 and healthcare provider 42 including other-operated services and devices 3-2. Smart-ticket 2 is shown to be capable of receiving a health verification regiment 2d-1 from an entity 40 that also issues / provides other elements of the smart-ticket such as the entity app and ticket datum 2-datum. Also depicted is a public health organization 44 that can also provide a health verification regiment 2d-2 to the smart-ticket 2. Smart-ticket 2 preferably includes bio-metric identification means 2-pid for confirming a person to be the registered ticket holder as well as time & location verification means 2-tlv for providing time and location datum in accordance with the teachings herein. Self-operated devices 3-1 include various health sensors 3-1-s1, 3-1-s2 and other sensors 3-1s3 as well as ticket holder ID means 3-1b for use in confirming that the ticket holder is the person for which a health measurement(s) have been taken by sensors 3-1-s1, 3-1-s2. Devices 3-1 preferably also include device ID means 3-1a for uniquely identifying a device 3-1 when communicating with smart-ticket 2. Healthcare provider 42 also comprises a ticket holder ID means 3-2b and a healthcare provider ID means 3-2a.

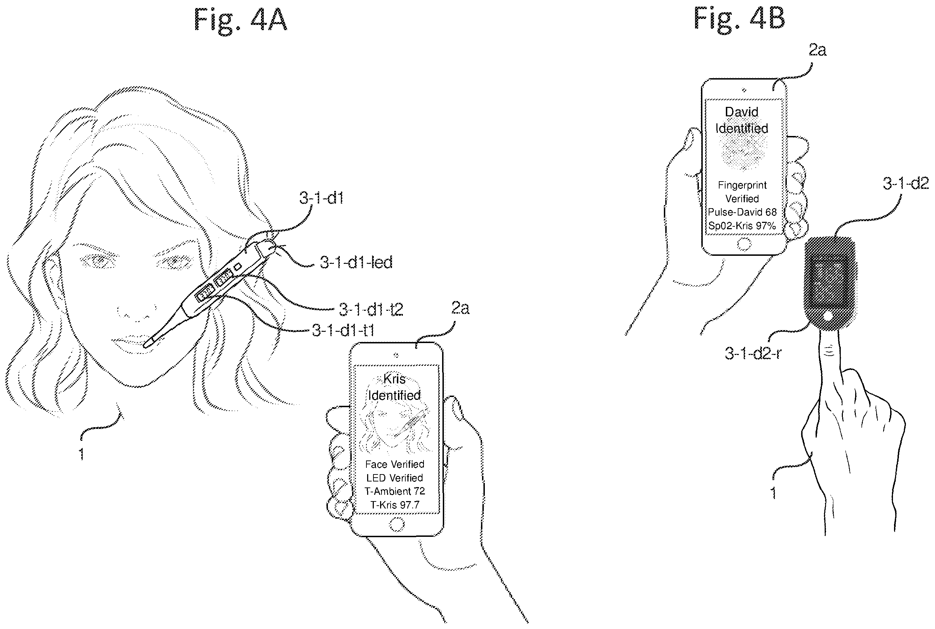

Fig. 4A is a pictorial depiction of a person 1 who is a ticket holder of smart-ticket 2 being imaged by smart-ticket 2 while taking a health measurement using a self-operated thermometer 3-1-d1. Thermometer 3-1-d1 comprises optional display 3-1-d1-t1 for providing a first temperature of person 1 as well as optional display 3-1-d1-t2 for providing a second temperature of the environment. Thermometer 3-1-d1 further comprises exemplary device ID means 3-1a that is a light output device such as LED 3-1-d1-led. Smart-ticket 2 is depicted as using bio-metric ID means 2-pid to confirm person 1 using facial recognition while substantially simultaneously communicating with self-operated device 3-1-d1 to cause an ID signal emitted using LED 3-1-d1-led for detection and confirmation by smart-ticket 2. Thermometer 3-1-d1 communicates health measurements to smart-ticket 2 for recording responsive to a health regiment 2d-1, 2d-2.
Fig. 4B is a pictorial depiction of a person 1 who is a ticket holder of smart-ticket 2 having their fingerprint detected by reader 3-1-d2-r comprised within pulse rate / oxygen sensor 3-1-d2 substantially simultaneously as a health measurement is being taken by the self-operated pulse/oxygen sensor 3-1-d2. Pulse rate / oxygen sensor 3-1-d2 communicates person 1 fingerprint and health measurements to smart-ticket 2 for recording responsive to a health regiment 2d-1, 2d-2.

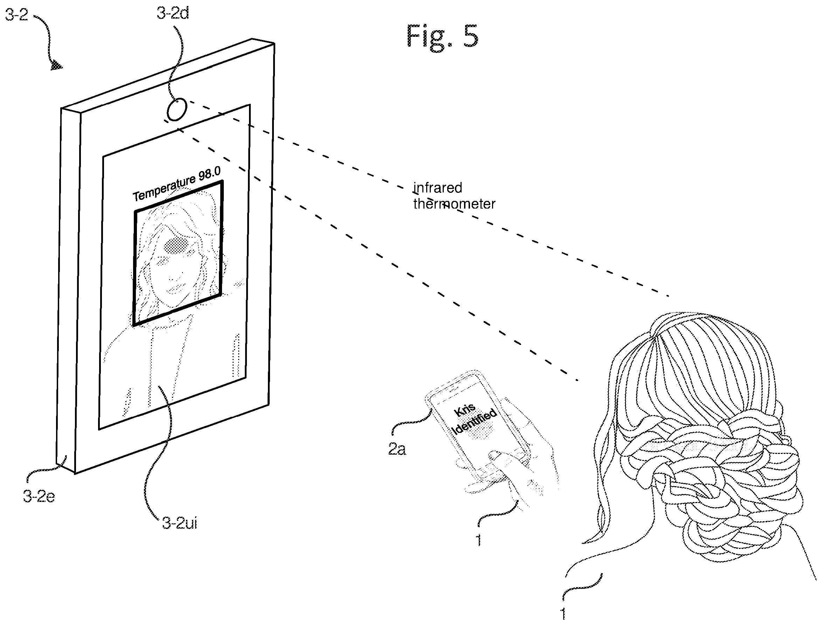

Fig. 5 is a pictorial depiction of a health-check kiosk 3-2 being used by a person 1 presumably situated near a point-of-access into a premises or a restricted area, where person 1 uses their smart-ticket 2 to both confirm their identity as a ticket holder 1 and to receive at least one biometric measurement such as a body temperature reading for example taken remotely by infrared thermometer 3-2d within casing 3-2e. Person 1 is preferably guided by mirror or display device 3-2ui while correctly positioning themselves to be scanned for the determination of the biometric.

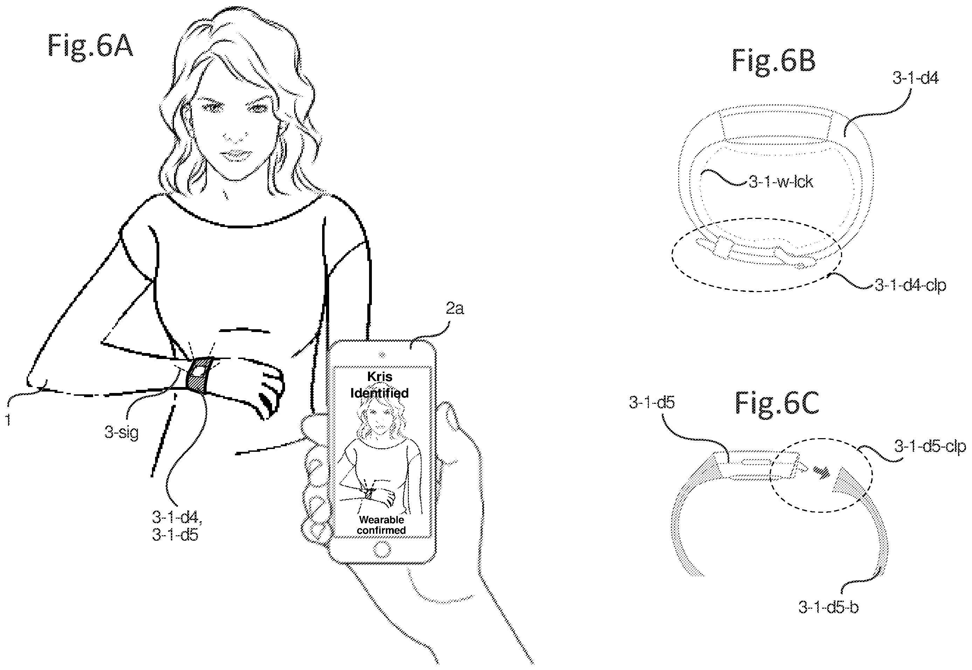

Fig. 6A is a pictorial depiction of a person 1 wearing a self-operated device 3-1-d4 or 3-1-d5 that is for example clasped to the wrist of person 1. Person 1 and device 3-1-d4 or 3-1-d5 are shown as being imaged together by smart-ticket 2 such that the identity of person 1 and the device identity of device 3-1-d4 or 3-1-d5 are confirmable using for example a single image or series of images captured by a camera on the smart-ticket 2. Wearable 3-1-d4 or 3-1-d5 preferably includes light output means for emitting verification signal 3-sig that is useable at least in part for confirming the device identity.
Fig. 6B depicts a side-view of wearable health measurement device 3-1-d4 comprising a wearable locked path 3-1-w-lck including band clasp 3-1-d4-clp.
Fig. 6C depicts a side-view of wearable health measurement device 3-1-d5 comprising a wearable locked path 3-1-w-lck (not depicted) including base clasp 3-1-d5-clp.

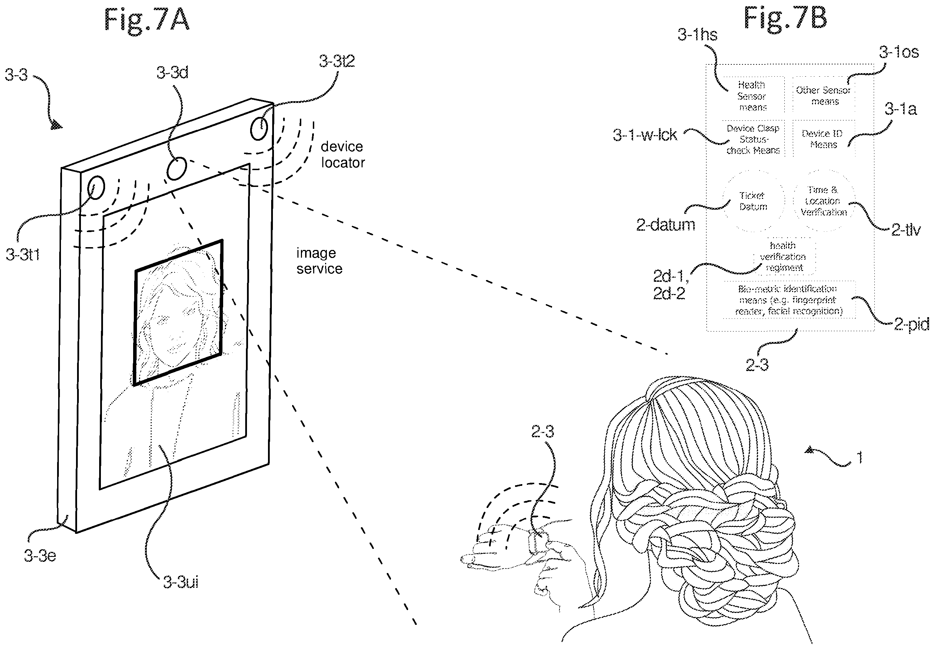

Fig. 7A is a pictorial depiction of an ID confirmation service kiosk 3-3 being used by a person 1 presumably situated near a point-of-access into a premises or a restricted area, where person 1 uses their wearable smart-ticket 2-3 in combination with the kiosk 3-3 to both confirm their identity and provide health regiment information or verification of sufficient health retirement information to the kiosk 3-3, where kiosk 3-3 may be connected to an entity system associated with the point-of-access. Kiosk 3-3 preferably includes enclosure 3-3e enclosing or holder mirror or display user interface 3-3ui, image service 3-3d and device locators such as 3-3t1 and 3-3t2.
Fig. 7B is a block diagram showing preferred and optional components of wearable smart-ticket 2-3 including: personal bio-metric identification means 2-pid, time & location verification means 2-tlv, ticket datum 2-datum, health verification regiment 2d-1 or 2-d2, device clasp status-check means 3-1-w-lck, device id means 3-1a, health sensor means 3-1hs and other sensor means 3-1os.

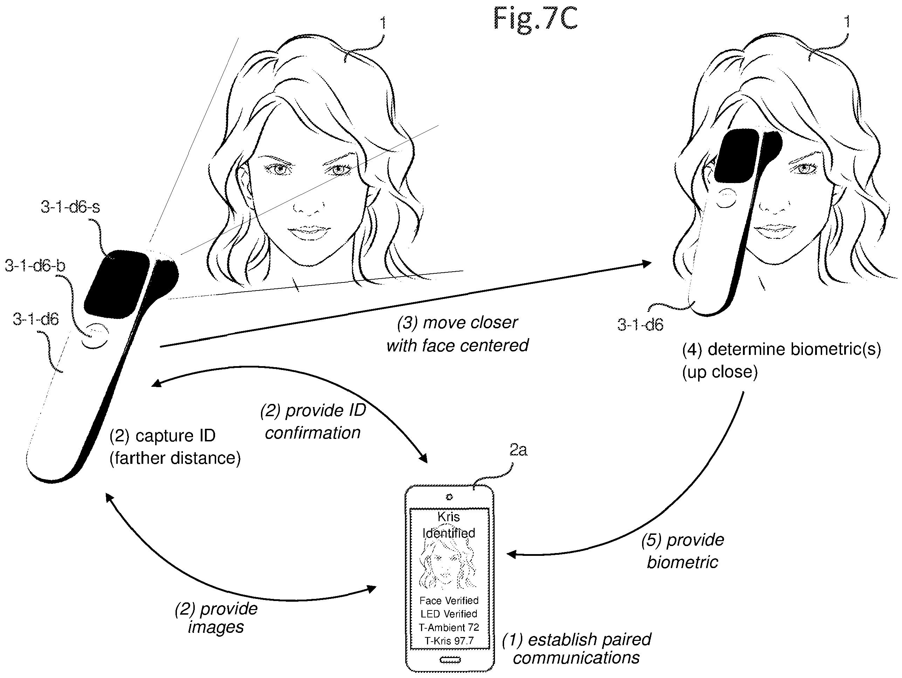

Fig. 7C is a pictorial diagram of process steps and operational movement of a "no-touch" thermometer 3-1-d6 in combination with a smart-ticket 2, where thermometer 3-1-d6 preferably includes a wireless communications link, can image capture device for capturing images of a person 1, a button 3-1-d6-b and a screen 3-1-d6-s.

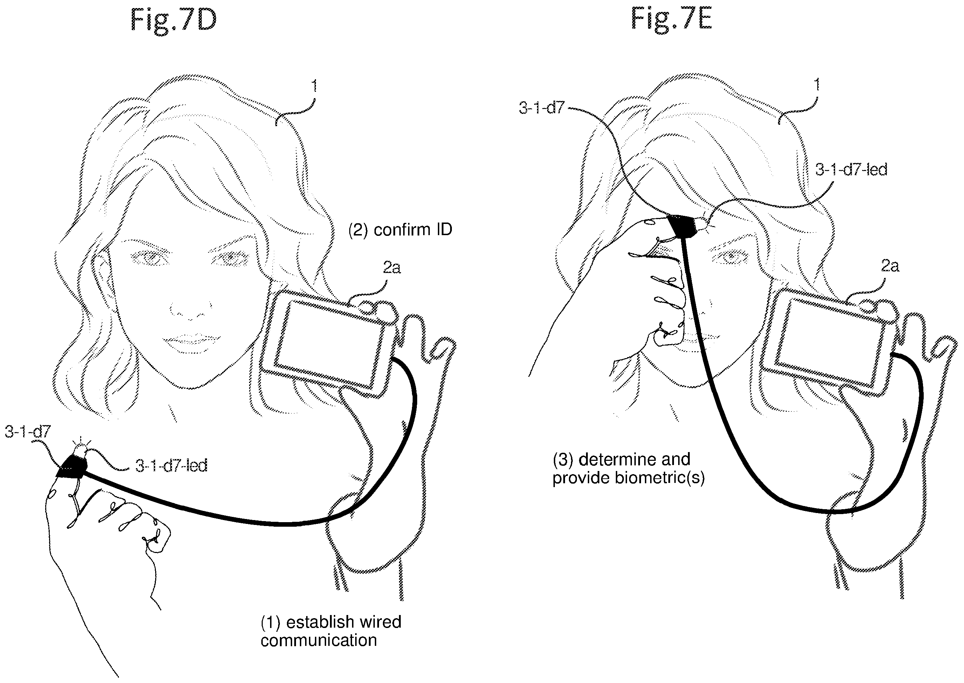

Fig. 7D is a pictorial diagram of a person 1 using a smart-ticket 2 in combination with a wired touch thermometer 3-1-d7 preferably comprising a contact thermometer and light emitting means 3-1-d1-led.
Fig. 7E is a pictorial diagram of person 1 touching thermometer 3-1-d7 to their forehead for the purpose of determining their a biometric such as their body temperature.

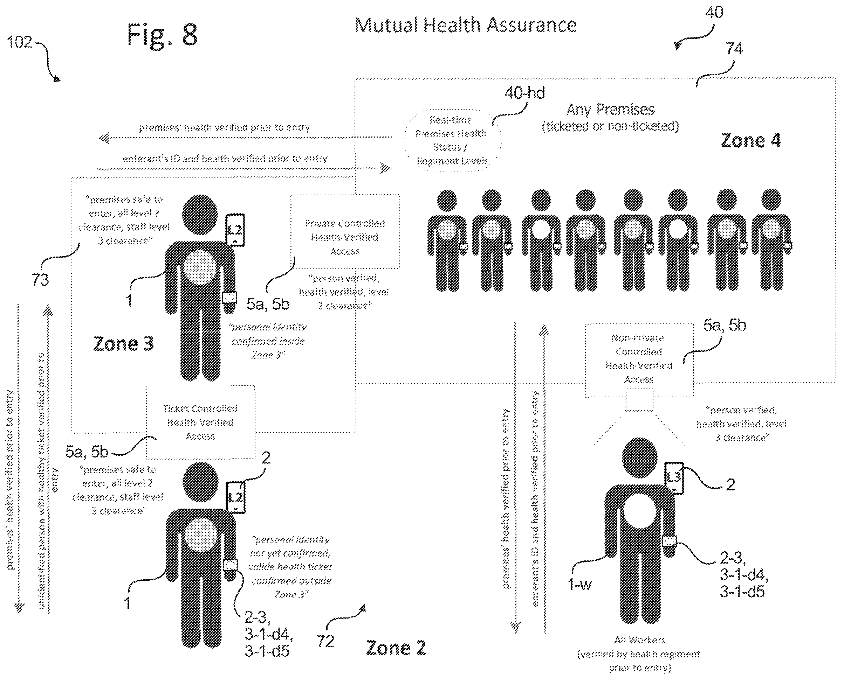

Fig. 8 is a block diagram of mutual health assurance system 102 comprising any premises ZONE4 74 as owned or otherwise operated by an entity 40, where ZONE4 74 has one or more access points 5a or 5b that are either of private controlled health-verified access points or non-private controlled health verified access points. Also shown is confirmation ZONE3 73 adjoining a private controlled health-verified access point 5a, 5b and a ZONE3 73 ticket controlled health-verified access point 5a, 5b. A person 1 that is presumably a visitor to premises ZONE4 74 is shown as carrying smart-ticket 2 and / or wearing wearable smart-ticket 2-3 and proceeding from a ZONE2 72 into ZONE3 73 and thereafter ZONE4 74. Also shown is a person 1-w that is presumably a worker proceeding directly from ZONE2 72 into ZONE4 74 via a non-private controlled health-verified access point 5a, 5b. Any premises ZONE4 74 is shown to comprise real-time premises health status / regiment levels data 40-hd for use in providing assurance of the overall health of the premises 74 to a person 1 or 1-w.

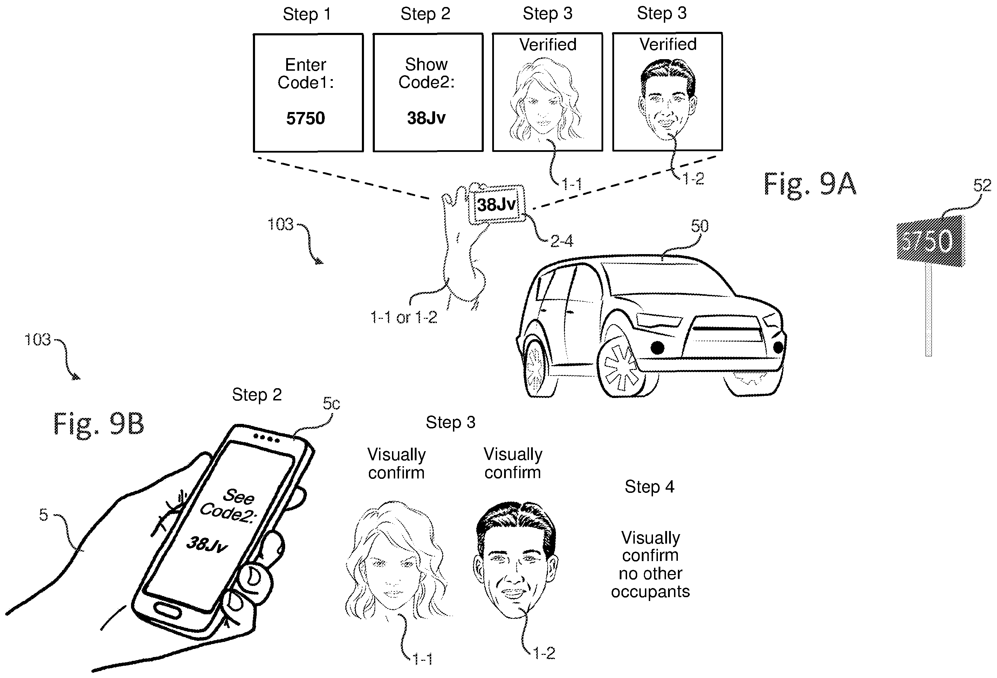

Fig. 9A is a pictorial representation of a health-verified guarded checkpoint system 103, where system 103 is a variation of the present invention 102. System 103 comprises smart-access mobile device 2-4 for entering a code1, generating and presenting a code2, and presenting confirmation images in order to gain access to a premises, where code1 is presented by a signage 52 displayed at a guarded premises driven to by persons 1-1 and 1-2 in a vehicle 50.
Fig. 9B is a pictorial representation of a health-verified guarded checkpoint system 103 showing guard 5 using access point device 5c to visually verify code2 and images 1-1 and 1-2 presented by a person using smart-access mobile device 2-4.

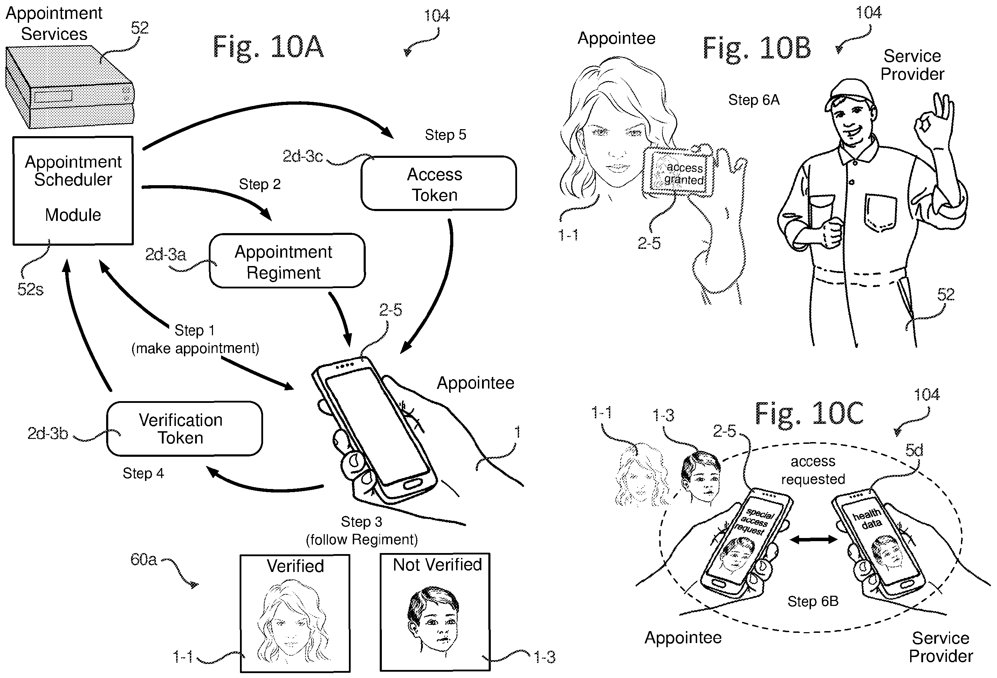

Fig. 10A is a combination pictorial and block diagram representation of health-verified appointment system 104, where system 104 is a variation of the present invention 102 sharing some similarities with variation system 103. In appointment system 104, a person such as 1-1 or 1-3 uses health-verified appointment device 2-5 to communicate with an appointment scheduler module 52s for conducting steps 1 through 5 in a health-verified appointment process.
Fig. 10B depicts a premises access step 6A in relation to appointment system 104.
Fig. 10C depicts an alternative premises access step 6B in relation to appointment system 104. In the following description, numerous specific details are set forth, such as examples of specific components, types of usage scenarios, etc. in order to provide a thorough understanding of the present disclosure. It will be apparent, however, to one skilled in the art that the present disclosure may be practiced without these specific details and with alternative implementations, some of which are also described herein. In other instances, well known components or methods have not been described in detail in order to avoid unnecessarily obscuring the present disclosure. Thus, the specific details set forth are merely exemplary. The specific details may be varied from and still be contemplated to be within the spirit and scope of the present disclosure.

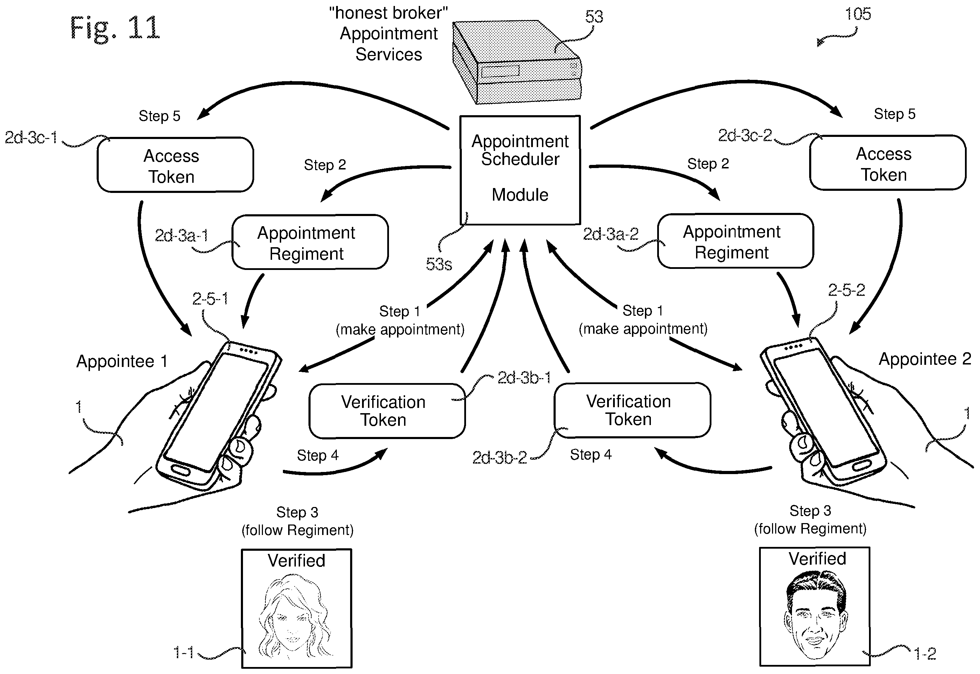

Fig. 11 is a combination pictorial and block diagram representation of health-verified honest broker appointment system 105, where system 105 is a variation of the present invention 102 sharing some similarities with variation system 103 and is an extension of appointment system 104. In honest broker appointment system 105, two or more persons such as 1-1 or 1-2 uses health-verified appointment devices such as 2-5-1 and 2-5-2 respectively to communicate with an appointment scheduler module 53s for conducting steps 1 through 5 in a health-verified appointment process prior to a planned meeting.

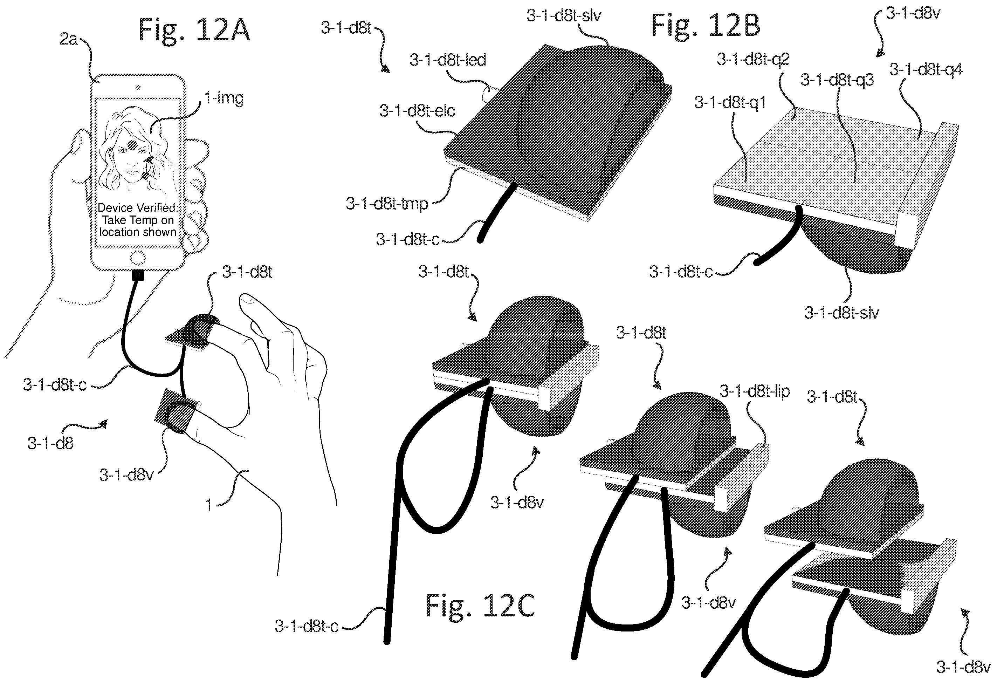

Fig. 12A is a pictorial diagram a two-piece touch electrodermal-thermometer 3-1-d8 that is a variation of one-piece touch thermometer 3-1-d7 discussed especially in relation to Fig. 7D and 7E. The variation 3-1-d8 is capable of measuring one or more electrical properties of a contact surface in addition to measuring temperature, and implements at least two basic method for verifying the proper function of the contact temperature sensor and therefore the veracity of any measured temperature.
Fig. 12B is a pictorial diagram depicting the two pieces of electrodermal-thermometer 3-1-d8. On the left a top-oriented view of thermometer-piece 3-1-d8t and on the right a bottom-oriented view of validator-piece 3-1-d8v.
Fig. 12C is a pictorial diagram showing the two pieces of electrodermal-thermometer 3-1-d8 being brought together in a pinching motion through a secession of three images moving from right to left. A convenience lip 3-1-d8t-lip is shown on validator-piece 3-1-d8v for guiding a person 1 when brining the validator-piece 3-1-d8v into proper alignment with the temperature-piece 3-1-d8t.

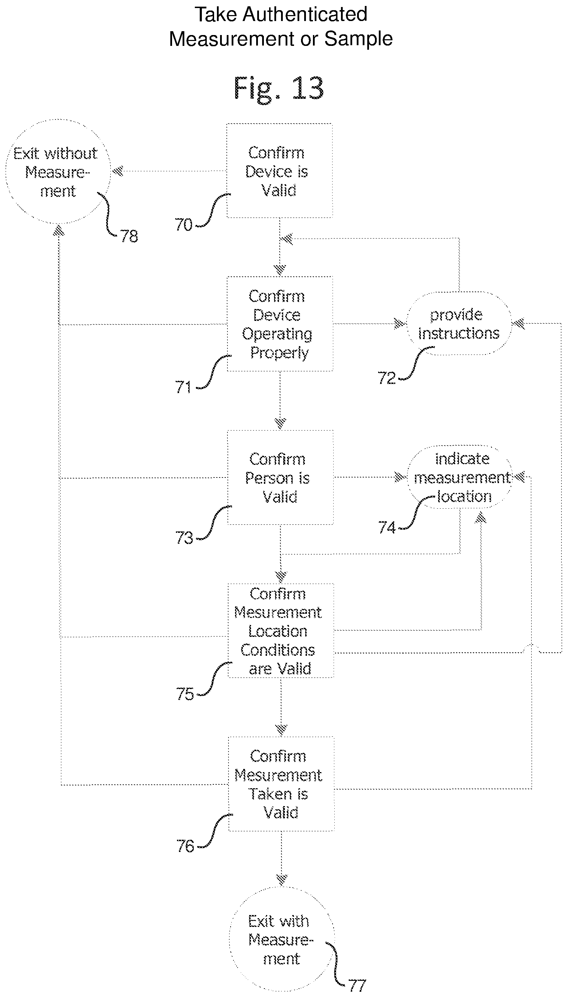

Fig. 13 is a flow diagram of preferred steps for use with any of self-operated health measurement devices 3-1 or other-operated devices 3-2. The steps include: confirming the health measurement device is valid, confirm the device is operating properly, confirming that the person using the device is the person registered to the health regiment / smart-ticket, confirming that the conditions of the measurement location (such as the forehead) are valid and confirming that the measurement has been taken.

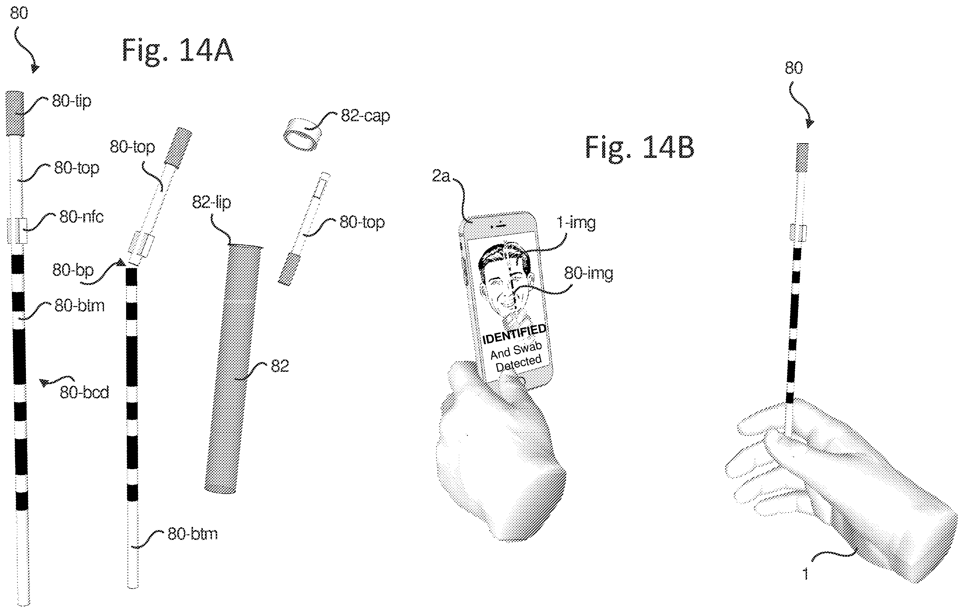

Fig. 14A is a pictorial representation of key components of an "at-home" or self-serve health test kit to be used by a person 1 for gathering biometric samples. The exemplary samples are saliva and mucus collected using a swab 80 that is breakable into two parts, a top 80-top with tip 80-tip, and a bottom 80-btm. The Top 80-top preferably includes an electronically readable tag 80-nfc for storing either or both of personalization and authentication information, while the bottom 80-btm includes visible markings 80-bcd, preferably decodable into a UPC code identify either or both the type of collection device or actual collection device. Also shown is a device container 82 with sealing lid 82-cap.
Fig. 14B is a pictorial diagram showing swab 80 being presented to device and app 2a, such that device and app 2a can substantially simultaneously image 1-img both the person 1 and the swab 80 with markings 80-bcd. This is shown to allow both verification of both the person 1 and the device (such as 80) for authenticating each collected biometric data sample.

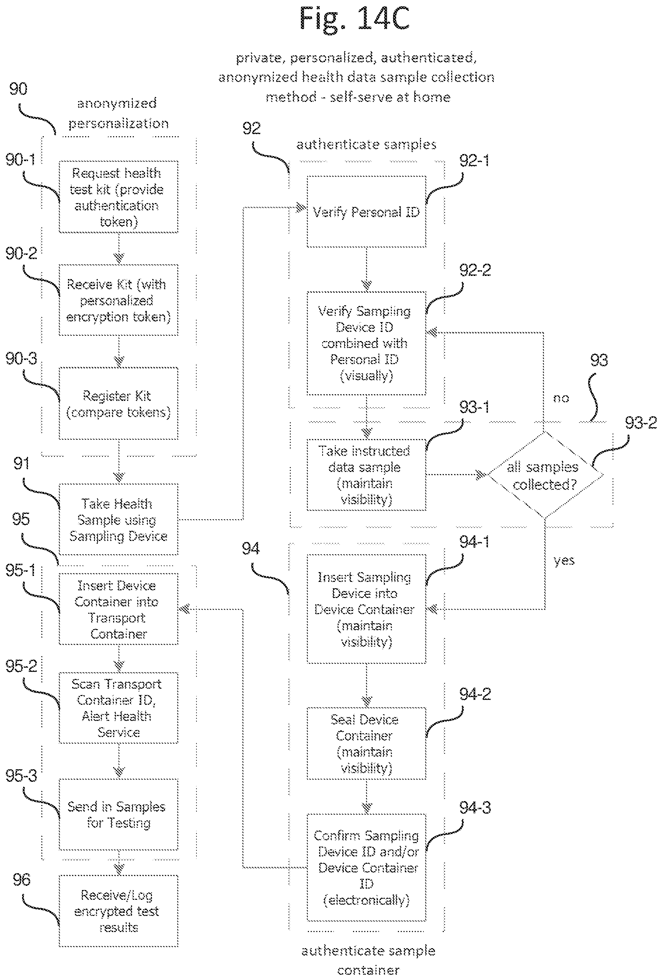

Fig. 14C is a flow diagram of the preferred steps for collecting at-home / private, personalized, authenticated, anonymized health data samples. The preferred steps include: step 90 for anonymization and personalization of a health kit, step 91 for taking samples, where step 91 comprises step 92 for authenticating each sample and step 93 for taking each actual sample, step 94 for inserting the sampling device into a container, sealing and confirming that the device is in the container, step 95 for inserting the device container into a transport container, and step 96 for receiving back to the device and app the encrypted test results based upon the collected data samples.

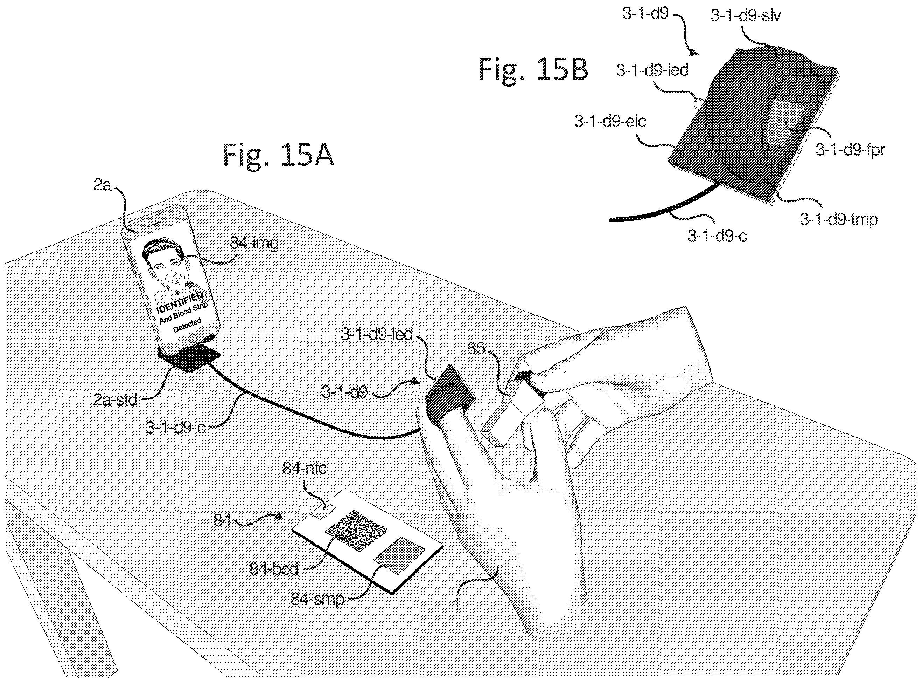

Fig. 15A there is shown a pictorial representation of an alternative at-home test kit for taking blood samples. The kit includes a traditional lancet 85 for pricking a finger causing blood to drip out of the skin, a sample strip 84 for collecting the blood sample, a stand 2a-std for holding the device and app 2a to allow a person 1 to use both their hands during the data collection process, and a fingerprint device 3-1-d9 for determining a fingerprint to be used in addition to a facial image during the data collection process in order to enhance verification of person 1. The sample strip 84 has been adapted with respect to traditional implementations to further include either or both an electronically readable tag 84-nfc (for storing at least either or both of health test information and authentication information) and visible markings 84-bcd for use in confirming the type or actual ID of the sample strip 84.
Fig. 15B is a pictorial diagram of fingerprint device 3-1-d9, where the fingerprint reader 3-1-d9-fpr has been added to a touch thermometer device as prior depicted.

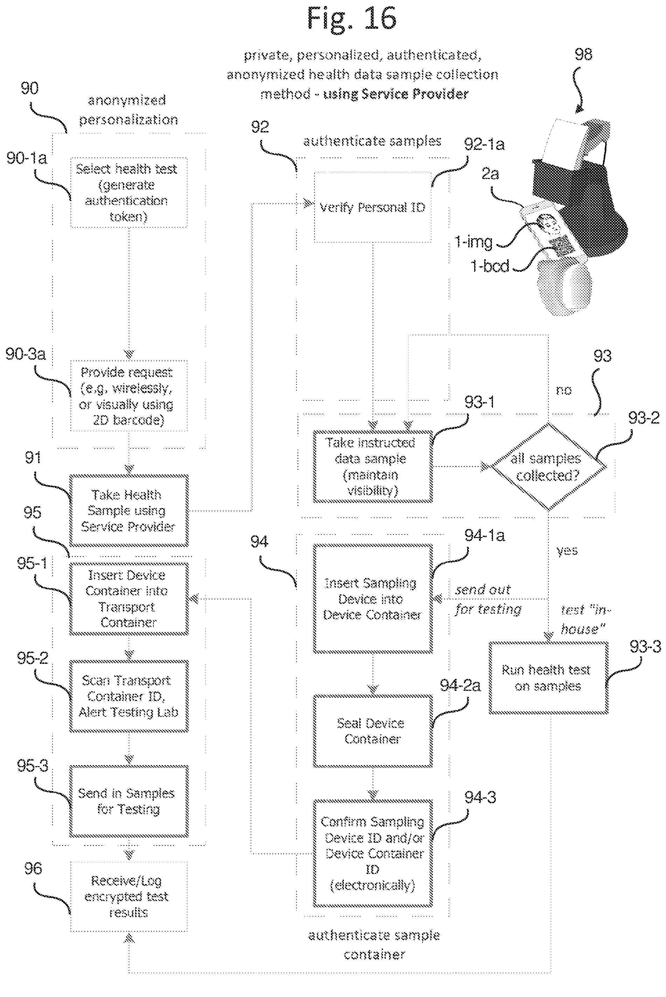

Fig. 16 is a flow diagram of the preferred steps for collecting at-service provider, private, personalized, authenticated, anonymized health data samples. Flow diagram 16 is a parallel with Fig. 14C with adaptations for use at a service provider (versus at-home) shown in diagram symbols with thicker borders. Also shown is a bar code scanner 98 for reading personalization and authentication data from the device and app 2a.

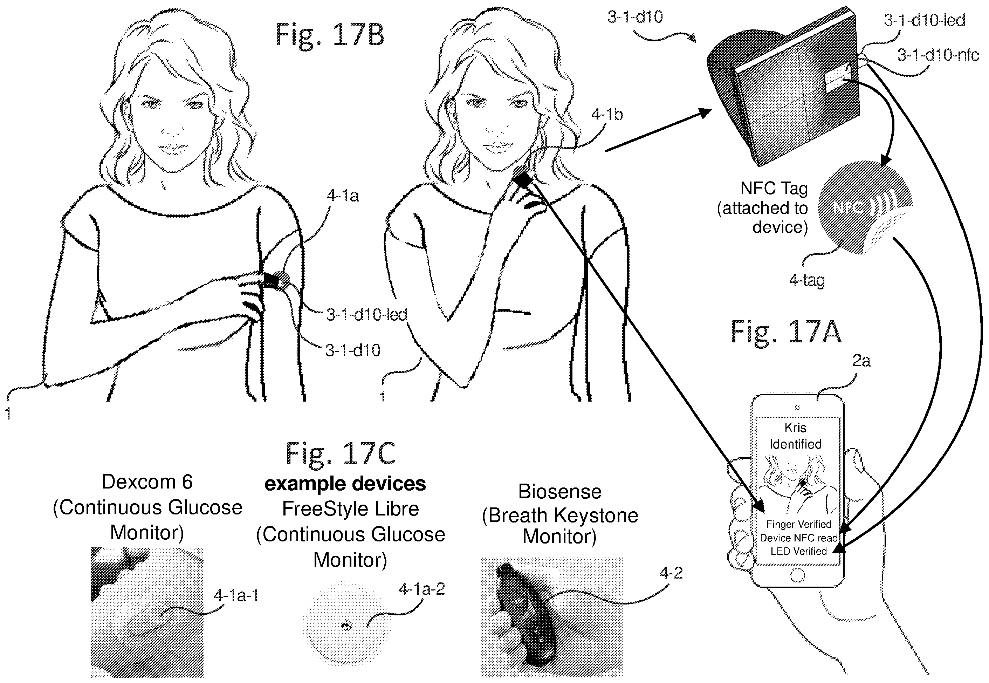

Fig. 17A is a pictorial diagram of a mobile device and app 2a being used in conjunction with a finger-worn device 3-1-d10. Device 3-1-d10 comprises light emitting component 3-1-d10-led such as an LED for use in authenticating device 3-1-d10 via its interaction with device and app 2a. Device 3-1-d10 is further adapted to comprise electronic tag reader 3-1-d10-nfc, such as an NFC reader, for use in reading and writing to electronically readable tags 4-tag such as an NFC tag that can be placed upon a health measurement device for use in authenticating the health measurement device via its interaction with device 3-1-d10.
Fig. 17B is a pictorial diagram showing a person 1 with exemplary health measurement devices including arm patch 4-1a and neck patch 4-1b. Person 1 is wearing finger-worn device 3-1-d10 that has been authenticated via interaction with mobile device and app 2a, where the interaction includes the emitting of light via light emitting component 3-1-d10-led. Person 1 is also shown substantially touching patches 4-1a and 4-1b such that finger-worn device 3-1-d10 is able to sense electronically readable tags such as 4-tag placed onto patches 4-1a and 4-1b while preferably the face of person 1, device 3-1-d10 and patch 4-1a, 4-1b are being simultaneously imaged by mobile device and app 2a, where the apparatus and method provides for authentication of patches 4-1a, 4-1b.
Fig. 17C is a pictorial diagram showing exemplary health measurement devices including continuous glucose monitoring patches Dexcom 6 and FreeStyle Libre, as well as breath analyzer Biosense that can be authenticated using the teachings related to Fig. 17A and 17B.

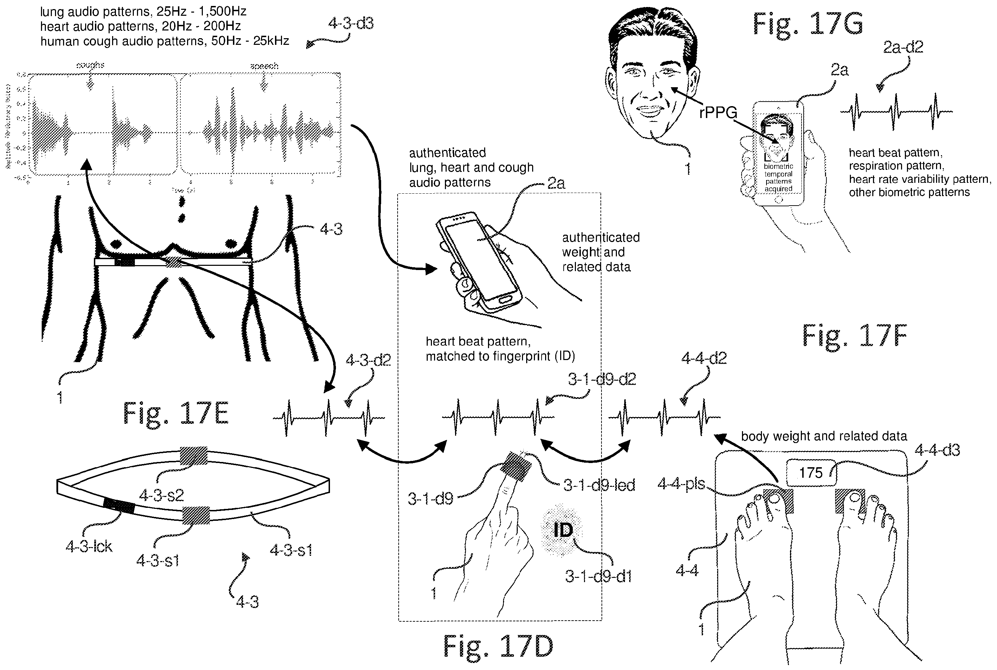

Fig. 17D is a pictorial diagram depicting mobile device and app 2a interacting with and authenticating a finger-worn device 3-1-d9 comprising a fingerprint reader for determining the fingerprint ID 3-1-d9-d1 of person 1. Fingerprint ID 3-1-d9-d1 along with the face of person 1 are considered to be "inter-identification" means each comprising a substantially unique spatial pattern useful for differentiating between persons. Finger-worn device 3-1-d9 is also configured to capture at least one "intra-identification" means including a temporal pattern such as the heart beat / pulse pattern 3-1-d9-d2 of person 1, where the temporal pattern is useful for determining if multiple health measurement devices are concurrently taking measurements of the same or different persons, thus providing means for authenticating additional health measurement devices.
Fig. 17E is a pictorial diagram of a chest band 4-3 comprising a lock 4-3-lck for indicating that band 4-3 has been fastened to the torso of a person 1. Chest band 4-3 further comprises one or more sensor groups such as 4-3-s1 and 4-3-s2. Preferred sensors including MEM microphones capable of detecting lung, heart and cough patterns using audible signals, where at least temporal heart patterns 4-3-d2 are comparable with finger-worn device 3-1-d9 determined heart patterns 3-1-d9-d2 serving as a means for authenticating chest band 4-3.
Fig. 17F is a pictorial diagram of a smart scale 4-4 for determining body weight 4-4-d3 and related metrics about a person 1. Scale 4-4 is configured to determine a temporal body pattern such as heart beat pattern 4-4-d2 of the person 1, for example using a pulse sensor 4-4-pls, where patterns 4-4-d2 are comparable with finger-worn device 3-1-d9 determined heart patterns 3-1-d9-d2 serving as a means for authenticating scale 4-4.
Fig. 17G is a pictorial diagram of mobile device and app 2a shown capturing an image of person 1 and using an imaging technology known as "rPPG" or "w" to at least determine one temporal body pattern such as heartbeat pattern 2a-d2, where heart beat pattern 2a-d2 is usable for intra-identification similar to heart beat pattern 3-1-d9-d2 determined by authenticated finger-worn device 3-1-d9.

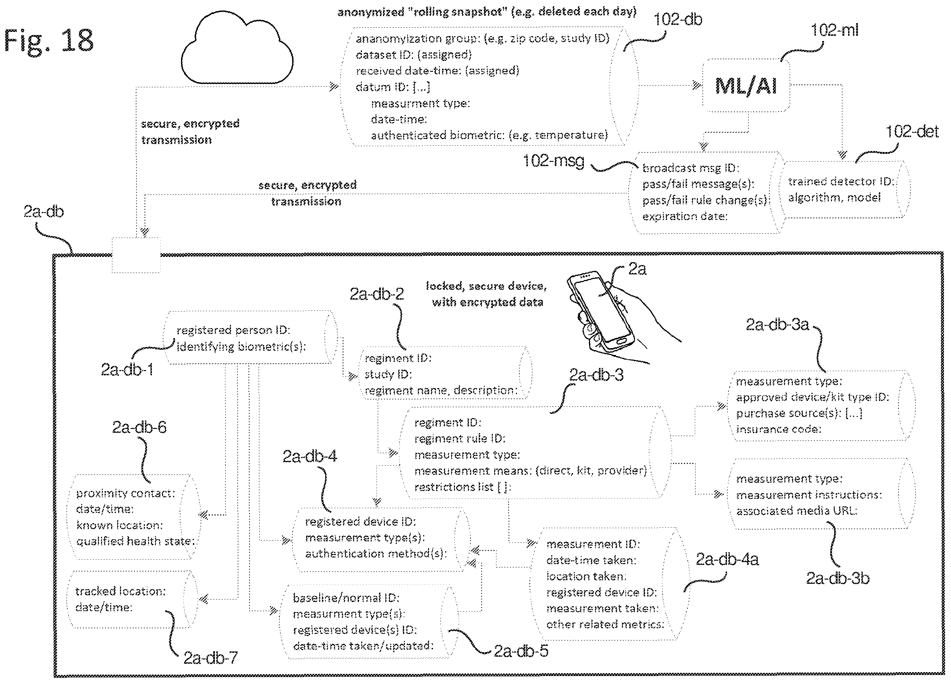

Fig. 18 is a block diagram depicting mobile device and app 2a comprising preferred datasets 2a-db including regiments and health measurement data 2a-db-1, 2a-db-2, 2a-db-3, 2a-db-3a, 2a-db-3b, 2a-db-4, 2a-db-4a, 2d-db-5 and optionally contact tracing quantification and qualification data 2a-db-6, 2a-db-7, some data of which is preferably transmitted to a remote database 102-db as a "rolling snapshot" of anonymized health information especially useful for analysis using algorithms known as machine learning and artificial intelligence (ML/AI) 102-ml. As a result of ML/AI or similar analysis, anonymous broadcast messages 102-msg including trained detectors 102-det such as ML data models are generally distributed to all known mobile device and apps 2a at least associated with database 102-db, where each mobile device and app 2a uses the message and / or trained detector 102-det to analyze local datasets 2a-db and provide information to a registered person based at least in part upon the results of the analysis.

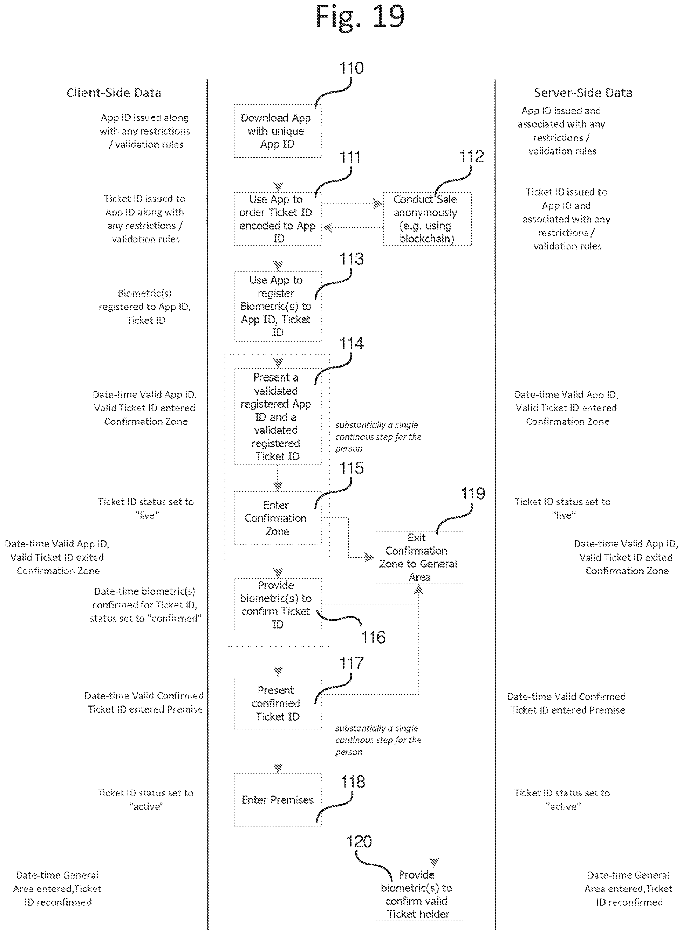

Fig. 19 is a flowchart for an alternative apparatus and method for a smart-ticket 2 as originally taught by Aman, et. al. with respect to the cross-referenced related application U.S. Patent No. 10,861,267 entitled THEME PARK GAMIFICATION, GUEST TRACKING AND ACCESS CONTROL SYSTEM. Like the prior art, the flow chart describes apparatus for using a confirmation zone to aid in the method for assuring a premise that a person 1 desiring self-access has both a valid "right-to-access" and is the authenticated / registered (i.e., "confirmed") holder of this right. The present teachings extend the prior art by employing a data-time fence in combination with a physically restricted confirmation area.

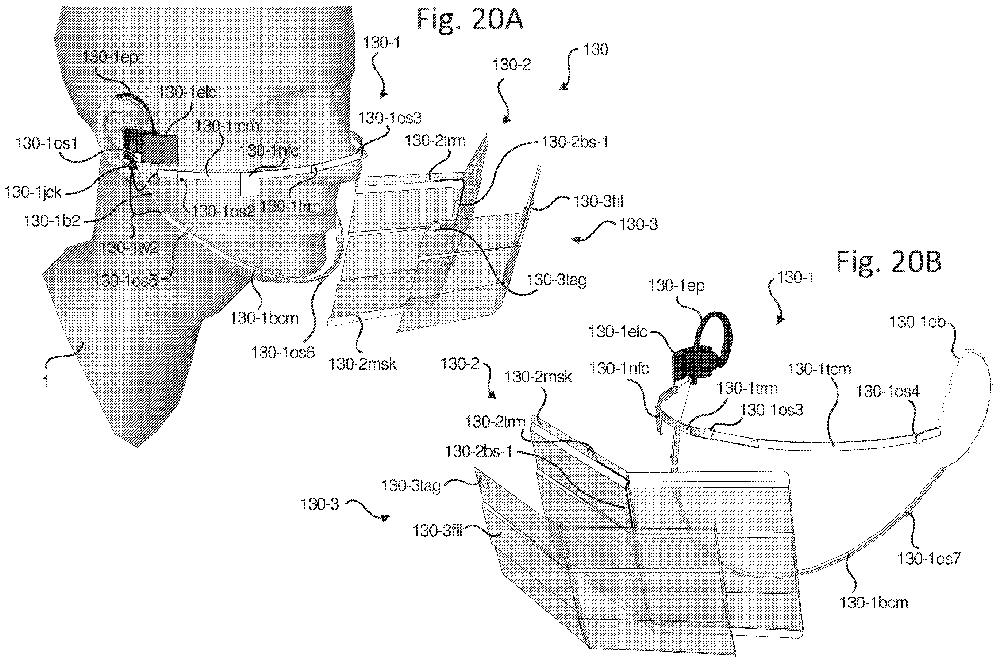

Fig. 20A and 20B are pictorial diagrams depicting two different perspectives of active mask 130 being worn by person 1. Active mask 130 comprises three main parts: a) mask frame and electronics 130-1, b) mask with breath sensor 130-2, and c) mask filter insert with electronic tag 130-3, where each part comprises multiple components. Active mask 130 preferably provides any one of or any combination of the following "active mask" functions (AM-functions): 1) communicating with mobile device and app 2a (or equivalent), 2) contact tracing using wireless communication means, 3) contact proximity notification to wearer, 4) mask fit determination, 5) filter insert determination, 6) contact qualification using at least fit and filter insert determination, 6) geo-location determination where for example mask 130 detects a wireless beacon or uses GPS or LPS technology to indicate that the mask and therefore the wearer 1 is now for example in a "public area XX" (where a mask is required) or in a "private area YY" (where a mask is optional), 8) communication with mask usage tracking system, and 9) mask usage tracking via mobile device and app 2a.

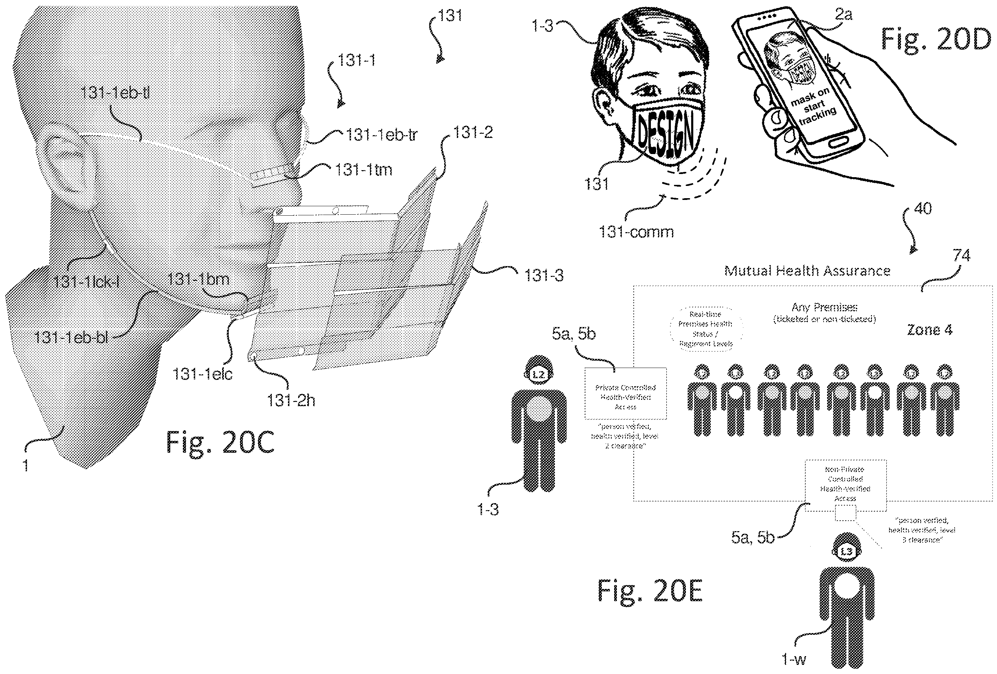

Fig. 20C is a pictorial diagram depicting an alternative active mask 131 that is expected to be lower in production costs than active mask 130. Like mask 130, alternative mask 131 comprises three main parts, mask frame and electronics 131-1, mask 131-2 and mask filter insert 131-1. Also like mask 130, mask 131 provides any one of or any combination of the active mask functions (AM-functions).
Fig. 20D is a pictorial diagram showing a child 1-3 wearing an active mask 131 that is in communications via wireless signal 131-comm with mobile device and app 2a being operated by a parent or guardian. Operation includes pairing between active mask 131 and device and app 2a, authentication of wearer 1-3 with respect to active mask 131, the initiating of active mask AM functions and any subsequent data exchanges related to the AM functions. Active mask 131 may be alternatively worn by a person such as child 1-3 with the same described functionality. Active mask 130 is shown comprising a decorative mask 131-2 and is taught to support multiple types of passive and active "add-on" decorations.
Fig. 20E is a block diagram showing a premise (referred to as "Zone 4", 74) owned or operated by an entity 40 that is implementing preferably both an authenticated health regiment 2d-1, 2d-2 and contact tracing, where access to the premise is controlled though any one of or any combination of one or more private controlled health-verified access points 5a, 5b connected through an adjoining confirmation Zone 3 (not depicted) for providing access to a "visitor" 1-3, or any one or more of non-private controlled health-verified access points 5a, 5b connected through an adjoining confirmation Zone 2 (not depicted) for providing access to a "worker" 1-w. (See also Fig. 8.) "Visitors" such as 1-3 and "workers" such as 1-w may use any of an active mask 130, 131 to serve as their smart-ticket wearable in addition to or replacement of 2-3, 3-1-d4, 3-1-d5. Premise 74 includes a mask usage tracking system for communicating with all active masks 130, 131, such that virtually all persons such as 1-3, 1-w moving about in a premise can be provided with quantified and qualified contact tracing as well as all other AM functions or experiences based at least in part upon data related to an AM functions, where experiences include gamification of the premise.

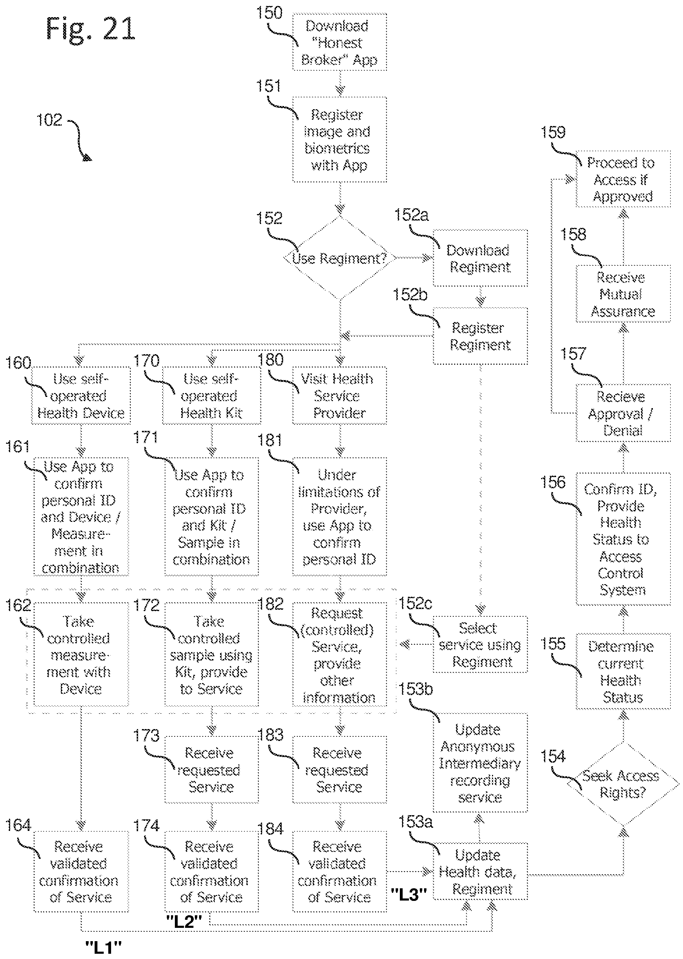

Fig. 21 is a flowchart describing the herein taught "publicly trustable" "proof-of-health" aspects of the mutual assurance system 102 at a mid-level of detail. System 102 is shown to comprise multiple interactions between person 1, self-operated authenticating health devices 3-1 (data "L1"), authenticating healthcare provider other-services and devices 3-2 (data "L2" and "L3"), and an access control system for regulating access of the person 1 to a premise or gathering. App 2a executing on a personal computing device such as a smartphone being used by the person 1 during any of the interactions is referred to as an "Honest Broker" and a "go-between" / "intermediary" that represents, authenticates, and protects the anonymity of the first-party person 1 during the interactions with the second party health devices 3-1 and other services 3-2, and the third-party access control system representing and entity 40 associated with a premise or gathering. The Honest Broker (intermediary) App 2a is shown to preferably use Regiments 2d comprising one or more rules, where the Regiments 2d are separately updatable from the App 2a and serve to define and regulate the many interactions. The system 102 is shown to provide for the "public trust" with respect to an anonymous "proof-of-health" being determined about person 1 at least in part by the Honest Broker App 2a, where this "publicly trustable" "proof-of-health" is anonymously provided by the App 2a to an access control system of a third-party. The "proof-of-health" is also generally referred to as a "current health status" preferably represented digitally as an encrypted "digital health certificate."

### DETAILED DESCRIPTION OF THE INVENTION

Fig. 1 exactly matches PRIOR ART "Fig. 1" as taught in the related U.S. Patent No. 10,861,267 filed on August 4th, 2017 and entitled THEME PARK GAMIFICATION, GUEST TRACKING AND ACCESS CONTROL SYSTEM.

Referring next to Fig. 2, there is shown mutual health assurance system 102 as a block diagram depicting smart-ticket 2 comprising either or both traditional paper ticket 2b or electronic ticket 2c as issued by an entity 40, all as described in the PRIOR ART of reference entitled THEME PARK GAMIFICATION, GUEST TRACKING AND ACCESS CONTROL SYSTEM. Entity 40 further issues a health verification regiment 2d-1 as electronic information for storage and processing by mobile device with entity app 2a. Mobile device 2a include cellphones or smartphones capable of app processing, tablets and any other mobile device sufficient for performing the functions specified in the reference art and herein regarding the smart-ticket 2. Mobile device 2a can verify the identity of the ticket holder, for example and preferably using a bio-metric method such as fingerprint or facial recognition.

Mobile device 2a is further capable of receiving, providing or otherwise exchanging information with any of self-operated health measurement devices 3-1 or other healthcare provided services using other-operated health measurement services and devices 3-2, where health measurements and related information are preferably maintained by the smart-ticket 2 as health regiment datum 2d. In accordance with the teachings of the incorporated reference art, smart-ticket 2 is capable of determining one or more zones, such as Zone 1 71, Zone 2 72, Zone 3 73 and Zone 4 74, where determination at least includes detecting the current physical location of the smart-ticket 2 with respect to the locations of each Zone for example as specified by the entity 40.

According to information provided in health verification regiment 2d-1, a ticket holder is required to obtain one or more health bio-metric measurements using any of self-operated health measurement devices 3-1 or other-operated health measurement services and devices 3-2 preferably administered by a healthcare provider 42. Entity 40 may also issue communications and / or receive communications, where the communications are depicted as healthcare provide messages 2e to and from a healthcare provider 42. Messages 2e can include specific technical instructions or requirements provided by the entity 40 to the healthcare provider 42 with respect to for example any given measurement or regiment. Messages 2e can also include transactional information from the healthcare provider 42 being transmitted to the entity 40 in relation to a given health care measurement being made with respect to a given ticket holder / person 1. To be discussed in relation to upcoming Fig. 3, public health organizations 44 (see Fig. 3) may also issue health verification regiments 2d-2 (see Fig. 3), and although not depicted in Fig.'s 2 or 3, a public health organization 44 may also provide any of healthcare provider messages 2e such as standard information on healthcare measurement services or devices 3-2 that a given entity 40 wishes to conform with.

Still referring to Fig. 2, regarding health measurements specified in a health verification regiment such as 2d-1 (or such as 2d-2 shown in upcoming Fig. 3), preferably one or more of the health measurements must be obtained within Zone 1 71 prior to an attempt to enter at least Zone 3, and also optionally Zone 2. Self-service access from Zone 1 to 2, Zone 2 to 3 and Zone 3 to 4 proceeds in accordance with the teachings of the PRIOR ART reference entitled THEME PARK GAMIFICATION, GUEST TRACKING AND ACCESS CONTROL SYSTEM with the additional provision that the health care measurements indicated as necessary by a regiment 2d-1 (or 2d-2 of Fig. 3) for a given Zone 1, 2, 3 or 4, must be also acceptably completed or overridden as allowed by the issuer of the regiment 2d-1 (or 2d-2) such as entity 40 (or entity 44) prior to entering into a next subsequent Zone 2, 3, 4 or 1.

For example, in addition to the teaching of the PRIOR ART, a ticket holder is preferably required to have obtained sufficient health measurements using any of devices 3-1 or services and devices 3-2 prior to proceeding from Zone 1 71 into Zone 2 72, but at least prior to proceeding from Zone 2 72 into Zone 3 73 through self-access points 5a (see Fig. 1) or some other access control such as an entity check point operated by an entity employee. Within Zone 3 73, a ticket holder may optionally be required to obtain a further health measurement using any of devices 3-1 or services and devices 3-2 prior to proceeding through self-access points 5a into Zone 4, a premises operated, owned or otherwise under the control of the entity 40. After obtaining one or more health measurements within Zone 3, the ticket holder may be blocked from proceeding into Zone 4 and required to exit Zone 3 through any of self-control exit points as described in the PRIOR ART or otherwise exit points. While within Zone 4, a ticket holder may optionally be required to obtain a further health measurement using any of devices 3-1 or services and devices 3-2, where upon the ticket holder may be required to either or both seek medical attention or leave Zone 4 based at least in part upon the health measurement obtained within Zone 4.

Referring next to Fig. 3, there is shown a block diagram depicting entity 40, smart-ticket device and app 2a, self-operated health measurement devices 3-1, healthcare provider 42 comprising other-operated health check services and devices 3-2 and public health organization 44. Public health organization 44, like entity 40, can provide or enable smart-ticket 2 include mobile device and app 2a as per the teachings of the PRIOR ART THEME PARK GAMIFICATION, GUEST TRACKING AND ACCESS CONTROL SYSTEM such that the mobile device and app 2a operates as a "general ticket" (i.e. with or without paper ticket 2b or electronic ticket 2c, or their equivalents) to access the premises of one or more other entities 40 that do not issue their own smart-ticket 2 or even app 2a for use on a mobile device. Public health organizations 44 may be any public organization such as a government agency, where for example a public organization is generally understood to be responsible for, or otherwise a steward of the health condition of a population operating within the organization 44's jurisdiction or influence. However, a public organization 44 can be any organization issuing a general smart-ticket 2a with a health verification regiment 2d-2 that can be adhered to by any holder of the smart-ticket 2a for use in gaining access to a premises, event or otherwise social gathering.

Like entity 40, public health organization 44 can provide a health verification regiment 2d-2, either in addition to or in replacement of entity 40 provided health verification regiment 2d-1. A regiment 2d-1 or 2d-2 specifies one or more health measurements to be taken along with any necessary limits or controlling information. Specifications at least include the type of measurement such as body temperature, pulse rate, blood oxygen levels, virus detection or tests or any of health assessments that for example can be made by healthcare providers such as doctor's offices, visiting nurses, health clinics or hospitals. In addition to specifying the type of measurement, a type of measuring device may also be specified, and even a specific unique device (for example with a unique ID) may be specified, where the unique device is optionally owned or otherwise controlled for example by the ticket holder or a guardian of the ticket holder and registered to the ticket during at least the ticket registration step (see step 4, Fig. 2 of the referenced PRIOR ART entitled THEME PARK GAMIFICATION, GUEST TRACKING AND ACCESS CONTROL SYSTEM). Unique health measurement device 3-1 registration can be accomplished for example by any of the ways well known in the art for pairing and registering one device (such as 3-1) with another device (such as smart-ticket 2a), where then once paired and / or registered a person 1 using the smart-ticket device and app 2a can then further uniquely identify themselves (such as through a finger print or facial recognition) wherein the unique person identity is then further associated with the pairing or registering device (such as 3-1).

Still referring to Fig. 3, measurements may also be specified as confined to a geographic location, such as a home, office or room, where the home, office or room can optionally be influenced or chosen by the ticket holder during at least the ticket registration step (see step 4, Fig. 2 of the referenced PRIOR ART entitled THEME PARK GAMIFICATION, GUEST TRACKING AND ACCESS CONTROL SYSTEM). Measurements may also be confined by a time of day, also optionally influenced, or chosen by the ticket holder in a manner similar to the geographic location. A measurement specification can also indicate that the measurement must be taken by a healthcare provider 42, specifying a general type of provider down to a specific provider 42, where the specific provider can optionally be influenced or chosen by the ticket holder during at least the ticket registration step (see step 4, Fig. 2 of the referenced PRIOR ART entitled THEME PARK GAMIFICATION, GUEST TRACKING AND ACCESS CONTROL SYSTEM). For example, the person 1 may choose a healthcare provider that they are familiar with or that is covered by their health insurance but is otherwise acceptable to the health regiment 2d-1 or 2d-2 issuer such as 40 or 44, respectively.

Measurements taken by a healthcare provider 42 can be and are preferably transmitted along with any other specified measurement information to the smart-ticket device and app 2a via any of communication means available to device 2a and systems or devices 3-2 operated by the healthcare provider including Bluetooth and wi-fi connections. Other specified measurement information optionally includes a healthcare provider ID provided to the smart-ticket device and app 2a via means 3-2a that may be any combination of an apparatus or method. For example, the device and app 2a could scan a NFC (near field communications) tag that is means 3-2a and then receive the healthcare provider ID. In another method, an authorized person of the health check provider 42 enters into a user interface on the device and app 2a an identifying code or provides the code to the person 1 operating the device 2a. When providing healthcare provider ID information, means 3-2a preferably also provides other qualifying information such as a location, time of day and person administering the measurement. It is also noted that the health check provider ID is not mandatory as the measurement taken by a device 3-2 may be encoded in such a way as to confirm is validity regardless of the health check provider 42 administering the health check. What is most important to see is that a health regiment can include a specification either requiring or not requiring that a health measurement is administered by an party other than the person 1 (self-operating a device 3-1), and that further this measurement is required or not required to be accompanied with health check provider ID, or even a services or device 3-2 ID.

In a preferred embodiment, healthcare provider 42 also confirms the identity of the person 1 for whom the measurement is being taken as being the ticket holder, whether a specific ticket issued by an entity 40, or a general ticket issued by a public health organization 44. The identify confirmation of the person 1 may be conducted using equipment available to the healthcare provider 40, or by personnel of the provider 40, or by interfacing with or approving a personal identification performed by the person 1 using the smart-ticket device and app 2a (e.g., by entering a fingerprint or taking a photograph that is verified by facial recognition while in the presence of the provider 40 personnel.) However, it is also possible that a health measurement is simply provided by the health care provider 42 using services or devices 3-2 to a person 1, where the person 1 for example has possession of their device and app 2a and then uses communications means on their device 2a (such as Bluetooth, wi-fi, or NFC reader) to receive the specified health measurement from service or device 3-2 preferably in a secure means. For example, a person 1 may be receiving a bio-check on a device 3-2 that can exchange secure information with a smart-ticket device, where the device 3-2 is generally more expensive but is situated in a public location and available for free access. In this use case, the health care provider 40 is not directly represented by a employee, but rather simply makes available the service or device 3-2 for example in the manner of a kiosk that is then operated by the ticket holder 1 (see especially upcoming Fig. 5 for one example service or device 3-2.)

A measurement specification within a regiment such as 2d-1 or 2d-2 can also indicate that a health measurement must be taken using the device and app 2a (see upcoming Fig.'s 4A, Fig. 4B and 4C) in a self-operated manner using a device 3-1, or optionally can be taken using either the smart-ticket 2 in a self-operated manner (with a device 3-1) or a healthcare provider 42 (with a service or device 3-2).

Any health regiment measurement specification that can be optionally influenced or chosen by the ticket holder, such as but not limited a one-time or recurring location for the measurement, a one-time or recurring time of the measurement, or the health check provider 42 for making the measurement, is optionally further limited to being approved by the health verification regiment 2d-1 or 2d-2 issuer entity 40 or entity 44, respectively. In this sense the ticket holder 1 is provided some convenience while the regiment issuer 40 or 44 is provided some final control. Such approval may be provided by a communication or transaction conducted between the smart-ticket device and app 2a and the issuing entity 40 or 42, for example via an internet connection or at least in any of well-known means for communicating between systems and devices.

In the preferred embodiment, a ticketing entity 40 such as a theme park, airline, cruise line, music concert, sporting event, etc. that pre-books reservations with a person 1 (not depicted) and provides or enables a smart-ticket 2 including device and app 2a in any of its configurations as taught in either the PRIOR ART THEME PARK GAMIFICATION, GUEST TRACKING AND ACCESS CONTROL SYSTEM or herein, further provides health verification regiment 2d-1 preferably including zone and time restrictions for obtaining any of the requested health measurements, where the zone may be specified as any one of, or any combination of zone 1, zone 2, zone 3 or zone 4. For example, a theme park might specify that any person desiring to visit the park purchase their ticket at least two weeks in advance of their planned visit, and that they follow a health regiment 2d-1 created by the theme park and / or follow or are already following a health regiment 2d-2 create by a specific public health organization 44. Such as regiment 2d-1 or 2d-2 might for example specify a number of health check measurements to be taken on some period basis, such as daily, with other health checks to be taken once prior to the visit within some "x" days of the visit. What is most important to see is that the entity 40 is provided a system as herein taught for allowing it to ensure that any visitor to an entity 40 premises is verified to be of a certain estimated quality of health.

Still referring to Fig. 3, in an alternate embodiment, where a non-ticketing entity 40 such as a restaurant, store, shopping center, school, office building or other establishment that does not typically pre-book reservations, or if reservations are pre-booked (like a restaurant) but the non-ticketing entity 40 does not typically provide or enable a smart-ticket 2 comprising a device and app 2a and either of a paper ticket 2b or electronic ticket 2c for use by the person 1, it is possible that the non-ticketing entity 40 make use of a general smart-ticket device and app 2a issued to the person 1 (such as by downloading an app onto person 1's smartphone acting as the mobile device with entity app 2a), where the general smart-ticket device and app 2a follows a general health verification regiment 2d-2.

For example, a public health organization 44 such as a governing body may issue a health verification regiment 2d-2 for any of various levels of health verification thoroughness, where for example level 3 is the least thorough and level 1 is the most thorough. Thereby, a person 1 in the general public may be following any of a public regiment level 1, 2 or 3 through a time period such as continuously in a year. The person 1 is then and thereby cleared for self-access or controlled access into a non-ticketing entity 40 such as a shopping center or food market with for example a level 1 followed public health regiment 2d-2. In another example person 1 is then and thereby cleared for self-access or controlled access into a non-ticketing entity 40 such as a school, restaurant, sporting event, amusement park, concert with for example a level 2 followed public health regiment 2d-2. And finally, the person 1 is then and thereby cleared for self-access or controlled access into a non-ticketing entity 40 such as an office building, secured military or government building, etc. with for example a level 3 followed public health regiment 2d-2. It is even possible that a person 1 who is unable to achieve any level of health regiment clearance is able for example to schedule access or otherwise obtain access preferably with a health alert notification to for example a doctor's office, clinic or hospital.

It should be understood that any of the health measurements taken for a person 1 with respect to any particular health regiment 2d-1 or 2d-2 issued by any entity 40 or public health organization 44 may be any combination of self-obtained using devices 3-1 or other-obtained using services and devices 3-2.

Still referring to Fig. 3, regardless of the provider or enabler of a smart-ticket device and app 2a, any given smart-ticket 2a may be provided any combination of health verification regiments 2d-1 and 2d-2 for use in combination with or instead of each other, with overlapping or nonoverlapping zone, geographic or time requirements, where differing specifications in use between different regiments are individually satisfied, and where a less stringent regiment may indicate that a more stringent measurement specification from another entity either 40 or 42 otherwise matching necessary less stringent requirements can be used as a replacement measurement specification in full satisfaction of the less stringent measurement requirements.

It is possible that a given person 1 with a single mobile device for use in 2a be operating any of a multiplicity of ticketing / access apps for use in 2a (see Fig. 1), where the multiplicity enables self-access or at least health verified and approved access to a multiplicity of ticketing entities 40 and non-ticketing entities 40. For example, a person 1 may be following a general public health regiment that is satisfactory for the majority of entity 40 access desired by the person 1, such as access to a school, place of work, shopping market, coffee shop, sporting event, etc., where the person 1 then also has a separate other app for use in smart-ticket 2a or at least a separate other regiment 2d-1 from one or more other entities 40 that have additional health regiment requirements different from the general public regiments 2d-2, and where for example an "other" entity 40 is an airline, theme park, secured building, etc.

Still referring to Fig. 3, a self-operated health measurement device 3-1 comprises any one of or any combination of health bio-metric sensors such as a thermometer 3-1s1, pulse rate detector, and / or oxygen level sensor 3-1s2, virus detector (not depicted), etc. Device 3-1 optionally further includes any of other sensors 3-1s3 such as local environment sensors including for example an ambient temperature sensor or humidity sensor. Device 3-1 includes communications means (not depicted) for exchanging information with the smart-ticket device and app 2a, where communication means are preferably wireless such as Bluetooth or wi-fi.

Any device 3-1 optionally further includes ticket holder ID means 3-1b such as a fingerprint reader or a camera for detecting or otherwise determining if a device 3-1 captured fingerprint or facial image sufficiently matches the fingerprint or facial image of the ticket holder / person 1 as registered to the smart-ticket device and app 2a and therefore confirms the identity of the person 1 responsible for following the health regiment 2d-1 or 2d-2, where determination of what is sufficiently matching is accomplished using a computer algorithm that is executed on any one of or any combination of the device 3-1 and the device and app 2a.

Still referring to Fig. 3, a device 3-1 optionally includes a device ID means 3-1a such as a unique ID code transmittable upon request to the device and app 2a, where a transmittable code is for example implemented as a light sync code pattern emitted by device 3-1 preferably at least in part responsive to sync code control signals received from the smart-ticket 2a, where the emitted light sync code pattern is detected by one or more sensors such as cameras on the device 2a and thereafter verified by algorithms executed on the device and app 2a. In the case where the transmittable code is implemented as a light sync code, device 3-1 for example further comprises any of light emitting devices (not depicted) such as an LED, LCD, OLED or other screen technology, where the light emitting device outputs any combination of a spatial and / or temporal visible light or non-visible light sync code pattern preferably at least in part responsive to the received control signals.

As will be well understood by those skilled in the art personal and portable device design and manufacturing, a preferred device 3-1 is of minimal form factor and self-powered such as by a rechargeable battery, where the recharging may be accomplished by any of wired or wireless means. It is also possible that the device 3-1 both receives power and communicates with the device and app 2a via a wired connection such as a USB cable.

Still referring to Fig. 3, there is depicted any of health check services and devices 3-2 being used by or in the control of a healthcare provider 42. Such services and devices 3-2 are meant to be other-operated either by an authorized person of the healthcare provider 42, or by the ticket holder 1 in cooperation with, in the presence of, or being otherwise examined by an authorized person (such as a nurse, technician or doctor). As will be shown with respect to upcoming Fig. 5, it is also anticipated that an other-operated device 3-2 is in the form of a health measurement kiosk or similar automatic system or device that is made available for use to the ticket holder 1.

Services and devices 3-2 optionally further comprise or otherwise cooperate with ticket holder ID means 3-2b similar at least in purpose to the ticket holder ID means 3-1b described above in relation to self-operated device 3-1. For services and devices 3-2, another authorized person at the healthcare provider that is not ticket holder 1 may also perform an ID verification of ticket holder 1, where verification may for example be accomplished by checking a government issued document such as a driver license or passport, and where appropriate verification information is then entered by the authorized person either directly into a UI provided by the smart-ticket app 2a, or otherwise into a healthcare provider 42 devices capable of both accepting the ID verification information from the authorized person regarding the ticket holder 1 and communicating any of the ID verification information electronically to the device and app 2a as required by the specification of the health measurement within the regiment 2d-1 or 2d-2, where electronic communication at least includes Bluetooth, wi-fi, NFC, cellular, or some wired connection such as USB.

Still referring to Fig. 3, services and devices 3-2 optionally further comprise or otherwise cooperate with healthcare provider ID means 3-2a, where ID means are any one of or any combination of apparatus or method for providing confirming identification regarding the healthcare provider 42 and optionally an authorized person (such as a nurse, technician or doctor) that provides verification that the health regiment specified health measurement was properly taken for the ticket holder 1.

And finally, still referring to Fig. 3, smart-ticket device and app 2a includes ticket datum 2-datum that is any datum as taught in the PRIOR ART THEME PARK GAMIFICATION, GUEST TRACKING AND ACCESS CONTROL SYSTEM or otherwise discussed herein including specific health measurements or related datum and or any data gathered with respect to the proper functioning of the smart-ticket 2a. Also depicted are time & location verification functions 2-tlv such as provided by a mobile device executing the smart-ticket app 2a, where for example the mobile device is capable of providing the current location of the smart-ticket 2a using any of global or local positioning means, where the location is either with reference to a global coordinate system (such as earth based longitude and latitude) or local coordinate system (such as in the sufficient proximity of a given and preferably identified other-operated health measurement device 3-2 (see upcoming Fig. 5)), or within a building or convention center for example as might be detected using a form of wi-fi location, all as is well-known to those skilled in the art of device tracking and positioning systems. Time & location functions 2-tlv are also capable of providing any of time information in any format, global (such as clock time) or relative (such as a duration from a set starting time).

Smart-ticket 2a preferably further comprises bio-metric identification means (such as a fingerprint reader or camera and algorithms for performing facial recognition), see the PRIOR ART THEME PARK GAMIFICATION, GUEST TRACKING AND ACCESS CONTROL SYSTEM. And finally, smart-ticket 2a also preferably stores or otherwise maintains data relevant to any of a health verification regiment 2d-1 provided by an entity 40 or a health verification regiment 2d-2 provided by a public health organization 44. Those familiar with device architectures as well as database and software architectures will understand that multiple variations of the teachings provided herein are possible without departing from the scope and true spirit of the invention, for example in relation to the necessary information contained within the smart-ticket 2a for implementing the teachings of both PRIOR ART THEME PARK GAMIFICATION, GUEST TRACKING AND ACCESS CONTROL SYSTEM and additionally the present application, many configurations and data groupings are possible.

Referring next to Fig. 4A, there is shown a person 1 that is a ticket holder having her temperature read by a thermometer 3-1-d1 all in accordance with the teachings provided herein. While it is possible that person 1 self-operates smart-ticket 2a as a careful consideration will show, it is also possible that some other person (as depicted) is operating smart-ticket 2a during the health measurement, which is then understood to be self-operated in either sense or case. What is different with respect to a self-operated health measurement device 3-1 and a other-operated health measurement service or device 3-2 is that the latter service or device 3-2 requires that an approved or otherwise qualified healthcare provider such as a nurse, technician or doctor take the measurement or at least supervise the taking of the measurement. As will be discussed in relation to upcoming Fig. 5, it is also possible that a service or device 3-2 is fully automated and thus a healthcare provider as a person is not required, and the health measurement is provided for example using a "health-check kiosk."

As will be understood by those skilled in the art, some health measurements can be taken using relatively inexpensive apparatus with simple methods of operation while other health measurements must be taken using relatively expensive apparatus and / or complex methods of operation or assessment, where the former case allows for devices 3-1 and the latter case would typically require services and devices 3-2. It is further anticipated that a health regiment 2d-1 or 2d-2 include not only measurements but also other health care directives, for example requiring that a physical be taken which is a generalized health measurement, or requiring that for example the ticket holder receives a dosage of medicine, such as a flu shot or vaccination, or even ingested or applied medications where the shot, vaccination, ingesting or application is optionally conducted or supervised by a healthcare professional / provider 42, thus being a service or device 3-2.

It is well known in the medical profession that many individuals, for example with chronic health issues, would be benefited by maintaining a regiment including at least the taking of shots, ingesting of pills or application of ointments or similar medical treatments and that such individuals may not for various reasons maintain the prescribed regiment. The present inventor anticipates that many of the teachings provided herein are useful without ultimately being directed or related to obtaining ticketed access, or even access, to an entity 40's premises. For example, as will be clear from a careful reading of the present invention, the teachings herein provided also relate to useful apparatus and methods for verifying that a non-ticketing regiment such as a prescribed plan of shots, pills and ointments is followed by a "ticket holder" person 1 that is then simply a "regiment follower," since following the prescribed health regiment is not then directly related to obtaining or being allowed access to an entity 40 premises, but rather is simply a benefit in and of itself.

As presently shown in Fig. 4A, the person 1 might be a young girl and the operator of the smart-ticket 2 could be her friend or guardian. With respect to the depicted exemplary thermometer 3-1-d1, the thermometer preferably includes the well-known ability to sense body temperature for example within the mouth, where the temperature is then typically on display on a readout 3-1-d1-t1 such as a small LED or LCD screen. The device 3-1-d1 preferably also includes a ambient temperature sensor with readout 3-1-d1-t2, where it is possible that a health regiment 3d-1 or 3d-2 requires that the smart-ticket 2a confirm the ambient temperature as well as the temperature of the ticket holder 1, even for multiple instances of time over a given consecutive or non-consecutive period of time. It is anticipated that some person 1 might desire or unwittingly effect their personal temperature by changing the ambient temperature or being in a certain ambient temperature, and thus the present teachings provide apparatus and methods for allowing the health regiment 2d-1, 2d-2 issuer 40 or 44 respectively, to gather additional ambient / non person 1 information for any health measurement assessment.

It is pointed out that in order to reduce the manufacturing cost of a device 3-1-d1 as currently depicted, it is not necessary to include either of readouts 3-1-d1-t1 or 3-1-d1-t2 as this information can be alternatively (or additionally) displayed through a visible UI on the smart-ticket 2a (for example as depicted). It is also pointed out that at least the ambient temperature could be determined by another device acting as other sensor(s) 3-1s3 (see Fig. 3) for the purpose of gathering for example environment data such as the ambient temperature or humidity, where these other sensors 3-1s3 could even be permanent sensors in a building with data made available for example over the "internet of things." Alternatively, such other sensors 3-1s3 can also be incorporated directly into the mobile device (executing the entity app, the combination of which is referred to as 2a). As the careful reader will see, there are many possible variations of at least the implementation of devices 3-1 and 3-2 including apparatus as well as means and methods for communicating with the smart-ticket 2a and even authenticating the device 3-1 and 3-2, and as such the teachings provided herein should be considered as exemplary rather than as limitations of the present invention.

The preferred thermometer further includes means for electronically communicating the sensed body temperature to the smart-ticket 2a, such that the person 1 or their friend or guardian operating the smart-ticket 2a is neither required to enter the temperature through a UI, nor able to change any of the temperature or related information determined for the person 1, thus ensuring that the given health measurement is not in any way altered to be different from the actual acquired, detected or determined measurement information. There are many well-known methods for communicating between devices such as 3-1-d1 (or generally 3-1 and 3-2) and a mobile device for using in 2a such as a smartphone, including Bluetooth, wi-fi or a wired connection, where other means and methods of communications may also be used or become available for use, all of which are acceptable for the present teachings, where it is preferred that a secured method of communication is used.

Still referring to Fig. 4A, device 3-1-d1 is further taught to include a device ID means 3-1a (or 3-2a for a service and device 3-2, see Fig. 3), where in the present depiction ID means 3-1a includes either a visible light or a non-visible light (such as IR) LED 3-1-d1-led which are well known in the art of electronics. What is most desirable is that the device 3-1-d1 be capable of causing a response signal to be emitted where the emission is any form of electromagnetic information (such as visible or non-visible light or even radio frequency (RF)) that can also be detected by the smart-ticket 2a (and therefore by the exemplary device of a smartphone, where detecting for example is accomplished using a visible light camera, a non-visible light camera or a RF antenna). As will be understood by those familiar with object tracking, using either the visible light or infrared light spectrum as opposed to radio-frequency (RF) spectrum allows for methods of receiving using a camera which can at the same time be capturing an image of the person 1, where such additional capturing is useful for determining that a health device such as 3-1-d1 is currently being properly used (in this case determined to be placed within the mouth of person 1). As a careful consideration will show, using such apparatus an methods as presently taught, it is then possible to deterministically differentiate between two different devices 3-1 (such as thermometer 3-1-d1) attempting to communicate a health measurement to smart-ticket 2a substantially at the same time, where a first device such as 3-1-d1 can be confirmed using the teachings herein to be properly located within the person 1's mouth while the second device can then simply be ignored by the smart-ticket.

In this regard, it is well known that device (such as 3-1-d1) capable of "pairing" communications with another device (such as smart-ticket 2a) for example using Bluetooth, such that multiple similar devices (such as 3-1-d1) might be simultaneously paired with the smart-ticket 2a. Each similar health measurement device 3-1 or 3-2 preferably also includes a device type code (e.g. thermometer vs. pulse rate detector) and unique identifier code (ID) that can be communicated with the smart-ticket 2a in a secure manner along with any health measurement or related datum. (It is even possible that the unique code of each device is then used by the smart-ticket 2a to confirm the validity of the device, for example by accessing the internet to check with an external database verifying that the device type and ID (e.g. a serial number) are valid, where other similar techniques are well known in the art, and that devices 3-1 or 3-2 determined to be not valid are ignored by the smart-ticket 2a with respect to providing a valid health measurement of a person 1).

The unique device ID (and preferably also device type code) allows for at least two methods of distinguishing between simultaneously paired health measurement devices 3-1 or 3-2. In one method, the smart-ticket 2a issues a general communication to all paired devices of any type, or of a specific device type as specified by a given health regiment 2d-1 or 2d-2 in association with a given health measurement, in which the communication requests to receive the device's unique ID. After receiving responses from any zero or more devices 3-1 or 3-2, the smart-ticket 2a then issues a series of general communications receivable by all paired devices 3-1 or 3-2, for example one general communication for each detected paired device (or at least each paired device of a certain device type), where then this general communication includes a specific unique device ID such that the only device to then respond (or respond correctly) to the general communication comprising the unique ID is the device 3-1 or 3-2 for which the unique ID is determined to be a match. In the present figure for example, two or more thermometers 3-1-d1 of the same device type and otherwise indistinguishable except for their unique ID may be simultaneously paired with the smart-ticket 2a, and simultaneously receive the same general communication comprising the unique ID of only one of the two or more thermometers 3-1-d1. In this case, it is possible that the internal algorithms of a thermometer 3-1-d1 are such that the response signal is responsive in part to the sync signal issued by the smart-ticket 2a (and therefore known to all paired devices) and in part to the internal device ID (known only to the specific device such one of the paired thermometers 3-1-d1). In this way, even if two simultaneously paired devices respond to the sync signal, only one response will correctly also effectively encode the correct thermometer's 3-1-d1 unique ID, thus allowing the smart-ticket 2a a means for filtering out or ignoring all other responses for thermometers 3-1-d1 not comprising the proper ID.

In a second method for distinguishing between simultaneously paired health measurement devices 3-1, the smart-ticket can effectively "un-pair" all but a single of the health measurement devices 3-1 or 3-2 such as a thermometer 3-1-d1, where this un-pairing then prevents the un-paired device 3-1 and 3-2 from even communicating with the smart-ticket 2a. What is then further desirable is that a person 1 can choose which health device such as thermometer 3-1-d1 is currently being paired with the smart-ticket 2a, of which several methods are possible and will be well understood to those skilled in the art. In at least one method, the thermometer 3-1-d1 can be further adapted to include a button for pressing by the any person, where the pressing of the button at least in part causes the health measurement device such as 3-1-d1 to emit a pairing request signal. When the pairing request is received by the smart-ticket 2a, if another device 3-1 or 3-2 of any type, or at least of the same type (e.g., a mouth thermometer) is already paired to the smart-ticket 2a, then the smart-ticket 2a ignores the pair request. Otherwise, the smart-ticket 2a can then pair with the requesting device and after pairing send a confirmation signal for example that is received by the now paired health measurement device 3-1 or 3-2, which then in the case of depicted device 3-1-d1 might visually confirm the pairing status by for example outputting a code on a display such as 3-1-d1-t1 or 3-1-d1-t2 or flashes led 3-1-d1-led. In addition to this or as an alternative, smart-ticket 2a might then display an indication of successful pairing on its visible UI (for example the mobile device screen used in 2a), where this indication can further include a serial number or some code that a person can compare to a code printed on the now paired device. As will be well understood by those skilled in the art of electronic pair and verification with unique devices, other methods are possible such that the present teachings in this regard should be considered as exemplary, rather than as a limitation of the present invention. What is most important is that apparatus and methods are provided that ensure or reasonably ensure that the health measurement device 3-1 or 3-2 providing a measurement to the smart-ticket 2a is the health measurement device 3-2 or 3-2 currently being use by the ticket holder / person 1.

Still referring to Fig. 4A, the smart-ticket 2a may be required by a regiment 2d-1 or 2d-2 to image the person 1 using the health measurement device 3-1 or 3-2 such as a thermometer 3-1-d1 placed in their mouth, and even being placed in their mouth and then remaining in their mouth for a certain period of time and or until a "measurement completed, successfully" or "measurement failed" message is received by the smart-ticket 2a from the device 3-1-d1. During this imaging step, using algorithms well-known in the art and yet to be developed, it is possible to both a) confirm the identity of person 1 (to be discussed in more detail shortly,) and b) confirm the placement of the tip of the thermometer 3-1-d1 into the person 1's mouth, or in the person 1's mouth, or exiting the person 1's mouth.

Regarding step (a), the confirmation of the identity of person 1, a careful review of the PRIOR ART THEME PARK GAMIFICATION, GUEST TRACKING AND ACCESS CONTROL SYSTEM will show that person 1 has already performed a "registration step" (see especially the teachings related to Fig. 2 in the PRIOR ART), where this step includes obtaining for example biometric data such as a finger print or a facial image to be associated permanently with the ticket, all preferably done within Zone 1 (where variations for registrations in other zones are also taught within the PRIOR ART). In regard to the PRIOR ART, it was taught that this registration could also be done even within ZONE 2 or ZONE 3 but must be done prior to a ticket holder 1 first entering ZONE 4, the entity 40 premises (such as a theme park). The PRIOR ART taught that once registered using biometrics such as a fingerprint or facial image, it was possible for the ticket holder / person 1 to enter the entity 40 premises, leave the premises and then attempt to come back into the premises. It was discussed that a person 1 might desire to give their paper ticket 2b or electronic ticket 2c for use with device and app 2a to some other person 1 when attempting the second entrance into the premises, but that the system and teachings of the PRIOR ART would prevent this as the second (not registered) person 1's biometrics would not match the first (registered) person 1.

In the present invention, it is further taught that when using a health regiment 2d-1 such as provided by the entity 40, or especially when using a general ticket health regiment 2d-2 as provided by a public health organization 44, that this regiment 2d-1 or 2d-2 may specify that the person 1 is required to register their smart-ticket device and app 2a within a certain zone (for example preferably Zone 1 71) by a certain date and time (for example at least two weeks before the smart-ticket device and app 2a will be valid for use allowing the person 1 to officially enter a given premises). If this requirement is not met, then for example: 1) the issuing entity 40 or 44 may cancel or withhold allowed premises access, 2) the issuing entity 40 or 44 may then include in their regiment 2d-1 or 2d-2 respectively a condition that essentially reverts to other health measurement requirements, or 3) the issuing entity 40 or 44 may then provide a new regiment 2d-1 or 2d-2, where other variations are possible for the entity 40 or 44 if a health regiment is not followed by the ticket holder 1 for example by not registering in the right zone or within the right time frame, all as will be obvious based upon a careful consideration of the teachings herein and of awareness of typical entity and premise operations. As such, the present teachings should be considered as exemplary, rather than as limitations.

It should be noted that it is possible to have reasons that a ticket holder 1 might not be able to for example register in the right place Zone 1 71 or even a portion of Zone 1 71 as determined by using for example GPS) or register by the specified registration time, for example 2 weeks before desired access to the premises. It is also possible that the ticket holder 1 does properly register, but then is unable to fully comply with all measurements in a health regiment 2d-1 or 2d-2. It is further possible that the ticket holder 1 "fails" a health measurement for any number of reasons, including that the ticket holder has some other biological or medical condition. In any and all of these cases, it is anticipated that the health regiment 2d-1 or 2d-2 can pre-script into the regiment alternative health measurements that can be sought man made by the ticket holder 1 to thus keep the ticket holder 1 in compliance with the regiment, for example allowing the ticket holder 1 to see a healthcare provider 42 as a means for "overriding" any one of or any combination of other health measurement requirements including in the regiment. It is also anticipated that the ticket holder 1 may contact for example the regiment issuing entity 40 or 44, or some other representative of the issuing entity 40 or 44, where during this communication satisfaction of or changes to the health regiment 2d-1 or 2d-2 are enacted, even including the electronic alteration of either the health regiment 2d-1 or 2d-2 or any of the ticket datum 2-datum, where this alteration is conducted in a secure manner as will be well understood by those skilled in the art of computing systems such that malicious tampering with the regiment 2d-1, 2-d2 or the ticket datum 2-datum is either rendered not possible or is made detectable.

Still referring to Fig. 4A, what is preferred and most important is that at some point before following a regiment 2d-1 or 2d-2, a person 1 registers a confirming biometric with the smart-ticket device and app 2a (that is either of a specific smart-ticket 2 issued by an entity 40 for a specific premises entrance date and time, or a general smart-ticket 2 issued by an entity 44 for a non-specific premises entrance date and time). The careful reader will note that an entity 44 can issue a health regiment 2d-2 indicating that after registering person 1 to the smart-ticket device and app 2a it is necessary for example to conduct a certain number and / or types of health measurements prior to the smart-ticket 2a then being authorized for general access into a premises, where for example the time period could be 2 weeks of health monitoring with a set A of required health measurements before achieving general level 1 premises access rights (see the prior teaching above and herein), 1 month of health monitoring with a set A and B of required health measurements before achieving general level 2 premises access rights, or even 1 day of health monitoring with a set C of measurements before achieving general level 3 premises access rights (where in this latter example, all that might be required for level 3 is that the person 1 visit a health care provider 42 to receive one or more specific health measurements, including for example an infectious disease test).

What is most important to see is that smart-ticket device and app 2a has confirming biometrics, in the present case depicted in Fig. 4A as a facial image of person 1. Using these biometrics and for example the facial image provided during a prior ticket registration step, as the person 1 is being imaged using a health measurement device such as thermometer 3-1-d1, their registered biometric (in this case facial image) is then compared to a currently captured and same biometric (again in this case a facial image). As will be well understood by those familiar with face recognition and object tracking, it is possible using today's algorithms and technology to both identify a current image captured of a person (even extracted from an ongoing video of images) with a prior captured image of that person (for example at least the image of person 1 provided during registration). It is also possible that the current image of person 1 captured during a health measurement such as using thermometer 3-1-d1 be required according to the regiment to be persisted as ticket datum 2-datum, and / or transmitted along with the current health measurements to some verifying or tracking organization, such as but not limited to the entity 40 or a public health organization 44.

Still referring to Fig. 4A, just as methods can be used to confirm the proper identity of the person 1 as associated with the smart-ticket 2a, it is possible to devise and use other methods that confirm the proper identity of the health measurement device 3-1 or 3-2, and in this case device 3-1-d1. One possible device ID means 3-1a (see Fig. 3) is that smart-ticket 2a communicates a unique sync signal to device 3-1-d1, where device 3-1-d1 then at least in parts uses this unique sync signal to provide any of a spatial or temporal pattern of emitted electromagnetic energy, in this example a specific sequence of LED 3-1-d1-led flashes. Those familiar with such electromagnetic emitting devices, for example emitting any of visible light, non-visible light or radio frequencies (RF) that are capable of being received by the device such as a smartphone being used in 2a of smart-ticket 2 will understand that many other variations are possible, and therefore the present example should be considered as exemplary, rather than as a limitation of the present invention.

What is most important is that: (a) the smart-ticket device and app 2a has apparatus and methods for communicating with the health measurement device 3-1 or 3-2 (and in this depiction 3-1-d1) substantially at the same time that the measurement device 3-1 or 3-2 is taking a health measurement of the person 1, (b) the smart-ticket 2a is able to confirm that person 1 is the ticket holder 1 as registered to the (specific or general) smart-ticket 2a, preferably by use of biometric means including a fingerprint reader or facial recognition, (c) the smart-ticket 2a is able to confirm that person 1 is actually using the health measurement device 3-1 or 3-2 (in this depiction by using image analysis to track the location of the tip of thermometer 3-1-d1 in relation to the recognized face of person 1 or in upcoming Fig. 4B by using an apparatus that requires the finger of the person 1 to be present and preferably the point of contact for taking the health measurement such that a fingerprint is captured by the same surface of the device 3-1 or 3-2 making the heath measurement), and (d) receiving in a secure means that are preferably an encoded electronic means, the actual health measurement and any regiment 2d-1, 2d-2 specified or otherwise related datum such as time and geo-location.

Still referring to Fig. 4A, as prior discussed, the health regiment 2d-1 or 2d-2 could also specify (e) that the health measurement be taken while the smart-ticket 2a is essentially in real-time communications with a remote system (such as operated by the entity 40, public health organization 44, or some third party on the behalf of 40 or 44), where the remote system has access to any of the information available to any of the smart-ticket or health measurement device 3-1 or 3-2, regardless of whether or not the person 1 is aware of any of this same communications or any of the specific information made available to the remote system. As will be understood by those familiar with data privacy rights, this last function and feature of the present invention is not mandatory for the usefulness of the invention, meaning that in a preferred operation of the invention none of the person 1's private data including any biometric or even any of the specific health measurements or their related datum are made available to the entity 40, a public health organization 44 or any other operator of a remote system, thus ensuring the privacy of person 1. In this preferred use, what is accomplished is that the regiment issuing entity 40 or 44 is assured by the proper function of the system that person 1 has sufficiently followed the prescribed health regiment 2d-1 or 2d-2 respectively, and as such is "cleared" for access, where clearing for access is then an additional datum of information necessary to achieve the status of "confirmed" for entry into a premises (see especially step / ticket status 7 in relation to the PRIOR ART Fig. 2). As will be well understood for those familiar especially with high priority facilities, there are also advantages to step (e) where in this case the personal privacy of the ticket holder 1 is not of concern to the ticket holder 1 or is at least understood by ticket holder 1 to be an accepted condition for premises entry.

Referring next to Fig. 4B, there is shown a second exemplary self-operated health measurement device 3-1-d2, where in this case the measurements are pulse rate and blood oxygen levels, for which the technology is well known in the art. In present figure, device 3-1-d2 is equipped with a ticket holder ID means 3-1b (see Fig. 3) that is a fingerprint reader 3-1-d2-r as opposed to a facial recognition camera and algorithm as described in Fig. 4A for device 3-1-d1. What is important to see is that the same finger of person 1 being sensed by device 3-1-d2 for determining the prescribed health measurements (such as pulse rate and blood oxygen level) can substantially simultaneously be sensed for determining a finger print to be used to verify that the person 1 is the registered ticket holder 1, for example by comparing the fingerprint obtained during the measurement by device 3-1-d2 with the fingerprint stored on the smart-ticket 2a during the prior registration step, all as previously taught with respect to the PRIOR ART THEME PARK GAMIFICATION, GUEST TRACKING AND ACCESS CONTROL SYSTEM and as then further discussed especially in relation to Fig. 4A. As those skilled in the art of computing systems will understand, a health measurement device 3-1 or 3-2 such as 3-1-d2 can be used to capture biometric data, where this biometric data is processed to confirm that it matches the ticket holder 1's registered fingerprint by any one of or any combination of computer processes executed on the device such as 3-1-d2 or the smart-ticket 2a. In the preferred embodiment, device 3-1-d2 captures the fingerprint of person 1 and provides this captured information in a sufficient dataset to smart-ticket 2a via a secured communication link. Smart-ticket 2a then confirms that the fingerprint read by device 3-1-d2 sufficiently matches the fingerprint registered in association with the smart-ticket 1.

As discussed in relation to Fig. 4A, this fingerprint data captured by a so equipped health device 3-1 or 3-2, such as device 3-1-d2, can remain private to the ticket holder 1 and therefore be deleted after confirmation or stored as ticket datum 2-datum not to be communicated to an entity 40 or 44 or an entity representative, or the fingerprint datum can be shared with or without notifying the person 1, all as prior discussed in relation to Fig. 4A.

Like device 3-1-d1 depicted in relation to Fig. 4A, device 3-1-d2 preferably also includes a device ID means 3-1a, whereby device 3-1-d2 identifies itself uniquely to the smart-ticket 2a. As the careful observer will note, in the case of a health measurement device 3-1-d2 that captures its health measurement essentially from the same body surface that its captures its biometric data, there is less need to separately confirm the device 3-1-d2's unique ID as the health measurement can be securely transmitted along with the determined fingerprint such that the set of data is more difficult to forge by a person so intending.

And finally, also with respect to Fig. 4B, it is possible that the smart-ticket 2a is operated by the person / ticket holder 1, or another person such as a friend or guardian of person / ticket holder 1. What is most important is that the health measurement is provided in some combination with sufficient biometric data (both taken from the person / ticket holder 1) to the smart-ticket 2a to be recorded in compliance with a health regiment 2d-1 or 2d-2. Optionally, the device ID of the measurement device such as 3-1-d2 is also recorded. Like the discussion with respect to device 3-1-d1, there are advantages to the system for only allowing a single health measurement device such as 3-1-d2 (or 3-1-d1) to be paired with the smart-ticket 2a at any given time when measurements are being taken, so as to limit the possibility of forged health measurements (all as prior discussed). A useful alternative is to only allow a single health measurement device such as 3-1-d2 (or 3-1-d1) of a certain type of device such as pulse rate and blood oxygen detector verses a thermometer to be paired with the smart-ticket 2a at any given time. Using this approach, therefore of simultaneously pairing one and only one unique device per type device type, a person could pair multiple devices of different types and leave these essentially paired or in an auto-pair mode so as to minimize their efforts or any delays when taking health measurements, where the delays might at least in part by caused by pairing concerns or operations.

With respect to both Fig. 4A and 4B, it is also possible that a health measurement device 3-1 or 3-2 be further adapted to include a unique ID that is an NFC tag which, for example, could then be detected by a properly equipped mobile device serving as part of the smart-ticket 2a. With this approach, a ticket holder holds the health measurement device close to the smart-ticket 2a's NFC reader to pick up the device ID and preferably also the device type (such as pulse rate and blood oxygen detector 3-1-d2 verses a thermometer 3-1-d1). This NFC reading action could then be used at least in part to automatically begin or otherwise allow a health measurement process, for example by bringing up a health measurement UI on the smart-ticket 2a, identifying the type of measurement to be taken and confirming that the measurement device 3-1 or 3-2 is properly paired and ready. The smart-ticket 2a could then also disallow another health measurement to be started at the same concurrent time, or at least not another health measurement using the same device type, all as will be evident from a careful reading of the present invention.

Referring next to Fig. 5, there is depicted a health-check kiosk 3-2 that for example is provided by or on behalf of an entity 40 or public health organization 44. In this depicted example, the health-check kiosk 3-2 uses what is known in the art as a thermal camera to detect and determine a person 1's body temperature without requiring contact with the person 1, and without requiring that the person 1 to contact the kiosk 3-2. In a preferred embodiment, when a person 1 holding their smart-ticket device and app 2a approaches the kiosk 3-2, there is an automatic pair by some wireless communication means such as Bluetooth or wi-fi such that the smart-ticket 2a is in communications with the kiosk 3-2. Once in communications, the person 1 uses the smart-ticket 2a to confirm that their identity matches the identity of the registered smart-ticket 2a holder and then the smart-ticket 2a can, if necessary, provide any one of or any combination of information that an identity match is confirmed, or further the ID of the ticket holder 1 (such as a name, customer number, employee number etc.), the ID of the ticket (such as a ticket number) or the actual confirming biometric. As prior discussed, the desired level of system security and the desired level of person 1 data privacy dictate several possible exchanges of information, all considered to be within the scope of the present invention.

For example, to maintain data privacy for the ticket holder 1, after the smart-ticket 2a is paired to the kiosk 3-2, and after the person 1 confirms their identity by for example: 1) providing their fingerprint to the smart-ticket 2a, or 2) allowing smart-ticket 2a to image their face for facial recognition, either of which is then compared to prior ticket registration biometric information, smart-ticket 2a simply requests a health measurement by sending a message to kiosk 3-2 that a health measurement is ready to be taken. In the depicted case, a screen or mirror is made available such that person 1 can align their face within a region demarked within either a real-time image being displayed by a screen, or within a region physically demarcated on a mirror. In either case, the person 1 is only assuring that their face for example is properly positioned to be best imaged by the thermal camera 3-2d for remotely determining their body temperature. As those familiar with image capture and processing systems will understand, many variations of aligning a person 1 with the kiosk 3-2 are possible, and as such the presently depicted and described methods should be considered as exemplary, rather than as a limitation of the present invention. For example, if the person 1 is simply standing at a pre-marked location on the floor and looking at a mark indicating where they should look to best align with the thermal camera 3-2d, then this can be sufficient and less costly of a system to manufacture.

Still referring to Fig. 5, once kiosk 3-2d has taken a measurement, this information is then preferably transmitted to smart-ticket 2a as secured data that cannot easily or substantially be tampered with. This health information is depicted in the present figure as being displayed on the kiosk, however since this is private information it is preferred that at most some confirmation indication is made by the kiosk, which could be a sound or a flash of light from an LED (not depicted) indicating that the measurement is completed, or simply a change made by the smart-ticket 2a on the UI telling the person 1 that their measurement is completed. As those familiar with entity 40 administration will understand, it is also possible that if the health measurement detects and out of range value, such as a high body temperature, any number of possible actions may be taken at least including: 1) providing a message on the kiosk asking the person 1 to see an entity representative, 2) sending a message to the smart-ticket asking the person 1 to see an entity representative, 3) either temporarily or permanently revoking clearance for the person 1 with respect to the smart-ticket 2a such that the person 1 cannot enter any further onto the premises through any self-access or entity operated access point, and 4) sending information to the entity 40 with any of data relating to the person 1 or their specific ticket number alerting the entity 40 that a health check measurement is out of range.

As the careful reader will see, the provided examples of self-operated devices 3-1-d1 and 3-1-d2 might normally be used in a Zone 1 71 prior to arriving at the premises, perhaps a significant amount of time in days, weeks or months before the ticket would otherwise become valid for entrance onto a premise. The health-check kiosk 3-2 is preferably situated on premises in a Zone 2 72 as described in PRIOR ART THEME PARK GAMIFICATION, GUEST TRACKING AND ACCESS CONTROL SYSTEM, a zone which is just outside a final confirmation Zone 3 73, where the entity is attempting to make sure the ticket holder 1 will be successful upon crossing from Zone 2 72 into Zone 3 73 before then providing within Zone 3 73 additional personal verification information such as their fingerprint or facial image such that their smart-ticket 2 is then confirmed to allow access from Zone 3 73 into the entity premises that are Zone 4 74.

In the exemplary case where the entity premises is not a ticketed premises such as a grocery store or similar public building, the person following a general ticket 2a health regiment 2d-2 as issued by a public health organization 44 might then be required to stop at a health check kiosk 3-2d to receive a health measurement confirming that entrance onto the non-ticketed premises will be allowed.

With respect to the present system 102, and as taught in relation to PRIOR ART THEME PARK GAMIFICATION, GUEST TRACKING AND ACCESS CONTROL SYSTEM, it is possible that a single mobile device 2a be used for multiple smart-tickets 2, for example when a family is visiting a theme park a single mobile device 2a (such as a smartphone) is shared by all smart-tickets 2. The reader is directed to the PRIOR ART THEME PARK GAMIFICATION, GUEST TRACKING AND ACCESS CONTROL SYSTEM for teachings in this regard which are equally applicable with respect to additional teachings of the present invention.

Referring next to Fig. 6A, there is shown ticket-holder / person 1 wearing self-operated device 3-1-d4 (see also Fig. 6B) or 3-1-d5 (see also Fig. 6C). As discussed in relation to Fig. 4A, person 1 is depicted as not also holding and operating smart-ticket device and app 2a, which for example is being held and operated by another person such as a friend or guardian. As a careful consideration will show, person 1 could also be holding and operating smart-ticket 2a, such as by standing in front of a mirror, such that the following description of functionality is equally applicable to either case and as such especially the depictions relating to Fig. 4A, 4B and 6A should be understood as exemplary with respect to the holding and operating of smart-ticket 2a, rather than as a limitation of the present invention.

Still referring to Fig. 6A, self-operated device 3-1-d4 or 3-1-d5 are generally wearables to be attached to the person 1's body in a secure manner, where after an attachment step there is a verification step such that the "wearable is confirmed" to in fact be attached to person 1 while also person 1's identity is confirmed. Although wearable 1 is depicted to be a type that is worn around person 1's wrist, many other types are possible for example including: being worn around the neck, the ankle, the waist, the torso, etc., and therefore the type of wearable should also be considered as exemplary, rather than as a limitation of the present invention. Those familiar with a specific product known as the "Fit Bit" will recognize that the exemplary wearable 3-1-d4 and 3-1-d5 may share similar apparatus and means similar to the Fit Bit for monitoring bio-health metric of person 1. (Conversely, devices such as the Fit Bit could be further adapted as herein described to function as a self-operated wearable 3-1 such as wearable 3-1-d4 and 3-1-d5.) These bio-health metrics for example can include a movement pace and distance (such as walking or running), calories burned, pulse rate, blood oxygen levels and sleep quality. What is most important to see is that the wearable comprises sensors such as 3-1s2 for measuring pulse/oxygen levels or other bio-metrics such as temperature as well as other sensors such as an accelerometer 3-1s3 for measuring movement or an ambient temperature sensor. Such Fit Bit devices often include a GPS tracking device for also measuring movement, where the present invention may also be so adapted.

The present inventor anticipates that other sensors can be added to any wearable such as 3-1-d4 and 3-1-d5 regardless of the type of wearable, i.e. wrist, neck, ankle, waist, torso, etc., such as well-known body temperature sensors 3-1s1 or other environmental sensors 3-1s3 such as acoustical sensors and ambient temperature and humidity sensors. What is most important to see is that many types of devices 3-1-d4 and 3-1-d5 are possible comprising many variations of sensors for sensing personal bio-metrics and / or the environment state, and therefore all possible variations of types of sensors are considered to fall within the spirit and scope of the present invention. It is also noted that those familiar with products referred to as "smart watches" will also understand that smart watches often also include such sensors and provide valuable health monitoring functions, and as such in this regard may serve as self-operated devices 3-1-d4 or 3-1-d5 given that they are further adapted described herein and especially with respect to Fig.'s 6A, 6B and 6C.

Still referring to Fig. 6A, wearables 3-1-d4 and 3-1-d5 are implementations of a self-operated health measurement device 3-1 with functions prior described especially in relation to Fig.'s 3, 4A and 4B. While the particular health measurement bio-metric sensors or other environment sensors are not depicted per se or discussed in relation to Fig.'s 6A, 6B and 6C, for the purposes of discussion, it is assumed that typical measurements of pulse, oxygen level and sleep patterns as provided with a modern Fit Bit or smart watch are being taken of person 1 by a wearable 3-1-d4 or 3-1-d5 over some duration of time. What is different about the present invention is that a typical market device such as a Fit Bit or a smart watch can be put on and taken off a person 1 at any time and therefore also exchanged between more than a single person 1 such that the bio-metrics and other data collected by the existing marketplace wearables cannot be authenticated as related to a specific person 1 that is a ticket holder.

Now referring to Fig. 6A, 6B and 6C, as depicted in the present Fig. 6A, a self-operated wearable health measurement device 3-1-d4 or 3-1-d5 is first secured onto the person 1's body, such as around their wrist. As shown in Fig. 6B, securing includes closing a detectable wearable locked path 3-1-w-lck. Those familiar with wearables and device electronics will recognize that many various solutions are possible for determining if a wearable clasp, such as the wristband of a Fit Bit or smart watch, has been closed and clasped around the person 1's wrist (or other body part in the case of a different type of wearable). In the example depiction of Fig. 6B, the wristband is a "one-piece" design fully attached (and therefore not readily detachable such as shown in relation to Fig. 6C), whereas in Fig. 6C, the wristband is detachable from the device 3-1-d5. It is herein also taught that while typically the biometric sensors and any other supporting electronics are enclosed in the case such as 3-1-d5 as opposed to the wrist band joined to the case, this restriction is not necessary for the present teachings. It is possible and anticipated for instance, that sensors might also be placed in the wristband (or clasping band in the case of other types of wearables), without departing from the spirit and scope of the present invention.

Regarding Fig.'s 6B and 6C, what is most important to see is that band-locked sensors and electronics are adapted into the wearable 3-1-d4 that includes an integrated body clasping band (in this case wrist band) and into the wearable 3-1-d5 that includes a detachable body clasping band (in this case also a wrist band), where in the case of wearable 3-1-d5 band-locked sensors are further adapted to determine that the clasping band (e.g. wristband) has remained attached to the case 3-1-d5. In a preferred embodiment, each detachable juncture such as band clasp 3-1-d4-clp of wearable 3-1-d4 or band-case clasp 3-1-d5-clp include some form of sensors or electronics for detecting at least the states of "closed" and "open" (or any similarly interpreted state concept) where "closed" means attached and secured / locked while "open" means detached and non-secured / unlocked. In the case of wearable 3-1-d4 as depicted, there is only 1 such juncture 3-1-d4-clp, whereas in the case of wearable 3-1-d5 as partially depicted there are 3 such junctures, such as 3-1-d5-clp located on either the left side (not depicted) or the right side (depicted) of case 3-1-d5 and a clasp such as 3-1-d4-clp shown in relation to Fig. 6B (not depicted in Fig. 6C), all as a careful consideration of the present drawings as well as the start-of-the-art in wrist wearables such as the Fit Bit will show.

Still referring to Fig.'s 6A, 6B and 6C, what is most important is that a wearable such as 3-1-d4 and 3-1-d5 (or any type that can be clasped to a body part) comprise electronic sensor means for determining that wearable locked path 3-1-w-lck is "closed" versus "open," regardless of the total number of junctures in the path 3-1-w-lck, where it is then also understood that any two or more junctures in the path 3-1-w-lck are treated as being in series rather than in parallel, such that all of any two or more junctures must be determined as "closed" for the entire path 3-1-w-lck to be considered as "closed," as will be well understood by those familiar with electrical circuits.

Referring again exclusively to Fig. 6A, it is assumed that person 1 has securely clasped the wearable such as 3-1-d4 or 3-1-d5 to their body, in this depiction person 1's wrist. Wearable 3-1-d4, 3-1-d5 includes wireless communication means for exchanging information with smart-ticket device and app 2a, like was described in relation to device 3-1-d1 (Fig. 4A) and device 3-1-d2 (Fig. 4B). Like self-operated health measurement device 3-1-d1 (Fig. 4A), wearable 3-1-d4, 3-1-d5 comprises a device ID means 3-1a including any of visible or non-visible light output means (such as any of well-known display elements or LEDs) for emitting verification signal 3-sig. As described in relation to device 3-1-d1, smart-ticket 2a preferably first pairs with wearable 3-1-d4, 3-1-d5, where it is also desirable that smart-ticket 2a pair with only a single wearable 3-1-d4, 3-1-d5 of a given type, all as described in relation to Fig. 4A, or at least a single wearable 3-1-d4, 3-1-d5 with respect to a single person / ticket holder 1. After successful pairing, smart-ticket 2a communicates with wearable 3-1-d4, 3-1-d5 and confirms that the wearable locked path 3-1-w-lck is "closed." Upon achieving this state of paired with closed wearable, smart-ticket 2a preferably captures an image (including a picture, series of pictures or video) including both the person 1 and at least a sufficient portion of wearable 3-1-d4, 3-1-d5 such that verification signal 3-sig can also be sufficiently detected in the same image(s).

As described in relation to Fig. 4A, smart-ticket 2a preferably emits sync code control signals uniquely encoded for wearable 3-1-d4, 3-1-d5, whereupon receiving the sync code control signals wearable 3-1-d4, 3-1-d5 causes any combination of a spatial or temporal pattern of verification signal 3-sig based at least in part upon the sync code control signal. As will be clear from a careful reading of the present teachings, many alternatives are possible including that the sync code control signal is simply used to initiate some pattern of verification signal 3-sig that is then decodable for indicating or confirming the ID of wearable 3-1-d4, 3-1-d5. What is most important to see is that smart-ticket device and app 2a is confirming that any bio-metric or environmental sensed data to be received from a wearable such as 3-1-d4, 3-1-d5 is in fact being received from the same wearable 3-1-d4, 3-1-d5 currently clasped and "closed" around person 1's body part, as opposed to for instance another wearable such as 3-1-d4, 3-1-d5 attempting to also provide sensed data. Also, a previously discussed, there are alternative ways of addressing this requirement including only pairing with a single wearable such as 3-1-d4, 3-1-d5 at a time, and then also confirming the wearable such as 3-1-d4, 3-1-d5 using for example a visible synchronization signal 3-sig as herein described.

Still referring to Fig. 6A, after smart-ticket 2a has confirmed the wearable 3-1-d4, 3-1-d5 by decoding sync signal 3-sig as received in an image also including at least the face of person 1, smart-ticket 2a then also uses any of well-known facial recognition to verify that the face captured in the image matches an earlier facial image(s) for example captured by the smart-ticket device and app 2a of person 1 during a registration step (see especially Fig. 2 of PRIOR ART THEME PARK GAMIFICATION, GUEST TRACKING AND ACCESS CONTROL SYSTEM). As the careful reader will note, the order of any individual steps as herein described may be changed without departing from the present teachings. For example, it is possible that smart-ticket 2a first confirms the identity of person 1 that is the ticket holder and then second synchronizes and confirms the identity of the wearable 3-1-d4, 3-1-d5. What is most important to see is that smart-ticket device and app 2a is able to confirm that person 1 is wearing some type of self-operated health measurement device such as a wearable 3-1-d4, 3-1-d5 for collecting health measurements and other environment data for presumably an extended period of time (e.g. as opposed to thermometer device 3-1-d1 presumed to take a single measurement(s) over a short time after which the device 3-1-d1 is disengaged from contact with the person 1).

This period of time for example could be hours, days, weeks, months, etc. During the course of time, person 1 may be desirous or have need of removing wearable 3-1-d4, 3-1-d5, in which case at least 1 juncture such as 3-1-d4-clp or 3-1-d5-clp must be "opened" and therefore the entire wearable locked path 3-1-w-lck is detected as "open," in which case all sensor measurements taken after the "path opened" detection are not associated with person 1 for the purposes of satisfying any health regiment 2d-1 or 2d-2 as taught herein. As the careful reader will see, after unclasping the wearable 3-1-d4, 3-1-d5, a person 1 that is the ticket holder might then re-clasp the same wearable 3-1-d4, 3-1-d5 at a future time, where upon the wearable confirmation steps prior discussed in relation to Fig. 6A must then be repeated in order for smart-ticket device and app 2a to confirm for wearable 3-1-d4, 3-1-d5 that measurements may again be taken and accumulated with respect to person 1.

And finally, with respect to Fig. 6A, as will be well understood by those skilled in the marketplace of wearables for health measurement detection, a wearable 3-1-d4, 3-1-d5 can operate in a "disconnected" mode, where disconnected means not in communications with a mobile device executing an entity app together included with the smart-ticket 2a. In this disconnected mode what is desirable is that wearable 3-1-d4, 3-1-d5 continue to collect health and environment measurements in relation to person / ticket holder 1 for a subsequent transmission to smart-ticket 2a for use in checking compliance with respect to a regiment 2d-1 or 2d-2. In this manner and as will be understood by those familiar with computer systems, the wearable 3-1-d4, 3-1-d5 can continue collecting health measurements in an "off-line" mode with respect to the smart-ticket 2a. In such an operation, what is preferred is that smart-ticket 2a provides wearable 3-1-d4, 3-1-d5 with datum for association with any health or environment measurements determined while wearable 3-1-d4, 3-1-d5 is "off-line," such that when wearable 3-1-d4, 3-1-d5 is again paired and in communications with smart-ticket 2a (and therefore back "on-line"), any measurements taken while off-line are provided to the smart-ticket 2a along with the datum for association earlier provided to the wearable 3-1-d4, 3-1-d5 by the smart-ticket 2a. As those skilled in the art of communication systems and data exchange will understand, there are many possible variations for ultimately ensuring that health measurements determined by wearable 3-1-d4, 3-1-d5 while off-line are properly associated with person 1.

For example, it is possible and preferable for smart-ticket 2a to record the time of pairing with, and confirming of, wearable 3-1-d4, 3-1-d5 and person 1 as ready for accepting any measurements. Thereafter, wearable 3-1-d4, 3-1-d5 may record any measurements along with any format of a date-time stamp (as is well-known in the art of computing), with the assumption that the measurements recorded at specific logged dates and times are applicable to person 1. If and when the wearable 3-1-d4, 3-1-d5's locked path 3-1-w-lck is detected as "opened," then the wearable 3-1-d4, 3-1-d5 can do any one of or any combination of: 1) insert data into its dataset for transmission to smart-ticket 2a indicating the time the path 3-1-w-lck was opened, 2) stop recording health measurement data, 3) indicate for each health measurement the state of the path 3-1-w-lck as "locked" or open. As those familiar with datasets and computer algorithms will understand, it is possible to use any of these means and methods discussed to provide sufficient data to smart-ticket 2a for determining which off-line health measurements taken by wearable 3-1-d4, 3-1-d5 are valid for person 1, where valid includes ensuring that the path 3-1-w-lck was continuously "locked" from the point of wearable confirmation up and through the point in time where the measurement was taken. Therefore, the specific descriptions in this regard should be considered as exemplary, rather than as limits of the present invention.

Referring next to Fig.'s 7A and 7B there is shown a combined health measurement device smart-ticket 2-3 incorporating functions described for the smart-ticket device and app 2a as the self-operated health measurement device 3-1 (see Fig. 3). Fig. 7B shows a block diagram of key components of smart-ticket 2-3 including health sensor means 3-1hs, other sensor means 3-1os, device clasp status-check means 3-1-w-lck, device ID means 3-1a, ticket datum 2-datum, time & location verification means 2-tlv, health regiment 2d-1, 2d-2 and bio-metric personal identification means 2-pid. It is noted that health sensor means 3-1hs include any sensors for determining a personal bio-metric or otherwise any information usable for determining a health measurement related to a person 1 and ideally a health regiment 2d-1, 2d-2 including a temperature sensor, pulse rate sensor, blood oxygen level sensor, sleep tracker, wearable electrodes, biochemical sensor, etc. There are many manufacturers of health sensors for use in mobile wearables, for example including Analog Devices "wearable technologies," and Infineon Technologies "wearables." It is further noted that other sensors 3-1os include any sensor, for example an environment sensor for detecting ambient temperature or humidity, an altimeter, proximity sensor, or a motion sensor such as an accelerometer or gyroscope (gyro sensor).

As prior discussed in relation to Fig. 6B and 6C, a device clasp status-check means 3-1-w-lck preferably includes electronics for operating at least one contact sensor for each open/close juncture, where any and all junctures must all be closed for determining a status-check of closed versus open. As those skilled in the art of electronics will understand, it is also possible that the band for holding a device such 3-1-d4 on a person 1's body part such as a wrist could be flexible band that is continuous and has no open/close junctures, and in that sense is always "closed." For example, such a flexible band for use on the wrist would then be stretched to fit over the hand and onto the wrist. Any of what are generally referred to as "stretch sensors" may be used to detect when the band is for example stretched more than X%, where X% is determined as approximately 50% of the total stretch necessary to fit over the wrist.

In an alternative design for a clasp with one or more junctures, a wearable device 2-3 fitted around the wrist would further include an attached flexible and unbroken band including a stretch sensor. In this design a person first pulls wearable device 2-3 over their fist that includes stretching the flexible band. Once device 2-3 is positioned at the wrist, the attached flexible band contracts to a minimum circumference based upon the person 1's wrist size. Next the person closes the one or more open junctures setting the device clasped status to "closed." The advantage of this alternative design is that the flexible band which could be very thin and attached to the band over for example at least 180 degrees of the circumference, would ensure that a "stretched" measurement is essentially available at the minimum possible circumference, even if then the band itself is not clasped tightly around the wrist, leaving room with respect to the clasp-able band for comfort and some movement. A careful consideration will then show that the wearable device 2-3 can combine measurements of "juncture contact broken" OR "flexible band stretched past X%" where either condition will cause the wearable device 2-3 clasp 3-1-w-lck to report as "open" versus "closed." Other variations will be obvious upon a careful consideration of the purposes stated herein, which purposes are to determine concerning the wearable 2-3: 1) that wearable 2-3 has been placed upon person 1's wrist (appropriate body part) and is ready to collect health measurements and otherwise function according to the teachings herein, 2) that wearable 2-3 is currently closed and remaining upon the person 1's wrist, and 3) that wearable 2-3 has been taken off the person 1's wrist (appropriate body part).

Referring next mainly to Fig. 7A for the purposes of describing the operation and uses of combined health wearable smart-ticket 2-3, it is first noted that the wearable 2-3 includes wireless communications means for communicating with other devices such as a smart-ticket 2, a self-operated health measurement device 3-1, an other-operated health measurement device 3-2, a health kiosk 3-2 or an other-operated ID confirmation service kiosk 3-3 (as depicted in the present Fig. 7A). Many communication technologies are known and will become known where preferable technologies for exchanging electronic information wirelessly include Bluetooth and wi-fi. It is also possible to use cellular communications in a wearable 2-3, although due to cost considerations the present description is demonstrating use without requiring a cellular link, which then also excludes the use of GPS (and thusly local positioning systems (LPS) are alternatively employed by time & location verification means 2-tlv as are well-known in the art, see also PRIOR ART THEME PARK GAMIFICATION, GUEST TRACKING AND ACCESS CONTROL SYSTEM).

Using a Bluetooth or wi-fi connection also allows wearable 2-3 to communicate to either a mobile device with app acting as smart-ticket 2a, or another computer such as a laptop that is alternatively running the entity app for performing all the functions described in relation to the smart-ticket 2 (see PRIOR ART THEME PARK GAMIFICATION, GUEST TRACKING AND ACCESS CONTROL SYSTEM). Hence, a person 1 may receive from the entity their electronic ticket 2c and health verification regiment 2d-1 using a (for example non-mobile, or practice not easily mobile) computer and internet connection. Following the teachings and descriptions in the incorporated PRIOR ART, a person 1 can then use their computer for example to register the smart-ticket (where the device and app 2a is then effectively the computer), where during registration the computer is used to capture a facial image, fingerprint or other identifying biometric (such as a retinal scan).

After registering the smart-ticket 2 using an internet connected computing device and app 2a, it is then possible for the smart-ticket device and app 2a to pair with the wearable smart-ticket 2-3 in order to: 1) receive and associate the unique ID of the wearable 2-3 as provided by the device ID means 3-1a with the smart-ticket 2 in combination with the person 1's biometrics and other personal information such as a name, as well as ticket information all as described in the referenced PRIOR ART, and 2) transfer the necessary smart-ticket information 2-datum to the wearable 2-3, where this information can be a time-recorded copy for the purposes of data synchronization (as the information 2-datum on the wearable 2-3 can then be changed over time becoming for example up-to-date with health measurements that are then transferred back to the smart-ticket device and app 2a as necessary), all as will be well understood by those familiar with computer systems and data sharing. It is important to see that the wearable 2-3 now has sufficient smart-ticket 2 information including the registered ticket holder person 1's biometric data including at least 1 biometric required by an entity 40 for which the wearable 2-3 is to be used to gain entrance onto the entity premises. It is anticipated that a fingerprint and / or a facial image will be sufficient, but for the present teachings, both biometrics are assumed to be transferred onto the wearable 2-3. It is also important to see that the wearable 2-3 now can function equivalently to the combination of a smart-ticket device and app 2a and at least some self-operated health measurement devices 3-1, all without requiring that the wearable have a cellular connection, as is the case when using a smartphone as a device 2a in the smart-ticket 2.

Referring still to Fig. 7A, person 1 has used a smart-ticket device and app 2a to first communicate with the entity and possibly a public health organization 44 for also receiving a health regiment 2d-2, and to second register the smart-ticket device and app 2a including: 1) providing personal bio-metrics such as a fingerprint and / or facial image, and 2) registering the wearable smart-ticket 2-3 to the smart-ticket device and app 2a. The person 1 has then transmitted smart-ticket 2a information 2-datum to the wearable 2-3 such that the wearable 2-3, and not the smart-ticket 2a may be worn about and used to gain access to ticketing and non-ticketing entity premises as herein disclosed. As will be understood by those familiar with device manufacturing, it is likely that the production cost of a wearable such as 2-3 would be significantly less than a typical smartphone or other (at least cellular enabled) smart device for running an entity app to become a smart-ticket 2a.

As those familiar with social trends will also understand, it is currently a social norm that children under 13 typically do not have a personal smartphone or mobile device that could then naturally serve as mobile device in 2a for running the entity app. However, most households where at least one person owns a cellphone or smartphone do also have an internet connection and a computing device, where the computing device could then serve as the device of 2a for downloading and running entity 40 apps. A careful consideration of the teachings herein will show that an entity could be a school such as kindergarten through high school, where at least a significant portion of the students will not have a smartphone (which is likely to have a camera and possibly a fingerprint reader, where as a cell phone typically does not). In this case, wearables such as 2-3 can be issued by the children to be registered and attached while at home by simply using a computer and internet connection, where also a majority of health measurements can be taken using the wearable or another self-operated health measurement device 3-1.

Given this understanding and still referring to Fig. 7A, a person 1 wearing a registered wearable 2-3 that has been securely clasped around a body part (such as the wrist as depicted) and then also made active (i.e. "wearable confirmed," see Fig. 6A) as herein taught, is now enabled to use the wearable 2-3 as a they would have used a smart-ticket device and app 2a for accessing either of: 1) premises with issued tickets (such as a theme park, cruise ship, stadium, etc.), or 2) premises without issued tickets (such as a shop, restaurant, school building, office building, etc.) by using a general ticket as prior described in relation to a public health organization 44. The listed ticketed and non-ticketed premises should be understood as exemplary, rather than as limitations of the present invention, as any entity can choose to issue a ticket even if this ticket is a no-cost item, where it might generally be referred to as a "pass" and not a ticket. For example, schools may issue "student passes" that operate in coordination with a school app (that is the "entity app" in accordance with the teachings of both the incorporated PRIOR ART and the present teachings). In another example, a typically non-ticketing entity such as a food store can also start requiring that any visitors (or workers) come through controlled access points such as 5a, 5b (see the incorporated PRIOR ART as well as Fig. 1 and Fig. 8 of the present application), where the controlled access points 5a, 5b will only allow access if the person 1 can present a valid "work pass" (including personal and health validation).

In one exemplary case, a person 1 with active and confirmed wearable 2-3 approaches a premises' ID confirmation service kiosk 3-3 while still outside of the premises / ZONE4. In accordance with the teachings of the Prior ART THEME PARK GAMIFICATION, GUEST TRACKING AND ACCESS CONTROL SYSTEM, the ID kiosk 3-3 could be within a restricted ZONE3. A careful reading of the PRIOR ART will show that ZONE3 was confined in such a way that only one unidentified person X could enter ZONE3 per any valid issued paper ticket 2b, electronic ticket 2c or mobile device 2a running an entity app. The PRIOR ART showed then that a family for example with multiple children could enter as a group having only 1 smartphone device and app 2a with entity app but having preferably 1 electronic ticket 2c per person entering ZONE3. The PRIOR ART taught that for example in such a family or group case all of the electronic tickets 2c could be registered with the same 1 smartphone device and app 2a. Once inside ZONE3, each person that had an electronic ticket 2c would then take a turn at verifying themselves against their pre-registered biometrics (stored on smart-ticket 2 mobile device 2a) while within ZONE3, such as by entering a fingerprint or facial image that matched the pre-registered information associated with the particular electronic ticket 2c. The PRIOR ART then taught that this confirmation step included setting a close-range readable memory 2c-2 to a confirmed status for each individual, thus meaning that each individual was able to use only the "confirmed" electronic ticket 2c to then cross through an access point such as 5a, 5b to enter the premises ZONE4.

The PRIOR ART taught that the enclosed ZONE3 substantially prohibited individuals from pre-registering and confirming an electronic ticket 2c to a first person while outside of ZONE4, and then giving this registered and confirmed ticket to a second person (who was then not the ticket holder) for entry into ZONE4. A key to this prohibition was the confinement of ZONE3, where a careful reading of the PRIOR ART THEME PARK GAMIFICATION, GUEST TRACKING AND ACCESS CONTROL SYSTEM is recommended. In the present invention, as a careful reader will see, it is now possible to not require a ZONE3 or even a ZONE2, but merely a ZONE1 and ZONE4, if a person 1 is wearing a wearable smart-ticket 2-3 as taught herein, for example by using a ID service kiosk 3-3 outside of ZONE4.

Still referring to Fig. 7A, after person 1 wearing the registered and closed wearable 2-3 approaches an ID service kiosk 3-3 (presumably after possibly waiting in a queue, such as an airport line, where ZONE4 is the baggage check and entrance into the terminal, or a stadium or theme park line where the access control point is fully automated 5a or manned 5b). Once the ID kiosk 3-3 is approached, it is preferred that the person 1 is standing in a semi-restricted area, for example a 6' x 6' open space free of other persons, where the area is monitored by for example entity personnel, or more preferably by the image service 3-3d as depicted. It is also possible that the space in front of the ID service kiosk 3-3 is enclosed, allowing only a single person 1 or perhaps a family into the enclosed space at a time, where the present inventor notes that such an enclosed space is effectively acting as a ZONE3 as taught in the incorporated PRIOR ART. Again, the present Fig. 7A assumes a semi-restricted area in front of the kiosk 3-3 without any necessary entity personnel monitoring the area and that after performing the ID check provided by kiosk 3-3, the person 1 will then be free to enter ZONE4 the premises through a controlled access point such as 5a, 5b. As such, the present teachings and preferred arrangements with respect to the ID kiosk 3-3 and its use as described, should be considered as exemplary where variations are possible without departing from the scope and spirit of the invention.

Still referring to Fig. 7A, with person 1 standing in a presumed semi-restricted area (such as an open 6' x 6' space), the person 1 lingers as the image service 3-3d detects their presence using image processing for body tracking that will be well understood by those skilled in the art of image processing, where the camera(s) comprising image service 3-3d can be of any type for sensing image data such as RGB pixels and preferably also sensing or otherwise determining depth data, such as by using time-of-flight or structured light apparatus built into the camera or camera sensor, or by using multiple cameras or any other viable imaging based technology. Once detected as properly positioned in front of the kiosk 3-3, the kiosk 3-3 then also, or substantially simultaneously, attempts to communicate with the wearable 2-3 being worn by person 1 (and not any other wearable potentially nearby and able to communicate).

In one embodiment, the device ID means 3-1a on wearable 2-3 includes an NFC tag at least including identifying information, and person 1 "taps" their wearable on the kiosk 3-3 or an extension thereof (neither of which is depicted but both of which are well known in the art and will be well understood by those skilled in mobile device NFC contact readers). In an alternative embodiment as shown in the present figure, the ID kiosk 3-3 for example includes multiple device locator transponders such as 3-t1, 3t-2. As will be well understood by those familiar with close range communication technologies including wi-fi or Bluetooth, assuming that the wearable 2-3 communicates in the same protocol, then kiosk 3-3 can effectively locate and roughly triangulate the wearable 2-3 as being within the semi-restricted area, as opposed to perhaps other wearables 2-3 determined to be nearby but triangulated as outside of the semi-restricted area. In both cases, where either the wearable 2-3 includes a short range NFC reader and is tapped to the kiosk 3-3 for transmitting an ID code after which the kiosk 3-3 then pairs with the wearable 2-3 having the transmitted ID code, or where the wearable 2-3 is triangulated and paired from a standoff distance after which kiosk 3-3 requests the wearable 2-3 to transmit its ID code, the result is the kiosk 3-3 now has the unique ID and is in communications with wearable 2-3.

Person 1 now properly positioned preferably alone in a semi-restricted area in front of kiosk 3-3 is imaged such that kiosk 3-3 using image service 3-3d that captures an image of the person 1's face and transmits sufficient image data (or post-processed image data) to the wearable 2-3, whereupon the wearable 2-3 compares the image data received from kiosk 3-3 to the facial data stored on the wearable 2-3 as pre-registered ticket biometrics in 2-datum. If the wearable 2-3 confirms that the image captured by kiosk 3-3 matches the pre-registered image of person 1, then the wearable smart-ticket 2-3 sets itself to be confirmed and person 1 is then able to pass through a controlled access point such as 5a, 5b. If the entity is also requiring that a health regiment 2d-1 and / or 2d-2 is also being followed, then the wearable 2-3 only sets the health check confirmed status to "yes" (or equivalent) if the regiment has been properly followed up and until this point in time by person 1. Hence, wearable 2-3 is both confirming that the health regiment 2d-1 and / or 2d-2 has been followed to date and that the person 1 is the registered ticket holder and therefore also the person about which the valid health regiment data is applicable. Thus, the person 1 is free to enter the premises ZONE4, and otherwise cannot enter ZONE4 if either the health regiment or the ID verification steps have failed. As will be obvious to the careful reader, there is no motivation for person 1 to approach ID kiosk 3-3 if their health regiment 2d-1 and / or 2d-2 as required for entrance into the entity premises ZONE4 is not currently confirmed as "yes" / "compliant to the regiment(s)."

In another alternative, image service 3-3d still monitors the semi-restricted area but then does not need to image person 1 and provide this data to wearable 2-3. In this case, the kiosk 3-3 alternatively (or additionally for a combined more secure check) preferably sends a signal to the wearable 2-3 that is noticed by the person 1, or otherwise provides a visual and / or audible que. After being queued in at least one manner, person 1 then places their finger on a fingerprint reader comprising the device ID means incorporated into wearable 2-3, where then wearable 2-3 confirms that the fingerprint now being processed (while in the semi-restricted area) matches the pre-registered fingerprint, and if so, sets its status to confirmed in a manner as described above when discussing facial matching. When using only fingerprint matching all conducted on the wearable 2-3, it is noted that person 1 never shares any personal bio-metrics with the entity 40 through ID kiosk 3-3. It is also preferred that when using the imaging service 3-3d to capture an image of person 1 to transmit to wearable 2-3 for facial recognition and comparison, that the image data of the person 1 is deleted by kiosk 3-3 after this verification usage. In any case, even if the image of the person 1 is kept by kiosk 3-3, kiosk 3-3 has not received any other personal information that might further identify the person 1, including a name, personal ID or even a valid ticket number. However, it is also possible that any or all of this additional personal information is now provided to the kiosk 3-3 such that an entity network in communications with the kiosk 3-3 could track the identity of person 1 entering the premises for more secure situations, the tradeoff's of which will be well understood by those skilled in facilities management and data privacy.

As the careful reader will see, Fig. 7A as depicted and then as described provides the critical functions of verifying that a smart-ticket is present, either 2a or 2-3, that the ticket is pre-registered with prior bio-metrics provided before reaching the kiosk 3-3 (see the PRIOR ART and other teachings herein), that the person 1 now standing in front of the kiosk 1 is matched biometrically to the pre-registered holder of the smart-ticket 2a, 2-3, and that the health data accumulated to date on the ticket 2a, 2-3 in association with the identified and pre-registered person 1 is currently compliant with any necessary regiments 2d-1 and / or 2d-2.

Several additional variations are possible. For example, in the case where a parent is escorting their children into the facility and none of the children are wearing wearable smart-tickets 3-3, but rather the parent is carrying a smart-ticket device and app 2a and preferably at least 1 electronic ticket 2c for each child (although a paper ticket 2b can also be used as discussed in the incorporated PRIOR ART). In this situation, the parent or guardian moves into the semi-restricted area with the one or more children and then uses their own smart-ticket device and app 2a to pair with the kiosk 3-3 in any manner such as described above. It is noted that the parent or guardian has already pre-registered each electronic ticket 2c (or paper ticket 2b) to at least one biometric for the associated child, for example a fingerprint and / or a facial image. If facial data has been used for pre-registering, the parent then positions a child in front of the kiosk 3-3 and the imaging system 3-3d whereupon the kiosk recognizes that the child is so position and presents through a user-interface an image with perhaps a square marker around the face (as depicted for the young woman). The kiosk 3-3 then sends the image to the parent's smart-ticket device and app 2a where it is then matched against the pre-registered child's image. Assuming a successful match, and as described in the PRIOR ART, the parent then preferably uses the NFC reader on their smart-ticket device and app 2a to set the status of the child's electronic ticket 2c as maintained in close-range readable memory 2d-2 to "confirmed," such that the child can now use the confirmed electronic ticket 2c to pass through the controlled access point 5a, 5b and enter ZONE4. In the case were a paper ticket 2b is being used for the child, the smart-ticket 2 maintains the confirmed status and transmits the paper tickets ticket number or otherwise ticket identification information 2b-1 to the kiosk 3-3, where the kiosk 3-3 shares the ticket number with for example only the local access point(s) 5a, 5b, and whereby the paper ticket is made valid for use as the local access point(s) 5a, 5b only, and the child is free to pass into ZONE4 only using a paper ticket 2b.

As will be clear to the careful reader, after confirming personal identification and health regiments using a kiosk such as 3-3 in combination with the smart-ticket device and app 2a or wearable 2-3, an alternative operation for all smart-ticket 2 configurations of 2a, 2b, 2c as well as variation wearable 2-3 is to communicate the ticket number (as held in all of 2a, 2b, 2c and 2-3) to the kiosk 3-3 for sharing with at least one (preferably nearby) access point 5a, 5b, where preferably the person 1 is time sequentially the next person to pass through the nearby access point 5a, 5b at which point they scan (for optical reading) or tap (for NFC reading) their ticket 2a, 2b, 2c and 2-3 preferably at the nearby access point 5a, 5b to provide the ticket number to the access point 5a, 5b, which upon confirmation by the access point 5a, 5b allows entry into ZONE4 for the person 1.

The careful reader will also see that it is possible that two children could be brought into the semi-restricted area and one child be used to confirm the ticket 2c or 2b's identity while another child is then given the ticket and then gains essentially invalid entry ZONE4 by using the confirmed ticket meant for the other child. The teachings of the PRIOR ART therefore preferred that the area of confirmation, referred to as the "confirmation ZONE3" was secured rather than semi-secured and could only be entered by individuals carrying a valid ticket, e.g. smart-ticket device and app 2a, wearable smart-ticket 2-3, or accompanying electronic ticket 2c or paper ticket 2b. (Hence, in this example, each of the two children were required by system 102 to have their own ticket (e.g. 2b or 2c) validated for the health regiment prior to entering the confirmation ZONE3, wherein ZONE3 both tickets for each child need to be confirmed by identity check and even if switched between the children the goals and purposes of the present system are achieved - although as a careful reading of the PRIOR ART and present invention will show, this possibility of switching valid tickets confirmed by health and ID can be prevented if the entity 40 so desires.) According to the teachings of the incorporated PRIOR ART, once in the restricted area, each ticket then had to be successfully confirmed or the ticket became invalid and the individuals were blocked from entering ZONE4, thus discouraging false ticket swapping. Other variants were taught in the PRIOR ART for further increasing the security when using tickets 2c and 2b.

In the present depiction, the combination of a semi-restricted area (e.g. where you must enter through a turn style and cannot leave) except through controlled entrance 5a or 5b into ZONE4 or exit 5a or 5b out of the semi-restricted area is in combination effectively a PRIOR ART ZONE3, an is herein considered as sufficient for most low-to-medium public access into for example an airport terminal or a theme park. As a careful reader will see, by enforcing one-at-a-time access, even kiosk 3-3 can be semi-restricted with no personnel monitoring. This means that it is possible to have a faster que for single individuals and a slower que for families working with children as described or otherwise associated groups of people sharing a single controlling device such as smart-ticket device and app 2a.

Still referring to Fig. 7A, it is possible that a person 1 enters a semi-restricted area and successfully confirms their identity and is ready or able to enter ZONE4 through a controlled access point 5a, 5b but for some reason chooses instead to leave the semi-restricted area back into effectively ZONE2. As the careful reader will see, since the smart-tickets 2a and 2-3 both comprise time & location verification means 2-tlv, for example either GPS or LPS (local position system) including the use of Bluetooth or wi-fi as is well known in the art, it is possible that when the person 1 exits back into ZONE2, at a far enough distance away from ZONE4 their smart-ticket 2a or 2-3 can automatically detect this far enough distance and reset the smart-ticket 2a or 2-3 status to "not confirmed," thus requiring the person 1 to return again to the kiosk 3-3 for effectively re-confirmation before entering ZONE4. It is also possible that instead of this geographic reset method, or in addition to physical distance resetting, the smart-ticket 2a or 2-3 (or for that matter 2b and 2c) can effectively be set to have the confirmed status "expire" in "X minutes" if the smart-ticket 2a or 2-3 is not used to pass through a controlled access point 5a, 5b into ZONE4 within the "X minutes." As the careful reader will see, many variations are possible without departing from the scope and spirit of the teachings provided herein, and as such the present depictions and descriptions should be considered as exemplary, where some variations have been discussed and others will be obvious based upon a careful reading of the present invention and the incorporated PRIOR ART, and otherwise obvious to those skilled in the necessary arts.

Referring next to Fig. 7C, there is shown a progression of preferred process steps and operational motions describing the use of a self-operated health measurement device 3-1-d6 that is based upon a well-known "no-touch" thermometer that is further adapted according to the teachings herein. As is known in the art, a no-touch thermometer includes remote thermal sensing means such as a thermal camera, where various implementations provide a range of stand-off distances. In some cases, such devices can read body temperature for example from the forehead at distances of many feet or more away and might be used in airports or theme parks to remotely sense the body temperature of potential entrants into a restricted area, whereas other less expensive and less powerful variations, such as for in-home use, read body temperature only when placed within inches of the forehead. While the present depiction should not be unnecessarily limited, the present figure shows use of and teaches the adaptation of an in-home no-touch thermometer 3-1-d6 that has been further adapted to: 1) include wireless communication means such as Bluetooth or wi-fi for communicating with a smart-ticket device and app 2a, and 2) either new camera means or adaptation of existing camera means for at least imaging preferably the face of the ticket holder 1 whose temperature is to be determined by the thermometer 3-1-d6.

In a step (1), smart-ticket device and app 2a pairs with no-touch thermometer 3-1-d6 in accordance with the teachings herein and prior described and otherwise as is well known by those skilled in the other art. The preferred pairing operation ensures secure communications between smart-ticket 2a and thermometer 3-1-d6. In a step (2), preferably the operator of thermometer 3-1-d6 initiates the capturing of ID confirmation information from the person 1 such as a current facial image captured by the further adapted thermometer 3-1-d6, where for example the operator pushes a button 3-1-d6-b and is also shown the image on a screen such as 3-1-d6-s. Step 2 also comprises either of: a) the thermometer 3-1-d6 transmitting sufficient ID confirmation data such as from the current facial image to the smart-ticket device and app 2a, where after smart-ticket 2a compares the received ID data such as the current facial image with a prior captured and registered ID data of a similar biometric, such as a registered facial image of the ticket holder 1, thus confirming or not confirming that the ID data of currently imaged person 1 sufficiently matches the ticket holder 1, and then preferably transmitting confirmation information minimally including a "proceed" indication to thermometer 3-1-d6, or b) the smart-ticket 2a transmitting sufficient ID confirmation data such as from the prior captured and registered facial image of the ticket holder 1 to the thermometer 3-1-d6, where after either existing or further adapted processing elements of thermometer 3-1-d6 compares the received registered ID data with a current capture of a similar biometric, such as a current facial image of a person 1, thus confirming or not confirming that the ID data of currently imaged person 1 sufficiently matches the ticket holder 1, and then preferably transmitting confirmation information minimally including a "confirmed" indication to smart-ticket 2a.

Still referring to Fig. 7C, in a step (3) of the preferred operation, the operator of thermometer 3-1-d6 moves the thermometer 3-1-d6 sufficiently close to the forehead of person 1 while also ensuring that the confirmed face of person 1 stays sufficiently viewable to the thermometer 3-1-d6. As will be well understood by those familiar with image processing and from a careful consideration of the present usage description, as the operator moves thermometer 3-1-d6 closer to person 1, the full-face of person 1 may not remain within the field-of-view of the thermometer 3-1-d6's appropriate camera. However, as is well known, a facial image comprises many features, often called "details," that will remain substantially visible thus confirming that person 1 is being approached, and otherwise using image processing it is also possible to determine that the appropriate camera's field-of-view was switched away from the face of person 1 and therefore confirming that person 1 is not being approached, all as will be understood by those skilled in the art of facial recognition and object tracking. As the careful reader will note, in the present teaching related to thermometer 3-1-d6, it is important to determine that the face of the person 1 being measured matches that of the ticket holder 1, and that to confirm this matching a first image must be captured at a distance necessitated by the appropriate camera an optics as well as the facial matching algorithm, all as will be well understood by those familiar with image capture systems, optics and facial recognition, where it is assumed but not necessarily so, that this distance for capturing a first image is outside of the range for then also capturing a thermal measurement, thus necessitating that teaching of keeping the person 1's face "in view" during at step (3).

In a step (4), once thermometer 3-1-d6 is moved within a sufficient proximity of preferably the forehead of person 1, thermometer 3-1-d6 automatically detects at least one biometric such as the body temperature of person 1 (confirmed in step 2 as the ticket holder 1) and preferably indicates to the operator such as by making an audible sound and / or displaying information on screen 3-1-d6-s that the temperature for person 1 has been successfully acquired, or otherwise needs to be reacquired. As will be appreciated by those skilled in the art of sensors including cameras as well as medical biometrics, it is possible that a no-touch sensor determine additional useful biometrics other than body temperature, for example including skin color (especially for comparison to prior base-line skin color samples from the same person 1 as will be well understood by those familiar with the art of "change tracking," where a detected change in skin color over time is an indicative biometric), or including "retinal scan data" where the visible and / or infrared camera comprising device 3-1-d6 are used to scan the retina of the person 1 for determining biometrics useful for any one of or any combination of identification means or health check means, and where like skin color, retinal scan biometrics preferably include change tracking over time at least when applied to a measurement of the health state of person 1.

It is also possible that after confirming that the identity of person 1 matches the ticket holder 1 as discussed in relation to step (2), when moving device 3-1-d6 closer to person 1 for the determining of any one or more biometrics some other body part of the person 1 could be sensed, for example the ear. What is important to see and as will be understood by those skilled in the art of image processing and object tracking, as the device 3-1-d6 continues to capture images of person 1 (after preferably first capturing a biometric confirmation such as a facial image or even a retinal image) it is possible to determine that each next captured image (especially given a sufficiently fast image capture rate such as 10 to 30 images per second) is comparable to the prior image with at least some overlap and translation (such as x-y-z translation and zoom translation). In this regard, it can be determined that device 3-1-d6 is still imaging person 1 but now possibly with a change of spatial orientation so as to be imaging person 1's ear, or person 1's neck, or any other body part. Thus, such apparatus and methods as taught herein have significant use for allowing the inspection of health characteristics of a person 1 that are also matched to a confirmed person 1 identity, such that the determined health characteristics including any of biometrics are then confirmed to be associated with person 1, presumably also a ticket holder 1. As those familiar with the healthcare industry will appreciate, the teachings provided herein for combining the taking of health measurements with the confirming of registered person identity has many uses beyond those taught herein and in association with ticketing and gaining access to a premises. Therefore, it should also be understood that certain apparatus and methods as taught herein have use beyond the goals of a mutual health assurance system 102 (see also upcoming Fig. 8) and therefore may be used for other health related or similar purposes without departing the scope anticipated by the present inventor.

And finally, in a step (5), thermometer3-1-d6 preferably transmits the determined bio-metric for person / ticket holder 1 to the smart-ticket device and app 2a for use as data compliant with a health regiment 2d-1 or 2d-2.

What is most important to see is that an implementation of smart-ticket 2 and a health measurement device (either self-operated 3-1 or other operated 3-2) such as thermometer 3-1-d6 receive, provide or otherwise exchange sufficient data for confirming that a health measurement is being determined for a valid ticket holder 1. While Fig. 7C provides a preferred teaching for thermometer 3-1-d6, from a careful consideration of the present invention, and as will be well understood by those familiar with devices, device communication, computer data processing and networks, other variations are possible without departing from the spirit of the present invention, and as such the present teachings should be considered as exemplary, rather than as a limitation of the present invention.

For example, it is also possible to at least implement a "group mode" or a "pre-programmed mode" where the registered facial images of one or more registered ticket holders 1 are transmitted by system 102 to thermometer 3-1-d6 for storage, such as by transmission from an implementation of smart-ticket 2. In such a case, it is then possible that thermometer 3-1-d6 could be operated without necessarily requiring any of the above described steps (1) or (2), since thermometer 3-1-d6 could capture a current image for comparison to a prior transmitted and stored registered image(s), where upon confirmation of a match the biometric such as the body temperature of person / ticket holder 1 is then even stored on thermometer 3-1-d6 for transmission to a smart-ticket device and app 2a or otherwise a component of system 102 at some later time. Such a group mode / pre-programmed operation might for example be used by a family, a nurse at a school, a health-security agent at an office building, or a worker spot-check officer at a secured facility such as a military vessel. It is even anticipated that in such as pre-programmed mode a person 1 is able to self-operate thermometer 3-1-d6, where for example screen 3-1-d6-s swings out similar to screens often used on what is known as a "camcorder" into a position that can be viewed by the person 1 while at the same time person 1 is essentially facing the temperature sensing apparatus and facial camera including within device 3-1-d6, and then where person 1 self-directs device 3-1-d6 closer to for example their forehead in order to determine a biometric such as their body temperature.

Again, what is most important to see is that system 102 provides means for confirming the ID of a person 1 as being a valid / authorized ticket holder 1 (or work pass holder 1 or equivalent as discussed in relation to upcoming Fig. 8), and that a health measurement was captured of this same valid / authorized person 1, where many variations have been shown and still yet many other variations are possible, all staying within the present teachings of system 102.

Referring next to Fig.'s 7D and 7E there is shown a progression of preferred process steps and operational motions describing the use of a self-operated health measurement device 3-1-d7 that is based upon a well-known "touch" or "contact" thermometer that is further adapted according to the teachings herein. As is known in the art, a touch thermometer includes a sensing surface for contacting the skin of the body and reaching a steady state that is determined to be the body's temperature. Such sensors are often wired to a controlling, powering, or data receiving apparatus, for example including a computer processor and software. A limitation of the wired approach is the distance of the wire therefore limiting the range of use of the temperature sensor such as 3-1-d7, whereas an advantage is at least the reduction in cost and the simplicity of the sensor 3-1-d7. The presently depicted thermal sensor 3-1-d7 is shown as a low-cost apparatus that could for example be provided at no charge to a ticket holder for use in complying with a health regiment, where for example the entity 40 is a theme park, airline or cruise ship and the person / ticket holder 1 is being required to confirm their body temperature over 1 or more instances of time before arriving at the entity 40's premises to gain desired entry with a validated ticket / access rights, and even while at the premises or after leaving the premises, all as to be discussed in greater detail with respect to upcoming Fig. 8.

Referring to Fig. 7D, in a step (1), smart-ticket device and app 2a is connected to touch thermometer 3-1-d7 as a matter of plugging the wire from thermometer 3-1-d7 into the smart-ticket 2a's appropriate port, all as will be well understood by those familiar with wire connection devices in general, and mobile devices such as smartphones (that can be used as mobile device of smart-ticket 2a) in particular. It is preferred that the portrayed wired connection provides both a secured data communications link as well as power for device 3-1-d7, all as will be well understood by those skilled in the art of device electronics and communications. In a step (2), smart-ticket 2a confirms the ID of person 1 to be ticket holder 1, for example using facial image capture and recognition, all as prior discussed herein, especially in relation to Fig. 4A.

Referring next to Fig. 7E, after in step (2) confirming person 1 to be of the proper identity as ticket holder 1, and still being in a connected communications state with touch sensor 3-1-d7, in a step (3) person 1 preferably brings thermal sensor 3-1-d7 into contact with a body part on their face, such as their forehead. As was taught in relation to prior Fig. 4A, health measurement sensor 3-1-d7 includes light communications means 3-1-d7-led such as a visible light or non-visible (e.g. IR) light LED, or any other light emitting device that is preferably small and low power. As was taught in relation to Fig. 4A, such a light communications means 3-1-d7-led is useful for confirming the device ID of device 3-1-d7 for example by outputting light for imaging by smart-ticket device and app 2a in combination with the face of person 1, where the outputting of light is preferably determined at least in part by a sync signal issued by the smart-ticket device and app 2a, all as prior taught herein. As will also be understood by those skilled in the art of communications, it is possible for device 3-1-d7 to communicate other data, such as a biometric including the determined body temperature of person 1, via light output signally using the light output means 3-1-d7-led, where this light communications path can be use either separately or in combination with the sending of data using the wired connection discussed first in relation to step (1). While both communication paths have advantages and disadvantages, what is important to see is that any one of, or any combination of at least these two communications paths may be used to transmit one or more determined biometrics regarding person 1 to a connected / communicating smart-ticket 2a.

Referring to Fig.'s 4B, 7D and 7E, as will be well understood by those familiar with bio-sensors including contact temperature sensors, contact pulse sensors and contact blood oxygen sensors, health measurement device 3-1-d7 could be further adapted to detect multiple biometrics such as body temperature, pulse and blood oxygen levels still conforming to the basic design and operation as described in relation to Fig.'s 7D and 7E. In this regard, both the devices 3-1-d2 (Fig. 4B) and 3-1-d7 (Fig.'s 7D and 7E) can be implemented as health measurement devices 3-1 (therefore self-operated) or 3-2 (therefore other operated) (see Fig. 3) for measuring at least any of body temperature, pulse rate and blood oxygen levels where device 3-1-d2 is wireless requiring power and preferably some form of ticketholder ID means 3-1b (in the case of self-operation) or 3-2b (in the case of other-operation) (see Fig. 3 and Fig. 4B) and device 3-1-d7 is wired and does not require ticketholder ID means as this function is performed by the smart-ticket device and app 2a. In either case, both devices 3-1-d2 and 3-1-d7 preferably include device ID means 3-1a (see Fig. 3).

In general, with respect to all self-operated health measurement devices 3-1 as described herein, and any variations as those familiar with the necessary arts will understand based upon a careful reading of the present invention, it should be well understood that the particular self-operated device 3-1 could also be other-operated, for example by a healthcare provider, and in this sense is functioning as an other-operated device 3-2. Therefore, the present descriptions of self-operated and other-operated should be understood in their descriptive use contexts as exemplary, rather than as limitations of the present invention as the context of self-operation versus other-operation can be change without departing from the spirit of the present invention.

Referring next to Fig. 8 there is shown an exemplary use of system 102 for controlling and verifying the: 1) the access of individual persons 1 that are working at the entity 40 premises ZONE4 74 or otherwise have reason to be at the location ZONE4 other than being a visitor/patron/guest, and 2) individual persons 1 that are visiting for example as patrons or guest the entity 40's premises ZONE4 74. For system 102, verifying preferably includes: a) assuring that the person 1 of either type has a valid smart-ticket (e.g. a visitor) or a valid "smart-work-pass" (e.g. a worker), where the ticket or work pass is specifically issued by the entity 40 or a general ticket or work pass issued by a public health organization 44, and where assuring includes determining that the smart-ticket (or pass) has a valid authentication code (such as 2b-1 or 2c-1, see Fig. 1 and the PRIOR ART THEME PARK GAMIFICATION, GUEST TRACKING AND ACCESS CONTROL SYSTEM) including a unique and preferably encrypted application ID or ticket (work-pass) number stored on or otherwise associated with the smart-ticket (pass) such as 2 (including forms 2a, 2b and 2c) as well as wearable 2-3, b) assuring that the person 1's identity is confirmed essentially at the point-of-entry into ZONE4 74, which is depicted in the present figure as being confirmed for a visitor in a ZONE3 73 that grants private identity entrance into ZONE4 and confirmed for a worker in a ZONE2 72 that will require the worker to share private biometrics such as a fingerprint and / or facial image with the entity for non-private identity entrance into ZONE4 74, and c) assuring that the smart-ticket confirmed to the person 1's identity includes sufficient health regiment verification information to ensure that the person requesting entry into ZONE4 meets the entity's health requirements, which may be different for the visitors (e.g. "Level 2 (L2)") than the workers (e.g. "Level 3 (L3)") as depicted.

What is also important to see regarding system 102 and Fig. 8 is that system 102 is able to provide "mutual health assurance," whereby the entity is assured that all "entrants" (being visitors or workers) have a valid ID and their health is verified prior to entry while then entrants are assured that premises' health is verified before they voluntarily enter ZONE4 74, where the premises is considered "healthy" if it has verified the identities and associated health levels of all prior entrants and only allowed entrance into ZONE4 74 of participant's with sufficient health measurements, therefore passing the regiment. Furthermore, for extended stay premises such as a theme park or cruise ship, all current entrants (especially including visitors) may be following a continuous health regiment that for example checks certain health metrics daily and as such all new entrants are assured that not only are those entering the premises ZONE4 74 "healthy" at the point and time of entry, but they are also being monitored for health while in the ZONE4 74 thusly increasing the assurance levels.

Still referring to Fig. 8, in the lower left corner of the figure there is shown a visitor person 1 carrying at least one of a smart-ticket 2 (including any form 2a, 2b and 2c) or a wearable smart-ticket 2-3, and possibly a self-operated health measurement device in any form such as a wrist-worn 3-1-d4 or 3-1-d5. It is anticipated that there are many possible ways for allowing a visitor person 1, or simply the general public be continuously shown a verification of the "health quality" of ZONE4, which might minimally include a smart-ticket 2 or 2-3 notification "all occupants comply with Level 2 health requirements" or "all workers comply with Level 3 health requirements and all visitors comply with health Level 2 requirements" presented on that devices 2, 2-3 user interface such as a screen or on signage outside of the ZONE4 74 premises.

It is anticipated that ZONE4 74 occupancy information is provided to any person 1 wanting to enter ZONE4 such as "ZONE4 is currently 75% occupied with respect to its visitor's capacity," etc. What is important to see is that system 102 supports creating this assurance and providing real-time information to any person 1, visitor or worker, desiring access into ZONE4 74.

Based upon a careful reading of the PRIOR ART THEME PARK GAMIFICATION, GUEST TRACKING AND ACCESS CONTROL SYSTEM in combination with the present teachings, it will be clear that a person 1 with at least one smart-ticket 2 (in any form 2a, 2b or 2c) or wearable smart-ticket 2-3 will first be able to gain access from ZONE2 72 into ZONE3 73 by having at least a validated and authenticated smart-ticket that also indicates that the health regiment followed by the registered person associated with the smart-ticket is currently "compliant" or "passing," or some similar indication of sufficiency to proceed. Once entering ZONE3 73, a person 1 can use any of the number of apparatus and methods described in the PRIOR ART and / or present invention to then confirm that their identity matches that of the registered person associated with the valid and authenticated ticket that gained access into ZONE3 73, and as such will then be able to choose to proceed into entity premises ZONE4, for which many example premises have already been identified and can include moving premises such as a bus, ship or plane. This type of access is described in the present figure as "private controlled health-verified access" 5a, 5b, where the privacy indicates that the person 1 is not required to transfer any identifying information to the premises such as their name, biometrics such as a fingerprint or facial image, or any other personal information. Any information such as an image captured by an ID kiosk such as 3-3 discussed in relation to Fig. 7A is assumed to be deleted by the kiosk 3-3 to retain person 1 data privacy. As a counter benefit, the entity 40 is assured that the person 1's health is compliant with a desired regiment and that the person 1 is properly identified as the owner of a valid and authenticated ticket registered to that person 1 only.

Still referring to Fig. 8, while it is possible that a premises uses a private controlled health-verified access 5a, 5b for allowing persons 1 that are workers onto the premises ZONE4 74, it is anticipated that at least some premises 40 will adopt more stringent "non-private controlled health-verified access" 5a, 5b as shown in the lower right-hand corner of the present figure. In this case, a person 1 that presumably is a worker has a smart-ticket 2 (in any form 2a, 2b or 2c) or a wearable smart-ticket 2 and approaches a controlled access point 5a, 5b that includes a traditional bio-check, such as a fingerprint reader and / or a facial recognition station. Worker person 1 might then tap their smart-ticket 2 or 2-3 to an NFC reader to identify their authenticated ticket / work pass after which the entity security system uses this information at least in part to identify pre-known bio-metrics associated with the authenticated work pass. The person 1 then provides the appropriate bio-metric, for example allowing their picture to be taken, after with this currently taken image of the person 1 is compared to the pre-known bio-metrics and the person 1 is validated for entry onto the premises, all as is well-known in the art of security systems. What is different is that the present teachings then further allow the security system to inquire upon the current state of the worker's health as in their current sufficient compliance with a health regiment 2d-1 and / or 2d-2, for example a public health organization 44's "Level 3" (L3) health check as depicted, whereupon successful health verification and bio-metric ID verification, the person 1 worker is then allowed entry into ZONE4 74. It is also understood that the entity's security system might then also retrieve and store any and all health measurements made by the person 1 in conjunction with their smart-ticket 2 or 2-3, all in accordance with the teachings herein. (Where it is also possible that each or any of these health measurements were already electronically communicated to the entity essentially substantially just after they were being taken, also as prior described.)

As the careful reader will see, the entity owning or otherwise controlling the premises ZONE4 74 will have gathered a significantly important amount of health verification information using the present teachings such that this information is then useable to provide assurance back to persons 1 having already entered the premises and persons 1 desiring to enter the premises.

Still referring to Fig. 8, as persons 1 of any type, visitor or worker, remain on the premises ZONE4 74, the present teachings provide for continuing the health regiment, where for example in the case of a cruise ship or theme park, oil rigs or platforms, nuclear submarines and other Navy ships or military installations, freight ships, space craft or stations, etc. visitors and possibly even workers remain in ZONE4 74 for a significant extended duration even including weeks at a time, health continues to be verified for the entire ZONE4 74 as desired by the entity, for example with certain health measurements by taken at least daily. Especially regarding health checks during extended stays in a ZONE4 74, it is well-known in the art of mobile device tracking, that it is possible to determine what mobile devices (such as 2a, 2-3 or even 3-1-d4 or 3-1-d4 or similar) come "near" or proximate to other mobile devices. Such a technique is for example being demonstrated and implemented for use by companies such as Google and Apple for tracking "contact" between individuals as they move about in the world. This type of contact tracing has been suggested as a means for helping for example to track individuals who may have been exposed or have exposed others to an infectious virus or other disease. One problem with such an approach is getting a sufficient majority of the individuals moving about in an area such as a large city to either carry mobile device, or if carried to run a necessary tracking app. Without this sufficient majority, the value of this contact tracking system is reduced significantly.

As the careful reader will recognize, under certain conditions where an entity 40 is essentially in control of the persons 1 being emitted into a controlled area ZONE4 74 such as a theme park, stadium, cruise ship, bus, airplane, building, etc., it is possible that one of the restrictions is that all persons 1 (visitors or workers) entering the premise be in possession of a smart-ticket 2 or more preferably a wearable smart-ticket 2-3 that cannot be separated from the person 1 without opening the clasp lock 3-1-d4-lck which can be used to signal the opening event and trigger a response from the entity motivated to get the wearable 2-3 reattached to the person 1. For example, if a theme park or cruise ship booking visits that might last days to even weeks includes a requirement that the visitors as persons 1 receive and register a wearable ticket 2-3 for example three weeks before their scheduled visit during which time they must adhere to a health regiment such as 2d-1 and / or 2d-2, then this health information collected prior to the person 1's attempt to enter the premises ZONE4 serves to certify that the health level of the person 1 is sufficient. Once person 1 enters the ZONE4, for example to remain for an extended 1 week stay, the person 1 must continue to wear the wearable 2-3 substantially throughout their visit, and perhaps for a period of time thereafter in order to monitor if they become sick after leaving, perhaps with a provision of removing the wearable 2-3 when the person 1 is tracked as being in their private room or a similar isolated or semi-isolated circumstance, were the tracking is enabled by any of the well-known types of technologies such as GPS or LPS (such as Bluetooth or wi-fi) that has been included in the wearable 2-3.

As will be evident from a careful consideration, the entity is then able to establish any of GPS or LPS smart-ticket 2a or wearable 2-3 tracking using the available communication technology built into the ticket 2a, 2-3 and a sufficient tracking infrastructure network positioned throughout the ZONE4 74, were for example a local positioning system (LPS) includes a network of signal transponders, emitters, beacons or devices otherwise sufficient for tracking tickets 2a, 2-3 in the chosen technology. Many options for technology are available in addition to GPS, Bluetooth and wi-fi of which are all considered to fall within the scope of the present invention, a number of which were discussed in the PRIOR ART THEME PARK GAMIFICATION, GUEST TRACKING AND ACCESS CONTROL SYSTEM including RFID, and others of which are known to those skilled in the art or will become known as technological advances in mobile device tracking continue into the future. The PRIOR ART even discussed using a combination of technologies, where for example in some circumstances cameras are used for performing facial recognition as, for example, both a means of determining what person 1 (guest) is currently sitting in a ride seat at a theme park and for creating video of that person 1 during the ride to be provided or sold back to that person 1.

Still referring to Fig. 8, what is most important to see is that the present invention provides teaching for a system that benefits all persons 1 by requiring all persons 1 to carry a smart-ticket device and app 2a or wear a wearable smart-ticket 2-3, or even a wearable health device such as 3-1-d4 or 3-1-d5. The PRIOR ART taught that the electronic ticket 2c comprised an extended range readable tracking number 2c-3 (see Fig. 1 and the PRIOR ART) that could for example be detected by a chokepoint wireless reader 6 (see Fig. 1 and the PRIOR ART). The PRIOR ART THEME PARK GAMIFICATION, GUEST TRACKING AND ACCESS CONTROL SYSTEM also showed how pressure sensitive mats (see element 14 at least in relation to Fig. 5b of the PRIOR ART) could be used in conjunction with other methods. The PRIOR ART INTERACTIVE GAME THEATER WITH SECRET MESSAGE IMAGING SYSTEM especially taught the use of cameras and pressure sensitive materials for use in tracking persons 1 (guests) at an entity (such as a theme park).

These same wearables being carried or worn by persons 1 can then also serve to create detailed location tracking data and person 1 to person 1 "contacts" within the ZONE4 74, where this additional detailed person 1 tracking was also discussed in the PRIOR ART including the INTERACTIVE OBJECT TRACKING MIRROR-DISPLAY AND ENTERTAINMENT SYSTEM that defined an entity 40 interactive gaming system (see especially element 48 in PRIOR ART Fig. 4) that in part uses information of guest whereabouts as determined by a guest tracking system 46 (same PRIOR ART Fig. 4) to direct an on-going entity game.

With respect to the person 1 to person 1 contact tracking, it is anticipated that the entity maintains an on-going log of all "contacts" between any and all persons 1 (visitors and workers) continuously throughout the operations of the entity's ZONE4 74. It is possible to keep this tracking anonymous by maintaining the contact information in association with a tracking code, where if it is necessary to notify a specific person 1 of for example a possible health concern such as a contact with a later determined symptomatic other person, this specific person can be notified through their smart-ticket device and app 2a or wearable 2-3. It is also then desirable for the entity 40 to require that each person 1 continue their health regiment after leaving the ZONE4 74, for example when a visitor leaves the theme park or cruise ship to return home. As those familiar with infections health conditions, it is then possible to determine if a first person 1 later becomes sick after leaving ZONE4 74 and additionally which other persons 1 would have been exposed to this now sick first person 1 at least while they were both inside ZONE4 74, whether the sick person 1 manifested symptoms either while within ZONE4 74, or after leaving ZONE4 74. Since essentially 100% of all persons in a ZONE4 74 at any given time are being monitored for person 1 to person 1 contacts, the careful reader will see that the present system 102 provides significant value in both infectious disease early detection and possible transmission contacts.

The careful reader will also see, especially after reviewing all the incorporated PRIOR ART, that tracking essentially 100% of the movements of persons 1 in ZONE4 provides at least two other major benefits. First, the entity 40 can use this information to redirect person 1 traffic flow throughout the ZONE4 74, accomplishing any one of or combination of the multiple goals including: 1) reducing person 1 density and therefore creating more safe distance with fewer possible person-to-person contacts, 2) reducing line queues and therefore wait times throughout the ZONE4 74, such as lines for getting on a theme park ride, and 3) using the tracking information at least in part for directing an entity-wide or similar interactive game (see especially the teachings for an interactive physical-virtual game incorporated PRIOR ART). A careful reading of the PRIOR ART THEME PARK GAMIFICATION, GUEST TRACKING AND ACCESS CONTROL SYSTEM will show that such detailed tracking information has useful benefits to detecting, identifying, and reuniting lost persons 1 (such as children in a group or family) to their group of family. In this regard, it is further anticipated that communications associated with this lost person 1 functionality including sending messages on any of the smart-ticket 2 (using form 2a), wearable 2-3 and even via a health-measurement device such as 3-1-d4 or 3-1-d5. And finally, a careful reading of the two incorporated PRIOR ART patents entitled PHYSICAL-VIRTUAL GAME BOARD AND CONTENT DELIVERY SYSTEM will teach the benefits of person 1 tracking information collected at an entity establishment ZONE4 for use at least in part as datum effecting the game play of an interactive board game.

Referring next to Fig.'s 9A and 9B, there is shown a pictorial representation of a health-verified guarded checkpoint system 103, where system 103 is a variation of the present invention 102. Examples of a guarded checkpoint especially include places for public gatherings that are not normally ticketed, for example like a shopping area such as Disney Springs in Orlando, Florida, a place of worship, a public park, a country fair, etc. Checkpoint 103 could also be a grocery store or restaurant as was discussed as exemplary premises for gaining access to with system 102. In a basic implementation of checkpoint 103, a person 1 has a smart-access mobile device 2-4 such as a smartphone running a health-verification smart-access app, where preferably, the smart-access app is made available for download via the internet.

Unlike system 102, guarded checkpoint system 103 does not require the use of a traditional paper ticket 2b or electronic ticket 2c, although in some variations it may be useful to include a health verification regiment 2d-1, and if so, then optionally also healthcare provider messages 2e. Whether or not system 103 is using a health regiment 2d-1, 2d-2, smart-access app running on device 2-4 supports interfacing with any of health measurement devices 3-1 or 3-2 as herein described or any alternatives as anticipated herein and would be obvious from a careful reading of the present invention. For example, smart-access device 2-4 interfaces with "no-touch" thermometer 3-1-d6 or wired touch thermometer 3-1-d7 in a manner as prior described with smart-ticket device and app 2a. What is important to see is that a person 1 still registers themselves using smart-access app 2-4, similar to smart-ticket 2a except that the one or more personal biometrics such as a fingerprint, facial image or retinal scan are not associated with a ticket number or even a particular entity, but rather with the app itself. A person 1 might register several people using smart-access device 2-4, for example the members in a family.

After having registered one or more persons 1 capturing personal biometrics with device 2-4, the one or more persons such as 1-1 or 1-2 might then be desirous of visiting a non-ticketing location, where this non-ticketing location has publicly available information regarding one or more types of personal health measurements that are required prior to visiting the non-ticketing location. For example, a shopping area might publish on its website that any visitors are required to show proof of having taken their verified temperature within 60 minutes of arriving for requested access. The non-ticketing location might also require that a person's temperature is to be taken at a geographic location that is a certain distance "d" away from the geographic location or area of the non-ticketed premises. For example, as a careful consideration will show, it is beneficial to require an individual to for example "take your temperature 60 minutes before arrival in your registered domicile location, and at least 1 mile from the premises." The ability for systems 102 and 103 to implement self-verified health-measurements that can be restricted to a geographic location (referred to as a "virtual geographic boundary" or a "geofence") and a specific time period provides a significant tool for maintaining social distances. In the converse, without such an ability, persons arrive at the non-ticketing location and for example have their temperature taken only to then find out after exposing others that they are running a low-grade fever.

While it is intended that system 103 be a simplified variation of system 102, it is possible that more than one type of health check is required and / or that a given health check is required to be taken more than once, for example over a multi-day period. Also, a separately communicated health regiment 2d-1 or 2d-2 as used in system 102, might alternatively be embedded into (and downloaded with) the smart-access app 2-4 in system 103, and in this sense an embedded health regiment as used in system 103 could be considered less flexible, updatable, and even "programable," as will be understood by a careful reading the present invention with relation to system 102. However, as a careful reader will note, it is also possible to have a smart-ticket device and app 2a that at times functions like a smart-access app 2-4, and for that matter includes both embedded health regiment(s) (like system 103) and updatable regiments (like system 102), and as such it is important to see the value of a regiment being an external "health rule" established as a requirement for access, where these health rules can be communicated using various means as will be understood by those skilled in the art of software systems. It is also important to see that in a guarded checkpoint system 103, it is desirable to include the minimal apparatus and methods for providing the simplest variations of health-verified access to a non-ticketed location.

Still referring to Fig. 9A, but assumed to be done prior to the arrival scene that is being depicted, a person using their smart-access app 2-4 then selects the type of personal health check, such as "personal body temperature," and uses an appropriate health measurement device such as 3-1-d6 or 3-1-d7 substantially as described in relation to Fig.'s 7C, 7D and 7E to comply with the health requirements published by the non-ticketing location.

Now specifically referring to Fig. 9A, in an exemplary operation a person 1-1 with a friend 1-2 wishes to visit a non-ticketed location such as a shopping area like Disney Springs in Orlando and already has a mobile device with the smart-access app downloaded, forming device 2-4. While Fig. 9A depicts the steps 1 - 3 to be conducted by persons 1 when arriving at a premises, it is assumed that prior to these steps 1 - 3 the smart-access device 2-4 has been used to register each person, such as 1-1 or 1-2, intending to use the device 2-4 to gain smart-access to the non-ticketing premises. Registering is like the teachings associated with system 102 except that there is no associated "access rights" (or ticket). For example, in system 103 registering preferably includes providing a verification facial image (so it can be viewed and verified by a guard 5, see upcoming discussion), one or more personal biometrics such as a fingerprint, the facial image itself, or a retinal image to be associated with the verification facial image, as well as other optional information such as a name for reference. For system 103, it is also understood that prior to the arrival steps 1 - 3 depicted in the present Fig. 9A, a person 1 has preferably obtained publicly available information to determine proper health measurement requirements for gaining access to the non-ticketed premises, for example including a type of health check such as "body temp," as well as a distance from the premises and time frame within which the required measurements must be taken (where again it is alternatively possible that this type of "regiment" information could also for example be conveniently downloaded from an entity website).

Similar to the teachings of system 102, in system 103 person's 1-1 and 1-2 then use smart-access device 2-4 with an appropriate personal health measurement device 3-1 or 3-2 to verify a personal health measurement in accordance with the requirements of the non-ticketing premises. For example, the requirement may be to verify their personal body temperatures within X minutes prior to, and d miles away from, the planned visit to the non-ticketing premises. To take their personal body temperature, persons 1-1 and 1-2 for example use either of no-touch temperature device 3-1-d6 or touch temperature device 3-1-d7, all as prior described in relation to system 102 and smart-ticket device and app 2a.

Still referring to Fig. 9A, persons 1-1 and 1-2 then enter their vehicle 50 and drive to the non-ticketed location. Upon arriving at the location, person 1-1 or 1-2 drives their vehicle 50 to a guarded checkpoint 103. At checkpoint 103 they see a code1 signage 52 displaying preferably a unique code updated for each next vehicle. For example, person 1-1 or 1-2 sees the code1 "5750" displayed on signage 52 after which either person 1-1 or 1-2 enters this code1 into their smart-access device 2-4 in a Step 1. After entering this code1 (such as "5750") into the smart-access app 2-4, app 2-4 uses the code1 along with other information preferably private to the app 2-4 and therefor unknown to the person 1-1 or 1-2 in order to generate a second code2 (such as "38Jv"). As will be understood by those familiar with unique code generation algorithms, there are many well-understood types of other information for use as input to an algorithm for generating code2 from codel, where for example this other information could include an encrypted location code downloaded with the app or automatically transmitted through a wireless connection as the vehicle 50 was coming onto the non-ticketed location premises. Again, what is preferred is that smart-access app 2-4 generates some unique code2 in such a way as to confirm that the app 2-4 is genuine.

Referring now to both Fig. 9A and 9B, in a step 2, person 1-1 or 1-2 preferably shows the code2 such as "38Jv" to a guard 5 for confirmation, as depicted in Fig. 9B. Guard 5 is preferably using an access point device 5c which is also displaying the same code2, such as "38Jv." In one variation, device 5c is electronically receiving information from system 103 regarding the updated code1 such as "5750" currently being displayed on signage 52, and is executing a algorithm that generates the code2 such as "38Jv" that is expected from a properly authorized and executing smart-access app 2-4.

Those skilled in the art of encryption systems will recognize that there are many possible variations for achieving what is essentially a verification that the smart-access app 2-4 is authentic. It is possible that the devices 2-4 and 5c exchange encrypted data as an alternative to this currently taught step 1 and step 2, thus even eliminating the need for code1 signage 52. As will be clear from a careful consideration of the situation addressed by system 103, it is preferable to optimize both the personal safety and convenience of persons 1-1 and 1-2 while also assuring the non-ticketing location that the smart-access app 2-4 is valid and therefore that the health-information to be provided in step 3 is also valid. In the present depiction, the operation of step 1 and step 2 is both simple and quick, thus serving the desired system needs.

Other variations are possible for confirming the validity and authenticity of smart-access app 2-4 without departing from the scope and spirit of the present teachings. Those familiar with entity premises management and software development will also recognize that it is not simple to create an application that forges the health data discussed in relation to the present teachings of either system 102 or 103, and that it is also possible to create an application that generates unique health measurement "certificates" for each measurement, something that can be made prohibitively difficult for a forged application to recreate. In this example alternative implementation, when a person 1 arrives at a premises for access, the health measurements sufficient for gaining access are not only confirmed to belong to the person but to have valid "certificates" that can be transmitted or otherwise communicated to the premises for verification. Thus, in this at least one alternative, the uniquely generated health certificates serve to also verify that the app running on either a smart-ticket 2a or smart-access device 2-4 is authentic, thus obviating the need for Step 1 as shown in the present Fig. 9A.

Referring still to Fig.'s 9A and 9B, after either person 1-1 or 1-2 uses smart-access device 2-4 to show a valid code2 to guard 5, and after guard 5 confirms that they see code2 on device 5c, either person 1-1 or 1-2 then uses their device 2-4 to show for example a first verification image 1-1 of a person in the vehicle 50. Guard 5 then confirms that they visually recognize this person 1-1 by comparison to their verification image, for example to be sitting in the vehicle 50. This step 3 is then repeated using smart-access device 2-4 for each person in the vehicle 50, in this depicted case for the person 1-2. It is noted that when each verification image such as 1-1 or 1-2 is displayed on smart-access device 2-4, either the image is only displayed if that person has followed the published health check requirement(s) (such as verifying their personal body temperatures within X minutes prior to the planned visit using a health measurement device such as 3-1-d6, 3-1-d7), or the image is displayed with a verifying indication, such as a green check mark or the words "verified." After each person in the vehicle 50 is confirmed by guard 5 to be in the vehicle 50, in a step 4 the guard 5 also visually checks to see that no other persons are in the vehicle 50.

As will be clear from a careful consideration of Fig. 9A and 9B, the present system 103 provides for a simple way for any number of persons wishing to visit a non-ticketed location to provide an assurance to that location that at least some one or more minimal health measurements have been taken and verified, such as a personal temperature check. Such a system 103, or variations as will be obvious to those skilled in the art of information systems and location access control, has several advantages over practices that are currently being implemented in the marketplace. For example, in some current marketplace situations, the persons such as 1-1 or 1-2 are being required to have their temperature read using a no-touch temperature sensing device such as 3-1-d6 shown Fig. 7C (without any of the adaptations taught herein for device 3-1-d6). This current marketplace method requires that the guard 5 come into an uncomfortable and even unsafe proximity to any persons such as 1-1 and 1-2, for example by putting the no-touch thermometer within an inch or so of the person such as 1-1's forehead. Furthermore, in the situation of a bus or even a van including many persons, guard 5 may find it challenging to undertake all the measurements without also putting themselves into an uncomfortable and even unsafe situation. In any case, conducting the temperature checks at the access point to the non-ticketed location, as is currently done in the marketplace, at least has the disadvantage of taking considerably longer in time duration per vehicle 50 that the teachings of system 103 provided herein.

It is also noted that a non-ticketing location can set up multiple paths for entry, where for example on one path persons in a vehicle 50 stop to have their temperature taken by the guard 5 using the current marketplace approach, and therefore do not need smart-access device 2-4 or sufficient health-measurement devices 3-1, 3-2, while in another path such as taught for system 103 herein, persons in vehicle 50 are pre-checked and uses their smart-access app 2-4 to save time and to be more comfortable. Such a dual-path approach is similar to toll roads that provide both a slower manually controlled onramp or a faster automated onramp, and where persons desiring the faster onramp then take the time to acquire the smart-access app 2-4 and any necessary health measurement devices such as 3-1-d6, 3-1-d7. It is even possible that a single vehicle comprises multiple persons some of which have had their health check pre-verified as described herein, and others which have not been pre-verified. In this case, when during step 3 an image such as 1-2 is shown to the guard, app 2-4 can include a symbol or wording such as "not-verified" indicating to the guard 5 that they should then use their own no-touch temperature device to verify person 1-2.

As will be clear to a careful reader, systems 102 and 103 have obvious blends and cross-over, where variations or combinations of the herein taught 102 or 103 apparatus or methods may be used without departing from the spirit and scope of the present invention. For example a combination of system 102 and 103 can be used to accommodate both ticketed and non-ticketed guests to a location that supports both access restricted and public access facilities, such as what is known as "City Walk" at the Universal Studios Park in Orlando, Florida. Therefore, system 103 as taught herein should be considered as exemplary, comprising key functions that have alternatives, and should not be considered to limit present invention.

As will also be clear from a careful reading and understanding of system 103 and the teachings related to Fig. 9A and 9B, persons 1 do not need to be in a vehicle 50 to be essentially "in a line" to gain health-verified access to a non-ticketed (or a ticketed) location / premises. For example, the persons could be in a walk-up line to access a store or get into a terminal at an airport or a bus station. There are many possible uses and as such those described herein, such as drive-up vehicles 50, should be considered as exemplary, rather than as limitations of the present invention. As will be further clear, there are already many situations were persons essentially get in line to gain access to a premises, both ticketed and non-ticketed, all of which can now gain additional benefits of health verification as a part of granting access.

It is be also well understood by those familiar with premises access using validated documents, in some of the access control situations, there are "other documents" that must be checked by an agent (like a guard 5), for example at an airport such an other-document would be a driver's license or passport. These documents are not currently thought of as "tickets," although for the purposes of the present invention they are necessary for determining "rights to access" (e.g. to board a plane or to enter a country upon deboarding a plane). As a careful understanding of these other documents will show, the documents typically comprise either visual or electronic means for verifying authenticity, where the agent or guard is trained to personally inspect the necessary document to confirm authenticity and often an identity match with the person providing the document (where the document for example includes an image of a person that the agent compares to the face of the person presenting the document).

The present invention further anticipates performing this "other document verification" step in combination with the teachings herein, where the smart-ticket device and app 2a or smart-access device 2-4 is then essentially performing these additional functions of the premise's agent or guard. For other documents that include electronic means for providing other document authenticity, such as an NFC readable tag, it is possible that either device 2a or 2-4 can use its own NFC reader (typically built into most of today's smartphones) to read and verify the electronic code in the other document, all while the person 1 is preferably remaining within a confined "confirmation" area as taught herein, for example confined by barriers or simply by a guard watching that only one person or a small group of persons at a time steps into the confined area (such as in a passport check line at a typical international airport). If this NFC readable information includes for example a document stored biometric such as a fingerprint, facial image or retinal scan, then the person 1 could use device 2a or 2-4 to subsequently provide a current biometric for automatic matching by device 2a or 2-4 with the document stored biometric, all while the person is within a confined "confirmation" area. In this sense, the devices 2a and 2-4 are confirming the person's identity as a valid other-document holder, that the other-document is itself valid, and that the person has complied with a required health regiment. In the case of smart-ticket 2a, the smart-ticket 2a also has the ability to confirm that the person is in possession of a valid ticket and is the valid registered ticket holder.

Referring next to Fig.'s 10A, 10B and 10C, there is shown a combination pictorial and block diagram representation of health-verified appointment system 104, where system 104 is a variation of the present invention 102 sharing some similarities with variation system 103. Examples of an appointment system include a doctor's office, a hair salon, or a government service such as a department of motor vehicles (that share some "walk-in" similarities with system 103 but can also have "scheduled appointment" similarities with system 104). Other examples will be clear from a careful reading and consideration of the teachings related to Fig.'s 10A, 10B and 10C. In a basic implementation of appointment system 104, a person 1 has a smart-appointment mobile device 2-5 such as a smartphone running a health-verification smart-appointment app, where preferably, the smart-appointment app is made available for download via the internet.

Unlike system 102, appointment system 104 does not require the use of a traditional paper ticket 2b or electronic ticket 2c, although as the careful observer will note, a verification or confirmation of a schedule appointment is a form of a "ticket" in that it is conferring a right to enter and be served at a certain place, date and time. What is different is that a person showing up for a traditional appointment does not normally come with a printed or carried ticket, but rather announces themselves upon arrival to be verified against an expected appointee list or otherwise simply recognized by the service provider. Unlike guarded checkpoint system 103, appointment system 104 does then have a form of access rights that are restricted to a place and time, and as such this "scheduled place and time" is important for the system to maintain. What is most important is that system 104 tracks the date and time of the upcoming appointment and synchronizes a preferably standardized health regiment around the appointment. The main benefit is that this allows the service provider to restrict (or otherwise alter) services to a person that fails to meet any one or more health regiment specifications, and in this sense system 104 is designed mainly to provide an assurance to the service provider of the acceptable health level of the appointee.

As will be clear from a careful consideration of the teachings herein, it is possible that the service providers are also using any of the teachings herein, such as the work-pass discussions especially in relation to Fig. 8, where the work-pass is a variation of a ticket and is used to assure the premises that each employee, non-visitor or other visitor entering the premises has a sufficiently verified health, where then the net total of the verified health of all employees, non-visitors or other visitors in a premises such as a doctor's office or hair salon can serve as information provided to the appointee (visitor) of the health quality of the service premises, thus becoming a modified appointment system with mutual health assurance, all as will be well understood by the careful reader.

Still referring to Fig.'s 10A, 10B and 10C, and like system103, appointment system 104 optionally uses a health regiment 2d-1 or 2d-2, where this regiment has been preferably downloaded onto health-verified appointment device 2-5 for use with the appointment app. As will be discussed shortly, when using a regiment 2d-1, 2d-2 the appointment regiment 2d-3a can be a list associated with a regiment 2d-1, 2d-2, effectively indicating which one or more health-measurements are required for the appointment, overriding any information such as the geofence or time restriction, or even adding new measurements or other restrictions to existing specified measurements. Otherwise, as will be discussed shortly, appointment regiment 2d-3a can be health regiment that is presumably similar to a regiment 2d-1 comprising one or more health measurements, and in this case provided by the appointment services 52, acting at least to that extent as an entity 40. Also, like alternative health-verified smart-access device 2-4, health-verified appointment device 2-5 is capable of interacting with any of health measurement devices 3-1 or 3-2 as specified herein, especially in relation to system 102.

Referring now specifically to Fig. 10A, after downloading the health-verification appoint app onto presumably a smartphone, thus forming health-verified appointment device 2-5, an appointee 1 (or person acting on behalf of the appointee) wishing to make an appointment preferably uses the appointment app to connect with an appointment scheduler module 52s being provided by the appointment services 52, all of which is well known in the art, and for which many variations are available. As those familiar with existing appointment systems will understand, it is typically the case that the appointee 1 is placing a voice call to a service representative, and the service representative is interacting with an appointment schedule module to then help the appointee 1 make their appointment. It is also well known that some traditional scheduler modules then send out one or more text messages to the appointee, first perhaps confirming the appointment and then perhaps sending one or more reminders as the appointment date draws near. In the present teachings, the appointee 1 has an appointment app 2-5, and regardless of how the appointment is made, that is by a service representative using scheduling module 52s, or by the appointee 1 using scheduling module 52s, preferably an appointment regiment 2d-3a or equivalent is transmitted from the appointment scheduler 52s to the appointment app running on device 2-5. What is most important about appointment regiment 2d-3a is that it provides sufficient information to either or both indicate a health regiment (for example by linking to a regiment 2d-1 or 2d-2) or to specify the health regiment including one or more health measurements with requirements.

What is also desirable about appointment regiment 2d-3a is that it further comprises health-verification specifications for use by app 2-5 in providing essentially confirmation of compliance with the on-going appointment health regiment 2d-3a, where a confirmation is transmitted from app 2-5 back to schedule module 52s via a verification token 2d-3b. For example, a preferred verification specification includes datum indicating a time period prior to the scheduled appointment (such as 3 days) when a health-status confirmation pertaining to the health measurements determined by device 2-5 regarding the appointee 1 must be transmitted to the scheduler module 52s. As has been prior discussed, the present system provides valuable teachings that allow for personal health information to be collected in detail about a person 1, but then only shared "in general" with an entity, where in general can mean shared as a "pass" / "fail" status, or a percent compliance or any other statistical measure that is useful to the entity without divulging private health data. It was also discussed that in some cases, the present teachings have value by also sharing detailed health measurement information, where examples include a more secure premises where a person 1 might not otherwise have the same rights to privacy. In the case of an appointment system, where the appointee 1 is a patient of a doctor with whom the appointment is being scheduled, it is also considered valuable that a verification specification included with 2d-3a has directives for communicating specific health measurement values, for example a body temperature, sleep cycle data, blood oxygen levels, or a glucose level measurement.

The careful reader will see that the system 104 teaches a feedback loop between a entity (in this case a services provider using an appointment services system 52) and the person (in this case an appointee 1), where the feedback including verification token 2d-3b can indicate a minimal amount of "on-going" health information (such as indications that the health regiment is being followed and that the appointee 1 passes all measurement thresholds) to a more substantial amount of health information (such as the actual health measurement values determined by any health measurement devices 3-1 or 3-2 along with perhaps the date and time of the measurement). The careful reader will then also understand that there are uses for providing this type of feedback loop using verification tokens 2d-3b at least with mutual health assurance system 102, and therefore the present specification of health-check feedback tokens 2d-3b in association with system 104 should be considered as exemplary, rather than as limiting. Just as system 102 and 103 have useful combinations and cross-overs, system 104 can have any of its novel elements combined with either system 102 or 103 as will be obvious to those skilled in the art of devices and software systems, as well as the marketplaces in consideration and their specific needs.

As will also be clear to the careful reader, this feedback of health regiment data has great applicability and advantages. For example, if an appointee is failing the health measurements 1 - 3 days prior to the scheduled appointment, then schedular module 52s can adapt by any combination of automatically interacting with the appointee 1 through communications with the appointment app 2-5 to reschedule the appointment, or alerting one or more specific service persons that a health problem may prevent an upcoming appointment, where after the specific service persons then contact the appointee 1 and work with the schedular 52s to create a new appointment date and time, after which a new appointment regiment 2d-3a may be transmitted to device 2-5. In another example where perhaps the services include healthcare monitoring, the services provider is alerted as to a developing health problem and can then take some preventative or proactive measure, such as sending a qualified nurse to visit with the appointee 1 and confirm their health condition. Thus, it will be recognized that the combination of a health regiment such as 2d-1, 2d-2 or 2d-3a along with triggered or scheduled feedback via verification tokens 2d-3b has significant applicability and is anticipated to provide for valuable additional functionality that will be obvious through a careful consideration of the present teachings and the marketplace needs.

Still referring to Fig. 10A, as with the use of smart-ticket device and app 2a and smart-access device 2-4, it is necessary for an appointee to register themselves with the health-verified appointment app 2-5 prior to starting compliance with a health regiment, all as prior described herein. After registration of for example persons 1-1 and 1-2, there are shown five key steps including: step 1, where the appointment is made, step 2, where an appointment regiment 2d-3a is transmitted to the app 2-5, step 3, where the appointee such as 1-1 or 1-2, conducts one or more health measurements, step 4, where preferably automatic feedback is provided via verification tokens 2d-3b, and step 5, where especially if the appointee 1's health check is properly verified (step 3) against a regiment 2d-3a, an access token 2d-3c is provided by the appointee for allowing for either of verified self-access to a premises or guarded access to a premises, all as herein discussed and as will be further discussed especially in relation to upcoming Fig. 10B and 10C.

As the careful reader will see, in system 102 access rights are granted in the form of a ticket (see especially ticket datum 2-datum in Fig. 3) to be pre-associated with registration biometrics and a verified health regiment, whereas in system 104 access rights are provided as token 2d-3c after registration and health regiment compliance are confirmed. Thus, the careful reader will also see that the present teachings should not be limited to the timing of the provision of any rights to access a premises, as useful variations are possible, and that in this regard what is important is that if necessary access rights are provided and ultimately combined with at least personal biometrics for confirming the identity of a person to whom the rights were conferred and personal health measurements for confirming the health status this same person, where the combination is usable for improving access control to a premises, and where the access control includes self-access or guarded access.

Still referring to Fig. 10A, as those familiar with appointment scenarios will understand, there are often situations where the appointment is made for a first person, for example a doctors appoint is made for a child 1-3, and where a second (or more) person(s) will also require access to the premises, for example a guardian such as adult 1-1. In a variation example, the service is a salon and the appointee is for example the adult 1-1 who is desirous of brining their child 1-3. It is also instructive to note that, for example in either case if the child 1-3 is sick or essentially "not verified" as passing a health regiment, the system 104 can respond in different ways. For example, in the case that the doctor's appointment is for the sick person 1-3, the system 104 has advantages in that it can provided verification of the health-state even including specific health measurements, and then could further provide a "restricted" access token 2d-3c that for example directs the guardian 1-1 to use a restricted accessway to the premises, and / or alerts one or more services workers that the sick person 1-2 is / will be arriving at a certain time. In the case where the appointment is for the healthy person, the system 104 has advantages in that it can disallow the healthy person from being permitted the access rights for additionally brining the sick person 1-3.

Referring still to Fig. 10A, what is also clear is that the preferred appointment system 52s be capable of allowing a person such as 1-1 to schedule an appointment for which they must then comply with a health regiment, and then also allowing this person 1-1 to request that additional non-appointees (such as 1-3) be allowed access, where then each additional person such as 1-3 needing access must also follow a health-regiment and then preferably are also provided an access token 2d-3c, or at least the access token 2d-3d provided to person 1-1 specifies what additional persons are also allowed on premises. As the careful reader will see, the appointment system 104 provides many novel and useful features that can be varied in specific step ordering, or even mixed in sequence (where for example steps 3 and 4 are an on-going mix of taking health measurements and sending tokens 2d-3b), and as such the present teachings should be considered as exemplary, rather than as limitations.

Referring next to Fig. 10B, there is shown an appointee 1-1 presenting their appointment app and device 2-5 to a access guard in a step 6A, where the visual information preferably includes the picture of the appointee 1-1 and an indication that access rights have been granted, where this type of access is similar to guarded access system 103. Upon visual confirmation by the access guard, the appointee 1-1 is then able to proceed onto the premises. As the careful reader will see, it is also possible to use any of self-access points such as 5a to efficiently allow the appointee 1-1 to obtain access to the premises without requiring a manned access point (essentially 5b of Fig. 1).

Referring next to Fig. 10C, there is shown the appointee such as 1-1 using device 2-5 to share information with a service provider preferably using portable access control app 5d in a step 6B for gaining access to a service or service premises. The shared information includes any of granted access information token(s) 2d-3c, personal ID information such as a visual image(s), or even current health status information, such as about person 1-3. As the careful reader will see, many variations are possible for allowing an appointee such as 1-1, and other possible persons such as 1-3, to gain health-verified access to an appointment, and that aspects of system 104 have combination uses with aspects of systems 102 and 103, and as such many variations are possible without departing from the spirit of the present invention.

As will also be clear to those familiar with the marketplace, a reservation system is similar to an appointment system in that a person is requesting access to a certain premises at a certain time, where for example a reservation might be made through a traditional app such as Open Table. Thus, the teachings relating especially to health-verification appointment system 104 should be understood to apply to other uses wherein a person is essentially scheduling a visit to a premises, whether the visit is a reservation, an appointment or any other variation known in the marketplace.

Referring next to Fig. 11, there is shown a combination pictorial and block diagram representation of health-verified honest-broker appointment system 105, where system 105 is a variation of the present invention 102 sharing some similarities with variation system 103 and is an extension of appointment system 104. Specifically, appointment system 104 addresses the operation of an entity such as a salon or doctor's office that sees customers or patients, respectively, on a scheduled appointment basis. In summary, appointment system 104 teaches the steps of: 1) making an appointment for an appointee to visit a premises, 2) transmitting an appointment regiment to the appointee, 3) following the regiment by the appointee, 4) providing verification tokens from the appointee to the premises, 5) providing an access token from the premises (scheduler) to the appointee, and 6) accessing the premises using the access token.

There are many situations where two or more individuals would like to arrange a meeting where the location or premises is secondary to the meeting. For example, a dating website can also be viewed as an honest broker for arranging verified meetings, where verification for example includes personal likes and dislikes, possible exchanged photographs, etc. In this case, the honest broker is a "meeting service" designed to help the two or more participants in the meeting to vet each other according to some one or more criteria. Systems such as a dating website are well-known in the art for vetting personal meetings. Other social gathering tools exist for arranging what is often referred to as a "meet-up," in which a group is losing formed around an area of interest and a date is set to meet around a particular topic or function, where often there is not specific vetting of individuals. In a more abstract form, two parents may call each other on the phone to arrange for their children to have a play-date or similar get together, where the approval of the parents is essentially the vetting process, and the parents are the honest brokers.

As those familiar with these and other example "meeting types" will see, there are significant advantages to adding a health verification check to the vetting process, even if there are otherwise no other vetting criteria. As the careful reader of the present invention will see, the teachings of the appointment system 104 are extensible for serving virtually any meeting type, those mentioned as examples and others not mentioned, whether the meeting is between two people or more, whether they have guardians arranging the meeting such as parents for children, or are making their own arrangements. In one exemplary use case, the honest broker appointment system 105 is a traditional dating service such as "eharmony," "Match," "It's Just Lunch," or "Tinder." What is most important to see is that these traditional services lack the ability and means to provide health-check related vetting along with the more traditional "personality vetting." Health check vetting can be for any health reason, where the regiment can specify some health measurements for verification that are on-going (such as a temperature check), where others are single instance (such as flu shot or a vaccination). Health check measurements can be made using self-operated health measurement devices 3-1 or other operated devices 3-2, all as prior taught. For example, a person wishing to comply with a health regiment might be required to visit a doctor to receive one or more tests for health verification, where the tests can be conducted anonymously as prior described such that the individual's results remain private and are only used to pass-fail a regiment 2d-1 or 2d-2.

Still referring to Fig. 11, it is also possible that an individual is following for example a publicly available health regiment 2d-2 with some levels of verification such as "L1," "L2," and "L3," all as prior discussed. In this case, appointment services 53 running schedule module 53s might provide an appointment regiment 2d-3a-1 or 2d-3a-2 that provides for health measurements and / or indicates that compliance with one or more specific other regiments (such as a public regiment 2d-2) is an acceptable alternative. In another case, the honest broker system 53 simply mandates that any participants desiring to come to a particular meeting must be compliant with some available regiment, again for example a public regiment 2d-2. In yet another case, the honest broker system allows a given "organizer" of a meeting, or otherwise any of the potential participants to a meeting, to select or agree upon a particular health regiment to follow, even perhaps creating their own health regiment to then be distributed to the participates serving as the health "agreement." In some cases, the honest broker system 53 and schedular module 53s are provided by a third party such as an on-line dating service or a meet-up organizing service. In other cases, the honest broker scheduling module 53s is downloadable software that can be executed on for example the meeting organizer's smart phone or computer, essentially making the organizer the honest broker as well as possibly a participant. In still yet another arrangement, multiple parents in a community have all download the honest broker scheduler 53s that provides for the establishment of a "private meeting network" where the parents are in control of agreeing upon the regiment and once agreed upon the downloaded schedule 53s then conducts the steps 1 - 6, where in this case the private meeting network may be scheduling some on-going series of ad-hoc meet-ups (e.g. play dates for their children, where optionally the parents also attend). As those familiar with computer system architecture will understand, there are many variations possible especially for at least distributing the functions of: 1) determining a health regiment, 2) determining a meeting time, 3) determining compliance with a regiment, 4) exchanging and aggregating compliance verifications, 5) changing arrangements, and 6) confirming meeting access. Hence, some functions can be executed locally on one or more personal devices such as 2-5-1 and 2-5-2, while other functions can be performed by a computing system remote from persons such as 1 and 2, such as honest broker services 53, while even the running of the processes verses the storing or replicating of data can be any combination of distributed over devices such as 2-5-1 and 2-5-2 or hosted on platforms such as services 53 running module 53s. What is most important to see is that the teaching specified herein in general, and for the appointment system 104 specifically, can be used to provide significant benefits for allowing groups of two or more individuals to arrange and hold health-verified meetings.

And finally, still in reference to Fig. 11, it is possible that the meeting point of the two or more participants following a health regiment approved by any variation of an honest broker system 53, is a premises that itself requires a health-check verification, such as a restaurant or theme park using system 102 or 103. As the careful reader will see, although the present figure depicts only two individuals 1-1 and 1-2, there is no restriction on the number of participants being served by system 103. It will also be obvious that variations of the steps 1 through 6 are possible with departing from the scope and spirit of the present invention. For example, the step 5 of providing access tokens followed by the step 6 of checking tokens before allowing access to a premises are optional, as for example on a date between strangers meeting for coffee at a café, or on a play date between to children meeting at a parent's house or a playground. Furthermore, the manner in which such steps as 6 are implemented is optional as well, for example at a large group meetup of unknown individuals there can be a single "accessway" system that verifies that each individual either has an access code or is still compliant and otherwise has the "right-to-meet" according to the terms of the meeting. In less formal arrangements, were for example a small group is meeting but where people are still less familiar, each person's device such as 2-5-1 or 2-5-2 can server as an accessway that verifies each other individual, either at the meeting or prior to arriving. And finally, for meetings between familiar individuals, all that may be required is to remotely exchange verified health status prior to the meeting where after each individual person such as 1-1 or 1-2 assumes the responsibility not to arrive at the meeting if they are not passing the regiment while those passing the regiment conversely know who to expect and who not to expect.

What is most important in this regard is that all individual participants have an assurance of the health state of the other participants prior to the meeting, and hence at the very least any given participant can choose to go, or to not go to the meeting, regardless of where it is held and regardless of any possible access check step 6. It is also possible in a private meeting network that for example all network participants have access to the current health-state of all other network participants, and that using this information can decide on an ad-hoc basis who to meet with and when. Those skilled in software architecture will then also understand that other software techniques can be used to confirm the current health state of an individual rather than the "pushing" of verification tokens from distributed participant device such as 2-5-1 or 2-5-2 to some shared schedule module 53s. It is also possible that the shared module 53s polls the current health state at any time as per request by any participant or as per a schedule for example tied to a planned meet-up date, where for example the schedule indicates to poll all participant devices, such as 2-5-1 or 2-5-2, for the current health state of the participants, such as 1-1 and 1-2 respectively, 1 day ahead of a planned meeting and then 2 hours ahead of the meeting. Also, as prior mentioned, the scheduling software 53s can be running on one or more participant's devices such as 2-5-1 or 2-5-2, where messages are passed between any two or more schedulers to exchange participant current health state information. Thus, the reader will see that the present teaching with regard to Fig. 12 are significantly extensible and should not be limited to a specific use case example or even the particular considerations of where module 53s is being executed or how health regiments (such as 2d-3a-1 or 2d-3a-2) and verified health states (such as tokens 2d-3b-1 and 2d-3b-2) are exchanged, as many variations are possible.

Referring next to Fig. 12A, there is shown a two-piece touch electrodermal-thermometer 3-1-d8 that is a variation of one-piece touch thermometer 3-1-d7 discussed especially in relation to Fig. 7D and 7E. Two-piece electrodermal-thermometer 3-1-d8 comprises a thermometer-piece 3-1-d8t that comprises a connection to both a smart-ticket such as device and app 2a and a validator-piece 3-1-d8v. The depicted connections are shown as wired via connector 3-1-d8t-c, where connector 3-1-d8t-c preferably provides both power and data communications as those skilled in the art of devices will appreciate. It is also possible that either or both pieces 3-1-d8t and 3-1-d8v include a battery and wireless communication means so that the connection wire 3-1-d8t-c is reduced or eliminated altogether, as will also be clear to those skilled in the art. (However, as will be shown in the preferred system, validator 3-1-d8v is passive and does not exchange data such that connector 3-1-d8t-c is simply acting as a tether with respect to validator 3-1-d8v.) Thermometer-piece 3-1-d8t is depicted as being worn on the forefinger of person 1 while validator-piece 3-1-d8v is depicted as being worn on the thumb of person 1. Other variations are possible, although the forefinger-to-thumb arrangement is preferred due to the opposing nature allowing for a simple pinching motion. Smart-ticket 2a is depicted as showing person 1 in the process of using two-piece electrodermal-thermometer 3-1-d8, where person 1 sees their image 1-img displayed on smart-ticket 2a along with messages such as "Device Verified: take temp on location shown" and overlaid graphics such as the circle-dot shown over the forehead of person 1 in image 1-img (but not labeled for the sake of clarity).

Referring next to Fig. 12B, there is shown on left a top-oriented view of thermometer-piece 3-1-d8t and on the right a bottom-oriented view of validator-piece 3-1-d8v. Referring first to the top-oriented view on the left, thermometer-piece 3-1-d8t preferably comprises finger sleeve 3-1-d8t-slv for fitting onto for example a forefinger, electronics 3-1-d8t-elc for controlling the electronic parts of the electrodermal-thermometer 3-1-d8t as well as communications via connector 3-1-d8t-c with a smart-ticket such as 2a, where communications includes the synchronized flashing of an light emitting device such as LED 3-1-d8t-led (all as prior taught in relation to other health measurement devices such as 3-1-d7). A main difference between the touch thermometer 3-1-d7 described in Fig.'s 7D and 7E and the electrodermal-thermometer component 3-1-d8-tmp is that component 3-1-d8-tmp comprises a touch thermometer than has been further adapted to also function as what is generally known in the art to be a electrodermal activity (EDA) sensor. An EDA sensor is also sometimes referred to as sensor for detecting "skin conductance," "galvanic skin response," electrodermal response," psychogalvanic reflex," "skin conductance response," "sympathetic skin response," or "skin conductance level."

An EDA sensor for determining electrodermal activity senses "a property of the human body that causes continuous variation in the electrical characteristics of the skin" (see the related Wikipedia definition). For the purposes of the present invention, determining continuous variation is of less importance as compared to determining an electrical characteristic that is indicative of the person 1's skin. For example, the resistance value of human skin is generally considered to be roughly 100,000 ohms when the skin is dry, where the resistance drops when the skin becomes wet or broken. The typical capacitance value of human skin to a far ground is generally considered to be roughly 100-200 pF. What is most important to see is that human skin has determinable electrical properties and that by further adapting a touch-thermometer (as depicted in relation to device 3-1-d7), electrodermal-thermometer 3-1-d8-tmp is able to both determine the temperature of the skin and at least one electrical property.

Referring in general to Fig.'s 12A, 12B and 12C, as well as upcoming Fig. 13, in operation it is preferable that device 3-1-d8 is first used to determine one or more baseline electrical properties of a person 1's skin, where then these baseline properties are retained for use during a later temperature measurement operation. For example, a baseline resistance measurement might be 95,000 ohms, where during a subsequent temperature measurement the current resistance value of the person 1's skin is recaptured for comparison to the baseline in consideration of a plus-minus variation threshold. If for example, during a temperature measurement, the person 1's skin resistance value is determined to be within 10% of the baseline value, the temperature measurement is accepted as valid. If, on the other hand, the resistance value is for example 54,000 ohms, well below the baseline, the smart-ticket 2a app preferably requests the person 1 to dry their skin and repeat the measurement, and / or directs the person 1 to try a different skin location.

One of the benefits anticipated by the further adaptation of the thermometer to include the measurement of at least one electrical property of the person 1's skin, is that this measurement is useful for detecting if the thermometer within the electrodermal-thermometer component 3-1-d8-tmp has been tampered with, for example by placing a substance or adhesive layer on the temperature sensing surface. As those familiar with electronics and devices will understand, this tampering for the purposes of altering the determination of the actual body temperature of person 1, will then most like also significantly alter at least one of the measured electrical properties of the skin with respect to the baseline and threshold. Thus, adding an electrodermal sensor for sensing at least one electrical property of the skin is useful for verifying the veracity of the temperature measurement of person 1. There are many variations of electrodermal sensors that are well-known in the art, where also there is significant continuing research. For the purposes of the present invention, what is most important to see is that by adding an electrodermal sensor additional measurements can be obtained and compared to a prior determined baseline (and / or even on-going average or all prior measurements) facilitating the confirmation of the proper and "un-tampered" use of a touch-temperature sensor. It is also noted that this is a particular advantage enabled by the use of a touch-temperature sensor versus a touch less-temperature sensor, i.e. that by coming into contact with the skin one or more electrical properties can be measured and used by any of the systems taught herein.

Thus the careful reader will understand that there are many sufficient types and arrangements of electrodermal activity sensors that may be adapted for use with the thermometer to form component 3-1-d8-tmp, any and all of which are sufficient for the purposes taught herein. The present component 3-1-d8-tmp should therefore in this respect be considered as exemplary and capable of achieving the stated purposes while not restricting to a particular electrodermal activity sensor or even a particular electrical characteristic, although the preferred characteristic is either or both of resistance and capacitance.

Referring now exclusively to Fig. 12B, on the right there is shown a bottom-oriented view of validator-piece 3-1-d8v. Validator piece 3-1-d8v is at least preferably tethered to temperature-piece 3-1-d8t by way of connector 3-1-d8t-c, where tethering assumes the function of holding together but not the function of supplying electrical power or communications, and thus tethering is a convenience function for person 1 helping to ensure that the two pieces 3-1-d8t and 3-1-d8v do not get separated and misplaced or lost. In the preferred arrangement, validator-piece 3-1-d8v is passive, where power and communications are therefore unnecessary. What is important is that the validator-piece 3-1-d8v includes on its surface for coming into contact with temperature-piece 3-1-d8t coatings or a surface material of a pre-known and / or calibrated value with respect to the one or more electrical properties to be measured by the electrodermal-thermometer 3-1-d8-tmp. In the present depiction, the contact surface of validator-piece 3-1-d8v is shown to be segmented into 4 quadrants, namely 3-1-d8t-q1, 3-1-d8t-q2, 3-1-d8t-q3 and 3-1-d8t-q4, where each quadrant is detectable as having a sufficiently different value with respect to the one or more electrical properties measurable by electrodermal-thermometer 3-1-d8-tmp. For example, if the measurable electrical property is resistance, quadrant 1 "q1" might have a value of 10,000 ohms, while q2 has a value of 50,000 ohms, q3 has a value of 100,000 ohms and q4 has a value of 150,000 ohms. As will be clear from a careful consideration, any arrangement of 1 or more coatings or materials into 1 or more areas of the contact surface of validator-piece 3-1-d8v, requires a sufficient construction of the electrodermal sensor arrangements such that the 1 or more areas can be sufficiently measured. In the simplest form, a single surface coating or material is preferred and sufficient for the teachings provided herein, where the depiction of 4 areas is an alternative designed to show a range of possibilities, where the range will be understood to possible increase the verification assurance.

Referring next to Fig. 12c, validator-piece 3-1-d8v can be used to first test / validate the proper functioning of temperature-piece 3-1-d8t, where the two pieces are brought together in a pinching motion as depicted in a secession of three images moving from right to left. Starting in the lower right where the validator-piece 3-1-d8v is depicted as the furthest away from temperature-piece 3-1-d8t, and then moving through the middle image where piece 3-1-d8v is moved closer to piece 3-1-d8t but is still not touching, to finally in the leftmost image where validator-piece 3-1-d8t is in contact with temperature-piece 3-1-d8t. To assist the person 1 in this proper alignment, a lip 3-1-d8t-lip is preferred or some similar adaptation to validator-piece 3-1-d8t such that the person can have a tactile feedback for guidance, as will be clear from a careful consideration of the described usage.

Once touching, the electrodermal sensor(s) included in electrodermal-temperature component 3-1-d8t-tmp may then be used to measure one or more electrical properties of the contact surface of validator-piece 3-1-d8v. While the validator-piece 3-1-d8v is depicted as having a plurality of measurable points, each preferably with different electrical characteristics, it is possible and useful that the contact surface of validator-piece 3-1-d8v comprises only a single coating yielding a one or more measurable electrical characteristics (i.e., as opposed to the 4 quadrants "q1," "q2," "q3," and "q4"). Thus, it should be understood that the actual number of surface divisions, from 1 (no division, but rather a single coating / material or other equivalent) to 4 (as shown) or more is exemplary, where many variations are possible and anticipated.

Referring in general to Fig.'s 12A, 12B and 12C, as well as upcoming Fig. 13, in operation it is preferable that validator-piece 3-1-d8v is first used in combination with temperature-piece 3-1-d8t to determine one or more electrical properties respective of the contact surface of validator-piece 3-1-d8v. After taking these validation measurements, they are comparable to pre-known values of the same contact surface and are expected to match within a very tight threshold, for example 1% - 2% versus for example the 10% threshold provided as an example threshold for use when validating as compared to a baseline skin electrical property measurement. Hence, validator-piece 3-1-d8v is anticipated to be of low cost in that it is passive, and to be of use for providing a more controlled contact surface than the skin for validating that the electrodermal-temperature component 3-1-d8-tmp itself is functioning properly. The careful reader will then also note, that in combination, while the validator-piece 3-1-d8v is useful for confirming component 3-1-d8-tmp within tighter thresholds, thermometer-piece 3-1-d8t is useful to measure at least one electrical property of the skin for then assessing if the "normality" of the skin, where this normality could potentially be altered by placing something on the skin in an attempt to alter a temperature reading.

Referring next to Fig. 13, there is shown a flowchart of preferred steps for use with any of self-operated health measurement devices 3-1 or other-operated devices 3-2. As will be clear to the careful reader, not all steps are implementable in all possible devices 3-1 or 3-2 taught or anticipated herein. It should therefore be understood that the teachings in relation to Fig. 13 are meant to show a full-range of the novel functions of the present invention with respect to the taking of health measurements but are otherwise not indented to be limiting in that many useful sequences of functions are possible and herein anticipated, either without implementing all of the functions depicted in Fig. 13, or without those functions being performed in the sequence depicted in Fig. 13.

Referring now to Fig. 13 in relationship to exemplary health measurement device 3-1-d8 as depicted in Fig.'s 12A, 12B and 12C, the following preferable operational steps are described.

In step 70, exemplary health measurement device 3-1-d8 comes into communication with a smart-ticket such as device with app 2a, where device with app 2a receives a preferably encrypted communication from device 3-1-d8 and verifies that the device 3-1-d8 has valid credentials. During this validation step, several variations are possible. For example, if the health device such as 3-1-d8 includes a processing element, the device itself can request an encrypted code be provided by the smart-ticket device with app 2a, whereupon health device 3-1-d8 will not operate if the encrypted code it has received from device 2a is not validated by the device 3-1-d8. It is further possible that the exchange of credentials is included in a process that creates encrypted return-confirmation codes, further ensuring that both the particular smart-ticket (such as device 2a) and particular health measurement device (such as 3-1-d8) are valid. For example, upon receiving an initial credential (such as a unique ID) from the companion (i.e. device 2a receiving device 3-1-d8's encrypted ID, and / or vice versa), the ID is then used at least in part to determine a second unique ID that is provided back to the companion as a return-confirmation code. As those familiar with device validation will understand, this type of generation of a return-confirmation code based at least in part upon the original device credential (e.g., ID) is harder to tamper with in that stealing an original device ID code is not sufficient. It would also be necessary to understand how the algorithm for generating a return-confirmation code works, which itself can also be designed to be unique to either or both of the companion devices. What is important to see is that once in communication, the variation of the smart-ticket such as device and app 2a and the health measurement device 3-1 or 3-2 act to confirm each other as valid before proceeding to step 71, and otherwise essentially exits without measurement at step 78.

In step 71, some of health measurement devices 3-1 or 3-2 may include means for validating that the device is operating properly. For example, in relation to exemplary health measurement device 3-1-d8, electrical properties are first determined as secondary measurements of one or more contact surfaces (e.g. validator-piece 3-1-d8), where the electrical properties must then meet an expected value and tolerance, for example in comparison to pre-known contact surface property values. If these expected values are not confirmed, the operator (assumed to be the person 1 being measured) is then preferably given further instructions (step 72), such as "clean off all contact surfaces," etc. After complying, the operator is then allowed to retake the secondary measurements for confirming that the health measurement device is operating properly. Ultimately, if confirmed the process proceeds to step 73, and otherwise if not confirmed essentially exits without measurement at step 78.

In step 73, a smart-ticket variation such as device and app 2a validates the identity of the person 1 to be measured, all as taught herein, for example by taking a personal biometric such as a finger print or facial image. However, it was also taught in relation to touchless temperature device 3-1-d6 that the responsibility of personal ID validation can be shifted from the smart-ticket variation such as 2a to the properly equipped health measurement device such as 3-1-d6 that confirms person identity through facial recognition. Health measurement device 3-1-d2 was shown to be able to validate personal identity by taking a fingerprint of the person 1. After validating person 1's identity with respect to the "right-to-access" health regiment (and possibly also a "right-to-access" ticket / pass), in step 74 instructions are preferably given to the person 1 as to where the measurement should be taken. Step 74 can be fulfilled in a number of ways, or even skipped altogether if there is an assumed understanding as to the location of the health measurement on person 1 (for example when working with finger pulse/oxygen detector device 3-1-d2). Because it includes a UI display screen, device and app 2a is ideal for providing a visualization including perhaps a graphic overlaid onto an image of the person 1 (such as 1-img in Fig. 12A) for indicating a measurement location. It is possible that other devices such as touchless thermometer 3-1-d6 that also include a display could provide the location information of step 74. As a careful consideration will show, it is useful for the device and app 2a working with the health regiment to log along with each health measurement the body location that was measured. For example, when taking temperature, it is possible to use many body locations, even on the person 1's face. By randomly moving the location around for each measurement, it is more difficult for a person to tamper with their skin prior to the measurement. It is even possible to essentially take a first measurement at a first location timewise directly followed by a second measurement at a second location, where the combination of measurements at different locations can increase veracity. Regardless of whether or not any health measurement location information is provided or which device is used to provide the information, if the person 1's identity is confirmed than the process ultimately proceeds to step 75, and otherwise if not confirmed essentially exits without measurement at step 78.

In step 75, some of health measurement devices 3-1 or 3-2 may include means for validating that the measurement location conditions are valid. For example, in relation to exemplary health measurement device 3-1-d8, electrical properties are first determined as secondary measurements of the body location (e.g., skin) surface, where the electrical properties must then meet an expected value and tolerance, for example in comparison to a prior determined baseline, or an average value calculated based upon several prior measurements, where the baseline and average can even be combined for a comparison. If these expected values are not confirmed by this secondary measurement, the operator (assumed to be person 1 being measured) is then preferably given further instructions (step 72), such as "dry off the skin as the specified location," or "measure the alternatively location shown," etc. After complying, the operator is then allowed to retake the secondary measurements for confirming that the health measurement device is operating properly. If confirmation step 75 fails, and afterwards the device operator is given instructions, the process is the preferably returned to step 71 in order to reconfirm that the device is still operating properly. While it is possible to assume that the device is still valid based upon the earlier passed device operation check (in the prior-executed step 71), thus skipping a return to step 71, in either case of repeating step 71 or not, it is preferable that person 1's validity is re-established by returning to step 73. As a careful consideration will show, if a measurement location is determined to have insufficient conditions (such as a skin resistance well below an expected baseline), than in compliance with any provided instructions of step 72, it is likely that the person 1 will no longer be in view of device and app 2a (or essentially the device that was used to confirm the person 1's identity), and as such that identity will have to be re-established if the health measurement is to be of the highest integrity. Ultimately, if confirmed the process proceeds to step 76, and otherwise if not confirmed essentially exits without measurement at step 78.

And finally, in step 76, a health measurement is taken using a validated health measurement device confirmed to be operating properly, of a validated person and on a body location confirmed to have valid surface properties. As prior taught, and preferably substantially simultaneous to the taking of the measurement, the device and app 2a emits a preferably encoded control signal that is received by the health measurement device and used to for example emit a series of coordinated LED flashes, where the confirming signal offers a final validation point detectable by device 2a for example using a camera included in 2a, and where it is even possible that the confirming signal transmits some of all of the data that is representative of the measured health value (e.g. flashing a sequence that is interpretable as "96.4"). It is preferable that device 2a (i.e. the device receiving the visually confirmed "flashing" encoded data), compares the visually received encoded data to the electronically communicated encoded data (either wired or wireless), where the comparison offers another integrity check. Ultimately, after the measurement is taken and preferably also confirmed, the process proceeds to step 77 and exits successfully, and otherwise if not taken or not confirmed essentially exits without measurement at step 78.

Still referring to Fig. 13, as those skilled in the arts of devices and software processes will understand, it is possible to accomplish the method steps taught in relation to Fig. 13 in a sequence that varies from that depicted. As also mentioned, at least some steps may be omitted without decreasing the overall integrity of the process, for example by omitting step 72 "provide instructions" or step 74 "indicate measurement location." It is also possible to provide meaningful integrity of the process without including all the confirmations, for example omitting step 71 "confirm device operating properly" or step 75 "confirm measurement location conditions are valid." These steps might be omitted because they could be substantially confirmed by other means, for example using image analysis of a thermometer such as 3-1-d1 being properly located in the mouth of person 1 (see Fig. 4A), where it is assumed that the conditions inside the mouth are "less alterable" than the skin conditions on the forehead. Some devices such as wearables 3-1-d4, 3-1-d5 may not require or even have a reasonable process step for confirming their proper operation. Thus, what is important to see is the present invention teaches a process including a set of variably sequenced steps that when completed substantially assure that a health measurement is taken using a validated health measurement device confirmed to be operating properly, of a validated person and on a body location confirmed to have valid surface properties.

Referring next to Fig. 14A, there is shown a pictorial representation of key components of an "at-home" or self-serve health test kit to be used by a person 1 for gathering biometric samples. At-home test kits are well known and for example are currently being used to test for the Coronavirus (COVID-19). A typical kit includes a sampling device such as swab 80. In at least one configuration, swap 80 includes an elongated shaft comprising a bottom 80-btm through to a top 80-top, where the intersection between the bottom 80-btm and top 80-top is scored (or pre-cut) at a break-point 80-bp, such that after taking a biometric sample the person 1 can apply reasonable pressure to top 80-top and bottom 80-btm causing the entire elongated shaft to be broken into the two parts, 80-top and 80-btm, substantially at the break point 80-bp. Top 80-top is then placed into a sample device container 82 for transport to a health service testing lab. Top 80-top includes a tip 80-tip that is the swab by which the health data sample(s) are collected (for example by sticking the swab into the mouth to touch and rub against both left and right tonsils and then afterwards sticking the swab deep inside each left and right nasal cavity).

It is noted that the separation of 80-top from 80-btm helps to avoid contamination from the bottom 80-btm during transportation and testing, as well as to reduce the device container 82 size. However, for the purposes of the present teachings, as will be clear from a careful consideration of the novel adaptations taught herein, it is not mandatory that swab 80 have a break point 80-bp or even that a top 80-top is able to be separated from a bottom 80-btm for transportation. Some currently available test kits are known to transport the entire swab 80 without first breaking, and as such the present invention should not be limited by the particular exemplary health test kit being used to teach the novel adaptations provided herein, as many variation test kits are known and can be so adapted as described especially in relation to Fig.'s 14A, 14B and 14C.

Still referring to Fig. 14A, the present invention teaches adapting swab 80 to further include an electronically readable / writable passive tag 80-nfc, where an NFC chip is preferred since a typical smartphone that could be used as a mobile device 2a for running the smart-ticket app includes an NFC reader / writer interface, while other technologies are possible such as Bluetooth or RFID. Swab 80 is also preferably adapted to further include visually readable markings 80-bcd such as a barcode or similar. As a careful reading with respect to upcoming Fig.'s 14B and 14C will show, by providing at least one of these preferred adaptations, it is possible to verify that the health data sample(s) being taken are from the identified person 1. As a careful consideration will show, this verification of the health data sample(s) then further extends to any health measurement results created by a health services lab using the verified health data sample(s).

Referring next to Fig. 14B, swab 80 is shown being held by person 1 while a smart-ticket device 2a is capturing an image 1-img of person 1 such that smart-ticket device 2a is substantially simultaneously identifying person 1 and the swab 80, where identification of swap 80 is confirmed by visually detecting and decoding the pattern of markings 80-bcd. As will be discussed shortly in greater detail with respect to upcoming Fig. 14C, this is a key step in the verification of the health data samples as belong to the person 1. A swab 80 often comes in a protective wrapping, where it is preferable that person 1 presents the swab 80 still in its protective wrapping for verification by device and app 2a using markings 80-bcd. After visual verification of swab 80, person 1 then removes the protective wrappings and the remaining sampling procedures continue while the swab 80 substantially remains in the view of device and app 2a, all as to be further discussed in detail below. As a careful consideration will show, although this additional step of imaging swab 80 before and after the protective wrappings is preferred, it is not necessary for accomplishing many of the other verification goals as presently taught. Furthermore, since accomplishing this unwrapping would typically require both hands for person 1, it is also preferable to set device and app 2a in a stand, all as discussed in upcoming Fig. 15A (see element 2a-std).

Referring next to Fig. 14C, there is shown a flowchart of the preferred use of a health sample device such as swab 80 for collecting at-home biometric samples. As indicated by the dashed lines grouping any two or more steps in the flow, the preferred process includes: 1) the anonymized personalization 90 of the health test kit, 2) the taking of health sample(s) 91 using the personalized kit including swab 80 (see Fig. 14A), where taking the samples includes 2a) authenticating each sample 92 using smart-ticket device 2a (see Fig. 14B) and 2b) taking each sample as instructed 93, where after 3) the sampling device (in this case at least the top 80-top of swab 80) is inserted into device container 82, 4) the device container 82 is then inserted in a transport container 95 for transport to a health service test lab or similar, and 5) where preferably encrypted results of the testing are provided in electronic format to smart-ticket device 2a associated with the person 1 preferably for inclusion as health measurements in association with a regiment 2d-1, 2d-2.

As those familiar with at-home test kits and data privacy will recognize, the present teachings especially as related to Fig.'s 14A, 14B, 14C and upcoming Fig.'s 15 and 16 have substantial value even when not combined with the use of a smart-ticket such as 2a or even a health regiment such as 2d-1, 2d-2. For example, the present teachings allow for a novel way that a person 1 uses a personal computing device such as their computer running a program or a website to order a personalized health test kit, take personal health measurements, to have these health measurements securely provided to them as encrypted results, or to include such non health related such as financial transaction. As such, the present invention should not be limited to requiring any specific apparatus or method steps as it is possible to use differing combinations of the apparatus and steps taught herein to accomplish new and useful benefits as will be clear from a careful consideration of the present application.

Still referring to Fig. 14C, and now focused on anonymized personalization 90, step 90 is best contrasted to current practices in which a person 1 simply goes online to a website and orders an at-home test kit, where each available kit itself has no "personalization" with respect to the person 1 and can essentially be used by any person (although the test kits do often include unique ID number). In this traditional method, after taking the at-home health samples, the person 1 then ships the sampling device (such as swab 80 or similar) containing the health samples to a testing service, where this shipment must then include "personalization" since after testing the results of the test must then be communicated back to the person. This personalization step may include logging into a website and registering personal information in association with the health test kit's unique ID number. In any case, using a traditional method personalization must include providing what is referred to as "personal identifiable information" (PII) including a "personal identifier" (PID), such as the person's name and address, where the health measurements are then returned for example by mail which is less private and essentially "non-encryptable." The present invention teaches a method capable of providing health data result in a fully anonymous manner (where no PID data is shared with the testing lab / facility), or in a partially anonymous manner where only an electronic address (such as a URL or email) is shared and where the health test results are encrypted in such a way that only the person 1 using smart-ticket 2a can decrypt the test results.

Additional advantages of step 90 for including personalization information prior to the test kit being sent to the person 1 are that: 1) it is then possible to embed personalization information on apparatus within the test kit such as the health sampling device 80, where using this embedded personalization information it is then possible for the person to verify that the received health test kit is intended for their health measurements of the anticipated type of measurement (such as a virus check), 2) it is possible to make a unique association of an individual health kit to the personalization information, thus making an association that can be verified by the testing service once the health kit is returned, where the present teachings show that it is possible to verify that the health data samples are of a given specific person 1, 3) it is possible to ensure that the personalized test kit is only used by the person who ordered / purchased the kit and eliminates the transferring of the kit to some other person, and 4) it is possible to customize the kit in some way to the pre-known or communicated needs of the person 1, where the needs can related to other health conditions such as allergies or disabilities.

Anonymized personalization step 90 begins with person 1 preferably using their smart-ticket device 2a to review a health regiment 2d-1, 2d-2 and in that review to select a health test that is sufficient for compliance with a requirement of the regiment. Preferably, health regiments 2d-1, 2d-2 are provided such that any given health measurement indicates the one or more means by which that health measurement can be acquired. For example, the present invention has already taught several health measurement devices 3-1, 3-2 for determining a health measurement. As test kits are typically provided by more than one testing service, it is possible that a given health regiment 2d-1, 2d-2 specifies any one or more test kits (and therefore testing services) that are acceptable for taking the biometric samples for testing, where the specifications of the test kit preferably include Health Kit Identification Data such as a "universal product code" (UPC) encoded using a barcode that is unique to that test kit, or even the combination of a unique ID for the Health Kit Manufacturer along with that Manufacturer's unique product code, all as will be well understood by those familiar with "universal product codes," barcodes and commerce.

After person 1 has selected a health kit (for example to test for COVID-19), they then use the smart-ticket app 2a to generate an authentication token 90-1, where the authentication token is preferably an encrypted code representing either the unique identifier of the smart-ticket app 2a, of the person 1, or of a particular smart-ticket 2. As a careful reading of the present invention and the prior related art of THEME PARK GAMIFICATION, GUEST TRACKING AND ACCESS CONTROL SYSTEM will show, in one configuration the smart-ticket includes a mobile device such as a smartphone running an app, where the combination is referred to as 2a. A person may then purchase or otherwise request "access rights" to a premises, where these rights are provided to the person 1 in either or both the form of a traditional paper ticket 2b or an electronic ticket 2c, where both have physical form (see especially Fig. 1 of the present application and the PRIOR ART). It was also taught that the mobile device and app 2a can contain electronic information sufficient for representing the "access rights" without then requiring the separate physical forms of 2b or 2c, and as such all variations including 2a and 2b, 2a and 2c, 2a and 2b and 2c, or simply 2a are usable as the smart-ticket.

It was also shown that when combined with 2a, physical forms 2b and 2c have some uses that are independent of 2a, such as for gaining verified access to the premises after 2a is used to confirm such verification.

For the purposes of describing step 90-1, the "unique smart-ticket app ID" relates to a unique authentication code provided with the app that was preferably downloaded onto a mobile device so as in combination to form 2a. The device and app 2a may be used over time, or at any given time, to acquire rights to access any number of premises, such as theme parks, airplanes, cruise ships, sporting events, etc., where each premises issues their own "unique ticket number / ID" and the combination of 2a with different representations of the unique ticket number / ID (such as 2b and 2c) forms a "smart-ticket 2." (Again, it has been shown that device 2a can be used solely as the smart-ticket 2 under certain circumstances, where in those cases the "access rights" / "unique ticket number / ID" are digital and held within the device and app 2a.) And finally, the device and app 2a can register one or more persons 1 for use with the app 2a (such as a family), where each person 1 can then be registered to one or more access rights / tickets. (It is noted that the referenced PRIOR ART taught that registration minimally includes one or more biometrics such as a facial image, fingerprint or retinal scan, but that the registration does not need to include a person 1's name and address or other traditional PID information. The PRIOR ART even taught that the person 1 could be given a unique ID or name as an alias. In this regard, the inclusion of a person 1 ID should be understood to preferably be an alias ID unique to the individual but otherwise not associated to the person's other PID information such as their name and address.)

As a careful consideration will show, each uniquely identifiable app (2a) may register one or more uniquely identifiable persons (1), where each uniquely identifiable person (1) may then be associated with any zero or more uniquely identifiable "rights of access" / tickets (2). As those familiar with encoding will understand, it is then also possible to create a unique ID (x) as a combination of any of (2a), (1) and (2), where any of (2a), (1), (2) or (x) provide some means for personalizing the health test kit. In step 90-1, the provided authentication token preferably is an encryption of any one of, or any combination of (2a), (1), (2) or (x), or some equivalent as will be obvious to those skilled in the art of information systems that use authentication. As prior mentioned, it is also desirable in step 90-1 to provide a unique ID representing the health test (e.g. MFG and Test Type) and then also possibly any pre-known health information about the person (such as allergies or pre-existing conditions) that can be used to help adapt or select a particular "most-suited" / "best-fit" kit, or even to information aid in the final sample testing procedures or interpretation. This additional information can either be included in the authentication token, or with the authentication token. As the careful reader will see, providing any of this personalization information as the authentication token being transmitted to the health kit provider allows the personalization information (as the authentication token) to be electronically stored on the sample device 80's electronically readable / writable passive tag 80-nfc prior to shipment to the person, thus personalizing the health test kit.

Still referring to Fig. 14c and step 90, in a step 90-2 the person receives the health test kit including a personalized encryption token. In a step 90-3, the person uses their smart-ticket such as 2a including an electronic information reader /writer using a technology such as NFC to scan the electronic tag 80-nfc included with the sample device 80. If the authentication token stored on the smart-ticket 2a does not match the authentication token stored on the tag 80-nfc, then the person 1 is informed that the health test kit should not be used and the app 2a preferably will not continue on to step 91 ("take health sample using sampling device").

Assuming that the authentication token comparison in step 9-3 yields a match, the person 1 then proceeds to take one or more biometric health samples, where the particulars of the health samples are not important for the present teachings. What is important to see is that each sample is authenticated in step 92. Step 92 begins with step 92-1 in which the person uses smart-ticket 2a to capture an image 1-img of themselves for comparison to images pre-known to the smart-ticket 2a from a registration step. In this step 92-1, it is preferable that the person 1 is being imaged without also holding up the health sampling device such as 80, where image 1-img is then processed more easily for comparison to the prior-registered information. As prior mentioned and as will be further discussed, it is possible to use multiple forms of identification including for example one or more facial images and a fingerprint, where each additional form of identification match increases the verification of person 1, all as will be understood by those familiar with biometric identification techniques.

Still referring to Fig. 14C, once person 1 has been verified, in step 92-2 person 1 maintains their visibility to the camera being used by device and app 2a while then presenting sample device (such as swab 80) to be in simultaneous viewed along with their face (as depicted in relation to Fig. 14B).

As a careful consideration of Fig. 14B will show, when the sampling device 80 is properly presented, image 1-img will be useable to both confirm that the identified person is still being imaged and that the markings 80-bcd are sufficiently visible for decoding using image analysis, all as will be well understood to those familiar with image analysis and especially bar code image analysis. It is preferable that an indication of the preferably alphanumeric string of the decoded markings 80-bcd is also provided to device and app 2a through tag 80-nfc. For example, along with the authentication code, preferably a bar code "number" that should be decodable by analyzing markings 80-bcd is stored on the tag 80-nfc, where the device and app 2a reads this provided bar code number during step 90-3 and uses it during step 92-2 to compare to the determined bar code number that is decoded using any of well-known image processing of image 1-img. Thus, in step 90-2, person 1 is confirmed as matching the prior identified facial image of the registered person and the health sampling device (such as swab 80) is confirmed as detected (and therefore also having a determined bar code value determined using 80-bcd the matches a provided bar code value).

As those familiar with facial recognition will realize, it is possible to combine the image processing of steps 92-1 and 92-2 into a single step, or what essentially seems to be a single step for the person 1. In some variations, the image 1-img, even with the presence of the health sampling device 80 (as depicted in Fig. 14B) is sufficient for both confirming the identity of person 1 and the identity of device 80. In yet other variations, both the device 80 and the person 1 are imaged simultaneously, and the user interface of device and app 2a directs the person to move the device 80 from a first position in a first image 1-img to a second position in a second image 1-img. Thus, what is important to understand is the present invention teaches the confirmation of both person 1 and the sampling device 80, where the person 1 matches prior registration images or biometrics and the sampling device 80 matches either of pre-known information or information provided by reading tag 80-nfc.

Still referring to Fig. 14C, after the person 1 and sampling device 80 are confirmed as valid, preferably a single health sample is taken in step 93-1 after which if an additional sample needs to be taken the person 1 returns to step 90-2 to first reconfirm both the sampling device 80 ID (i.e. 80-bcd) and the person 1's ID. It is also necessary that person 1 maintains substantially continuous visibility with the camera being used by device and app 2a (such as an integrated front or back camera or a non-integrated wired or wirelessly accessed camera) during at least steps 92 and 93 (and preferably also step 94), so that image analysis of continuing images being captured during at least steps 92 and 93 can be used to confirm that person 1 has not left the view of the device and app 2a, all as will be well understood by those familiar with image processing and especially object and people tracking. In relation to upcoming Fig. 16, another type of health test kit for taking a blood sample is shown, where in this example a device 2a stand is provided for holding the smart-ticket 2a on a table, thus allowing the person 1 to conduct the taking of health samples during steps 92 and 93 without needing to also hold the device 2a, where both approaches can work and be sufficient for the teachings provided herein. As will be obvious by a careful consideration, it is also possible that a second person is assisting person 1, and that this second person holds and operates the device and app 2a, and where for example the screen of the device and app 2a is facing second person while the device and app 2a uses the back facing camera to substantially continuously image the person 1, sampling device 80 and preferably also the device container 82 during steps 92, 93 and 94.

As those familiar with a virus detection kit will understand, a typical kit that includes a swab 80 instructs the person 1 to take 4 samples, starting with the tonsils (both right and left being 2 samples) and then proceeding to the nostrils (both right and left being 2 samples). In this exemplary case, the person 1 alternates through steps 92 and 93 verifying the identity of the person 1 and device 80 for each successive sample taken (i.e., each 1 of 4), all while substantially maintain visibility with the camera being used by device and app 2a. As those familiar with such tests will understand, the entire 4 samples can be taken without a substantial amount of movement of the person 1's head. However, as prior stated, it may be easier for the person 1 to place the device and app 2a in a stand or get the assistance of a second person while performing a succession of steps 92 and 93 to gather multiple samples, any solution of which is considered to fall within the scope of the present teachings.

Still referring to Fig. 14C, after all samples are taken, in a step 94-1 person 1 may break the top 80-top of swab 80 by applying pressure on either side of break point 80-bp (assuming that the swab includes a breakpoint or similar). After braking the swab 80 (if necessary) to individuate the top 80-top, person 1 then inserts the top 80-top into the device container 82, all while substantially maintaining visibility with the camera being used by device and app 2a. In step 94-2, person 1 then places cap 82-cap onto device container 82 still maintaining substantially visibility, where the construction of container 82 and cap 82-cap is any of well-known constructions for ensuring that once cap 82-cap is affixed to container 82, cap 82-cap is substantially no longer removable. One well known non-removable construction includes at least using a lip such as 82-lip on the opening end of the device container (see Fig. 14B) such that the lip then catches and holds the cap 82-cap in accordance with the internal structure of cap 83-cap and after 82-cap is placed over and pushed down upon the container 82.

What is important to see is that the health samples are inserted into the device container 82 and sealed shut while still be substantially visible by the device and app 2a, such that the device and app 2a are able to use image processing of successive images being capture throughout steps 92, 93 and 94 to ensure that at least person 1 and health sampling device (such as 80) remain visible during at least steps 92 and 93, and that at least sampling device (such as 80) and device container 82 remain visible during step 94. As a careful consideration will show, the combination of steps 90, 92 and 93 then serve to assure that the health sample taken is of the person 1 and successfully placed an sealed into device container 82, where again by using a properly constructed container 82 and cap 82-cap, the sealed device container 82 can no longer be opened without obvious signs of damage to the container 82 thus disqualifying the health samples that were taken. In step 94-3, it is preferably to then use device and app 2a to reread the tag 82-nfc while it is being held in the sealed device container 82 as a final confirmation.

After sealing container 82 and preferably confirming the device ID of the contained sampling device (such as 80), in step 95-1 the device container is placed into a transport container (such as a pre-labeled shipping box provided along with the health kit). In step 95-2, smart-ticket device and app 2a are then preferably used to scan the transport container ID (such as a well-known 2D barcode included with the shipping label) for determining the unique transport container to be matched with the device container 82 that is containing the preferably unique sampling device (such as 80). After scanning, device and app 2a then preferably sends an electronic notification to the health service that will be receiving the transport container to alert them that the health samples are "in-transit."

In a step 95-3, the transport container is then shipped in accordance with the labeling on the transport container. Upon receiving and testing the health samples to determine one or more health measurements, in step 96 the one or more health measurements are then either: 1) transmitted to the device and app 2a as an encrypted electronic communication, 2) stored as encrypted information in a database of health sample results, or 3) mailed as printed information to the address of person 1, where the printed information can also be encrypted such as by using a 2D barcode that must be interpreted in combination with the authentication token. The encrypted information preferably includes both the one or more health measurements and the authentication token, such that when the device and app 2a receives the encrypted information, it first can check to determine that the authentication token received with the encrypted health measurements matches the pre-known authentication token. If the received and pre-known authentication tokens do not match, it is preferred that the device and app 2a both 1) does not read the encrypted health measurements, and 2) is otherwise unable to decrypt either the health measurements or the received encrypted authentication token, all as will be well understood by those familiar with encryption technology. Assuming that the received and pre-known authentication tokens do match, then the device and app 2a proceeds, and is able to, decode the encrypted health measurements for storage as person 1 health measurements in compliance with a regiment 2d-1, 2d-2.

And finally, still referring to Fig. 14C and with respect to step 96, if the health service stores the encrypted information (health measurements) in a database (method 2) as opposed to (method 1) transmits the encrypted (health measurements) as an electronic communication, or (method 3) mails the encrypted information, than person 1 preferably uses device and app 2a to gain preferably password access to the database where upon the device and app 2a are able to read or otherwise request to receive the encrypted health measurements. As a careful consideration will show, in the second case (secure database 2) where the health measurements are not electronically or otherwise communicated to the person 1, it is then possible to further ensure the anonymity of person 1 since their electronic "address" such as an email or mailing address is not shared with the health service. Thus, the health service has determined and encrypted health measurements, stored them in association with an encrypted authentication token, but otherwise has no information whereby either the encrypted health measurements or the encrypted authentication token can be associated with person 1 - thus maintaining anonymity. Only person 1 may retrieve the encrypted measurements, and only after providing to the database services being used by the health services to store the encrypted data a valid authentication code that matches the stored authentication code associated with the health measurements, all as will be well understood by those familiar with databases, remote services and encryption.

As those familiar with health test kits will appreciate, there are several types of biometric samples which are possible to obtain of a person 1 for processing into health measurements. At least some of the biometrics include saliva or mucus samples as described for sampling device swab 80, while other sampling devices may sample saliva only, or the blood (see Fig. 15A). It is also well-known that at least some sample saliva test kits use a type of saliva-reactive paper, such that after placing a saliva sample on the sample device, the sampling device may change into different visually interpretable colors or color patterns, whereupon device and app 2a could then be used to image the saliva-reactive sampling device to immediately determine at least one health measurement. Especially after consideration of the combined teachings with respect to Fig.'s 14A, 14B, 14C and 15, it will be clear to the careful reader that the novel teachings herein for enabling the process flow described in relation to Fig. 14C may be applied to other types of health sampling devices. Thus, the present invention is not meant to be limited to the depictions of Fig.'s 14A, 14B, 14C, 15A and 15B, but rather is meant to exemplify apparatus and methods for providing private, personalized, authenticated, anonymized health data sample collections. The careful reader and those familiar with health sampling kits will also recognize that several variations of the teachings provided herein are possible without departing from the scope and spirit of the present teachings.

Referring next to Fig. 15A, there is shown a pictorial representation of key components of an alternative "at-home" or self-serve health test kit to be used by a person 1 for gathering biometric samples. At-home test kits are well known and for example are currently being used to take blood samples for determining various health measurements and are often referred to as "finger-prick blood tests." Traditional kits typically include a lancet 85 for pricking a finger and some form of a "sample strip" for collecting blood droplets in a sufficient quantity. The present invention uses lancet 85 in its traditional form but further adapts the sample strip to be identifiable sample strip 84 comprising sample collection area 84-smp, visual markings 84-bcd (such as a 2D barcode) and electronically readable / writable passive tag 80-nfc. With respect to the teachings of Fig. 14A, 14B and 14C regarding the saliva and mucus at-home test kit, markings 84-bcd function like markings 80-bcd while electronic tag 84-nfc functions like tag 80-nfc. Also depicted is smart-ticket device and app 2a that is shown placed in a device stand 2a-std such that the camera being used by device 2a (e.g., its front-facing camera) can substantially maintain continuous viewing of the person 1 providing the blood sample throughout the entire process (see Fig. 14C). (As prior discussed, it is also possible that a second person could be holder and or operating the device 2a to maintain continuous viewing of person 1, all as will be obvious though careful consideration and any solution of which that substantially maintains continuous viewing is sufficient.)

Referring now to both 15A and 15B, there is also shown fingerprint thermometer 3-1-d9 that is a variation of thermometer 3-1-d7 and 3-1-d8t. Like electrodermal-thermometer 3-1-d8t, fingerprint thermometer 3-1-d9 comprises a sleeve 3-1-d9-slv for fitting over a finger (where the sleeve can be of any construction sufficient for the purpose of at least attaching to a finger or thumb and is preferably adjustable or at least deformable to fit and grip an inserted finger or thumb or includes an adjustable member such as a Velcro strap or similar), electronics 3-1-d9-elc for communicating over wired connection 3-1-d9-c with device and app 2a and for controlling onboard electronic devices including temperature sensor 3-1-d9-tmp and LED(s) 3-1-d9-led, where it is preferably that temperature sensor 3-1-d9-tmp further includes electrodermal sensor(s) all as prior taught in relation to electrodermal-thermometer 3-1-d8t (see especially Fig. 12B). Fingerprint thermometer 3-1-d9 further comprises a fingerprint reader 3-1-d9-fpr that is preferably placed within the cavity formed by the arrangement of the described parts and as depicted, such that any inserted finger or thumb is readable substantially simultaneously with any other use for example to take a person 1's temperature. In the present example however, fingerprint thermometer 3-1-d9 is only being used to capture the person 1's fingerprint for identification as will be described in more detail forthwith.

With respect to the variations of the wired thermometer devices 3-1-d7, 3-1-d8 and 3-1-d9, it is important to see that multiple configurations are possible, some of which have been depicted or describe and some of which will be clear and obvious from a careful reading of the present specification. For example, and of such devices 3-1-d7, 3-1-d8 and 3-1-d9 can be further adapted, if they are already not so adapted, to also measure blood pressure, pulse and / or blood oxygen levels using any of well-known sensors. Any of devices 3-1-d7, 3-1-d8 and 3-1-d9 could for example omit the electronics (such as 3-1d8t-elc or 3-1d9-elc) such that the device and app 2a effectively performs the functions of the removed electronics for controlling and interacting with any of the one or more sensors included in the device (such as but not limited to a touch thermometer, an electrodermal sensor, a fingerprint reader, a pulse detector, a blood pressure detector, or an oxygen detector). Especially when the electronics are removed in favor of controlling a health measurement device 3-1 or 3-2 (such as devices 3-1-d7, 3-1-d8 and 3-1-d9), it is preferably that the device 3-1 or 3-2 includes an internal readable memory for providing at least a device type code and preferably also a unique ID. With respect to the information related to a device type code, the controlling device and app 2a can use this device type as a parameter for: (1) executing an algorithm or function call for operating the particular device 3-1 or 3-2, or (2) executing a set of method steps with respect to: (a) the functions of the device and app 2a, (b) the health measurement device 3-1 or 3-2, and (c) the person 1, where for example the steps substantially follow a process such as depicted in Fig.'s 13 or 14C, or as otherwise described or implied by the present specification, all as will be well understood by those familiar with systems and device controls.

It is preferable that each health measurement device 3-1 or 3-2 (such as devices 3-1-d7, 3-1-d8 and 3-1-d9) comprise an electronically (or visibly) readable unique ID where this unique ID is usable for also guarding against counterfeit devices and for logging to the person 1 and their health regiment data, all as will be well understood by those familiar with device authentication and information systems. As a careful consideration will show, even when some or all of the necessary controlling electronics such as 3-1d8t-elc or 3-1d9-elc are included on the health measurement device 3-1 or 3-2, it remains preferable and advantageous that the health measurement device 3-1 or 3-2 includes information indicative of a device type and unique ID for at least the prior stated purposes.

As a careful consideration will show, it is also possible to use multiple finger worn health devices such as depicted in relation to Fig.'s 12A and 12B (see 3-1-d8t and 3-1-d9v), where it is possible that either or both devices have any combination of the anticipated or other health sensors or have some or no controlling electronics. Thus, the reader will understand that the present examples include fingerprint thermometer 3-1-d9 are exemplary and should not be considered as limiting the invention, but rather as showing possible variations while including at least one of the many novel teachings as provided herein, especially for supporting authenticated health measurements or authenticated biometric health sample collection.

Referring to Fig. 15A, the preferred procedure for self-collecting a blood sample is substantially similar to the process discussed especially in relation to Fig. 14C for collecting health samples of saliva and mucus, including the steps of anonymized personalization 90, taking a health sample using a sampling device 91 further comprising authenticating each sample 92, following instructions to take the sample 93, and placing the sampling device into a sealable container 94, followed by placing the sealed container in a transport container 95 for transmission to a health testing service and then receiving back encrypted test results 96. Steps 92 and 93 are of particular interest with respect to the presently depicted blood sample test kit versus the saliva and mucus test kit depicted in Fig.'s 14A and 14B, where a careful consideration of Fig.'s 15A and 15B in relation to the teachings of Fig.'s 14A, 14B and 14C will show that the other steps (included as deemed necessary for the overall purposes of the health test kit) including steps 90, 95 and 96 remain substantially as described with the understanding that markings 84-bcd (Fig. 15A) function like markings 80-bcd (Fig. 14A) while electronic tag 84-nfc (Fig. 15A) functions like tag 80-nfc (Fig. 14A).

With respect to authentication step 92 of Fig. 14C, it is still preferable that device and app 2a images person 1 as an initial form of ID verification all as herein discussed especially with relation to Fig. 14C. What is new with respect to Fig. 15A and 15B is that preferably while capturing image 84-img, device and app 2a is substantially also communicating with fingerprint reader device 3-1-d9 to capture and validate the person 1's fingerprint. It is then further desirable that while capturing person 1's fingerprint, the device and app 2a at least in part causes fingerprint device 3-1-d9 to blink light emitting LED 3-1-d9-led for capturing in one or more images 84-img as a means for further authenticating that person 1 being captured in multiple images 84-img is currently operating device 3-1-d9 that is blinking LED(s) 3-1-d9-led detected over multiple images 84-img, all as prior discussed. One advantage of using two forms of authentication, therefore both facial recognition and fingerprint recognition, is to increase the accuracy of the authentication, where both facial recognition and fingerprint recognition are not full-proof, whereas the combination is more full-proof. As those familiar with smartphones will recognize, if the device and app 2a is using a properly equipped device such as a smartphone, device 2a may include a integrated fingerprint reader. However, as a careful consideration will reveal, it is possible for a first person 1 to be positioned so as to appear in image 84-img while a second person that is not person 1 provides a finger print on the device and app 2a's integrated fingerprint reader. Thus, by moving the fingerprint reader to a separate extended device 3-1-d9 with LED 3-1-d9-led for optional additional confirmation, the authentication of person 1 is increased.

Still referring to Fig. 15A, after person 1 completes the essentially enhanced verify personal ID step 92-1, person 1 then preferably presents identifiable sample strip 84 to be in view of the camera being used by device and app 2a, such that image 84-img simultaneously captures their image and the visual markings 84-bcd, all similar to the descriptions provided with respect to visual markings 80-bcd and the procedure for operating a self-test kit comprising swab 80 as discussed in Fig. 14C. Note that the same requirements for maintaining substantially continuous visualization of person 1 while collecting the samples are preferred, where "substantially" implies that a person's head for example may partially and presumably unintentionally exit the captured images 84-img, but not so completely leave the view of the camera being operated by device and app 2a such that the person can no longer be detected in some verifiable way, all as will be clear to those familiar with object tracking as well as facial and body tracking. As will be clear from a careful consideration of the present teachings, it is possible to use the fingerprint reader 3-1-d9 in for example the process included with swab device 80 in order to provide additional person 1 verification, and as such the depictions and / or descriptions of the taught and any alternative health test kit may be further adapted to include the fingerprint reader 3-1-d9. As will also be clear, it is not necessary that fingerprint reader 3-1-d9 comprise for example a touch temperature sensor 3-1-d9-tmp, or even electronics 3-1-d9-elc (as prior discussed since device and app 2a may perform the functions of electronics 3-1-d9-elc over wire 3-1-d9-c).

Referring still to Fig. 15A, as those familiar with at-home blood test kits will understand, once person 1 and sample strip 84 have been verified in step 92, person 1 then proceeds to prick a finger using lancet 85 as is well known in the art, after which person 1 then proceeds to squeeze their finger and otherwise cause their blood to drip from their pricked finger onto the sample strip 84 in accordance with any of the well-known strip configurations. After sufficiently saturating strip 84 with their blood, person 1 then places strip 84 into a device container (not depicted but otherwise similar in concept to 82 (see Fig. 14A) but in form and shape to strip 84). Preferably, like container 82, the container for holding blood saturated strip 84 can be sealed in such a way that it cannot be reopened with essentially destroying the container, thereby making it obvious to a health testing service that the sample was potentially contaminated or tampered with. As a thoughtful consideration will show, the descriptions of step 94 for authenticating the sample container, 95 for inserting the sample container into a transportation container that is preferably scanned and notice of transit is sent to the testing service, as well as step 96 for receiving and logging encrypted test results using device and app 2a are equally applicable to blood sample kit depicted in Fig. 15A and 15B, with all of the same benefits, variations, and considerations.

Referring next to Fig. 16, there is shown a flowchart of the preferred use of a health sample device such as swab 80 or blood test 84 with lancet 85 for collecting biometric samples at a health service provider such as a clinic or doctor's office. The flow of the preferred process is exactly similar to that described in relation to Fig. 14C and briefly described comprises the major steps of: 1) the anonymized personalization 90 of the health test, 2) the taking of personalized health sample(s) 91 using health sampling devices (for examples see Fig. 14A, 15A), where taking the samples includes 2a) authenticating each sample 92 using smart-ticket device 2a and 2b) taking each sample as instructed 93, where after 3) the sampling device is inserted into an appropriate device container such as 82, 4) the device container such as 82 is then inserted in a transport container 95 for transport to a health service test lab or similar, and 5) where preferably encrypted results of the testing are provided in electronic format to smart-ticket device 2a associated with the person 1 preferably for inclusion as health measurements in association with a regiment 2d-1, 2d-2. It is also possible that the testing lab or equivalent referenced in step's 95-2 and 95-3 is "in-house" at the same facility as the service provider and as such it is unnecessary to perform at least step 95 and possibly also step 94. As such, unlike Fig. 14C, the present figure depicts a step 93-3 that follows step 93-2 "all samples collected?", where step 93-3 "run health test on samples" is then directly executed at the service provider without a need to first insert samples into a device container and seal step 94 or then to insert the sealed device container into the transport container and ship out for testing step 95. Those skilled in the art of medical samples and testing will also recognize that it may still be beneficial to follow step 93-2 with step 94 and then to set the collected samples aside for later testing or to provide them to another portion of the service facility in a sealed device container, where step 93-3 is then essentially following step 94 (or some portion of step 94) rather than following directly from step 93-2.

Still referring to Fig. 16, the anonymized personalization step 90 is different from the at-home process described in relation to Fig. 14C in that it is assumed the a person 1 walks into a service facility that already has health test kits or at least the equivalent contents (i.e. working parts like a swab 80, sample strip 84 and lancet 85). In this case, person 1 (or their guardian) preferably uses their device with app 2a to select a health test 90-1a, where the selection of the health test is similar to the discussions related to parallel at-home step 90-1. After selecting a desired test, and like prior discussed (see Fig. 14C) in relation to step 90-1 specifically, and step 90 in general, an authentication token is generated. In step 90-3a, the person 1 (or their guardian) preferably provides a request (e.g., either wirelessly, or visually using a 2D barcode) to the service provider indicating the desired health test and an authentication code. In one example, the person 1 or their guardian uses device and app 2a to provide a visible 2D barcode 1-bcd that encodes the necessary information (for reading by health service reader 98), where the necessary information includes but not limited to any combination of (1) an indication of the desired health test (such as a UPC code all as prior discussed in relation to step 90-1), and (2) some form of an authentication code indicative of the person 1 (all as prior discussed). As prior mentioned, an advantage of the present system is that it is not necessary to share any of person 1's PID data such as a name, address, telephone number, etc., thus allowing for full anonymity. While it is still possible that the health testing service will ask for some additional identification such as a government ID, it is not necessary other than for compliance with any government laws, regulations or otherwise accepted best practices.

Using device and app 2a, it is possible for the person 1 to confirm their own biometrics such as by capturing their facial image while preferably standing substantially alone in front of a healthcare service agent. Device and app 2a then captures person 1's image and confirms that the image matches the registered person 1, where this registered person 1 is associated with the requested health test and the provided authentication token such that all test results will be encrypted and only associable with this verified person 1 and their personal health regiment data. As the careful reader will note, even if a person 1 is a for example a child or otherwise needs assistance and their parent or guardian is present to assist in the verification of their facial image by operating device and app 2a, the health service agent will be able to oversee the process visually confirming that the person 1 is being imaged by device and app 2a and then verified. As a careful consideration will also show, to increase identity verification, assuming that that the device of device and app 2a is a smartphone with an integrated fingerprint reader, it is also additionally possible or alternately possible that person 1 provides their fingerprint for personal verification. (It is noted that a careful consideration will show that unlike the requirements described in relation to Fig. 15A and 15B, where a non-integrated fingerprint reader 3-1-d9 was attached to device and app 2A so that the fingerprint could be captured substantially simultaneously in view with a facial image, since a service agent is present to oversee the process, the use of the integrated smartphone fingerprint reader is acceptable.) And finally, device and app 2a preferably displays an image 1-img of person 1 along with an indication of "verified" as an additional set of information that can be provided to the health service agent for authenticating the person 1 and proceeding onto the taking of health samples 91 (similar to the teachings in relation to Fig.'s 9A, 9B, 10B and 10C).

Still referring to Fig. 16, after completing step 90 for selecting a health test and providing an authentication token as a means of assuring anonymized personalization, the service agent preferably works with person 1 to collect one or more biometric samples, such as but not limited to blood, saliva, mucus, urine, or any other type of sample as dictated by the health test. It is noted that some of the shapes shown in relation to Fig. 16 (such as the rectangle in step 91) are shown with a thicker boarder as compared to their parallel step shape as described in relation to Fig. 14C. In all cases, the thicker border is indicating that the particular step is being performed at least with the assistance of a health care service agent, unlike the at-home testing where no agent is available for assistance (although others may be assisting as prior discussed).

In step 92, it is preferred that the taking of each health sample is authenticated. As the careful reader will note, if the person 1 has already successfully identified themselves for example in step 90-3a when the request for the health test was provided, this step may be unnecessary as the health service agent has a memory of the person 1 and thus does not need any re-confirmation. However, it is also possible that the person 1 first requests the health test in step 90-3a with a first health service agent, and then waits for some period of time, after which the person 1 is then assisted in the taking of health samples by a second health service agent. In this case, the system provides the flexibility that allows the person 1 to use device and app 2a for each situation when a health measurement is to be taken and the assisting agent is not familiar with the person 1 and thus might require authentication of identity. It is noted that this step 92-1a is shown as a thinner border rectangle because the person 1 verifies their own person ID using device and app 2a, not requiring assistance per se from the health service agent (although the agent must then be provided this confirmation of identity such as by seeing the device and app 2a displaying the person 1's image 1-img and an indication of verification, all as prior discussed).

Still referring to Fig. 16, step 93 proceeds in a similar fashion to that prior described in relation to the at-home process of Fig. 14C, except that the health service agent is available to "maintain visibility" of person 1 as opposed to the device and app 2a maintaining visibility using a camera. As those familiar with health test services will understand, it is not necessary for the agent to maintain strict visibility, as it is possible that for example person 1 is permitted to use a private bathroom when providing a urine sample, and as such the present use of visibility should be considered as matching the current (or future) health practices.

After all health samples are collected in step 93, it is possible that the samples can be taken straight into testing (step 93-3) but it is more likely that the collected samples will first be put into some device container (e.g. similar to 82) by the health agent, such as in step 94-1a. It is noted that using the authentication code, it is possible to print for example a bar code label to put onto any device container (or even a transport container as described in step 95), where the agent then affixes the bar code label to the device container (such as 82). Regardless of any follow-on step 95, where the device container is inserted into a transport container and shipped off to a testing facility, at the point where the testing is to be completed, it is then possible to receive an "anonymous" device container that simply includes a bar code with information including an indication of the tests to be run and at least some portion of the authentication token. Once received for testing, the device label can then be scanned, the tests completed resulting in one or more biometric measurements or indications, after which the results can be digitally stored in association with the authentication token and therefore essentially remain anonymous.

As discussed in relation to the prior Fig. 14C, it is then possible to either electronically communicate this information to the person 1 preferably in an encrypted form that only a valid person 1 using a valid device and app 2a could decrypt, see and save in relation to their personal health regiment, or to allow the person 1 using their device and app 2a to electronically connect with and otherwise access and retrieve the information. Thus, as the careful reader will see, the present descriptions in relation to the present Fig. 16, like the descriptions in relation to Fig. 14C, provide the same teachings for a private, personalized, authenticated, anonymized health data sample collection method, with the difference that the samples are collected with the assistance of a health service agent. (It is also noted that the health service agent could be for example a "traveling nurse" that comes to the person 1's home or shelter to assist in the proper collection of the health samples, and as such this situation while being "at-home" falls into the process of the present Fig. 16 "using a service provider" rather than Fig. 14C.)

And finally, as a careful consideration will show, any remaining steps of Fig. 16 that have not been described in more detail such as 94-3 or 95, even if accomplished with the assistance of a health service agent, are like the parallel steps shown in Fig. 14C and thus will be well understood. Like the process of Fig. 14C, the present Fig. 16 describes a preferable process that can be modified and adjusted, where steps are skipped, accomplished in a different sequence, or other different steps not specified are added, all without departing from the true spirit and teaching of the present invention. Thus, the current figure and descriptions should be considered as exemplary rather than as limiting the invention to the exact process flow.

Referring now to both Fig. 14C and 16, where Fig. 14C includes a process in which a person using a mobile device and app and a self-operated / administered health test kit interacts with a testing lab to ultimately receive personal lab results, and where 16 includes a process in which a person using a mobile device and app and a service provider-operated / administered test kit interacts with a testing lab to ultimately receive personal lab results, both processes can be modified to include an additional "data-exchange intermediary," where the intermediary ultimately provides the personal lab results to the person. While this alternative process will be described in relation to Fig. 14C and specifically a "self-operated / administered" health test kit, a careful reading will show how these alternatively teachings may also be applied to the process described in Fig. 16 that uses the "service provider-operated" health test kit.

In the alternative process with respect to Fig. 14C but still applicable to the process of Fig. 16, a person using a test kit works with a data-exchange intermediary as well as the traditional lab for analyzing the health samples. Specifically, the person using their mobile device and app chooses a lab to process their health samples as well as an intermediary to deliver their results. Preferably, each test kit produced by the lab includes a sampling device (such as a swab or blood test strip) as described herein or an alternative variation thereof, where the sampling device includes an electronically readable tag, and included with the tag is preferably: (LabID) identifying the lab that will be processing the health samples, and (LabKey) a lab public key at least unique to the lab and preferably unique to the test kit, all as will be well understood by those familiar with "public-key" cryptography also referred to as asymmetric cryptography or asymmetric encryption. Preferably also, after the person has used their mobile device and app to also select the intermediary, the intermediary uses secure transmission to electronically provide to the mobile device and app: (IntID) identifying the intermediary, and (IntKey) an intermediary public key. (The data (IntID) identifying the selected intermediary is for example any data usable for conducting an electronic communication with the intermediary.)

In a first step, the person takes one or more authenticated data samples using the sampling device as described herein or an alternative variation thereof, where the process step includes electronically transmitting an authentication code from the person's mobile device and app onto the tag included with the sampling device prior to shipping the sampling device to the lab for analysis, and where the authentication token at least includes: (TransNo) a unique transaction number preferably generated by the person's mobile device and app, (IntID), (IntKey), and (AppKey) a public key provided by the mobile device and app, where then each of data (TransNo), (IntID), (IntKey) and (AppKey) are then encrypted using (LabKey). Also in this first step, the mobile device and app uses the (IntID) to identify and transmit a preferably secure communication to the intermediary including: (DataRequest) comprising (LabID), (TransNo) and (ReturnID), where prior to transmitting the mobile device and app encrypts (DataRequest) using (IntKey). (The data (ReturnlD) identifying the mobile device and app is for example any data usable for conducting an electronically communication with the mobile device and app.)

In a second step, the lab receives and processes the person's data samples into digitally encoded results (Results). The lab uses the lab's private key ("paired" with (LabKey)) to decrypt the authentication token included on the sample device's tag. Those familiar with asymmetric encryption will understand that the result of this decryption by the lab with be the recovery of: (TransNo), (IntID), (IntKey), and (AppKey). The lab then uses (AppKey) to encrypt and the digitally encoded the lab (Results), where those familiar with asymmetric encryption will understand that the (AppKey) encrypted (Results) are then only decryptable / readable by the mobile device and app with the "paired" private key, and therefore the intermediary is not able read or understand the (AppKey) encrypted (Results). The lab then uses the (IntID) to identify and transmit a preferably secure communication to the intermediary including: (ResultsPack) compirsing (LabID), (TransNo) and [(AppKey) encrypted (Results)], where prior to transmitting the lab encrypts (ResultsPack) using (IntKey). Also in this second step, the intermediary receives the (DataRequest) from the mobile device and app and uses the intermediary's private key "paired" with (IntKey) to decrypt the (DataRequest) and then store the (LabID), (TransNo) and (ReturnlD) in preferably a secure database.

In a third step, the intermediary receives (ResultsPac) and uses the intermediary's private key "paired" with (IntKey) to decrypt the (ResultsPac) to recover: (LabID), (TransNo) and [(AppKey) encrypted (Results)]. The intermediary then checks their stored data in the secure database to confirm the valid combination of (LabID) and (TransNo). Upon confirmation, the intermediary uses the (ReturnlD) to identify and transmit a preferably secure communication to the mobile device and app including: (ReqestedData) comprising (TransNo) and [(AppKey) encrypted (Results)].

In a fourth and final step, the mobile device and app receives (ReqestedData), confirms the (TransNo) as expected from the intermediary and then uses the mobile device and app's private key "paired" with (AppKey) to decrypt the [(AppKey) encrypted (Results)] as determined by the lab. Where the decrypted (Results) may then be used in any of regiments 2d-1, 2d-2.

As those familiar with databases, internet communication, encryption and software systems will understand, there are many possible variations to the just described variation where the health kit results are "run through" and intermediary as opposed to: a) being received directly (i.e. "pushed") from the lab (thus risking deanonymization of the lab results by allowing the lab to have e.g. the mobile device and app's (ReturnlD)), or b) being "pulled" from the lab's secure database (thus requiring a polling mechanism implemented by the mobile device and app where the timing of the availability of the lab results is not known or necessarily guaranteed). What is important to see is that by using an intermediary, it is possible to ensure that the lab does not have information for identifying the mobile device and app (e.g. the "ReturnID" within the "DataRequest"), and that the intermediary is not able to decrypt and read the lab results, and furthermore has no information regarding what the lab test was testing for as well as no personal information relating to the person associated with the health samples. Using this intermediary method, the transmission of the lab results is also expected to be "automatic" from the person's perspective and to occur substantially immediately after the (digitally encoded) results (Results) are determined by the lab, as the time to electronically transmit the (Results) from the lap to the intermediary to the mobile device and app is expected to be minimal.

As will also be clear from a careful reading, the above description is taught as four "steps," where each of these steps includes obvious sub-steps, and where some of the sub-steps can be rearranged and even moved into a different step without departing from the scope and spirit of the teaching.

Referring next to Fig. 17A, there is shown mobile device with entity app 2a being used in conjunction with variation finger-worn identifying device 3-1-d10. Many variations of health measurement devices 3-1 have been described in the present invention, where several variations such as 3-1-d7, 3-1-d8 and 3-1-d9 are finger-worn devices comprising different exemplary configurations of components. As prior discussed, such a finger-worn health measurement device can be either wired or wireless, each with well-known solutions and trade-offs. The present figure is now alternatively depicting a wireless variation 3-1-d10 that minimally includes: 1) process controller means such as a micro-controller for connecting with and operating the various electronic components comprising the finger-worn device (such as 3-1-d10 or any other variant such as 3-1-d7, 3-1-d8 and 3-1-d9) and for managing data communications between the finger-worn device and the mobile device 2a (or any of device 2a's variants), where process control means are well known in the art (but not presently depicted), 2) wireless communication means such as Bluetooth or wi-fi components, all of which are well known in the art (but not presently depicted), 3) battery and power management means, all of which are well known in the art (but not presently depicted), 4) light output means 3-1-d10-led that is preferably an LED, 4) finger print reader preferably similar to 3-1-d9-fpr of device 3-1-d9 (see Fig. 15B), as well as further adaptation 5) NFC reader 3-1-d10-nfc. While no particular health measurement sensors such as a temperature sensor (see for example 3-1-d8t-tmp depicted in Fig. 12B) are being described in relation to identifying device 3-1-d10, as will be well understood from a careful reading of the present invention any combination of health measurement devices or in general features and functionality of at least prior described finger-worn devices 3-1-d7, 3-1-d8 and 3-1-d9 may be incorporated into device 3-1-d10, including altering device 3-1-d10 to be a wired rather than wireless device.

What is important to see about finger-worn identifying device 3-1-d10 is that like the prior described combination of devices 3-1-d7, 3-1-d8 and 3-1-d9 it preferably includes at least three means for creating or verifying identity: 1) an embedded and preferably encrypted form of a device ID using any of the well-known means, often included for the purposes of what is referred to as "anti-counterfeiting," 2) light output means such as an LED 3-1-d10-led that can be controllably blinked or flashed in accordance with a signal provided by the connect mobile device 2a or any of its variations, and 3) a fingerprint reader similar to that described in Fig. 15B as 3-1-d9-fpr. What is also important to see is that device 3-1-d10 also includes a remote tag sensor 3-1-d10-nfc such as an NFC reader cable of sensing and at least reading and optionally writing an electronic tag 4-tag, such as any of well-known NFC tags. While NFC technology is preferred in that it is typically found a smartphone such as would be preferably used as a mobile device 2a, this is not a requirement as other tag sensing technology can be used, some of which are currently well known in the art, other of which may be created in the future with alternative advantages.

Referring now to Fig. 17B, there is depicted on the left person 1 using finger-worn identifying device 3-1-d10 to sense the device ID of a body worn health metric patch 4-1a. There are many health metric patches currently available on the market, such as patches for monitoring glucose levels in the blood, where some patches adhere to the body using a form of an adhesive, and other patches also include some form of an implant that pricks and is otherwise penetrated into the skin. The particular type of patch, manufacturer of patch or otherwise health purpose of patch 4-1a is not material to the teachings in relation to device 3-1-d10, where it is anticipated that device 3-1-d10 can be used in combination with any existing patches or any patches that will be conceived of and sold in the future. Those familiar with patches will also understand that typical health metric patches 4-1a comprise some form of preferably wireless communication means for providing any determined health metrics to a companion data storage and typically also processing device, such as a smartphone. In several patches such as a "continuous glucose monitor" patch 4-1a (see variations 4-1a-1 and 4-1a-2 in Fig. 17C) wireless data is exchanged from the patch 4-1a to a smartphone executing a controlling app, such that a person 1 merely "swipes" there smartphone over the patch 4-1a to read the data comprised on the patch 4-1a.

As a careful consideration of current health metric patch 4-1a will show, such patches can include device ID means, and can exchange health metrics with an external data collection and processing device such as a smartphone. However, there are no current means for authenticating that a given patch 4-1a is verified to be affixed to and collecting health metrics of a specific person 1. The present invention describes a preferred device 3-1-d10 that is finger-worn and can be used to authenticate that the health metrics determined by any body-worn patch 4-1a are in fact of a person 1 registered with the smart-ticket device and app 2a, and therefore can also serve as authenticated health measurements for use with respect to a regiment 2d-1, 2d-2 as herein described. Also depicted in Fig. 17B there is shown health metric patch 4-1b, where for example some patches are created to be worn on the neck/throat as a preferred location for determining health metrics related to breathing and cough. As will be clear from a careful reading of the present invention, patches such as 4-1a and 4-1b are preferably placed on a body location such as the arm or neck that can be viewed simultaneously with the face of person 1, where it should be understood that other body locations such as the front torso also comply with this requirement.

Referring now to Fig. 17A and 17B, a process for authenticating any body-worn health metric patch such as 4-1a or 4-1b comprises:
Step 1: adapting patch 4-1a, 4-1b to include an electronically detectable tag 4-tag or otherwise device ID of which many variations are available, or otherwise adapting patch 4-1a to communicate with finger worn device 3-1-d10 to provide an already manufacturer embedded device ID, where adapting at least includes the person 1 or their proxy attaching a tag 4-tag to the patch 4-1a, 4-1b.
Step 2: adhering patch 4-1a, 4-1b to a body location that allows the face of person 1 to be imaged substantially simultaneously with the patch, where locations at least include the arm, neck and front torso.
Step 3: using the present teachings to identify person 1 at least using facial recognition and optionally also in combination with a fingerprint reader preferably (but optionally) included with the finger worn identifying device 3-1-d10.
Step 4a: directing person 1 to touch the tag reading means 3-1-d10-nfc such as an NFC reader to the patch 4-1a, 4-1b, and then causing device 3-1-d10 to sense or otherwise receive the device ID of the patch 4-1a, 4-1b, where the sensed or received device ID is communicated to the mobile device and app 2a and recorded preferably along with the date and time of the determination of the device ID.
Step 4b: substantially simultaneously with the sensing of the patch device ID in step 4a, causing light emitting component 3-1-d10-led to blink in accordance with a signal provided by mobile device and app 2a, where the blinking of 3-1-d10-led is detectable as data in images captured by device and app 2a, and where the images additionally comprise the face or some sufficiently identifying portion of person 1's body, all as prior described herein.
Step 5: determining health metrics using patch 4-1a, 4-1b, and communicating the determined health metrics to an associated device and app, where the associated app is preferably the smart-ticket app being executed on a mobile device 2a as herein described, and where the communication includes at least one metric preferably along with the date and time of the determination of the metric, or alternatively the date and time of the communication of the determined metric, where the date and time of the communication may either be determined by the patch 4-1a, 4-1b or by the communicating device and app such as 2a receiving the metric from the patch 4-1a, 4-1b, as will be understood by those familiar with "data logging."
Step 5a: where if the associated device and app is not the smart-ticket device and app 2a, or at least the associated app is not the same app as the smart-ticket 2a app (but may be running on the mobile device of 2a), communicating from the non-smart-ticket device and / or app to the smart-ticket device and app 2a any of the health metrics determined by the patch 4-1a, 4-1b along with a date and time of preferably the determination of the metric or at least the original communication of the metric from the patch 4-1a, 4-1b.
Step 6: logging any health metric determined by the patch 4-1a, 4-1b after the date and time of the detection of the device ID as a valid and authenticated health metric for use with respect to a regiment 2d-1, 2d-2.

As will be clear from a careful consideration, while these steps are listed in a given sequence, some steps may be rearranged to be done in a different order without departing from the scope and spirit of the present teachings. For example, a person 1 can apply the patch 4-1a, 4-1b (Step 2) and then affix and tag 4-tag to the patch 4-1a, 4-1b (Step 1).

Those familiar with patches 4-1a, 4-1b will understand that typically after a patch is removed from the body of person 1 it will stop functioning and otherwise cannot then also be reattached, thus ensuring no health metrics are created by the patch 4-1a, 4-1b after being removed from the body of person 1. As the careful reader will see, all health metrics determined by patch 4-1a, 4-1b after the date and time of the detection of the device ID and up until the date and time when the patch is removed can then be considered as authenticated to be of the identified person 1.

Still referring in general to Fig.'s 17A and 17B, several variations of the apparatus and process steps taught in relation to Fig.'s 17A and 17B are possible. Before describing these possible variations, it is important to note that the current state-of-the-art is able to provide at least electronically communicated health metrics from a patch 4-1a, 4-1b to a companion device and app, where the communicated metrics can be logged with a date and time. It is even possible to include a device ID of the patch 4-1a, 4-1b to any extent that this would add value for the current practices. What is necessary for complying with the teachings herein of a regiment 2d-1, 2d-2 adhered to by a registered person 1 providing authenticated health measurements, is that each measurement, or the device (such as patch 4-1a, 4-1b) capturing each measurement, is confirmed / authenticated / verified to belong to that person 1, and only that person 1. In the current state-of-the-art, patches 4-1a, 4-1b are simply assumed to be associated with a person who has self-identified themselves with the companion app, and there is no teaching for confirming that the self-identified person is in fact a person 1 registered to a "regiment."

With respect to the apparatus and steps taught in relation to Fig.'s 17A and 17B, a first variation excludes incorporating a fingerprint reader into finger-worn device 3-1-d10, or at least excludes the necessary use of any fingerprint reader built into device 3-1-d10 (or any of variants 3-1-d7, 3-1-d8 and 3-1-d9). Thus, in Step 3 described above, the person 1's facial image is recognized by device and app 2a for the purposes of confirming the person 1's identity, but a separate fingerprint is not obtained. As the careful reader will note, while the fingerprint is valuable for increasing the authentication of person 1, it is not mandatory and otherwise the remaining steps are still able to authenticate that metric received from a patch 4-1a, 4-1b are of the person 1 whose face was recognized by the device and app 2a. In this variation, a careful consideration will show that the controlled blinking of the output LED 3-1-d10-led is useful for confirming that the finger worn device 3-1-d10 being imaged along with the face of person 1 is the same device 3-1-d10 that reads / is reading the patch 4-1a, 4-1b's device ID.

While it is possible and anticipated herein to alternatively put unique markings upon the finger worn device 3-1-d10 for identifying the device 3-1-d10 as an alternative to causing device 3-1-d10 to blink through LED 3-1-d10-led, those familiar with image processing will understand that it is challenging to detect a highly intricate marking (sufficient for distinguishing 1,000's and more of devices 3-1-d10) using image processing that is also zoomed back to include the person 1's face. Furthermore, the shape and configuration of device 3-1-d10 is not ideally suited to bear any such complex markings. Those familiar with counterfeiting will also recognize that markings are more easily altered as compared to a unique blinking pattern controlled by device and app 2a. It is also possible to put unique markings on each patch 4-1a, 4-1b as opposed to using a tag 4-tag or similar electronically detectable patch ID. However, it should again be understood that this raises additional complications as the patch size gets smaller and the size of the patch also gets smaller within the image as the image is zoomed out to image the person's face. For these reasons, it is preferred that the finger-worn device 3-1-d10 uses some combination of: 1) an electronic tag reader 3-1-d10-nfc, and 2) a light output means such as LED 3-1-d10-led for controllably emitting light in response to a controlling signal provided by the device and app 2a.

It is also important to recognize that simply confirming the embedded device ID of finger-worn device 3-1-d10 and then confirming that this device 3-1-d10 is in sole communications with device and app 2a is not sufficient for authentication of patch 4-1a, 4-1b metrics as it is possible that a person 1 could be touching a patch 4-1a, 4-1b that is adhered to another person. For this reason, authentication is preferred to include imaging the finger-worn device 3-1-d10 substantially simultaneously with the person 1's face, and substantially simultaneously as the device 3-1-d10 reads the patch 4-1a, 4-1b's device ID (for example from a tag 4-tag that has been adhered "after-market" to the patch 4-1a, 4-1b by the person or included during manufacturing of the patch 4-1a, 4-1b). Those familiar with image processing will understand that by imaging the person 1, the person 1's face, the device 3-1-d10, the blinking of LED 3-1-d10-led and the patch 4-1a, 4-1b, it is possible to segment the image and confirm each of person 1, person 1's face, device 3-1-d10, blinking light 3-1-d10-led and patch 4-1a, 4-1b to be in an expected and sufficient arrangement (where for example a patch 4-1a, 4-1b being worn on a second person's arm that is inserted into the image is detectable using image processing as an insufficient arrangement).

Those familiar with image processing will also understand that it is possible that device and app 2a start by capturing an image that includes person 1, person 1's face and patch 4-1a, 4-1b (and therefore the device 3-1-d10 is not being used). In this arrangement, after confirming the person's 1's identity using facial recognition, it is possible to adjust the viewpoint of device and app 2a to become closer and closer to patch 4-1a, 4-1b even to the point where the person 1's face is no longer present in the images being captured by device and app 2a. During this sequence of images, at least starting with the person 1's face being in a image and identified with facial recognition (where the patch 4-1a, 4-1b may or may not be present in the starting image including the person 1's face), and progressing through a series of images that include at least some same portion of person 1 in each of any two successive images, until finally reaching and imaging patch 4-1a, 4-1b, it is possible to confirm that the device and app 2a were continuously imaging and confirming the same person 1 as the device and app 2a's associated cameras was repositioned to image patch 4-1a, 4-1b at a closer distance, all as will be understood by those familiar with image processing and especially feature extraction, matching and tracking. As device and app 2a are then brought closer to patch 4-1a, 4-1b, it is possible to for example: 1) read a more complex pattern using image processing such as a label with a 2D barcode that was affixed to the patch 4-1a, 4-1b by the person "after-market," 2) read a more complex pattern using image processing such as a 2D barcode affixed or otherwise included with the patch 4-1a, 4-1b by the manufacturer, or 3) sense an electronically detectable ID, for example by using the NFC reader found in a typical mobile device that might be used for device and app 2a (such as a smartphone) to read an after-market tag 4-tag that has been applied to the patch 4-1a, 4-1b, or otherwise a tag included with the patch 4-1a, 4-1b by the manufacturer or any of their agents).

Thus, in this variation it is possible for device and app 2a to register a uniquely identifiable patch without the use of a finger-worn device such as 3-1-d10, where registering includes determining the patch 4-1a, 4-1b's unique ID (e.g. by image processing a bar code or sensing a electronic tag) and that the patch is in fact adhered to the person 1's body (using image processing), where the person 1 has been identified using facial recognition. Such processing will be understood to be more useful in some situations and less useful in others, e.g., depending upon where on the body the patch 4-1a, 4-1b is typically affixed and the dexterity of the operator of the device and app 2a. Therefore, what is most important to see is that by any of several variation apparatus and methods the present invention is able to associate the patch 4-1a, 4-1b's unique ID with the unique ID of a person 1 who is complying with a regiment 2d-1, 2d-2, such that the health metrics determined at least in part by the patch 4-1a, 4-1b are authenticated to be of the person 1. It should also be understood that the device and app 2a may be: 1) used by the person 1 exclusively, where person 1 holds device and app 2a in a "selfie" style, 2) used by the person 1 exclusively, where person 1 places the device and app 2a in a secured position (such as smartphone stand 2a-std depicted in Fig. 15A), or 3) is operated by another person (as presently depicted).

The teachings with respect to Fig.'s 17A and 17B should therefore be considered as exemplary rather than as limitations, as several useful variations have been shown all capable of at least confirming the device ID of the patch 4-1a, 4-1b, confirming that the patch 4-1a, 4-1b is attached to the person 1, confirming the identity of person 1, and using the combination of this information to confirm that health metrics determined by patch 4-1a, 4-1b while attached to person 1 are "authenticated" to be "of" or "about" the person 1. The device 3-1-d10 should also be considered as exemplary and can include other sensors and components, especially but not limited to sensors and components prior described in relation to 3-1-d7, 3-1-d8 and 3-1-d9.

Referring next to Fig. 17C, there are shown three exemplary devices for use with the teachings of Fig. 17A and 17B. Patch 4-1a-1 is a "Continuous Glucose Monitor" (CGM) sold under the product name of Dexcom 6. Patch 4-1a-2 is a "Continuous Glucose Monitor" (CGM) sold under the product name of FreeStyle Libre. Patches 4-1a-1 and 4-1a-2 are examples of arm patches 4-1a, where it is understood that the exact location on the body can change according at least to the limits provided by the manufacturer. Neck patches 4-1b for detecting breathing and cough are at least described in research literature. Device 4-2 is a handheld "breath keytone monitor" sold by Biosense. It is noted that device 4-2 is handheld, as opposed to be affixed to the body of person 1.

In the present invention, the person 1 first registers a handheld device 4-2 as might be purchased I the open market by: 1) affixing a tag 4-tag to the handheld device 4-2 after the purchase, 2) sensing the unique tag 4-tag using the mobile device and app 2A, for example where the tag 4-tag is an NFC tag and the mobile device has an NFC reader, and 3) registering the device 4-2 with the mobile device and app 2A using the device's unique ID as provided by the tag 4-tag. Alternatively, the device 4-2 may come with an electronically detectable device ID, or even a visually encoded ID such as a 1D or 2D barcode. An encrypted electronically readable device ID is preferred as being more tamperproof, as compared to a visible marking that can be altered. It is preferred that any tag 4-tag applied by the manufacturer before the sale, or "after-market" by the person 1, is a permanent tag, such that once applied its is substantially not possible to remove and reuse the tag. Hence, each tag 4-tag can be applied to only one device. An encrypted, embedded electronic tag built into the device 4-2 by the manufacturer is further preferred and is a typical feature of many existing electronic devices. In this case, the mobile device and app 2a requires sufficient information and permission to communicate with the device 4-2 for obtaining this manufacturer's device ID, where if not possible an after-market solution is implemented.

After registering a handheld device 4-2 with the mobile device and app 2a, a person can then take measurements in accordance with the directions for the device 4-2 and in combination with the teachings provided herein for person-device-measurement authentication. For example, the presently depicted device 4-2 is a breath analyzer, where measurement authentication would include: 1) using the mobile device and app 2a, capture image(s) of the person 1's face for facial recognition, where person 1 is matched to a registered person using 2a, 2) hold the device 4-2 in such a way to be simultaneously viewed along with the person's 1 face by the device and app 2a, 3) using finger-worn identity device 3-1-d10, touch and scan the electronically readable ID affixed to, or embedded within device 4-2, 4) hold device 4-2 to the mouth and breath into the device 4-2's mouthpiece as directed by the manufacturer of device 4-2, 5) using image processing, detect the alignment of the breath analyzer with person 1's mouth and optionally cause additional visible blinks through LED 3-1-d10-led, 6) record the date and time of the detected use, and 7) receive from device 4-2 one or more metrics either preferably associated with a date and time assigned from device 4-2 or provided substantially real-time by device 4-2, where either approach allows device and app 2a to correlate the one or more metrics with the detected alignment and use of the breath analyzer.

As a careful reading of the present teachings will show, especially in consideration of the variations discussed in relation to authenticating health measurements provided from patches 4-1a, 4-1b, there are many variations possible with respect to device 4-2. What is important is that unlike existing devices 4-2 and marketplace uses, device 4-2 is further adapted to include a device ID if a device ID is not already provided by the manufacturer or is not accessible by mobile device and app 2a, after which the device 4-2 is 1) registered to the mobile device and app 2a for use with respect to a regiment 2d-1, 2d-2, 2) confirmed each time it is used to capture a health measurement, where confirmation includes determining the device ID while substantially simultaneously confirming the identity of a registered person 1, such as by using facial recognition, 3) detected as taking a measurement such as by using image processing to recognize the placement of the device 4-2 with respect to the person 1's body, or the activation of the device 4-2 while taking a measurement, where the time of detection is recorded by the device and app 2a, and 4) causing device 4-2 to transmit the determined health measurement to device and app 2a preferably along with an indication of the date and time of the measurement, or in a real-time manner associable with the current date and time, or otherwise allowing device and app 2a to acquire this information from device 4-2, where the acquired information is then useable as authenticated health measurements.

Referring next to Fig. 17D, there is shown a combination of the herein taught mobile device and app 2a working in conjunction with a finger-worn health metric device such as 3-1-d9 that capable of detecting at least the pulse 3-1-d9-d2 of person 1 and preferably also the fingerprint 3-1-d9-d1 of person 1, where the device and app 2a are capable of using facial recognition to identify the person 1 as a registered person and to confirm the identity of the finger-worn device such as 3-1-d9 using either or both an encrypted embedded device ID and a detectable LED visual confirmation pattern emitted by the device such as 3-1-d9 in response to a control signal provided by the device and app 2a. Thus, the combination of the device and app 2a along with a sufficiently configured health metric device such as 3-1-d9 is able to: 1) perform "inter-identification" by uniquely identifying a person 1 using at least one body spatial-pattern (e.g. the face or finger spatial feature patterns), confirming that person 1 is a registered person associated with the device and app 2a and therefore optionally one or more regiments 2d-1, 2d-2, 2) and performing "intra-identification" by determining at least one body temporal pattern (e.g. the heart temporal feature patterns).

As the careful reader will note, other devices taught herein are also capable, either individually or in some combination of likewise determining both inter-identification and intra-identification, either in a configuration explicitly described herein, or in a configuration anticipated and made obvious by the teachings herein, and as such the present exemplary combination of 2a and 3-1-d9 depicted in the present figure should be considered as exemplary, rather than as limiting. What is important to see is that some one or more devices as taught herein, such as the combination of 2a and 3-1-d9, is/are able to determine two types of identification of person 1, both inter-identification and intra-identification, where inter-identification is a substantially constant and measurable body spatial-pattern such as a face, a fingerprint, retina, etc. that is usable to differentiate the person 1 from other persons, and where intra-identification is substantially a body-temporal pattern of information such pulse (heartbeat) patterns, electrodermal (skin electrical response) activity, electrocardiogram (EKG, ECG) popularly referred to as "heart waves," electroencephalogram (EEG) popularly referred to as "brain waves," or any detectable vibratory signature of the body that is useable to confirm through temporal-correlation two distinct sets of measured biometrics as belonging to the same person 1.

Referring next to Fig. 17E, there is shown a chest band 4-3 health measurement device 3-1 comprising chest band 4-3-s1 for holding one or more sensor mounts such as 4-3-s1 and 4-3-s2 to the torso of person 1, where band 4-3-s1 preferably includes a band-lock 4-3-lck. Just as there are patches such as 4-1a, 4-1b (see Fig. 17B) known in marketplace and research, there are also many types of "bands" substantially capable of holding one or more biometric sensors to some portion of the body such as the torso, head, neck, face, arm, wrist, leg, etc. Information is typically off-loaded from the one or more biometric sensors using either a wire or wireless approach, were wireless communication is preferred for the chest band 4-3 herein described. There are several useful sensors than can be held by the chest band 4-3 for determining person 1 biometrics. For example, a technology referred to as MEM microphones that are typically found in for example a smartphone, can be used to detect audio signals emanating from the chest cavity, where it is well known that heart activity patterns can be detected in the audio frequencies between 20Hz - 200Hz, lung activity patterns can be detected in the audio frequencies between 25Hz - 1,500Hz, and human cough and voice patterns can be detected in the audio frequencies between 50Hz - 25kHz.

Those familiar with state-of-the-art digital microphones will recognize that several manufactures such as TDK Corporation, Cirrus Logic, Infineon and STMicroelectronics currently sell miniaturized digital microphones either individually or in a combination of two or more covering the desired frequency range from 20Hz to 25kHz. The present invention anticipates using one or more such digital microphones, for example one in senor mount 4-3-s1 and another in sensor mount 4-3-s2, where it is further anticipated that additional sensor mounts (not depicted) could be added for use with chest band 4-3. It is not the purpose of the present invention to teach the algorithms necessary for converting signals received by the digital microphones into useful biometrics relating to any one or more of heart data, lung data and voice/cough data, as such algorithms are well known in the art. For example, several manufactures such as Eko sell what are generally referred to in the art as "digital stethoscopes," although Eko's design and form factors are meant to match a traditional stethoscope as would be used by a physician. The interested reader is also directed to the research literature on the topic, such as "Technologies for Developing Ambulatory Cough Monitoring Devices" by Justice Amoh and Kofi Odame or "Classification of Voluntary Cough Sound and Airflow Patterns Pulmonary Function" by Abaza, A.A., Day, J.B., Reynolds, J.S. et al. In the present teaching, the chest band 4-3 detects audio patterns over preferably the spectrum of 20Hz - 25kHz, filters this spectrum into the three ranges of 20Hz - 200Hz for determining heart data, 25Hz - 1,500Hz for determining lung data, and 50Hz - 25kHz for determining voice/cough data, where these filtered data sets are then processing into heart, lung and at least cough patterns, respectively.

In one configuration, sensor mounts such as 4-3-s1 and 4-3-s2 comprise computing and communication means sufficient for wirelessly transmitting any of the digital microphone "raw" or processed data, where raw data is substantially the data provided by the microphone(s), and processed data is for example the raw data that has been further filtered into a distinct frequency range and then possibly also processed into meta-data, or heart, lung or cough pattern data, using any of well-known algorithms and techniques, including both deterministic algorithms and non-deterministic machine learning. Senor mounts 4-3-s1 or 4-3-s2 can also be in wired communication, where the wire runs through the chest band 4-3, and where the communication allows for a configuration where one mount such as 4-3-s1 is the "master" and the other mount(s) such as 4-3-s2 is the "slave." In this type of master-slave relationship, the master mount includes more electronics such as a microprocessor for preferably 1) controlling and interfacing with the digital microphones (or other types of sensors to be discussed shortly), 2) controlling communications with the mobile device and app 2a, such as through the use of Bluetooth or wi-fi electronics either built into the microprocess or comprised in a separate "chip," and 3) controlling power management for driving the functioning of the chest band 4-3. As those familiar with device electronics will understand, many possible arrangements and configurations of electronics are sufficient for meeting the scope and spirit of the present teachings, such that the current teachings with respect to Fig.'s 17D and 17E should be considered as exemplary, rather than as limitations of the invention.

Still referring to Fig.'s 17D and 17E, data captured by any sensors present in chest band 4-3 are communicated either in "real-time," or stored for subsequent transmission to mobile device and app 2a, where device and app 2a may then further process the received data into for example heart, lung and/or cough pattern data. In the case where subsequent transmission is desirable, the sensor group 4-3-s1, 4-3-s2 that is at least the master group preferably comprises memory storage and possibly also data compression electronics, all as will be well understood by those skilled in the art of electronic devices. The present invention prefers real-time transmission of filtered and compressed raw data, where this data is also optionally encrypted, and where the real-time transmissions are received by the companion electronics such as mobile device and app 2a for conversion into any one of, or any combination of heart, lung and cough pattern data.

What is important to see is that the data being transmitted by chest band 4-3 is essentially "of any person" and still needs to be authenticated as being "of the registered person" 1. In a preferred exemplary approach, where chest band 4-3 is first fitted to person 1 and clasped shut using lock 4-3-lck, it is either automatically engaged for processing (such as in response the closing of lock 4-3-lck), or controllably engaged for processing (such as in response to the combination of the closing of lock 4-3-lck and either or both of an initiation signal transmitted by the companion electronics such as mobile device and app 2a or by an human interface such as a button available on for example sensor group 4-3-s1 that is pressed by person 1). Substantially at this time of engagement, person 1 is using mobile device and app 2a to establish communications with both chest band 4-3 and a finger-worn device such as 3-1-d9 that is capable of detecting an intra-identification pattern such as the heartbeat of person 1 and for being used in person 1 identity confirmation, for example through any of the means discussed herein including but not limited to facial recognition using device and mobile app 2a in combination with blinking using LED 3-1-d9-led, preferably but not necessarily with additional identity confirmation using fingerprint 3-1-d9-d1 determined by a fingerprint reader component of 3-1-d9.

During the time of engagement, mobile device and app 2a are essentially performing inter-identity confirmation by checking one or more spatial-body patterns such as the face and / or fingerprint, where the blinking LED 3-1-d9-led is used as a means for confirming that the finger-worn device such as 3-1-d9 is collecting data respective of the inter-identified person 1. Mobile device and app 2a then preferably is also in communications with chest band 4-3 for example through a master sensor group 4-3-s1, where band 4-3 includes sensors such as digital microphones sufficient for determining intra-identity information comparable with intra-identity information being determined substantially simultaneously by the finger-worn device 3-1-d9. For example, audible chest cavity signals detectable by at least some MEMs microphones is sufficient for determining heart beat temporal patterns 4-3-d2 of the wearer that are then comparable to the heartbeat patterns 3-1-d9-d2 of the registered person 1 detected for example by a pulse-oximeter component included in device 3-1-d9. Mobile device and app 2a compare the two heartbeat patterns 4-3-d2 and 3-1-d9-d2 provided by the chest band 4-3 and the finger-worn device 3-1-9, respectively, where a sufficiently matched set of patterns then serves as intra-identification confirming that the wearer 1 of chest band 4-3 is also the registered person 1 confirmed by mobile device and app 2a. In this manner, intra-identification data is used to essentially extend the total set of biometrics being determined with respect to person 1, where the firstly inter-identified person 1 is using one or more health measurement devices 3-1 that are not capable of inter-identification but are capable of providing at least one data set sufficient for intra-identification, therefore when properly matched, intra-identification data is useable as a "proxy" for inter-identification data.

Still referring to Fig.'s 17D and 17E, as the careful reader will note, there are many options for verifying that chest band 4-3 is collecting information regarding a registered person and as such the described exemplary technique of using a temporal heartbeat data pattern 4-3-d2 should be considered as exemplary, rather than as a limitation of the present invention. For example, as prior discussed for other wearable health measurement devices 3-1, it is possible to include a light output device such as an LED. In this alternatively, chest band 4-3 would then include an LED for example in master sensor group 4-3-s1 that can be controllably activated by a signal provided by mobile device and app 2a. As prior discussed, using mobile app and device 2a, the person 1's face and at least the master sensor group 4-3-s1's LED can be simultaneously imaged, where the blinking of the LED is captured in combination with the person 1's face, and using image processing at least the master sensor group 4-3-s1 via the LED is determined to be located (for example) on the person 1's chest (or some other body location such as the head, arm, wrist or even leg). Many mobile devices such as smartphones now include what are known as "IR sensors," which are typically cameras that detect at least the near infrared, all as will be well understood by those familiar with mobile device cameras. It is also well known that infrared is both non-visible and can penetrate many of the fabrics used in traditional clothing, such that it is also possible that the chest band 4-3 be equipped with infrared LEDs so that the person 1 wearing the chest band 4-3 maybe wearing a shirt or top during the authentication process that includes LED blinking as described herein.

Also, as prior described in relation to Fig.'s 17A, 17B and 17C, it is also possible to use a finger-worn device such as (3-1-d10) including an electronic tag reader (see 3-1-d10-nfc) to read an electronically readable ID from an electronic tag (see 4-tag) preferably embedded in for example the master group sensor 4-3-s1. In yet another variation, it is possible to use the mobile device and app 2a to perform what is referred to in the art as "remote photoplethysmography" (rPPG) (see upcoming Fig. 17G), a technique that allows for example at least heartbeat pulse information to be determined by processing subtle color variations detected in the face of person 1, all as will be understood by those skilled in the art of photoplethysmography. Using rPPG, mobile device and app 2a is then able to image the face of person 1 for determining both inter-identification (i.e., facial feature spatial pattern recognition) and intra-identification (i.e. heart beat temporal patterns recognition). Using the intra-identification heart beat patterns determined by rPPG (similar to patterns 3-1-d9-d2), the mobile device and app 2a can then compare these rPPG patterns to heartbeat patterns 4-3-d2 determined by the chest band 4-3, where sufficiently matching heartbeat patterns then serve to authenticate that the chest band 4-3 is being worn by the person 1 determined by the mobile device and app 2a to be a registered person 1, and as such any biometric data, such as heart, lung and cough data determined by the chest band 4-3 can be included as person 1 health measurement data for use with respect to a regiment 2d-1, 2d-2. As the careful reader will also see, when clasp 4-3-lck is unfastened so as to remove the chest band 4-3 from the wearer 1 that was determined to be a registered person 1, any and all data subsequently determined (but not necessarily subsequently transmitted to mobile device and app 2a), is no longer "authenticated."

And finally, the careful reader will also understand that it is possible to configure chest band 4-3 to include a fingerprint reader for example as a part of master group 4-3-s1, where to authenticate the chest band 4-3 person 1: 1) fastens chest band 4-3 around their torso and clasps lock 4-3-lck, 2) touches their finger to the finger print reader preferably included in master group 4-3-s1, 3) uses mobile device and app 2a to detect information from chest band 4-3 indicating that the device 4-3 is locked and reading a fingerprint, where the chest band 4-3 preferably communicates the determined fingerprint to the mobile device and app 2a for authentication as the registered person 1 such that any health measurements being determined by health band 4-3 are considered as authenticated up until health band lock 4-3-Ick is unlocked. As will be clear from a careful consideration, while configuring chest band 4-3 to including a fingerprint reader increases the cost and complexity of the chest band 4-3, it offers a simplification for authenticating the health measurements of the chest band 4-3 by at least not requiring the use of finger worn device 3-1-d9 or even the use of rPPG by mobile device and app 2a to determine heart beat pattern 2a-d2 for intra-identification comparison with chest band 4-3 determined heart beat pattern 4-3-d2.

Referring to Fig. 17E, there are many biometric sensors that can be adapted for use in a sensor group such as 4-3-s1 or 4-3-s2, where tradeoffs typically include form factor including shape and size as well as power requirements, both considerations of which tend to be continually improved by the marketplace such that sensors that might currently be considered to "bulky" or power consuming for use in a chest band 4-3, could well in the future be sufficiently sized and/or power efficient. Furthermore, battery technology continues to advance, thus making the use of either or both more individual sensors or more power consuming sensors (or electronics) possible. In this regard, it is anticipated that the chest band 4-3 can include more processing power such that the master group sensor can convert more of the sensor data into a higher "meta" form that either or both requires less memory storage (especially if temporarily saved as data on the chest band 4-3) or requires less transmission bandwidth, all as will be well understood by those familiar with the art of electronic devices.

Other sensors especially of interest for use in chest band 4-3 are MEM (digital) accelerometers (sold by Signalquest, STMicroelectronics, Analog Devices, TE, etc.) and gyroscopes such as the L3G4200D 3-axis MEMs gyroscope should by STMicroelectronics in chip form or sold integrated into a in a packaged unit by Diligent (see the Pmod Gryro). MEM ("micro-elctro-mechanics) Accelerometers are useful for detecting vibrations at different frequencies, where for example vibrations sensed from essentially contact with the body can transmit lower frequency information that is generally not detectable as an audio signal, thus providing useful additional biometric data. Many existing smartwatches uses accelerometers to aid in the measurement for example of footsteps taken or sleep patterns (where more movement is interpreted as "lighter sleeping" to "not asleep"). Accelerometers are both small in form factor and power requirements, as well as low in cost, and as such the preferred chest band 4-3 includes one or more accelerometers which are anticipated to be useful both in cough detection and in sleep pattern determination, especially in combination with one or more digital microphones.

Unlike an accelerometer which cannot sense rotation, a digital MEM gyroscope uses angular momentum to indicate orientation. Using at least one gyroscope in chest band 4-3 provides means for collecting both vibrational information (like an accelerometer) as well body/torso orientation including a determination if person 1 is likely sitting or standing upright verses laying down. When combined with time of day information the orientation of the torso of person 1 can provide useful information. For example, a decrease in energy is often associated with an infectious disease, where the infected person may be resting (lying down) at a pattern that is increased from their normal baseline or the baselines of people with similar sex, age, weight, etc. characteristics. Authenticated torso orientation, when then also combined with authenticated heart rate, breathing, cough patterns, temperature, blood oxygenation levels, body weight and related metrics (see Fig. 17F) provides significantly useful data for tracking symptoms related to infectious diseases.

In addition to accelerometers and gyroscopes, other sensors for use in chest band 4-3 include what are referred to as "ECG electrodes,' or at least what are commonly referred to as "1-lead ECG" systems comprising a single electrode are optionally used in chest band 4-3 for recording electrocardiogram data. ECG technology is well known and useful for providing more detailed and accurate heart health information.

Besides one or more MEM microphones for detecting heart, lung and cough patterns, the preferred chest-band 4-3 comprises at least one MEM gyroscope for determining torso orientation as well as chest vibrations, or alternatively includes at least one accelerometer for determining body vibrations or preferably what is known as a 3-axis accelerometer that also provides data useful for determining body torso orientation. The preferred chest band 4-3 further comprises a blood oxygenation sensor for detecting blood oxygen levels and a contact temperature sensor for detecting body temperature such that the preferred chest band is able to authenticate body temperature, blood oxygen levels, heart beat patterns, breathing patterns (such as shortness of breath), cough patterns, sleep patterns and abnormalities (using torso vibration / movement and torso orientation especially in combination with time-of-day and recorded baseline patterns), where authentication at least includes the determination that the wearable is "clasped" or "locked" to person 1 and then confirmed via any of the inter-identification or intra-identification apparatus and methods described herein.

Based upon a careful reading of the present invention, those familiar with health devices will understand that there are many possible and useful variations with respect to the authenticatable body wearables defined and anticipated herein, and especially with respect to the chest band 4-3. Thus, the present teachings especially with respect to the chest band 4-3, should be considered as exemplary rather than as limiting of the present invention. What is important to see is that the present invention teaches apparatus and methods for creating a chest band 4-3 that ultimately provides authenticated health measurements that can be used in compliance with a health regiment 2d-1, 2d-2 for determining an on-going health state of the authenticated person 1.

As will be clear from a thoughtful consideration of the teachings with respect to the chest band 4-3, one or multiple sensor groups 4-3 may be used with chest band 4-3, where band 4-3 may be constructed to receive / release these multiple sensor groups as attachable / detachable, where attaching may include connecting to a centralized wired communications link running along the chest band 4-3, or where any of the attached sensor groups communicate with each other and / or the mobile device and app 2a via wireless means such as Bluetooth or wi-fi. As will also be clear to those familiar with device electronics, battery and power management preferably included with chest band 4-3 can optionally employ remote charging (also referred to as inductive, wireless or cordless) versus connected (or plug-in) charging.

And finally, with respect to both Fig. 17D and 17E, it is advantageous that mobile device and app 2a be within audible range of person 1 for example during sleep time when cough activity often increases, such that ideally both chest band 4-3 detects person 1 cough and voice patterns 4-3-d3 as does mobile device and app 2a, where these two separately collected datasets may then be compared or combined for increasing signal detection and analysis, preferably by mobile device and app 2a, all as will be well understood by those skilled in the art of audio signal processing. As prior mentioned, vibrational data as collected by using one or more accelerometers in chest band 4-3 will also provide useful information especially for determining cough patterns when multiple people are both within the audible range of the mobile device and app 2a's microphones and within the audible range of the chest band 4-3 microphones (although noise cancellation can be used with the chest band 4-3 microphones to help ensure that the audio data collected by the chest band is substantially limited to the wearer, rather than for example a bed partner). Those familiar with lower frequency vibrational data versus higher frequency audio data will recognize that there will be a correlation between chest vibration and audible cough with respect to a person 1 wearing a chest band 4-3, whereas a bed partner coughing might be detected audibly by either the mobile device and app 2a or even to some lesser extent by microphones included in chest band 4-3, whereas the bed partner's chest vibrations will not be substantially detectable by either 2a listening to both person 1 and the bed partner, or 4-3 being worn by person 1. Thus, it is clear that vibrational data is usable to help correlate audible data with a particular person 1 wearing a properly equipped chest band 4-3.

Referring next to Fig.'s 17E and 17F, there is shown scale 4-4 for determining body weight and optionally related data such as "body mass index" (BMI), body fat, body type, visceral fat, body water, muscle mass, bone mass, "base metabolic rate" (BMR) and metabolic age. Such scales 4-4 are often referred to in the art as "smart scales," or "body composition scales," where these smart scales typically include a wireless communication means such as Bluetooth for working with a companion app. One such smart scale manufacturer is Arboleaf. A careful consideration of these existing scales will show that the collected and determined biometric data is assumed (without inter-identification) to be with regard to some person using the companion app. What is preferable is to adapt smart scales such that their collected and determined biometric data can be authenticated (using intra-identification) as belonging to a person 1 registered (using inter-identification) to the mobile device and app 2a, and preferably registered in association with one or more regiments 2d-1, 2-d2, such that the biometric data is usable as regiment authenticated health measurements.

As described in relation to the authentication of chest band 4-3 data (see Fig. 17E), what is needed for authenticating a smart scale is intra-identification data such as but not limited to a heartbeat pattern 4-4-d2 that can be compared and confirmed to match an authenticated pattern 3-1-d9-d2 (or for example a similar pattern determined by device and app 2a using rPPG as prior discussed). One means for capturing pattern 4-4-d2 is to further adapt the smart scale to include one or more pulse detection sensors 4-4-pls such as a pulse oximeter. Some smart scales such as the "RENPHO premium smart body scale" are already tracking the weighed person 1's heart rate, and as such are already presumed capable of providing heart pattern data 4-4-d2 to mobile device and app 2a, along with preferably other scale determined biometrics, such that mobile device and app 2a can thereby use the intra-identification pattern 4-4-d2 as a means of authenticating that the weighed person 1 currently using the smart scale is the registered person 1, thus allowing the smart scale determined biometrics to be included as authenticated health measurements preferably with respect to a regiment 2d-1, 2d-2.

As those familiar with health metric tracking will understand, there are many valuable uses for gathering authenticated health measurements as herein taught, especially when combined with a regiment 2d-1, 2d-2. As will also be understood, such regiments and authenticated measurements applicable to a registered person 1, can at least provide value as: 1) a means for pass/fail determination with respect to gaining access to a premises or otherwise a gathering of people, 2) a means for verifying compliance with respect to an insurance policy or medically determined treatment, 3) a means for conducting medical / medication trials, 4) a means for tracking personal health goals. As will also be understood, the present invention teaches low cost, easy to use apparatus and methods for authenticating at least the health measurements of body temperature, blood oxygen levels, and cough patterns, where these three metrics are known to be highly indicative of many infectious diseases including COVID 19. There is also research indicating that body weight, specifically being under weight, normal weight, or overweight can effect a person's natural response to an infectious diseases including COVID 19, and as such at least these four metrics are considered as valuable to track by the medical community. The present invention allows these and other metrics such as blood glucose levels, chemicals determinable via breath analyzers, etc. to be authenticated and anonymously tracked. As will be subsequently discussed herein, this authenticated data can be anonymized and aggregated across a population to provide a valuable dataset for using both deterministic and non-deterministic algorithms to track and control the spread of infectious diseases within the population.

Referring next to Fig.17G, there is shown a mobile device and app 2a using a technology known in the art as "rPPG" or remote photoplethysmogram (also referred to as "imaging PPG" (iPPG or PPGi) or "non-contact PPG" (ncPPG)). Photoplethysmogram (PPG) is the technology used for a conventional pulse oximeter (see 3-1-d2, Fig. 4B) that is typically worn on the finger. Conventional PPG uses and uses LEDs to emit light (typically centered at 660 nm (red) and 940 nm (infrared)), where absorption of light at these frequencies varies significantly between blood loaded with oxygen and blood lacking oxygen. Using this information, a contact pulse oximeter such as 3-1-d2 can at least determine heartbeat patterns and blood oxygenation levels. Research is on-going, but at least indicates that using a conventional camera as found it a typical mobile device 2a, it is possible to at least detect the heart beat patterns of a person 1 by analyzing a series of images of the face of person 1. rPPG is capable of detecting subtle momentary changes in the subject (i.e. person 1's) skin color, where the changes in color are at least indicative of the heartbeat pattern 2a-d2. While rPPG has different signal-to-noise ratios across different skin tones, it is general considered accurate enough to determine pulse (heartbeat). Other research and commercially available technology (such as "VitalSigns Camera - Technology" available for licensing from Phillips) also indicates that rPPG is able to detect the person 1's temporal pattern of breathing, or respiration pattern. Still other companies such as Binha.Al claim that rPPG can be used to determine what is known in the art has "heart rate variability" (HRV) pattern the represents the variation in the time interval between heartbeats. Each of these temporal patterns can be used as intra-identification in accordance with the teachings provided herein.

Referring to the combination of Fig.'s 17G and 17E, after clasping a chest band 4-3 around the torso of person 1, chest band 4-3 equipped with any of digital microphones or contact pulse oximeters is at least able to distinguish heart beat patterns by analyzing either microphone data or pulse oximeter data, all as is well known in the art. Rather than using the combination of mobile device and app 2a along with a finger-worn device 3-1-d9 (that includes a pulse oximeter) to generate the combination of inter-identification (e.g. face recognition via 2a and fingerprint recognition via 3-1-d9) as well as intra-identification (e.g. 3-3-d9-d2 heartbeat patterns via 3-1-d9), it is possible using rPPG that mobile device and app 2a can determine both inter-identification spatial patterns (e.g. the face feature patterns used in facial recognition) and intra-identification temporal patterns (e.g. the small color variations detectable in the face in accordance with the heart beat). Hence, mobile device and app 2a furthered configured to preform rPPG on person 1 is then able to communicate with chest band 4-3 and to at least determine that chest band 4-3 is "clasped / locked" and to authenticate that chest band 4-3 is currently being worn by person 1 through at least the comparison and sufficient matching of heart beat patterns 2a-d2 and 4-3-d2, respectively.

Referring to the combination of Fig.'s 17G and 17F, after person 1 stands on a smart digital scale 4-4 it is possible for the scale to determine at least intra-identification patterns of the heartbeat 4-4-d2. Hence, mobile device and app 2a furthered configured to preform rPPG on person 1 is then able to communicate with smart scale 4-4 and to at least determine that scale 4-4 is currently operating to take body measurements and to authenticate that scale 4-4 is currently measuring person 1 through at least the comparison and sufficient matching of heart beat patterns 2a-d2 and 4-4-d3, respectively.

As a careful reading of the present invention will also show, it is possible to authenticate other wearables such as a finger worn device (3-1-d7, 3-1-d8 or 3-1-d9) if the finger worn device is equipped with a sensor for at least determining the heart beat pattern of the wearer (person 1) that is also being currently imaged by the mobile device and app 2a, which is configured to use rPPG for determining at least the heart beat pattern 2a-d2. However, a consideration of the cost of electronic components will also show that the additional cost of adding a pulse oximeter to the finger worn device(s) is more than the cost of a sufficient LED. Both solutions are possible and taught within the present invention as viable alternatives, where for example by adding the blood pulse oximeter to the finger worn device(s) it is then also possible to then determine the blood oxygen level of person 1 as well as to authenticate person 1 using the matching of a temporal "intra-identification" pattern such as the heartbeat pattern, although the cost, size and complexity of the resulting device are expected to increase.

Referring to the combination of Fig.'s 17G and 6A, 6B, 6C, and by implication of Fig.'s 6A, 6B, 6C the authentication of wrist worn wearable devices such as 3-1-d4, 3-1-d5 and 2-3, it is possible to authenticate wearables such as a wristband or "smart watch" or Fitbit / health monitoring band if the wearable is equipped with a sensor for at least determining the heart beat pattern of the wearer (person 1), where person 1 is also currently being imaged by the mobile device and app 2a, which is configured to use rPPG for determining at least the heart beat pattern 2a-d2. Many current smart watches (such as the Apple Watch) and wrist worn health monitoring bands (such as Fitbit) do already include pulse oximeters or the equivalent that are capable of determining at least pulse rate and typically also blood oxygenation, where the data for determining the pulse rate is usable for determining the heart beat pattern for comparison with 2a-d2, and where the sufficiently matching heart beat patterns as detected by device 2a using rPPG and the wrist worn wearable are sufficient for authenticating that the measurements being taken by the wearable are of person 1.

Referring next to Fig. 18, there is shown a block diagram of mobile device and app 2a comprising preferred datasets 2a-db, some data of which is preferably transmitted to a remote database 102-db. As those familiar with database schemas and architectures will understand, there are many possible arrangements for the data presented in Fig. 18, as well as many variations either or both excluding some of the data discussed with relation to Fig. 18 or including additional data either expressly discussed or implied by the present invention, or obvious to those skilled in the art of database design, or obvious to those skilled in other arts related to the present invention, such as ticketing systems, destination reservation and tracking systems, healthcare systems, contact tracing systems, government clearance systems, etc. Therefore, the present teaching relating to Fig. 18 should be considered as exemplary, and designed to point out key information and processing, rather than as limitations of the present invention.

Mobile (smart-ticket) device and app 2a preferably includes well known data encryption such that datasets 2a-db remain essentially private to each registered person 1, of which multiple registered persons 1 might be using the same mobile device and app 2a. Alternatively, for example with a family, at least datasets 2a-db are encrypted and private to the mobile device and app 2a, although this is not a requirement and under some circumstances the data may be accessible by design to at least some authority for the purposes agreed upon by the authority and the person(s) 1. As shown in registration data 2a-db-1, each person 1 registered to use the mobile device and app 2a is represented by preferably a unique ID and related inter-identification biometrics (such as facial data, fingerprint data, retinal data, etc.), where again inter-identification biometrics are useful for uniquely identifying the person 1 from amongst other possible persons that are not 1. Other information about person 1 is also desirable to know and would often be stored with registration data 2a-db-1, such as a person 1's name, nickname, image (for viewing), as well as any other useful data as the present application dictates. In both the present application as well as the cross-referenced and related applications mentioned herein, and especially the prior related application U.S. Non-Provisional Application No. 16/055,078 entitled THEME PARK GAMIFICATION, GUEST TRACKING AND ACCESS CONTROL SYSTEM, the present inventors describe many details of preferred data and the uses thereof. For the sake of clarity, the present teachings related to Fig. 18 are intentionally abbreviated, and therefore again should not be seen as limiting the present invention.

Still referring to Fig. 18, each registered person 1 and therefore registered ID is ideally associated with one or more regiments identified by a regiment ID as shown in dataset 2a-db-2. It is further anticipated that a person 1 may be complying with one or more regiments, each with their own regiment ID and related data (see datasets 2a-db-3 and their associated datasets), and / or may be associated with a "study," such as a vaccine trial, for which dataset 2a-db-2 then includes a study ID, or similar. What the careful reader should recognize, is that the present invention teaches a novel means for creating highly useful authenticated health measurements regarding a person 1, and that these health measurements and their related data may be grouped into sets for any purpose, where one set is a regiment (such as 2d-1, 2d-2) and another set is a study, and still yet other groupings or sets are possible as will be appreciated by those skilled in the necessary arts. As will also be appreciated, these sets or groups would benefits by having status information, summary information, etc., as well as additional descriptive information such as a regiment name or description, where this additional descriptive information can be greatly extended as needed, for example to include a governing agency name or ID for the regiment, a pharmaceutical company name for the study, related phone numbers and contact names, etc.

For each grouping (e.g. regiment, study) of health measurements related to a registered person 1, a grouping dataset 2a-db-2 is preferably associated with a "rules" dataset 2a-db-3. The purpose of the rules dataset 2a-db-3 is to relate the given group to one or more specific types of measurements along with a list of zero or more restrictions, limits, control settings or in general measurement "details." Those familiar with rules-based systems will understand that many arrangements are possible for establishing and processing rules. What is important to see is that a sufficient rules dataset is made available in association with a group such as the regiment or study, and thus each regiment or study can have its own rules. It is further anticipated that multiple regiments or studies being concurrently adhered to by a registered person 1, can be aggregated, where for example a daily process preferably executed on mobile device and app 2a considers the rules associated with each regiment / study in dataset 2a-db-2, and creates a daily plan for the person 1, where perhaps a single health measurement is sufficient for complying with a rule from two or more regiments / studies. Measurements rules 2a-db-3 preferably also include "measurement means" such as a "direct" measurement (indicating that the person 1 will use a specific health measurement device 1 as herein described, the use of which will directly provide at least on health measurement such as temperature), a "kit" (indicating that the person 1 will collect typically a body fluid sample such as saliva, mucus, blood or urine, where this authenticated collected body fluid is provided to for example a lab that analyzes the body fluid to determine one or more health measurements such as the presence of virus antibodies), or a provider (indicating that person 1 will be visiting or otherwise interacting with for example a healthcare professional to take one or more health measurements such as a blood test, EKG/ECG, x-rays, etc. and / or will be receiving a health treatment such as a flu shoot or vaccine).

Preferably associated with each measurement type referred to in dataset 2a-db-3, there is a measurement type dataset 2a-db-3a associating additional information such as an approved device/kit type ID, as might be provided for example by the issuer of the regiment or study. It is preferred that approved devices include encrypted device ID or otherwise what are often referred to as "anti-counterfeiting" means, thus ensuring for the regiment/study/group originator that the health measurements are going to be determined by a sufficient health measurement device 3-1 or device used by a service 3-2. A careful consideration will show that current health care practices to not include a means of specifying that the body temperature should be taken "at a given body location" using "a given type of devices." In the present invention, the approved devices include a list of 1 or more possible devices, each sufficiently encrypted with identification to validate the device's credentials as conforming to a given regiment/study/grouping rule. For example, a person in a vaccine study may decide to use a touchless temperature reader whereas a thermometer or a contact / touch temperature would be preferred for accuracy, and where some thermometers or contact / touch temperatures are of lesser build quality and performance and as such it is important to be able to specify (and then confirm through interacting with the device 3-1, 3-2) what devices are considered sufficient. Those familiar with the difficulties presented to companies and agencies by the current pandemic will especially appreciate this technical advantage of the present system, as touchless temperature checks in particular are considered as unreliable at least because of the measurement type (e.g. remote IR temperature detectors are less reliable). Unlike the state-of-the-art, the present system provides for health measurement "restrictions" (e.g. that the temperature should be taken off the forehead when the person has been present within an ambient temperature between for example 72 to 80 degrees Celsius).

Still referring to Fig. 18 and measurement type dataset 2a-db-3a, the preferred information also includes purchase sources to assist in the purchase of the health measurement device 3-1 (or the location of a health service provider for using devices 3-2) along with related insurance coverage, such as could be indicated by a code. Again, the information depicted should be considered as exemplary, where the many technical advantages of the present teachings will be evident from an overall consideration of the system and also the exemplary data described in relation to the present figure. It will be obvious to those familiar with sales of medical devices and insurance providers that other information may be preferred either replacing and / or adding to the teachings herein, while remaining within the scope and spirit of the invention. Additionally, measurement types found in rules dataset 2a-db-3 are preferably associated with instructions and media 2a-db-3b including links to videos or other health related information considered to be useful, informative, or otherwise meaningful for providing to the person 1 that is complying with the regiment-rule.

The dataset 2a-db preferably also includes a registered device dataset 2a-db-4 to be preferably associated with both the registered person in dataset 2a-db-1 and one or more rule(s) in the rules dataset 2a-db-3. For strict authentication, as described herein, it is important to register a verified person 1, a verified regiment with rules, and verified health measurement devices, such that any single health measurement of a person following a regiment using a device is itself then authenticated. As with all other datasets currently being described, the registered device dataset 2a-db-4 is representative and can include additional useful information such as but not limited to one or more standard authentication methods, such as "encrypted device ID," "blinking light output," "additional biometric" (such as fingerprint), and otherwise any other method herein described to obvious that is useful for confirming a registered device.

Associated with a given regiment/study/group rule dataset 2a-db-3, there may be one or more actual measurements as recorded in a dataset 3a-db-4a. The measurement dataset 3a-db-4a preferably includes a unique ID for each measurement, the date and time the measurement was taken, the location (e.g. a GPS location, zip code, city, etc.) where the measurement was taken, the registered device ID that was used to determine the "measurement" (or otherwise provide any of the services described herein including test kits, healthcare services such as vaccines, flu shots, etc.). Measurement dataset 2a-db-4a also includes the one or more measurements taken (such as temperature, blood oxygen, pulse, cough detected, etc., vaccine administered, flu shot taken, etc., all in compliance with a regiment rule) as well as any other related measurements such as ambient temperature, ectodermal activity, etc.

Still referring to Fig. 18, the present invention also teaches the use of baselines or "normals" determined about a person 1 that are useful when determining the overall veracity of a health measurement. For example, the measurement of electrodermal activity is taught for recording a baseline of a person 1's skin resistance and / or capacitance, where these electrical properties of the skin are known to fluctuate for at least two reasons. First, a person may be in a stressed situation in which their body is essentially excreting water through the epidermis (skin), where this water content can significantly alter the skin's electrical properties. Second, a person may apply a substance onto their skin, the substance of which can significantly alter the skin's electrical properties. What is desirable is that certain measurements, such as but not limited to temperature, pulse / heart beat patterns and other stress influenced metrics are taken when the person is determined to have a baseline metric (such as skin resistance and / or capacitance, or resting heart rate) that substantially coincides with baselines prior determined with respect to person 1. In other cases, especially where a measurement is to be taken through contact with the skin, it is desirable to use an electrodermal sensor for reading any of current electrodermal activity metrics to compare with a prior baseline and / or average that is known to be representative of "normal, dry skin" without any applied substances.

As will be clear from a careful consideration of the teaching provided herein, the use of such normals serves to increase the veracity of individual health measurements and is another component of the present teachings that along with authentication of person 1's identify, serves as a technical advantage for creating highly useful data about person 1. Baseline ("normals") dataset 2a-db-5 preferably comprises a unique ID for each baseline / normal / average, etc., an association with one or more measurement types (for which the baseline/normal may be specified as a restriction in association with a rule, as will be understood by those understanding rule-based system and through a careful consideration of the teachings herein), a registered device ID that was used at least in part to establish the baseline/normal, as well as the time that baseline/normal was taken or updated, or in the case of an average value any relevant date/time information such as start time of average, end time of average or last updated. Just as with the other datasets comprising mobile device and app 2a dataset 2a-db, baseline/normal dataset 2a-db-5 may be implemented in a variety of sufficient arrangements, excluding some of the information described herein, including other information not described herein, being segregated (encapsulated) into its own dataset, being incorporated into another dataset, etc. It should be therefore understood that dataset 2a-db-5, along with all datasets within dataset 2a-db, should be considered primarily for their functionality, purpose and technical advantage, rather than their particular data representation, implementation or "schema," as those skilled in the art of databases will understand that many variations and alternatives are possible without departing from the true spirit, reason for and uses of the data specified herein, especially including dataset 2a-db.

Still referring to Fig. 18, and finally with respect to dataset 2a-db, many teachings are available and well known for creating "contact tracing" data that is useful for determining at least in part the "contacts" a given person 1 has made with one or more other persons. The reader is directed to the teachings that are readily known regarding contact tracing, especially for example with decentralized and anonymized solutions as implemented by companies including Google and Apple. What is important to see is that the present invention tracks an authenticated health state regarding the person 1, where the combination of this authenticated health state and contact tracing offers a significant technical advantage and usefulness over current practices and teachings. For example, in the teachings for contact tracing systems currently known in the art, any person A coming within an electronically determinable distance of a person B, can cause a "contact," where there is no consideration of the health state of either person's A or B. In relation to the current CV19 pandemic, if it is known that person A is following a regiment that includes a rule requiring vaccination, and the person A has complied with this rule through the authentication methods at least described herein, then it is acceptable to not log the contact with person A in person B's contact tracing database. Likewise, if person A is vaccinated and person B is either not vaccinated or is unknown with respect to a vaccination, the interaction with person B could be logged but then treated with less emphasis, of if the person B was also known to have authenticated proof of vaccination, then the contact of person B can be omitted from the contact tracing log of person A.

Using the teachings of the present system, a person A can have a maintained "proof-of-health" that is on-going, regardless of the current CV19 pandemic, since there are many other infectious diseases that are determinable or at least have symptoms that are determinable or indicative. Hence, on an on-going basis, if a person A is following a regiment and taking for example temperature, blood oxygenation and possible cough pattern measurements (such as by using the chest band 4-3 configured to measured and authenticate body temperature, blood oxygenation levels as well as heart, lung and cough pattern data), it is possible to determine when person A may be showing unhealthy symptoms, perhaps because they are coming down with "the common cold," where then this abnormal symptom state, while not diagnosed, could be used as an indication when logging any other person such as B or C's interaction with A within person B or C's respective contact tracing datasets. As will be discussed shortly in relation to dataset 102-db, this type of contact tracing in combination with authenticated health status offers novel advantages for the tracking of infectious diseases within a given population.

As a careful consideration will show, the present teachings for combining authenticated health state with contact tracing offer real advantages that serve to highlight significant drawbacks to the current practices and teachings regarding contact tracing, where one drawback includes not having a technical means for qualifying (versus quantifying) contacts between individuals. It is further well known that the existing contact tracing methods are being implemented on mobile devices such as smartphones, where the smartphones are using embedded Bluetooth technology for determining person-to-person contacts. Unfortunately, while many people may have smartphones, they will not all have downloaded or otherwise enabled the "contact tracing app," or they will not be carrying their smartphone, such this combination causes a significant net reduction in the number of person-to-person contacts being traced verses the actual number of person-to-person contacts. Hence, current methods of contact tracing suffer technical disadvantages related to both their quantification of contacts as well as qualification of contacts.

The present invention has also taught the use of low cost wearables such as 3-1-d4, 3-1-d5 and 2-3 that at least within a confined premises (such as an office complex, mode of transportation such as an airplane, bus or ship, a stadium or theme park) can be used to collect virtually 100% of all contacts between persons, where then these 100% of contacts can then also be qualified according to the teachings herein with respect to the authenticated health-pass / health status. The present invention further provided a means for not allowing symptomatic persons essentially "failing" a regiment to even enter the confined premises, thus at least ensuring that all the virtually 100% of contacts are with asymptomatic persons. Regiments were shown to include rules for example requiring a person to take a health test using a health kit, where means were shown to authenticate the body fluid samples of a person using a health kit, such that it was then possible to authenticate the results of the health kit testing. Regiments were also shown to include rules that require a person to seek a healthcare provider to receive for example a shot or vaccination. These additional verification methods, i.e. health kits testing for example for the specific state of a person with respect to a specific infectious disease, such as but not limited to CV19, and vaccinations with respect to a specific infectious disease, being a part of the authenticated health state of a person A enables a contact tracing system to qualify any contact with person A in a substantially different way from a contact with a person such as B or C for which no authenticated health state is known.

A careful consideration will show that authenticated health states in accordance with regiments 2d-1, 2d-2 provide valuable authenticated health information for qualifying person-to-person contacts in relation to a contact tracing system, where the valuable information at least includes knowing that a given person 1: 1) has symptoms or no symptoms that may or may not be indicative of a particular infectious disease, 2) has used a health kit (or otherwise taken) a body fluid test indicative of currently having, having had, or not having had an infectious disease, or 3) has had, or has not had a vaccination or otherwise treatment for an infectious disease. The reader is also directed to upcoming Fig. 20 for an "active contact tracing" mask that provides a novel means for combining the functions of a mask for protecting persons during the outbreak of an infectious disease (such as but not limited to CV19 within a population), for carrying the current authenticated health state of a person A wearing the mask such that the health state A can be communicated to any contacted person such as B for the purposes of qualifying the contact A, for likewise receiving any authenticated health state B of other persons B that have contacted A, for determining that the face mask being worn by A has an approved "filter" and for determining that the face mask is being worn properly by A so as to be effective.

As a careful consideration will show, and as will be discussed in relation to upcoming Fig. 20, the active face mask as herein taught serves as another level of qualification for use in enhancing any contact tracing system. A careful consideration will also show, in many situations the total number of people wearing a face mask can far exceed the number of people carrying a smartphone with a contact tracing app enabled (especially when considering children that often do not even have a smartphone but will be wearing a mask), and thus the present teachings of at least the authenticated wearables including an active face mask, provide not only a way of qualifying each contact for a contact tracing system, but also can greatly increase the quantification of contacts determined, thus in total providing a significant technical advantage over the prior art.

Referring to Fig. 18, contact tracing datasets preferably include a contact log 2a-db-6 and a location log 2a-db-7. Contact log 2a-db-6 includes information regarding known / detected / determined actual contacts (e.g. an encrypted identifier of the contacted person as is well known in the art), a date and time of contact (where there may be multiple contacts and the state-of-the-art records contacts on a "period" basis, where for example a period is every 5 minutes), a location where the contact took place (such as a GPS location), as well as any known authenticated health state of the contacting person (thus serving to qualify the contact). The location log 2a-db-7 preferably includes locations a person was determined to have entered, along with the date-time of entering and existing. A location could be a geographically well-defined area, such as a building, airplane, cruise ship or theme park, or a virtual geographic area, where for example just like a day is broken into distinct time segments for logging contacts, a geographic area (such as a city) can be broken into area segments representing for example an area that can be traversed on foot with "x" minutes. The present invention anticipates using both GPS and wi-fi or Bluetooth to determine well defined areas, where for example the entrance to a well define area is using a wireless local communication means such as Bluetooth or wi-fi to essentially "broadcast" a premise identifier for use in the tracking log 2a-db-7, or where the global positioning "GPS" communication means are used in reference to a mapping system to determine entrance into and exit from a well-defined area.

Still referring to Fig. 18, the present system teaches the anonymized aggregation of preferably a "rolling snapshot" of health state datum related to the multiplicity of persons within a group, such as a geographic group related to for example a zip code, or virtual group related to for example a "study" (such as a vaccine trial). Those familiar with the U.S. Governments Health Portability and Accountability Act (HIPAA) Privacy rules will understand the concept of the "de-identification" (also referred to as anonymization) of a data set related to a person, such that "protected health information" (PHI) is not shared without the consent of the person. The HIPPA guidelines state that PHI includes any information that personally identifies the person, such as what are generally known as PID (personal identifiers) or PII (personally identifiable information), including but not limited to a name, address, phone number, SSN, healthcare service bill, insurance account number, etc. HIPPA also specifies that aggregated health data that "does not identify any individual member" and "there is no reasonable basis to believe that it could be used to identify an induvial" is essentially "de-identified" or "anonymized." It is not the purpose of the present invention to teach the rules for compliance with HIPPA for the de-identification or anonymization of data, for that the reader is directed to publicly available information. What is important to see that the present teachings collect novel authenticated health measurements within dataset 2a-db that are preferably encrypted (symbolized by the bold surrounding rectangle) and remain private to the person 1 using mobile device and app 2a (or its equivalents and alternatives). The present teachings further show that at least some of this useful authenticated health measurement data can be transmitted over preferably any of well known "secure, encrypted transmission" apparatus and methods to be aggregated in a centralized database 102-db regarding a "population" or "anonymized group" of persons.

Database 102-db preferably includes a "rolling snapshot" of data about a multiplicity of people in the anonymized group, were for example a rolling snapshot includes the last two weeks of known measurements of temperature, blood-oxygen levels and cough patterns, where it is understood that virtually any health metric described herein or determinable using the means and methods taught herein is available for use at some level depending upon the implementation of de-identification. A rolling snapshot provides a current picture of an anonymous person X included in the group / population being tracked. Preferably this centralized aggregation of rolling snapshots is deleted each period of updating, for example each day. As will be clear from a careful consideration, if a rolling snapshot were to cover 14 days, and be updated on a daily basis, then each next snapshot would contain essentially 13 days of identical information, where the oldest (14^{th} day) is "rolled off," (shifting days 1 to 13 to become 2 to 14), and the current day is "rolled on" becoming day 1. While it is possible to instead simply transfer each next day along with an anonymous identifier representing the person X, where each next day of information is first matched upon receiving according to the anonymous identifier and then stored with prior days inside of database 102-db, this method is considered less anonymous because the person X has an on-going "identifier" that could be used to determine the person if for example that person X's local dataset 2a-db is "hacked," and / or the transmission from mobile device and app 2a to the remote dataset 102-db is compromised.

While this eventuality is considered highly improbable given the state-of-the-art especially in regard to what is known as "asynchronous" or "public/private" key encryption or simply "public-key cryptography," the careful reader will understand that the preferred method of uploading a rolling snapshot for example each day, for each person X, covering for example a period of 14 days, is even more secure in that each day a new rolling snapshot is transmitted from the mobile device and app 2a to the centralized database 102-db, where the snapshot can be provided with a different anonymous identifier for that person X. However, it should be understood that the exact apparatus and methods for transmission, or the frequency and composition of the transmission from each person X's private dataset 2a-db to an aggregated database 102-db has many possible implementations, each with tradeoffs, and as such the preferred teachings with respect to especially Fig. 18 should be considered as exemplary, rather than as limitations of the present invention as many useful alternatives are possible without departing from the scope and spirit of the present invention. It should also be understood that the term "dataset" is being use with respect to a single person, while "database" is being used with respect to an aggregation of persons, or an aggregation of datasets, and that these terms in and of themselves should not be considered as limitations, as many interpretations are possible whereas the present invention is in the simplest sense referring to a collection of datum for both 2a-db and 102-db.

Still referring to Fig. 18 and aggregated database 102-db, each dataset received from a person X is preferably given a unique dataset ID, where this dataset ID can be different from the anonymous ID comprised within the transmission. However, if the anonymous ID provided with the transmission is used as the dataset ID, it then serves as a check against duplicated data, as will be understood by those skilled in the art of databases. It is also preferable to record the received date-time, although this is of less value when considering a daily reset of all rolling snapshot data, and as such represents information that can be omitted without departing from the scope of the invention. Each transmitted dataset from a person X is presumed to be a subset of the total information available in person X's local dataset 2a-db, where the transmission preferably at least includes a datum ID for associating with a combination of a measurement type (such as "temperature" or "blood oxygen level" or "cough pattern severity level," etc.), a date-time of the measurement (preferably ensured to be within the rolling snapshot window of for examples the last 14 days) along with at least the authenticated measurement. Preferably, each regiment rule in dataset 2a-db-3 includes an indication (data not depicted) of which types of measurements and what information is allowed to be aggregated, and even an identifier as to what "URL" or other identifying internet address the allowed data should be transmitted to.

A careful reader familiar with data transmission will understand that the present teachings are describing a "push" type transmission, where each mobile device and app 2a preferably automatically prepares and upload their transmission each period providing it to the computing system including or acting as a gateway to the centralized database 102-db. While it is possible to also use a "pull" approach, where the computing system including or acting as a gateway to the centralized database 102-db establishes communication with each mobile device and app 2a for the purposes of requesting or "pulling out" of dataset 2a-db the desired information, this approach is considered to be less favorable for reasons that will be apparent to those familiar with shared database connectivity and to those familiar with anonymization.

Like local dataset 2a-db, centralized database 102-db is expected to comprise information not described in relation to Fig. 18, or to exclude information described in relation to Fig. 18, as necessary for the purposes of the centralizing "authority." As such, database 102-db as described herein should be considered as exemplary, rather than as a limitation of the present invention, as many alternative configurations of datum are possible without departing from the scope and spirit of the invention. It should again be understood that "measurement" is being used as a general term to refer to at least any datum complying with a health rule in a regiment 2d-1, 2d-2, where an authenticated measurement of temperature by the person X is only one example. Any authenticated datum derived from the authenticated use of a health test kit is another example. Any authenticated healthcare service received by the person X, such as a vaccination or a flu shot is yet another example. What should be clear is that a regiment may specify a broad range of health tasks to be accomplished and authenticated by a person X, from self-measuring to other measuring to obtaining medication or treatments, etc., and that any one or more rules in a regiment may indicate that some portion of authenticated information related to the rule should / can be aggregated. Those familiar with the tracking of at least infectious diseases, or the administering of vaccine trials, will understand that the taught apparatus and methods offer significant technical advantages over the sate-of-the-art, where these technical advantages themselves result in substantial benefits.

Still referring to Fig. 18, a careful consideration will show that contact tracing data comprised within personal dataset 2a-db is not being transmitted in the rolling snapshot for aggregation in centralized database 102-db. This is a preference of the invention but not a limitation, as at least some contact tracing data could be provided, for example a code indicating the number of different "well defined areas" or "virtual geographic areas" (see above) where visited by a person X each day. This type of data is considered to still be anonymous, whereas more precise locations visited can add to means for de-anonymizing the data, or in other words determining who person X might be based upon for example exact locations visited, or a pattern of locations over the rolling period. The present invention anticipates that contact tracing data could for example be transmitted to a centralized authority for use in combination with rolling snapshot database 102-db, for example when and if a person X becomes aware by whatever means (such as a health test kit of a healthcare provided diagnosis, each covered within the scope of a regiment) that they may have an infectious disease. The state-of-the-art contact tracing systems have already defined this preferred "person approved" sharing of location data for the use by a central authority to contact other persons who may have been exposed to the person X. While it is not the purpose of the present invention to described how this contact tracing data may be anonymously provided, shared or otherwise used, it should be understood as prior mentioned that the present invention teaches novel means and methods for both including health state qualifications regarding any sharing of contact tracing information, as well as novel means and methods for increasing the quantification contact tracing.

Still referring to Fig. 18, as those familiar with data analysis will understand, the aggregated anonymized rolling snapshots database 102-db of health status information related to a multiplicity of people in a population (anonymization group) offers significant opportunities for deriving critical information. The processes for analyzing this database 102-db are generally represented in the present figure as "machine learning / artificial intelligence" ML/AI process(es) 102-ml. It is not the purpose of the present invention to teach a particular machine learning, artificial intelligence or otherwise data analysis process for analyzing or deriving useful information from database 102-db, as those skilled in the art will understand that many processes are possible, each with differing usefulness and advantages. However, the present invention prefers the use of machine learning where what is known as the "algorithm" is separated from what is referred to as the "model."

In machine learning, an algorithm processes data, the processing of which results in a model unique to the data processed. The model is a data structure and mathematical formula coefficients that are then usable to make predictions on the data, or future unknown data. Hence, the algorithm generates a unique data structure and coefficients representative of a desired mathematical function for separating, classifying, or otherwise recognizing a pattern in the data being processed. A known limitation of this machine learning algorithm/model approach is that the model that is then used to create "predictions" about future unknown data is not able to be rationally explained, where rationally explained is comparable to a person that is able to provide deductive logic or reasoning as to why they denied customer X credit approval but approved customer Y. A machine learning algorithm simply denies or approves in this credit example, where it is essentially not possible to review the data structures and coefficients of the model in such a way as to recognize any particular reason or "rules." For the present invention, this algorithm/model approach inherent to ML has advantages for providing an "obscured" message transmitted back to the population of the persons contributing data to the database 102-db.

Still referring to Fig. 18, after processing database 102-db using preferably a machine learning algorithm 102-ml that creates an obscured model, if one or more anonymous persons X are determined to be possibly at risk or otherwise separated, classified or recognized to be exhibiting an undesirable pattern of health related information, it is then desirable to communicate a message to these one or more anonymous persons X. However, due to the anonymity of the data structure and processes, it is not possible to determine exactly which device and app 2a, let alone what registered user of a device and app 2a to send the message to. It is preferred that the centralized system transmits a broadcast message 102-msg substantially to all devices and app 2a that either participated in the population of database 102-db, or even also to devices and app 2a that have similar data but did not provide data used in database 102-db. As will be understood by those familiar at least with machine learning, the broadcast message 102-msg preferably includes at least 1 "trained detector" algorithm or preferably data model 102-det. For example, this trained detector 102-det that is a learned data model can be provided to any device and app 2a for execution using a known companion algorithm, such that the detector 102-det processes personal data comprised within a dataset 2a-db to determine a result, such as "this data indicates a possible problem" (i.e. akin to "denied") versus "this data indicates no problems" (i.e. akin to "approved"), without also offering any reasons that might be interpretable as a diagnosis.

Ideally, the person X whose data is considered problematic, is encouraged to seek professional medical assistance to ascertain if the person is for example, infected by a disease. In addition to this, a recommendation could be provided in the form of a rule change or update to a regiment, where the rule change or update could for example indicate additional measurement types that should be taken by or of the person X, where for example a person X may have been taking temperature and blood oxygen measurements and because these measurements are combining to classify the person X as "possibly infected" according to the trained detector 102-det, the message 102-msg carries a rule change to require that person X start also tracking their breathing during their sleep to look for indications of coughing. The rule change could also require a health kit test, or that a person does in fact need to see a professional. While it is then up to the person X to comply with the new rules and therefore updated regiment 2d-1, 2d-2, if the person X chooses not to comply then their regiment health status changes to "failing" or some similar indication.

Conversely, a broadcast message could alter rules to allow for less health measurements if the person X is determined to be most likely "not infected" (or otherwise at risk), where the message also carries information regarding which regiment rules are the "additional health measurements" only mandatory when other measurements are indicative of a possible problem. For example, a rule for taking a health kit test or having a virtual visit with a healthcare professional (often referred to as telemedicine) might be removed from a regiment for a person who is not exhibiting what is determined to be a healthy pattern of health measurements. And finally, it is also preferred to include an expiration date on each message, where for example the mobile device and app 2a will only process messages on the day they are received, or up until their expiration date.

Those familiar with database analysis, anonymization, machine learning and AI will recognize that the present teachings provide a significant technical advantage over the current state-of-the-art, and that alterations and variations are possible with respect to the present teachings of a message 102-msg with associated trained detector 102-det without departing from the scope and spirit of the invention. What is important to see is that an analysis is conducted over a population of persons using their anonymized data, where the aggregated datasets do not provide a means for communicating directly with any particular person, and that the present apparatus and methods can be used to generate a broadcast message that is sent to all persons contributing to the database 102-db, or even a larger group of persons, where the messages carry a means for processing local datasets 2a-db and based upon the outcome of the processing to provide recommendation messages and even changes to rules of a particular regiment on a particular mobile device and app 2a for a particular registered person 1. **It** is also important to see that the trained detector 102-det can itself be "anonymized" by at least using a machine learning data model that in and of itself is not reasonably interpretable when considered simply as a data model apart from a particular data set that it is used to operate upon. Therefore, the present teachings especially with relation to the broadcast messages 102-msg and trained detectors 102-det should be considered as exemplary rather than as limitations of the present invention. As with all other datasets described especially in relation to Fig. 18, some data may be omitted while other data may be added, and any collections or arrangements of data may be altered or changed as will be well understood by those familiar with databases without departing from the scope and teachings provided herein.

Regarding Fig. 18, the relational arrows and their directions as well as the dataset shapes, relative positioning, size and any other features should be considered as informative of concepts without intending to be limiting, as many different approaches to dataset charting are known, not all of which follow the same conventions as chosen herein. Some associations using relational arrows are intentionally not shown for the sake of clarity, and datasets have been simplified. However, the careful reader that is familiar with software systems and data descriptions will understand that key concepts and novel technical advantages are presented in Fig. 18 that have many sufficient symbolic representations including Fig. 18. The depictions in Fig. 18 should therefore be considered as exemplary and informative for teaching purposes rather than limiting the apparatus and methods of the present invention. Furthermore, the present teachings may be implemented in many different types of databases such that any resemblance of Fig. 18 as describing a particular database implementation, such as a relational database, are considered to be immaterial as it is well understood that a given schema in a given database type can be substantially and sufficiently represented with a different schema and / or in a different database without departing from the scope and spirit of the teachings herein.

Referring next to Fig. 19 there is shown a flowchart for an alternative apparatus and method for a smart-ticket 2 as originally taught by Aman, et. al. with respect to the cross-referenced related application entitled THEME PARK GAMIFICATION, GUEST TRACKING AND ACCESS CONTROL SYSTEM. Like the prior art, the flow chart describes apparatus for using a confirmation zone to aid in the method for assuring a premise that a person 1 desiring self-access has both a valid "right-to-access" and is the authenticated / registered (i.e., "confirmed") holder of this right. The present teachings extend the prior art by employing a data-time fence in combination with a physically restricted confirmation area. Along with the preferred steps in this alternative smart-ticket process, the present figure depicts "client-side data" to the left of the flow chart representing information determined, received or otherwise "known" to the mobile device and app 2a that is the smart-ticket 2. To the right of the flow chart symbols, the present figure depicts "server-side data" representing information determined, received, or otherwise "known" to the premise or otherwise the agent system substantially monitoring and controlling the confirmation area and self-access points 5a (see Fig. 1 and as a careful reading of the present and related 10,861,267 patent will make clear). It is important to see that unlike traditional control self-access systems, and even unlike traditional guarded access system, the anonymity of the person 1 gaining access to a premise is never compromised, meaning that no personal information is ever determined or known by the premise or agent relating to the person 1 while at the same time the person gaining access is ensured to the premise to be the authenticated owner of the "right-to-access" and the right-to-access is currently valid and not-restricted.

Still referring to Fig. 19, in step 110 a person 1 downloads a smart-ticket enabled app preferably comprising a unique app ID onto their mobile device, such that the combination mobile device and smart-ticket app form smart-ticket 2a, all as prior described. What is known on both the client (2a) and server (premise) sides after step 110 is minimally that an app ID has been issued. Preferably, the downloaded app 2a includes means for requesting the purchase of a ticket or "right-to-access" a premise and in step 111, person 1 uses mobile device and app 2a to order a ticket, where the ticket has an encoded ticket ID that is preferably encrypted such that only the person 1 with a device and app 2a comprising the app ID of the app 2a used to make the purchase can then decrypt the ticket ID (hence being able to use the issued ticket).

During this ticket purchase transaction, preferably the sale is conducted anonymously in substantially simultaneous communication step 112, where for example the transaction is processed using blockchain (or similar anonymous transaction technology such as bitcoin all as will be well understood by those skilled in the art) such that at the conclusion of steps 111 / 112, what is additionally known to both the client and server sides is a given ticket ID associated with a given app ID, but otherwise the server side has no information related to person 1.

Those familiar with apps, web services, blockchain, encrypted transmission and in general what is known as "e-commerce" will recognize that there are many alternative solutions regarding the conducting of a sale over the internet. It is not the purpose of the present invention to teach or claim any technical advantages per se relating to e-commerce, and as such the steps 111 and 112 should be considered as exemplary and representative, rather than as limitations.

For example, it is possible that a person 1 uses a separate app or even a premise website to place an order for a ticket, where during this process there is preferably a communication of the person 1's app ID as the necessary credentials for issuing an associated ticket(s) ID.

In traditional systems, it is typical to require at least some personal information for associating with an issued ticket, all as is well-known in the art. The careful reader will recognize at least one advantage of conducting the sale using the same mobile device and app 2a that maintains the preferably encrypted app ID, where the advantage is that the person 1 is never required and cannot know the app ID that is encrypted to their mobile device and app 2a, but the device and app 2a does know the app ID and can use this within encrypted communication to conduct the desired sales transaction represented in steps 111 and 112.

In step 113, person 1 uses mobile device and app 2a to register their at least ticket ID to their own unique biometric(s) preferably but not mandatorily while outside of the premise and the premise confirmation zone. Furthermore, it is also preferred that person 1 register their biometrics with the app ID as well which could be done prior to the purchase of any ticket, for example when using the mobile device and app 2a as a "health-pass" as described herein to provide a sufficient health status prior to entering what is in effect a publicly accessible premise that requires no ticket or right-to-access. It should also be clear from a careful reading of the present and related applications that multiple persons may each be registered to the same mobile device and app 2a (therefore also associated app ID), but only a single person 1 can be registered to an individual app ID - ticket ID, or even ticket ID. It is noted that while the client side now includes personal biometrics related to person 1 for associating with the app ID and ticket ID, none of this biometric or otherwise personal information is necessary to share with the server (premise), which is unlike traditional systems that typically maintain at least a personal biometric along with at ticket number (ID) / right-to-access.

There are at least two ways for determining that person 1 is outside of the premise and the premise confirmation zone when registering including the geo-fencing first discussed in relation to U.S. Non-Provisional Application No. 16/055,078, as well as the use of date-time transactions as further taught herein. Regarding date-time transactions, the person 1 who has successfully downloaded the premise app 2a and purchased a ticket with ID to enter a premise, has received a date-time associated with both their app ID and ticket ID. As will be made clear in relation to Fig. 19, the person 1 is blocked from entering the confirmation area (and therefore the premise) without carrying with them their mobile device and app 2a (in any of its form) that is usable to confirm their biometrics once within the confirmation area. **It** is further preferred the person is also blocked if the mobile device and app 2a indicates that any associated ticket **ID** has not been registered (i.e. there is not registration date-time). Alternatively, the mobile device and app 2a indicates to the self-access control point 5a (Fig. 1 and prior related art 16/055,078) that leads into the confirmation area which if any ticket ID(s) have been registered, such that self-access control point 5a then also only allows these registered tickets IDs to enter the confirmation zone, where any other ticket IDs associated with the app **ID** but not registered must then be first registered while the person is outside of the confirmation zone.

One advantage to this "register-first" restriction is that it ensures that person 1 take the time to conduct the registration process prior to arriving at the premise or at least prior to requesting/gaining access to the confirmation zone of the premise, thus minimizing the time expected to be required for a person 1 (and possible an entire family) to use a single mobile device and app 2a to register once within the confirmation zone and prior to entering the premises from the confirmation zone, all as prior discussed especially in relation to related art 16/055,078.

The careful reader will note that a given ticket may be valid for two or more days and as such should be registered only once, where the app 2a does not permit the registered person 1 with respect to a given ticket to be changed to a different person or "deleted," and thus a person must register once and then confirm themselves each time the ticket **ID** is used, while within the confirmation zone and prior to gaining self-access to a premise from within a confirmation zone. **It** should then also be noted, that a ticket that has not yet been registered (but was alternatively allowed into the confirmation zone) could then be simply registered within the confirmation zone and used to enter a premise for the first time, where the registration is then serving the same general purpose of the confirmation. Thus, the present flow chart should be considered as exemplary of a preferred embodiment and describing several new and useful technical advantages but should not be considered as limiting the present invention as several alterations are possible without departing from the scope and spirit of the present invention.

Still referring to Fig. 19, presumably at some point in time a person 1 desires to gain self-access into a premise for which the ticket **ID** was acquired, and in step 114 the person 1 presents preferably a valid and registered app **ID** and a valid and registered ticket **ID** at a self-access means 5a (see Fig. 1 in both the present application and related U.S. non-provisional 16/055,078), where "valid" means that the app ID and ticket ID are currently complying with all associated "restrictions and validation rules" as provided by the premise (or premise agent), see steps 110, 111 and 112.

Several variations (which the related art called "forms") of a smart-ticket 2 have been described herein and where first taught in relation to related art U.S. patent 10,861,267, including the mobile device and app 2a acting alone, or at least the mobile device and app 2a acting in conjunction with either or both of an electronic ticket 2c or traditional paper ticket 2b (see Fig. 1 in both the present application and related U.S. patent 10,861,267). The reader is directed to the prior teachings for more detail as the present teachings related to Fig. 19 are being purposefully limited for clarity to the exemplary use by a person of the mobile device and app 2a with a paper ticket 2b, where it is also clear that a paper ticket 2b has a cost advantage over electronic ticket 2c (as will be understood by those familiar with printed electronics).

In step 114, what is minimally required is that each person 1 attempting entry into a premise's confirmation zone through a self-serve access point 5a present a registered and valid app ID and a registered and valid ticket ID, where the ticket ID is either for example in the form of a paper ticket 2b with scannable bar code or a mobile device and app 2a that has generated a 2d bar code including a representation of a registered and valid ticket ID. Even when using a paper ticket 2b to provide a ticket ID to access point 5a, it is still necessary to use the mobile device and app 2a to provide to the access point 5a preferably encoded information indicating the app ID and that the app ID and associated ticket(s) ID(s) have been registered and are valid, where preferably the encoded information is in the form of a 2d bar code or similar (but could also be in the form of a wireless communication such as with wi-fi, Bluetooth or NFC).

It is preferred that for example when a group such as a family of four are attempting to enter a confirmation zone and have used a single mobile device and app 2a to purchase and manage all of their four tickets, that the family first present to access point 5a the encoded information as generated by the mobile device and app 2a, where the encoded information preferably indicates which ticket(s) ID(s) will be used to request entrance (and thus some associated ticket(s) ID(s) may be "unselected for use" and "self-prohibited"). As discussed, the encoded information confirms to access point 5a that these ticket(s) ID(s) (along with the app ID) "requesting entry" have been registered to a person and are currently valid according to the restrictions and validation rules associated with the app ID and ticket(s) ID(s), respectively.

In any case, even an individual entering a confirmation area through access point 5a with a single associated ticket ID preferably first presents their mobile device and app 2a's "encoded information" (such as 2d bar code), where it is then understood that with a single ticket ID being requested for entry that access point 5a is preferably configured to not require a scanning of the associated single ticket ID (where a careful consideration will show that the mobile device and app 2a suffices as evidence of both the registered and validated app ID and the registered and validated ticket ID when a single person 1 is attempting to gain access to the confirmation area and thereafter the premise).

Hence, a family of four could be sharing a single mobile device and app 2a, that could serve as the access ticket ID (using the generated 2d bar code) for one of the individuals, but then after entry that individual would still have possession of the mobile device and app 2a and thus the remaining three individuals in the family would need to use paper tickets 2b, or otherwise all four members could simply use their paper tickets 2b. As the careful reader will note, it will be necessary that the mobile device and app 2a used to register each ticket be present in the confirmation zone to perform the confirmation process (see upcoming step 116).

With respect to validation of the app ID and the ticket ID, while it is possible and preferred that the mobile device and app 2a use the "restriction and validation rules" that were provided with the app ID and ticket ID (see steps 110, 111 and 112) to perform a validation check prior to and as a requirement for accessing the confirmation area via an access point 5a, as a careful consideration will show, either instead of this "pre-entrance" 2a validation check, or in addition to this "pre-entrance" 2a validation check, access point 5a also has the "restriction and validation rules" with respect to the app ID and ticket ID and can therefore also preform the validation check, either allowing or disallowing entrance of the person 1 into the confirmation zone.'

In this manner, it is ensured that all persons entering a confirmation zone through an access point such as 5a have the necessary mobile device and app 2a associated with their ticket(s), and that then no ticket ID is allowed to enter the confirmation zone without its associated mobile device and app 2a. As each person sequentially presents their mobile device and app 2a and / or paper ticket 2b to a self-access point 5a, the then enter the confirmation zone in step 115, such that steps 114 and 115 should be considered as substantially continuous with respect to a person. As a result of step 114, the date-time that a validated registered ticket ID and the associated validated registered app ID entered the confirmation zone becomes available as data known to both the client and the server. As a result of step 115, the status of each ticket **ID** is preferably set to "live" (or equivalent, see related U.S. patent 10,861,267 for more information on "ticket status codes").

Since the server-side has a record of all issued app IDs and associated ticket IDs, along with all restriction and validation rules, entrance into the confirmation zone through an access point 5a is preferably only allowed by access point 5a if both the app IDs and ticket IDs are meeting the restrictions / validation rules. For example, if a ticket **ID** is restricted to not be allowed on a certain day that is the current day or restricted to not be allowed at a certain time that is the current time, then it is preferable that the system block access of a person with the ticket **ID** from entering the confirmation area.

Still referring to Fig. 19, upon entering the confirmation zone through an access point 5a as described, a person 1 might for some reason then desire in a step 119 to exit the confirmation zone rather than proceed into the premise in which case the person 1 is required to exit through a self-access point 5a leading from the confirmation zone to an area outside the confirmation zone that is not the premise (referred to herein as a "general area"). The person is minimally required to present a ticket **ID** either using their paper ticket 2b or using their mobile device and app 2a. However, it will also be necessary that person 1 have their mobile device and app 2a in order to complete a requirement related to a next step 120, and as such it is also desirable to require that a person cannot exit the confirmation zone to the general area without having and presenting their mobile device and app 2a to the self-access point 5a leading from the confirmation zone to the general area. It is preferred that the person 1 use their mobile device and app 2a to generate a 2d bar code associated with the ticket(s) ID(s) desiring to exit, where the 2d bar code is presented to the self-access point 5a leading off-premises.

It is also required that the ticket ID that is attempting to exit the confirmation area into the general area has at least been registered to the person 1 exiting through access point 5a into the general area with the ticket ID. Hence, if a person 1 is allowed to enter the confirmation zone in step 115 with an un-registered valid ticket ID (which is not preferred but can be accommodated) along with the mobile device and app 2a comprising the associated app ID**,** then the person 1 will be required to register the ticket ID to themselves prior to attempting to leave the confirmation zone with the (what would otherwise be an) un-registered ticket.

As will be discussed after the description of each step in Fig. 19, in one scenario, a person A with a valid "season pass" correctly registered to themselves, could enter the confirmation area with a person B that has an unregistered "day pass." The person A could then confirm their season pass giving it to person B within the confirmation zone, and then take person B's day pass, still unregistered, and use it to attempt to leave the confirmation zone into the general zone while person B proceeds into the premise using person A's confirmed season pass.

By requiring that the unregistered ticket be first registered within the confirmation zone (or preferably before even entering the confirmation zone), then if it is registered to person B, person A will not be able to confirm that the ticket belongs to them in the upcoming step 120 after leaving the confirmation area into the general area (and thus the day pass will be considered "used" by the premise). If the unregistered day pass is registered to person A (who is also registered on the season pass now being used by person B), then person A can exit the confirmation area into the general area in step 119 and confirm that the now registered day pass ticket "belongs" to them (where in the future only person A could then use the "day pass"). In both cases, the careful reader will see that by requiring that any unregistered ticket entering the confirmation area is registered before either entering the premise or existing back into the general area, it is not possible to violate the premise ticketing rules (in that sense attempting to "cheat the system"). As a result of step 119, both the client and server will have data indicating the date-time a valid ticket ID and preferably the associated valid app ID exited the confirmation zone.

Still referring to Fig. 19, in step 120 a person 1 exiting the confirmation zone into the general area is then required to use the associated mobile device and app 2a to confirm that they have the ticket ID that was just used to exit the confirmation area, and that they are the registered person associated with the ticket ID that just exited. Those familiar with software applications will understand that mobile device and app 2a can be configured to capture an image of for example a bar code visible on the exiting ticket and then processing the bar code to confirm the ticket ID. This is a critical step in the process described in Fig. 19, for if the person does not use their mobile device and app 2a to confirm that they have the exiting ticket (ID) and that they are the registered person associated with the ticket, then as will be shown, the premise preferably assumes the ticket has not left the premise and essentially debits the ticket for the day's (or event's) use.

As a result of step 120, both mobile device and app 2a (the "client-side") and the server-side will have data including the date-time the confirmation zone was exited into the general area as well as the app ID and ticket ID used during the exit. At least the mobile device and app 2a (client-side) will also have the date-time that the exiting ticket ID was then confirmed by the registered (exiting) owner of the ticket ID (i.e., in this step 120). It is further preferable but not necessary that this information is also made available to the server side (premise), where for example the mobile device and app 2a could wirelessly transmit this same information or some equivalent, or the exiting person 1 could be required to use their mobile device and app 2a to generate encoded information (such as a 2d barcode or similar) and to present this encoded information to a station for scanning made available in the general area by the premise, such that the existing person is letting the premise server-side system know that the exited ticket ID should not be debited for use as it has been properly reconfirmed while in the general area as described in step 120.

In step 116, once within the confirmation zone the person 1 is then required to use the mobile device and app 2a associated with their ticket ID to confirm their ticket ID, where the process of confirmation was taught in the related U.S. patent 10,861,267 and should be understood to include the person 1 that is registered to the ticket ID presenting one or more personal biometrics such as the face or fingerprint for comparison to the registration biometrics, where sufficiently matching biometrics "confirm" that the person 1 is the person registered to the ticket ID.

Given the understandings as presented thus far, as a careful consideration will show, even a ticket ID that is allowed to enter the confirmation zone without being registered is ensured to be: 1) not used as of yet, and therefore 2) registerable to only a single person 1. Thus, if the person 1 is allowed to enter the confirmation zone with a valid ticket ID that is not registered but the person does have the mobile device and app 2a that is associated with the valid (unregistered) ticket ID, then even if the person has not yet registered themselves with the app ID, they can still proceed to register themselves using the mobile device and app 2a with the ticket ID, where this registration then also serves in this first and only time as the ticket ID's "confirmation."

As a result of step 116, the client data will include the date-time that the person's biometrics where provided for either registering or preferably just confirming (after a prior registering outside of the confirmation zone) the associated ticket ID. It is noted that person 1 may desire after completing step 116 to exit the confirmation zone into the general area, for which prior described steps 119 and 120 must then be executed.

Still referring to Fig. 19, in step 117 person 1 presents their validated, registered / confirmed ticket **ID** to an access point 5a leading from the confirmation zone into the premise and then substantially in one continuous step proceeds to enter the premise in step 118. As a result of steps 117 and 118 both the client and server have data including the date-time the validated, registered / confirmed ticket **ID** was used to enter the premise as well as the updated ticket **ID** status being set to "active" (see especially Fig. 2 and Fig. 3 of the related U.S. patent 10,861,267 for more detail on the status codes such as "live," "confirmed," and "active"). The careful reader will understand that the app **ID** associated with the valid ticket is already known to both the client and server.

It is once again noted that a careful review of the server-side data will show that at no time is any personal information regarding the ticket holder (or mobile device and app 2a owner / user) provided to, or necessary to provide to, the premise (or otherwise operator of the premise, confirmation zone and access point 5a). Thus the present teaching provides a tool for ensuring that: 1) a ticket ID is associated with only one app ID and therefore one mobile device and app 2a / smart-ticket 2, 2) only validated, registered and confirmed ticket IDs and app IDs are allowed to enter a premise, where validation includes ensuring that any "restrictions or validation rules" prescribed by the premise and associated with the ticket IDs and / or app IDs are in compliance, 3) valid tickets entering a confirmation zone and then re-exiting back into the general area (that is not the premise) must be registered, accompanied by the registered person, and then re-confirmed after exiting into the general area, 4) no personal information regarding any person associated with the mobile device and app 2a or a ticket is ever provided to or necessary to provide to the server-side (premise or operator), and 5) the entire process can be conducted at minimum with a mobile device and app 2a and optionally a printed ticket 2b, where an electronic ticket 2c could be used but is not required, and a mobile device and app 2a could be used alone without even a printed ticket 2b if there is only one person 1 attempting to use mobile device and app 2a with a single ticket ID, and therefore also only one associated ticket ID that entered to confirmation zone in association with the mobile device and app 2a.

Based upon a careful reading of the prior descriptions with respect to Fig. 19, it will be evident that:
1) an app ID cannot be forged or counterfeited, as the counterfeiter has no reasonable means for inserting the counterfeited app ID into the server-side data "app ID issued" as accomplished in step 110, where it is also understood that the premise or app ID issuer is preferably using any of well-known encryption technology for keeping app ID's and their structure essentially unknowable for patterning by a forger.
2) a ticket ID cannot be forged or counterfeited, as the counterfeiter has no reasonable means for inserting the counterfeited ticket ID into the server-side data "ticket ID issued to app ID" as accomplished by the steps 111 and 112, where it is also understood that the premise or ticket ID issuer is preferably using any of well-known encryption technology for keeping ticket ID's and their structure essentially unknowable for patterning by a forger.
3) a person cannot enter the confirmation area without both a mobile device and app 2a for presenting a currently valid / not-currently-restricted app ID, and a means for presenting a currently valid / not-currently-restricted ticket ID, where the means at least include a paper ticket 2b, an electronic ticket 2c, smart-ticket 2 ankle wearable 16 (see Fig. 1), or wrist wearable 3-1-d4 (see Fig.'s 6A, 6b, 8), or wrist wearable 3-1-d5 (see Fig.'s 6A, 6c, 8), or wrist wearable 2-3 (see Fig's 7A, 7B, 8), or mobile device and app 2a that includes both an app **ID** and associated ticket **ID** and is able to generate a visible symbol such as a 2d barcode for presenting at least a valid ticket ID (or otherwise provide wireless communication of the same information).
4) the person can be blocked from entering the confirmation zone if either the app ID and / or ticket ID they are presenting includes a restriction that is determined to be in effect (such as a date / time restriction), where the system provides the advantage of preferably storing or otherwise allowing access to any validation and restriction rules associated with either the app ID or the ticket ID by the mobile device and app 2a, such that the person is able to determine prior to attempting entrance into the confirmation zone if the entrance is either restricted or not valid.
5) a person cannot enter the premise from the confirmation area with a ticket ID that has never been used at the premise, without having registered the ticket ID to one or more of their biometrics, such as a face and / or fingerprint.
6) a person cannot enter the premise from the confirmation area with a ticket ID that has been prior used at the premise, without having confirmed that their biometrics match the registered biometrics prior associated with the ticket ID in relation to the ticket ID's first use (2).
7) if a person enters the confirmation area (as allowed or not allowed by the premise) with a ticket ID that has not been registered, the person cannot then exit the confirmation area either into the premise or general area (not the premise) without first registering the ticket ID.
8) a person cannot exit the confirmation area into the general area without both a mobile device and app 2a for presenting a valid app ID, and a means for presenting a valid ticket ID associated with the valid app ID such as a paper ticket 2b (see (3) for the list of other means).
9) the ticket ID presented by a person exiting the confirmation area into the general area will be debited by the premise as having been used for an established period (such as a day, or event) if the person does not after existing use the mobile device and app 2a to reconfirm that they: 1) have the ticket ID in any of its forms, 2) re-confirm their biometric(s) as matching the biometrics registered to the ticket ID, thereby proving that they are the authenticated owner of the ticket ID, where it is also understood that the mobile device and app 2a can be further adapted to require that these two reconfirming steps are any one of or any combination of: a) accomplished before attempting to reenter the confirmation area with either or both of the mobile device and app 2a or ticket ID, b) accomplished within a time limit, for example X minutes.
10) after exiting the confirmation area into a general area with a ticket ID and then using their mobile device and app 2a to reconfirm their registration to the exiting ticket ID**,** the person can use their mobile device and app 2a to provide information to the premise (server side) such that the exiting ticket ID is not debited, and
11) a ticket ID can only be used by a person in control of or otherwise having access to, or being associated with a person in control of or otherwise having access to a mobile device and app 2a (or any of its smart-ticket equivalents such as the wearables 3-1-d4, 3-1-d5, 2-3) with an app ID that is associated with the ticket ID, thus even a printed ticket 2b or electronic ticket 2c cannot be lost or stolen and used by another person that is then not able to provide proof of the associated app ID.

Still in consideration of the novel apparatus and methods taught in relation to Fig. 19, and emphasizing that the premise or server-side never has any personal information relating to a person, a set of exemplary scenarios will now be discussed to demonstrate the ability of the teaching related to Fig. 19 to ensure the premise that issued tickets are not misappropriated or misused, etc.

Scenario 1: Person A has a mobile device and app 2a and attempts to enter the premises without an associated ticket.

Result: In step 114, person A will not be able to present both an app ID and an associated ticket ID. Even though it is not necessary to have a paper ticket 2b with a ticket ID to enter the confirmation area in step 115, without a ticket ID the person A must use the mobile device and app 2a to generate a bar code or scannable symbol (or otherwise communicated information) that is based upon a purchased ticket ID (see steps 111 and 112) that is associated with the app ID. Since the person has no associated ticket ID on the mobile device and app 2a, this cannot be done, and the person A will be stopped from entering the confirmation zone and premise. It is also possible that the premise require that all guests have a printed ticket 2b with ticket ID (or an electronic ticket 2c), in which case a ticket ID generated by the mobile device and app 2a is removed as an form of ticket ID presentation, also stopping person A from entering the confirmation area and premise.

Scenario 2: Person A exchanges their upgraded ticket for person B's non-upgraded / normal ticket:
For example, 1) person A has a "season pass without date restrictions" and person B has a "day pass with a blocked-out date" of the current day, 2) person A has a "fast pass" allowing them to enter different / presumably shorter queues within the premise and person B has a "normal pass" for which they must use the normal / presumably longer queues, 3) person A has a ticket allowing for "extended hours" and person B has a ticket restricted to normal hours, etc. (It is noted that in many situations the premise does not normally block the exchanging of tickets, e.g. where a person A might have a ticket to sit in a club box at a sporting event and person B might have a ticket to sit in general seating. It will also be clear from a careful reading of the present invention that the teachings herein do allow the premise a means for registering each ticket ID with differing validation rules such that it becomes possible to limit the club box ticket to a single person A where it is not sharable with a person B, providing that sufficient self-assess means 5a are also used to control ticketed access in this case to the club box).

Result: There are two cases to consider in this scenario. In case 1, both person A and B enter the confirmation area, correctly validate / register / confirm each of their tickets as enforced by the mobile device and app 2a, and then both enter the premise. In this case 1, since both tickets are validated to enter the premise at the current date and time, then the tickets could be exchanged between person A and B either while in the confirmation area or after entering the premise. (It is also noted that such tickets could also be exchanged after entering the premise in the current state-of-the-art).

Result: However, those familiar with premise operations, for example including a theme park or destination, will recognize that some upgraded features of a ticket such as access to a "fast lane" or "private showing" must be reconfirmed within the premise before using another self-access point 5a to enter a given fast lane or building/area providing the private show, where reconfirmation includes providing the registered ticket holder's sufficiently matching biometric(s). Given this understanding, if person A exchanges their upgraded ticket with person B, person B will still not be able to avail themselves of any upgraded features as they will not be able to provide reconfirming biometrics matching those of person A that are registered with the upgraded ticket.

(The reader is directed to especially see the teachings of U.S. patent 10,861,267 and the concept of a "venue" (being the entire premise) comprising "sub-venues." These teachings of a nested arrangement of access points 5a allowed for sophisticated validation and restriction rules. In the prior example, the theme park is the venue (premise) and is entered via a first access point 5a following the teachings provided herein. Once within the venue (theme park), a fast lane is a "sub-venue" and is entered via a second access point 5a following the teachings provided herein, thus requiring two confirmations from person A and prohibiting person A from allowing person B to use their upgraded ticket.)

Result: In the other case where either of the upgraded or normal tickets are not valid for the given date and time, or are currently otherwise restricted for any reason, then the person attempting to enter the confirmation zone with a non-valid or currently restricted ticket will be blocked, and only the person with the valid / not restricted ticket will be allowed to enter, where if this entering person is not person registered to the ticket ID used for entry, they will then not be able to confirm the ticket ID and are subsequently required to exit the confirmation area into the general area, find their friend, and then have their friend use the mobile device and app 2a to reconfirm that they have the ticket and that their friend's biometric(s) sufficiently match the biometrics registered to the ticket (see step 120).

Referring now in general to the present teachings of the entire application and the teachings with respect to Fig. 19, especially including "restrictions and validation rules" associated with the app **ID** (see step 110) and the "restrictions and validation rules" associated with the ticket **ID** (see steps 111-112), it will be clear that restrictions and or validation rules can include "health status" checks in any form as taught herein. For example, restrictions and validation rules can include any level of validated and authenticated health regiment 2d-1, 2d-2 compliance (such as "L1," "L2," or "L3" as described herein).

Even when a person is using an otherwise not restricted app **ID** and ticket **ID** (where "right-to-access" is essentially "unrestricted" and even not ticketed), it is possible for the premise to limit entry through a self-access point 5a to only mobile devices and apps 2a that can present a valid app ID with a sufficient "proof-of-health" / regiment 2d-1, 2d-2 compliance. It is even possible that a person is allowed to enter a premise/venue with "L1" health regiment status, and then within the premise it is only possible to enter a sub-venue with "L2" status, and within the L2-sub-venue another sub-venue with "L3" status, or any possible combinations thereof as will be clear to the careful reader and those familiar especially with destinations that include many sub-venues / sub-areas / buildings etc. Also, as prior mentioned, it is possible to use the teachings herein to determine allowable / not-allowable access based solely upon the app ID and health regiment compliance, where therefore the "ticket ID" representing for example a pre-purchased or obtained "right-to-access" to a premise is not required, as would for example be the case when entering a premise that does not otherwise require a ticket / pass, etc.

Referring now to the teachings related to Fig.'s 19 and at least Fig.'s 7A and 7B describing a wearable smart-ticket 2-3, the careful reader will see that: 1) wearable smart-ticket 2-3 includes clasp-status check means 3-1-w-lck for use in authenticating that the wearable 2-3 is being worn by a given person A, where person A has already been registered (step 113) to a downloaded app ID and purchased ticket ID (steps 110, 111, 112) using a companion mobile device and app 2a, and where 2a was used to authenticate that 2-3 (see Fig.'s 6A, 6B and 6C) is being worn by person A, 2) wearable smart-ticket 2-3 is able to receive via communication with device and app 2a at least validated and registered app ID and ticket ID (i.e. "ticket datum" 2-datum) to be used by the authenticated person A to gain self-access into a premise, 3) wearable 2-3 includes bio-metric identification means 2-pid (preferably a fingerprint reader), and wearable 2-3 can also be configured to 4) include "other sensor means" 3-1os such as an NFC reader that is useable read or provide information to another NFC reader.

Given this understanding, a person A wearing authenticated wearable 2-3 has already completed steps 110, 111, 112 and 113 and wearable 2-3 includes as ticket datum 2-datum both the person A's validated and registered app ID and validated and registered ticket ID. Person A then: 1) uses the other sensor 3-1os / NFC reader to present the validated and registered app ID and validated registered ticket ID to an access point 5a leading into the confirmation area (step 114), where the access point 5a has been configured to include an NFC reader, 2) enters the confirmation zone (step 115) with only their wearable 2-3 and not needing their companion mobile device and app 2a, 3) provides biometrics to confirm the registered ticket ID (step 116) included in the ticket datum 2-datum using the bio-metric identification means 2-pid (such as a fingerprint reader) included in the wearable 2-3, 4) uses the other sensor 3-1os / NFC reader to present the confirmed ticket ID to an access point 5a leading from the confirmation area into the premise (step 118), where the access point 5a has been configured to include an NFC reader.

A careful consideration will show that the herein described wearable 2-3 is also: a) usable in steps 119 and 120 to exit the confirmation area into the general area and then reconfirm the exiting ticket ID, b) usable to receive from companion mobile device and app 2a any regiment 2d-1, 2d-2 health status or verification, and then usable to provide any such received status (e.g. "L1," "L2," or "L3") to an access point 5a (for gaining "proof-of-health" access), c) alternatively usable as a validated "health compliant" ticket ID with respect to any restrictions and validation rules associated with the app ID or ticket ID, where the health compliance was determined by the companion device and app 2a and transmitted as ticket datum 2-datum to the wearable 2-3 for providing to an access point 5a, d) usable to determine, receive, store or otherwise maintain all necessary date-time and related data 2-tlv as described in relation to the steps of Fig. 19, and e) usable to take spot health measurements such as a touch temperature using a health senor means 3-1hs included in the wearable 2-3.

Those familiar with devices such as the wearable "Disney Magic Band" will see that the herein taught apparatus and methods with respect to wearable 2-3 and as just reviewed provide significant technical advantages beyond the state of the art, where solutions such as the Disney band require personal information to be known to the premise, do not include means for either of authentication or biometric confirmation of the ticket holder, cannot be used to provide an authenticated health status, and cannot be used to spot check a health metric such as body temperature. A careful reading of the present teachings, especially with respect to Fig. 8, will also show that the wearables such as 3-1-d4, 3-1-d5 and 2-3 include wireless communication means such as Bluetooth and wi-fi that can be used for contract tracing at least while present within a premise, where the combination of the health status information comprised within 2-3 is then further useable to qualify all contacts traced while within the premise, the combination of features and functions of which are not currently available in the state-of-the-art wearable tickets such as the Disney Magic Band.

Still referring to Fig. 19, and regarding step 112 for conducting the sale anonymously, a preferred alternative to using methods such as blockchain or bitcoin includes the use of a third-party "anonymous transaction financial intermediary". This type of anonymous and encrypted transaction includes to following sub-steps:
1) the person 1 is using their mobile device and app 2a that includes an encrypted app **ID** that is unique to the device and app 2a and preferably kept unknown to the person 1.
2) preferably person 1 has registered their biometrics with respect to the mobile device and app 2a and indicated that before any sales transaction can be conducted, the registered biometrics must be reconfirmed by the person 1.
3) person 1 initiates a sales transaction request with the premise (or its agent) using the mobile device and app 2a, where 2a provides the app **ID** as the "purchaser" (and shares no other personal information regarding person 1 with the premise).
4) the premise requests that the mobile device and app 2a reconfirm the purchaser's biometrics, after which the person 1 for example provides either or both their face and fingerprint for matching with the biometrics registered to the device and app 2a.
5) the mobile device and app 2a responds to the premise indicating that the purchaser has been authenticated (locally on the device and app 2a, where again it is understood that no biometrics are ever provided to the premise / server-side).
6) the premise's server-side then interacts with the mobile device and app 2a to allow the purchaser to search for, review and select one or more ticket(s), where the ticket(s) are preferably added to a traditional "shopping cart" and where the user interface provides no identity information as is often provided in a traditional on-line shopping experience.
7) to complete the transaction, rather than ask for personal information such as name, address, and credit card information, the premise's server-side connects with a third-party financial intermediary and provides a request to that the intermediary conduct the financial portion of the pending sale, where the server side does not (cannot) provide any personal information regarding person 1 but instead provides at least either of:
   a. the app ID and the sale amount, or
   b. a unique transaction ID, for example associated with the combination of the app ID and the current purchase transaction, along with the sale amount, where in this case the premise then also provides the unique transaction ID to the mobile device and app 2a.
8) the mobile device and app 2a then interacts with the third-part financial intermediary preferably in a continuous experience for the person 1, were the intermediary first requests that device and app 2a provide either the app ID or transaction ID associated with the purchase (see 112-7a, 112-7b, respectively).
9) upon receiving the app ID or transaction ID from device and app 2a, the intermediary then conducts a traditional sale prompting the user for information such as their name, address and credit card information, after which the intermediary contacts the appropriate banking institution to determine the availability of funds and effectively "charge the person 1" all as is well-known in the art.
10) after the intermediary successfully charges the person 1, the intermediary then provides a transaction on behalf of the person 1 to the premise, where the transaction references either the app ID or the transaction ID (as provided by the premise in step 112-7) and uses traditional methods to provide payment to the premise.
11) upon receiving payment, the premise then:
   a. generates ticket(s) ID(s) that are unique to the app ID and records the ticket(s) ID(s) in association with the app ID.
   b. encrypts the ticket(s) ID(s) such that that only the mobile device and app 2a with the matching app ID can effectively decrypt and otherwise use the ticket(s) ID(s);
   c. transmits the encrypted ticket IDs to the mobile device and app 2a. 12) the mobile device and app 2a then receives and stores the ticket(s) ID(s) in association with the app ID essentially completing the sales transaction.

As those familiar with on-line sales transactions will understand, the above stated sub-steps provide a means for conducting a sale between the premise and the person 1 while also keeping the identity of person 1 anonymous with respect to the premise. Furthermore, the third-party-intermediary also does not receive information regarding what is being purchased by person 1 and in the case where the premise provides a transaction ID (step 112-7b) rather than the app ID (step 112-7a) to the intermediary, the mobile device and app 2a's unique app ID remains private between the premise and the device and app 2a (i.e., unknown to both the person 1 and the intermediary). This "anonymous transaction financial intermediary" as described herein is for example different from e-commerce websites that collect personal information and then link to a third party financial intermediary service such as PayPal, where PayPal is then being used as a convenience for the customer pre-established with PayPal, and / or as a known secure method to take payments for websites that do not have equivalent technology.

Those familiar with on-line sales transactions will also understand that variations of steps 112-1 through 112-12 are possible all without departing from the scope and spirit of the preferred alternative sub-steps 1 - 12 provided herein. Therefore, sub-steps 1 - 12 should be considered as exemplary, rather than as limiting to the present invention, especially in consideration of additional well-known details that have been omitted for the sake of clarity.

Referring next to Fig. 20A and 20B there are shown two different perspectives of active contract tracing and health qualifying mask 130. Active mask 130 comprises three main parts: a) mask frame and electronics 130-1, b) mask with breath sensor 130-2, and c) mask filter insert with electronic tag 130-3, where each part comprises multiple components. Active mask 130 preferably provides any one of or any combination of the following "active mask" functions (AM-functions): 1) communicating with mobile device and app 2a (or equivalent), 2) contact tracing using wireless communication means, 3) contact proximity notification to wearer, 4) mask fit determination, 5) filter insert determination, 6) contact qualification using at least fit and filter insert determination, 6) geo-location determination where for example mask 130 detects a wireless beacon or uses GPS or LPS technology to indicate that the mask and therefore the wearer 1 is now for example in a "public area XX" (where a mask is required) or in a "private area YY" (where a mask is optional), 8) communication with mask usage tracking system, and 9) mask usage tracking via mobile device and app 2a.

Mask frame and electronics 130-1 preferably comprises: 1) earpiece 130-1ep and ear band 130-1eb for attaching frame 130-1 to the ears of a person / wearer 1, 2) electronics 130-1elc attached to earpiece 130-1ep for controlling various sensors and providing, or determining and providing information useful for implementing an AM-function, 3) jack 130-1jck for plugging into electronics 130-1elc, where jack 130-1jck provides electrical connection between electronics 130-1elc and bottom cross member 130-1bcm via electrical wire 130-1w2 and between electronics 130-1elc and top cross member 130-1tcm via electrical wire 130-1w1 (depicted but not labeled), and where jack 130-1jck provides stretchable physical connection from electronics 130-1elc to bottom cross member 130-1bcm via stretch band 130-1b2 and from electronics 130-1elc to top cross member 130-1tcm via stretch band 130-1b1 (depicted but not labeled), 4) frame orientation sensors 130-1os1, 130-1os2, 130-1os3, 130-1os4, 130-1os5, 130-os6 and 130-1os7 for sensing the orientation of electronics 130-1elc (using 130-1os1), top cross member 130-1tcm (using 130-1os2, 130-1os3, 130-1os40, and bottom cross member 130-1bcm (using 130-1os5, 130-os6,130-1os7), 5) electronic tag reader 130-1nfc for sensing mask filter insert 130-3's electronically readable tag 130-3tag, and 6) terminal 130-1trm for making electrical connection to mask with breath sensor 130-2 via terminal 130-2trm.

Mask with breath sensor 130-2 preferably comprises: 1) mask 130-2msk for attaching to top cross member 130-1tcm and bottom cross member 130-1bcm of frame and electronics 130-1, 2) terminal 130-2trm for making electrical connection to electronics 130-1 via terminal 130-1trm, 3) one or more breath sensors such as 130-2bs-1 for detecting wearer 1 breath and communicating signals through terminals 130-2trm and 130-1trm for communication with electronics 130-1elc, and 4) insert pocket (not depicted) or other sufficient means for accepting and holding removable mask filter insert with electronic tag 130-3.

Mask filter insert with electronic tag 130-3 preferably comprises: 1) filter insert 130-3fil, and 2) electronically readable tag 130-3tag.

Regarding the preferred components comprising mask frame and electronics 130-1, earpieces such as earpiece 130-1ep are well known in the art and are often made of plastic and shaped to be draped or otherwise fitted over one ear of wearer 1, where many possible designs and materials are possible including the use of a stretch ear band like ear band 130-1eb. What is important is that earpiece 130-1ep and ear band 130-1eb, regardless of materials and form serve to provide means for attaching and detaching active mask 130 to the wearer 1. What is also important is that earpiece 130-1ep is constructed of materials and with a rigidity that is more substantial for example as compared to stretch band 130-eb, where this construction of earpiece 130-1ep better supports the mounting of electronics 130-1elc, or even supports a design wherein earpiece 130-1ep and electronics 130-1elc are designed to be attachable / detachable with respect to each other, such that earpiece 130-1ep is replaceable, exchangeable, and comes in different fits, sizes, colors, etc., all as will be well understood by those with experience including ear mounted electronics. Stretch ear band 130-1eb is typically made of a stretchable thread material and used to attached frame 130-1 to the ear opposite the ear substantially holding electronics 130-1elc. While earpiece 130-1ep is depicted as being attached to the right ear of wearer 1 and stretch ear band 130-1eb to the left ear of wearer 1, a careful consideration will show that this can be reversed wherein the use of earpiece 130-1ep and therefore the placement of electronics 130-1elc reverses to the opposite ear, and that many well-known materials and forms are available for the construction of earpiece 130-1ep and stretch band 130-1eb.

Electronics 130-1elc preferably comprises a microcontroller or otherwise sufficient processing means with preferably a built-in wireless communication such as Bluetooth and / or wi-fi, of which many well-known choices are available such as the "RF System on a Chip - SoC Multiprotocol Bluetooth Smart/ANT/2.4GHz SoC" as sold by Nordic Semiconductor. The microcontroller preferably connects to or includes integrated power management which attaches to a battery for powering active mask 130. Many solutions for battery and power management are well known, including those for plug-in recharging and wireless (inductive) recharging. There are many well-known connectors for use as jack 130-1jck for making a pluggable / unpluggable connection into electronics such as 130-1elc. What is different about jack 130-jck is that it not only includes traditional wire connections 130-1w1 (not labeled) and 130-1w2 for communicating between electronics 130-1elc and the sensors included in active mas 130, it also includes attached stretch bands 130-1b1 (not labeled) and 130-1b2.

A careful consideration will show that this arrangement allows the electronics 130-1elc and earpiece 130-1ep combination to be detached/reattached both electrically and physically from the remainder of mask frame and electronics 130-1. Furthermore, wires 130-1w1 and 130-1w2 are dangling apart from stretch bands 130-1b1 and 130-1b2 and are of sufficient extra length such that the wires provide extra "play" as bands 130-1b1 and 130-1b2 are stretched for fit upon the face of wearer 1, thus wires 130-1w1 and 130-1w2 do not impede the stretching. Those familiar with "stretchable wire" such as at least a technology referred to as "iStretch" sold by iStretch, will also recognize that a single stretchable wire can be used to replace for example the combination of wire 130-1w2 and stretch band 130-1b2, or the combination of 130-1w1 and 130-1b2 (shown but not labeled).

It is also preferred that jack 130-1jck is useable for a connection to a plug-in battery recharge means, such that when active mask 130 is not being worn, the battery within electronics 130-1elc is recharged by plugging in a power adapter that provides power through a jack 130-1jck, all as will be well understood by those familiar with rechargeable electronics. Wireless recharging of batteries in electronics is also well known and can be used as an alternative approach to recharging the battery(s) preferably held within electronics 130-1elc. Those familiar with connector cables will also recognize that a power cord for example using standard cell phone type charging and that includes jack 130-1jck for plugging into mask 130, can then also be plugged into a computing device such as a desktop computer, where the mask 130 is then being charged and / or able to communicate any of mask 130 data to the desktop computer, thus providing the AM function of "communicating with mobile device and app 2a" via a wired means, where the "mobile device" is replaced by the desktop computer thus forming alternate "desktop computer and app" 2a.

And finally, with respect to recharging the battery of an active mask such as 130 (or mask 131, see Fig. 20C), it is possible to include any of well known solar charging means or to employ what is known as "kinetic energy" (or the energy of motion) to provide a substantially on-going recharge energy source for recharging masks 130 (or 131), where for example when the masks are being used at a theme park where the wearer 1 is expected to do significant walking and participation in rides, there will be an increased amount of kinetic energy to draw from, all as will be well understood by those familiar with energy transformation systems and electronics. With respect to the battery and the remaining "charge" and "time" available for use, there are many well-known solutions for providing notifications and warnings to a user of an electronic device such as an active mask 130, 131. For example, battery low warnings for mask 130, 131 may include providing audible or visible indications using mask electronics 130-1elc, 131-1elc, respectively, and / or communicating information to the companion mobile device and app 2a, such as a text message or "notification." When combined with a mask usage tracking system (see Fig. 20E) as taught herein, it is further possible to notify the premise if a mask 130, 131 being currently worn by a person within the premise is running low on battery such that a premise agent may then use information provided by the mask tracking system to locate the person and provide replacement batteries or otherwise recharging services.

Top cross member 130-1tcm is preferably made of a material that is substantially deformable in the horizontal plane running side-to-side across the face of wearer 1, such that the top cross member can be shaped to at least some extent to the curvature of the wearer 1's face. Preferably crossmember 130-tcm is more rigid in the vertical plane, such that 130-tcm maintains a substantially level horizontal plane when being worn by wearer 1. In at least one preferred design, the cross member also includes a curvature in the nose area to "bump out" and better rest upon the wearer 1's nose. The preferred design also includes a hinge or equivalent located roughly in the middle of the length of member 130-1tcm, for example to either side of the "bump out." This hinge allows the top cross member 130-1tcm to be folded for easier storing when the active mask 130 is removed from the wearer 1. Bottom cross member 130-1bcm is like top cross member 130-1tcm with the exclusion of any "bump out" for the wearer 1's nose, hence bottom cross member 130-1bcm is also deformable in the horizontal plane for conforming to the face but is more rigid in the vertical plane, such that 130-tcm maintains a substantially level horizontal plane when being worn by wearer 1.

As a careful consideration of the present Fig.'s 20A and 20B will show, both top member 130-1tcm and bottom member 130-1bcm are physically connected to each other on one end by ear band 130-1eb and on the other end by jack 130-1jck, where ear band 130-1eb fits over one ear and jack 130-1jck plugs into electronics 130-1elc / earpiece 130-1ep combination for fitting over the opposite ear and connecting to power and the microcontroller. Thus, the "physical circuit" from jack 130-1jck, through top cross member 130-1tcm, through ear band 130-1eb, through bottom cross member 130-1bcm and back to jack 130-1jck form the "detachable frame" of mask frame and electronics 130-1. (As those familiar with electrical circuits will understand, electrical signals communicating between electronics 130-1elc and any of sensors or other electrical components comprising active mask 130 are not required to travel through ear band 130-1eb.)

In the preferred implementation, this detachable frame is separately attachable to / detachable from mask with breath sensor 130-2, where then mask with breath sensor 130-2 is attachable to / detachable from mask filter insert 130-3. However, as a careful consideration will show, there are advantages to combining the detachable frame with mask with breath sensor 130-2, including eliminating the need for electrical connection terminals 130-1trm and 130-2trm for maintaining an electrical signal path between electronics 130-1elc and the one or more breath sensors 130-2bs-1. However, it remains beneficial that mask filter insert 130-3 is insertable / removable with respect to mask with breath sensor 130-2, regardless of whether or not part 130-2 is separate from or combined with the detachable frame of part 130-1.

In an alternate embodiment of part 130-2, the one or more breath sensors such as 130-2bs-1 are omitted such that part 130-2 has no electronics. In this alternate embodiment, filter part 130-3 may be combined with or otherwise integrated into a single piece with mask 130-2, where then the combination of 130-2 and 130-3, with or without electronics remains preferably separated from the detachable frame of 130-1. What a careful consideration will show is that the more expensive parts of active mask 130 are being separated into removable parts, where then ultimately the frequently replaced and less expensive mask filters 130-3 can be attached / detached or inserted / removed such that the active mask 130 continues to have cleaned or new filters 130-3 without the need to discard any of the more expensive electronics, all as will be well understood to those familiar with safety masks and electronic devices.

Each mask filter 130-3fil preferably has an embedded electronically readable tag 130-3tag, where the tag includes preferably an encrypted code indicating for example the manufacturer and product code or even lot number and unique serial number of the filter. Those familiar with traditional safety masks such as worn during the current CV19 pandemic, will understand that there are many different manufacturers of face masks, with many different levels of filter quality, where for example an "N95" mask is considered to be of higher quality.

The preferable active mask 130 is capable of reading tag 130-3tag for use in qualifying the level of filter protection being worn by person 1, where the qualifying information is used for any one of or any combination of: 1) sharing with "contacts" of other contact tracing devices such as other active masks 130 being worn by other persons, mobile device and apps 2a (or alternatives) configured for contact tracing, or other mobile devices such as smartphones configured for contact tracing (but not configured for health tracking as taught herein, thus not serving as 2a), where each quantitative contact is then qualified by the level of filter protection being used by the contacting person / face mask 130 wearer 1 as well as the determined mask fit (see explanation below), 2) communicating to mobile device and app 2a as a usable "health measurement" in combination with a rule associated with a regiment (see upcoming Fig. 20D), where the health measurement is for example the quality of the filter, the use-time of the mask 130, and the mask fit determination with respect to mask 130, where mask fit determination includes data indicating if the mask is being properly worn by wearer 1 such that the top cross member 130-1tcm is substantially resting upon the wearer's nose above the nostrils and bottom cross member 130-1bcm is substantially underneath wearer's chin but at least below the mouth, the arrangement combination providing that the wearer 1's breath is being substantially filtered by mask filter 130-fil, 3) communicating to a wireless "mask tracking" systems (see upcoming Fig. 20E) capable of detecting and tracking face masks 130 such as within a building, a transportation means such as an airplane, bus, subway or cruise ship, or destination area such as a theme park where currently non-active face masks are required, where the electronics 130-1elc within each active face mask 130 preferably includes a unique identifier registered to the wearer 1, and where each face mask 130 communicates with for example the mask 130 tracking system to provide a mask status, such as but not limited to the filter tag 130-3tag information and the mask fit determination, and 4) use by electronics 130-1elc to generate audible signals to the wearer 1, where audible signals for example indicate if a filter mask 130-3fil is not being detected or if the mask is determined to not be properly fit, especially when electronics 130-1elc determines that another contact is entering the proximity of the wearer 1, where "entering the proximity" will be well understood by those familiar with contract tracking especially using mobile devices (such as mask 130 or a smartphone) equipped with a wireless technology such as Bluetooth.

Still referring to Fig.'s 20A and 20B, mask with breath sensors 130-2 preferably uses one or more breath sensors such as 130-2bs-1. Those familiar with thermistors, thermocouples, humidity sensors and what are known as "heat flux sensors" (see for example "thin-film heat flux sensors" as sold by FluxTeq) will recognize that several alternative technologies are sufficient and available for substantially determining if a wear 1's breath is properly flowing through what is essentially the vertical / horizontal middle of mask 130-2 and therefore also the vertical / horizontal middle of filter 130-3. As will be well understood by those familiar with electronic devices, the alternatives for breath sensors offer at least different cost points, design complexity, detection accuracy and power requirements such that various configurations of active face mask 130 in general, and face mask part 130-2 in particular, are anticipated for addressing the larger and diverse marketplace.

A preferred "lower-cost" solution includes an array of well-known "resistance temperature detectors" (also referred to as "RDT sensors"), where RDT sensors are also preferred for use as contact temperature sensors for including with the various wearable devices described herein including the finger-worn devices such as 3-1-d7, 3-1-d8 and 3-1-d9 or the chest band 4-3. The RDT sensors operate over a temperature range that exceeds the typical human environment and offer fast response times and temperature sensitivity. RDT surface mount sensors sold by EI Sensor Technologies come in a small form-factor of less than 2mm square and 1.3 mm high and have low power requirements. Multiple such surface mount sensors may then be embedded within face mask part 130-2 and connected via wires to terminal 130-2trm, where terminal 130-2trm then electrically connects with and through terminal 130-1trm to provide signal communications with electronics 130-1elc, all as will be well understood by those familiar with device electronics.

What is important to see with respect to face mask and breath sensor part 130-2 is that a sensing technology for detecting the changes in temperature or the "flux of heat" substantially caused by the inhaling and expelling of air by the wearer 1 is embedded within part 130-2 such that the embedded sensing technology is usable to substantially determine the presence of the wearer 1's breath located at the vertical / horizontal area of mask parts 130-2, 130-1. As will be discussed shortly, this breath detection information is then usable by electronics 130-1elc either by itself or in combination with the orientation sensing information provided by one or more orientation sensors such as 130-1os1 through 130-1os7 for determining the "quality of the current fit" of part 130-2, where the preferred fit is sufficiently placed over the nose and mouth of wearer 1 such that substantially all of the wearer's breath is being transmitted through attached filter part 130-3.

What is also important to see is that one or more breath sensors such as 130-2bs-1 are properly arranged to cover the area of mask part 130-2 through which the wearer's breath is designed to be filtered by part 130-3fil, and that the choice and positioning of the breath sensors such as 130-2bs-1 is such that preferably the ability of part 130-2 to deform is not substantially restricted. Regarding the deformability of part 130-2, easy and sufficiently pliable deformation is expected and useful at least when the top cross member 130-1tcm is pulled down under the nose and even under the mouth by the wearer 1, and where the use of multiple small sensors 130-2bs-1 such as depicted and anticipated using at least RDT temperature sensors provides sufficient pliability as the sensors "fold" along with the material of face mask parts 130-2 and 130-3.

With respect to multiple orientation sensors 130-1os1 through 130-1os7, there are also at least two well-known technologies for use including what are known as MEM gyroscopes and MEM accelerometers. For example, STMicroelectronics sells multiple configurations of MEM gyroscopes including the L3G20HTR and NXP sells multiple of what are known as 3-axis accelerometers including MMA8653FCR1, where typically the cost of the gyroscope is roughly two times the cost of a 3-axis accelerometer. What is preferred, is to detect motion in each of three perpendicular "X, Y, Z" planes, as will be understood by those familiar with gyroscopes and accelerometers. By placing at least one sensor such as 130-1os1 on electronics 130-1elc, that are expect to not move relative the wearer 1's head, at least one orientation sensor is then useable to determine the current orientation of the wearer 1's head.

If preferably three other sensors 130-1os2, 130-1os3 and 130-1os4 are mounted on the top cross member 130-1tcm then they are sufficient for determining the current orientation of the top cross member 130-1tcm with respect to the head of wearer 1. As a careful consideration will show, determining the relative orientation of top cross member 130-1tcm provides useful orientation information related to the relative orientation of mask with breath sensors 130-2 and the filter insert 130-3. Likewise, other sensors 130-1os5, 130-1os6 and 130-1os7 are mounted on the bottom cross member 130-1bcm and provide indication of the orientation of the bottom cross member 130-1bcm with the respect to the head of wearer 1, as well as the bottom of part 130-2 and the bottom of part 130-3, all as a careful consideration of Fig.'s 20A and 20B will show. And finally, what will also be apparent is that determining the relative orientation of top cross member 130-1tcm relative to the head and bottom cross member 130-1bcm relative to the head, provides a means for determining the relative orientation of the two cross members 130-1tcm and 130-1bcm to each other.

A careful consideration of the typical use of a face mask will show that two distinct types of "mask deformation" indicative of the "quality of mask fit" are usual. In a first use case, a wearer 1 unhooks either earpiece 130-1ep or more expectantly ear band 130-1eb from the respective ear, such that the entire face mask 130 drops down or dangles down from the ear to which it remains attached. In a second use case, a wearer 1 leaves both earpiece 130-1ep and ear band 130-1eb attached to their ears while doing either of both of: 1) then pulling down top cross member 130-1tcm such that 130-1tcm is lowered to be under the nostrils or even further down below the mouth or chin, or 2) pulling up bottom cross member 130-1bcm such that 130-1bcm is raised at least above the mouth.

Those familiar with the information provided by gyroscopes and 3-axis accelerometers will recognize that such sensors provide information such that when being moved "globally," therefore where the sensor's positions and orientations relative to each other are not substantially changing, the provided information can be processed to filter out the global movement, thus showing that there is substantially no relative movement between any of the sensors. For example, when active mask 130 is being worn by wearer 1 and presumed in a proper fit, as wearer 1 moves their head about in any the possible directions and orientations, all orientation sensors 130-1os1 through 130-1os7 are anticipated to substantially indicate the same (global) movement in the three planes x, y and z, or similar information.

A careful consideration will show that in a first variation of the first use case, where the wearer 1 unhooks the face mask 130 from the ear being attached to by band 130-1eb, all cross member orientation sensors 130-1os2 through 130-1os7 will record a combination of mostly global movement as all cross member sensors are rotated downward, with some relative movement as the mask 130 deforms. Conversely, the earpiece-electronics orientation sensor 130-1os1 will remain substantially "fixed" relative to the wearer's head and as such the cross member orientation sensors 130-1os2 through 130-1os7 will indicate substantial relative movement with respect to sensor 130-1os1.

In the second variation of the first use case, where the wearer unhooks the face mask 130 from the ear being attached to by earpiece 130-1ep, then all orientation sensors 130-1os1 through 130-1os7 will rotate downward together providing mostly global movement.

Regarding the second use case as stated above, a careful consideration will also show that if the wearer 1 leaves mask 130 attached to both ears but then substantially lowers the top cross member 130-1tcm and / or raises bottom cross member 130-1bcm up, at least "top cross bar" sensors 130-1os2, 130-1os3 and 130-1os4 are expected to show significant relative movement with respect to "bottom cross bar" sensors 130-1os5, 130-1os6 and 130-1os7, and the "moved cross member" (either or both the top 130-1tcm or bottom 130-1bcm) is expected to also show relative movement with respect to sensor 130-1os1 that is essentially remaining "fixed" in its orientation and position with respect to the wearer 1's head.

Those familiar with machine learning (ML) will understand that any one of or any combination of mask frame 130-1 orientation sensor data or mask with breath sensor 130-2 breath sensor data provides significant data for training a model capable of at least classifying a face mask 130 as "fitting" or "not fitting" properly on the face of wearer 1. While the preferred embodiment of mask 130 includes multiple orientation sensors and multiple breath sensors, a careful consideration will show that at least the number of orientation sensors as well as the number of breath detection sensors can each be decreased (even to zero sensors) and or increased with resulting changes in effectiveness with respect to the overall performance of the "fit detection" ML algorithm / data model or any other algorithm such as a deterministic algorithm, all as will be well understood by those familiar with software algorithms. Those familiar with sensors will also recognize that other types of sensors can be included in mask 130 for detecting additional information or for replacing the information detected be either or both the orientation sensors or breath sensors in order to facilitate in "fit detection." For example, the upcoming alternate active face mask 131 (see Fig. 20C) at least uses skin contact sensors. Another possible type of other sensors includes piezoelectric thread or "conductive textiles" for preferably including in mask part 130-2, where one use of piezoelectric material is to determine the stretching of a fabric such that a fully extended mask part 130-2 properly covering the face provides a detectably different electrical signal then the "crumbled" / deformed mask part 130-2 that is not properly covering the face.

Referring next to Fig. 20C, there is shown an alternative active mask 131 that is expected to be lower in production costs than active mask 130. Like mask 130, alternative mask 131 comprises three main parts, mask frame and electronics 131-1, mask 131-2 and mask filter insert 131-1. Also like mask 130, mask 131 provides any one of or any combination of the active mask functions (AM-functions).

Mask frame and electronics 131-1 preferably comprises: 1) top member 131-1tm for substantially resting upon and generally fitting the curvature of at least what is known as the "nasal ridge" of the nose preferably above the nostrils / "nasal base" (and possibly extending laterally beyond the nose to also cover a portion of the wearer 1's cheek nearest to the nose to provide either of both of more stability or additional skin contact points as explained below), where top member 131-1tm includes one or more skin contact sensors placed on the underside of the top member that will be in contact with the wearer 1's nose / skin, 2) bottom member 131-1bm for substantially resting upon and generally fitting the curvature of the chin, where bottom member 131-1bm includes one or more skin contact sensors placed on the underside of the bottom member that will be in contact with the wearer 1's chin / skin, 3) electronics 131-1elc that is combinable with bottom cross member 131-1bm, where electronics 131-1elc is preferably attachable to / detachable from member 131-1bm and thereby includes an electrical connection to bottom cross member 131-1bm including any of well-known means, and where alternatively electronics 131-1elc is built into bottom cross member 131-1bm as a single integrated unit, 4) top-left electrical wire / stretch band 131-1eb-tl for electrically and physically connecting to the left side of top member 131-1tm and via left lock 131-1lck-l connecting to the bottom-left electrical wire / stretch band 131-1eb-bl, where the bottom-left electrical wire / stretch band 131-1eb-bl then electrically and physically connects to the left side of bottom member 131-1bm, such that an electrical circuit if formed starting from electronics 131-1elc, though bottom member 131-1bm, through bottom-left band 131-1eb-bl, through left lock 131-1lck-l, through top-left band 131-1eb-tl, into top member 131-1tm, and 5) a similar "physical only" circuit (therefore preferably not including any electrical circuit) for connecting from the right of top member 131-1tm to the right of bottom member 131-1bm, where the physical circuit includes a top-right stretch band 131-1eb-tr, a right lock (not depicted), and a bottom-right stretch band (also not depicted).

Mask 131-2 for attaching to the mask frame and electronics 131-1 and for holding mask filter insert 131-3, where for example mask 131-2 includes an insert pocket or similar for allowing the inserting and removing of a filter 131-3. A preferred means for attaching mask 131-2 to frame 131-1 is to include multiple holes along substantially the top and bottom edges of mask 131-2, such as bottom left hole 131-2h, where a careful consideration will show that prior to connecting the top-left band 131-1eb-tl to the bottom-left band 131-1eb-bl via left lock 131-1lck-l, it is possible to thread the top-left band 131-1eb-tl through the top-left holes (not labeled) in mask 131-2 and to thread the bottom-left band 131-1eb-bl through the bottom-left band holes (only hole 121-2h is labeled), and similarly before connecting the top-right band 131-1eb-tr to the bottom-right band (not depicted) via right lock (not depicted), it is possible to thread the top-right band through the top-right holes in mask 131-2 and to thread the bottom-right band through the bottom-right band holes, such that mask 131-2 is thereby attached to mask frame and electronics 131-1.

Mask filter insert performs the function of filtering the air inhaled and exhaled by wearer 1 for many of well-known reasons and uses, and alternately includes an electronic tag (such as described with respect to filter 130-3 of Fig.'s 20A and 20B), where such an electronic tag preferably uses NFC technology and can therefore be read (or written to) using traditional NFC readers that are typically built into most smartphones, where the smartphones are then also useable as the mobile device for executing the herein described mobile app forming mobile device and app 2a such that the mask 131 data including any data stored on a filter insert electronic tag is usable as information for compliance with a health regiment, all as careful reader will understand.

Regarding the preferred components comprising mask frame and electronics 131-1, those familiar with the configuration of the human face will understand that there are a range of possible nasal ridge curvatures, such that the preferred mask 131-1 is sold with, or at least available to work with multiple top members 131-1tm of different sizes and shapes for best fitting the shape of the wearer 1's nasal ridge.

Those familiar with skin contact sensors will also understand that there are many well-known technologies for determining if the underside of a top member 131-1tm is in contact with the skin of the wearer 1, where it is preferred that the underside includes two or more contact points, where for example one contact point is substantially on the left-side of the nasal ridge, another contact point is substantially in the middle of the nasal ridge, while a possible third contact point is on the right-side of the nasal ridge. What is important to see is that the shape of the top member 131-1tm substantially conforms to only one location one the face of the wearer 1 that is at least above the nostrils, for example the nasal ridge, such that if removed from the nasal ridge of the nose, at least one of the two or more contact points included on the underside of the top member 131-1tm will not sense contact with the skin of the wearer and therefore top member 131-1tm (and by extension mask 131) can be determined to be "not properly fitted." A preferred contact sensor technology uses surface mount wires / electrical conducting material included in the underside surface of top member 131-1tm, where the electrical resistance of the surface mount wires / conducting material is detectably altered when in contact with the human skin versus air, all as will be well understood by those familiar with human touch sensors. However, the present invention should not be limited by the choice for a skin contact sensor, as many variations are well-known and available in the art, each with different tradeoffs, such that multiple variations of a mask 131 are possible and anticipated using different skin touch sensor technologies.

Top member 131-1tm can alternately be constructed and sold with a permanently attached top-left electrical wire / stretch band 131-1eb-tl and a permanently attached top-right electrical wire / stretch band 131-1eb-tr, where a careful consideration will show that even with permanently attached bands 131-1eb-tl and 131-1eb-tr the attached bands can still be threaded through preferable holes such as 131-2h included with mask 131-2. However, top member 131-1tm can also be constructed with one (or more) attachment points for attaching top-left band 131-1eb-tl to 131-1tm and one (or more) attachment points for attaching top-right band 131-1eb-tr to 131-1tm (where multiple attachment points provide custom fit options), where attachment points for top-left band 131-1eb-tl include an electrical connection (or alternately top-right band 131-1eb-tr if the electrical signals are to be run through the right-side of mask 131, or both sides of mask 131 as those familiar with electronic devices will understand).

A careful understanding with respect to top member 131-1tm can be applied to the understanding of bottom member 131-1bm, where the main difference with respect to skin contact sensing is the shape of the chin (regarding 131-1bm) versus the nasal ridge (regarding 131-1tm). Therefore, for the sake of brevity the details of the bottom member with respect to skin contact sensing technologies and attachment types and means for connecting with bottom-left band 131-1eb-bl and bottom-right band (not depicted) will not be discussed further. It is again noted that electronics 131-1elc can be either of embedded into bottom member 131-1bm, or permanently mounted to, or preferably attachable to / detachable from bottom member 131-1bm.

Especially in consideration of a wearer 1 with significant facial hair that impedes sufficient skin contact sensing (as will be well understood by those familiar with the electrical properties of hair versus skin), several aspects of bottom member 131-1bm can be altered, where it is even possible to include at least one orientation sensor (such as 130-1os3 through 130-1os7 described above in reference to active mask 130) in top member 131-1tm and at least one orientation sensor in bottom member 131-1bm. A careful consideration will show that as long as (nose) skin contact is detected via the skin contact sensors in top member 131-1tm, it can be assumed that the orientation information provided by the at least one orientation sensor in top member 131-1tm is "global" and therefor indicative of the wearer 1's head orientation.

Keeping this understanding in mind, a careful consideration of the typical use of a face mask will show that two distinct types of "mask deformation" indicative of the "quality of mask fit" are usual. In a first use case, a wearer 1 unhooks either combination top-left band 131-1eb-tl and bottom-left band 131-1eb-bl from one ear, or combination top-right band 131-1eb-tr and bottom-right band (not depicted) from the other ear, such that the entire face mask 131 drops down or dangles down from the ear to which it remains attached. In a second use case, a wearer 1 leaves mask 131 attached to both ears while doing either of both of: 1) then pulling down top member 130-1tm such that 130-1tm is lowered to be under the nostrils or even further down below the mouth or chin, or 2) pulling up bottom member 130-1bm such that 130-1bm is raised at least above the mouth.

A careful consideration will show that in the first use case, where the wearer 1 unhooks the face mask 131 from either ear, at least one (and most likely all) top member 131-1tm skin contact sensor(s) will indicate the loss of skin contact and if an orientation sensor has been included in top member 131-1tm, the top member orientation sensor will also show significant changes in orientation. In this first use case, if the bottom member 131-1bm includes skin sensors and the wearer 1 for example does not have sufficient facial hair to impede the skin sensors, at least one (and most likely all) bottom member 131-1bm skin contact sensor(s) will indicate the loss of skin contact if an orientation sensor has been included in bottom member 131-1bm, the bottom member orientation sensor will also show significant changes in orientation.

Regarding the second use case as stated above, a careful consideration will also show that if the wearer 1 leaves mask 131 attached to both ears but then substantially lowers the top member 131-1tm, at least one top member 131-1tm skin contact sensor (and most likely all) will indicate the loss of skin contact and if an orientation sensor has been included in top member 131-1tcm, the top member orientation sensor will also show significant changes in orientation. In this second use case, if the wearer 1 leaves mask 131 attached to both ears but then substantially raises the bottom member 131-1bm, at least one (and most likely all) bottom member 131-1bm skin contact sensor(s) will indicate the loss of skin contact and if an orientation sensor has been included in bottom member 131-1bm, the bottom member orientation sensor will also show significant changes in orientation. If either the top member 131-1tm or bottom member 131-1bm is lowered or raised, respectively, but not both are moved, and if at least one orientation sensor is included with each of the top member 131-1tm and bottom member 131-1bm, then the top and bottom member orientation sensors will show significant relative motion with respect to each other. A careful consideration will also show that significant relative motion between and with respect to orientations sensors included in both the top member 131-1tm and the bottom member 131-1bm will be detected in the case where both the top member 131-1tm is lowered and the bottom member 131-1bm is raised while mask 131 remains attached the wearer 1's ears.

As with masks 130, those familiar with machine learning (ML) will understand that any one of or any combination of mask frame 131-1 skin contact sensor data or mask frame 131-1 orientation sensor data provides significant data for training a model capable of at least classifying a face mask 131 as "fitting" or "not fitting" properly on the face of wearer 1. While the preferred embodiment of mask 131 includes multiple skin contact sensors in top member 131-1tm and multiple skin contact sensors in bottom member 131-1bm, as well as at least one orientation sensor in each of the top member 131-1tm and the bottom member 131-1bm (especially if the wearer 1 has facial hair essentially covering the chin), a careful consideration will show that at least the number of skin contact sensors as well as the number of orientation sensors can each be decreased (even to zero) and or increased changing the overall performance of the "fit detection" ML algorithm / data model or any other algorithm such as a deterministic algorithm, all as will be well understood by those familiar with software algorithms.

The teachings regarding active mask 130 with respect to Fig.'s 20A and 20B as well as the teachings regarding active mask 131 with respect to Fig.'s 20C are meant to describe two different "best-mode" embodiments, where each best-mode embodiment has cost / benefit tradeoffs in the marketplace, but otherwise the descriptions of masks 130 and 131 are not meant to be restrictive and as such should be considered as exemplary, rather than as limitations of the present invention. Various teachings with respect to mask 130 may be incorporated into mask 131, while conversely teachings with respect to mask 131 may be incorporated into mask 130, all without departing from the true scope and spirit of the invention, where both masks 130 and 131 as described, as well as many possible variations as will be well understood by those skilled in the various arts including but not limited to electronics and what are known as "e-textile," "smart fabric," "functional fabrics," and "smart textiles." What is important to see is that both masks 130 and 131 can be configured to perform any one of or any combination of the "active mask" (AM) functions as described and taught herein.

With respect to both masks 130 and 131, it should also be recognized that various components such as the electronics 130-1elc and 131-1elc, respectively, can be divided into sub-components, where the sub-components can either located as taught or relocated. For example, a traditional hearing aid typically places the electronics (including batteries) behind the ear, where then it is even possible to place either additional electronics or additional batteries for performing the same or additional AM functions behind both ears. Also, in another example variation, stretchable bands 131-1eb-tl and 131-1eb-bl are specified to include electrical wires for communicating signals between electronics 131-1elc and any and all sensors included in mask 131. However, the construction of these bands 131-1eb-tl and 131-1eb-bl could be changed to be similar to that shown for active mask 130, where the stretchable portion (see for example 130-1b2) of the band is separated from the electrical portion (see for example 130-1w2). As will also be clear from a careful consideration, the closed / connected state of a jack such as 130-1jck or lock with electrical connectivity such as 131-1lck-l (or any electrical juncture in the electric circuit of the mask 130 or 131) is detectably distinct from the open / disconnected state, also providing valuable information for providing AM functions.

As will also be understood by those familiar with wearable electronics, it is possible to include GPS technology with either of electronics 130-1elc or 131-1elc, where the GPS technology enables the near continuous communications between an active mask such as 130 or 131, respectively, with for example any of a mobile device and app 2a or a mask tracking system (see especially upcoming Fig. 20E). In some situations, depending upon GPS coverage, a GPS signal is not available but a wi-fi signal is. In other situations, neither a GPS or wi-fi signal is available, where each active mask 130 and 131 continues to track the "quality" of fit (and optionally mask filter insert), as well as the "quantity" of contacts determined using contact tracing, and whereupon regaining sufficient communications signal each mask 130 or 131 can then upload any data determined while "disconnected" from the / any remote mask tracking device (such as 2a) or system (such as shown in Fig. 20C). Such additional functionality is for example useful for companies or agencies with workers that are required to wear masks while performing services, and furthermore are required to leave the company premise to for example make a delivery (such as a UPS, Amazon or USPS worker) or make a in-house call (such as a nurse practitioner or a special needs teaching aid associated with a public school).

Those familiar with real-time Bluetooth / wi-fi communications will also recognize that active masks 130, 131 may be "paired" with for example the wearer 1's mobile device and app 2a, where the combination of the mask 130, 131 and the mobile device and app 2a are in substantially continuous communications (for example where the person 1 is wearing the mask 130, 131 while also carrying the mobile device and app 2a). Since a typical mobile device such as a smartphone used to form mobile device and app 2a comprises GPS technology, the paired masks may then be considered as configured with "accessible" GPS technology as opposed to "included" or "embedded" GPS technology as described in the prior paragraph.

Furthermore, it will be clear that using any of accessible, included, or embedded GPS technology, an active mask 130, 131 has the means for staying in communication with any of a mobile device and app 2a or a mask tracking system. As taught herein, various "zones" can be established using geolocation means such as GPS or LPS (e.g. wi-fi), were a given health regiment rule can be configured to a specific zone, and where for example zones include a person 1's private residence, private transportation, public space or work space, all as will be well understood by those familiar with both global positioning systems (GPS) and local positioning systems (LPS). Thus, a careful consideration will show that mask qualitative and quantitative tracking and logging can be adjusted by at least geolocated zones, such that a person 1 has different health regiment rules applying to a mask 130, 131 at least while in a private space as opposed to a public space or a workspace.

Regarding masks parts 130-2 and 131-2, especially because the parts include a replaceable filter part 130-3 and 131-3, respectively, it is possible to allocate additional money and features to masks 130-2 and 131-2. Once such preferred upgrade is to use a more expensive material for masks 130-2 and 131-2, such as what is referred to as "brr Nylon" or what is referred to as "brr Polyester" and sold by "Brrr," where these products offer superior cooling effect when in contact with the face / skin, and therefore offer a significant advantage for situations where masks 130 or 131 are to be worn either in warmer climates and / or for longer periods of time.

Referring next to Fig. 20D, there is shown a child 1-3 wearing active mask 131 (or 130) that is in wireless communication 131-comm with mobile device and app 2a being operated by guardian or parent, where such communication is referred to as AM function (1) communicating with mobile device and app 2a (or equivalent). With respect to the construction of mask 131 and the teachings related to Fig. 20C, mask part 131-2 (not labeled) is shown to include a "design" or branding, where this decorative aspect to masks is well-known in the art, typically raising the cost of the mask. The present invention prefers mask part 131-2 with a replaceable filter 131-3, even where the filter includes an electronically readable tag, such that the additional cost of design possibilities to be discussed shortly, are amortized over a longer period of use than a typical "throw-away" mask, all as will be well understood by those familiar with product design.

Other masks currently being sold by companies such as "Light Up Masks" also include a battery power source and control multiple LEDs for adding additional decorative effect. At least some such masks currently being sold with LEDs also include a sound sensor, where data collected from the sounds including "nearby noises, voices, or music" are used to automatically drive the flashing of the LEDs. Other marketplace masks synch wirelessly with a mobile device and app for the purposes of entering messages to be "lit up" using the mask LEDs, or otherwise to control the effect of the LEDs. As a careful reader will see, the present teachings relating to both active masks 131 and 130, and in particular mask parts 131-2 and 131-1, respectively, are able to support and benefit by the use of decorations including printed colors, LEDs, and other attached materials. The careful reader will also see, that by employing information determined by any one of or any combination of breath sensors such as 130-2bs-1 (see Fig. 20A) or orientation sensors such as 130-1os1 - 130-1os7 (see Fig. 20A), whether included in mask 130 (of Fig. 20A) or mask 131 (of Fig. 20C), it is possible to control special active decorative functions such as the flashing of lights triggered by "the wearer's voice" as opposed to the current marketplace solutions that are limited to less discerning "nearby noises, voices, or music."

Still referring to Fig. 20D, the careful reader will also understand that both masks 131 and 130 as taught herein offer significant differences with respect to existing marketplace "decorative" masks that include power and electronics, where each of the prior listed "active mask" functions (AM-functions) are only enabled by the present teachings. Again, the AM functions at least include: 1) communicating with mobile device and app 2a (or equivalent), 2) contact tracing using wireless communication means, 3) contact proximity notification to wearer, 4) mask fit determination, 5) filter insert determination, 6) contact qualification using at least fit and filter insert determination, 6) geo-location determination where for example mask 130 detects a wireless beacon or uses GPS or LPS technology to indicate that the mask and therefore the wearer 1 is now for example in a "public area XX" (where a mask is required) or in a "private area YY" (where a mask is optional), 8) communication with mask usage tracking system, and 9) mask usage tracking via mobile device and app 2a.

In the present figure, the parent or guardian is using a mobile device and app 2a to: 1) authenticate that the mask 131 (or 130) is being worn by registered person 1-3, and 2) to set "mask tracking" to "on." Sufficient authentication preferably includes determining a unique device ID for the mask 131, 130 being worn by wearer 1-3, for example as provided by electronics 131-1elc or 130-1elc, respectively, during wireless communications 131-comm. In addition to determining the device ID, using device and app 2a, parent or guardian: 1) captures an image of person 1-3 for facial recognition as a registered person, 2) instructs person 1-3 to put on the mask 131, 130 thus occluding a significant portion of person 1-3 now wearer 1-3's face, 3) continues to capture images of the mask-occluded face of wearer 1-3 to continue to confirm person 1-3 to be the wearer 1-3 of the mask 131, 130, 4) sends controlling signals to mask 131, 130 to initiate a sequence of LED flashes as emitted by an LED included with mask 131, 130 and located in any of a number of positions, whereupon substantially simultaneously imaging wearer 1-3 and detecting the sequence of LED flashes, device and app 2a authenticates using image processing that registered person 1-3 is wearing mask 131, 130 that includes a given device ID, all as a careful reading of the present invention will show.

In an alternative approach to authentication, step (4) "sends controlling signals..." is replaced by a step (4a), where the parent or guardian moves mobile device and app 2a closer to the wearer 1-3's face, even to the point where the wearer 1-3's face is substantially not seen in the current images being captured by device and app 2a, but where a sequence of images are captured by 2a as the device and app 2a are moved from a place of first seeing the face of wearer 1-3 to a place of being oriented sufficiently close to the electronics 131-1elc, 130-1elc of mask 131, 130, respectively, such that device and app 2a can detect an electronically readable tag embedded in mask electronics. As those familiar with image processing will understand, using the "sequence of images" captured by 2a during step (4a) it is possible to determine and track a changing set of visible feature points formed by any of the wearer 1-3's face or the mask 131, 130, where any two consecutive images in the sequence of images will contain at least some visible feature points in common, where the common feature points are then usable to confirm that the wearer 1-3 and mask 130, 131 are not changing, for example to some different person X wearing a different or same mask 130, 131.

Regarding the common visible feature points usable for confirmation / authentication by image processing and pattern matching, it is possible to place the electronically readable tag (such as an NFC tag like 130-3tag embedded in filter 130-3fil, see Fig. 20A) at a location such as the top of mask part 131-2 or 130-2, such that the sequence of images captured during step (4a) is likely to provide more visible feature points with respect to the face of wearer 1-3 for use in confirming wearer 1-3, than for example as would be expected by embedding the electronically readable tag for example under the chin in electronics 131-1elc, or to the side of the face in electronics 130-1elc, all as will be understood from a careful consideration of the teachings provided herein.

Still referring to Fig. 20D, once mask 131, 130 is confirmed and authenticated, quantitative and qualitative tracking of mask 131, 130 is then initiated by turning "mask tracking on," where again qualitative tracking at least includes any one of or any combination of the AM functions (4) mask fit determination and (5) filter insert determination, and quantitative tracking at least includes (2) contact tracing using wireless communication means. As will be understood from a careful reading of the teachings provided herein, and from a knowledge of contact tracing technology as for example provided by Google or Apple, during contact tracing information is exchanged between the contacting electronic devices (such as smartphones in the traditional case as addressed by Google and Apple), where the present invention teaches a mask 131, 130 capable of exchanging both the traditional data supporting a quantitative log being established on each contact tracing device, as well as qualification data at least including any one of or any combination of: a) mask fit determination data, b) filter insert determination data, or c) health regiment status data, where mobile device and app 2a has provided such health regiment status data via wireless communications 131-comm preferably at the time of initiating the tracking of mask 131, 130. This ability of mask 131, 130 to exchange both the traditional data supporting a quantitative log being established on each contact tracing device, as well as qualification data is referred to herein as AM function (6) contact qualification using at least fit and filter insert determination.

In the exemplary use case being discussed in relation to the present figure, once the mask tracking is initiated, the wearer 1-3 may then go and engage in activities that are being conducted potentially out of the sight and communication range of the parent or guardian. In one mode, where mask 131, 130 is unable to maintain a substantially continuous communication (e.g. using GPS or wi-fi), or even intermittent communication with the controlling device such as mobile device and app 2a (or its equivalent, or a mask tracking system, see Fig. 20E), mask 131, 130 at least logs data for subsequent uploading to the controlling device, where the data is determined by any of AM functions such as (4) mask fit determination, (5) filter insert determination, (2) contact tracing using wireless communication means and (6) contact qualification using at least fit and filter insert determination, where the careful reader will understand that AM function (6) provides for active mask 131, 130 being worn by wearer 1-3 to receive qualification data from other masks 131, 130 being worn for example by playmates of wearer 1-3. For situations where the mask 131, 130 remains either in continuous or intermittent communications with the controlling device, data sharing or uploading between the mask 131, 130 and the controlling device can happen with greater frequency, all as will be well understood by those familiar with systems and communications and from a careful reading of the teaching provided herein.

Fig. 20D should be recognized as exemplary, where for example the teachings are equally valuable for other use cases including as prior mentioned, employees or service personal associated with a company or agency, where the employees or service personal are required to wear properly fitted masks 131, 130 with proper inserts 131-3, 130-3 and where qualitative contract tracing is highly desirable, such as within a factory floor filled with employees and possibly outside contractors or vendors, all required to also wear masks 131, 130 and all coming into and out of contact with each other.

After initiating mask tracking, mask 131, 130 is also enabled to begin providing AM function (3) contact proximity notification to wearer, where for example the child 1-3 is alerted through an audible signal emitted by a speaker included in mask 131, 130 and controlled by electronics 131-1elc, 130-1elc, respectively, to properly adjust their mask when another device capable of contract tracing (such as a mask 131, 130 being worn by a playmate, or smartphone configured for contract tracing and data sharing with mask 131, 130 and being carried for example by an adult) is detected as approaching, all as will be understood by those familiar with the processing of wireless signals such as Bluetooth or wi-fi that are useable to estimate distances between emitting devices based on various teachings such as signal strength or encodes signal timing, or by those familiar with the art of contact tracking and local positioning systems.

It is also possible that for example LEDs are included with masks 131, 130 for generating signals visible to any contact passing by, where preferably a "green" light is emitted to indicate for example that the wearer 1-3 has a properly fitted mask, with a proper insert, and has a minimum health qualification (such as "L1" described herein), or conversely a "red" light or even a "yellow" light is emitted to provide a wearer 1-3 status that is less than that sufficient for the "green" light, thus mask 131, 130 is capable of providing functional information (as opposed to, or in addition to decorative information) using any included means for outputting visible light such as an LED, or even by transmitting the equivalent information using wireless communication 131-comm (or 130-comm with respect to mask 130).

In further consideration of Fig. 20D, it is also possible that the wearer 1-3 uses their own mobile device and app 2a to authenticate and initiate their own mask 131, 130, such as an employee beginning a day and heading into work. Thus the employee is turning on their active mask 131, 130 to both collect valuable information for their personal benefit, and to share valuable information for the benefit of other persons (preferably with active masks 131, 130 of their own, or at least with another contact tracing device such as a smartphone), where this use case is covered further with respect to upcoming Fig. 20E and the "mask tracking system." As prior discussed herein, especially in relation to Fig. 11 describing a "private meeting network," it is possible for a person participating in the private network to see and review both quantitative and especially qualitative information relating to any other member in the group, depending upon the privacy configuration chosen and enabled by the group, all as will be well understood by a careful reading herein. For example, a play group of six friends covering four families can all be tracked where the parents or guardians are able to see and access real-time or near-real time quantitative and qualitative information as herein described, especially in relation to the AM functions. Such personal networks should be considered broadly as including any two or more individuals, such as but not limited to: friends, family, workers, teachers, contractors, delivery agents, traveling health practitioners, attendees, visitors, patrons, guests, or any of the roles well-known in societies.

Still referring to Fig. 20D, and for example the use case where wearer 1-3 is using their active mask 131, 130 at a theme park or a birthday party, it is also possible to provide / sell decorative add-ons that for example attach to a top member such as 131-1tm or 130-1tcm, respectively, or bottom member such as 131-1bm or 130-1bcm, respectively, such as false noses, mustaches or chins, where these add-on can be configured to even draw power from electronics 131-1elc or 130-1elc, respectively, so as to then be "active add-ons" that provide for example light or sound output. With proper additional configurations of mask 131, 130, "active add-ons" could also be provided for the ears of the wearer 1-3, or virtually any location connectable to / attachable with the mask 131, 130.

And finally, in a broader consideration including or excluding such add-ons, active or passive, the careful reader will recognize that the information created by the teachings provided herein with respect to both masks 131, 130 (or any variations of such masks 131, 130), as well as the controlling device such as mobile device and app 2a or a mask tracking system (see Fig. 20E), provides many opportunities to introduce what is referred to in the market as "gamification" for use in at least encouraging the proper use of masks and the following of health regiments, where gamification includes any one of or any combination of: 1) accumulating gamification points based at least in part upon any AM function information or health regiment information, where for example the points are rewards for wearing a mask properly and / or following a regiment, 2) creating perceivable indications including any of light, sound or tactile indications, on a mask 131, 130 or mobile device and app 2a, where the indications are determined at least in part using any AM function information or health regiment information, and where the indications increase or decrease gamification points, or create a presentation discernable to others of a person 1/ wearer 1's achievements, 3) exchanging gamification points for decorative parts at least including masks 131-2, 130-2 or decorative add-ons, where the decorative parts are either active or passive, and where when providing active decorative parts, additional controlling functions are also optionally provided or enabled within the mobile device and app 2a, such that the rewarded person can cause new special effects in accordance with their achievement level, or 4) exchanging gamifications points for advantages or otherwise benefits associated with a "premise-wide" on-going game, such as a "physical-virtual" game, were the reader is directed to see the many teachings of the cross-reference related applications.

Referring next to Fig. 20E, there is shown a premise (referred to as "Zone 4", 74) owned or operated by an entity 40 that is implementing preferably both an authenticated health regiment 2d-1, 2d-2 and contact tracing, where access to the premise is controlled though any one of or any combination of one or more private controlled health-verified access points 5a, 5b connected through an adjoining confirmation Zone 3 (not depicted) for providing access to a "visitor" 1-3, or any one or more of non-private controlled health-verified access points 5a, 5b connected through an adjoining confirmation Zone 2 (not depicted) for providing access to a "worker" 1-w. The reader is directed to Fig. 8 provided herein for more detail related to a system for providing "mutual health assurance" at a premise / zone 74.

What is important to see with respect to Fig. 20E is that both premise "visitors" such as 1-3 and "workers" such as 1-w may use any of an active mask 130, 131 as taught herein to serve as their smart-ticket wearable in addition to or replacement of 2-3, 3-1-d4, 3-1-d5 (see Fig. 8). Functioning as a smart-ticket includes either or both: 1) determining, receiving, or otherwise providing authenticated "right-to-access," where for example the "right-to-access" is provided to a self-access point 5a, 5b for gaining access into premise / ZONE4, 74, and 2) determining, receiving, or otherwise providing authenticated "proof-of-health," where for example the "proof-of-health" is provided to a self-access point 5a, 5b for gaining access into premise / ZONE4, 74, all as taught herein.

Like wrist-worn wearables 2-3, 3-1-d4, 3-1-d5, active masks 130, 131 are capable of communications with mobile device and app 2a for receiving from mobile device and app 2a any of determined "right-to-access" or determined "proof-of-health," where the wearables such as active masks 131, 131 then carry the "rights" and "proofs" for conveniently presenting to at least the various access points 5a, 5b to a premise / ZONE74, 74, as all described herein. In the preferred operation, the mobile device and app 2a conducts and performs substantially all of regiment 2d-1, 2d-2 tracking and compliance including the taking or receiving of various authenticated health measurements using for example any of health measurement devices 3-1, 3-2, all as described herein. A careful reading will show that the companion devices such as wrist-worn wearables 2-3, 3-1-d4, 3-1-d5 or face-worn mask 130, 131 are capable of acting as self-operated health measurement devices 3-1 for providing health metrics to the mobile device and app 2a to be processed in accordance with the rules of a regiment 2d-1, 2d-2. For example, active masks 130, 131 acting as health measurement devices 3-1 can determine and provide to mobile device and app 2a multiple health measurements for use in relation to a regiment 2d-1, 2d-2, where exemplary health measurements include: 1) dates / times / duration of a mask 130, 131 being worn by a wearer such as 1-3, 2) the quantity and / or quality of contacts encountered by a wearer such as 1-3, for example including statistics such as average proximity and average quality formulated as "exposure risk," 3) the extent of "proper mask fit" and "proper insert filter" of mask 130, 131 being worn by a wearer such as 1-3, or 4) any information derived at least in part from any AM function.

As a background for better understanding the benefits provided by the current active mask 130, 131 and mask usage tracking system teachings as provided herein, it is noted that traditional automated contact tracing is accomplished using mobile devices such as a smartphone, where a careful consideration will show that: 1) many individuals such as children do not possess personal smartphones, 2) many other people are not carrying their smartphones or otherwise do not have them properly configured for contact tracing, 3) there is no current precedence for requiring that all individuals to carry a smartphone while within a premise or otherwise a congregation area such as a cityscape, 4) smartphones are not currently "authenticated" to a carrier as described in the teachings herein, 5) there is no convenient and public way to confirm that an individual is carrying a smartphone for example in compliance with a public ordinance supporting contact tracing, 6) the cost of a smartphone far exceeds the anticipated cost of an active face ask 130, 131, thus virtually eliminating the "equity" concern were people with lesser financial means are deprived of a public health benefit, 7) the active face mask 130, 131 as herein described provides an important key function of breath and air filtration for reducing the transmission of infectious diseases and thus provides an important additional function that a smartphone cannot provide, 8) manual based contact tracing methods have been overwhelmed by the current CV19 pandemic, 9) semi-automated and automated contact tracking (also referred to as "digital contact tracing") using smartphones has been estimated by researchers to required 56% to 95% "population uptake" in order to be effective at reducing infectious disease transmission, where researchers indicate that "automated contact tracing could potentially reduce transmission with sufficient population uptake" however as of yet no automated systems has proven sufficient for reaching the necessary "uptake" levels (for which the interested reader is directed to "Automated and partly automated contact tracing: a systematic review to inform the control of COVID-19" published August 19, 2020, authors Braithwaite, Callender, Bullock and Aldridge), 10) a smartphone based approach using what is known as a "decentralized" technology such as implemented by Google and Apple can protect personal privacy for example by representing individual smartphone carriers using anonymous, encrypted "rolling proximity identifiers" (RPI) that change for example every 10 minutes, where then each contact shares their current encrypted RPI such that a given smartphone for example used in a crowded city might record hundreds to even thousands of "contacted RPIs," and where if a notice is then generally published that a given RPI has been determined to be from a possibly infected person, each smartphone must then expend a significant amount of energy / power to decrypt daily lists of contacts in search for a possible match, and 11) the current state-of-the-art only includes contact quantification, for example listing zero to potentially thousands of contacts per day, where none of the contacts is qualified, nor is the carrier qualified, where the benefit of qualification at least includes ignoring or not recording any contact between a carrier who is tracked as wearing a proper fit and filter mask and "contacting" another person also determined to be wearing a proper fit and filter mask, or ignoring or not recording any contact with a known and authenticated vaccinated carrier, thus saving potentially significant amounts of energy / power to be expended on decryption algorithms and contact list processing.

Still referring to Fig. 20E, one of the benefits of using an active mask 130, 131 as a smart-ticket is that in many situations, especially as related to the current pandemic, the premise operator 40 dictates that all "visitors" such as 1-3 and "workers" such as 1-w are required to wear a mask. Hence, by using an active mask 130, 131 as opposed to a traditional mask, it is possible to achieve a "premise-population uptake" of virtually 100% (thus making automated contact tracing effective), where the active mask 130, 131 becomes the only electronic device capable of virtually 100% usage for contact tracing that is essentially ensured to be worn by all persons (such as 1-3, 1-w) present in a premise / ZONE4, where the active masks 130, 131 are authenticated as being worn by registered persons, and where compliance is made enforceable due at least in part to the visible nature of active masks 130, 131 (potentially configured with publicly viewable "proper fit / filter" LED indicators) and due at least in part to the active mask 130, 131's ability to self-report to the mask tracking system the extent and continuity of "proper fit and filter" during a date / time duration or otherwise to provide essentially "real-time mask-alerts" including a premise location and registered person to a premise agent such that the agent can then quickly find and instruct the wearer 1 to properly align their mask 130, 131. It is also noted that when staying at a premise such as a cruise ship or theme park that offers an extended stay to a guest / "visitor" 1-3, the visitor will spend at least some time self-confined to personal quarters, such as a berth or room, respectively, wherein the present teachings of mask 130, 131 have shown that such private geolocations can be identified by the mask 130, 131, or the mobile device and app 2a in communication with the mask 130, 131, for example using any of GPS, LPS or location beacons, such that a wearer 1-3 is aided in the decision as to when a mask 130, 131 must be properly worn.

Virtually any location with controlled access, as well as other locations with enforced "mask rules," can be considered a premise / ZONE4 including " Even open cityscapes with encouraged mask rules can benefit by the population using active masks 130, 131 for quantitative and qualitative contact tracing, where for example the current practices are limited to populations wearing non-active masks incapable of providing the AM functions, smartphones for providing contact tracing but otherwise unable to provide authenticated "proof-of-health," where the net combination of contacts traced is often under 20% of the population, thus rendering contact tracing to be of limited to no real value. The careful reader will understand, that in the majority of populations under consideration, especially in a pandemic type of situation, the majority of the population (or at least substantially more than 20%) will be wearing a mask that can then be an active mask 130, 131, such that the quantitative and qualitative contact tracking using active masks 130, 131 provides a significant technical improvement and advantage over the current state-of-the-art.

Another important use case to consider are school campuses where young children 1-3 are at risk of infection and are required by school rules, along with all other teachers and workers 1-w of the school, to be wearing a mask at all times (except when separated for eating), even including when outside for example in a play area. By using active masks 130, 131, and equipping the school premises with a mask tracking system, the school is provided with a significant technical advantage for dealing with a health crisis such as the current CV19. As those familiar with school systems will understand, a typical school already includes a wi-fi network that substantially covers the school buildings and premise, where the exiting wi-fi system can be used by the mask tracking computer system as a means for communicating with active masks 130, 131. **It** is further possible to include what are known as "beacons" at the entrances to for example all rooms or gathering areas within the school buildings and grounds, such that the mask tracking system is enabled to log essentially the real-time location of all masks 130, 131 (assumed to be virtually 100%) of the students and persons at currently at the school.

Those familiar with the challenges faced by schools (as well as virtually all other gathering places where there is a recurring population often on a daily basis), if a particular person or child is determined to be infected, then the current practice is to shut down the entire school for a "quarantine" period of typically two weeks, thus causing significant disruptions to the educational process and the lives of the families with students. Using active masks 130, 131 and a mask tracking system as taught herein, it is now possible to identify using quantitative and qualitative contact tracing all individuals that have come into various levels of contact with a known infected person, where then it is possible to only send home for quarantine those contacts deemed sufficiently exposed (based upon both quantification of exposure, and qualification of exposure), thus avoiding the significant disruptions.

The careful reader will also note that in the school use case, all students, teachers or other school related persons are also preferably using the teachings provided herein to comply ("in-private, at-home") with an on-going health regiment such that the students, teachers or other school related persons are effectively blocked from coming to school when they are detected as sufficiently symptomatic, or perhaps if they have not yet taken a person health kit test for infection, or even have not yet received an available vaccination. Other individuals not complying with a personal health regiment may then also be restricted to interacting with the school at some safe distance or through some safe means, thereby shielding the school's population.

The use cases described in relation to Fig. 20E are exemplary, as virtually all gathering places of sufficiently sized populations can receive significant benefit by use of the teachings provided herein including the active masks 130, 131 and the mask tracking system. Other well-known examples include theme parks and cruise ships whose premises are considered as "discretionary," where the use the many teachings such as herein described that restricted visitors to: a) those who have complied with and "passed" an authenticated health regiment 2d-1, 2d-2 preferably including up to two weeks or more of health measurements for example including temperature, blood oxygenation and cough detection taken prior to arriving at the premise, a health kit test taken with negative results determined within 1 week prior to the visit, and / or a vaccination received at least 3 days prior to the visit, and b) those who have agreed to wear an active mask 130, 131 while on premise for use with a mask tracking system, is expected to provide the premise with a significant technical advantage for providing "mutual health assurance."

In terms of contact tracing within stadiums using assigned seating that is typically associated with the wearer 1's original ticket / "right-to-access," since the teachings herein associate the right-to-access (preferably including in this example a section & seat number), it is possible to record detailed stadium location data in any mask tracking system's database. Regarding mask 130, 131 enforcement, while within a premise requiring the use of masks, the mask tracking system will also have access to virtually real-time contact tracing data concerning an average "social distancing metric" per mask 130, 131 that can remove people in "trusted groups" from the metric calculation, where for example a trusted group includes family or friends for which a person 1 is expected and allowed to be within the otherwise enforced social distance (such as 6 feet). Individual persons determined to be insufficiently complying with social distancing rules can be sent automatic alerts, such as to a companion mobile device and app 2a, or even directly to the mask 130, 131 where for example an audible signal or message is emitted to be heard by the wearer. Individuals receiving and continuing to ignore alerts can be more easily located and approached by premise agents for additional enforcement. The "social distancing metric" is also useful for stadium and premise gamification, where for example those in compliance are offered benefits including discounts or early access to premise exits, where exiting the premise is conducted in a controlled manner to support social distancing, and where the controlled manner includes notifications from the mask tracking system to either of the mask 130, 131 or a companion mobile device and app 2a alerting a mask wearer 1 when and where they may exit the premise.

Still referring in general to active masks 130, 131 and a mask tracking system used at a premise 74, especially where the use includes gamification, at least frames 130-1 and 131-1, respectively, can include what are known in the art as electrodermal sensors, often referred to as "stress sensors." In the example where a premise 74 is a theme park, masks 130, 131 then provide additional "stress" information regarding the stress levels of persons as they participate in premise activities such as rides, shows or events including exciting or "scary" scenes, where the stress information can be used for both gamification and otherwise useful health metrics to inform for example a parent or guardian with respect to a child or older parent. As those familiar with ride construction will understand, it is even possible to use the substantially real-time stress information gathered with respect to a wearer 1 of an active face mask 130, 131 (or a wearable such as 2-3, 3-1-d4, 3-1-d5 equipped with for example an electrodermal sensor or pulse / heart rate sensor) to effect or otherwise control at least in part the experience provided to the wearer 1 during the remainder of the ride / experience.

Referring next to Fig. 21 there is shown a summary flowchart of the mutual assurance system 102. The present Fig. 21 is provided as an overview bringing together many of the prior discussed key parts of the present invention, where the present invention also comprises aspects of the PRIOR ART smart-ticket 2. Therefore, it should be understood that the teachings provided in relation to the present Fig. 21 are meant to show the larger system 102 primarily at a mid-level of detail, where the reader is directed to the both the current specification and the referenced PRIOR ART smart-ticket 2 (see Patent No. 10,861,267 entitled THEME PARK GAMIFICATION, GUEST TRACKING AND ACCESS CONTROL SYSTEM issued on December 8^{th}, 2020) for additional and more detailed descriptions of the various apparatus and methods (key parts) discussed in relation to the present flowchart.

It should also be understood that one of the purposes of the teachings related to Fig. 21 is to provide additional discussions of different exemplary variations to key apparatus and methods, where the variations are based upon or otherwise like the teachings herein provided and where still other variations will be obvious to those skilled in the various arts, such that the teachings in relation to the present Fig. 21 should be considered both an overview and exemplary, without limiting further variations that are possible while staying within the true scope and spirit of the present invention.

A careful review of the flowchart in Fig. 21 will show a first step 150 of a person downloading or otherwise obtaining an "Honest Broker" App 2a for use on a personal computing device such as but not limited to a smartphone, thus the App 2a and smartphone in combination becoming the basis for the smart-ticket 2 (all as prior described herein). The present flowchart does not review the apparatus and methods that are addressed to the processing of a "ticket" or "right-to-access," but rather focusses on the processing of the person's "self-authenticated" "proof-of-health." (Again, the absence of the discussion of a "ticket" or "right-to-access" with respect to the overview flowchart of the present figure, or the absence of any other teachings already provided in the present specification, should not be interpreted as indicating that these many other given teachings are not a part of system 102, but rather that the present inventors have chosen for the purposes of clarity with respect to the "proof-of-health" centered goals of Fig. 21 to omit many additional teachings. From this point forward in the discussion related to Fig. 21, little to no attention or mention of these purposefully omitted additional teachings will be made.)

Still referring to Fig. 21 and initial step 150, it is important to see that the obtained App 2a serves as an "Honest-Broker" acting on behalf of at least (a) the person 1 desirous of "proving their current health status," (b) the providers of health measurements and services referred to herein as "L1" "L2" and "L3" that are useable to affect, indicate, substantiate or otherwise inform the health status (where the providers comprise self-operated devices 3-1, other-operated devices 3-2 or otherwise provider 42 services, especially healthcare services), and (c) at least agents of a premise or gathering that are desirous of knowing the person 1's current health status prior to making a decision to allow entrance, participation other otherwise activity that would expose other workers, guests, patrons, customers, etc. to possible transmission of an infectious diseases or illness. With this understanding, it is noted that a "final step" (albeit potentially recurring) is "proceed to access if approved" 159, where is should be understood that this grant-of-access to "proceed" is provided to a person 1 based upon a sufficient "proof-of-health" status.

As a careful reading of the present specification will show, a major teaching and goal of the present system 102 is that the person 1 have the option to maintain anonymity when gaining access in step 159, and where the anonymity means that no personal information is ever shared or provided to the agent of the premises (such as buildings, destinations, forms of transportation, or otherwise any place people aggregate) or gathering (where the implication is group oriented as opposed to place oriented, such as a group of friends meeting at different locations, or a travel youth sports team meeting at different fields of play). Hence, using the present system 102 it is possible that a person 1 enters a destination or building without ever providing their name, any identifying information and or having to provide a biometric such as a facial or fingerprint scan but otherwise does provide a "publicly trustable" "proof-of-health." All that is required is the person has provided identifying information to the "go-between" "Honest Broker" App 2a, such that the App 2a has confirmed, verified, and otherwise authenticated and communicated a current health status to the access control system of the premises or gathering, where the access control system may be any combination ranging from fully automated, to partially automated, to manually conducted. The Honest Broker App 2a is thus acting as an intermediary for conducting an anonymous yet authenticated exchange of proof-of-health information between the person 1 and the access control system.

For example, in some methods discussed herein, the access control system is fully automated and the person using their personal computing device goes through a method that includes providing an electronic transmission such as an "access token," where preferably the access token is encrypted by the App 2a and indicates that the person 1 who is requesting access is currently of a given "authenticated health status," but otherwise the App 2a in a preferably "anonymous mode" and does not provide any other personal information regarding the person 1. Once the access token is received by the fully automated access control system, if the person 1's current health status is confirmed by the access control system as sufficient, then the person is allowed to proceed or is otherwise given access, where many options for authorized proceeding and accessing are herein discussed and possible and still others will be obvious to those skilled in the art of automated access systems.

In a different example, the access control system is fully manual such as a premise guard and the person 1 presents the screen of their personal computing device and App 2a to the guard, where the App 2a is presenting information for recognition by the guard sufficient for the guard to make an "allow/deny" entry decision. The screen may for example show a picture of the person 1 along with a code or symbol, where the guard confirms that the picture is of the person 1 and that the code or symbol matches an expected code or symbol. Teachings were provided herein showing that the code or symbol can be generated by the App 2a in response to information provided to the App 2a by the access control system (e.g. by wireless communication, or a code that is show to the person 1 by the guard and then entered by the person 1 into the App 2a), such that the "proper response" code or symbol is then not easily deduced or forged by the person 1 using some bogus / non-official app.

Those skilled in the art of access control systems will understand that fully automated systems and fully manual systems, or some combination thereof, can include a "challenge" by the system to the person 1 / App 2a, where the challenge is for example specific information, and where the specific information is ultimately provided by some communication means (e.g. wireless or a "user interface" for information entry) to the App 2a such that the App 2a, and only the App 2a as the Honest Broker (thus not the person) "understands" how to properly respond to the specific information challenge, where the proper response is communicated (by some means, e.g. electronic or visually presented) as response information, and where the proper response may also include an indication of the person 1's health status, or for example be a different code or symbol based upon the health status.

To provide even tighter security, it is even possible that for example a person (such as a guard) acting as a component in the access control system also does not know the proper response, and therefore may for example enter the response code or symbol provided by the App 2a into an access control system device with user interface, where then the access control system (that presumably generated the specific information) then also confirms that the provided response code or symbol is sufficient - thus the guard cannot override or otherwise ignore and incorrect response. Many variations are possible, but again what should be seen is that all variations support protecting the anonymity of the person 1, while at the same time protecting the "public trust" of the access control system because the grant-of-access is based upon authenticated health information ultimately provided by the intermediary Honest Broker App 2a (as the present flowchart further reviews).

Still referring to step 159, in another variation the person 1 presents the screen of their personal use computing device executing the Honest Broker App 2a to the access control system, where the screen includes encrypted information such as a QR code / 2D barcode, like that used for an airline boarding pass. The 2D barcode for example can provide unique information generated by an algorithm only known to the App 2a and the access control system, for example where the unique information is generated based upon the date, time, an access location code, etc. such that the 2D barcode would be extremely difficult for a person to forge using a bogus app.

The careful reader will see that many access control systems and scenarios are possible without departing from the true scope and purpose of step 159, which is that person 1 using App 2a provides to the access control system preferably (but not necessarily) anonymous information determined at least in part by the App 2a, where the presented information serves to confirm to the access control system the person 1's "authentic" "proof-of-health" / current health status (where "authentic" includes meaning that the health status is "of" the person 1 and that each piece of the person 1's underlying "L1," "L2," and "L3" health information used at least in part to for determining the health status has been "vetted" / confirmed / authenticated). As will be made clear by a careful reading of the present specification and by the present overview and flowchart, assurance is provided by the person 1 to the access control system based upon the understanding that the Honest Broker App 2a operates cooperatively but independent of the person 1 to authenticate, gather, determine and assess a potential multiplicity of health measurements and services, any one or more of which may serve at least in part as the basis for the person 1's current health status.

Still referring to Fig. 21, a careful review will also show that it is possible, and optional, that the Honest Broker App 2a works with one or more "Regiments" 2d (see discussions related to steps 152, 152a, 152b, and 152c), where a Regiment 2d provides many advantages as discussed herein. As will be clear from a careful reading of the present invention, a Regiment 2d comprises a set of one or more rules, where the rules are preferably known to and even changeable by some external authority including an entity 40 (Regiment 2d-1) owning, operating, or representing the premises or gathering, or a governing body / public health organization 44 (Regiment 2d-2), but otherwise preferably not changeable by the person 1 or at least not changeable in anyway determined as detrimental to the goals of authenticity and proof-of-health.

The rules of a Regiment 2d comprise significant information and can: (1) implement "conditional logic" thus also supporting overrides (such as seeking a doctor's approval if a health status is otherwise currently "failing"), (2) combine with other Regiments 2d forming more complex Regiments 2d or covering "special occasions" such as a planned visit to a highly secured facility or premises, (3) implement geofencing and timing restrictions on any given rule(s) for example supporting quarantines, (4) be updated dynamically based upon for example generalized broadcast messages 102-msg from an ML/AI system 102-ml that is learning based at least in part upon an anonymized "rolling snapshot" database of health metrics 102-db updated from a larger population of persons 1, (5) interface with a contact tracing dataset 2a-db-6, 2a-db-7, or (6) include specification and limitation regarding health measurement devices and services, all amongst other key advantages discussed herein.

In the simplest sense, a Regiment 2d comprising a single rule covers receiving a healthcare service, such as a doctor's checkup or a vaccination, where a more complex Regiment 2d as will be discussed in relation to the present Fig. 21, might include three levels, "L1" (see steps 160 - 164) pertaining to on-going measurements of personal health metrics (thus being indicative of a symptomatic or asymptomatic state), "L2" (see steps 170 - 174) pertaining to measurements related to body fluid samples provided as a part of an "at-home" / "privately used" "health kit," and "L3" (see steps 180 - 184) pertaining to professional services such as a vaccination.

As will be clear to those familiar with software systems, Regiments 2d allow for the separation of: (a) the controlling data (i.e. "the rules") from (b) the processing application (i.e. App 2a) and (c) the pertinent health data generated by the application's processing of the rules, where then a more broadly applicable App 2a becomes more specifically adaptable to the combined requirements of a larger variety of persons, premises and gatherings, where these formalized Requirements 2d might then also need to be changed / updated more often with respect to any need to change an algorithm comprising the App 2a. However, as will also be understood, it is possible that the rules are essentially a part of the App 2a and therefore not necessarily separable, and to this extent the person 1 is simply "using an App 2a" as opposed to an "App 2a with a Regiment 2d" for implementing the teachings of system 102.

As such, the present Fig. 21 portrays the Regiment 2d steps 152, 152a, 152b and 152c as "optional," where the system 102 benefits from Regiments 2d but is not otherwise limited to functioning with a Regiment 2d such as described herein, where again sufficient rules may be included with the App 2a and therefore pre-se not a Regiment 2d, or more specifically not a set of information external or separate from the App 2a, where many variations are possible.

Still referring to Fig. 21, another overview note is about step 153a to "update Health data, Regiment." What is important to see is that preferably the health information (i.e. "Health data") derived using the many apparatus and methods of flow paths "L1," "L2," and "L3," or otherwise any health information derived using the teachings of the system 102 such as health measurement data or other related data including information supporting the validation of a given health measurement (such as electrodermal activity data or ambient environment data), or data related to the registration of a person (such as a biometric), is stored as encrypted data on the person 1's personal use device that is also executing the Honest Broker App 2a.

Those skilled in the art of devices will understand that the data may be stored on any of internal or external storage media or may alternatively or additionally be provided by communication means (preferably encrypted) to remote storage (such as secured "cloud storage"), or any combination thereof or similar, as many variations are possible. Those familiar with datasets will also understand that if a Regiment 2d is being used, it is advantageous to store the "Health data" in relation to the "Regiment" and even in relation to a specific Regiment-rule for which the data was captured, where "in relation" can be implemented in many variations using computing database or data representation methodologies, all as will be understood by those skilled in the art. What is preferred is that a two-way association is made, such that for example starting with a Regiment 2d / rule, it is possible for the App 2d to "find" (i.e. "search the database for," or "look-up") the captured and associated health data, or conversely, starting with the health data it is possible for the App 2d to find the associated Regiment 2d / rule.

As a careful consideration of system 102 will show, a potential disadvantage of the decentralized and anonymized architecture is that if the personal use device (storing a person 1's preferably encrypted data) is lost or damaged, then the person 1 may no longer be able to sufficiently recreate their current health status. However, there are many sufficient remedies to this disadvantage that still maintain personal data privacy and anonymity, for example by maintaining an encrypted backup at the option of the person on an external storage media controlled exclusively by the person, and / or an encrypted backup maintained on a secured third-party storage such as Microsoft's One Drive, where the encrypted data is otherwise not useable even if "stolen" from the cloud storage.

Especially in relation to the upcoming discussion of step 184 "receive validated confirmation of service," where for example the service was a visit to a doctor, a vaccination shot, or otherwise some provision of healthcare, it will be shown that the service provider preferably communicates to the App 2a a "digital certificate" (or similar) that includes encrypted verification of service provided. Preferably, the service provider maintains a database of all digital certificates provided to all App's 2a as a course of doing business, where each certificate is stored in relation to an "authentication token" uniquely generated by the App 2a at the time of service, where the authentication tokens are not themselves useable to identify a specific person 1.

Should a person lose their health data as stored on their personal computing device, or for example lose their digital certificate representative of an important healthcare service such as a vaccination, it is then possible that person 1 uses their App 2a to communicate with the service provider's (preferably secured) information system and database to retrieve any and all digital certificates (or otherwise health information) being stored in the database in relation to the authentication token presented by the App 2a, all as will be understood by those familiar with software systems and databases. (As will also be shown in relation to step 153b, either the App 2a or the service provider transmits the "proving health service information" such as a digital certificate, along with the person 1 / App 2a's authentication token to some third-party intermediary that acts as a storage and retrieval service for a community of service providers and persons 1, their customers, clients, workers, citizens, etc.)

As a careful consideration will show, the service provider may never actually know who the person 1 is that was provided the service (such as a vaccination or other health treatment), nor know who the person 1 is that is currently retrieving a copy of the digital certificate (or otherwise "proof-of-service"), since the digital certificate copy is linked to anonymous data / authentication token that only the Honest Broker App 2a will know (where it is further preferred that even person 1 not be aware of the data encrypted within a given authentication token or similar). In this type of retrieval by authentication token of proof-of-service, it is preferred that the person 1 uses their App 2a as the Honest Broker to first identify themselves to the App 2a and then to request that the App 2a "negotiate" the retrieval from the service provider (or for example a governing body that holds copies of the digital certificates of service or similar - see step 153b), where negotiations includes preferably secure communications providing the request and receiving the digital certificate / "proof-of-service" copy, all as will be well understood by those familiar with information systems.

As will be discussed again in relation to step 153a, what is preferred is that not only the health data comprising "L1," "L2," and "L3" measurements and services (paths 160, 170 and 180, respectively), but also some form of "receipt" / "validation" of the measurement (especially for "L2" and "L3" data where a second-party is providing services) is maintained on the personal use device executing the App 2a, for use by the App 2a in maintaining the current health status of the person 1.

With respect to the well-known disadvantage of "locally maintained data" being lost, a careful consideration will show that: 1) lost "L1" health data relating to the tracking of symptoms, such as temperature, cough, blood oxygenation levels, resting heart rate, etc. is by its nature "time sensitive" in any regard (e.g. where the "current" health status uses a 7 or 14 day rolling window of measured symptoms), such that if "L1" data is lost a person 1 can essentially restore their current status by taking more measurements over the next 7 or 14 days, and 2) lost "L2" health data related to a "health kit / test" can also essentially be replaced simply by taking another similar health kit / test. Hence, the storage purposes indicated in step 153a is fulfilled in many possible ways and allows for the App 2a to maintain access to captured and determined "L1," "L2," "L3" and otherwise any health data regardless of classification, either in relation to a Regiment 2d or not, for "determining a current health status" in step 155 as a part of choosing to "seek access rights" to a given premises or gathering step 154.

Still referring to Fig. 21, but now more in a step-by-step flow approach, after downloading or otherwise obtaining the Honest Broker App 2a in step 150, the person is then required to "register" with the App 2a in step 151. Again, the App 2a is acting as a trusted agent / intermediary in a multi-party transaction where "public trust" in the person 1's provided current health status is necessary. As a first step towards this public trust, the person 1 provides personal information to the App 2a, such as at least one or more biometrics, where a biometric comprises any information that may be sensed about the person (as opposed to "entered in" by the person, like a password) that is essentially impossible or at least difficult to forge and otherwise sufficiently accurate for confirming the person 1's identification according at least to the needs of the particular access control system. Those familiar with personal biometrics, such as facial recognition or fingerprint recognition, will understand that these methods are not typically considered "full-proof," where especially some methods such as facial recognition are less reliable based upon the race of the person, the camera used and lighting within which a facial image was captured, as well as the settings chosen for the algorithm and / or the algorithm itself, and methods such as facial and fingerprint recognition can be affected overtime by the changing or wearing out of the face / finger features.

Regardless, what will also be understood is that multiple forms of biometrics (often referred to as "multi-modal" identification) can be combined to create increased veracity. As will also be understood, existing methods such as facial recognition are always being improved, for example by improving algorithms and / or sensors, where improving sensors includes for example capturing 2D or 3D facial images in combinations of visible light, IR light (both presently possible using current smartphone technology) and polarized light (expected in the near future, e.g. see Sony's existing "polarization sensors" IMX250MZR and IMX250MYR using technology that is anticipated to someday be made available in smartphones).

Newer techniques are also being provided, such as identification using what is known as "ECG" (electro-cardiogram) sensors such as manufactured by companies including Nymi. Measuring heartbeat characteristics is also possible using rPPG (remote Photoplethysmography) and PPG (Photoplethysmography), where these characteristics can be used as either a part of "intra-identification" that is used to authenticate two or more health devices capturing data from the same person 1 substantially during a common duration of time, or even "inter-identification" that is used to authenticate / uniquely differentiate an individual person 1, all as herein discussed. The present invention even teaches devices such as finger-worn device 3-1-d9 that capture a fingerprint and combine this with light signaling for unique device authentication, such that the captured fingerprint of person 1 can be authenticated to be of the same person 1 whose facial image is also be recognized by the App 2a, all as taught herein.

What is desirable is that one or more biometrics are captured of the person 1 for registration with the App 2a if not also registration with a Regiment 2d (see step 152b) being used by the App 2a. In the case of registering with a specific Regiment 2d, the "general App 2a registration" step 151 is either essentially repeated or otherwise the biometrics of the "general registration" (step 151) are associated for use as the biometrics of the "specific registration" (step 152b). Step 152b follows step 152a "Download Regiment," where step 152b is like step 151 except that preferably the Regiment 2d indicates the one or more biometrics required by the specific Regiment 2d, and otherwise the captured and registered biometrics are associated with the Regiment 2d, where it is possible that the App 2a has one set of "general registration" biometrics along with multiple "specific registration" biometrics corresponding to multiple specific Regiments 2d, where each Regiment 2d then has its own biometrics and / or uses biometrics managed in common by the App 2a.

Still referring to Fig. 21 and now step 152, the person 1 preferably uses the App 2a to select and otherwise chose to "Use a Regiment." Regiments 2d are anticipated to be available from preferably secure sources using digital certificates and other forms of encryption and security such that the person 1 is assured to be receiving through the App 2a a "proper" Regiment 2d, where a proper Regiment 2d includes digital certification, and / or is communicated to the App 2a in a secured manner such that person 1 is assured that the Regiment 2d is "of" / "from" the "regiment provider."

A Regiment 2d optionally includes a public key associated for example with the issuer ("regiment provider") of the Regiment 2d (such as a governing body / public health organization 44 or entity 40). As those familiar with public/private key encryption will understand, it is then possible for the App 2a to encrypt the health status or any measurement or service confirmation associated with the Regiment 2d using the Regiment 2d's public key when providing a health status to an access control system, where it is then also understood that the access control system must have the private key to decrypt the health status, measurement or service confirmation.

Those familiar with encryption will recognize that this or a like approach helps (a) to assure the person 1 that their health status is not susceptible for misappropriation when communicated to a given access control system (although secured communication methods between the App 2a and the access control system are preferably used also mitigating this concern), and b) to assure the access control system that the person 1's health status is based upon an authorized Regiment 2d. Those familiar with data and software systems will recognize that variations are possible without departing from the scope of the present invention, and that furthermore since the Regiment 2d is preferably obtained by the Honest Broker App 2a, where the App 2a itself is already secured as herein discussed and as discussed in the referenced smart-ticket PRIOR ART, the present system 102 has significant benefits even if the Regiments 2d do not include a public key or otherwise additional security measures.

Those familiar with software systems will also recognize that preferably the App 2a helps the person 1 search for available and applicable Regiments 2d, for example where relationships are presented between types of premises and gatherings, or specific premises and gatherings, and one or more "accepted Regiments" 2d.

Still referring to Fig. 21, assuming the person 1 uses the App 2a to choose (step 152) and download (step 152a) a Regiment 2d, the person 1 then proceeds at some point in time thereafter to "Register the Regiment" in step 152b as prior discussed. Each given Regiment 2d might require no "specific registration" (e.g., because it relies upon the App 2a's "general registration") or require a "specific Regiment registration" with any one or more biometrics or other registration methods such as passwords, challenge questions, etc., all as will be well understood by those in the art of secured software systems.

As prior stated, Regiments 2d comprise one or more rules for example specifying types of health measurements (see flowchart path's beginning at steps 160, 170 and 180 for determining "L1," "L2," and "L3," health data respectively) that are employed by the Regiment 2d. The present specification provides significant teachings for rules and Regiments 2d, where it is again noted that the present summary flowchart is not intending to review these teachings in detail and thus the reader is directed to the entire specification for a full understanding.

In a step 152c, the person 1 chooses to "follow" a Regiment 2d and thereafter is preferably reminded by App 2a using notification(s) according to a schedule embedded or implied by a Regiment 2d. Alternatively, in step 152c the person selects the Regiment 2d and possibly a Regiment 2d rule by their own initiative without prompting, rather than in response to a notification / prompting from App 2a. (In either case, or by any other implementation of Regiment 2d "activation" or selection in step 152c, the person 1 then engages in the determination of one or more and possibly on-going "L1" type health measurement, metrics, etc. that may involve "L2" type services that require providing body fluid samples or "L3" type services such as provided by a healthcare provider.)

If a Regiment 2d is being used by a person 1, the present flowchart depicts that this step 152c is preferably related to all paths "L1," "L2," and "L3," and thus potentially all types of health measurements. However, as a careful consideration will show, any particular Regiment 2d if used (where zero or more Regiments 2d may be in use by the person 1 at any time) may be limited to any one of or any combination of the types of measurements such as "L1," "L2," and "L3," where it should also be understood that the nomenclature of "L1," "L2," and "L3," is not restrictive to the invention but merely illustrative, as there are no limits for example on naming or classifying a Regiment 2d, nor on the types or numbers of rules a given Regiment 2d comprises. However, what is preferred is that a Regiment rule 2d is at least available to the App 2a for "governing" for example any of steps 162 "Take controlled measurement with Device," 172 "Take controlled sample using Kit, provide to Service," and 182 "Request (controlled) Service, provide other information," where "governing" in general relates to placing any type of restriction or limit, or otherwise influencing or effecting the measurement to be taken or service to be provided, where governing in general helps to increase "public trust," as a careful consideration will show.

Still referring to Fig. 21, the present invention specifies at least three general types of health measurements all extensively taught in the specification. The flow path starting with step 160 focuses for example on a person 1's "at-home" / "taken-in-private" health measurements (or otherwise health measurements obtained under their own control and without help from a service provider or second-party), such as taking their own temperature and blood oxygen using a finger-worn device such as 3-1-d7, monitoring their sleep, breathing, heart, etc. using a chest-band device such as 4-3, measuring their weight and other body metrics using a smart scale such as 4-4, monitoring their exercise, heart rate, sleep, etc. using a wrist wearable such as 3-1-d4, or monitoring their glucose with a patch such as 4-1a, where again the reader is directed to the extensive teachings provided herein.

The careful reader and those familiar with health devices will understand that many devices are available and will continue to become available for use "at-home" allowing a person to capture at least one health metric or measurement that can be useful for tracking their own "private" and current health status. What is different about the present invention is that conventional metrics or measurements are not "authenticated" to be "of" the person, where without authentication "private" measurements cannot necessarily be "publicly trusted." Thus, the present invention has several ways for authenticating each one or more "L1" measurements taken "at-home" / "in-private" by person 1 using a given "authenticating" health measurement device, where the device includes the necessary authentication apparatus and / or otherwise follows the necessary authentication methods all as taught herein, or with variations that will be obvious to those skilled in the art based upon the teachings herein.

A careful reading of the present invention will show that what are depicted as steps 161 "Use App to confirm personal ID and Device / Measurement in combination" and 162 "Take controlled measurement with Device" can also be considered a single continuous step, for example conducted over a continuous duration of time. Step 161 for confirming the person 1's ID, thus "authenticating" the measurement is given a separate focus because of its importance in differentiating the present invention. Hence, many current devices are currently available in the marketplace for use in "taking a personal health measurement" / metric (step 162), but again these devices cannot also confirm or authenticate the measured health data (step 161).

Of the many teachings providing herein, it should also be understood that some of the teachings provide means for authenticating a personal-use device which then remains authenticated until some future event (such as unclasping of a wearable, or the loss of the detection of an authenticated heartbeat), such that step 161 can also be seen as a form of "initiating" a sequence of on-going personal measurements in step(s) 162 that do not require additional authentication of step 161 but rather "remain authenticated," all as a careful reading will show.

After completing an "L1" type personal measurement in step 162, App 2a, in step 164 "Receive validated confirmation of Service" App 2a may optionally receive from a healthcare device used during step 162 some form of transaction receipt or otherwise "certificate of authenticity." This certificate is similar in concept to "proof" provided by a healthcare provider (such as a vaccination card with the person 1's name on it) that for example a vaccination was indeed provided to the person 1, where authenticity information regarding the device as a provider might include: 1) the type of device and even serial number or encrypted digital ID, 2) date, time, and other related data concerning the measurement(s), 3) supporting measurements, such as non-person 1 sensed data that serves to validate the environment (such as current room temperature, humidity, light levels, etc.) and non-measurement but still person 1 sensed data that also serves to validate the state of the measured person 1 (such as skin electrodermal properties).

While it is anticipated that a person 1 especially using a Regiment 2d might be acquiring significantly more "L1" type measurements, the amount of data required to substantiate or "confirm" (step 164) an "L1" measurement is anticipated to be minimal. In any case this provision of receipt step 164 is considered optional for the "L1" path steps 160 - 164, or at least less critical than for example corresponding paths starting with steps 174 ("L2") and 184 ("L3) as a careful consideration will make clear.

With or without a confirmation receipt for the measurement, App 2a in step 152a preferably stores all necessary measurement data along with any confirmation data into preferably an encrypted storage media or dataset, where this media can be local or remote and the dataset can be of any suitable form, all as will be well understood by those familiar with data storage technologies. As will also be understood, if a Regiment 2d and particular Regiement-rule(s) were referenced or otherwise employed during the steps 161 or 162, it is preferred that the data stored in step 152a comprise some indication, association or link to any such employed Regiment 2d / rule.

Like the "L1" path, a person 1 may use system 102 to determine an "L2" type measurement steps 170 - 174, where these measurements at least include taking a body fluid sample as a part of an "at-home" / "private-use" health kit, all as discussed herein and as will be understood by those familiar with health kits. Traditional health test kits can be used at-home, for example to collect typically body fluid samples such as blood, mucus, saliva, or urine, where the process includes discharging or otherwise providing the sample fluid to a transport medium / container / sample holder of some sort, where the sample is then sent along with personal information typically to a lab to be processed into health measurements / metrics. After the lab analysis is done, typically the results are made know to the person 1 and are in association to their personal information, which the teachings of the present invention consider to be "non-private" results.

As a careful reading of the present invention will show, the present specification teaches a means for authenticating in step 171 "Use App to confirm personal ID and Kit / Sample in combination" health samples taken "at-home" / "in-private," where the personal ID confirmation is accomplished for example by capturing a biometric to be compared with a registration biometric, and where Kit / sample confirmation includes: 1) using sample collection means (such as swab 80 or blood strip 84) that comprise identifying markings (80-bcd, 84-bcd, respectively) such that the App 2a can image the markings for registering the collection device with the person 1 and the particular test / sample, and 2) using sample collection means (such as a swab 80 or test strip 84) that comprise preferably passive, electronically writable data storage (for example an NFC tag), such that as a part of collecting the private samples in step 172 "Take controlled sample using Kit, provide to Service" (or in combination with step 171) a person 1 uses App 2a to write an authentication token or otherwise transaction related information onto the sample collecting device, where the written token and / or transaction information is preferably encrypted and where the sample is then "anonymously" sent off to a "Service" lab for analysis.

The Service lab results created in step 173 "Receive requested Service" where then shown to preferably be encrypted and made available in association with the authentication token or otherwise transaction data uniquely identifying the provided sample(s), such that the App 2a was then useable by the person 1 to retrieve (preferably electronically in step 174 "Receive validated confirmation of Service") the confirmed service results, where it is also preferable that these "lab results" include a "digital certificate" validating the service used and results created.

As a careful consideration will show, using the present invention 102, the person 1 is able to obtain "L2" "anonymous health kit results" including "validated proof" for virtually any body fluid "at-home" "in-private" test kit. After retrieving confirmed results in step 174, or otherwise receiving the lab results in any of many possible means of communications including traditional and even "non" or "less-anonymous" means (such as a QR code sent in a physically mailed letter where the QR code includes the encrypted results and confirmation such that substantially only the App 2a that conducted steps 171 and 172 can decrypt the information), App 2a preferably stores in step 153a some of all of the results and confirming digital certificate either or not in association with a Regiment 2d / rule.

Again, it is noted that some health kit results could be obtained by the person 1 using the present invention 102 and App 2a unrelated to a Regiment 2d, for example when taking a health kit test prior to traveling that is a requirement of a travel service or a location being visited but otherwise not associated with an (on-going) Regiment 2d, while concurrently the person 1 is also using some Regiment 2d for example to track their current "L1" symptomatic state of health. It is also possible that travel requirements specifying necessary health tests are also included as one of one or more rules in a "travel regiment" provided by the transportation service, location to be visited or a governing body.

Still referring to Fig. 21, the system 102 has been shown to be useable for collecting "L1" personal health data "at-home" / "in-private," where the results are both useable as proof-of-health and authenticated such that the proof therefore maintains the "public trust" (as not being forged, altered, faked, etc.). System 102 has also been shown to be useable for collecting "authenticated" body fluid or otherwise personal samples "at-home" / "in-private" related to a health kit, where the "L2" health kit results can remain anonymous and include a "digital certificate" as additional means of proof, also maintaining the "public trust."

System 102 as herein taught and as depicted in Fig. 21, can also be used in step 180 when "Visiting a Health Service Provider" presumably to obtain a health service. There are many types of healthcare services anticipated for use with the present system 102, where virtually any service comprises a person 1 interacting with a (second-party) service provider to receive the service, typically on-site at a healthcare facility, but with increasing regularity at-home as a part of a rising "remote-health" industry. What is important to see in that during this engagement between the person 1 and the service provider, regardless of the location of the services, the person 1 typically is identified by the service provider, where identification can often include personal identifying information as well as health-history and current symptom verification, and thus healthcare services are typically not anonymous.

As those familiar with the concept of a "vaccine passport" will understand, in the current CV19 pandemic situation, there is some desire to "officially know" or be certain for the sake of "public trust" that a given person 1, especially a traveler, has received a proper vaccination, where the "vaccine passport" is typically paper, or could be a digital certificate provided to the person 1's personal computing device such as a smartphone, but regardless this "vaccine passport" is then used by the person as "proof-of-health." Thus, at least a vaccination is a healthcare service that a person 1 would be desirous of receiving using the present system 102 whereby it is possible to achieve the benefits of a vaccine passport while maintaining personal anonymity, all as to be discussed as an exemplary use case.

It is well-known that current approaches to a vaccine passport, or "proof-of-vaccination," are not anonymous, where for example the person 1 must identify themselves to the service provider as a part of receiving the vaccination shot(s). After receiving the non-anonymous service, the person 1 then receives some form of "official proof" as issued by the service provider. If the proof is in a paper form, it has already been demonstrated that forged documents (i.e. forged "vaccine passports") are being created. If the proof is a digital certificate, it is considered much more difficult to forge and thus ultimately a better system for providing "public trust."

Using the present teachings as specified herein, a somewhat more detailed vaccination / "vaccine passport" use case is provided in relation to steps 180 - 184, where it is shown that the present teachings allow for anonymous vaccine services with fully authenticated and therefore trustable "proof-of-vaccination." The main purpose of this more detailed discussion within the summary of Fig. 21 is to highlight the novel and beneficial teaching provided herein using a currently pressing world need.

In a preferred "vaccination use case," person 1 enters a vaccination facility with their personal computing device (such as a smartphone) pre-loaded with the "Honest Broker App" thus together forming what is referred to herein as "2a" (sometimes called a "smart-ticket," although the ticketing aspect that includes proving a "right-to-access" along with a "proof-of-health" will not now be discussed and are already detailed herein). As prior mentioned, App 2a is preferably tamper-proofed, meaning that any of currently available or future techniques such as using hash-codes are implemented to ensure that the App 2a is executing properly and as intended, and without nefarious changes.

It is also preferred that person 1 has already registered with App 2a prior to entering the vaccination facility (although a careful consideration will show that what is important is that the person has registered prior to receiving the service, or alternatively registers during the controlled interaction with the service provider, preferably then in step 181 "Under limitations of Provider, use App to confirm Personal ID"). As prior discussed, this preferred "pre-registration" requirement is accommodated by either using a Regiment 2d rule, or a rule included / "built-into" the App 2a, that specifies that registration must be for example accomplished in a "Zone 1." (Again, see the present specification for a more detailed understanding.)

In one implementation, a healthcare service facility includes an electronic beacon that emits a signal (such as Bluetooth) detected by the App 2a, where the signal indicates that the facility is for example a "Zone 2" "3" or "4," but otherwise not "Zone 1." Upon receiving this signal, the App 2a will not function for the purposes of engaging a service of the service provider located in the facility unless at least one person 1 is determined by App 2a to have already registered with App 2a (where, as it will become apparent, this "at least one person 1" is the person 1 who then will be seeking a service in step 182 "Request (controlled) Service, provide other information" and must re-identify themselves to the App 2a in step 181 in the presence of the beacon signal thus authenticating the transaction).

The present invention has referred to such an electronic based "location confining" technique under the general term of a "geo-fence," where any of currently well-known or future geo-fence methods can be used, such that a beacon approach should be considered exemplary. It is also possible that the App 2a be required to connect to the facilities public (preferably secured) wi-fi network, and that once connected the App 2a functions as if it is within a restricted "Zone" (such as 2, 3 or 4, but not for example "Zone 1"), or that once connected the App 2a uses any of well-known or future wi-fi "local position determining methods" (such as "RSSI" or "AoA") to determine the App 2a's relative location within a larger facility, where this "relative location" is the smaller area in the larger facility where the vaccine service is administered, and where then the restriction requiring pre-registration of App 2a is enforced with respect to the "smaller area" inside the "larger area" based upon the detected local position, all as will be understood by those skilled in the art of "local positioning systems" (LPS). Such LPS systems are now also being implemented using Bluetooth signals that are readily available on personal computing devices such as a smartphone. However, it is again stated that it is possible that the registration step 151 or 152b could be accomplished as a part of any of "confirming steps" 161, 171, 181, where the act of first registration is "confirming."

As prior discussed in relation to step 151 "Register image and biometrics with App" (or otherwise also step 152b "Register Regiment"), it is preferred that the registering person 1 provide to the Honest Broker App 2a personal information, thus allowing the App 2a to anonymously confirming the person 1 to an access control system, i.e. as opposed to the person 1 providing such personal information directly to the access control system. For example, during registration the App 2a may require person 1 to use the App 2a (and therefore personal computing device such as a smartphone) to capture one or more pictures of themselves. After capturing the image(s) which are then preferably processed by a facial recognition algorithm within the App 2a to determine and model the person 1's relevant facial features for subsequent facial recognition confirmation, App 2a may then further require that the person use a finger-print reader preferably present in the personal computing device to also provide their finger-print. Thus, the App 2a acting as the Honest Broker will have two forms of biometrics for confirming the person 1 after registration, where this is often referred to as "multi-model" identification and is generally considered to be more accurate.

Those familiar with personal identification methods and multi-model identification will also understand that its possible, and even beneficial to the person 1, that the App 2a determine additional forms of authentication, including but not limited to other forms of biometrics, where the biometrics may be sensed using the personal device and App 2a, or sensed using a "connected device," for example that takes a finger-print such as wired or wireless finger-worn device 3-1-d9. Myris currently sells for example an "iris authenticator" marketed as EyeLock that connects to personal computing device via USB, and thus could be used to provide iris biometrics to App 2a. A company called Bionym makes a product that uses an advanced electrocardiogram (ECG) to determine a person 1's unique heartbeat characteristics. Smartphones continue to advance to include more and more "authentication" sensors, especially for use in "locking" and "unlocking" the smartphone to maintain the person 1's secured data privacy. Some smartphones, such as the Fujitsu ARROWS NX F-04G can directly perform "iris authentication." Smartphones may also be used to create voice prints, for example where the person 1 reads a given text and their voice is analyzed for its unique characteristics.

While some forms of biometrics are more accurate than others, each additional next biometric increases the overall accuracy of the Honest Broker App's "identification" of a given person 1. As a careful consideration of the present invention will show, each next form of biometric should be / is preferably repeated during "confirmation" of the person 1 to commensurately increase the accepted "accuracy of confirmation," such as during step's 161, 171 and 181, or the biometric as captured during registration will essentially add no benefit to the App 2a's confirmation function. Given this understanding, some service providers and access control systems may have rules requiring more or less forms of biometric identification to be used by the Honest Broker App 2a, where the rules even adapt or change based upon some other aspects of the person "known" to the Honest Broker App 2a such as other registration data, or based upon changing situations at the service provider or premise / gathering using the access control system, all depending upon the security needs of the service providers and access control systems. This need for "rule adaptation" highlights an advantage of the present teachings of using Regiments 2d, where the rules can be externalized from the App 2a and more easily changed and adapted overtime.

Beyond forms of biometrics, App 2a may alternately be adapted to require a "pin number" or "password," or even a set of challenge questions and answers. What is important to see is that the Honest Broker App 2a during registration can be selectively given (or required by a Regiment 2d) multiple pieces of information that are useful and sufficient for later "re-identifying" or "confirming the identity of" a person 1, where these pieces of information (including biometrics) are otherwise personal information and remain as encrypted data accessible only to the App 2a.

Still referring to Fig. 21, in the example vaccination use case, the person 1 has provided a facial image, a fingerprint, a password and three challenge questions and answers to the App 2a during registration, preferably in a "Zone 1" that is different from a for example "confirmation Zone 3" that is the preferably electronically detectable Zone associated with the vaccination location within a facility (although this "confirmation Zone 3" could simply be implemented as a queue of persons where ultimately the next person 1 to be serviced is "isolated" in front of an agent, all as will be understood from a careful consideration of the present teachings). When the person 1 arrives or is otherwise substantially within the confirmation Zone 3, the App 2a uses for example the wi-fi or Bluetooth apparatus internal to the personal computing device to preferably sense the Zone 3, enable the service provider (steps 181-184) functionality, and then preferably also connect with the service provider's secured communications (a wi-fi network).

The person 1 is presumably then waiting in a queue to receive a service and ultimately approaches an agent of the vaccination service. Eventually, still in the Zone 3 and now in the visible presence of the agent, the person 1 is directed by the agent to initiate a "identity confirmation" using their App 2a. Person 1 then uses the App 2a (whose screen is not necessarily being viewed by the agent) to follow prompts and capture their image and fingerprint, where the App 2a then reconfirms the person's identity by comparing the currently captured image and fingerprint to the registered image(s) and fingerprint(s). App 2a may also ask the person 1 to enter their "pin code" or password, although as a careful consideration will show, a "pin code" requirement alone does not strictly confirm a person 1's identity as a pin codes is easier to steal than faces and fingerprints are to be mimicked.

**It** is also possible that the App 2a in communication with the service provider's wi-fi network and therefore preferably a computing device such as a networked computer being currently used by the agent, provides the agent will the total number of challenge questions pre-registered with the App 2a, for example where the agent's networked computer indicates that "3 questions are available." The agent then enters a number such as "2" which is communicated back to the App 2a, where then App 2a challenges the person 1 to answer "challenge question 2" by for example entering the answer as text. If the answer is correct, the App 2a then provides a communication to the agent over the facility's network that confirms for example that: 1) the facial image matches (without giving a facial image), 2) the fingerprint matches (without giving a fingerprint), 3) the password matches (without giving the password), and 4) the challenge questioned answered correctly (without giving the question or the answer).

Again, as a careful consideration will show, many variations are possible, where it is important to see that the App 2a is acting as the "Honest Broker" to confirm that the person 1 standing in front of the agent matches the person 1 registered to the App 2a, and / or to the Regiment 2d associated with the vaccine passport, all while the agent is watching and therefore can see that no other persons are entering or providing information to the Honest Broker App 2a. As a careful consideration will show, it is not necessary that the agent themselves ever confirm any of the identifying information used during registration and then during this confirmation step, only that the agent confirms that the connected App 2a is an accepted App 2a, perhaps even requesting that the App 2a provide its own digital certification or otherwise encrypted "proof-of-self"/ "proof-of-integrity" such as a recomputed current hash code, where the information provided by the App 2a is then used by the agent's system to confirm the validity of the Honest Broker App 2a such that the App 2a's authentication / confirmation of the person 1 essentially standing in front of the agent can be accepted ("trusted") by the agent.

In some variations, "password matches" confirmation (3) and "challenge questioned answered correctly" confirmation (4) are omitted, where a careful consideration will show that the facial (1) and fingerprint (2) recognition by App 2a will provide significant assurance to the agent that the person 1 they are currently looking at is the registered person 1 (where other biometrics could be added to further increase confirmation accuracy as discussed). Thus, the present use-case example should be considered exemplary, as many variations are possible without departing from the true scope and teachings of the present invention.

For example, it is also possible that the App 2a does not reconfirm the person 1 at all, meaning that App 2a does not require the person 1, while standing in front of the agent, to capture a facile image or enter a fingerprint, but rather the person 1 is instructed to hold their personal computing device's screen such that the agent can see the screen. The agent then enters a code or otherwise makes and indication via the user interface on their service provider's computing device, where then the service provider's computing device communicates to the App 2a, and the App 2a displays this same code or symbol, or some algorithmic transformation thereof, on the App 2a's (personal computing device's) screen, while preferably at the same time showing an image of the pre-registered person 1. If for example response code provided by the App 2a is based upon the combination of the agent / service provider's initial code plus the operation of an App 2a algorithm, then it is also possible that this response code is provided as data to the service provider's system that then validates the response code as correct, where many variations are possible without departing from the scope of the invention.

The agent then looks that the image of the pre-registered (or essentially "prior registered" person 1) and confirms for themselves that this is the same person that is currently standing in front of them. The agent also then sees the unique code they (the agent) caused to be transmitted to and displayed by the Honest Broker App 2a, proving to the agent that the App 2a is the app displaying the person 1's image, all as a careful consideration will show. Again, what is important to see is that the Honest Broker App 2a can itself be verified, where then the verified App 2a can in one of many ways either verify the person 1 or provide information to the agent in an anonymous manner that the agent can then use to verify the person, or some combination thereof.

Still referring to Fig. 21 and step 181, it is also possible that App 2a provides a 2D barcode including for example information such as the App 2a's unique digital certificate or other confirming data, where the agent then uses a barcode scanner (or otherwise appropriate technology) to read and decrypt the App 2a's barcode and thereby confirm the App 2a. This 2D barcode can be in addition to or in replacement of a secured wireless transmission from the App 2a to the agent's computer system comprising identifying information, as prior discussed. One advantage of using the 2D barcode is that the App 2a then does not require an active network connection to the agent / service facility's network. After receiving and confirming the App 2a's 2D barcode that "proves" the integrity and identity of the Honest Broker 2a, the agent then receives confirmation of the person 1's identity, perhaps as an additional 2D barcode or by any of the means prior stated or as will be clear to those familiar with security systems, of which there are many variations.

As a careful consideration will show, some of the means for the App 2a confirming the person 1's identity use a network connection, while others do not, where not requiring a network connection has advantages and disadvantages and as such the system 102 can be configured according to the many needs of the person 1 and the service provider. In all of the many possible configurations, the person 1's personal information is never shared, including a biometric, such that the person 1's anonymity is protected.

In brief recap, in step 181 person 1 is preferably standing in view of the agent, or otherwise being positioned or cordoned off (by any of many possible means and variation of means) such that the agent is able to recognize / confirm / "be assured" that only the person 1 has access to the agent's "agent" (which is the Honest Broker App 2a). In step 182 the "isolated" person 1 preferably first confirms the (currently unconfirmed) App 2a to the agent (for example by presenting a digital certificate in the form of a "QR code" / encrypted barcode or in the form of an electronic communication preferably secured and wireless), and then second confirms themselves using the (now confirmed) App 2a, providing this second personal confirmation of their valid identity to the agent via preferably similar communication means as the first App 2a confirmation.

Those familiar with software systems will understand that many variations of step 181 are possible, for example where the person alternatively first confirms themselves with and to the Honest Broker App 2a while "properly" isolated without first confirming the App 2a to the agent. After first confirming themselves to App 2a, the person 1 then uses App 2a to communicate with the service provider a simultaneous confirmation of both the App 2a's identity (and optionally also integrity) as well as the person's 1's confirmed identity, where then this "single confirming" act is accomplished for example by displaying an encrypted visual code (such as a QR code) or by communicating a certificate over the service providers wi-fi network or alternatively a Bluetooth connection, where many variations are possible without departing from the teachings provided herein.

Still referring to Fig. 21, in step 182 "Request (controlled) Service, provide other information" the person 1 either explicitly (by speaking with the agent or using the App 2a to select a choice and transmit) or implicitly (simply by being "next-in-line") requests a service such as a vaccination shot. In the step 182, the agent may then ask the person 1 questions or have them fill out documentation, where it is noted that it is possible and anticipated that at least some of the agent's requested information can be personally identifying information (such as a name and address).

The system 102 provides for an alternative means of providing service-related information to the agent where for example the agent causes the service provider's system to communicate sufficient information to the App 2a such that the person 1 uses their personal computing device and App 2a (rather than for example paper forms provided by the agent) to provide all requested information. After the information is completed by the person 1 using App 2a, the App 2a can then provide to the agent an electronic communication with the requested information except for the personally identifying information.

Hence, to maintain the person 1's anonymity, App 2a provides a unique code identifying the person 1 along with the requested information but otherwise no personal information. Other than a unique code identifying the person 1, it is alternately possible that the App 2a simply provides a unique transaction number, either as preferably first supplied by the service provider's system, or as generated by the App 2a, where other variations will be evident to those skilled in the art of security systems. In any case, by using the present system 102 and Honest Broker App 2a means described herein, it is possible that the App 2a serves as a go-between / intermediary for verifying to the agent that the person 1 did provide the requested information while also providing only that information that is considered as "not personally identifying," thus maintaining that person 1's anonymity.

Still referring to Fig. 21 and the present use-case flow of a person 1 requesting a vaccination, some reasons for the service provider maintaining personally identifying information relating to person 1 include: 1) uniquely tracking the provision of the vaccination, for example because it is a first of two or more shots where the later shot should be provided on a certain day to the same person, 2) providing some sort of personalized documentation to the person 1 as "proof-of" the vaccination service, such as a "vaccination card," and 3) having record of each uniquely served person 1 for reporting to a governing agency, an insurance agency, or other vested parties to the health service transaction. As a careful consideration will show, the presently taught system 102 provides for each of (1) uniquely tracking the vaccination, (2) providing "proof-of-vaccination," and (3) supporting recorded keeping with vested parties, all while maintaining person 1's anonymity.

Specifically with respect (1) "unique tracking of the vaccination" (such as a first shot of two), the Honest Broker App 2a provides a "unique code" as described above representing either or both of the person 1 and / or the "service transaction," where this code preferably is combined with other related data such as the date, time, location and other details of the service provided. Upon receiving this code (and other related data) via some means such as a visually displayed QR code or an electronically communicated "transaction dataset," the service provider's system is then able to store this uniquely "tagged" information, where those familiar with databases and software will understand that a tag (or "key") is a means for future data retrieval, and where preferably the tag is unique, such as a unique transaction code.

As a careful consideration will show, the agent might then schedule for example a second shot providing this scheduled date / time and other related data back to the App 2a. The App 2a then serves to store this information and to be a reminder system for the person 1, or perhaps "post an appointment event" to any of many possible calendar systems (such as Outlook or Google Calendar) being used by the person, all as will be well understood by those familiar with application integration. In any case, when the person 1 returns at some future date / time to request and receive a second shot, the Honest Broker App 2a provides this identifying transaction code to the agent / service provider's system (and not any personally identifying information), where the agent using the service provider's system is then able to retrieve the necessary stored information for example using the transaction code as the "lookup key" or "tag," as will be well understood by those familiar with database systems. Thus, the function of (1) "unique tracking of the vaccination" is accomplished by system 102 without compromising any of the person 1's personal information, hence maintaining anonymity.

Specifically with respect to (2) "providing proof-of-vaccination," as prior discussed and as will be discussed again with respect to upcoming step 184, after providing the service such as a vaccination shot, the agent uses the service provider's system to preferably generate a unique digital certificate along with related transaction data (such as date, time, location of service, type of service, service provider code, agent code, etc.) that is provided to the App 2a for preferably encrypted storage (see discussion of step 153a). It is noted that this unique digital certificate and related transaction data need not include any information personal to the person 1, but rather can be trusted based at least upon the App 2a's confirmed identity, the transaction code known both to the App 2a and the service provider (but preferably not to the person 1 or any other person such as the agent), and the implemented means and methods of system 102, for example such as "isolating" the person 1 and App 2a within a "geofenced" or otherwise "confined" / "monitored" location during the entire process and then using communication means that are preferably encrypted and limited to the confined location and thus are not easily "eavesdropped" or "spied upon" by others looking to electronically copy the digital certificate and related data.

As a careful consideration will show, even if the digital certificate is copied, the certificate preferably includes an encryption of the App 2a's unique ID such that any other valid Honest Broker App 2a or other "imposter App" not also knowing the unique App ID of the person 1's App 2a will be unable to use the certificate, where using presumably includes providing a matching App ID along with the valid digital certification from the service provider that includes an encrypted copy of the App ID. Those familiar with public / private key encryption will also understand that it is possible that the encryption of the App 2a's unique ID provided by the service with or within the digital certificate can remain "not decryptable" by the App 2a (because it does not have the necessary "key"), but decryptable to some third-party attempting to confirm that the App 2a's ID does match the unique ID provided by the service within the certificate. Thus, the function of (2) "providing proof-of-vaccination" is accomplished by system 102 without compromising any of the person 1's personal information, hence maintaining anonymity.

Specifically with respect to (3) "supporting recorded keeping with vested parties," as a careful consideration will show, at least a governing body such as a state or federal agency, need not necessarily know which individuals are being vaccinated in order to accumulate useful information such as the rate of vaccinations (or any service provided), etc. During the current pandemic, many individuals are expressing a concern that some one or more governing agencies will be accumulating information including personal information with regards to a "vaccine passport," where the additional concern is that this passport will naturally grow in use and scope beyond vaccines (an example of "L3" data) and into other health (or related) data (such as "L1" and "L2" data), similar to the present invention 102 that tracks virtually any type of health measurement, whether made by the person 1 ("L1" flow 160 - 164), using a health kit with lab services ("L2" flow 170 - 174) or using a service provider ("L3" flow 180 - 184).

With respect to the vested parties (3), what is more problematic is any money related parties such as an insurance company that might be reimbursing the service provider for the provided service. In such arrangements, it is typical that the person 1 identifies themselves and their "insurance plan" "policy" or other similar information to the service provider's agent prior to receiving the service. Typically, the agent of the service provider uses the provided insurance information to confirm that the person 1 does in fact have sufficient coverage for the service. Thus, using this more traditional approach, the person 1 must necessarily provide personal information to the agent / service provider.

However, as a careful consideration will also show, it is possible that the Honest Broker App 2a conduct an "approval process" communication with a system controlled by or representing the insurer, such as an intermediary system or some system for example implementing what is known as "blockchain" that can maintain anonymity during a transaction. Hence, the Honest Broker App 2a is also acting as a go-between the insurer, insurer's agent, or otherwise an intermediary and the service provider, where the App 2a communicates an "insurance approval code" along with any related information for example necessary to allow the service provider to be reimbursed for the provided service. Thus, the function of (3) "supporting recorded keeping with vested parties" is accomplished by system 102 without compromising any of the person 1's personal information, hence maintaining anonymity.

Still referring to Fig. 21, in step 183 "Receive requested Service" the service provider provides the requested service (such as a vaccination shot) to the person 1. During this step 183, it is likely that the service provider will record the provision first as data entered into the service provider's system for tracking. Using this record of provision, an agent of the service provider preferably then allows for, initiates or otherwise causes a "services receipt" to be provided to the App 2a in step 184 "Receive validated confirmation of Service." This receipt-of-service preferably includes information usable by the App 2a as "proof-of-service" on behalf of the person 1 for example during a step 156 "Confirm ID, Provide Health Status to Access Control System," where the Health Status is broadly defined herein to be comprehensive of any one of or any combination of "L1," "L2" and "L3" health metrics / measurements / services provided, where for example the provision in step 184 of a "digital certificate of vaccination" from a service provider is an example of providing a "Health Status" in step 156.

Referring next to step 153a and briefly recapping prior discussions, the App 2a preferably stores in an encrypted format, preferably on a locally accessible data store, all necessary health measurements, metrics, related data and confirmations of service determined by system 102 with respect to the "L1," "L2" or "L3" "flow paths" of the presently discussed Fig. 21. As will be clear from a careful reading of the present specifications, preferably this "personal health information" will remain privileged and private to the person 1 and only accessible by that person 1's App 2a, where the App 2a only provides to the access control system (see step 156) the minimal (but "publicly trustable") data required (such as a "yes/no" "symptomatic" (L1) status, or "yes/no" "passed health kit" (L2) status, or a "yes/no" "received vaccination" (L2) status, all without sharing any personally identifying information.

Still referring to Fig. 21 and now to optional step 153b "Update Anonymous Intermediary recording service," in general it is beneficial that a person 1 might desire their health data to be anonymously stored in a remote encrypted database, where the person 1's health data is for example encrypted using a private key held by only App 2a such that only App 2a could decrypt the person 1's remote health data, all as will be understood by those familiar with encryption technology. The data is anonymized by not including any personal information, also as well defined at least in relation to what are referred to as the "Hippa Laws" ("Health Insurance Portability and Accountability Act), all as will be understood by those familiar with data privacy issues.

It is preferred that the person 1's health data stored on the remote or "intermediary" database is stored in relation to an authentication token provided by the App 2a, where a function of the "intermediary" is to ensure that only an App 2a providing a matching authentication token can retrieve the associated and prior stored health data of the person 1, where other intermediary functions and verifications can also be performed such as ensuring that any App requesting to retrieve data first prove their own "Honest Broker" authenticity and integrity using for example any of digital certificates, hash codes and other well understood or yet to be created techniques, all as will be understood by those familiar with the art of digital rights management and otherwise app verification. Thus, in step 153a App 2a may store any of "L1," "L2," and "L2" data, and otherwise any of data stored during step 153a "Update Health Data, Regiment," as a means of providing safe keeping and backup against data loss of what is otherwise not centralized information.

Still referring to Fig. 21, steps 154 through 159 represent the flow of interactions and key functions whereby a person: 1) assures a premise or gathering of their identity and current health status, 2) possibly receives assurance of the health status of the premise or gathering (i.e. being a collective status of all other persons 1 of any kind such as customer, vendors, workers, guests, etc. currently tracked or otherwise known to be present in the premise or at the gathering), and 3) gains entry to the premise or gathering based upon sufficient health status as deemed by the access control system of the premise or gathering. Thus system 102 implements an anonymous, authenticated, and decentralized mutual assurance system for governing a person's access to a premise or gathering based at least in part upon their health status.

In step 154 "Seek Access Rights?" a person 1 decides to determine if all other matters being acceptable, they will or will not likely be granted access to a premise or gathering upon request, where "other matters" for example can include having a proper ticket (also addressed herein but not with respect to Fig. 21), being properly attired such as wearing a face mask, or any other rules established by the premise or gathering. It is noted that due to the many novel teachings of the present invention, this step 154 can be conducted for example while a person 1 is still "at-home" or otherwise distant from the premise or gathering (such as hours to days away from the intended visit, see the present specification). Hence, the person 1 uses App 2a to make a preferably electronic connection to the access control system of the premise or gathering they intend to visit at some presumably near-future time, where the App 2a "Determines the Current Health Status" of the person 1 (like step 155) and then provides this Current Health Status to the access control system (without necessarily providing confirmation of identity via the App 2a, thus not fully complying with step 156 to be discussed shortly).

While not specifically shown as essentially a variant of step 155, a careful consideration will show that this ability to "pre-confirm" that a person 1 is likely to be granted access provides significant advantages to the marketplace, such as: 1) saving a person 1 travel time should their current health status be deemed "currently insufficient," 2) avoid unnecessary exposure of a possibly infected person to other citizens during travel to a premise or gathering, where using current methods and technologies travel (with possible exposure) is required so that the person 1's health status can be determined / verified locally at the premise or gathering, thus providing "public trust" in the assessment, and 3) help the person 1 and the premise or gathering with their scheduling, especially if a prior appointment should be changed based upon the "pre-confirmation" step, where other benefits will be made clear by a careful consideration of the present specification.

Whether or not a person 1 has pre-determined a likelihood for grant-of-entry to a premise or gathering in combination with the decision of step 154 to "Seek Accessing Rights?", the person 1 at some time presumably travels to the premise or gathering to attempt entry. At the premise or gathering, like the discussion in relation to step 180 and especially 181 "Under limitations of Provider, use App to confirm personal ID," there are many possible ways of "confining," "limiting" or otherwise "restricting the location of" a person 1 while person 1 is using a personal computing device and app 2a to gain entry (for example by using a geofenced "confirmation Zone 3" as taught in the Prior Art relating to the confirmation of a ticket / right-to-access, and as reviewed and expanded upon herein). What is important to see is that like an airport security-line checking passports, there is an advantage for an access control system to essentially "deal with one person 1 at a time."

In a step 155 "Determine current Health Status" the isolated person initiates a process using App 2a to generate a current health status preferably under the rules of a Regiment 2d recognized as acceptable by the premise / gathering. While it is possible that this "current health status" is essentially constantly maintained by App 2a (for example every period such as a day, half-day or hour, and / or every time new "L1," "L2," or "L3" health data is generated), as a careful consideration will show it is beneficial for the assurance of the access control system to determine the Current Health Status of the person 1 while the person is attempting / seeking physical entry and "isolated," or at least to some extent in a controlled queue leading to an entry point (see teachings herein).

It is also preferred that the App 2a generate a time stamp to go with a digital "current health" certificate, where the premise / gathering for example is also providing an electronically detectable access code or validation code, and the App 2a detects this code when substantially properly isolated and writes the code onto the "digital health certificate" along with the current time of health certificate determination. (Those familiar with the use of "digital certificates" and what are being called "digital health passes" will understand that the term is herein being generalized to mean preferably encrypted data that presents information supporting authenticity, confirmation of identity, confirmation of integrity, etc., for example of the App 2a, of the person 1, or of the person 1's "current health status.")

In a step 156 "Confirm ID, Provide Health Status to Access Control System" the person 1 while preferably still "isolated" by any of various possible means uses App 2a to provide one or more biometrics for comparison to the biometrics provided during a prior registration, such as made in a step 151 or 152b, where isolation assures the access control system that only the person 1 is able to interact with the Honest Broker App 2a (where the careful reader will see that the App 2a is acting on behalf of both the access control system and the person 1 as a "go-between"). This step 156 provides sufficient data that typically includes at least biometric data for the "confirmation" of person 1's identity, where this sufficient data is provided exclusively to the Honest Broker App 2a, but is otherwise not shared with the accessing control system, thus time maintaining person 1's anonymity.

After the person 1's identity is confirmed by the Honest Broker App 2a, and still in step 156, the App 2a provides / communicates sufficient but preferably anonymous "person confirmed" information to the access control system, using any of sufficient means such as but not limited to the discussions related to step 181, where preferably sufficient information is in the form of an encrypted "digital health certificate" or "encrypted proof-of-health." This sufficient information preferably includes: 1) proof-of-authenticity and integrity for the App 2a, 2) an indication of the confirmed health status of the person 1 (such a calculation made with respect to the analysis of any "L1," "L2" or "L3" data, and / or an copy of for example a healthcare provider's certificate of service provided with respect to "L2" or "L3" data), where the indication may provide details including a controlling Regiment 2d and some classifications, for example "asymptomatic for last 14 days," "passed health kit test xxx," and "received full vaccination, vaccination in full effect," and 3) an indication that the identity of person 1 has been confirmed by the App 2a, and then by implication confirming that the provided health status pertains to the identified person 1.

Still referring to Fig. 21, as the careful reader will see at least steps 155 and 156 can be considered as a single step, or in some sense reordered. For example, the person 1 may "confirm their ID" first to App 2a while preferably isolated, after which the App 2a sensing electronically provided indications from the access control system automatically processes the person 1's health data to provide a requested health status (thus the person does not necessarily cause or prompt the App 2a to generate the current health status). As will be clear from a careful reading, the present system 102 provides multiple apparatus and methods, where many variants are possible, such that the present discussion in relation to Fig. 21 should be considered as exemplary and limited in detail for the sake of clarity and should not be considered as limiting the functions of system 102.

What will also be clear is that system 102 does operate to provide mutual assurance as defined herein, and that the assurance is provided while maintaining person 1's anonymity, where the person 1's health status includes health data that is "publicly trustable" but otherwise was not determined by for example by the access control system (such as a remote IR temperature check of the person 1), where this health data can therefore be considerably more extensive that any data that can reasonably be gathered during a traditional access grant process, where the traditional process must necessarily determine and / or confirm each "piece" of health data (which is especially problematic when trying to confirm if a person 1 is "symptomatic," a determination that reasonably requires many measurements preferably made over the prior 7 to 14 days), and where the details of the health data need not be disclosed but rather can be reported as a "current health status" / summary / "yes/no" "pass-fail."

Still referring to Fig. 21, in step 157 "Receive Approval / Denial" the access control system processes the information provided in step 156 and either approves or denies access to the person 1. (It is noted that a "denial-of-entry" is an uncomfortable event for both the person 1 and the agents of the premise / gathering, which highlight's the benefit of system 102's ability to provide preliminary denial prior to the person 1 even before "leaving home," all as prior discussed.)

In an optional step 158, person 1 uses App 2a to also determine a current "collective" health status of the premise / gathering, where using the current pandemic concerns as an example, this status might include the number of people already allowed access the premise / gathering versus the legally allowed limit, and the percentage of these people that are for example: 1) "a symptomatic," 2) "passing a health test within the last 48 hours," 3) full vaccinated, and 4) wearing masks, as well as for example the 5) average social distance being maintained between persons within the premise / gathering. If the health status of person 1 is approved by the access control system, and if person 1 likewise approves of the health status of the premise / gathering, then presumably in step 159 the person 1 "Proceeds to Access if Approved."

And finally, still referring to Fig. 21 and now also the teachings of the entire specification, the presently taught mutual assurance system 102 can be considered as comprising a multi-part algorithm where: 1) the first-part algorithm operates as the Honest Broker App 2a for exchanging information with the second, third and fourth-part algorithms, 2) the second-part algorithm operates in cooperation with the first-part to determine authenticated health measurements, 3) the third-part algorithm operates in cooperation with the first-part to regulate grant-of-access based upon anonymous information derived from the authenticated health measurements determined using the second-part, and 4) the forth-part algorithm operates in cooperation with the first-part to store, maintain or communicate anonymous person 1 health data or derivative analysis to other related parties including the person 1.

Using this numbered "part" nomenclature to describe system 102's structure, the first-part algorithm relates to what is also referred to as the "smart ticket" 2, where the smart-ticket 2 comprises various combinations of a mobile device with app 2a and a paper ticket 2b or an electronic ticket 2c, where the mobile device is also referred to as a "personal computing device," and where the tickets 2b and 2c are related to the "right-to-access" of a person 1 using app 2a, which "right-to-access" is combinable with the "proof-of-health," where only the "proof-of-health" is summarized with respect to Fig. 21. The smart-ticket 2 provides significant technical advantages and marketplace benefits without implementing any "right-to-access" functions, thus "proof-of-health" functionality is accomplished and beneficial separately from "right-to-access" functionality, where "proof-of-health" functionality does not require the use of tickets 2b and 2c. At least the mobile device with app 2a is also referred to as acting as an "Honest Broker" and preferably uses "regiments" 2d comprising rules, where the regiments 2d are separate, and separately updatable, from the app 2a, and where a regiments 2d's rules implement a sufficient health standard for achieving "grant-of-access" to a premise or gathering.

The combination of anonymous, authenticated and "publicly trustable" "right-to-access" and "proof-of-health" is herein taught and shown to have significant advantages over the current state-of-the-art that trades-off the anonymity of the person 1 in exchange for "public trust," largely because of the many shortcomings with respect to the current means for the authentication of a person 1's "right-to-access" and "proof-of-health."

Using this numbered "part" nomenclature to describe system 102's structure, the second-part algorithm relates to what is also referred to as "self-operated health measurement devices" 3-1 and "other-services and devices" 3-2, where for example self-operated devices 3-1 include a finger-worn device such as 3-1-d7, 3-1-d8, 3-1-d9 and 3-1-d10, a wearable such as chest-band 4-3 or wrist-band or health-watch 3-1-d4, 3-1-d5, and 2-3, a body-adhesive device such as a patch 4-1a-1 and 4-1a-2, a breath analyzer such as 4-2, a smart-scale such as 4-4, and an active mask such as 130, 131, and where other-services and devices 3-2 additionally comprise services related to health-kits such as 80 and 84, or otherwise healthcare provider 42 services. Taking authenticated measurements using devices 3-1, 3-2 is discussed in detail with relation to the flowchart of Fig. 13, while taking health kit samples using apparatus 80, 84 is discussed in detail with relation to the flowcharts of Fig. 14C and 16.

Using this numbered "part" nomenclature to describe system 102's structure, the third-part algorithm relates to what is also referred as "access control systems" using devices such as kiosks 3-2 and 3-3, operating with areas such as "confirmation Zone 3" and "premises Zone 4" with "private controlled health-verified access" means implemented as 5a, 5b and / or "non-private controlled health-verified access" means implemented as 5a, 5b, using guarded systems such as 103 and 104, and using appointment services such as 105. Allowing mutually assured and personally anonymous health-verified access to a premise or gathering based upon the combination of an authenticated "right-to-access" and an authenticated "proof-of-health" is discussed in detail with relation to Fig. 8. Allowing "ticketed" and anonymously authenticated proof of "right-to-access" when accessing a premise or gathering using an access control system is discussed in detail with relation to the flowchart of Fig. 19.

Using this numbered "part" nomenclature to describe system 102's structure, the fourth-part algorithm relates to what is also referred as a "secure remote database" such as 102-db, and an "anonymous intermediary recording service" or simply an "intermediary."

### CONCLUSION AND RAMIFICATIONS

Thus, the reader will see that the present invention accomplishes its objective of teaching a mutual health assurance system comprising apparatus and methods for: 1) providing assurance to an entity that a person desiring to access a premises has both a validated / authenticated right to enter the premises and a validated / authenticated health status, and 2) providing assurance to a person desiring to access a premises that the premises have a validated / authenticated aggregated health status. While accessing has been described as a person entering a premises (ZONE4), as will be clear from a careful consideration, the present invention is useful for also assuring that material deliveries, just like persons, can be required to have a validated health status prior to entering a premises, where at least the validated health status of any one or more persons making the materials or providing handling and delivery of the materials is associable with the material deliveries for at least in part forming the validated health status of the materials. Likewise, the present system can be used to assure a person taking possession of a material being provided by a premises that the material at least conforms to a validated health status by virtue of the validated health status of the premises and / or the specific persons associated with the making, handling or delivery of the material. Examples of an entity receiving health validated materials include product deliveries from a carries such as Walmart, Amazon or UPS, where for example the health of the UPS driver(s) are associated with the delivered product, even if the driver(s) themselves do not require entry onto the premises. Examples of a person receiving health validated materials include a person receiving a take-out order from a restaurant, even if the person does not enter the restaurant to receive the take-out order.

The present invention taught that a validated health status was predicated on one or more measurements taken in accordance with one or more health regiments, where the health regiment is provided by any organization including the entity owning or otherwise controlling the premises or a public health organization including a government organization. Health regiments can be combined, can be conditional and can be updated or overridden, all as will be well understood by a careful consideration of the teachings herein and of the purpose and uses of a health regiment. Health regiments may be for a short period of time such as leading up to a planned visit to a premise, or for an on-going period such as necessary for providing continuing on-going access.

Health measurements were shown to be determined by health measurement devices capable of determining any one or more metrics including biometrics of a person or environmental metrics. The devices were shown to be capable of self-operation or other-operation, where other-operation included a healthcare provider performing a health service.

Various combinations of health measurement devices and smart-tickets were shown that both determined one or more health measurements and validated that the identity of the person about whom the measurements were determined sufficiently matched the identity of the access rights holder, such as a ticket holder or work pass holder. The present invention was shown to be useful when the premises does not otherwise issue tickets or require validated access rights but would still benefit by being able to ensure that any person desiring to enter a premises has a validated health status, for example including a grocery store or restaurant.

Health measurement devices were shown to communicate wirelessly or through a wired connection to a smart-ticket. Health measurement devices were also shown to require contact with the person or being able to determine measurements without contact, even being shown implemented as a kiosk. Health measurement devices were shown to include means for verifying the veracity of their measurements with further adaptations to measure secondary pre-known properties, such as an electrical property in regard to a surface such as the skin or a supplied pre-known validation surface.

Device ID means were taught for incorporation in a health measurement device for at least in part assuring that a health measurement was being determined and / or provided by a validated device. Person ID means where taught for incorporation in a health measurement device for at least in part assuring that a health measurement was being determined about a validated person. Personal validation information concerning one or more persons was shown to be communicated by the system to a health measurement device prior to the taking of a health measurement and then reusable by the health measurement device for taking multiple measurements over time of any one or more persons, where the health measurement device was able to self-validate the person for each subsequent measurement.

Combination devices such as a wrist-worn wearable was taught to provide some of the functions of the smart-ticket and some of the functions of a health device. Using the mutual health assurance system it was shown that substantially 100% of all contacts between persons could be tracked by an entity regarding a premises, and that the health of persons could continue to be tracked especially while staying within a premises for an extended period of time and even after the person left the premises, and that at any point that a first person's health was determined to be adversely changed, other persons having come into contact could then be identified with substantially 100% assurance. As will be clear by a careful consideration, without 100% assurance of all contacts, it is possible that a tracked person experiencing an adverse health condition could come into contact with a person not being tracked for contact, where the tracked person could then potentially be affected by the adverse condition and also further transmit the adverse condition.

The mutual health assurance system was shown to be beneficial for collecting detailed location tracking information about persons in a premises substantially in real-time, where this information was at least in part usable for influencing the flow of persons, which in turn has the beneficial use of reducing person-to-person density thereby measuring and effecting average social distance. The same tracking data was discussed as desirable for use by an interactive gaming system, where it will also be understood by those skilled in the art of people management that compliance typically increases when people are positively incentivized, such as by playing a game that for example includes rewards for positive health behaviors such as maximizing social distance.

As will be well understood by a careful reading of the present invention and by an understanding of technologies for contact tracing, the present substantially 100% contact tracing assurance system can also be used to create a "social distance score" including for example the number of total contacts, new contacts and even a calculated average proximity, where the closeness of the proximity can be measured for example by a number of means well known to those familiar with GPS and LPS such as wi-fi and Bluetooth technologies including the measurement of signal strength during contact. It is even possible then that this measure of closeness can be associated with each contact providing a greater understanding or "typing" / classification of each person-to-person contact, e.g., "within 3 feet" vs. "from 3 to 6 ft."

The presently taught mutual health assurance system was shown to be capable of further assuring data privacy to a person, where for example biometrics such as a fingerprint, facial image or retinal scan or any of health measurements are not shared with an entity without the person's permission and that a simple confirmation of identity and / or health regiment compliance can be shared with an entity without disclosing personal information.

The present invention also taught useful variations to the mutual health assurance system including a health-verified guarded checkpoint system and a health-verified appointment (or reservation) system. The variations included the use of an app preferably running on a mobile device for receiving and following a health regiment. Like the mutual health assurance system, the regiment included taking one or more measurements with either a self-operated or other operated device capable of also confirming identity. The appointment system was focused on gaining access to a premise that requires "rights," or pre-authorization including a date and time period of access. While such appointment system do not typically include a printed ticket, these access rights are shown to be transferable and associable with both personal biometrics and health measurements such that a person's access to an appointment is then "health assured." The guarded checkpoint system addressed gaining access to a premise where it is generally not necessary to require "rights," but where it is still desirable to only allow access for persons passing a verified health regiment.

The present invention not only teaches apparatus and methods for determining authenticated health measurements where the person about whom the measurements apply is verified by at least one biometric match such as facial recognition, fingerprint, etc., the invention teaches apparatus and methods for determining authenticated health samples, where the health samples are at some later time converted into health measurements, and where these later converted health measurements are therefore also authenticated. Both the authentication of direct health measurements as well as direct health samples (supporting "indirect" health measurements) was shown to be possible under the two general cases of "self-operated" devices and "other-operated" devices, where "other" at least included healthcare professionals or agents.

It was shown that when taking health samples, the process can be personalized in that the apparatus and methods (including a health test kit) can be selected and even adjusted based upon the person requesting the test kit. This personalization includes associating a preferably encrypted authentication token and associated test type with restrictions information with the kit, apparatus and / or data samples, where the authentication token carries anonymized data such that the results are both personalized and anonymized. Regardless of self-operated "at-home" test kits or other-operated test kits implemented at for example a doctor's office or otherwise in-patient or out-patient healthcare service, the present teachings also provide for the use of a "data-exchange intermediary" for further anonymizing any lab results. It was shown that the process of a person or service administering the test kit can include steps for using at least asymmetric encryption to both ensure that the testing lab has no identifying "return address" type information with respect to the person, but rather forwards the encrypted lab results to an intermediary along with a transaction number provided by the person's mobile device and app, where upon receiving the encrypted lab results and transaction number, the intermediary retrieves the person's "return address" and preferably electronically transmits the encrypted lab results essentially and preferably immediately upon receiving, such that the person remains anonymous to the lab and the lab results remain anonymous to the intermediary.

Variations of the at-home test kits were shown and / or described that adapt traditional devices for collecting samples, such as a swab for saliva or mucus, or a sample strip for blood. The adaptations included attaching electronically readable means for carrying the preferably encrypted authentication token to follow with the data samples, where other information such as the type of health test or any special (personal) restrictions or notes can be included for use in the sample testing process. The adaptations also included providing markings on the health sample collection devices where the person then uses their device and app to image these markings substantially simultaneously as they are imaging their own face. The markings are for example a form of a 1D or 2D barcode that can be decoded into a UPC code for example identifying the type of device, or uniquely identifying the actual sample collection device.

It was shown that this allows the app to both verify / identify the person through facial recognition while also verifying / identifying the health sample collection device. The process was also shown to make use of an external fingerprint reader that could be attached to for example a smartphone being used as the smart ticket device and app, where the attachment was shown as wired but could also be wireless. The fingerprint reader was shown as an adaptation of a touch temperature finger-worn health device but could also be a separate "single function" "fingerprint identifier" authentication device for the purposes of the present invention. The reader device preferably included an LED for controlled blinking such that the mobile device and app could substantially: (1) capture an image(s) of the person being verified / authenticated, (2) over multiple images still including the person, capture an image of the fingerprint device blinking the LED on and off in accordance with provided signals, and / or (3) capture the person's fingerprint to combine with the facial recognition for providing enhanced verification / authentication, or to use a single means for personal identification without also using facial recognition.

It was shown that health devices that are authenticatable using for example the LED for controlled blinking to be detected by the mobile device and app (substantially simultaneously with the persons face), could then be further adapted to include a reader for detecting electronically readable IDs, such as an NFC tag. The tags are then attached to a third-part health device that for example neither includes means for personal identification (like a fingerprint reader) nor includes a light emitting element such as an LED (for verification by the mobile device and app) and otherwise cannot be controllably confirmed by the mobile device and app. Using the further adapted health device, such as a finger-worn device with LED and NFC reader, it was shown that the finger-worn device could be used to allow the mobile device and app to first authenticate the finger-worn device and then also authenticate the third party health device via the NFC read tag, where the person is using the finger-worn device to substantially touch the third-party device while simultaneously reading the NFC tag and blinking the LED on the finger-worn device. Third party devices such as arm patches, neck patches and breath analyzers were all described for exemplary use with the finger-worn NFC reading device.

The present invention taught a distinction between body spatial patters and body temporal patterns, where the distinction can be exploited as a means for verify health measurement devices that are unable or not configured to determine body spatial patterns. Spatial patterns are a multiplicity of detectable "spatial" features for example on a face or finger, where the spatial features substantially do not change over time, or change slowly overtime at least in the aggregate, and where spatial features are typically best determined through some form of image processing, whether of a face, a fingerprint, a footprint, a retinal scan, etc. A unique spatial configuration of spatial features comprising a spatial pattern is typically sufficient for substantially uniquely identifying one person from another person, thus called "inter-identification" means. Multiple distinct spatial patterns, such as a face and a fingerprint, increase the assurance of unique personal identification.

Temporal patterns include for example a heartbeat or breathing pattern, or any other detectable on-going body rhythm that can be determined by multiple distinct health devices for correlation or otherwise sufficiently confirming that the temporal patterns "align" or match, where any two health devices capturing sufficiently matching temporal patterns can reasonably be assumed to be measuring the same person. Thus, temporal patterns are referred to as "intra-identification" means and it was shown that if a number of distinct health related devices all each capable of intra-identification and using intra-identification are confirmed to be measuring the same person, then if one of the devices also includes inter-identification means, this inter-identification is thereby extendable to all of the intra-identified device datasets.

The mobile device and app that is capable of inter-identification was shown to also be capable of substantially simultaneous intra-identification using for example rPPG image analysis technology. This allows the mobile device and app to then correlate and accept data from other devices only capable of intra-identification without needing to simultaneously image the device. The finger-worn device configured with and LED and a fingerprint reader was shown to also be capable of inter and intra-identification as well as authentication by the mobile device and app using flashing signals, where then the finger-worn device can be authenticated using either inter-identification and / or flash signals, and where the finger-worn device is usable to correlate other devices only capable of intra-identification.

The present invention has shown that once a distinct "non-identifying" health measurement device is confirmed to be "locked" to a person, then even if the non-identifying device cannot determine a spatial pattern but can be configured to determine a temporal pattern (where the heartbeat is typically available for detection by virtually any device at least coming into contact with the person's skin), then this temporal pattern can be correlated for example to the mobile device and app that is able to determine (thus an "identifying device") both a spatial pattern (e.g. using facial recognition or fingerprint recognition using a built-in fingerprint reader) and temporal pattern (e.g. detecting the heartbeat using rPPG), where the correlation of the temporal pattern captured by the non-identifying device with respect to the temporal pattern captured by the identifying device is then sufficient to authenticate the non-identifying device.

This ability to essentially turn a non-identifying device into an identifying device through intra-identification provides a substantial technical advantage for creating significant private and authenticated health data, usable in and of itself for example to communicate to a healthcare provider in a "telemedicine" application, or to use in conjunction with a regiment, where then a global assessment such as "pass / fail" or even a score such as "83% compliant" becomes an authenticated assessment, something that is currently not available in the marketplace. Authenticated assessments are shown herein to be useful for at least determining if the assessed person should be allowed entrance into a premise or a "group meeting." Authentication across the population of persons currently allowed into or otherwise at a premise or a meeting was shown to be a real-time metric of the premise or group, such that the premise or group can then have its own "health metrics," thus as an "entity" comprising two or more persons, a premise or group is also able to follow a regiment.

With respected to a "device locked to a person" it was shown that wearables, such as the wrist wearable or chest band can substantially be attached to a person's body by wrapping around a portion of the body. Besides devices such as a smartwatch, or activity tracker (e.g. a "FitBit") that are meant to be fastened / lock to a wrist, the present invention has shown other example wearables attached around the chest and the ankle. Other devices such as the "Oura Smart Ring" are similar in purpose to a wrist worn watch or fitness tracker, where they detect their information while "fastened" to a finger. It was shown that devices such as wrist, ankle and chest worn wearables can be detected as fastened at least by using a latch or "lock," where the closing and opening of the latch is datable, such that once latched / locked onto the wrist, ankle or chest, these devices can the be authenticated using various combinations of inter-identification and intra-identification.

Those familiar with various sensors for determining information while in contact with or otherwise within close proximity to the skin, such as electrodermal activity sensors, pulse oximeters, and otherwise "skin contact" sensors, will also understand that by at least sensing a person's skin a different form of a "lock" is possible. Using this this "skin in contact / skin in proximity" "virtual lock," after a wearable is put on by a person the skin is detected, and the virtual lock is considered "closed." After this, the wearable may be authenticated for example using an intra-identification means such as a pulse pattern, or a flashing light pattern emanating from the wearable while the wearable is being imaged by the mobile device and app in combination with the person's face, or the inclusion of a inter-identification means in the wearable, all as taught herein. Authentication may include using the same sensors within the wearable that are also a part of the virtual locking mechanism.

After "locked-authentication," if the wearable is then removed from the person, the person's skin contact / proximity or some other sensed value regarding the authenticated person is substantially no longer detectable, and thus the virtual lock is "opened" / "un-locked" and any data collected by the wearable after this un-locking is no longer considered as authenticated. However, a careful consideration will show that the "lock-check" period, such as every minute versus every second, in which the wearable attempts to re-sense the contact or proximity of the person, effects the ability to ensure that the wearable was not quickly removed and then placed onto another person, where the sensor then detects the other person's skin contact or proximity and assumes that the virtual lock has remained closed during the period. Those familiar with other sensors such as accelerometers and gyros will then also recognized that by using a combination of other sensors it is possible to extend the period, although with potential added cost and complexity with respect to the wearable.

A finger-worn device such as that taught herein is not locked onto the fingertip, but instead can be configured to capture a fingerprint (inter-identification) and / or at least a pulse (intra-identification) such that either alone or in combination with the mobile device and app the finger-worn device is "authenticatable." A finger-worn device such as a "smart ring" that is slipped onto the finger and does not rest upon a fingertip, can be configured for example to include a light emitting devices such as an LED, and / or configured to capturing a temporal pattern such as the heartbeat for intra-identification, where both "flashing light signal" and the intra-identification can at least be used in accordance with the teachings of the present invention to authenticate a "smart ring." In the cases where the detection of the intra-identification pattern is the "lock," then when the pattern is no longer detectable, the device is determinable as "un-locked," such that putting on a wearable and then picking up a heartbeat correlated to a companion device capable of both inter and intra-identification is sufficient for authenticated the device, even if the physical lock-closed detection such as discussed for the smart-watch and chest band is not implemented.

However, by using a physical lock in combination with intra-identification, it is not necessary to retain substantially "continuous" determination of the intra-identification pattern (for the purposes of determining a substantially continuous locked status). Hence, after putting on a wearable and closing or clasping a physical lock, at that time the intra-identification pattern can be detected if only for determining authentication, where afterwards all data determined or collected by the wearable (even if that data does not include the intra-identification pattern), can be considered as authenticated up until the point in time where the physical lock is unlocked.

It should therefore be well understood that the many examples provided herein are exemplary, where for example a "lock" is taught in specific forms, but should also be understood as a function or "distinctly detectable event," where it will be evident to those skilled in the particular type of wearable what apparatus and methods will provide the best-mode lock for a given device, and thus these and other teachings of the present invention should be understood both in light of the exemplary apparatus and method configurations as well as the functional configurations such that the present invention is not limited to the many teaching examples provided herein, but rather also understood in the spirit and scope of the taught functional descriptions as many apparatus and method implementations are possible.

Regarding wearables, example devices include a chest band for measuring signals emanating from the inside of the person's chest, and / or for measuring signals taken from the skin of the chest. The chest band was shown to be capable of comprising multiple types of sensors, for example microphones, pulse-oximeters, accelerometers, and gyros, while virtually any other type of sensor for use with skin contact or otherwise chest cavity signal detection are also usable with the adaptable chest band. The chest band, as with any health device specified herein, can also include environment sensors for example detecting ambient temperature, ambient air oxygenation levels, ambient light, etc., all of which can be combined with personal biometrics to create a contextual understanding of the personal data. Those familiar with what are referred to as "e-textiles" will also understand that using newer materials such as piezoelectric threads incorporated for example in the chest band itself that is fastened onto the person's torso and used to hold other sensor groups, it is possible to provide a means for measure blood pressure.

The chest band as a wearable, was shown to have a physical lock like the wrist-worn wearables, where after locking the chest band is identified using intra-identification such that all data detected and determined after the time of locking and intra-identification up until the time when the chest band is unlocked is usable as authenticated health measurements of a person. Another example health measurement device included a smart scale, where the smart scale determined for example the intra-identification heartbeat pattern for correlating directly to the mobile device and app using an rPPG heartbeat pattern, or indirectly with the mobile device and app for example using the finger-worn device configured to also detect the heartbeat pattern.

A body patch was shown to be a different type of wearable, where body patches are well-known such as a continuous glucose monitoring patch. The "lock" function of such patches can be implemented by creating a patch that when applied (typically using a glue) to the skin can be operated, but once removed from the skin is rendered inoperable, and therefore "unlocked." Circuity and electronics that is both low profile and low cost are well-known for determining at least the heartbeat pattern while in contact with the skin, and as such body patches that are not already configured to measure an intra-identification temporal pattern that can be readily determined by other devices also capable of inter-identification, are anticipated to at least be further adaptable to determine the heartbeat pattern and thusly become "authenticable."

Another example patch at least discussed in the research literature is designed to monitor human stress levels (see "A Flexible and Wearable Human Stress Monitoring Patch," authors Yoon, Sim and Cho, published in Nature "Scientific Reports," 23 March 2016.) The proposed patch includes "three sensors of skin temperature, skin conductance, and pulsewave in the size of a stamp (25 mm x 15 mm x 72 um) in order to enhance wearing comfort with small skin contact area and high flexibility." The "flexible pulsewave sensor" in particular is considered by the authors to be a significant advantage and is made of "flexible piezoelectric member supported by a perforated polyimide membrane." The present invention teaches that at least the detected pulsewave is usable as a temporal pattern for determining intra-identification such that the patch as proposed by the authors can be further adapted as taught herein to be "authenticatable" to a specific person. Furthermore, the physically very small nature of the author's proposed technology represents a means for further adapting any other type of health patch or otherwise wearable that comes into contact with the skin, such that by at least using the author's proposed "flexible pulsewave sensor" it is possible to enable authentication of virtually any such patch or wearable.

Many technologies are continuing to be developed for determining health metrics of a person, where the present invention anticipates new technologies such as the "flexible pulsewave sensor" for use in determining intra-identification and as such the present invention should not be limited to the exemplary apparatus described herein for determining an inter or intra-identification pattern, but rather should be considered both in light of the specific exemplary embodiments and in the light of the function of an inter-identification or intra-dentification pattern, where many other apparatus and methods for determining inter-identification and intra-identification patterns are anticipated and considered to fall within the scope and spirit of the present invention.

In yet another alternate configuration of a wearable, it is possible to further adapt an existing smartwatch or or activity tracker (e.g. a "FitBit") to include a spatial pattern detector, e.g. a camera for detecting the face, or a fingerprint reader for detecting the fingerprint. Using a camera, the wearable has means to both determine a spatial pattern (of facial features) and a temporal pattern (of colorization changes in the face, i.e. "rPPG"), where then the wearable has means for associating a heartbeat with a face / unique person. If the wearable then also is configured to determine a correlating temporal pattern (such as the heartbeat) from the portion of the wearable that is in contact with the person's skin, it is then possible for the wearable to "self-authenticate" the health metrics it is determining, all as a careful reading and consideration will show. Furthermore, when equipped with a physical lock, the wearable once locked, can then self-authenticate a single time where any collected metrics remain authenticated upon until the point in time where the lock is determined to be "unlocked." A careful consideration will also show that a wearable using a fingerprint detector that then also determines for example the heartbeat using substantially the same skin surface from which the fingerprint was (preferably concurrently) determined, can then also self-authenticate any health metrics being collected or determined by the same wearable device using any one or more other sensors oriented for example to the body and not to the finger.

Those familiar with current smartphone technology will appreciate that a typical smartphone includes at least a "front facing" camera that in some configurations is used for facial recognition to "unlock" the phone. Some of these smartphones then further comprise a fingerprint reader, of which there are several technologies including newer technologies capable of being integrated substantially underneath the smartphone screen. What is important to see is that the smartphone comprising both a camera and a fingerprint reader can be further adapted to increase identification accuracy by further adapting the smartphone to include a heartbeat / pulse detector for determining the heartbeat / pulse pattern of substantially the same skin surface from which the fingerprint is then also concurrently being detected. If then the concurrent images being captured by presumably the front-facing camera (but depending upon configuration possibly also a "back-facing" camera) are analyzed, by at least using rPPG technology, it is possible to determine a second temporal heartbeat pattern from the person's face, where then the heartbeat determined from the person's face is compared to the heartbeat determined from their finger, providing stronger evidence that the same person is being detected, thus increasing smartphone security, or the security of any similarly configured mobile device, computing device or simply any device adapted with the necessary electronics such as a security door-lock to a home of facility.

Regarding rPPG technology, the present invention anticipates the use of what are commonly referred to as "polarimetric" sensors or cameras, such as the IMX250MZR manufactured and sold by Sony. Polarimetric cameras detect what is referred to as the "angle of linear polarization" of the incoming wavefront, where a traditional camera is along able to detect what is referred to as the frequency (color) and amplitude (intensity) of a given wavefront. The polarimetric sensor, by determining "polarization" characteristics in addition to frequency and amplitude, provide significant additional information. It is well-known for example that at least using polarization characteristics, it is possible to determine the spatial orientation of the surface that is emitting and / or reflecting the wavefront. While it is often difficult to determine this spatial orientation to a high rotational accuracy, none-the-less changes in the polarimetric measurements with respect to a given area of an object, such as a cheek of a person, are at least interpretable as fluctuations in spatial alignment, where these spatial fluctuations can be used by themselves or in combination with colorization and / or intensity fluctuations that are the traditional means for determining a pulse pattern using rPPG. It is also well-known that polarization characteristics are more stable across a larger "dynamic range" (or color and intensity) such that the polarization data can often be considered more reliable for example in "low light" situations. Thus, using a polarization sensor, the present invention teaches a means for improving rPPG technology and therein also improving at least means for determining intra-identification patterns.

Just as any authenticated health measurement device creates personal biometrics that are useable with a regiment, all as taught herein, it should be well understood that some metrics are not necessarily a "biometric," but rather for example a usage statistic. For example, with a wearable as taught herein, it is possible to determine not only that a wearable is being worn (and thus also presumably collecting health metrics) but also what is the total duration time the wearable is being used. Furthermore, the time duration can be correlated with a geolocation. Thus, using the teachings provided herein, it is also possible to implementing a "selfquarantine" health rule for use in a regiment. In one such quarantine use case, the person is traveling to a new state wherein they are required to stay either in chosen limited location or at a location of their choice wherein they must limit then limit their movement. As a careful consideration will show, this limited location is a "ZONE". At least when the person arrives at the limited location, they then are required to for example fasten a wrist-wearable that using any of the teachings provided herein, is lockable and authenticatable, either by capturing its own inter and intra-identification patterns or by working with a combination device such as a mobile device and app. Once in the "quarantine ZONE," the device must remain on / "locked" and potentially also collecting a periodic combination of inter and intra-identification patterns for "re-authentication."

Hence, the present teachings provide significant benefit beyond the determining of health metrics, where this beneficial additional information is also then usable within or without a regiment. As will be further understood, a self-authenticating wearable that is in substantial continuous communication with a remote tracking system, where the wearable is for example providing information on locked status, authentication status, current geolocation, and even a period biometric, is also usable in law enforcement for example when tracking parolees.

Thus, the reader will see that significant technical advantages are provided herein for creating authenticated health measurements being collected by disparate health measurement devices and confirmed by various combinations of inter and intra-identification.

The present invention further taught means for ensuring that all data related to a regiment and otherwise at least health measurements can be kept private and encrypted on the mobile device and app, where the minimally shared information is related to confirmation (such as "pass/fail") of a "right-to-access" and / or "proof-of-health." While considered private personal information, the health metrics are known to have significant public value for analysis and pattern matching across a population. It was shown that HIPPA compliant methods can be used to provide a dataset of the personal health metrics to a centralized authority without giving up personal information, thus remaining "anonymous." The preferred solution was to securely transmit selected health metrics in association with an anonymization group such as a zip code or study ID, where the metrics represented a significant period such as two to three weeks. These "rolling snapshots" of anonymous data are preferably uploaded daily, processed centrally for pattern matching and value extraction, and then deleted. This approach of daily uploading and deleting a rolling snapshot increases privacy as opposed to uploading health metrics for example each next day, where the metrics must then be matched to the prior day(s), thus requiring a constant identifier associated with the metrics that could possibly be used to deanonymize the data.

However, the present invention has value and use both with the preferred rolling snapshot and with a daily upload, and therefore should not be limited by any particular implementation of private data shared publicly (including or not including personal information), or method of transmission (including secured and unsecured), or method of aggregation (such as centralized or decentralized), or database implementation means (such as a specific technology), as many variations are possible and will be well understood from a careful reading without departing from the scope and spirit of the present invention.

**It** was also shown that after processing the private now centralized anonymized data, it was possible to send a public "broadcast message" to a population of private mobile devices and apps that included a public algorithm or data model for detecting significant patterns in the distributed private datasets. The "detector" was shared with the entire population and was usable on the private mobile devices and apps to privately assess and select / deselect a person registered to the mobile device and app to receive a message if the private health data related to that person correlated to the centrally determined "public" pattern. The broadcast message was shown to possibly include regiment updates including additional new rules, deletion of rules or otherwise modifications to any rule.

Those familiar with data processing will also understanding that the broadcast messages can contain a unique compilation of data values and / or data limits as a means for filtering and selecting a given private set of health metrics for receiving a message. For example, if within a population of 100,000s of persons, a given set of temperature, blood oxygenation and cough patterns data values is considered to be matching a pattern of concern as determined by analyzing for example the centralized data, and it is determined that only 1 individual matches the pattern, then it is possible to simply send the set of actual temperature, blood oxygenation and cough pattern values (preferably with some form of date-time or sequence information) as a "data value unique signature" matching the 1 individual after which all mobile device and apps in the population compare the unique signature to their own private data, for which only one registered person on one mobile device an app will be determined as matching.

However, if 10 people are found to be matching, then when compiling the signature for any given date / day-sequence, it is possible to include a data range (e.g. temperatures between X and Y) or a list of data values (e.g. temperatures 98, 100, 101). As those with experience in pattern matching will understand, as the data becomes more complex (e.g. including more dimensions / parameters), sending a signature comprising values or limits on values becomes increasingly problematic, and thus at a given threshold of complexity, sending for example a trained **ML** data model is more efficient. What should be understood is that the broadcast message as taught herein is designed to deliver a "message" to a select group of one or more registered persons, where the message comprises for example instructional information, diagnostic information, encouragement, etc. and / or a regiment rule update, and where there are multiple possible ways for providing additional "selection criteria" for parsing the local private databases and determining a person as being in the selected group, where the selected criteria is acting as the herein referred to "trained detector." Thus, the present teachings should be also considered functionally (e.g. "a means for selecting") and not simply limited to the technical embodiments (e.g. using a ML data model) described herein, where especially in relation to data filtering and selection many well-known algorithms and others yet to be devised may be used while staying within the scope and spirit of the invention.

Thus, the present invention teaches a powerful new tool that securely and anonymously collects and authenticates a broad range of private authenticated health metrics, and while assuring the privacy of the individual provides significant public value through data aggregation and analysis. The system is adaptable, where the centralized authority is essentially receiving "real-time" population metrics, discerning patterns and disseminating regiment rules to most effectively respond to any health crisis such as the current pandemic, where new reliable health data is of highest value for developing new solutions and strategies to increase public safety. This system is expected to have significant lasting value as multiple infectious diseases as treatable but otherwise with respect to the world's population cannot reasonably be eradicated.

Significant teaching was providing that shows the drawbacks of state-of-the-art contact tracing using smartphones. This "quantitative" tracing was shown to be ineffective at least because of the low population "uptake" and "unequitable" nature of the necessary underlying technology, for example the high cost of a necessary smartphone. The present invention teaches an active mask that can determine "proper fit and filtration" and communicate this to a private mobile device and app as well as a mask tracking system. The active masks are significantly less expensive (i.e. more equitable) than a smartphone and in many situations are required to be worn by all persons at a premise, such as office buildings, campuses, healthcare facilities, public transportation vehicles such as airplanes, buses and subway cars, theme parks, stadiums, public parks, schools and universities, military bases, etc., thus the requirement to be worn supports a population uptake of virtually 100%. The active masks were configured with a wireless communication means including any of GPS, Bluetooth, wi-fi, etc. and therefore capable of quantitative contact tracing, thus providing virtually 100% "quantitative" contact tracing.

The present invention showed that using any combination of the health measurement data determined by the mobile device and app, especially in accordance with a public regiment, along with at least the "proper fit and filtration" determination made by the active mask, it was possible to "qualify" all "quantified" contacts. For example, using a regiment a person may be determined as: 1) being asymptomatic over the last 14 days, 2) having received a negative test result using an at-home test kit within the last 3 days, 3) having been vaccinated, and 4) using the active mask that is currently "properly fit" and "properly filtered," such that any contact with this person is qualified differently than a person with less stringent health qualification and mask usage data. Similarly, the asymptomatic, vaccinated person who has passed a health kit test can at least disregard or minimize exposure concerns from other select "qualified" contacts, especially other contacts likewise determined or estimated to be healthy or otherwise less-probable for infections transmission. Such health qualification of contacts was taught to significantly impact the time and energy costs for a smartphone or electronic device to decrypt and assess a possibly large number of contacts when looking for a particular contact generally reported to have been infected with a disease.

Active masks where also shown to allow for example parents to better monitor if their children are properly wearing their masks when playing with friends, or similarly for a company to determine if its employees are properly wearing their masks either while working on or off premise. A mask tracking system was taught that for example allows a school campus to apply stricter enforcement of mask usage and by using regiments to identify symptomatic persons more quickly for separation and isolation. Person's determined to be infected can be quarantined along with anyone else they came into contact with, rather than simply sending home the entire school for quarantine causing great disruption to both the educational process and the home lives of the students and their families.

A careful reading of these variation systems will show that combinations and cross-overs between the systems are possible and anticipated for providing even more alternatives. As will be well understood by those familiar with the marketplace, many premises have need to limit access to those who have pre-paid for access rights and / or pre-scheduled an appointment (reservation) to visit, while these same premises also provide for access that is "general public," free and unscheduled. In all cases, it has been shown herein possible to always provide for both the ensuring and assurance of the sufficient health of a person wishing to enter a premises, and the ensuring and assurance that the premises is only occupied by other person who also have a sufficient health.

The careful reader will understand the significant benefit and uses of the present teachings across virtually all types of premises, either privately owned or publicly owned, either indoors only, outdoors only or a combination, either with limited controlled access or free access, either with general non-ticketed access, ticketed private access, or highly restricted non-private access. Premises need not be stationary and include forms of transportation or otherwise moving premises. Persons can be of any type of relationship to the premises including a visitor, a patron, a worker or a service provider.

The careful reader will also see that the present teachings provide significant apparatus and means for verifying / authenticating a person during a "transaction," where the transaction includes determining "transaction information" (e.g. the "measurement") as well as "transaction verification" (e.g. sensing and recording a location, time, environmental metric, or even a personal metric such as electrodermal activity). In the present teachings, much emphasis has been placed on transactions that determine health related information about the person. However, a transaction could alternatively include business related information such as a contact, where the system's novel apparatus and means for authentication during a business transaction provide significant opportunities within the art generally referred to as "electronic signatures." It will be understood that a person at-home and in private can use their Honest Broker App to for example review a digital copy of a document, where preferably before or during the "review transaction," the Honest Broker App first authenticates the person and then second associates with the digital copy an Authentication Token that preferably includes evidence of the person's confirmed identity. Many variations are possible, especially when combined with current "docu-sign" workflows, all as will be well understood by those familiar with electronic signatures.

As a careful reader will see, the present invention provides many apparatus and methods, some of which are novel and useful by themselves or in various combinations, as will be obvious to those skilled in the various arts. Those skilled in the various art herein described will also understand that newer technologies including newer health measurements will become available for providing the same or anticipated functions as herein described. As such, the present invention and all of its preferred and alternative embodiments should be considered both as apparatus and as functions that are exemplary and not limitations of the present invention, as these same functions may be better implemented in the future using newly developed technologies not available at the time of the present invention. Furthermore, while the present invention has uses covering entities such as theme and amusement parks, museums, sporting and music venues, convention centers, air ports and even secure office buildings, this list and any other types of entities and premises mentioned herein or within the PRIOR ART incorporated by reference should not be considered as limiting, as virtually any location for example including stores, restaurants, places of worship, schools, healthcare facilities, etc. are able to benefit from the present teachings.

## Claims

1. A system (102, 103, 104) for governing a person's (1) access to a premises or gathering (74) based at least in part upon an anonymous publicly compliant authenticated health status (2d) of the person (1), where the person (1) uses a computing device and app (2a, 2-3, 2-4, 2-5) as a means for providing private selfauthentication, comprising:
an access control system (73) for governing the person's (1) access to the premises or gathering (74);
the computing device (2a, 2-3, 2-4, 2-5) operated by the person (1) seeking access to the premises or gathering (74);
at least one authenticating health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) or at least one authenticating healthcare service provider (42), where the at least one health measurement device (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131) determines at least one health measurement (2d, 2a-db-4a) that is authenticated to be of the person (1), where the at least one healthcare service provider (42) determines at least one health measurement (2d, 2a-db-4a) that is authenticated to be of the person (1) or provides at least one healthcare service (2d, 2a-db-4a) that is authenticated as provided to the person (1);
an intermediary app (2a) executing on the computing device (2a, 2-3, 2-4, 2-5) where the person (1) registers one or more biometrics with the intermediary app (2a) for subsequent use during a (function a) for authenticating the person (1), where the (function a) comprises confirming the identity of the person (1) by determining and comparing a current biometric with the one or more registered biometrics;
one or more public regiments (2d-1, 2d-2) for determining the public compliance of the authenticated health status (2d), where the one or more public regiments (2d-1, 2d-2) comprise digital information specifying one or more rules, where a regiment (2d-1, 2d-2) is distinct from the intermediary app (2a), where the intermediary app (2a) additionally functions to receive, retrieve, update or otherwise obtain the one or more public regiments (2d-1, 2d-2) or to combine or otherwise process rules from any two or more public regiments (2d-1, 2d-2), where a rule defines, limits or otherwise governs the processing of the intermediary app (2a) when determining the at least one authenticated health measurement (2d, 2a-db-4a) and the at least one authenticated healthcare service (2d, 2a-db-4a), and where the rules specify a formulation of any information associated with any one of or any combination of the at least one authenticated health measurement (2d, 2a-db-4a) and the at least one authenticated healthcare service (2d, 2a-db-4a) for use in determining and otherwise maintaining the publicly compliant authenticated health status (2d) of the person (1), and
where prior to the person (1) attempting access into the premises or gathering (74) in a (function 3) the person (1) at least in part uses the computing device and intermediary app (2a, 2-3, 2-4, 2-5) to perform any one of or any combination of a (function 1) and a (function 2), wherein: the (function 1) comprises communicating with the at least one health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) to initiate the at least one authenticated health measurement (2d, 2a-db-4a), where during or after initiation the computing device and intermediary app (2a, 2-3, 2-4, 2-5) performs the (function a) to confirm the identity of the person (1) by determining at least one biometric for comparing with a registration biometric while substantially concurrently performing a (function b1) to confirm that a sensing surface of the device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) is substantially touching, in close proximity of, or otherwise co-located with the body of the person (1) for ensuring that the at least one health measurement is taken of the person (1), and any of concurrently or thereafter performing a (function c1) to receive, retrieve or otherwise obtain the at least one health measurement (d2, 2a-db-4a) from the device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131), where the received health measurement (2d, 2a-db-4a) is therein authenticated to be of the person (1), and
the (function 2) comprises communicating with the at least one healthcare service provider (42) to initiate or respond to the initiation of the at least one health measurement (2d, 2a-db-4a) or the at least one healthcare service (3-2), where during or after initiation the computing device and intermediary app (2a, 2-3, 2-4, 2-5) performs the (function a) to confirm the identity of the person (1) by determining at least one biometric for comparing with a registration biometric while substantially concurrently performing a (function b2) to provide anonymous information to the healthcare service provider (42) for associating with the at least one health measurement (2d, 2a-db-4a) or the at least one healthcare service (3-2), where the anonymous information does not comprise personally identifying information regarding the person (1), and where if the healthcare service provider (42) determined at least one healthcare measurement (2d, 2a-db-4a) then any of concurrently or thereafter the computing device and intermediary app (2a, 2-3, 2-4, 2-5) performs a (function c2-1) to receive, retrieve or otherwise obtain the at least one health measurement (2d, 2a-db-4a) from the healthcare service provider (42), and if the healthcare service provider (42) provided at least one healthcare service (3-2) then any of concurrently or thereafter the computing device and intermediary app (2a, 2-3, 2-4, 2-5) performs a (function c2-2) to receive, retrieve or otherwise obtain a confirmation-of-service receipt from the healthcare service provider (42) confirming that the at least one healthcare service (3-2) has been provided to the associated anonymous person (1), where the received, retrieved or otherwise obtained at least one health measurement (2d, 2a-db-4a) or at least one healthcare service (3-2) is therein authenticated to be of the person (1), and
where after the person (1) at least in part uses the computing device and app (2a, 2-3, 2-4, 2-5) to perform the any one of or any combination of the (function 1) and the (function 2) the person (1) then uses the computing device and intermediary app (2a, 2-3, 2-4, 2-5) to perform the (function 3) comprising communicating with the access control system (73) prior to the access control system (73) granting or denying the person (1) access to a premises or gathering (74), where during the (function 3) the computing device and intermediary app (2a, 2-3, 2-4, 2-5) functions to request a grant-of-entry, where the process of the requesting entry comprises the (function a) to confirm the identity of the person (1) by determining at least one biometric for comparing with a registration biometric and performing a (function d) providing the authenticated health status (2d) regarding the person (1) to the access control system (73), where the authenticated health status (2d) is anonymous and does not comprise personally identifying information regarding the person (1), where the authenticated health status (2d) is determined based at least in part on any one of or any combination of the at least one authenticated health measurement (2d, 2a-db-4a) of the person (1) and the at least one authenticated healthcare service (3-2) provided to the person (1), and where the access control system (73) allows or denies access by the person (1) to the premises or the gathering (74) based at least in part upon the publicly compliant anonymous authenticated health status (2d) of the person (1).

2. The system of claim 1 where the (function b1) comprises performing a cooperative data sharing function between the computing device and app (2a, 2-3, 2-4, 2-5) and the at least one health measurement device (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131), where the shared data comprises any one of or any combination of a device measured spatial or temporal body pattern of the person (1), an emitted light signal responsive to a control signal provided by the computing device and app (2a, 2-3, 2-4, 2-5), information indicative of a secured state of the at least one health measurement device (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131), visible device markings, and an electronically provide ID, where the shared data is compared for sufficient matching with nondevice supplied information either pre-known by the computing device and app (2d, 2a-db-4a) or concurrently provided by a companion device (3-1-d9, 3-1-d10) to the computing device and app (2d, 2a-db-4a).

3. The system of claim 1 wherein the at least one health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) is a temporary engagement device (3-1-d1, 3-1-d2, 3-2, 3-1-d6, 3-1-d7, 3-1-d8, 3-1-d9, 4-4), and where when using the temporary engagement device (3-1-d1, 3-1-d2, 3-2, 3-1-d6, 3-1-d7, 3-1-d8, 3-1-d9, 4-4) performing the (function 1) comprising the combination of the (function a), the (function b1), and the (function c1) comprises any of:
face of person (1) recognition concurrent with light signaling confirmation, where the temporary engagement device is a light-signaling temporary engagement device (3-1-d1, 3-1-d7, 3-1-d8, 3-1-d9) comprising a light emitter (3-1-d1-led, 3-1-d7-led, 3-1-dt8-led, 3-1-d9-led) and one or more sensors for determining one or more of the health measurements (2d) of the person (1), where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) (i) provides a control signal to the light-signaling temporary engagement device (3-1-d1, 3-1-d7, 3-1-d8, 3-1-d9), (ii) captures two or more images substantially comprising a concurrent view of the face of the person (1) and of the light-signaling temporary engagement device (3-1-d1, 3-1-d7, 3-1-d8, 3-1-d9) located substantially in contact with or in sufficient proximity with the body of the person (1) for determining one or more of the health measurements (2d), where the light-signaling temporary engagement device (3-1-d1, 3-1-d7, 3-1-d8, 3-1-d9) while in the concurrent view of the camera and using at least in part the control signal causes the light emitter (3-1-d1-led, 3-1-d7-led, 3-1-dt8-led, 3-1-d9-led) to emit a response signal comprising light detectable by the camera, where the computing device and app (2a, 2-3, 2-4, 2-5) (iii) processes the two or more images for (iii-a) comparing the captured face of the person (1) with the pre-registered facial image, (iii-b) comparing the emitted response signal with the provided control signal, and (iii-c) confirming the substantial contact or otherwise sufficient proximity between the light-signaling temporary engagement device (3-1-d1, 3-1-d7, 3-1-d8, 3-1-d9) and the body of the person (1) for ensuring that one or more of the health measurements (2d) are of the person (1), and whereupon sufficient matches and confirmation the computing device and app (2a, 2-3, 2-4, 2-5) performs (function c1) and receives or otherwise accepts the ensured one or more of the health measurements (2d) from the light-signaling temporary engagement device (3-1-d1, 3-1-d7, 3-1-d8, 3-1-d9) as being authenticated of the person (1);
specific body location of person (1) spatial pattern matching concurrent with colocated health measurement, where the temporary engagement device is a spatial-body-pattern temporary engagement device (3-1-d2, 3-1-d6, 3-1-d9) comprising means for concurrently determining from substantially a same specific spatial body location of the person (1) both of a comparison spatial body pattern and one or more of the health measurements (2d), where the specific spatial body location comprises a finger, a face, or otherwise a uniquely identifiable location on the body of the person (1), where the spatial body pattern comprises a uniquely identifying pattern of the body of person (1) determined at the specific spatial body location, where the uniquely identifying pattern comprises a fingerprint, a facial image, or otherwise a uniquely identifiable spatial body pattern, where the pre-registered biometric of the person (1) comprises the spatial body pattern of person (1) substantially taken from the same specific spatial body location, where at some time during or otherwise over some concurrent duration of the (function a) and the (function b1) the computing device and app (2a, 2-3, 2-4, 2-5) (i) receives from the spatial-body-pattern temporary engagement device (3-1-d2, 3-1-d6, 3-1-d9) the comparison spatial body pattern, and (ii) compares the comparison spatial body pattern with pre-registered spatial body pattern, and whereupon sufficient match the computing device and app (2a, 2-3, 2-4, 2-5) performs (function c1) and receives or otherwise accepts the one or more of the colocated health measurements (2d) from the spatial-body-pattern temporary engagement device (3-1-d2, 3-3, 3-1-d6, 3-1-d9) as being authenticated of the person (1);
use of a companion device (3-1-d9) to provide a companion spatial body pattern along with a concurrent and colocated companion temporal body pattern, where the companion device (3-1-d9) comprises means for concurrently determining from substantially a same specific spatial body location of the person (1) both of the companion spatial body pattern (3-1-d9-d1) and the companion temporal body pattern (3-1-d9-d2), where the same specific spatial body location comprises a finger or otherwise a uniquely identifiable location on the body of the person (1), where a spatial body pattern (3-1-d9-d1) comprises a uniquely identifying pattern of the body of person (1) determined at the same specific spatial body location, where the uniquely identifying pattern comprises a fingerprint or otherwise a uniquely identifiable spatial body pattern, where the companion temporal body pattern comprises a measurement sequence of heartbeats of the person (1), where the temporary engagement device is a temporal-body-pattern temporary engagement device (4-4) comprising means for determining a sample temporal body pattern (4-4-d2) that comprises a measurement sequence of the heartbeats of the person (1) taken from a different body location than the same specific spatial body location of the person (1) and comprising one or more sensors coming into substantial contact, sufficient proximity, or otherwise mechanical engagement with the body of the person (1) for determining one or more of the health measurements (2d) of the person (1), where at least one biometric of the person (1) pre-registered with the computing device and app (2a, 2-3, 2-4, 2-5) comprises a spatial body pattern of person (1) substantially taken from the same specific spatial body location, where at some time during or otherwise over some concurrent duration of the (function a) and the (function b1) the computing device and app (2a, 2-3, 2-4, 2-5) (i) receives from the companion device (3-1-d9) the companion spatial body pattern (3-1-d9-d1), (ii) receives from the companion device (3-1-d9) the companion temporal body pattern (3-1-d9-d2), (iii) receives from the temporal-body-pattern temporary engagement device (4-4) the sample temporal body pattern (4-4-d2), (iv) compares the companion spatial body pattern (3-1-d9-d1) to the pre-registered spatial body pattern, and (v) compares the companion temporal body pattern (3-1-d9-d2) to the sample temporal-body-pattern (4-4-d2), and whereupon sufficient matches the computing device and app (2a, 2-3, 2-4, 2-5) performs (function c1) and receives or otherwise accepts the one or more of the health measurements (2d) from the temporal-body-pattern temporary engagement device (4-4) as being authenticated of the person (1);
face of person (1) recognition concurrent with temporal body pattern matching, where the temporary engagement device is a temporal-body-pattern temporary engagement device (4-4) comprising means for determining a sample temporal body pattern (4-4-d2) that comprises a measurement sequence of the heartbeats of the person (1) and comprising one or more sensors coming into substantial contact, sufficient proximity, or otherwise mechanical engagement with the body of the person (1) for determining one or more of the health measurements (2d) of the person (1), where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) (i) receives from the temporal-body-pattern temporary engagement device (4-4) the sample temporal body pattern (4-4-d2), (ii) captures two or more images substantially comprising the face of the person (1), (iii) processes the two or more images for (iii-a) comparing the captured face of the person (1) with the pre-registered facial image, (iii-b) determining a computing device temporal body pattern (2a-d2) comprising a measurement sequence of heartbeats of the person (1), and (iii-c) comparing the computing device temporal body pattern (2a-d2) to the sample temporal body pattern (4-4-d2), and whereupon sufficient matches the computing device and app (2a, 2-3, 2-4, 2-5) performs (function c1) and receives or otherwise accepts the one or more of the health measurements (2d) from the temporal-body-pattern temporary engagement device (4-4) as being authenticated of the person (1), and
walk-up bio-measurement device (3-2) under the control of the access control system (74) to provide health measurements, where the temporary engagement device is a walk-up bio-measurement device (3-2) operated by or otherwise in communications with the access control system (74) for detecting and communicating with the computing device and app (2a, 2-3, 2-4, 2-5) being operated by the person (1) while the person (1) is substantially isolated and aligned with the temporary engagement walk-up bio-measurement device (3-2) so as to sufficiently ensure that any of the health measurements (2d) subsequently determined by the temporary engagement walk-up bio-measurement device (3-2) and provided to the computing device and app (2a, 2-3, 2-4, 2-5) are of the person (1), where at some time during or otherwise over some concurrent duration of the (function a) and the (function b1) the person (1) carrying, wearing, or otherwise having the computing device and app (2a, 2-3, 2-4, 2-5) enters into the isolated physical alignment with the temporary engagement walk-up bio-measurement device (3-2) and remote communications are established between the temporary engagement walk-up bio-measurement device (3-2) and the computing device and app (2a, 2-3, 2-4, 2-5), whereafter the isolated and aligned person (1) uses the computing device and app (2a, 2-3, 2-4, 2-5) to privately provide to the computing device and app (2a, 2-3, 2-4, 2-5) at least one confirmation biometric for comparison with the one or more pre-registered bio-metrics, whereupon sufficient match the computing device and app (2a, 2-3, 2-4, 2-5) provides an anonymous indication of the authenticity of the person (1) to the temporary engagement walk-up bio-measurement device (3-2) and upon receiving the anonymous indication the temporary engagement walk-up bio-measurement device (3-2) determines one or more of the health measurements (2d) of the sufficiently isolated and positioned person (1) for provision to the computing device and app (2a, 2-3, 2-4, 2-5), and where the computing device and app (2a, 2-3, 2-4, 2-5) performs (function c1) and receives or otherwise accepts the one or more of the health measurements (2d) from temporary engagement walk-up bio-measurement device (3-2) as being authenticated of the person (1).

4. The system of claim 1 wherein the at least one health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) is a sustained engagement wearable device (3-1-d4, 3-1-d5, 4-1a, 4-1b, 4-3, 131), and where when using the sustained engagement wearable device (3-1-d4, 3-1-d5, 4-1a, 4-1b, 4-3, 131) performing the (function 1) comprising the combination of the (function a), the (function b1), and the (function c1) comprises any of:
face of person (1) recognition concurrent with light signaling confirmation and wearable secured state confirmation, where the sustained engagement wearable is a light-signaling sustained engagement wearable (3-1-d4, 3-1-d5) comprising a light emitter, one or more sensors for determining one or more of the health measurements (2d) of the person (1), and means for determining any of secured state information for use at least in part to determine that the light-signaling sustained engagement wearable (3-1-d4, 3-1-d5) is fastened, locked, latched, or otherwise secured to the body of the person (1), where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some concurrent duration of the (function a) and the (function b1) the computing device and app (2a, 2-3, 2-4, 2-5) (i) provides a control signal to the light-signaling sustained engagement wearable (3-1-d4, 3-1-d5), (ii) captures two or more images substantially comprising a concurrent view of the face of the person (1) and of the light-signaling sustained engagement wearable (3-1-d4, 3-1-d5) located substantially in contact with or in sufficient proximity with the body of the person (1) for ensuring that the light-signaling sustained engagement wearable (3-1-d4, 3-1-d5) is affixed, secured, or otherwise being worn on the body of the person (1), and (iii) receives from the light-signaling sustained engagement wearable (3-1-d4, 3-1-d5) the secured state information, where the light-signaling sustained engagement wearable (3-1-d4, 3-1-d5) while in the concurrent view of the camera and using at least in part the control signal causes the light emitter to emit a response signal (3-sig) comprising light detectable by the camera, where the computing device and app (2a, 2-3, 2-4, 2-5) (iv) processes the two or more images for (iv-a) comparing the captured face of the person (1) with the pre-registered facial image, (iv-b) comparing the emitted response signal (3-sig) with the provided control signal, and (iv-c) confirming the substantial contact or otherwise sufficient proximity between the light-signaling sustained engagement wearable (3-1-d4, 3-1-d5) and the body of the person (1) for ensuring that light-signaling sustained engagement wearable (3-1-d4, 3-1-d5) is affixed, secured, or otherwise being worn on the body of the person (1), whereupon sufficient matches and confirmation and in combination with the determination by the computing device and app (2a, 2-3, 2-4, 2-5) that the light-signaling sustained engagement wearable (3-1-d4, 3-1-d5) is fastened, locked, latched, or otherwise secured to the body of the person (1) using at least in part the secured state information the computing device and app (2a, 2-3, 2-4, 2-5) determines that the light-signaling sustained engagement wearable (3-1-d4, 3-1-d5) is entering a duration of secured time, where the duration of secured time continues up until such a subsequent time when subsequent secured state information is received by the computing device and app (2a, 2-3, 2-4, 2-5) from the light-signaling sustained engagement wearable (3-1-d4, 3-1-d5) and used at least in part by the computing device and app (2a, 2-3, 2-4, 2-5) to determine that the light-signaling sustained engagement wearable (3-1-d4, 3-1-d5) is no longer sufficiently secured to the body of the person (1) ending the duration of secured time, and where the computing device and app (2a, 2-3, 2-4, 2-5) performs (function c1) and receives and accepts the one or more of the health measurements (2d) determined during the duration of secured time from the light-signaling sustained engagement wearable (3-1-d4, 3-1-d5) as being authenticated of the person (1);
use of a companion device (3-1-d9) to provide a companion spatial body pattern along with a concurrent and colocated companion temporal body pattern, where the companion device (3-1-d9) comprises means for concurrently determining from substantially a same specific spatial body location of the person (1) both of the companion spatial body pattern (3-1-d9-d1) and the companion temporal body pattern (3-1-d9-d2), where the same specific spatial body location comprises a face, an eye, a finger, or otherwise a uniquely identifiable location on the body of the person (1), where a spatial body pattern (3-1-d9-d1) comprises a uniquely identifying pattern of the body of person (1) determined at the same specific spatial body location, where the uniquely identifying pattern comprises a facial image, a retinal scan, a fingerprint, or otherwise a uniquely identifiable spatial body pattern, where the companion temporal body pattern comprises a measurement sequence of heartbeats of the person (1), where the sustained engagement wearable is a temporal-body-pattern sustained engagement wearable (3-1-d4, 3-1-d5, 4-1a, 4-1b, 4-3) comprising means for determining a wearable temporal body pattern comprising a measurement sequence of the heartbeats of the person (1) taken from a different body location than the same specific spatial body location of the person (1), one or more sensors for determining one or more of the health measurements (2d) of the person (1), and means for determining any of secured state information for use at least in part to determine that the temporal-body-pattern sustained engagement wearable (3-1-d4, 3-1-d5, 4-1a, 4-1b, 4-3) is fastened, locked, latched, or otherwise secured to the body of the person (1), where at least one biometric of the person (1) pre-registered with the computing device and app (2a, 2-3, 2-4, 2-5) comprises a spatial body pattern of person (1) substantially taken from the same specific spatial body location, where at some concurrent duration of the (function a) and the (function b1) the computing device and app (2a, 2-3, 2-4, 2-5) (i) receives from the companion device (3-1-d9) the companion spatial body pattern (3-1-d9-d1), (ii) receives from the companion device (3-1-d9) the companion temporal body pattern (3-1-d9-d2), (iii) receives from the temporal-body-pattern sustained engagement wearable (3-1-d4, 3-1-d5, 4-1a, 4-1b, 4-3) the wearable temporal body pattern, (iv) receives from the temporal-body-pattern sustained engagement wearable (3-1-d4, 3-1-d5, 4-1a, 4-1b, 4-3) the secured state information, (v) compares the companion spatial body pattern (3-1-d9-d1) to the pre-registered spatial body pattern, and (vi) compares the companion temporal body pattern (3-1-d9-d2) to the wearable temporal-body-pattern, whereupon sufficient matches and in combination with the determination by the computing device and app (2a, 2-3, 2-4, 2-5) that the temporal-body-pattern sustained engagement wearable (3-1-d4, 3-1-d5, 4-1a, 4-1b, 4-3) is fastened, locked, latched, or otherwise secured to the body of the person (1) using at least in part the secured state information the computing device and app (2a, 2-3, 2-4, 2-5) determines that the temporal-body-pattern sustained engagement wearable (3-1-d4, 3-1-d5, 4-1a, 4-1b, 4-3) is entering a duration of secured time, where the duration of secured time continues up until such a subsequent time when subsequent secured state information is received by the computing device and app (2a, 2-3, 2-4, 2-5) from the temporal-body-pattern sustained engagement wearable (3-1-d4, 3-1-d5, 4-1a, 4-1b, 4-3) and used at least in part by the computing device and app (2a, 2-3, 2-4, 2-5) to determine that the temporal-body-pattern sustained engagement wearable (3-1-d4, 3-1-d5, 4-1a, 4-1b, 4-3) is no longer sufficiently secured to the body of the person (1) ending the duration of secured time, and where the computing device and app (2a, 2-3, 2-4, 2-5) performs (function c1) and receives and accepts the one or more of the health measurements (2d) determined during the duration of secured time from the temporal-body-pattern sustained engagement wearable (3-1-d4, 3-1-d5, 4-1a, 4-1b, 4-3) as being authenticated of the person (1), and
face of person (1) recognition concurrent with temporal pattern matching and wearable secured state confirmation, where the sustained engagement wearable is a temporal-body-pattern sustained engagement wearable (3-1-d4, 3-1-d5, 4-1a, 4-1b, 4-3) comprising means for determining a wearable temporal body pattern comprising a measurement sequence of the heartbeats of the person (1), one or more sensors for determining one or more of the health measurements (2d) of the person (1), and means for determining any of secured state information for use at least in part to determine that the temporal-body-pattern sustained engagement wearable (3-1-d4, 3-1-d5, 4-1a, 4-1b, 4-3) is fastened, locked, latched, or otherwise secured to the body of the person (1), where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) (i) receives from the temporal-body-pattern sustained engagement wearable (3-1-d4, 3-1-d5, 4-1a, 4-1b, 4-3) the secured state information, (ii) receives from the temporal-body-pattern sustained engagement wearable (3-1-d4, 3-1-d5, 4-1a, 4-1b, 4-3) the wearable temporal body pattern, (iii) captures two or more images substantially comprising the face of the person (1), (iv) processes the two or more images for (iv-a) comparing the captured face of the person (1) with the pre-registered facial image, (iv-b) determining a computing device temporal body pattern (2a-d2) comprising a measurement sequence of heartbeats of the person (1), and (iv-c) comparing the computing device temporal body pattern (2a-d2) to the wearable temporal-body-pattern, whereupon sufficient matches and in combination with the determination by the computing device and app (2a, 2-3, 2-4, 2-5) that the temporal-body-pattern sustained engagement wearable (3-1-d4, 3-1-d5, 4-1a, 4-1b, 4-3) is fastened, locked, latched, or otherwise secured to the body of the person (1) using at least in part the secured state information the computing device and app (2a, 2-3, 2-4, 2-5) determines that the temporal-body-pattern sustained engagement wearable (3-1-d4, 3-1-d5, 4-1a, 4-1b, 4-3) is entering a duration of secured time, where the duration of secured time continues up until such a subsequent time when subsequent secured state information is received by the computing device and app (2a, 2-3, 2-4, 2-5) from the temporal-body-pattern sustained engagement wearable (3-1-d4, 3-1-d5, 4-1a, 4-1b, 4-3) and used at least in part by the computing device and app (2a, 2-3, 2-4, 2-5) to determine that the temporal-body-pattern sustained engagement wearable (3-1-d4, 3-1-d5, 4-1a, 4-1b, 4-3) is no longer sufficiently secured to the body of the person (1) ending the duration of secured time, and where the computing device and app (2a, 2-3, 2-4, 2-5) performs (function c1) and receives and accepts the one or more of the health measurements (2d) determined during the duration of secured time from the temporal-body-pattern sustained engagement wearable (3-1-d4, 3-1-d5, 4-1a, 4-1b, 4-3) as being authenticated of the person (1).

5. The system of claim 1 wherein the at least one health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) is a biometric sample collection device (4-2, 80, 84), and where when using a biometric sample collection device (4-2, 80, 84) performing the (function 1) comprising the combination of the (function a), the (function b1), and the (function c1) comprises any of:
face of person (1) recognition concurrent with scanning of visual markings (80-bcd, 84-bcd) to determine a sample collection device ID, where the biometric sample collection device (80, 84) collects, receives, takes, or otherwise obtains a bodily fluid or otherwise sample of the person (1) using a sample receptacle (80-tip, 84-smp), where a bodily fluid or otherwise sample comprises mucus, saliva, or blood, where the biometric sample collection device (80, 84) comprises visual markings (80-bcd, 84-bcd) for use at least in part to determine the sample collection device ID, where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) captures two or more images substantially comprising a concurrent view of the face of the person (1), the biometric sample collection device (80, 84) and the visual markings (80-bcd, 84-bcd), and the person (1) using the biometric sample collection device (80, 84) to collect, receive, take, or otherwise obtain a bodily fluid or otherwise sample of the person (1), where the computing device and app (2a, 2-3, 2-4, 2-5) (i) processes the two or more images for (i-a) determining the sample collection device ID based at least in part upon the visual markings (80-bcd, 84-bcd), (i-b) comparing the captured face of the person (1) with the pre-registered facial image, and (i-c) confirming the substantial contact or otherwise sufficient proximity between the sample receptacle (80-tip, 84-smp) and the body of the person (1) for ensuring that each of any one or more of the collected, taken, or otherwise obtained biometric samples are of the person (1), and whereupon sufficient match and confirmation the computing device and app (2a, 2-3, 2-4, 2-5) performs (function c1) and determines an association between the sample collection device ID and the person (1) for use at least in part for associating with a future assessment of the collected, taken, or otherwise obtained one or more of the biometric samples such that the future assessment is authenticated to be of the person (1), where the future assessment comprises one or more of the authenticated health measurements (2d);
face of person (1) recognition concurrent with scanning of an electronic tag (80-nfc, 84-nfc) to determine a sample collection device ID, where the biometric sample collection device (80, 84) collects, receives, takes, or otherwise obtains a bodily fluid or otherwise sample of the person (1) using a sample receptacle (80-tip, 84-smp), where a bodily fluid or otherwise sample comprises mucus, saliva, or blood, where the biometric sample collection device (80, 84) comprises an electronic tag (80-nfc, 84-nfc) for providing the sample collection device ID, where the computing device and app (2a, 2-3, 2-4, 2-5) comprises or otherwise communicates with a camera for capturing images and comprises electronic tag reading means for electronically scanning the electronic tag (80-nfc, 84-nfc) to determine the sample collection device ID, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) captures two or more images substantially comprising a concurrent view of the face of the person (1), the biometric sample collection device (80, 84), and the person (1) using the biometric sample collection device (80, 84) to collect, receive, take, or otherwise obtain a bodily fluid or otherwise sample of the person (1), where the computing device and app (2a, 2-3, 2-4, 2-5) (i) uses the computing device electronic tag reading means for determining the sample collection device ID by electronically scanning the electronic tag (80-nfc, 84-nfc), (ii) processes the two or more images for (ii-a) comparing the captured face of the person (1) with the pre-registered facial image, and (ii-b) confirming the substantial contact or otherwise sufficient proximity between the sample receptacle (80-tip, 84-smp) and the body of the person (1) for ensuring that each of any one or more of the collected, taken, or otherwise obtained biometric samples are of the person (1), and whereupon sufficient match and confirmation the computing device and app (2a, 2-3, 2-4, 2-5) performs (function c1) and determines an association between the sample collection device ID and the person (1) for use at least in part for associating with a future assessment of the collected, taken, or otherwise obtained one or more of the biometric samples such that the future assessment is authenticated to be of the person (1), where the future assessment comprises one or more of the authenticated health measurements (2d);
face of person (1) recognition concurrent with use of a companion device (3-1-d9, 3-1-d10) to provide a companion light signal with scanning of either visual markings (80-bcd, 84-bcd) or an electronic tag (80-nfc, 84-nfc) to determine a sample collection device ID, where the companion device (3-1-d9, 3-1-d10) comprises a light emitter (3-1-d9-led, 3-1-d10-led) for emitting the companion light signal and optionally comprises electronic tag reading means (3-1-d10-nfc) for electronically scanning the electronic tag (80-nfc, 84-nfc) to determine the sample collection device ID, where the biometric sample collection device (80, 84) collects, receives, takes, or otherwise obtains a bodily fluid or otherwise sample of the person (1) using a sample receptacle (80-tip, 84-smp), where a bodily fluid or otherwise sample comprises mucus, saliva, or blood, where the biometric sample collection device (80, 84) either or both comprises visual markings (80-bcd, 84-bcd) for use at least in part to determine the sample collection device ID and comprises an electronic tag (80-nfc, 84-nfc) for providing the sample collection device ID, where the computing device and app (2a, 2-3, 2-4, 2-5) comprises or otherwise communicates with a camera for capturing images and comprises electronic tag reading means for electronically scanning the electronic tag (80-nfc, 84-nfc) to determine the sample collection device ID, where at least one biometric of the person (1) pre-registered with the computing device and app (2a, 2-3, 2-4, 2-5) comprises a facial image, where at some time during or otherwise over some concurrent duration of the (function a) and the (function b1) the computing device and app (2a, 2-3, 2-4, 2-5) provides a control signal to the companion device (3-1-d9) while concurrently capturing two or more images substantially comprising a concurrent view of the face of the person (1), the companion device (3-1-d9, 3-1-d10), the biometric sample collection device (80, 84), any of the visual markings (80-bcd, 84-bcd) present on the biometric sample collection device (80, 84), and the person (1) using the biometric sample collection device (80, 84) to collect, receive, take, or otherwise obtain a bodily fluid or otherwise sample of the person (1), where the companion device (3-1-d9, 3-1-d10) while in the concurrent view of the camera and using at least in part the control signal causes the light emitter (3-1-d9-led, 3-1-d10-led) to emit a response signal comprising light detectable by the camera, where the computing device and app (2a, 2-3, 2-4, 2-5) determines the sample collection device ID by any one of or any combination of using the computing device electronic tag reading means to electronically scan the electronic tag (80-nfc, 84-nfc) and processing the two or more images for determining the sample collection device ID based at least in part upon the visual markings (80-bcd, 84-bcd), and where the computing device and app (2a, 2-3, 2-4, 2-5) (i) processes the two or more images for (i-a) comparing the captured face of the person (1) with the pre-registered facial image, (i-b) comparing the emitted response signal with the provided control signal, and (i-c) confirming the substantial contact or otherwise sufficient proximity between the sample receptacle (80-tip, 84-smp) and the body of the person (1) for ensuring that each of any one or more of the collected, taken, or otherwise obtained biometric samples are of the person (1), and whereupon sufficient matches and confirmation the computing device and app (2a, 2-3, 2-4, 2-5) performs (function c1) and determines an association between the sample collection device ID and the person (1) for use at least in part for associating with a future assessment of the collected, taken, or otherwise obtained one or more of the biometric samples such that the future assessment is authenticated to be of the person (1), where the future assessment comprises one or more of the authenticated health measurements (2d), and
face of person (1) recognition along with use of a companion device (3-1-d10) to provide a companion light signal and to scan an electronic tag to determine a sample collection device ID, where the companion device (3-1-d10) comprises a light emitter (3-1-d10-led) and an electronic tag reader (3-1-d10-nfc) for electronically scanning either of an embedded or an applied electronic tag (4-tag) to determine the sample collection device ID, where the biometric sample collection device is a breath analyzer sample collection device (4-2) comprising either of an embedded or an applied electronic tag (4-tag) for providing the sample collection device ID and comprising means for receiving and processing the expelled breath of the person (1) for use in a breath analysis resulting in one or more of the health measurements (2d), where the computing device and app (2a, 2-3, 2-4, 2-5) comprises or otherwise communicates with a camera for capturing images, where at least one biometric of the person (1) pre-registered with the computing device and app (2a, 2-3, 2-4, 2-5) comprises a facial image, where at some time during or otherwise over some concurrent duration of the (function a) and the (function b1) the person (1) concurrently uses the breath analyzer sample collection device (4-2) to receive breath from the person (1) and to perform the breath analysis of the person (1) and uses the companion device electronic tag reader (3-1-d10-nfc) to determine the sample collection device ID for provision to the computing device and app (2a, 2-3, 2-4, 2-5) while the computing device and app (2a, 2-3, 2-4, 2-5) provides a control signal to the companion device (3-1-d10) and concurrently captures two or more images substantially comprising a concurrent view of the face of the person (1), the companion device (3-1-d10), the breath analyzer sample collection device (4-2), and the person (1) using the breath analyzer sample collection device (4-2) to receive the breath from the person (1), where the companion device (3-1-d10) while in the concurrent view of the camera and using at least in part the control signal causes the light emitter (3-1-d10-led) to emit a response signal comprising light detectable by the camera, where the computing device and app (2a, 2-3, 2-4, 2-5) (i) processes the two or more images for (i-a) comparing the captured face of the person (1) with the pre-registered facial image, (i-b) comparing the emitted response signal with the provided control signal, and (i-c) confirming the substantial contact or otherwise sufficient proximity between the breath analyzer temporary engagement device (4-2) and the mouth of the person (1) for ensuring that the breath analysis comprising the one or more of the health measurements (2d) is of the person (1), and whereupon sufficient matches and confirmation the computing device and app (2a, 2-3, 2-4, 2-5) performs (function c1) and receives or otherwise accepts the one or more of the health measurements (2d) from the breath analyzer sample collection device (4-2) as being authenticated of the person (1).

6. The system of claim 5 for determining the one or more of the authenticated health measurements (2d) based at least in part upon the assessment of the one or more of the authenticated biometric samples, further comprising any one of:
a health service test lab that provides the person (1) with a health test kit (90-1) comprising the biometric sample collection device (80, 84) and a sealable biometric sample collection device container (82), where the two or more images captured by the computing device and app (2a, 2-3, 2-4, 2-5) at some time during or otherwise over some concurrent duration of the (function a) and the (function b1) comprising the person (1) using the biometric sample collection device (80, 84) to collect, receive, take, or otherwise obtain an authenticated bodily fluid or otherwise sample of the person (1) further or otherwise additionally and subsequently comprise two or more images of the person (1) placing at least the sample receptacle (80-tip, 84-smp) or otherwise the portion of the biometric sample collection device (80, 84) comprising the biometric sample into the sealable biometric sample collection device container (82) for ensuring that the sealable biometric sample collection device container (82) is authenticated as containing the authenticated biometric sample of the person (1), where the computing device and app (2a, 2-3, 2-4, 2-5) while processing the two or more images additionally confirms that at least the portion of the biometric sample collection device (80, 84) comprising the sample receptacle (80-tip, 84-smp) or otherwise the biometric sample was placed into the biometric sample collection device container (82) and that the container (82) was subsequently sealed, where after sealing the container (82) the person (1) uses any of available shipping services or otherwise delivery methods to provide the sealed container (82) containing at least the biometric sample receptacle (80-tip, 84-smp) or otherwise the biometric sample to the health service test lab, where the provision of the biometric sample receptacle (80-tip, 84-smp) or otherwise the biometric sample to the health services test lab is anonymous and comprises no personally identifying information, where the health service test lab determines the assessment comprising the one or more of the health measurements (2d) based at least in part upon the provided authenticated sample receptacle (80-tip, 84-smp) or otherwise the biometric sample and stores digital information indicative of the one or more of the health measurements (2d) in association with the sample collection device ID in an assessment database accessible by the computing device and app (2a, 2-3, 2-4, 2-5), and where at some time after the provision of the sealed container (82) to the health service test lab the person (1) uses the computing device and app (2a, 2-3, 2-4, 2-5) to perform (function c1) by accessing the assessment database to retrieve the one or more of the health measurements (2d) by at least in part providing the sample collection device ID to the assessment database and thereafter receiving and accepting the one or more of the health measurements (2d) determined by the health test lab assessment as being authenticated of the person (1), and
a health service test lab that provides the person (1) with a health test kit comprising the biometric sample collection device (80, 84) further comprising a reactive medium for providing and immediate assessment, where the sample receptacle (80-tip, 84-smp) further comprises or is otherwise placeable in contact with the sample reactive medium provided in the health test kit, where the sample reactive medium changes in visual appearance based at least in part upon coming into sufficient contact with the bodily fluid or otherwise the biometric sample of the person (1), where the changes in visual appearance are indicative of the one or more of the health measurements (2d), where the person (1) uses the computing device and app (2a, 2-3, 2-4, 2-5) to perform (function c1) by capturing at least one image of the sample reactive medium after the occurrence of any changes in the visual appearance of the sample reactive medium and thereafter processing the at least one image to determine the one or more of the health measurements (2d) based at least in part upon the visual appearance of the sample reactive medium, and where the computing device and app (2a, 2-3, 2-4, 2-5) accepts the one or more of the health measurements (2d) as being authenticated of the person (1).

7. The system of claim 1 wherein the at least one health measurement device (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) is any of a temporary engagement device (3-1-d1, 3-1-d2, 3-2, 3-1-d6, 3-1-d7, 3-1-d8, 3-1-d9, 4-2, 4-4), a sustained engagement wearable device (3-1-d4, 3-1-d5, 4-1a, 4-1b, 4-3, 131), or a biometric sample collection device (4-2, 80, 84), and where:
the temporary engagement device (3-1-d1, 3-1-d2, 3-2, 3-1-d6, 3-1-d7, 3-1-d8, 3-1-d9, 4-2, 4-4) is any of a finger-worn device (3-1-d2, 3-1-d7, 3-1-d8, 3-1-d9), a device inserted into a body orifice (3-1-d1), a remote sensing device (3-2, 3-1-d6), a device for analyzing the person's (1) breath (4-2), or a device for measuring the person's (1) body weight or otherwise body exerted forces and related data (4-4);
the sustained engagement wearable device (3-1-d4, 3-1-d5, 4-1a, 4-1b, 4-3, 131) is any of a wrist, neck, ankle, waist, torso, or finger worn device (3-1-d4, 3-1-d5, 4-3), a face-worn device or mask (131), and a skin adhered device or patch (4-1a, 4-1b), and
the biometric sample collection device (4-2, 80, 84) is any of a swab (80) comprising a swab tip (80-tip), a test strip (84) comprising a sample collection area (84-smp), or a breathalyzer (4-2).

8. The system of claim 1 where the healthcare service provider (42) provides the at least one healthcare service (3-2), and when providing the at least one healthcare service (3-2) performing the (function 2) comprising the combination of the (function a), the (function b2), and the (function c2-2) comprises:
the person (1) using the computing device and app (2a, 2-3, 2-4, 2-5) to perform the (function a) and the (function b2) while substantially isolated within a confirmation zone (73), where during the (function b2) the computing device and app (2a, 2-3, 2-4, 2-5) additionally or alternatively provides to any of a system or service agent of the healthcare service provider (42) a certificate of authentication or otherwise confirmation information comprising any one of or any combination of an app (2a) identity code or unique digital certificate, a unique transaction number, insurance information, a response code caused at least in part by an initial code previously provided by the system or the service agent, an image of the person (1), or other data confirming that the computing device and app (2a, 2-3, 2-4, 2-5) has determined in the (function a) that the person (1) currently isolated within the confirmation zone (73) is authenticated by the computing device and app (2a, 2-3, 2-4, 2-5) to be the pre-registered person (1), where the certificate of authentication or otherwise confirmation information is provided in a format comprising any of electronically transmitted information or visually presented information presented on a screen of the computing device and app (2a, 2-3, 2-4, 2-5) and does not comprise any personally identifying information beyond an image of the person (1) such that the person (1) remains anonymous, whereafter receiving the certificate of authentication or otherwise confirmation the service agent or otherwise any agent of the healthcare service provider (42) provides the at least one healthcare service (3-2) to the person (1), whereafter providing the at least one healthcare service (3-2) the system or a service agent of the healthcare provider (42) transmits the confirmation-of-service receipt to the computing device and app (2a, 2-3, 2-4, 2-5) authenticating that the at least one healthcare service (3-2) was provided to the person (1), where the confirmation-of-service receipt comprises any one of or any combination of an identification of the healthcare service provider (42), an identification of the one or more service agents, an identification of the at least one healthcare service (3-2) provided, insurance information, any of the certificate of authentication or otherwise confirmation information, or otherwise transaction data related to the at least one provided healthcare service (3-2), where the confirmation-of-service receipt is provided in a format comprising any of electronically transmitted information or visually presented information, and where the computing device and app (2a, 2-3, 2-4, 2-5) performs (function c2-2) and receives the confirmation-of-service receipt for use at least in part in confirming that the at least one healthcare service (3-2) has been provided to the associated anonymous person (1).

9. The system of claim 1 where the access control system (73) comprises at least one controlled health-verified access point (5a, 5b) separating and co-joining a confirmation zone (73) and the premises or gathering (74), where the confirmation zone (73) comprises any of a physically, electronically, or visually restricted, semi-restricted, or otherwise monitored area (73) for substantially isolating the person (1) while anonymously receiving the authenticated health status (2d) sufficient to ensure that the received status (2d) is of the person (1), where the access control system (73) performs (function 3) by communicating with the computing device and app (2a, 2-3, 2-4, 2-5) to negotiate the request-for-entry while the person (1) is within the confirmation zone (73), and where the access control system (73) governs the access to the premises or gathering (74) by the person (1) through the at least one controlled health-verified access point (5a, 5b) based at least in part upon the received anonymous authenticated health status (2d).

10. The system of claim 9 wherein the person (1) uses the computing device and app (2a, 2-3, 2-4, 2-5) while within the confirmation zone (73) to additionally provide any one of or any combination of:
personally identifying information, and
electronic information substantiating a ticketed right-of-entry (2b, 2c) into the premises or gathering (74), where the ticketed right-of-entry (2b, 2c) is either for anonymous public person (1) entrance or nonanonymous private person (1-w) entrance, where the access control system (73) governs the access to the premises or gathering (74) by the public person (1) or the private person (1-w) based at least in part upon the received authenticated health status (2d) and at least in part upon the authenticated right-of-entry (2b, 2c).

11. The system of claim 1 wherein a multiplicity of persons (1) have received and are following any of the one or more public regiments (2d-1, 2d-2) for determining the privately authenticated health status (2d) of each person (1) comprising the multiplicity of persons (1), further comprising:
a shared public health database (102-db) for receiving anonymized private health data (2a-db) from any of the multiplicity of persons (1), where the anonymized private health data (2a-db) comprises any one of or any combination of information related to the health status (2d) of each of the any of the multiplicity of persons (1), and
one or more public health algorithms (102-ml) for processing any of the anonymized private health data (2a-db) comprising the shared public health database (102-db), where the one or more public health algorithms (102-ml) determine one or more broadcast messages (102-msg) based at least in part upon any of the processing, where each of the one or more broadcast messages (102-msg) comprises at least one trained detector (102-det), no personally identifying information, and any one of or any combination of recommendations or updates to any of the one or more public regiments (2d-1, 2d-2), where the one or more broadcast messages (102-msg) are electronically distributed to the computing devices and apps (2a, 2-3, 2-4, 2-5) being used by some or all of the multiplicity persons (1), whereupon receiving a broadcast message (102-msg) the receiving computing device and app (2a, 2-3, 2-4, 2-5) executes the trained detector (102-det) for processing any of the private health data (2a-db) of the each person (1) associated with the receiving computing device and app (2a, 2-3, 2-4, 2-5) to determine one or more private results, and where the receiving computing device and app (2a, 2-3, 2-4, 2-5) either or both provides the recommendations to the associated each person (1) and updates any of the one or more public regiments (2d-1, 2d-2) currently in use by the associated each person (1) based at least in part upon the private results of the each person (1).

12. The system of claim 1 where the premises or gathering (74) is or otherwise occurs in any of a fixed structure or a movable transportation vehicle owned or operated by an entity (4), where the access control system (73) for governing access to the premises or gathering (74) comprises one or more public or private access points (5a, 5b) each for governing the public or private access by any of a multiplicity of persons (1, 1-w) based at least in part upon receiving a sufficiently compliant health status (2d) from the persons (1, 1-w) attempting access, wherein the entity (4) provides mutual assurance information regarding the current health state of the premises or gathering (74) to any of persons (1, 1-w) considering access, currently seeking access, or already having accessed the premises or gathering (74), where the mutual assurance information is any indication of the health status (2d) of some or substantially all persons (1, 1-w) currently within, occupying, or otherwise attending the premises or gathering (74) at or over some period of time, and where the mutual assurance information is based at least in part upon the health status (2d) provided by each person (1, 1-w) permitted into or onto the premises or gathering (74) by the access control system (73) during the period of time.

13. The system of claim 1 where the premises or gathering (74) is or otherwise occurs in any of a fixed structure or a movable transportation vehicle owned or operated by an entity (4), where the access control system (73) for governing access to the premises or gathering (74) comprises one or more public or private access points (5a, 5b) each for governing the public or private access by any person (1, 1-w) of a multiplicity of persons (1, 1-w) based at least in part upon receiving a sufficiently compliant health status (2d) from the any person (1, 1-w) attempting access, where one or more of the any persons (1, 1-w) admitted access into the premises or gathering (74) by the access control system (73) are a tracked admitted person wearing a secured wearable (2-3, 3-1-d4, 3-1-d5) comprising electronic tracking means, where the premises or gathering (74) comprises a sufficient tracking infrastructure network positioned throughout some or substantially all of the premises or gathering (74) for substantially tracking the movements of all secured wearables (2-3, 3-1-d4, 3-1-d5) being worn by the one or more tracked admitted persons (1, 1-w) sufficient for determining contacts within a given proximity between any two or more tracked admitted persons (1, 1-w) currently wearing a secured wearable (2-3, 3-1-d4, 3-1-d5) and currently within the premises or gathering (74), where the entity (4) provides contract tracing information (2a-db-6) to any of the one or more tracked admitted persons (1, 1-w) currently wearing or having worn a secured wearable (2-3, 3-1-d4, 3-1-d5) and currently present or having been present at the premises or gathering (74) based at least in part upon any of the determined contacts involving the tracked admitted person (1, 1-w) and at least in part upon the health status (2d) of any other one or more of the other tracked admitted persons (1, 1-w) currently wearing or having worn a secured wearable (2-3, 3-1-d4, 3-1-d5) and involved with the determined contacts.

14. The system of claim 13 where either or both occurs:
at least one of the tracked admitted persons (1, 1-w) after being admitted to the premises or gathering (74) remains at the premises or gathering (74) for a sufficient duration of time to require the determination of at least one additional health measurement (2d, 2a-db-4a) or at least one additional healthcare service (2d, 2a-db-4a) for use in determining an updated health status (2d) in order to remain compliant with the one or more public regiments (2d-1, 2d-2) after remaining at the premises or gathering (74), where the entity (4) requires that the at least one tracked admitted and remaining person (1, 1-w) continues to follow and remain compliant with the one or more public regiments (2d-1, 2d-2), where the entity (4) continues to determine and otherwise receive the updated health status (2d) of the at least one tracked admitted and remaining person (1-w) throughout the duration of time that the at least one tracked admitted and remaining person (1, 1-w) is within, occupying, or otherwise attending the premises or gathering (74), and whereupon a sufficient change in the health status (2d) of the at least one tracked admitted and remaining person (1, 1-w) the entity (4) provides contract tracing information to any one or more other tracked admitted persons (1, 1-w) that were prior determined by the entity (4) to have come within sufficient proximity contact with the at least one tracked admitted and remaining person (1, 1-w) while concurrently being present at the premises or gathering (74), and
at least one of the tracked admitted persons (1, 1-w) after being admitted to the premises or gathering (74) leaves the premises or gathering (74) for a sufficient duration of time to require the determination of at least one additional health measurement (2d, 2a-db-4a) or at least one additional healthcare service (2d, 2a-db-4a) for use in determining an updated health status (2d) in order to remain compliant with the one or more public regiments (2d-1, 2d-2) after having left the premises or gathering (74), where the entity (4) requires that the at least one tracked admitted and leaving person (1, 1-w) continues to follow and remain compliant with the one or more public regiments (2d-1, 2d-2) after leaving the premises or gathering (74) for an extended duration of time greater than or equal to a sufficient duration of time, where the entity (4) continues to determine and otherwise receive the updated health status (2d) of the at least one tracked admitted and leaving person (1, 1-w) throughout the extended duration of sufficient time after which the at least one tracked admitted and leaving person (1, 1-w) has left the premises or gathering (74), and whereupon a sufficient change in the health status (2d) of the at least one tracked admitted and leaving person (1, 1-w) after leaving the premises or gathering (74) the entity (4) provides contract tracing information to any one or more other tracked admitted persons (1, 1-w) that were prior determined by the entity (4) to have come within sufficient proximity contact with the at least one tracked admitted and leaving person (1, 1-w) while concurrently being present at the premises or gathering (74).

15. The system of claim 1 where the premises or gathering (74) is owned or operated by an entity (4), where the person (1) schedules access to the premises or gathering (74), further comprising either one of:
a person-to-service appointment system (104, 52) operated by or for the entity (4), wherein:
the person (1) interacts with the person-to-service appointment system (104, 52) to schedule at least a date or a date and time of a planned access to the premises or gathering (74), whereupon or after interacting with the person-to-service appointment system (104, 52) the person-to-service appointment system (104, 52) provides or otherwise makes available to the computing device and app (2a, 2-3, 2-4, 2-5) registered to and being used by the person (1) an appointment regiment (2d-3a) comprising any of information sufficient for serving as, updating, amending, or otherwise constituting a part or all of a health regiment (2d-1, 2d-2) for compliance by the person (1) prior to attempting the scheduled access, where prior to attempting the scheduled access to the premises or gathering (74) the computing device and app (2a, 2-3, 2-4, 2-5) provides one or more verification tokens (2d-3b) comprising any of verification datum based at least in part upon any one of or any combination of health information relating to any of the appointment regiment (2d-3a) or the one or more public regiments (2d-1, 2d-2), where health information comprises information related to the health status (2d) of the person (1), where the person-to-service appointment system (104, 52) upon receiving the one or more verification tokens (2d-3b) acts to do any one of or any combination of:
(i) rescheduling the date or the date and time of the planned access to the premises or gathering (74);
(ii) providing or otherwise making available to the computing device and app (2a, 2-3, 2-4, 2-5) updates to or otherwise a new appointment regiment (2d-3a);
(iii) alerting or otherwise updating any one of or any combination of one or more personnel working for the entity (4) or otherwise expected to interact with the person (1) when or after the person (1) accesses the premises or gathering (74);
(iv) providing an access token (2d-3c) to the computing device and app (2a, 2-3, 2-4, 2-5), where the access token (2d-3c) is based at least in part upon sufficient compliance by the person (1) with at least one appointment regiment (2d-3a) provided by the scheduling system (104, 52), and
where upon interacting with the access control system (73) at the scheduled date or date and time of the planned access the person (1) uses the computing device and app (2a, 2-3, 2-4, 2-5) to provide any of access token information based at least in part upon the access token (2d-3c) either alternatively or additionally to providing the anonymous, authenticated health status (2d), and where the access control system (73) governs access by the person (1) to the premises or gathering (74) based at least in part upon the access token (2d-3c) information,
and
a person-to-person appointment system (105, 53) operated by or for the entity (4) or otherwise one or more people managing a gathering between two or more persons (1), wherein:
the two or more persons (1) interact with the person-to-person appointment system (105, 53) to schedule at least a date or a date and time of a planned gathering or otherwise meeting (74), where upon interacting with the person-to-person appointment system (105, 53) the person-to-person appointment system (105, 53) provides or otherwise makes available to each of the computing devices and apps (2a, 2-5-1, 2-5-2) registered to and being used by each of the two or more persons (1) an appointment regiment (2d-3a-1, 2d-3a-2) comprising any of information sufficient for serving as, updating, amending, or otherwise constituting a part or all of a health regiment (2d-1, 2d-2) for compliance by the two or more persons (1) prior to attempting to participate in the planned gathering or otherwise meeting (74), where prior to attempting to participate in the planned gathering or otherwise meeting (74) the each computing device and app (2a, 2-5-1, 2-5-2) provides one or more verification tokens (2d-3b-1, 2d-3b-2) comprising any of verification datum based at least in part upon any one of or any combination of health information relating to any of the appointment regiment (2d-3a-1, 2d-3a-2) or the one or more public regiments (2d-1, 2d-2), where health information comprises information related to the health status (2d) of any of the two or more persons (1), where the person-to-person appointment system (105, 53) upon receiving the one or more verification tokens (2d-3b-1, 2d-3b-2) acts to do any one of or any combination of:
(i) rescheduling the date or the date and time of the planned gathering or otherwise meeting (74);
(ii) providing or otherwise making available to any of the each computing devices and apps (2a, 2-5-1, 2-5-2) updates to or otherwise a new appointment regiment (2d-3a-1, 2d-3a-2);
(iii) alerting or otherwise updating any one of or any combination of one or more personnel working for the entity (4), managing the gathering or otherwise meeting (74), or otherwise expected to interact with any of the two or more persons (1) when or after the any of the two or more persons (1) accesses the planned gathering or otherwise meeting (74), and any of the two or more persons (1) planning to attend the gathering or otherwise meeting (74);
(iv) providing an access token (2d-3c-1, 2d-3c-2) to any of the each computing devices and apps (2a, 2-5-1, 2-5-2), where the each provided access token (2d-3c-1, 2d-3c-2) is based at least in part upon sufficient compliance by the each person (1) of the two or more persons (1) with at least one appointment regiment (2d-3a-1, 2d-3a-2) provided by the scheduling system (105, 53), and
where after receiving an access token (2d-3c) the each person (1) of the two or more persons (1) is enabled to attend the planned gathering or otherwise meeting (74), and where attending optionally comprises either of interacting with the access control system (73) at the scheduled date or date and time of the planned gathering or otherwise meeting (74) to provide any of access token information based at least in part upon the received access token (2d-3c-1, 2d-3c-2) either alternatively or additionally to providing the anonymous, authenticated health status (2d), or interacting with any of the each computing devices and apps (2a, 2-5-1, 2-5-2) being used by any other of the each two or more persons (1) to provide any of access token information based at least in part upon the received access token (2d-3c) either alternatively or additionally to providing the anonymous, authenticated health status (2d).

## Patentansprüche

1. System (102, 103, 104) für , das den Zugang einer Person (1) zu einem Ort oder einer Versammlung (74) zumindest teilweise auf der Grundlage eines anonymen, öffentlich konformen, authentifizierten Gesundheitszustands (2d) der Person (1) regelt, wobei die Person (1) ein Computergerät und eine App (2a, 2-3, 2-4, 2-5) als Mittel zur Bereitstellung einer privaten Selbstauthentifizierung verwendet, umfassend:
ein Zugangskontrollsystem (73) zur Regelung des Zugangs der Person (1) zu dem Ort oder der Versammlung (74);
das Computergerät (2a, 2-3, 2-4, 2-5), das von der Person (1) betrieben wird, die Zugang zu zu dem Ort oder der Versammlung (74) sucht;
mindestens ein authentifizierendes Gesundheitsmessgerät (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) oder mindestens einem authentifizierenden Gesundheitsdienstleister (42), wobei das mindestens eine Gesundheitsmessgerät (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131) mindestens eine Gesundheitsmessung (2d, 2a-db-4a) bestimmt, die als von der Person (1) stammend authentifiziert ist, wobei der mindestens eine Gesundheitsdienstleister (42) mindestens eine Gesundheitsmessung (2d, 2a-db-4a) bestimmt, die als von der Person (1) stammend authentifiziert ist,
oder mindestens eine Gesundheitsdienstleistung (2d, 2a-db-4a) erbringt, die als für die Person (1) erbracht authentifiziert ist;
eine Intermediär-App (2a), die auf dem Computergerät (2a, 2-3, 2-4, 2-5) ausgeführt wird, wobei die Person (1) eine oder mehrere biometrische Daten bei der Intermediär-App (2a) für die spätere Verwendung während einer (Funktion a) zur Authentifizierung der Person (1) registriert, wobei die (Funktion a) die Bestätigung der Identität der Person (1) durch Bestimmen und Vergleichen einer aktuellen biometrischen Daten mit der einen oder den mehreren registrierten biometrischen Daten umfasst;
ein oder mehrere öffentliche Gesundheitsregime (2d-1, 2d-2) zum Bestimmen der öffentlichen Konformität des authentifizierten Gesundheitsstatus (2d), wobei das eine oder die mehreren öffentlichen Gesundheitsregime (2d-1, 2d-2) digitale Informationen umffassen, die eine oder mehrere Regeln spezifizieren , wobei ein Gesundheitsregime (2d-1, 2d-2) von der Intermediär-App (2a) verschieden ist, wobei die Intermediär-App (2a) zusätzlich die Funktion hat, das eine oder die mehreren öffentlichen Gesundheitsregime (2d-1, 2d-2) zu empfangen, abzurufen, zu aktualisieren oder anderweitig zu erhalten oder Regeln aus zwei oder mehreren öffentlichen Gesundheitsregime (2d-1, 2d-2) zu kombinieren oder
anderweitig zu verarbeiten , wobei eine Regel definiert, die Verarbeitung der Intermediär-App (2a) bei der Bestimmung der mindestens einen authentifizierten Gesundheitsmessung (2d, 2a-db-4a) und des mindestens einen authentifizierten Gesundheitsdienstes (2d, 2a-db-4a) definiert,
einschränkt oder anderweitig regelt, und wobei die Regeln eine Formulierung jeglicher Information spezifizieren, die mit einer oder einer Kombination der mindestens einen authentifizierten Gesundheitsmessung (2d, 2a-db-4a) und des mindestens einen authentifizierten Gesundheitsdienstes (2d, 2a-db-4a) zur Verwendung bei der Bestimmung und anderweitigen Aufrechterhaltung des öffentlich konformen authentifizierten Gesundheitsstatus (2d) der Person (1) verbunden ist, und
wobei die Person (1), bevor sie in einer (Funktion 3) versucht, zu dem Ort oder der Versammlung (74) zu erhalten, zumindest teilweise die Rechenvorrichtung und die Intermediär-App (2a, 2-3, 2-4, 2-5) verwendet,
um eine beliebige oder eine Kombination aus einer (Funktion 1) und einer (Funktion 2) durchzuführen, wobei:
die (Funktion 1) das Kommunizieren mit dem mindestens einen Gesundheitsmessgerät (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131), umfasst, um die mindestens eine authentifizierte Gesundheitsmessung (2d, 2a-db-4a) zu initiieren, wobei während oder nach der Initiierung das Computergerät und die zwischengeschaltete App (2a, 2-3, 2-4, 2-5) die (Funktion a) ausführt, um die Identität der Person (1) zu bestätigen, indem sie mindestens ein biometrisches Merkmal zum Vergleich mit einem biometrischen Registrierungsmerkmal bestimmt, während sie im Wesentlichen gleichzeitig eine (Funktion b1) ausführt, um zu bestätigen, dass eine Erfassungsfläche des Geräts (3-1, 3-1-d1, 3-1-d2, 3-1-d4, 3-1-d5, 3-2, 3-3, 3-1-d1, 3-1-d2, 3-1-d4, 3-1-d5, 3-2, 3-1-d6, 3-1-d7, 3-1-d8, 3-1-d9, 3-1-d10, 4-1a, 4-1b, 4-2, 4-3, 4-4, 80, 84, 131) den Körper der Person (1) im Wesentlichen berührt, sich in unmittelbarer Nähe befindet oder anderweitig mit dem Körper der Person (1) zusammen angeordnet ist, um sicherzustellen, dass die mindestens eine Gesundheitsmessung an der Person (1) vorgenommen wird , und gleichzeitiges oder anschließendes Ausführen einer (Funktion c1) zum Empfangen, Abrufen oder anderweitiges Erhalten der mindestens einen Gesundheitsmessung (d2, 2a-db-4a) von dem Gerät (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131), wobei die empfangene Gesundheitsmessung (2d, 2a-db-4a) darin als von der Person (1) stammend authentifiziert wird, und
die (Funktion 2) die Kommunikation mit dem mindestens einen Gesundheitsdienstleister (42) umfasst, um die Initiierung der mindestens einen Gesundheitsmessung (2d, 2a-db-4a) oder des mindestens einen Gesundheitsdienstes (3-2) zu initiieren oder darauf zu reagieren, wobei während oder nach der Initiierung die Computer- und Intermediär-App (2a, 2-3, 2-4, 2-5) während oder nach der Initiierung die (Funktion a) ausführt, um die Identität der Person (1) zu bestätigen, indem sie mindestens ein biometrisches Merkmal zum Vergleich mit einem biometrischen Merkmal der Registrierung bestimmt, während sie im Wesentlichen gleichzeitig eine (Funktion b2) ausführt, um anonyme Informationen an den Gesundheitsdienstleister (42) zu übermitteln, um sie mit der mindestens einen Gesundheitsmessung (2d, 2a-db-4a) oder der mindestens einen Gesundheitsdienstleistung (3-2) zu verknüpfen, wobei die anonymen Informationen keine persönlich identifizierenden Informationen über die Person (1) umfassen, und wobei, wenn der Gesundheitsdienstleister (42) mindestens eine Gesundheitsmessung (2d, 2a-db-4a) bestimmt hat, dann führt die Rechenvorrichtung und die zwischengeschaltete App (2a, 2-3, 2-4, 2-5) gleichzeitig oder danach eine (Funktion c2-1) aus, um die mindestens eine Gesundheitsmessung (2d, 2a-db-4a) von dem Gesundheitsdienstleister (42) zu empfangen, abzurufen oder anderweitig zu erhalten, und wenn der Gesundheitsdienstleister (42) mindestens eine Gesundheitsdienstleistung (3-2) erbracht hat, dann führt die Rechenvorrichtung und die zwischengeschaltete App (2a, 2-3, 2-4, 2-5) gleichzeitig oder danach eine 2-5) eine (Funktion c2-2) aus, um eine Leistungsbestätigung von dem Gesundheitsdienstleister (42) zu empfangen, abzurufen oder anderweitig zu erhalten, die bestätigt, dass die mindestens eine Gesundheitsdienstleistung (3-2) für die zugehörige anonyme Person (1) erbracht wurde, wobei die empfangene, abgerufene oder anderweitig erhaltene mindestens eine Gesundheitsmessung (2d, 2a-db-4a) oder mindestens eine Gesundheitsdienstleistung (3-2) darin als von der Person (1) stammend authentifiziert wird, und
wobei die Person (1), nachdem sie das Computergerät und die App (2a, 2-3, 2-4, 2-5) zumindest teilweise verwendet hat, um eine oder eine Kombination der (Funktion 1) und der (Funktion 2) auszuführen, dann das Computergerät und die dazwischenliegende App (2a, 2-3, 2-4, 2-5), um die (Funktion 3) auszuführen, die darin besteht, mit dem Zugangskontrollsystem (73) zu kommunizieren, bevor das Zugangskontrollsystem (73) der Person (1) den Zugang zu einem Ort oder einer Versammlung (74) gewährt oder verweigert, wobei während der (Funktion 3) das Computergerät und die zwischengeschaltete App (2a, 2-3, 2-4, 2-5) in der Weise funktionieren, dass sie eine Zutrittsgewährung anfordern, wobei der Prozess der Zutrittsanforderung die (Funktion a) umfasst, um die Identität der Person (1) zu bestätigen, indem mindestens ein biometrisches Merkmal zum Vergleich mit einem biometrischen Merkmal der Registrierung bestimmt wird, und eine (Funktion d) auszuführen, die den authentifizierten Gesundheitsstatus (2d) bezüglich der Person (1) an das Zugangskontrollsystem (73) liefert, wobei der authentifizierte Gesundheitsstatus (2d) anonym ist und keine persönlich identifizierenden Informationen bezüglich der Person (1) umfasst, wobei der authentifizierte Gesundheitsstatus (2d) zumindest teilweise auf der Grundlage einer oder einer Kombination der mindestens einen authentifizierten Gesundheitsmessung (2d, 2a-db-4a) der Person (1) und der mindestens einen authentifizierten Gesundheitsdienstleistung (3-2), die der Person (1) zur Verfügung gestellt wird, bestimmt wird, und wobei das Zugangskontrollsystem (73) zu dem Ort oder der Versammlung (74) zumindest teilweise auf der Grundlage des öffentlich einwilligungsfähigen anonymen authentifizierten Gesundheitsstatus (2d) der Person (1) erlaubt oder verweigert.

2. System nach Anspruch 1, wobei die (Funktion b1) die Durchführung einer kooperativen Datenaustauschfunktion zwischen dem Computergerät und der App (2a, 2-3, 2-4, 2-5) und dem mindestens einen Gesundheitsmessgerät (3-1, 3-1-d1, 3-1-d2, 3-1-d4, 3-1-d5, 3-2, 3-2d, 3-3d, 3-1-d6, 3-1-d7, 3-1-d8, 3-1-d6, 3-1-d7, 3-1-d8, 3-1-d10, 4-1a, 4-1b, 4-2, 4-3, 4-4, 131), wobei die gemeinsam genutzten Daten eines oder eine beliebige Kombination aus einem von einem Gerät gemessenen räumlichen oder zeitlichen Körpermuster der Person (1), einem emittierten Lichtsignal, das auf ein von dem Computergerät und der App (2a, 2-3, 2-4, 2-5), Informationen, die einen gesicherten Zustand des mindestens einen Gesundheitsmessgeräts (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131) anzeigen, sichtbare Gerätemarkierungen und eine elektronisch bereitgestellte ID, wobei die gemeinsam genutzten Daten auf ausreichende Übereinstimmung mit nicht vom Gerät gelieferten Informationen verglichen werden, die entweder dem Computergerät und der App vorher bekannt sind (2d, 2a-db-4a) oder dem Computergerät und der App gleichzeitig von einem Begleitgerät (3-1-d9, 3-1-d10) bereitgestellt werden (2d, 2a-db-4a).

3. System nach Anspruch 1 , wobei die mindestens eine Gesundheitsmessvorrichtung (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) eine temporäre Eingriffsvorrichtung (3-1-d1, 3-1-d2, 3-2, 3-1-d6, 3-1-d7, 3-1-d1, 3-1-d2, 3-2, 3-1-d6, 3-1-d7, 3-1-d8, 3-1-d9, 4-4) ist, und wobei bei Verwendung der temporären Eingriffsvorrichtung (3-1-d1, 3-1-d2, 3-2, 3-1-d6, 3-1-d7, 3-1-d8, 3-1-d9, 4-4) die Durchführung der (Funktion 1), die die Kombination der (Funktion a), der (Funktion b1) und der (Funktion c1), eine der folgenden ist:
Erkennung des Gesichts der Person (1) gleichzeitig mit einer Lichtsignalbestätigung, wobei die temporäre Einschaltvorrichtung eine Lichtsignalvorrichtung (3-1-d1, 3-1-d7, 3-1-d8, 3-1-d9) ist, die einen Lichtsender (3-1-d1-led, 3-1-d7-led, 3-1-dt8-led, 3-1-d9-led) und einen oder mehrere Sensoren zur Bestimmung einer oder mehrerer Gesundheitsmessungen (2d) der Person (1) umfasst, wobei das Computergerät und die App (2a, 2-3, 2-4, 2-5) eine Kamera zur Aufnahme von Bildern umfasst oder anderweitig damit kommuniziert, wobei mindestens ein biometrisches Merkmal der Person (1), das bei dem Computergerät und der App (2a, 2-3, 2-4, 2-5) vorregistriert ist, ein Gesichtsbild umfasst, wobei die Rechenvorrichtung und App (2a, 2-3, 2-4, 2-5) zu irgendeinem Zeitpunkt während oder anderweitig über eine gleichzeitige Dauer der (Funktion a) und der (Funktion b1) (i) ein Steuersignal an die lichtsignalgebende temporäre Einschaltvorrichtung (3-1-d1, 3-1-d7, 3-1-d8, 3-1-d9) liefert, (ii) zwei oder mehr Bilder aufnimmt, die im Wesentlichen eine gleichzeitige Ansicht des Gesichts der Person (1) und der lichtsignalgebenden temporären Eingriffsvorrichtung (3-1-d1, 3-1-d7, 3-1-d8, 3-1-d9) umfassen, die sich im Wesentlichen in Kontakt mit oder in ausreichender Nähe zum Körper der Person (1) befindet, um eine oder mehrere der Gesundheitsmessungen (2d) zu bestimmen, wobei die lichtsignalgebende temporäre Eingriffsvorrichtung (3-1-d1, 3-1-d7, 3-1-d8, 3-1-d9), während sie sich im gleichzeitigen Blickfeld der Kamera befindet und zumindest teilweise das Steuersignal verwendet, den Lichtemitter (3-1-d1-led, 3-1-d7-led, 3-1-dt8-led, 3-1-d9-led) veranlasst, ein Antwortsignal auszusenden, das von der Kamera detektierbares Licht umfasst, wobei die Rechenvorrichtung und die App (2a, 2-3, 2-4, 2-5) (iii) die zwei oder mehr Bilder verarbeitet, um (iii-a) das erfasste Gesicht der Person (1) mit dem vorregistrierten Gesichtsbild zu vergleichen, (iii-b) das ausgesendete Antwortsignal mit dem bereitgestellten Steuersignal zu vergleichen, und (iii-c) Bestätigen des wesentlichen Kontakts oder der anderweitig ausreichenden Nähe zwischen der Lichtsignaleinrichtung (3-1-d1, 3-1-d7, 3-1-d8, 3-1-d9) und dem Körper der Person (1), um sicherzustellen, dass eine oder mehrere der Gesundheitsmessungen (2d) von der Person (1) stammen, und woraufhin bei ausreichender Übereinstimmung und Bestätigung das Computergerät und die App (2a, 2-3, 2-4, 2-5) die gesicherte eine oder mehrere der Gesundheitsmessungen (2d) von der Lichtsignaleinrichtung (3-1-d1, 3-1-d7, 3-1-d8, 3-1-d9) empfängt oder anderweitig als von der Person (1) stammend akzeptiert (Funktion c1); Abgleich des räumlichen Musters des spezifischen Körperortes der Person (1) gleichzeitig mit der kolokalisierten Gesundheitsmessung , wobei die temporäre Eingriffsvorrichtung eine temporäre Eingriffsvorrichtung (3-1-d2, 3-1-d6, 3-1-d9) mit räumlichen Körpermustern ist, die eine Einrichtung zur gleichzeitigen Bestimmung sowohl eines räumlichen Vergleichs-Körpermusters als auch einer oder mehrerer Gesundheitsmessungen (2d) von im Wesentlichen demselben spezifischen räumlichen Körperort der Person (1) umfasst, wobei die spezifische räumliche Körperstelle einen Finger, ein Gesicht oder eine andere eindeutig identifizierbare Stelle auf dem Körper der Person (1) umfasst, wobei das räumliche Körpermuster ein eindeutig identifizierendes Muster des Körpers der Person (1) umfasst, das an der spezifischen räumlichen Körperstelle bestimmt wird, wobei das eindeutig identifizierende Muster einen Fingerabdruck, ein Gesichtsbild oder ein anderes eindeutig identifizierbares räumliches Körpermuster umfasst, wobei das vorregistrierte biometrische Merkmal der Person (1) das räumliche Körpermuster der Person (1) umfasst, das im Wesentlichen an demselben spezifischen räumlichen Körperort aufgenommen wurde, wobei zu irgendeinem Zeitpunkt während oder anderweitig über eine gleichzeitige Dauer der (Funktion a) und der (Funktion b1) die Rechenvorrichtung und die App (2a, 2-3, 2-4, 2-5) (i) von der Vorrichtung (3-1-d2, 3-1-d6, 3-1-d9) zur temporären Erfassung des räumlichen Körpermusters das räumliche Vergleichs-Körpermuster empfängt, und (ii) das räumliche Vergleichs-Körpermuster mit einem vorregistrierten räumlichen Körpermuster vergleicht, und woraufhin die Rechenvorrichtung und die App (2a, 2-3, 2-4, 2-5) eine ausreichende Übereinstimmung durchführen (Funktion c1) und die eine oder mehrere der kolokierten Gesundheitsmessungen (2d) von der temporären Eingriffsvorrichtung (3-1-d2, 3-3, 3-1-d6, 3-1-d9) mit räumlichem Körpermuster als von der Person (1) authentifiziert empfängt oder anderweitig akzeptiert;
Verwendung einer Begleitvorrichtung (3-1-d9), um ein räumliches Begleit-Körpermuster zusammen mit einem gleichzeitigen und kolokalisierten zeitlichen Begleit-Körpermuster bereitzustellen, wobei die Begleitvorrichtung (3-1-d9) Mittel umfasst, um gleichzeitig von im Wesentlichen demselben spezifischen räumlichen Körperort der Person (1) sowohl das räumliche Begleit-Körpermuster (3-1-d9-d1) als auch das zeitliche Begleit-Körpermuster (3-1-d9-d2) zu bestimmen, wobei die gleiche spezifische räumliche Körperstelle einen Finger oder eine andere eindeutig identifizierbare Stelle am Körper der Person (1) umfasst, wobei ein räumliches Körpermuster (3-1-d9-d1) ein eindeutig identifizierendes Muster des Körpers der Person (1) umfasst, das an der gleichen spezifischen räumlichen Körperstelle bestimmt wurde, wobei das eindeutig identifizierende Muster einen Fingerabdruck oder ein anderweitig eindeutig identifizierbares räumliches Körpermuster umfasst, wobei das begleitende zeitliche Körpermuster eine Messsequenz von Herzschlägen der Person (1) umfasst,
wobei die temporäre Eingriffsvorrichtung eine temporäre Körpermuster-Eingriffsvorrichtung (4-4) ist, die Mittel zum Bestimmen eines zeitlichen Musterkörpermusters (4-4-d2) umfasst, das eine Messsequenz der Herzschläge der Person (1) umfasst, die von einem anderen Körperort als demselben spezifischen räumlichen Körperort der Person (1) genommen wurde, und die einen oder mehrere Sensoren umfasst, die in wesentlichen Kontakt kommen, (2d) der Person (1) zu bestimmen,
wobei mindestens ein biometrisches Merkmal der Person (1), das mit dem Computergerät und der App vorregistriert ist (2a, 2-3, 2-4, 2-5) ein räumliches Körpermuster der Person (1) umfasst, das im Wesentlichen von demselben spezifischen räumlichen Körperort aufgenommen wurde, wobei zu irgendeinem Zeitpunkt während oder anderweitig über eine gleichzeitige Dauer der (Funktion a) und der (Funktion b1) die Rechenvorrichtung und App (2a, 2-3, 2-4, 2-5) (i) von der Begleitvorrichtung (3-1-d9) das räumliche Körpermuster (3-1-d9-d1) der Begleitperson empfängt, (ii) empfängt von der Begleitvorrichtung (3-1-d9) das zeitliche Körpermuster (3-1-d9-d2) des Begleiters, (iii) empfängt von der Vorrichtung (4-4) zur temporären Einbindung des zeitlichen Körpermusters das zeitliche Musterkörpermuster (4-4-d2), (iv) vergleicht das räumliche Körpermuster (3-1-d9-d1) des Begleiters mit dem vorregistrierten räumlichen Körpermuster und (v) das zeitliche Begleitkörpermuster (3-1-d9-d2) mit dem zeitlichen Musterkörpermuster (4-4-d2) vergleicht, und woraufhin eine ausreichende Übereinstimmung zwischen der Rechenvorrichtung und der App (2a, 2-3, 2-4, 2-5) besteht, die (Funktion c1) ausführt und die eine oder mehrere Gesundheitsmessungen (2d) von der Vorrichtung (4-4) zur vorübergehenden Erfassung des zeitlichen Körpermusters als von der Person (1) authentifiziert empfängt oder anderweitig akzeptiert;
Erkennung des Gesichts der Person (1) gleichzeitig mit dem zeitlichen Körpermusterabgleich , wobei die temporäre Eingriffsvorrichtung eine temporäre Körpermuster-Eingriffsvorrichtung (4-4) ist, die Mittel zur Bestimmung eines zeitlichen Körpermusters (4-4-d2) aus einer bestimmten räumlichen Körperposition der Person umfasst, das eine Messsequenz der Herzschläge der Person (1) umfasst, und die einen oder mehrere Sensoren umfasst, die in wesentlichen Kontakt, ausreichende Nähe, oder anderweitig mechanisch mit dem spezifische räumliche Körperposition der Person (1) in Kontakt kommen, um eine oder mehrere der Gesundheitsmessungen (2d) der Person (1) zu bestimmen, wobei das Computergerät und die App (2a, 2-3, 2-4, 2-5) eine Kamera zur Aufnahme von Bildern umfasst oder anderweitig damit kommuniziert, wobei mindestens ein biometrisches Merkmal der Person (1), das bei der Rechenvorrichtung und der App (2a, 2-3, 2-4, 2-5) vorregistriert ist, ein Gesichtsbild umfasst, wobei die Rechenvorrichtung und die App (2a, 2-3, 2-4, 2-5) zu irgendeinem Zeitpunkt während oder anderweitig über eine gleichzeitige Dauer der (Funktion a) und der (Funktion b1) (2a, 2-3, 2-4, 2-5) (i) von der Vorrichtung (4-4) zur temporären Einbindung des zeitlichen Körpermusters das Muster des zeitlichen Körpermusters (4-4-d2) empfängt, (ii) zwei oder mehr Bilder aufnimmt, die im Wesentlichen das Gesicht der Person (1) umfassen, (iii) die zwei oder mehr Bilder verarbeitet, um das aufgenommene Gesicht der Person (1) mit dem vorregistrierten Gesichtsbild zu vergleichen und ein zeitliches Körpermuster (2a-d2) der Rechenvorrichtung zu bestimmen, das eine Messfolge von Herzschlägen der Person (1) umfasst, und vergleicht des zeitlichen Körpermusters der Rechenvorrichtung (2a-d2) mit dem zeitlichen Musterkörpermuster (4-4-d2), und woraufhin bei ausreichender Übereinstimmung die Rechenvorrichtung und die App (2a, 2-3, 2-4, 2-5) die eine oder mehrere Gesundheitsmessungen (2d) von der zeitlichen Körpermuster-vorübergehenden Eingriffsvorrichtung (4-4) als von der Person (1) authentifiziert empfängt oder anderweitig akzeptiert, und
eine begehbare Biomessvorrichtung (3-2) unter der Kontrolle des Zugangskontrollsystems (74), um Gesundheitsmessungen bereitzustellen, wobei die temporäre Eingriffsvorrichtung eine begehbare Biomessvorrichtung (3-2) ist, die von dem Zugangskontrollsystem (74) betrieben wird oder anderweitig mit diesem kommuniziert, um die Computervorrichtung und die App (2a, 2-3, 2-4, 2-5), die von der Person (1) betätigt wird, während die Person (1) im Wesentlichen isoliert und auf die vorübergehend eingreifende, begehbare Biomessvorrichtung (3-2) ausgerichtet ist, um hinreichend sicherzustellen, dass alle Gesundheitsmessungen (2d), die anschließend von der vorübergehend eingreifenden, begehbaren Biomessvorrichtung (3-2) bestimmt und an die Computervorrichtung und App (2a, 2-3, 2-4, 2-5) bestimmt und an das Computergerät und die App (2a, 2-3, 2-4, 2-5) übermittelt werden, von der Person (1) stammen, wobei die Person (1), die das Computergerät und die App (2a, 2-3, 2-4, 2-5) trägt, trägt oder anderweitig besitzt, zu irgendeinem Zeitpunkt während oder anderweitig während einer gleichzeitigen Dauer der (Funktion a) und der (Funktion b1) in die isolierte physische Ausrichtung mit dem vorübergehend eingreifenden begehbaren Biomessgerät (3-2) eintritt und eine Fernkommunikation zwischen dem vorübergehend eingreifenden begehbaren Biomessgerät (3-2) und dem Computergerät und der App (2a, 3-2) und dem Computergerät und der App (2a, 2-3, 2-4, 2-5) hergestellt werden, woraufhin die isolierte und ausgerichtete Person (1) das Computergerät und die App (2a, 2-3, 2-4, 2-5) verwendet, um dem Computergerät und der App (2a, 2-3, 2-4, 2-5) privat mindestens ein biometrisches Bestätigungsmerkmal zum Vergleich mit dem einen oder den mehreren vorregistrierten biometrischen Merkmalen zur Verfügung zu stellen, oder (ii) von dem Walk-up-Biometriegerät die mindestens eine Bestätigungsbiometrie zum Vergleich mit der einen oder den mehreren vorab registrierten Biometrien empfängt, woraufhin das Computergerät und die App (2a, 2-3, 2-4, 2-5) bei ausreichender Übereinstimmung eine anonyme Angabe über die biometrischen Merkmale liefert, 2-5) eine anonyme Anzeige der Authentizität der Person (1) an die temporäre Begehungs-Biomessvorrichtung (3-2) liefert, und bei Erhalt der anonymen Anzeige die temporäre Begehungs-Biomessvorrichtung (3-2) eine oder mehrere der Gesundheitsmessungen (2d) der ausreichend isolierten und positionierten Person (1) zur Bereitstellung an die Rechenvorrichtung und App (2a, 2-3, 2-4, 2-5) zu übermitteln, und wobei das Computergerät und die App (2a, 2-3, 2-4, 2-5) die eine oder die mehreren Gesundheitsmessungen (2d) von der temporären begehbaren Biomessvorrichtung (3-2) als von der Person (1) authentifiziert durchführt (Funktion c1) und empfängt oder anderweitig akzeptiert.

4. System nach Anspruch 1, wobei die mindestens eine Gesundheitsmessvorrichtung (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) eine tragbare Vorrichtung mit anhaltendem Eingriff (3-1-d4, 3-1-d5, 4-1a, 4-1b, 4-3, 131) ist, und wobei bei Verwendung der tragbaren Vorrichtung (3-1-d4, 3-1-d5, 4-1a, 4-1b, 4-3, 131) mit anhaltendem Eingriff die Durchführung der (Funktion 1), die die Kombination der (Funktion a), der (Funktion b1) und der (Funktion c1) umfasst, eine der folgenden ist:
Erkennung des Gesichts der Person (1) gleichzeitig mit einer Lichtsignalbestätigung und einer Bestätigung des gesicherten Zustands des Wearables, wobei das Wearable mit anhaltendem Eingriff ein Wearable mit anhaltendem Eingriff mit Lichtsignal (3-1-d4, 3-1-d5) ist, das einen Lichtsender, einen oder mehrere Sensoren zum Bestimmen einer oder mehrerer der Gesundheitsmessungen (2d) der Person (1) und Mittel zum Bestimmen einer beliebigen Information über den gesicherten Zustand zur Verwendung zumindest teilweise zum Bestimmen, dass das Wearable mit anhaltendem Eingriff mit Lichtsignal (3-1-d4, 3-1-d4, 3-1-d5) am Körper der Person (1) befestigt, verriegelt, eingerastet oder anderweitig gesichert ist, wobei die Rechenvorrichtung und die App (2a, 2-3, 2-4, 2-5) eine Kamera zum Aufnehmen von Bildern umfasst oder anderweitig damit kommuniziert, wobei mindestens ein biometrisches Merkmal der Person (1), das bei der Rechenvorrichtung und der App (2a, 2-3, 2-4, 2-5) vorregistriert ist, ein Gesichtsbild umfasst, wobei die Rechenvorrichtung und die App (2a, 2-3, 2-4, 2-5) bei einer gewissen gleichzeitigen Dauer der (Funktion a) und der (Funktion b1) (2a, (Funktion a) und der (Funktion b1) (i) ein Steuersignal an das lichtsignalgebende, dauerhafte Eingriffs-Wearable (3-1-d4, 3-1-d5) liefert, (ii) zwei oder mehr Bilder aufnimmt, die im Wesentlichen eine gleichzeitige Ansicht des Gesichts der Person (1) und des lichtsignalgebenden, dauerhaften Eingriffs-Wearables (3-1-d4, 3-1-d5) umfassen, das sich im Wesentlichen in Kontakt mit dem Körper der Person (1) oder in ausreichender Nähe zu diesem befindet, um sicherzustellen, dass das lichtsignalgebende, dauerhafte Eingriffs-Wearable (3-1-d4, 3-1-d5) befestigt ist, (3-1-d4, 3-1-d5) am Körper der Person (1) befestigt, gesichert ist oder anderweitig am Körper der Person (1) getragen wird, und (iii) von dem Lichtsignal-aufrechterhaltenden Eingriffs-Wearable (3-1-d4, 3-1-d5) die Informationen über den gesicherten Zustand empfängt, wobei das Lichtsignal-aufrechterhaltende Eingriffs-Wearable (3-1-d4, 3-1-d5), während es sich im gleichzeitigen Blickfeld der Kamera befindet und zumindest teilweise das Steuersignal verwendet, den Lichtemitter veranlasst, ein Antwortsignal (3-sig) zu emittieren, das von der Kamera detektierbares Licht umfasst, wobei die Rechenvorrichtung und die App (2a, 2-3, 2-4, 2-5) (iv) die zwei oder mehr Bilder verarbeitet, um (iv-a) das erfasste Gesicht der Person (1) mit dem vorregistrierten Gesichtsbild zu vergleichen, (iv-b) das ausgesendete Antwortsignal (3-sig) mit dem bereitgestellten Steuersignal zu vergleichen, und (iv-c) den wesentlichen Kontakt oder eine anderweitig ausreichende Nähe zwischen dem lichtsignalgebenden dauerhaften Eingriffs-Wearable (3-1-d4, 3-1-d5) und dem Körper der Person (1) zu bestätigen, um sicherzustellen, dass das lichtsignalgebende dauerhafte Eingriffs-Wearable (3-1-d4, 3-1-d4, 3-1-d5) am Körper der Person (1) befestigt, gesichert oder anderweitig getragen wird, woraufhin bei ausreichenden Übereinstimmungen und Bestätigungen und in Kombination mit der Feststellung durch die Rechenvorrichtung und die App (2a, 2-3, 2-4, 2-5), dass das Lichtsignalunterstützende Eingriffs-Wearable (3-1-d4, 3-1-d5) am Körper der Person (1) befestigt, verriegelt, eingerastet oder anderweitig gesichert ist, die Rechenvorrichtung und die App (2a, 2-3, 2-4, 2-5) feststellt, dass das lichtsignalgebende Wearable (3-1-d4, 3-1-d5) mit dauerhaftem Eingriff in eine Dauer der gesicherten Zeit eintritt, wobei die Dauer der gesicherten Zeit bis zu einem solchen späteren Zeitpunkt andauert, zu dem nachfolgende Informationen über den gesicherten Zustand von dem lichtsignalgebenden Wearable (3-1-d4, 3-1-d5) mit dauerhaftem Eingriff von der Rechenvorrichtung und der App (2a, 2-3, 2-4, 2-5) empfangen und zumindest teilweise von der Rechenvorrichtung und der App (2a, 2-3, 2-4, 2-5) verwendet werden, um festzustellen, dass das lichtsignalgebende Wearable (3-1-d4, 3-1-d5) mit dauerhaftem Eingriff in den Körper der Person (1) gesichert ist, (2a, 2-3, 2-4, 2-5) verwendet wird, um festzustellen, dass das lichtsignalgebende Wearable (3-1-d4, 3-1-d5) mit anhaltendem Eingriff nicht mehr ausreichend am Körper der Person (1) befestigt ist, was die Dauer der gesicherten Zeit beendet, und wobei das Computergerät und die App (2a, 2-3, 2-4, 2-5) die eine oder mehrere der Gesundheitsmessungen (2d), die während der Dauer der gesicherten Zeit von dem lichtsignalgebenden Wearable (3-1-d4, 3-1-d5) mit anhaltendem Eingriff ermittelt wurden, durchführt (Funktion c1) und als von der Person (1) authentifiziert empfängt und akzeptiert;
Verwendung einer Begleitvorrichtung (3-1-d9), um ein räumliches Begleit-Körpermuster zusammen mit einem gleichzeitigen und kollokierten zeitlichen Begleit-Körpermuster bereitzustellen, wobei die Begleitvorrichtung (3-1-d9) eine Einrichtung zum gleichzeitigen Bestimmen sowohl des räumlichen Begleit-Körpermusters (3-1-d9-d1) als auch des zeitlichen Begleit-Körpermusters (3-1-d9-d2) von im Wesentlichen demselben spezifischen räumlichen Körperort der Person (1) umfasst, wobei derselbe spezifische räumliche Körperort ein Gesicht, ein Auge, einen Finger oder eine andere eindeutig identifizierbare Stelle am Körper der Person (1) umfasst, wobei ein räumliches Körpermuster (3-1-d9-d1) ein eindeutig identifizierendes Muster des Körpers der Person (1) umfasst, das an derselben spezifischen räumlichen Körperstelle bestimmt wird, wobei das eindeutig identifizierende Muster ein Gesichtsbild, einen Netzhautscan, einen Fingerabdruck oder ein anderes eindeutig identifizierbares räumliches Körpermuster umfasst, wobei das begleitende zeitliche Körpermuster eine Messsequenz von Herzschlägen der Person (1) umfasst, wobei das tragbare Gerät mit anhaltendem Eingriff ein tragbares Gerät mit zeitlichem Körpermuster und anhaltendem Eingriff (3-1-d4, 3-1-d5, 4-1a, 4-1b, 4-3) ist, das Mittel zum Bestimmen eines tragbaren zeitlichen Körpermusters umfasst, das eine Messsequenz der Herzschläge der Person (1) umfasst, die von einem anderen Körperort als demselben spezifischen räumlichen Körperort der Person (1) genommen wurde, einen oder mehrere Sensoren zum Bestimmen einer oder mehrerer der Gesundheitsmessungen (2d) der Person (1), und Mittel zum Bestimmen einer beliebigen gesicherten Zustandsinformation zur Verwendung zumindest teilweise, um zu bestimmen, dass das zeitlich-körperliche Muster des anhaltenden Eingriffs-Wearables (3-1-d4, 3-1-d5, 4-1a, 4-1b, 4-3) am Körper der Person (1) befestigt, verriegelt, eingerastet oder anderweitig gesichert ist, wobei mindestens ein biometrisches Merkmal der Person (1), das bei der Rechenvorrichtung und der App (2a, 2-3, 2-4, 2-5) vorregistriert ist, ein räumliches Körpermuster der Person (1) umfasst, das im Wesentlichen an demselben spezifischen räumlichen Körperort aufgenommen wurde, wobei die Rechenvorrichtung und die App (2a, 2-3, 2-4, 2-5) (i) von der Rechenvorrichtung und der App (2a, 2-3, 2-4, 2-5) (i) während einer gewissen gleichzeitigen Dauer der (Funktion a) und der (Funktion b1) ein räumliches Körpermuster der Person (1) empfängt, 2-5) (i) von der Begleitvorrichtung (3-1-d9) das räumliche Begleit-Körpermuster (3-1-d9-d1) empfängt, (ii) von der Begleitvorrichtung (3-1-d9) das zeitliche Begleit-Körpermuster (3-1-d9-d2) empfängt, (iii) von dem tragbaren Gerät (3-1-d4, 3-1-d5, 4-1a, 4-1b, 4-3) das tragbare zeitliche Körpermuster, (iv) empfängt von dem tragbaren zeitlichen Körpermuster mit anhaltendem Eingriff (3-1-d4, 3-1-d5, 4-1a, 4-1b, 4-3) die gesicherte Zustandsinformation, (v) vergleicht das räumliche Begleitkörpermuster (3-1-d9-d1) mit dem vorregistrierten räumlichen Körpermuster, und (vi) vergleicht das zeitliche Körpermuster des Begleiters (3-1-d9-d2) mit dem zeitlichen Körpermuster des Trägers, woraufhin ausreichende Übereinstimmungen und in Kombination mit der Feststellung durch die Rechenvorrichtung und die App (2a, 2-3, 2-4, 2-5), dass das tragbare Zeit-Körper-Muster (3-1-d4, 3-1-d5, 4-1a, 4-1b, 4-3) befestigt, verriegelt, eingeklinkt oder anderweitig am Körper der Person (1) gesichert ist, wobei zumindest teilweise die Informationen über den gesicherten Zustand verwendet werden, das Computergerät und die App (2a, 2-3, 2-4, 2-5) feststellt, dass das Zeit-Körper-Muster des Wearables (3-1-d4, 3-1-d5, 4-1a, 4-1b, 4-3) in eine Dauer der gesicherten Zeit eintritt, wobei die Dauer der gesicherten Zeit bis zu einem solchen späteren Zeitpunkt andauert, zu dem eine nachfolgende gesicherte Zustandsinformation von der Rechenvorrichtung und App (2a, 2-3, 2-4, 2-5) von dem Zeit-Körper-Muster-Wearable (3-1-d4, 3-1-d5, 4-1a, 4-1b, 4-3) empfangen und zumindest teilweise von dem Computergerät und der App (2a, 2-3, 2-4, 2-5) verwendet wird, um festzustellen, dass das zeitlich-körperliche Wearable (3-1-d4, 3-1-d5, 4-1a, 4-1b, 4-3) nicht mehr ausreichend am Körper der Person (1) befestigt ist, um die Dauer der befestigten Zeit zu beenden, und wobei die Rechenvorrichtung und App (2a, 2-3, 2-4, 2-5) ausführt (Funktion c1) und die eine oder mehrere der während der Dauer der gesicherten Zeit ermittelten Gesundheitsmessungen (2d) von dem zeitlich-körperlichen Muster des Wearables (3-1-d4, 3-1-d5, 4-1a, 4-1b, 4-3) als authentifiziert der Person (1) empfängt und akzeptiert, und
Erkennung des Gesichts der Person (1) gleichzeitig mit dem zeitlichen Musterabgleich und der Bestätigung des gesicherten Zustands des Wearables, wobei das Wearable mit anhaltendem Eingriff ein Wearable mit zeitlichem Körpermuster und anhaltendem Eingriff (3-1-d4, 3-1-d5, 4-1a, 4-1b, 4-3) ist, das Mittel zum Bestimmen eines zeitlichen Körpermusters des Wearables, das eine Messsequenz der Herzschläge der Person (1) umfasst, einen oder mehrere Sensoren zum Bestimmen einer oder mehrerer der Gesundheitsmessungen (2d) der Person (1) umfasst, und Mittel zum Bestimmen einer beliebigen gesicherten Zustandsinformation zur Verwendung zumindest teilweise, um zu bestimmen, dass das Zeit-Körpermuster-aufrechterhaltende Eingriffs-Wearable (3-1-d4, 3-1-d5, 4-1a, 4-1b, 4-3) am Körper der Person (1) befestigt, verriegelt, eingeklinkt oder anderweitig gesichert ist, wobei das Computergerät und die App (2a, 2-3, 2-4, 2-5) eine Kamera zum Erfassen von Bildern umfasst oder anderweitig damit kommuniziert, wobei mindestens ein biometrisches Merkmal der Person (1), das bei der Rechenvorrichtung und der App (2a, 2-3, 2-4, 2-5) vorregistriert ist, ein Gesichtsbild umfasst, wobei die Rechenvorrichtung und die App (2a, 2-3, 2-4, 2-5) zu irgendeinem Zeitpunkt während oder anderweitig über eine gleichzeitige Dauer der (Funktion a) und der (Funktion b1) (i) von dem zeitlich-körperlichen Muster des Wearables (3-1-d4, 3-1-d5, 4-1a, 4-1b, 4-3) die Informationen über den gesicherten Zustand, (ii) empfängt von der tragbaren Person (3-1-d4, 3-1-d5, 4-1a, 4-1b, 4-3) mit dem zeitlichen Körpermuster mit anhaltendem Eingriff das tragbare zeitliche Körpermuster, (iii) nimmt zwei oder mehr Bilder auf, die im Wesentlichen das Gesicht der Person (1) umfassen, (iv) verarbeitet die zwei oder mehr Bilder, um (iv-a) das aufgenommene Gesicht der Person (1) mit dem vorher registrierten Gesichtsbild zu vergleichen, (iv-b) Bestimmen eines zeitlichen Körpermusters (2a-d2) der Rechenvorrichtung, das eine Messsequenz von Herzschlägen der Person (1) umfasst, und (iv-c) Vergleichen des zeitlichen Körpermusters (2a-d2) der Rechenvorrichtung mit dem tragbaren zeitlichen Körpermuster, woraufhin ausreichende Übereinstimmungen und in Kombination mit der Bestimmung durch die Rechenvorrichtung und die App (2a, 2-3, 2-4, 2-5), dass das tragbare Zeit-Körper-Muster (3-1-d4, 3-1-d5, 4-1a, 4-1b, 4-3) befestigt, verriegelt, eingeklinkt oder anderweitig am Körper der Person (1) gesichert ist, wobei zumindest teilweise die Informationen über den gesicherten Zustand verwendet werden, die Rechenvorrichtung und die App (2a, 2-3, 2-4, 2-5) feststellt, dass das Wearable (3-1-d4, 3-1-d5, 4-1a, 4-1b, 4-3) mit dem zeitlich-körperlichen Muster des anhaltenden Eingriffs in eine Dauer der gesicherten Zeit eintritt, wobei die Dauer der gesicherten Zeit bis zu einem solchen späteren Zeitpunkt andauert, zu dem nachfolgende gesicherte Zustandsinformationen von der Rechenvorrichtung und der App (2a, 2-3, 2-4, 2-5) von dem zeitlich-körperlichen Wearable mit anhaltendem Eingriff (3-1-d4, 3-1-d5, 4-1a, 4-1b, 4-3) empfangen und zumindest teilweise von der Rechenvorrichtung und der App (2a, 2-3, 2-4, 2-5) verwendet werden, um zu bestimmen, dass das zeitlich-körperliche Wearable mit anhaltendem Eingriff (3-1-d4, 3-1-d5, 4-1a, 4-1b, 4-3) nicht mehr ausreichend am Körper der Person (1) befestigt ist, um die Dauer der gesicherten Zeit zu beenden, und wobei das Computergerät und die App (2a, 2-3, 2-4, 2-5) die eine oder mehrere der Gesundheitsmessungen (2d), die während der Dauer der gesicherten Zeit von dem zeitlich-körperlichen Muster des Wearables (3-1-d4, 3-1-d5, 4-1a, 4-1b, 4-3) mit anhaltendem Eingriff bestimmt wurden, als von der Person (1) authentifiziert durchführt (Funktion c1) und empfängt und akzeptiert.

5. System nach Anspruch 1, wobei die mindestens eine Gesundheitsmessvorrichtung (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) eine biometrische Probensammelvorrichtung (4-2, 80, 84) ist, und wobei bei Verwendung einer biometrischen Probensammelvorrichtung (4-2, 80, 84) die Durchführung der (Funktion 1), die die Kombination der (Funktion a), der (Funktion b1) und der (Funktion c1) umfasst, eine der folgenden ist:
Erkennung des Gesichts der Person (1) gleichzeitig mit dem Scannen von visuellen Markierungen (80-bcd, 84-bcd), um eine ID der Probensammelvorrichtung zu bestimmen, wobei die biometrische Probensammelvorrichtung (80, 84) eine Körperflüssigkeit oder eine andere Probe der Person (1) unter Verwendung eines Probenbehälters (80-tip, 84-smp) sammelt, empfängt, nimmt oder anderweitig erhält, 84-smp), wobei eine Körperflüssigkeit oder eine andere Probe Schleim, Speichel oder Blut umfasst, wobei die biometrische Probensammelvorrichtung (80, 84) visuelle Markierungen (80-bcd, 84-bcd) umfasst, die zumindest teilweise zur Bestimmung der ID der Probensammelvorrichtung verwendet werden, wobei die Rechenvorrichtung und die App (2a, 2-3, 2-4, (2a, 2-3, 2-4, 2-5) eine Kamera zur Aufnahme von Bildern umfasst oder anderweitig mit dieser kommuniziert, wobei mindestens ein biometrisches Merkmal der bei der Rechenvorrichtung und der App (2a, 2-3, 2-4, 2-5) vorregistrierten Person (1) ein Gesichtsbild umfasst, wobei die Rechenvorrichtung und die App (2a, 2-3, 2-4, 2-5) zu einem bestimmten Zeitpunkt während oder anderweitig über eine gewisse gleichzeitige Dauer der (Funktion a) und der (Funktion b1) ein Gesichtsbild erfasst, 2-3, 2-4, 2-5) zwei oder mehr Bilder aufnimmt, die im Wesentlichen eine gleichzeitige Ansicht des Gesichts der Person (1), der biometrischen Probensammelvorrichtung (80, 84) und der visuellen Markierungen (80-bcd, 84-bcd) umfassen, und die Person (1) die biometrische Probensammelvorrichtung (80, 84) verwendet, um eine Körperflüssigkeit oder eine andere Probe der Person (1) zu sammeln, zu empfangen, zu nehmen oder anderweitig zu erhalten, wobei die Computervorrichtung und die App (2a, 2-3, 2-4, 2-5) (i) die zwei oder mehr Bilder verarbeitet, um (i-a) die ID der Probensammelvorrichtung zumindest teilweise auf der Grundlage der visuellen Markierungen (80-bcd, 84-bcd), (i-b) Vergleichen des erfassten Gesichts der Person (1) mit dem vorregistrierten Gesichtsbild, und (i-c) Bestätigen des wesentlichen Kontakts oder der anderweitig ausreichenden Nähe zwischen dem Probenbehälter (80-tip, 84-smp) und dem Körper der Person (1), um sicherzustellen, dass jedes der gesammelten, entnommenen oder anderweitig erhaltenen biometrischen Daten mit dem Körper der Person (1) übereinstimmt, der gesammelten, entnommenen oder anderweitig gewonnenen biometrischen Proben von der Person (1) stammt, und woraufhin die Rechenvorrichtung und die App (2a, 2-3, 2-4, 2-5) bei ausreichender Übereinstimmung und Bestätigung eine Zuordnung zwischen der ID der Probensammelvorrichtung und der Person (1) vornimmt (Funktion c1) und bestimmt, die zumindest teilweise für die Zuordnung zu einer künftigen Bewertung der gesammelten, entnommenen oder anderweitig gewonnenen der gesammelten, entnommenen oder anderweitig erhaltenen biometrischen Proben zu assoziieren, so dass die zukünftige Bewertung als von der Person (1) stammend authentifiziert wird, wobei die zukünftige Bewertung eine oder mehrere der authentifizierten Gesundheitsmessungen (2d) umfasst;
Erkennung des Gesichts der Person (1) gleichzeitig mit dem Scannen eines elektronischen Etiketts (80-nfc, 84-nfc), um eine Probensammelvorrichtungs-ID zu bestimmen, wobei die biometrische Probensammelvorrichtung (80, 84) eine Körperflüssigkeit oder eine andere Probe der Person (1) unter Verwendung eines Probenbehälters (80-tip, 84-smp) sammelt, empfängt, nimmt oder anderweitig erhält, 84-smp), wobei eine Körperflüssigkeit oder eine andere Probe Schleim, Speichel oder Blut umfasst, wobei die biometrische Probensammelvorrichtung (80, 84) ein elektronisches Etikett (80-nfc, 84-nfc) zur Bereitstellung der ID der Probensammelvorrichtung umfasst, wobei die Rechenvorrichtung und die App (2a, 2-3, 2-4, 2-5) eine Kamera zum Aufnehmen von Bildern umfasst oder anderweitig mit dieser kommuniziert und ein elektronisches Etikettlesemittel zum elektronischen Scannen des elektronischen Etiketts (80-nfc, 84-nfc) umfasst, um die Probenentnahmevorrichtungs-ID zu bestimmen, wobei mindestens ein biometrisches Merkmal der Person (1), die bei der Computervorrichtung und App (2a, 2-3, 2-4, 2-5) vorregistrierte biometrische Merkmal der Person (1) ein Gesichtsbild umfasst, wobei die Computervorrichtung und App (2a, 2-3, 2-4, 2-5) zu irgendeinem Zeitpunkt während oder anderweitig über eine gleichzeitige Dauer der (Funktion a) und der (Funktion b1) zwei oder mehr Bilder aufnimmt, die im Wesentlichen eine gleichzeitige Ansicht des Gesichts der Person (1), der biometrischen Probensammelvorrichtung (80, 84) und der Person (1) unter Verwendung der biometrischen Probensammelvorrichtung (80, 84), um eine Körperflüssigkeit oder eine andere Probe der Person (1) zu sammeln, zu empfangen, zu nehmen oder anderweitig zu erhalten, wobei die Rechenvorrichtung und die App (2a, 2-3, 2-4, 2-5) (i) die Leseeinrichtung für das elektronische Etikett der Rechenvorrichtung verwendet, um die ID der Probensammelvorrichtung durch elektronisches Scannen des elektronischen Etiketts (80-nfc, 84-nfc), (ii) verarbeitet die zwei oder mehr Bilder, um (ii-a) das erfasste Gesicht der Person (1) mit dem vorregistrierten Gesichtsbild zu vergleichen, und (ii-b) den wesentlichen Kontakt oder eine anderweitig ausreichende Nähe zwischen dem Probenbehälter (80-tip, 84-smp) und dem Körper der Person (1), um sicherzustellen, dass jede der gesammelten, entnommenen oder anderweitig erhaltenen biometrischen Proben von der Person (1) stammt, und woraufhin bei ausreichender Übereinstimmung und Bestätigung die Rechenvorrichtung und die App (2a, 2-3, 2-4, 2-5) bei ausreichender Übereinstimmung und Bestätigung eine Assoziation zwischen der ID der Probensammelvorrichtung und der Person (1) durchführt (Funktion c1) und bestimmt, die zumindest teilweise für die Assoziation mit einer zukünftigen Bewertung der gesammelten, entnommenen oder anderweitig erhaltenen einen oder mehreren biometrischen Proben verwendet wird, so dass die zukünftige Bewertung als von der Person (1) stammend authentifiziert wird, wobei die zukünftige Bewertung eine oder mehrere der authentifizierten Gesundheitsmessungen (2d) umfasst;
Erkennung des Gesichts der Person (1) gleichzeitig mit der Verwendung eines Begleitgeräts (3-1-d9, 3-1-d10), um ein Begleitlichtsignal mit dem Scannen von entweder visuellen Markierungen (80-bcd, 84-bcd) oder einem elektronischen Etikett (80-nfc, 84-nfc) zu erzeugen, um eine Probenentnahmegeräte-ID zu bestimmen, wobei die Begleitvorrichtung (3-1-d9, 3-1-d10) einen Lichtemitter (3-1-d9-led, 3-1-d10-led) zum Emittieren des Begleitlichtsignals und optional eine elektronische Markierungsleseeinrichtung (3-1-d10-nfc) zum elektronischen Abtasten der elektronischen Markierung (80-nfc, 84-nfc) zum elektronischen Scannen des elektronischen Etiketts (80-nfc, 84-nfc), um die ID der Probensammelvorrichtung zu bestimmen, wobei die biometrische Probensammelvorrichtung (80, 84) eine Körperflüssigkeit oder eine andere Probe der Person (1) unter Verwendung eines Probenbehälters (80-tip, 84-smp) sammelt, empfängt, nimmt oder anderweitig erhält, wobei eine Körperflüssigkeit oder eine andere Probe Schleim, Speichel oder Blut umfasst, wobei die biometrische Probensammelvorrichtung (80, 84) entweder visuelle Markierungen (80-bcd, 84-bcd) zur Verwendung zumindest teilweise zur Bestimmung der Probenentnahmevorrichtungs-ID und ein elektronisches Etikett (80-nfc, 84-nfc) zur Bereitstellung der Probenentnahmevorrichtungs-ID umfasst, wobei die Rechenvorrichtung und die App (2a, 2-3, 2-4, 2-5) eine Kamera zur Aufnahme von Bildern umfasst oder anderweitig mit dieser kommuniziert und ein elektronisches EtikettLesemittel zum elektronischen Scannen des elektronischen Etiketts (80-nfc, 84-nfc) umfasst, um die Probenentnahmevorrichtungs-ID zu bestimmen, wobei mindestens ein biometrisches Merkmal der Person (1), das bei der Rechenvorrichtung und App (2a, 2-3, 2-4, 2-5) vorregistrierte Person (1) ein Gesichtsbild umfasst, wobei die Rechenvorrichtung und App (2a, 2-3, 2-4, 2-5) zu irgendeinem Zeitpunkt während oder anderweitig über eine gleichzeitige Dauer der (Funktion a) und der (Funktion b1) ein Steuersignal an die Rechenvorrichtung und App (2a, 2-3, 2-4, 2-5) ein Steuersignal an die Begleitvorrichtung (3-1-d9) liefert, während sie gleichzeitig zwei oder mehr Bilder aufnimmt, die im Wesentlichen eine gleichzeitige Ansicht des Gesichts der Person (1), der Begleitvorrichtung (3-1-d9, 3-1-d10), der biometrischen Probensammelvorrichtung (80, 84), einer der visuellen Markierungen (80-bcd, 84-bcd), die auf der biometrischen Probenentnahmevorrichtung (80, 84) vorhanden sind, und die Person (1), die die biometrische Probenentnahmevorrichtung (80, 84) verwendet, um eine Körperflüssigkeit oder eine andere Probe der Person (1) zu sammeln, zu empfangen, zu entnehmen oder anderweitig zu erhalten, wobei die Begleitvorrichtung (3-1-d9, 3-1-d10), während sie sich im gleichzeitigen Blickfeld der Kamera befindet und zumindest teilweise das Steuersignal verwendet, den Lichtemitter (3-1-d9-led, 3-1-d10-led) veranlasst, ein Antwortsignal auszusenden, das von der Kamera erfassbares Licht umfasst, wobei die Rechenvorrichtung und die App (2a, 2-3, 2-4, 2-5) die ID der Probensammelvorrichtung durch eine oder eine Kombination aus der Verwendung der Leseeinrichtung für das elektronische Etikett der Rechenvorrichtung, um das elektronische Etikett (80-nfc, 84-nfc) elektronisch zu scannen, und der Verarbeitung der zwei oder mehr Bilder zur Bestimmung der ID der Probensammelvorrichtung zumindest teilweise auf der Grundlage der visuellen Markierungen (80-bcd, 84-bcd) bestimmt, und wobei die Rechenvorrichtung und die App (2a, 2-3, 2-4, 2-5) (i) die zwei oder mehr Bilder verarbeitet, um (i-a) das erfasste Gesicht der Person (1) mit dem vorregistrierten Gesichtsbild zu vergleichen, (i-b) das ausgesendete Antwortsignal mit dem bereitgestellten Steuersignal zu vergleichen und (i-c) den wesentlichen Kontakt oder die anderweitig ausreichende Nähe zwischen dem Probenbehälter (80-tip, 84-smp) und dem Körper der Person (1) zu bestätigen, um sicherzustellen, dass jede der einen oder mehreren gesammelten, genommenen oder anderweitig erhaltenen biometrischen Proben von der Person (1) stammt, und woraufhin bei ausreichenden Übereinstimmungen und Bestätigungen die Computervorrichtung und die App (2a, 2-3, 2-4, 2-5) eine Assoziation zwischen der ID der Probensammelvorrichtung und der Person (1) zur Verwendung zumindest teilweise für die Assoziation mit einer zukünftigen Bewertung der gesammelten, entnommenen oder anderweitig erhaltenen einen oder mehreren biometrischen Proben durchführt (Funktion c1) und bestimmt, so dass die zukünftige Bewertung als von der Person (1) stammend authentifiziert wird, wobei die zukünftige Bewertung eine oder mehrere der authentifizierten Gesundheitsmessungen (2d) umfasst, und
Erkennung des Gesichts der Person (1) zusammen mit der Verwendung einer Begleitvorrichtung (3-1-d10), um ein Begleitlichtsignal bereitzustellen und ein elektronisches Etikett zu scannen, um eine Probensammelvorrichtungs-ID zu bestimmen, wobei die Begleitvorrichtung (3-1-d10) einen Lichtemitter (3-1-d10-led) und einen elektronischen Etikettleser (3-1-d10-nfc) zum elektronischen Scannen entweder eines eingebetteten oder eines aufgebrachten elektronischen Etiketts (4-tag) umfasst, um die Probensammelvorrichtungs-ID zu bestimmen, wobei die biometrische Probensammelvorrichtung eine Atemanalyse-Probensammelvorrichtung (4-2) ist, die entweder ein eingebettetes oder ein angebrachtes elektronisches Etikett (4-tag) zum Bereitstellen der Probensammelvorrichtungs-ID umfasst und Mittel zum Empfangen und Verarbeiten des ausgestoßenen Atems der Person (1) zur Verwendung in einer Atemanalyse umfasst, die zu einer oder mehreren der Gesundheitsmessungen (2d) führt, wobei die Rechenvorrichtung und die App (2a, 2-3, 2-4, 2-5) eine Kamera zur Aufnahme von Bildern umfasst oder anderweitig mit ihr kommuniziert, wobei mindestens ein biometrisches Merkmal der Person (1), das bei der Rechenvorrichtung und der App (2a, 2-3, 2-4, 2-5) vorregistriert ist, ein Gesichtsbild umfasst, wobei die Person (1) zu irgendeinem Zeitpunkt während oder anderweitig über eine gleichzeitige Dauer der (Funktion a) und der (Funktion b1) gleichzeitig die Atemprobenentnahmevorrichtung (4-2) des Atemanalysators verwendet, um Atemluft von der Person (1) zu empfangen und die Atemanalyse der Person (1) durchzuführen, und den elektronischen Tag-Leser (3-1-d10-nfc) des Begleitgeräts verwendet, um die ID der Probenentnahmevorrichtung für die Bereitstellung an die Rechenvorrichtung und die App (2a, 2-3, 2-4, 2-5) zu bestimmen, während das Computergerät und die App (2a, 2-3, 2-4, 2-5) ein Steuersignal an das Begleitgerät (3-1-d10) liefert und gleichzeitig zwei oder mehr Bilder aufnimmt, die im Wesentlichen eine gleichzeitige Ansicht des Gesichts der Person (1), des Begleitgeräts (3-1-d10), des Atemprobenentnahmegeräts (4-2) des Atemanalysators und der Person (1), wobei die Person (1) die Atemanalyse-Probenentnahmevorrichtung (4-2) verwendet, um den Atem von der Person (1) zu empfangen, wobei die Begleitvorrichtung (3-1-d10), während sie sich in der gleichzeitigen Ansicht der Kamera befindet und zumindest teilweise das Steuersignal verwendet, den Lichtemitter (3-1-d10-geführt) veranlasst, ein Antwortsignal zu emittieren, das von der Kamera detektierbares Licht umfasst, wobei die Rechenvorrichtung und App (2a, 2-3, 2-4, 2-5) (i) die zwei oder mehr Bilder verarbeitet, um (i-a) das erfasste Gesicht der Person (1) mit dem vorregistrierten Gesichtsbild zu vergleichen, (i-b) das ausgesendete Antwortsignal mit dem bereitgestellten Steuersignal zu vergleichen, und (i-c) den wesentlichen Kontakt oder die anderweitig ausreichende Nähe zwischen der Atemanalysevorrichtung (4-2) und dem Mund der Person (1) zu bestätigen, um sicherzustellen, dass die Atemanalyse, die die eine oder mehrere der Gesundheitsmessungen (2d) umfasst, von der Person (1) stammt, und woraufhin die Computervorrichtung und die App (2a, 2-3, 2-4, 2-5) ausreichende Übereinstimmungen und Bestätigungen durchführen (Funktion c1) und die eine oder mehrere der Gesundheitsmessungen (2d) von der Atemalysegerät-Probensammelvorrichtung (4-2) als von der Person (1) authentifiziert empfangen oder anderweitig akzeptieren.

6. System nach Anspruch 5 zur Bestimmung des einen oder der mehreren authentifizierten Gesundheitsmesswerte (2d), die zumindest teilweise auf der Bewertung des einen oder der mehreren authentifizierten biometrischen Proben beruhen, ferner umfassend eines der:
einem Gesundheitsdienst-Testlabor, das der Person (1) ein GesundheitstestKit (90-1) zur Verfügung stellt, das die biometrische Probensammelvorrichtung (80, 84) und einen verschließbaren Behälter (82) für die biometrische Probensammelvorrichtung umfasst, wobei die zwei oder mehr Bilder, die von der Rechenvorrichtung und der App (2a, 2-3, 2-4, 2-5) zu irgendeinem Zeitpunkt während oder anderweitig über eine gleichzeitige Dauer der (Funktion a) und der (Funktion b1), die die Person (1) unter Verwendung der biometrischen Probensammelvorrichtung (80, 84) zum Sammeln, Empfangen, Entnehmen oder anderweitigen Erhalten einer authentifizierten Körperflüssigkeits- oder anderweitigen Probe der Person (1) umfassen, ferner oder anderweitig zusätzlich und anschließend zwei oder mehr Bilder der Person (1) umfassen, die zumindest den Probenbehälter (80-tip, 84-smp) oder anderweitig den Teil der biometrischen Probensammelvorrichtung (80, 84), der die biometrische Probe enthält, in den verschließbaren Behälter (82) der biometrischen Probensammelvorrichtung setzt, um sicherzustellen, dass der verschließbare Behälter (82) der biometrischen Probensammelvorrichtung als die authentifizierte biometrische Probe der Person (1) enthaltend authentifiziert wird, wobei die Rechenvorrichtung und die App (2a, 2-3, 2-4, 2-5) bei der Verarbeitung der zwei oder mehr Bilder zusätzlich bestätigt, dass zumindest der Teil der biometrischen Probensammelvorrichtung (80, 84), der das Probengefäß (80-tip, 84-smp) oder sonst die biometrische Probe umfasst, in den Behälter (82) der biometrischen Probensammelvorrichtung eingebracht wurde und dass der Behälter (82) anschließend versiegelt wurde, wobei die Person (1) nach dem Versiegeln des Behälters (82) einen der verfügbaren Versanddienste oder andere Liefermethoden nutzt, um den versiegelten Behälter (82), der zumindest das biometrische Probengefäß (80-tip, 84-smp) oder anderweitig die biometrische Probe enthält, an das Testlabor des Gesundheitsdienstes zu liefern, wobei die Bereitstellung des biometrischen Probengefäßes (80-tip, 84-smp) oder anderweitig der biometrischen Probe an das Testlabor des Gesundheitswesens anonym ist und keine persönlich identifizierenden Informationen enthält, wobei das Testlabor des Gesundheitswesens die Bewertung, die eine oder mehrere der Gesundheitsmessungen (2d) umfasst, zumindest teilweise auf der Grundlage des bereitgestellten authentifizierten Probengefäßes (80-tip, 84-smp) oder anderweitig der biometrischen Probe bestimmt und digitale Informationen, die auf die eine oder mehrere der Gesundheitsmessungen (2d) hinweisen, in Verbindung mit der ID des Probenentnahmegeräts in einer Bewertungsdatenbank speichert, auf die das Computergerät und die App (2a, 2-3, 2-4, 2-5) zugreifen können, und wobei die Person (1) zu einem bestimmten Zeitpunkt nach der Bereitstellung des versiegelten Behälters (82) an das Gesundheitsdienstleistungs-Testlabor das Computergerät und die App (2a, 2-3, 2-4, 2-5) verwendet, um (Funktion c1) auszuführen, indem sie auf die Bewertungsdatenbank zugreift, um den einen oder die mehreren Gesundheitsmesswerte (2d) abzurufen, indem sie zumindest teilweise die ID der Probenentnahmevorrichtung der Bewertungsdatenbank zur Verfügung stellt und danach den einen oder die mehreren Gesundheitsmesswerte (2d), die durch die Bewertung des Gesundheitstestlabors bestimmt wurden, als von der Person (1) authentifiziert empfängt und akzeptiert, und
ein Gesundheitstestlabor, das der Person (1) ein Gesundheitstestkit zur Verfügung stellt, das die biometrische Probensammelvorrichtung (80, 84) umfasst, die ferner ein reaktives Medium zur Bereitstellung und unmittelbaren Bewertung umfasst, wobei das Probengefäß (80-tip, 84-smp) ferner das in dem Gesundheitstestkit bereitgestellte reaktive Medium umfasst oder anderweitig in Kontakt mit diesem gebracht werden kann, wobei das probenreaktive Medium sein visuelles Erscheinungsbild zumindest teilweise ändert, wenn es in ausreichenden Kontakt mit der Körperflüssigkeit oder anderweitig mit der biometrischen Probe der Person (1) kommt, wobei die Änderungen des visuellen Erscheinungsbildes auf die eine oder mehrere der Gesundheitsmessungen (2d) hinweisen, wobei die Person (1) die Rechenvorrichtung und die App (2a, 2-3, 2-4, 2-5) verwendet, um (Funktion c1) auszuführen, indem sie mindestens ein Bild des probenreaktiven Mediums nach dem Auftreten irgendwelcher Änderungen im visuellen Erscheinungsbild des probenreaktiven Mediums aufnimmt und danach das mindestens eine Bild verarbeitet, um die eine oder mehrere der Gesundheitsmessungen (2d) zumindest teilweise auf der Grundlage des visuellen Erscheinungsbildes des probenreaktiven Mediums zu bestimmen, und wobei die Computervorrichtung und die App (2a, 2-3, 2-4, 2-5) die eine oder mehrere der Gesundheitsmessungen (2d) als von der Person (1) authentifiziert annimmt.

7. System nach Anspruch 1, wobei es sich bei der mindestens einen Gesundheitsmessvorrichtung (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) um eine temporäre Einschaltvorrichtung (3-1-d1, 3-1-d1, 3-1-d2, 3-2, 3-1-d6, 3-1-d7, 3-1-d8, 3-1-d9, 4-2, 4-4), eine tragbare Vorrichtung zum dauerhaften Eingriff (3-1-d4, 3-1-d5, 4-1a, 4-1b, 4-3, 131) oder eine Vorrichtung zur Sammlung biometrischer Proben (4-2, 80, 84) ist, und wobei:
die temporäre Eingriffsvorrichtung (3-1-d1, 3-1-d2, 3-2, 3-1-d6, 3-1-d7, 3-1-d8, 3-1-d9, 4-2, 4-4) eine am Finger getragene Vorrichtung (3-1-d2, 3-1-d7, 3-1-d8, 3-1-d9), eine in eine Körperöffnung (3-1-d1) eingeführte Vorrichtung eine Fernerkundungsvorrichtung (3-2, 3-1-d6), eine Vorrichtung zur Analyse des Atems der Person (1) (4-2) oder eine Vorrichtung zur Messung des Körpergewichts der Person (1) oder sonstiger vom Körper ausgeübter Kräfte und zugehöriger Daten (4-4);
die am Handgelenk, am Hals, am Knöchel, an der Taille, am Rumpf oder an den Fingern getragene Vorrichtung (3-1-d4, 3-1-d5, 4-1a, 4-1b, 4-3, 131), eine im Gesicht getragene Vorrichtung oder Maske (131) und eine auf die Haut geklebte Vorrichtung oder ein Pflaster (4-1a, 4-1b) ist, und
die biometrische Probensammelvorrichtung (4-2, 80, 84) ein Tupfer (80) mit einer Tupferspitze (80-tip), ein Teststreifen (84) mit einem Probensammelbereich (84-smp) oder ein Alkoholtester (4-2) ist.

8. System nach Anspruch 1, bei dem der Gesundheitsdienstleister (42) die mindestens eine Gesundheitsdienstleistung (3-2) erbringt und bei der Erbringung der mindestens einen Gesundheitsdienstleistung (3-2) die Durchführung der (Funktion 2), die die Kombination aus der (Funktion a), der (Funktion b2) und der (Funktion c2-2) umfasst, umfassend:
die Person (1) verwendet das Computergerät und die App (2a, 2-3, 2-4, 2-5), um die (Funktion a) und die (Funktion b2) durchzuführen, während sie im Wesentlichen innerhalb einer Bestätigungszone (73) isoliert ist, wobei während der (Funktion b2) das Computergerät und die App (2a, 2-3, 2-4, 2-5) während der (Funktion b2) zusätzlich oder alternativ einem System oder einem Service-Agenten des Gesundheitsdienstleisters (42) ein Authentifizierungszertifikat oder anderweitige Bestätigungsinformationen bereitstellt, die eine oder eine Kombination aus einem Identitätscode der App (2a) oder einem eindeutigen digitalen Zertifikat, einer eindeutigen Transaktionsnummer, Versicherungsinformationen, einem Antwortcode, der zumindest teilweise durch einen zuvor von dem System oder dem Service-Agenten bereitgestellten Anfangscode verursacht wurde ein Bild der Person (1) oder andere Daten, die bestätigen, dass das Computergerät und die App (2a, 2-3, 2-4, 2-5) in der (Funktion a) festgestellt hat, dass die Person (1), die sich derzeit isoliert in der Bestätigungszone (73) befindet, durch das Computergerät und die App (2a, 2-3, 2-4, 2-5) als die vorregistrierte Person (1) authentifiziert ist, wobei das Authentifizierungszertifikat oder eine andere Bestätigungsinformation in einem Format bereitgestellt wird, das eine elektronisch übermittelte Information oder eine visuell dargestellte Information umfasst, die auf einem Bildschirm des Computergeräts und der App (2a, 2-3, 2-4, 2-5) dargestellt wird, und keine persönlich identifizierende Information außer einem Bild der Person (1) umfasst, so dass die Person (1) anonym bleibt, wobei nach dem Empfang des Authentifizierungszertifikats oder einer anderen Bestätigung der Service-Agent oder ein anderer Agent des Anbieters von Gesundheitsdienstleistungen (42) die mindestens eine Gesundheitsdienstleistung (3-2) für die Person (1) bereitstellt, wobei nach der Bereitstellung der mindestens einen Gesundheitsdienstleistung (3-2) das System oder ein Service-Agent des Anbieters von Gesundheitsdienstleistungen (42) die Empfangsbestätigung der Dienstleistung an das Computergerät und die App (2a, 2-3, 2-4, 2-5), die bestätigen, dass die mindestens eine Gesundheitsdienstleistung (3-2) für die Person (1) erbracht wurde, wobei die Leistungsbestätigung eine beliebige oder eine Kombination aus einer Identifikation des Gesundheitsdienstleisters (42), einer Identifikation des einen oder der mehreren Dienstleistungsagenten einer Identifikation der mindestens einen erbrachten Gesundheitsdienstleistung (3-2), Versicherungsinformationen, einem Authentifizierungszertifikat oder anderen Bestätigungsinformationen oder anderen Transaktionsdaten in Bezug auf die mindestens eine erbrachte Gesundheitsdienstleistung (3-2), wobei die Leistungsbestätigung in einem Format bereitgestellt wird, das elektronisch übermittelte Informationen oder visuell dargestellte Informationen umfasst, und wobei das Computergerät und die App (2a, 2-3, 2-4, 2-5) die Leistungsbestätigung ausführt (Funktion c2-2) und empfängt, um zumindest teilweise zu bestätigen, dass die mindestens eine Gesundheitsdienstleistung (3-2) für die zugehörige anonyme Person (1) erbracht wurde.

9. System nach Anspruch 1, wobei das Zugangskontrollsystem (73) mindestens einen kontrollierten, gesundheitsüberprüften Zugangspunkt (5a, 5b) umfasst, der eine Bestätigungszone (73) und dem Ort oder der Versammlung (74) trennt und miteinander verbindet, wobei die Bestätigungszone (73) einen physisch, elektronisch oder visuell eingeschränkten, halb-eingeschränkten oder anderweitig überwachten Bereich (73) umfasst, um die Person (1) im Wesentlichen zu isolieren, während der authentifizierte Gesundheitsstatus (2d) anonym empfangen wird, was ausreicht, um sicherzustellen, dass der empfangene Status (2d) von der Person (1) stammt, wobei das Zugangskontrollsystem (73) die Funktion 3 durchführt, indem es mit dem Computergerät und der App (2a, 2-3, 2-4, 2-5) kommuniziert, um die Zugangsanfrage auszuhandeln, während sich die Person (1) innerhalb der Bestätigungszone (73) befindet, und wobei das Zugangskontrollsystem (73) den Zugang zu dem Ort oder der Versammlung (74) durch die Person (1) über den mindestens einen kontrollierten gesundheitsgeprüften Zugangspunkt (5a, 5b) zumindest teilweise auf der Grundlage des empfangenen anonymen authentifizierten Gesundheitsstatus (2d) regelt.

10. System nach Anspruch 9, bei dem die Person (1) das Computergerät und die App (2a, 2-3, 2-4, 2-5) verwendet, während sie sich in der Bestätigungszone (73) befindet, um zusatzlich eine oder eine Kombination zu liefern von: persönlichen Identifizierungsinformationen, und elektronischen Informationen, die eine Zugangsberechtigung (2b, 2c) zu dem Ort oder der Versammlung (74) belegen, wobei die Zugangsberechtigung (2b, 2c) entweder für den anonymen Zugang einer öffentlichen Person (1) oder den nicht-anonymen Zugang einer Privatperson (1-w) gilt, wobei das Zugangskontrollsystem (73) den Zugang zu dem Ort oder der Versammlung (74) durch die öffentliche Person (1) oder die Privatperson (1-w) zumindest teilweise auf der Grundlage des empfangenen authentifizierten Gesundheitsstatus (2d) und zumindest teilweise auf der Grundlage der authentifizierten Zutrittsberechtigung (2b, 2c) regelt.

11. System nach Anspruch 1, bei dem eine Vielzahl von Personen (1) eines der ein oder mehreren Gesundheitsregimes (2d-1, 2d-2) zur Bestimmung des privat authentifizierten Gesundheitszustands (2d) jeder Person (1), die die Vielzahl von Personen (1) umfasst, erhalten haben und befolgen, ferner umfassend:
eine gemeinsam genutzte öffentliche Gesundheitsdatenbank (102-db) zum Empfangen anonymisierter privater Gesundheitsdaten (2a-db) von einer beliebigen Person aus der Vielzahl von Personen (1), wobei die anonymisierten privaten Gesundheitsdaten (2a-db) eine beliebige Information oder eine beliebige Kombination von Informationen in Bezug auf den Gesundheitszustand (2d) einer jeden Person aus der Vielzahl von Personen (1) umfassen, und
einen oder mehrere Algorithmen des öffentlichen Gesundheitswesens (102-ml) zum Verarbeiten beliebiger der anonymisierten privaten Gesundheitsdaten (2a-db), die die gemeinsam genutzte Datenbank des öffentlichen Gesundheitswesens (102-db) umfassen, wobei der eine oder die mehreren Algorithmen des öffentlichen Gesundheitswesens (102-ml) eine oder mehrere Rundsendenachrichten (102-msg) zumindest teilweise auf der Grundlage einer der Verarbeitungen bestimmen, wobei jede der einen oder mehreren Broadcast-Nachrichten (102-msg) mindestens einen trainierten Detektor (102-det), keine persönlich identifizierenden Informationen und eine oder eine Kombination von Empfehlungen oder Aktualisierungen für eines der einen oder mehreren Gesundheitsregimes (2d-1, 2d-2) umfasst, wobei die eine oder mehreren Broadcast-Nachrichten (102-msg) elektronisch an die Computergeräte und Apps (2a, 2-3, 2-4, 2-5) elektronisch verteilt werden, die von einigen oder allen der Vielzahl von Personen (1) verwendet werden, wobei das empfangende Computergerät und die empfangende App (2a, 2-3, 2-4, 2-5) nach dem Empfang einer Broadcast-Nachricht (102-msg) den trainierten Detektor (102-det) zur Verarbeitung beliebiger privater Gesundheitsdaten (2a-db) jeder Person (1), die mit dem empfangenden Computergerät und der App (2a, 2-3, 2-4, 2-5) verbunden ist, ausführt, um eine oder mehrere private Gesundheitsdaten (2a-db) zu bestimmen, 2-5) assoziierten Person (1) ausführt, um ein oder mehrere private Ergebnisse zu ermitteln, und wobei die empfangende Rechenvorrichtung und App (2a, 2-3, 2-4, 2-5) der assoziierten Person (1) entweder die Empfehlungen bereitstellt oder eines der ein oder mehreren Gesundheitsregimes (2d-1, 2d-2) aktualisiert, die derzeit von der assoziierten Person (1) zumindest teilweise auf der Grundlage der privaten Ergebnisse der Person (1) verwendet werden.

12. System nach Anspruch 1, bei dem dem Ort oder der Versammlung (74) ein festes Gebäude oder ein bewegliches Transportfahrzeug ist oder anderweitig in einem solchen stattfindet, das einer Einrichtung (4) gehört oder von dieser betrieben wird, wobei das Zugangskontrollsystem (73) zur Regelung des Zugangs zu dem Ort oder der Versammlung (74) einen oder mehrere öffentliche oder private Zugangspunkte (5a, 5b) umfasst, die jeweils dazu dienen, den öffentlichen oder privaten Zugang durch eine beliebige aus einer Vielzahl von Personen (1, 1-w) zu regeln, und zwar zumindest teilweise auf der Grundlage des Empfangs eines hinreichend konformen Gesundheitszustands (2d) von den Personen (1, 1-w), die den Zugang versuchen, wobei die Einrichtung (4) jeder der Personen (1, 1-w), die den Zugang erwägen, eine gegenseitige Zusicherungsinformation bezüglich des aktuellen Gesundheitszustands dem Ort oder der Versammlung (74) zur Verfügung stellt, 1-w), die den Zutritt erwägen, gegenwärtig Zutritt suchen oder bereits Zutritt zu dem Ort oder der Versammlung (74) haben, wobei die gegenseitige Zusicherungsinformation ein Hinweis auf den Gesundheitszustand (2d) einiger oder im Wesentlichen aller Personen (1, 1-w) ist, die sich gegenwärtig in den Räumlichkeiten oder der Versammlung befinden, diese bewohnen oder anderweitig an dem Ort oder der Versammlung (74) zu einem bestimmten Zeitpunkt oder über einen bestimmten Zeitraum teilnehmen, und wobei die Informationen über die gegenseitige Sicherheit zumindest teilweise auf dem Gesundheitszustand (2d) basieren, der von jeder Person (1, 1-w) angegeben wird, der das Betreten dem Ort oder der Versammlung (74) durch das Zugangskontrollsystem (73) während des Zeitraums gestattet wird.

13. System nach Anspruch 1, wobei dem Ort oder der Versammlung (74) ein festes Gebäude oder ein bewegliches Transportfahrzeug ist oder anderweitig in einem solchen stattfindet, das einer Einrichtung (4) gehört oder von dieser betrieben wird, wobei das Zugangskontrollsystem (73) zur Regelung des Zugangs zu dem Ort oder der Versammlung (74) einen oder mehrere öffentliche oder private Zugangspunkte (5a, 5b) umfasst, die jeweils zur Regelung des öffentlichen oder privaten Zugangs einer beliebigen Person (1, 1-w) aus einer Vielzahl von Personen (1, 1-w) dienen, die zumindest teilweise auf dem Empfang eines hinreichend konformen Gesundheitszustands (2d) von der beliebigen Person (1, 1-w), die versucht, Zutritt zu erhalten, wobei eine oder mehrere der beliebigen Personen (1, 1-w), denen das Zutrittskontrollsystem (73) Zutritt zu dem Ort oder der Versammlung (74) gewährt, eine verfolgte zugelassene Person sind, die ein gesichertes Wearable (2-3, 3-1-d4, 3-1-d5) trägt, das elektronische Verfolgungsmittel umfasst, wobei dem Ort oder der Versammlung (74) ein ausreichendes Verfolgungsinfrastrukturnetz umfasst, das in einigen oder im Wesentlichen allen Bereichen dem Ort oder der Versammlung (74) positioniert ist, um im Wesentlichen die Bewegungen aller gesicherten Wearables (2-3, 3-1-d4, 3-1-d5), die von einer oder mehreren verfolgten zugelassenen Personen (1, 1-w) getragen werden, zu verfolgen, die ausreichen, um Kontakte innerhalb einer gegebenen Nähe zwischen zwei oder mehreren verfolgten zugelassenen Personen (1, 1-w) zu bestimmen, die gegenwärtig ein gesichertes Wearable (2-3, 3-1-d4, 3-1-d5) tragen und sich gegenwärtig innerhalb dem Ort oder der Versammlung (74) befinden, wobei die Einrichtung (4) jeder der einen oder mehreren verfolgten zugelassenen Personen (1, 1-w), die gegenwärtig ein gesichertes Wearable (2-3, 3-1-d4, 3-1-d5) tragen oder getragen haben, Vertragsverfolgungsinformationen (2a-db-6) bereitstellt, 3-1-d4, 3-1-d5) trägt oder getragen hat und derzeit auf dem Ort oder bei der Versammlung (74) anwesend ist oder war, zumindest teilweise auf der Grundlage der ermittelten Kontakte, an denen die verfolgte zugelassene Person (1, 1-w) beteiligt ist, und zumindest teilweise auf der Grundlage des Gesundheitszustands (2d) einer oder mehrerer anderer verfolgter zugelassener Personen (1, 1-w), die derzeit ein gesichertes Wearable (2-3, 3-1-d4, 3-1-d5) tragen oder getragen haben und an den ermittelten Kontakten beteiligt sind.

14. System nach Anspruch 13, bei dem entweder eines oder beides eintritt: mindestens eine der nachverfolgten aufgenommenen Personen (1, 1-w) verbleibt nach der Aufnahme in zu dem Ort oder der Versammlung (74) für eine ausreichende Zeitdauer in dem Ort oder der Versammlung (74), um die Bestimmung von mindestens einer zusätzlichen Gesundheitsmessung (2d, 2a-db-4a) oder mindestens einer zusätzlichen Gesundheitsdienstleistung (2d, 2a-db-4a) zur Verwendung bei der Bestimmung eines aktualisierten Gesundheitszustands (2d) zu verlangen, um nach dem Verbleib in dem Ort oder der Versammlung (74) mit dem einen oder den mehreren Gesundheitsregimes (2d-1, 2d-2) konform zu bleiben, wobei die Einrichtung (4) verlangt, dass die mindestens eine nachverfolgte aufgenommene und verbleibende Person (1, 1-w) weiterhin das eine oder die mehreren Gesundheitsregimes (2d-1, 2d-2) befolgt und konform bleibt, wobei die Einrichtung (4) weiterhin den aktualisierten Gesundheitszustand (2d) der mindestens einen erfassten zugelassenen und verbleibenden Person (1-w) während der gesamten Zeit, in der sich die mindestens eine erfasste zugelassene und verbleibende Person (1, 1-w) in dem Ort oder der Versammlung (74) aufhält, dort verweilt oder sich anderweitig dort aufhält, ermittelt und anderweitig erhält, und wobei die Einrichtung (4) bei einer ausreichenden Änderung des Gesundheitszustands (2d) der mindestens einen erfassten zugelassenen und verbleibenden Person (1, 1-w) stellt die Einrichtung (4) Informationen zur Vertragsverfolgung an eine oder mehrere andere verfolgte zugelassene Personen (1, 1-w) bereit, die zuvor von der Einrichtung (4) als in ausreichendem Nahkontakt mit der mindestens einen verfolgten zugelassenen und verbleibenden Person (1, 1-w) stehend ermittelt wurden, während sie gleichzeitig in in der sich die mindestens eine erfasste zugelassene und verbleibende Person (1, 1-w) in dem Ort oder der Versammlung (74) aufhält, anwesend waren, und
mindestens eine der verfolgten aufgenommenen Personen (1, 1-w), nachdem sie in dem Ort oder der Versammlung (74) aufgenommen wurde, dem Ort oder der Versammlung (74) für eine ausreichende Zeitdauer verlässt, um die Bestimmung mindestens einer zusätzlichen Gesundheitsmessung (2d, 2a-db-4a) oder mindestens einer zusätzlichen Gesundheitsdienstleistung (2d, 2a-db-4a) zur Verwendung bei der Bestimmung eines aktualisierten Gesundheitszustands (2d) zu erfordern, um mit dem einen oder mehreren Gesundheitsregimes (2d-1, 2d-2) nach dem Verlassen dem Ort oder der Versammlung (74), wobei die Einrichtung (4) verlangt, dass die mindestens eine nachverfolgte zugelassene und ausreisende Person (1, 1-w) nach dem Verlassen dem Ort oder der Versammlung (74) für eine längere Zeitspanne, die größer oder gleich einer ausreichenden Zeitspanne ist, weiterhin die eine oder mehreren Gesundheitsregimes (2d-1, 2d-2) befolgt und diese einhält, wobei die Einrichtung (4) weiterhin den aktualisierten Gesundheitszustand (2d) der mindestens einen verfolgten aufgenommenen und austretenden Person (1, 1-w) während der verlängerten Dauer der ausreichenden Zeit, nach der die mindestens eine verfolgte aufgenommene und austretende Person (1, 1-w) dem Ort oder der Versammlung (74) verlassen hat, ermittelt und anderweitig erhält, und woraufhin eine ausreichende Änderung des Gesundheitszustands (2d) der mindestens einen verfolgten aufgenommenen und austretenden Person (1, 1-w) nach dem Verlassen dem Ort oder der Versammlung (74) eine hinreichende Änderung des Gesundheitszustands (2d) der mindestens einen erfassten aufgenommenen und abgereisten Person (1, 1-w) eintritt, stellt die Einrichtung (4) Informationen zur Vertragsverfolgung einer oder mehrerer anderer erfasster aufgenommener Personen (1, 1-w) zur Verfügung, die zuvor von der Einrichtung (4) ermittelt wurden und in ausreichendem Nahkontakt mit der mindestens einen erfassten aufgenommenen und abgereisten Person (1, 1-w) standen, während sie gleichzeitig in dem Ort oder der Versammlung (74) anwesend waren.

15. System nach Anspruch 1, bei dem dem Ort oder der Versammlung (74) im Besitz einer Einrichtung (4) ist oder von dieser betrieben wird, wobei die Person (1) den Zugang zu dem Ort oder der Versammlung (74) plant, und das auBerdem eines von Folgendem umfasst:
einem Person-zu-Service-Terminvereinbarungssystem (104, 52), das von oder für die Einrichtung (4) betrieben wird, wobei:
die Person (1) mit dem Person-zu-Service-Terminsystem (104, 52) interagiert, um mindestens ein Datum oder ein Datum und eine Uhrzeit eines geplanten Zugangs zu dem Ort oder der Versammlung (74) zu planen, woraufhin oder nach der Interaktion mit dem Person-zu-Service-Terminsystem (104, 52) das Person-zu-Service-Terminsystem (104, 52) dem Computergerät und der App (2a, 2-3, 2-4, 2-5), die bei der Person (1) registriert sind und von dieser verwendet werden, ein Terminregime (2d-3a) zur Verfügung stellt oder anderweitig verfügbar macht, das Informationen umfasst, die ausreichen, um als Teil oder als Ganzes eines Gesundheitsregimes (2d-1, 2d-2) zu dienen, dieses zu aktualisieren, zu ändern oder anderweitig zu bilden, damit es von der Person (1) vor dem Versuch des geplanten Zugangs eingehalten wird, wobei das Computergerät und die App (2a, 2-3, 2-4, 2-5) vor dem Versuch des geplanten Zugangs zu dem Ort oder der Versammlung (74) ein oder mehrere Verifizierungs-Token (2d-3b) bereitstellt, die ein beliebiges Verifizierungsdatum umfassen, das zumindest teilweise auf einer beliebigen Gesundheitsinformation oder einer beliebigen Kombination von Gesundheitsinformationen basiert, die sich auf das Terminregiment (2d-3a) oder das eine oder die mehreren Gesundheitsregimes (2d-1, 2d-2), wobei die Gesundheitsinformationen Informationen in Bezug auf den Gesundheitszustand (2d) der Person (1) umfassen, wobei das Terminvereinbarungssystem (104, 52) nach Erhalt des einen oder der mehreren Verifizierungs-Token (2d-3b) eine der folgenden Maßnahmen oder eine Kombination davon ergreift:
(i) Verschiebung des Datums oder des Datums und der Uhrzeit des geplanten Zugangs zu dem Ort oder der Versammlung (74);
(ii) Bereitstellung oder anderweitige Bereitstellung von Aktualisierungen für das Computergerät und die App (2a, 2-3, 2-4, 2-5) oder ein neues Terminregime (2d-3a);
(iii) Benachrichtigung oder anderweitige Aktualisierung eines oder einer Kombination von einem oder mehreren Mitarbeitern, die für die Einrichtung (4) arbeiten oder von denen anderweitig erwartet wird, dass sie mit der Person (1) interagieren, wenn oder nachdem die Person (1) dem Ort oder der Versammlung (74) betritt;
(iv) Bereitstellen eines Zugangstokens (2d-3c) für die Computervorrichtung und die App (2a, 2-3, 2-4, 2-5), wobei der Zugangstoken (2d-3c) zumindest teilweise auf der ausreichenden Einhaltung mindestens eines vom Planungssystem (104, 52) bereitgestellten Terminregimes (2d-3a) durch die Person (1) basiert, und
wobei die Person (1) bei der Interaktion mit dem Zugangskontrollsystem (73) an dem geplanten Datum oder Datum und Uhrzeit des geplanten Zugangs die Computervorrichtung und die App (2a, 2-3, 2-4, 2-5) verwendet, um eine beliebige der Zugangstoken-Informationen bereitzustellen, die zumindest teilweise auf dem Zugangstoken (2d-3c) basiert, entweder alternativ oder zusätzlich zur Bereitstellung des anonymen, authentifizierten Gesundheitsstatus (2d) bereitzustellen, und
wobei das Zugangskontrollsystem (73) den Zugang der Person (1) zu dem Ort oder der Versammlung (74) zumindest teilweise auf der Grundlage der Informationen des Zugangstokens (2d-3c) regelt,
und
ein Person-zu-Person-Verabredungssystem (105, 53), das von oder für die Einrichtung (4) oder anderweitig von einer oder mehreren Personen betrieben wird, die eine Zusammenkunft zwischen zwei oder mehreren Personen (1) verwalten, wobei:
die zwei oder mehr Personen (1) mit dem Person-zu-Person-Terminsystem (105, 53) interagieren, um mindestens ein Datum oder ein Datum und eine Uhrzeit eines geplanten Treffens oder anderweitigen Treffens (74) zu planen, wobei bei der Interaktion mit dem Person-zu-Person-Terminsystem (105, 53) das Person-zu-Person-Terminsystem (105, 53) jedem der Computergeräte und Apps (2a, 2-5-1, 2-5-2), die bei jeder der zwei oder mehr Personen (1) registriert sind und von diesen genutzt werden, ein Terminreglement (2d-3a-1, 2d-3a-2) zur Verfügung stellt oder anderweitig verfügbar macht, das Informationen umfasst, die ausreichen, um als Teil oder als Ganzes eines Gesundheitsregimes (2d-1, 2d-2) zu dienen, dieses zu aktualisieren, zu ändern oder anderweitig zu bilden, das von den zwei oder mehr Personen (1) einzuhalten ist, bevor sie versuchen, an der geplanten Zusammenkunft oder anderweitigen Zusammenkunft (74) teilzunehmen, wobei vor dem Versuch, an dem geplanten Treffen oder anderweitigen Treffen (74) teilzunehmen, jedes Computergerät und jede App (2a, 2-5-1, 2-5-2) ein oder mehrere Verifizierungs-Token (2d-3b-1, 2d-3b-2) bereitstellt, die ein beliebiges Verifizierungsdatum umfassen, das zumindest teilweise auf einer beliebigen Gesundheitsinformation oder einer beliebigen Kombination von Gesundheitsinformationen in Bezug auf ein beliebiges Terminregiment (2d-3a-1, 2d-3a-2) oder dem einen oder den mehreren öffentlichen Gesundheitsregime (2d-1, 2d-2) basieren, wobei die Gesundheitsinformationen Informationen in Bezug auf den Gesundheitszustand (2d) einer der zwei oder mehreren Personen (1) umfassen, wobei das Person-zu-Person-Terminsystem (105, 53) nach Erhalt des einen oder der mehreren Verifizierungs-Token (2d-3b-1, 2d-3b-2) eine der folgenden Maßnahmen oder eine Kombination davon ergreift:
(i) Verlegung des Datums oder des Datums und der Uhrzeit der geplanten Zusammenkunft oder des sonstigen Treffens (74);
(ii) Bereitstellung oder anderweitige Bereitstellung von Aktualisierungen oder eines neuen Terminplans (2d-3a-1, 2d-3a-2) für jedes der Computergeräte und Apps (2a, 2-5-1, 2-5-2)
(iii) Benachrichtigung oder anderweitige Aktualisierung einer oder mehrerer Personen, die für die Einrichtung (4) arbeiten, die Versammlung oder anderweitige Zusammenkunft (74) leiten oder von denen anderweitig erwartet wird, dass sie mit einer der beiden oder mehreren Personen (1) interagieren, wenn oder nachdem die eine der beiden oder mehreren Personen (1) auf die geplante Versammlung oder anderweitige Zusammenkunft (74) zugreift, und einer der beiden oder mehreren Personen (1), die planen, an der Versammlung oder anderweitigen Zusammenkunft (74) teilzunehmen;
(iv) Bereitstellen eines Zugangstokens (2d-3c-1, 2d-3c-2) für jedes der Computergeräte und Apps (2a, 2-5-1, 2-5-2), wobei der jeweils bereitgestellte Zugangstoken (2d-3c-1, 2d-3c-2) zumindest teilweise auf der ausreichenden Einhaltung mindestens eines vom Planungssystem (105, 53) bereitgestellten Terminplans (2d-3a-1, 2d-3a-2) durch jede Person (1) der zwei oder mehr Personen (1) beruht, und
wobei nach dem Empfang eines Zugangstokens (2d-3c) jede Person (1) der zwei oder mehr Personen (1) in die Lage versetzt wird, an der geplanten Versammlung oder anderweitigen Zusammenkunft (74) teilzunehmen, und
wobei die Teilnahme optional entweder die Interaktion mit dem Zugangskontrollsystem (73) an dem geplanten Datum oder Datum und Uhrzeit der geplanten Versammlung oder anderweitigen Zusammenkunft (74) umfasst, um eine beliebige Zugangstoken-Information bereitzustellen, die zumindest teilweise auf dem empfangenen Zugangstoken (2d-3c-1, 2d-3c-2), entweder alternativ oder zusätzlich zur Bereitstellung des anonymen, authentifizierten Gesundheitsstatus (2d), oder die Interaktion mit einem der jeweiligen Computergeräte und Apps (2a, 2-5-1, 2-5-2), die von einer anderen der zwei oder mehr Personen (1) verwendet werden, um eine der Zugangstoken-Informationen bereitzustellen, die zumindest teilweise auf dem empfangenen Zugangstoken (2d-3c) basieren, entweder alternativ oder zusätzlich zur Bereitstellung des anonymen, authentifizierten Gesundheitsstatus (2d).

## Revendications

1. Système (102, 103, 104) pour régir l'accès d'une personne (1) à des locaux ou à un rassemblement (74) sur la base, au moins en partie, d'un état de santé authentifié anonyme et conforme au public (2d) de la personne (1), lorsque la personne (1) utilise un dispositif informatique et une application (2a, 2-3, 2-4, 2- 5) comme moyen de fournir une auto-authentification privée, comprenant:
un système de contrôle d'accès (73) pour régir l'accès de la personne (1) aux locaux ou au rassemblement
(74);
le dispositif informatique (2a, 2-3, 2-4, 2-5) utilisé par la personne (1) cherchant à accéder aux locaux ou au rassemblement (74);
au moins un dispositif de mesure de la santé authentifiant (3-1, 3-1-d1, 3-1-d2, 3-1-d4, 3-1-d5, 3-2, 3-3, 3- 1-d6, 3-1-d7, 3-1-d8, 3-1-d9, 3-1-d10, 4-1a, 4-1b, 4-2, 4-3, 4-4, 80, 84, 131) ou au moins un prestataire de services de santé authentifiant (42), où l'au moins un dispositif de mesure de la santé (3-1, 3-1-d1, 3-1-d2, 3-1-d4, 3-1-d5, 3-2, 3-2d, 3-3d, 3-1-d6, 3-1-d7, 3-1-d8, 3-1-d9, 3-1-d10, 4-1a, 4-1b, 4-2, 4-3, 4-4, 131)
détermine au moins une mesure de santé (2d, 2a-db-4a) authentifiée comme étant celle de la personne (1), où l'au moins un prestataire de services de santé (42) détermine au moins une mesure de santé (2d, 2a-db-4a) authentifiée comme étant celle de la personne (1) ou fournit au moins un service de santé (2d, 2a-db-4a) authentifié comme étant fourni à la personne (1);
une application intermédiaire (2a) exécutée sur le dispositif informatique (2a, 2-3, 2-4, 2-5) dans laquelle la personne (1) enregistre une ou plusieurs données biométriques avec l'application intermédiaire (2a) pour une utilisation ultérieure pendant une (fonction a) d'authentification de la personne (1) , dans laquelle la (fonction a) comprend la confirmation de l'identité de la personne (1) en déterminant et en comparant une donnée biométrique actuelle avec une ou plusieurs données biométriques enregistrées;
un ou plusieurs registres publics (2d-1, 2d-2) pour déterminer la conformité publique de l'état de santé authentifié (2d), le ou les registres publics (2d-1, 2d-2) comprenant des informations numériques spécifiant une ou plusieurs règles , un registre (2d-1, 2d-2) étant distinct de l'application intermédiaire (2a), lorsque l'application intermédiaire (2a) a en outre pour fonction de recevoir, d'extraire, de mettre à jour ou d'obtenir d'une autre manière un ou plusieurs régiments publics (2d-1, 2d-2) ou de combiner ou de traiter d'une autre manière des règles provenant de deux ou plusieurs régiments publics (2d-1, 2d-2) , lorsqu'une règle définit, limite ou régit d'une autre manière le traitement d'une ou de plusieurs règles,
limite ou régit d'une autre manière le traitement de l'application intermédiaire (2a) lors de la détermination d'au moins une mesure de santé authentifiée (2d, 2a-db-4a) et d'au moins un service de santé authentifié (2d, 2a-db-4a), et lorsque les règles spécifient une formulation de toute information associée à l'une des mesures de santé authentifiées (2d, 2a-db-4a) ou à une combinaison de celles-ci et à l'un des services de santé authentifiés (2d, 2a-db-4a) pour la détermination et le maintien de l'état de santé authentifié (2d) de la personne (1), conforme aux règles publiques, et
lorsqu'avant que la personne (1) ne tente d'accéder aux locaux ou au rassemblement (74) dans une (fonction 3), la personne (1) utilise au moins en partie le dispositif informatique et l'application intermédiaire (2a, 2-3, 2-4, 2-5) pour effectuer l'une des fonctions suivantes ou une combinaison de celles-ci (fonction 1) et (fonction 2), dans laquelle:
la (fonction 1) comprend la communication avec au moins un dispositif de mesure de la santé (3-1, 3-1-d1, 3-1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84,
131) pour lancer au moins une mesure de santé authentifiée (2d, 2a-db-4a) , où, pendant ou après le lancement, le dispositif informatique et l'application intermédiaire (2a, 2-3, 2-4, 2-5) exécutent la fonction (fonction a) pour confirmer l'identité de la personne (1) en déterminant au moins un élément biométrique à comparer à un élément biométrique d'enregistrement tout en exécutant sensiblement simultanément une (fonction b1) pour confirmer qu'une surface de détection de l'appareil (3-1, 3-1-d1, 3-1-d2, 3-1-d4, 3- 1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131) touche
sensiblement le corps de la personne (1), se trouve à proximité de celui-ci ou est autrement située au même endroit que lui pour garantir que l'au moins une mesure de l'état de santé de la personne (1) est prise , et l'une ou l'autre des fonctions suivantes (fonction c1) pour recevoir, extraire ou obtenir d'une autre manière l'au moins une mesure de l'état de santé de la personne (1), récupérer ou obtenir d'une autre manière l'au moins une mesure de santé (d2, 2a-db-4a) à partir du dispositif (3-1, 3-1-d1, 3-1-d2, 3- 1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131), où la
mesure de santé reçue (2d, 2a-db-4a) est authentifiée comme étant celle de la personne (1), et
la (fonction 2) comprend la communication avec au moins un fournisseur de services de santé (42) pour initier ou répondre à l'initiation d'au moins une mesure de santé (2d, 2a-db-4a) ou d'au moins un service de santé (3-2), où, pendant ou après l'initiation, le dispositif informatique et l'application intermédiaire (2a, 2-3, 2-4, 2-5) exécutent la fonction (fonction a) pour confirmer l'identité de la personne (1) en déterminant au moins un élément biométrique à comparer à un élément biométrique d'enregistrement, tout en exécutant sensiblement en même temps une fonction (fonction b2) pour fournir des informations anonymes au prestataire de services de santé (42) afin de les associer à l'au moins une mesure de santé (2d, 2a-db-4a) ou au moins un service de soins de santé (3-2) , les informations anonymes ne comprenant pas d'informations d'identification personnelle concernant la personne (1), et si le prestataire de services
de soins de santé (42) a déterminé au moins une mesure de santé (2d, 2a-db-4a), le dispositif informatique et l'application intermédiaire (2a, 2-3, 2-4, 2-5) exécutent une (fonction c2-1) pour recevoir, récupérer ou obtenir autrement l'au moins une mesure de santé (2d, 2a-db-4a) auprès du prestataire de services de santé (42), et si le prestataire de services de santé (42) a fourni au moins un service de santé (3-2), le dispositif informatique et l'application intermédiaire (2a, 2-3, 2-4, 2-5) exécutent une (fonction c2-2) pour recevoir, récupérer ou obtenir d'une autre manière un reçu de confirmation de service du prestataire de services de santé (42) confirmant que l'au moins un service de santé (3-2) a été fourni à la personne anonyme associée (1), où l'au moins une mesure de santé (2d, 2a-db-4a) ou l'au moins un
service de santé (3-2) reçu, récupéré ou obtenu d'une autre manière est authentifié comme étant celui de la personne (1), et lorsque la personne (1) utilise, au moins en partie, le dispositif informatique et l'application (2a, 2-3, 2-4, 2-5) pour exécuter l'une des fonctions suivantes ou une combinaison de celles-ci (fonction 1) et (fonction
2) , la personne (1) utilise ensuite le dispositif informatique et l'application intermédiaire (2a, 2-3, 2-4, 2-5) pour exécuter la (fonction 3) consistant à communiquer avec le système de contrôle d'accès (73) avant que le système de contrôle d'accès (73) n'accorde ou ne refuse à la personne (1) l'accès à un local ou à un rassemblement (74) , au cours de la (fonction 3), le dispositif informatique et l'application intermédiaire (2a, 2-3, 2-4, 2-5) fonctionnent pour demander une autorisation d'entrée, le processus de demande d'entrée comprenant la (fonction a) confirmation de l'identité de la personne (1) en déterminant au moins un élément biométrique à comparer à un élément biométrique d'enregistrement et l'exécution d'une (fonction d) fourniture de l'état de santé authentifié (2d) concernant la personne (1) au système de contrôle d'accès (73), l'état de santé authentifié (2d) est anonyme et ne comprend pas d'informations d'identification personnelle concernant la personne (1), l'état de santé authentifié (2d) est déterminé au moins en partie sur la base de l'une ou de la combinaison d'au moins une mesure de santé authentifiée (2d, 2a-db-4a) de la personne (1) et d'au moins un service de santé authentifié (3-2) fourni à la personne (1), et où le système de contrôle d'accès (73) autorise ou refuse l'accès de la personne (1) aux locaux ou au rassemblement (74) sur la base, au moins en partie, de l'état de santé authentifié anonyme et conforme au public (2d) de la personne (1).

2. Le système de la revendication 1 dans lequel la (fonction b1) comprend l'exécution d'une fonction de partage coopératif de données entre le dispositif informatique et l'application (2a, 2-3, 2-4, 2-5) et l'au moins un dispositif de mesure de la santé (3-1, 3-1-d1, 3-1-d2, 3-1-d4, 3-1-d5, 3-2, 3-2d, 3-3d, 3-1-d6, 3-1- d7, 3-1-d8, 3-1-d9, 3-1-d10, 4-1a, 4-1b, 4-2, 4-3, 4-4, 131), où les données partagées comprennent l'un des éléments suivants ou une combinaison de ceux-ci: un schéma corporel spatial ou temporel de la personne (1) mesuré par le dispositif, un signal lumineux émis en réponse à un signal de commande fourni par le dispositif informatique et l'application (2a, 2-3, 2-4, 2-5), des informations indiquant un état sécurisé de l'au moins un dispositif de mesure de la santé (3-1, 3-1-d1, 3-1-d2, 3-1-d4, 3-1-d5, 3-2, 3-2d, 3- 3
3d, 3-1-d6, 3-1-d7, 3-1-d8, 3-1-d9, 3-1-d10, 4-1a, 4-1b, 4-2, 4-3, 4-4, 131), des marques visibles du dispositif et un identifiant fourni par voie électronique, les données partagées sont comparées à des informations non fournies par le dispositif informatique et l'application (2d, 2a-db-4a) ou fournies simultanément par un dispositif compagnon (3-1-d9, 3-1-d10) au dispositif informatique et à l'application (2d, 2a-db-4a), afin de s'assurer qu'elles correspondent suffisamment.

3. Le système de la revendication 1 dans lequel l'au moins un dispositif de mesure de la santé (3-1, 3-1-d1, 3- 1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131)
est un dispositif d'engagement temporaire (3-1-d1, 3-1-d2, 3-2, 3-1-d6, 3-1-d7, 3-1-d8, 3-1-d9, 4-4), et où, lors de l'utilisation du dispositif d'engagement temporaire (3-1-d1, 3-1-d2, 3-2, 3-1-d6, 3-1-d7, 3-1-d8, 3-1- d9, 4-4), l'exécution de la (fonction 1) comprenant la combinaison de la (fonction a), de la (fonction b1) et de la (fonction c1) comprend l'un des éléments suivants:
reconnaissance du visage de la personne (1) en même temps que la confirmation de la signalisation lumineuse, le dispositif d'engagement temporaire étant un dispositif d'engagement temporaire à signalisation lumineuse (3-1-d1, 3-1-d7, 3-1-d8, 3-1-d9) comprenant un émetteur de lumière (3-1-d1-led, 3-1-d7-led, 3-1-dt8-led, 3-1-d9-led) et un ou plusieurs capteurs pour déterminer une ou plusieurs mesures de la santé (2d) de la personne (1), le dispositif informatique et l'application (2a, 2-3, 2-4, 2-5) comprennent ou communiquent avec un appareil photo pour capturer des images, et au moins un élément biométrique de la personne (1) préenregistré avec le dispositif informatique et l'application (2a, 2-3, 2-4, 2-5) comprend une image faciale, à un moment donné pendant ou autrement au cours d'une certaine durée simultanée de la (fonction a) et de la (fonction b1), le dispositif informatique et l'application (2a, 2-3, 2-4, 2-5) (i) fournissent un signal de commande au dispositif d'engagement temporaire à signalisation lumineuse (3-1-d1, 3-1-d7, 3-1-d8, 3-1-d9), (ii) capture deux images ou plus comprenant essentiellement une vue simultanée du visage de la personne (1) et du dispositif d'engagement temporaire à signal lumineux (3-1-d1, 3-1-d7, 3-1-d8, 3-1-d9) situé essentiellement en contact ou à proximité suffisante du corps de la personne (1) pour déterminer une ou plusieurs mesures de la santé (2d), lorsque le dispositif d'engagement temporaire (3-1-d1, 3-1-d7, 3-1-d8, 3-1-d9) émettant un signal lumineux se trouve dans le champ de vision simultané de la caméra et utilise au moins en partie le signal de commande pour que l'émetteur de lumière (3-1-d1-led, 3-1-d7-led, 3-1-dt8-led, 3-1-d9-led) émette un signal de réponse comprenant de la lumière détectable par la caméra, lorsque le dispositif informatique et l'application (2a, 2-3, 2-4, 2-5) (iii) traitent les deux images ou plus pour (iii-a) comparer le visage capturé de la personne (1) avec l'image faciale préenregistrée, (iii-b) comparer le signal de réponse émis avec le signal de contrôle fourni, et (iii-c) confirmer le contact substantiel ou la proximité suffisante entre le dispositif d'engagement temporaire à signal lumineux (3-1-d1, 3-1-d7, 3-1-d8, 3-1-d9) et le corps de la personne (1) pour s'assurer qu'une ou plusieurs des mesures de santé (2d) sont celles de la personne (1), et à la suite de correspondances et de confirmations suffisantes, le dispositif informatique et
l'application (2a, 2-3, 2-4, 2-5) exécutent (fonction c1) et reçoivent ou acceptent d'une autre manière une ou plusieurs mesures de santé (2d) garanties provenant du dispositif d'engagement temporaire à signalisation lumineuse (3-1-d1, 3-1-d7, 3-1-d8, 3-1-d9) comme étant authentifiées de la personne (1);
localisation spécifique du corps de la personne (1) par comparaison des formes spatiales en même temps que les mesures de santé colocalisées , lorsque le dispositif d'engagement temporaire est un dispositif d'engagement temporaire à forme spatiale du corps (3-1-d2, 3-1-d6, 3-1-d9) comprenant des moyens pour déterminer simultanément, à partir d'une localisation spatiale du corps sensiblement identique de la personne (1), à la fois une forme spatiale du corps de comparaison et une ou plusieurs des mesures de santé (2d), l'emplacement spécifique du corps dans l'espace comprend un doigt, un visage ou tout autre emplacement identifiable sur le corps de la personne (1), le schéma du corps dans l'espace comprend un schéma d'identification unique du corps de la personne (1) déterminé à l'emplacement spécifique du corps dans l'espace , le schéma d'identification unique comprenant une empreinte digitale, une image faciale ou tout autre schéma du corps dans l'espace identifiable de manière unique, lorsque la biométrie préenregistrée de la personne (1) comprend le schéma corporel spatial de la personne (1) pris en grande partie au même endroit spécifique du corps spatial, lorsqu'à un moment donné pendant ou autrement sur une certaine durée simultanée de la (fonction a) et de la (fonction b1) le dispositif informatique et l'application (2a, 2-3, 2-4, 2-5) (i) reçoivent du dispositif d'engagement temporaire du schéma corporel spatial (3-1-d2, 3-1-d6, 3-1-d9) le schéma corporel spatial de comparaison, et (ii) compare le schéma corporel spatial de comparaison avec le schéma corporel spatial préenregistré, après quoi le dispositif informatique et l'application (2a, 2-3, 2-4, 2-5) exécutent (fonction c1) et reçoivent ou acceptent d'une autre manière une ou plusieurs mesures de santé colocalisées (2d) provenant du dispositif d'engagement temporaire à schéma corporel spatial (3-1-d2, 3-3, 3-1-d6, 3-1-d9) comme étant authentifiées de la personne (1);
utilisation d'un dispositif compagnon (3-1-d9) pour fournir un schéma corporel spatial compagnon ainsi qu'un schéma corporel temporel compagnon concomitant et colocalisé, le dispositif compagnon (3-1-d9) comprenant des moyens pour déterminer simultanément à partir d'un emplacement corporel spatial spécifique sensiblement identique de la personne (1) à la fois le schéma corporel spatial compagnon (3-1- d9-d1) et le schéma corporel temporel compagnon (3-1-d9-d2), lorsque le même emplacement spatial spécifique du corps comprend un doigt ou un autre emplacement identifiable sur le corps de la personne (1), lorsqu'un motif spatial du corps (3-1-d9-d1) comprend un motif d'identification unique du corps de la personne (1) déterminé au même emplacement spatial spécifique du corps, le schéma d'identification unique comprend une empreinte digitale ou tout autre schéma corporel spatial identifiable de manière unique, le schéma corporel temporel d'accompagnement comprend une séquence de mesure des battements cardiaques de la personne (1), le dispositif d'engagement temporaire est un dispositif d'engagement temporaire à schéma corporel temporel (4-4) comprenant des moyens de détermination
d'un échantillon de schéma corporel temporel (4-4-d2) comprenant une séquence de mesure des battements cardiaques de la personne (1) prélevée à un emplacement corporel différent du même emplacement corporel spatial spécifique de la personne (1) et comprenant un ou plusieurs capteurs entrant en contact de manière substantielle avec la personne (1), une proximité suffisante ou un autre engagement mécanique avec le corps de la personne (1) pour déterminer une ou plusieurs mesures de santé (2d) de la personne (1), où au moins une donnée biométrique de la personne (1) préenregistrée avec le dispositif informatique et l'application (2a, 2-3, 2-4, 2-5) comprend un schéma corporel spatial de la personne (1) pris essentiellement à partir du même emplacement corporel spatial spécifique, où à un moment donné pendant ou autrement sur une certaine durée simultanée de la (fonction a) et de la (fonction b1) le dispositif informatique et l'application (2a, 2-3, 2-4, 2-5) (i) reçoivent du dispositif compagnon (3-1-d9) le schéma corporel spatial compagnon (3-1-d9-d1), (ii) reçoit du dispositif compagnon (3-1-d9) le schéma corporel temporel compagnon (3-1-d9-d2), (iii) reçoit du dispositif d'engagement temporaire du schéma corporel temporel (4-4) le schéma corporel temporel échantillon (4- 4-d2), (iv) compare le schéma corporel spatial compagnon (3-1-d9-d1) au schéma corporel spatial préenregistré, et (v) compare le schéma corporel temporel du compagnon (3-1-d9-d2) à l'échantillon de schéma corporel temporel (4-4-d2), après quoi le dispositif informatique et l'application (2a, 2-3, 2-4, 2- 5) exécutent (fonction c1) et reçoivent ou acceptent d'une autre manière une ou plusieurs mesures de santé (2d) provenant du dispositif d'engagement temporaire de schéma corporel temporel (4-4) comme étant authentifiées de la personne (1);
reconnaissance du visage de la personne (1) en même temps que la correspondance du schéma corporel temporel , le dispositif d'engagement temporaire étant un dispositif d'engagement temporaire à schéma corporel temporel (4-4) comprenant des moyens de détermination d'un échantillon de schéma corporel temporel (4-4-d2) comprenant une séquence de mesures des battements cardiaques de la personne (1) et comprenant un ou plusieurs capteurs entrant en contact substantiel, en proximité suffisante ou en engagement mécanique avec le corps de la personne (1), ou un autre engagement mécanique avec le corps de la personne (1) pour déterminer une ou plusieurs mesures de santé (2d) de la personne (1), lorsque le dispositif informatique et l'application (2a, 2-3, 2-4, 2-5) comprennent ou communiquent avec un appareil photo pour capturer des images, au moins une donnée biométrique de la personne (1) préenregistrée auprès du dispositif informatique et de l'application (2a, 2-3, 2-4, 2-5) comprend une image faciale, à un moment donné pendant ou autrement au cours d'une durée concomitante de la (fonction a) et de la (fonction b1), le dispositif informatique et l'application (2a, 2-3, 2-4, 2-5) (i) reçoivent du dispositif d'engagement temporaire de schéma corporel temporel (4-4) l'échantillon de schéma corporel temporel (4-4-d2), (ii) capturent deux images ou plus comprenant substantiellement le visage de la personne (1), (iii) traitent les deux images ou plus pour (iii-a) comparer le visage capturé de la personne
(1) avec l'image faciale préenregistrée, (iii-b) déterminer un schéma corporel temporel du dispositif informatique (2a-d2) comprenant une séquence de mesure des battements cardiaques de la personne, et (iii-c) comparer le schéma corporel temporel du dispositif informatique (2a-d2) au schéma corporel temporel de l'échantillon (4-4-d2), après quoi le dispositif informatique et l'application (2a, 2-3, 2-4, 2-5) exécutent (fonction c1) et reçoivent ou acceptent d'une autre manière l'une ou plusieurs des mesures de santé (2d) provenant du dispositif d'engagement temporaire à schéma corporel temporel (4-4) comme étant authentifiées de la personne (1), et (iii-c) comparer le schéma corporel temporel du dispositif informatique (2a-d2) au schéma corporel temporel de l'échantillon (4-4-d2).
un dispositif de bio-mesure de passage (3-2) sous le contrôle du système de contrôle d'accès (74) pour fournir des mesures de santé , où le dispositif d'engagement temporaire est un dispositif de bio-mesure de passage (3-2) exploité par ou autrement en communication avec le système de contrôle d'accès (74) pour détecter et communiquer avec le dispositif informatique et l'application (2a, 2-3, 2-4, 2-5) utilisé par la personne (1) alors que la personne (1) est sensiblement isolée et alignée avec le dispositif de bio- mesure de déambulation à engagement temporaire (3-2) de manière à garantir suffisamment que toutes les mesures de santé (2d) déterminées ultérieurement par le dispositif de bio-mesure de déambulation à engagement temporaire (3-2) et fournies au dispositif informatique et à l'application (2a, 2-3, 2-4, 2-5) sont celles de la personne (1), où à un moment donné pendant ou autrement au cours d'une certaine durée simultanée de la (fonction a) et de la (fonction b1), la personne (1) qui porte, arbore ou a autrement le dispositif informatique et l'appli (2a, 2-3, 2-4, 2-5) entre dans l'alignement physique isolé avec le dispositif de bio-mesure de déambulation à engagement temporaire (3-2) et des communications à distance sont établies entre le dispositif de bio-mesure de déambulation à engagement temporaire (3-2) et le dispositif informatique et l'appli (2a, 2-3, 2-4, 2-5), après quoi la personne isolée et alignée (1) utilise le dispositif informatique et l'application (2a, 2-3, 2-4, 2-5) pour fournir en privé au dispositif informatique et à l'application (2a, 2-3, 2-4, 2-5) au moins une biométrie de confirmation à comparer avec une ou plusieurs biométries préenregistrées, après quoi une correspondance suffisante du dispositif informatique et de l'application (2a, 2-3, 2-4, 2-5) fournissent une indication anonyme de l'authenticité de la personne
(1) au dispositif de bio-mesure de l'engagement temporaire (3-2) et, sur réception de l'indication anonyme, le dispositif de bio-mesure de l'engagement temporaire (3-2) détermine une ou plusieurs des mesures de santé (2d) de la personne (1) suffisamment isolée et positionnée pour les fournir au dispositif informatique et à l'application (2a, 2-3, 2-4, 2-5), et où le dispositif informatique et l'application (2a, 2-3, 2-4, 2-5) exécutent (fonction c1) et reçoivent ou acceptent d'une autre manière la ou les mesures de santé (2d) du dispositif de bio-mesure de l'engagement temporaire (3-2) comme étant authentifiées de la personne (1).

4. Le système de la revendication 1 dans lequel l'au moins un dispositif de mesure de la santé (3-1, 3-1-d1, 3- 1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131)
est un dispositif portable à engagement soutenu (3-1-d4, 3-1-d5, 4-1a, 4-1b, 4-3, 131), et où, lors de l'utilisation du dispositif portable à engagement soutenu (3-1-d4, 3-1-d5, 4-1a, 4-1b, 4-3, 131), l'exécution
de la (fonction 1) comprenant la combinaison de la (fonction a), de la (fonction b1) et de la (fonction c1) comprend l'un des éléments suivants:
reconnaissance du visage de la personne (1) en même temps que la confirmation de la signalisation lumineuse et la confirmation de l'état sécurisé du vêtement, le vêtement à engagement prolongé étant un vêtement à engagement prolongé à signalisation lumineuse (3-1-d4, 3-1-d5) comprenant un émetteur de lumière, un ou plusieurs capteurs pour déterminer une ou plusieurs mesures de santé (2d) de la personne (1), et des moyens pour déterminer l'une des informations d'état sécurisé à utiliser au moins en partie pour déterminer que le vêtement à engagement prolongé à signalisation lumineuse (3-1-d4, 3-1-d5) est attaché, verrouillé, verrouillé ou autrement fixé au corps de la personne (1), où le dispositif informatique et l'application (2a, 2-3, 2-4, 2-5) comprennent ou communiquent autrement avec une caméra pour capturer des images, où au moins une donnée biométrique de la personne (1) préenregistrée avec le dispositif informatique et l'application (2a, 2-3, 2-4, 2-5) comprend une image faciale, où à une certaine durée simultanée de la (fonction a) et de la (fonction b1), le dispositif informatique et l'application (2a, 2-3, 2-4, 2-5) (i) fournissent un signal de commande au dispositif portable à activation prolongée par signal lumineux (3-1-d4, 3-1-d5), (ii) capturent deux images ou plus comprenant essentiellement une vue simultanée du visage de la personne (1) et du dispositif portable à activation prolongée par signal lumineux (3-1-d4, 3-1-d5) situé essentiellement en contact ou à proximité suffisante du corps de la personne (1) pour s'assurer que le dispositif portable à activation prolongée par signal lumineux (3-1-d4, 3-1-d5) est apposé, fixé ou autrement porté sur le corps de la personne (1), et (iii) reçoit du dispositif portable (3-1-d4, 3-1-d5) à signalisation lumineuse à engagement soutenu les informations relatives à l'état sécurisé, le dispositif portable (3-1-d4, 3-1-d5) à signalisation lumineuse à engagement soutenu se trouvant dans le champ de vision simultané de la caméra et utilisant au moins en partie le signal de commande pour faire en sorte que l'émetteur de lumière émette un signal de réponse (3-sig) comprenant de la lumière détectable par la caméra, le dispositif informatique et l'application (2a, 2-3, 2-4, 2-5) (iv) traitent les deux images ou plus pour (iv-a) comparer le visage capturé de la personne (1) avec l'image faciale préenregistrée, (iv-b) comparer le signal de réponse émis (3-sig) avec le signal de commande fourni, et (iv-c) confirmer le contact substantiel ou une autre proximité suffisante entre le vêtement à signaux lumineux à engagement prolongé (3-1-d4, 3-1-d5) et le corps de la personne (1) pour s'assurer que le vêtement à signaux lumineux à engagement prolongé (3-1-d4, 3-1-d5) est fixé, sécurisé ou autrement porté sur le corps de la personne (1), à la suite de correspondances et de confirmations suffisantes et en combinaison avec la détermination par le dispositif informatique et l'application (2a, 2-3, 2-4, 2-5) que le dispositif portable (3-1-d4, 3-1-d5) à signalisation lumineuse et à engagement prolongé est fixé, verrouillé, verrouillé ou autrement sécurisé sur le corps de la personne (1) en utilisant au moins en partie les informations relatives à l'état sécurisé, le dispositif informatique et l'application (2a, 2-3, 2-4, 2-5) déterminent que le dispositif portable (3-1-d4, 3-1-d5) à signalisation lumineuse à engagement prolongé entre dans une durée de temps sécurisé, la durée de temps sécurisé continuant jusqu'à un
moment ultérieur où des informations d'état sécurisé ultérieures sont reçues par le dispositif informatique et l'application (2a, 2-3, 2-4, 2-5) en provenance du dispositif portable (3-1-d4, 3-1-d5) à signalisation lumineuse à engagement prolongé et utilisées au moins en partie par le dispositif informatique et l'application (2a, 2-3, 2-4, 2-5) pour déterminer que le dispositif portable (3-1-d4, 3-1-d5) à signalisation lumineuse et à engagement prolongé n'est plus suffisamment fixé au corps de la personne
(1) à la fin de la période sécurisée, et où le dispositif informatique et l'application (2a, 2-3, 2-4, 2-5) exécutent (fonction c1) et reçoivent et acceptent une ou plusieurs mesures de santé (2d) déterminées pendant la période sécurisée à partir du dispositif portable (3-1-d4, 3-1-d5) à signalisation lumineuse et à engagement prolongé, comme étant authentifiées par la personne (1);
l'utilisation d'un dispositif compagnon (3-1-d9) pour fournir un schéma corporel spatial compagnon ainsi qu'un schéma corporel temporel compagnon concomitant et colocalisé, le dispositif compagnon (3-1-d9) comprenant des moyens pour déterminer simultanément, à partir d'un emplacement corporel spatial spécifique sensiblement identique de la personne (1), à la fois le schéma corporel spatial compagnon (3-1- d9-d1) et le schéma corporel temporel compagnon (3-1-d9-d2), le même emplacement corporel spatial spécifique comprenant un visage, un œil, un doigt, etc, ou tout autre emplacement identifiable sur le corps de la personne (1), où un schéma corporel spatial (3-1-d9-d1) comprend un schéma d'identification unique du corps de la personne (1) déterminé au même emplacement corporel spatial spécifique, où le schéma d'identification unique comprend une image faciale, un balayage rétinien, une empreinte digitale, ou tout autre schéma corporel spatial identifiable, où le schéma corporel temporel compagnon comprend une séquence de mesure des battements cardiaques de la personne (1), lorsque le dispositif portable d'engagement prolongé est un dispositif portable d'engagement prolongé à schéma corporel temporel (3- 1-d4, 3-1-d5, 4-1a, 4-1b, 4-3) comprenant des moyens pour déterminer un schéma corporel temporel portable comprenant une séquence de mesure des battements cardiaques de la personne (1) prise à partir d'un emplacement corporel différent du même emplacement corporel spatial spécifique de la personne (1), un ou plusieurs capteurs pour déterminer une ou plusieurs des mesures de santé (2d) de la personne (1), et des moyens pour déterminer toute information d'état sécurisée à utiliser au moins en partie pour déterminer que le dispositif portable d'engagement soutenu par le schéma corporel temporel (3-1-d4, 3-1-d5, 4-1a, 4-1b, 4-3) est fixé, verrouillé, verrouillé ou autrement sécurisé sur le corps de la personne (1), où au moins une donnée biométrique de la personne (1) préenregistrée avec le dispositif informatique et l'application (2a, 2-3, 2-4, 2-5) comprend un schéma corporel spatial de la personne (1) pris essentiellement à partir du même emplacement corporel spatial spécifique, où à une certaine durée simultanée de la (fonction a) et de la (fonction b1), le dispositif informatique et l'application (2a, 2-3, 2-4, 2-5) (i) reçoivent du dispositif compagnon (3-1-d9) le schéma corporel spatial du compagnon (3-1-d9-d1),
(ii) reçoivent du dispositif compagnon (3-1-d9) le schéma corporel temporel du compagnon (3-1-d9-d2),
(iii) reçoivent du dispositif portable à engagement soutenu du schéma corporel temporel (3-1-d4, 3-1-d5, 4-1a, 4-1b, 4-3) le schéma corporel temporel portable, (iv) reçoit du schéma corporel temporel portable à
engagement soutenu (3-1-d4, 3-1-d5, 4-1a, 4-1b, 4-3) les informations sur l'état sécurisé, (v) compare le schéma corporel spatial compagnon (3-1-d9-d1) au schéma corporel spatial préenregistré, (vi) compare le schéma corporel spatial compagnon (3-1-d9-d2) au schéma corporel spatial préenregistré, (vii) compare le schéma corporel spatial compagnon (3-1-d9-d2) au schéma corporel spatial préenregistré, et (vi) compare le schéma corporel temporel du compagnon (3-1-d9-d2) au schéma corporel temporel du vêtement, après quoi des correspondances suffisantes et en combinaison avec la détermination par le dispositif informatique et l'application (2a, 2-3, 2-4, 2-5) que le motif corporel temporel du vêtement à engagement soutenu (3-1-d4, 3-1-d5, 4-1a, 4-1b, 4-3) est fixé, verrouillé, verrouillé ou autrement fixé au corps de la personne (1) en utilisant au moins en partie les informations sur l'état sécurisé, le dispositif informatique et l'application (2a, 2-3, 2-4, 2-5) déterminent que le dispositif vestimentaire (3-1-d4, 3-1-d5, 4-1a, 4-1b,
4-3) à engagement soutenu selon un schéma temporel-corporel entre dans une durée de temps sécurisé, où la durée de temps sécurisé se poursuit jusqu'à un moment ultérieur où des informations d'état sécurisé ultérieures sont reçues par le dispositif informatique et l'application (2a, 2-3, 2-4, 2-5) en provenance du dispositif portable d'engagement soutenu du schéma corporel temporel (3-1-d4, 3-1-d5, 4- 1a, 4-1b, 4-3) et utilisées au moins en partie par le dispositif informatique et l'application (2a, 2-3, 2-4, 2- 5) pour déterminer que le dispositif portable d'engagement soutenu (3-1-d4, 3-1-d5, 4-1a, 4-1b, 4-3) n'est plus suffisamment fixé au corps de la personne (1) à la fin de la durée sécurisée, et où le dispositif informatique et l'application (2a, 2-3, 2-4, 2-5) exécutent (fonction c1) et reçoivent et acceptent une ou plusieurs mesures de santé (2d) déterminées pendant la durée du temps sécurisé à partir du dispositif portable à engagement soutenu (3-1-d4, 3-1-d5, 4-1a, 4-1b, 4-3) comme étant authentifiées de la personne (1), et que
reconnaissance du visage de la personne (1) en même temps que la correspondance du schéma temporel et la confirmation de l'état sécurisé du dispositif portable, le dispositif portable à engagement prolongé étant un dispositif portable à engagement prolongé avec schéma corporel temporel (3-1-d4, 3-1-d5, 4-1a, 4-1b, 4-3) comprenant des moyens pour déterminer un schéma corporel temporel portable comprenant une séquence de mesure des battements cardiaques de la personne (1), un ou plusieurs capteurs pour déterminer une ou plusieurs mesures de la santé (2d) de la personne (1), et des moyens pour déterminer l'une des informations d'état sécurisées à utiliser au moins en partie pour déterminer que le dispositif vestimentaire (3-1-d4, 3-1-d5, 4-1a, 4-1b, 4-3) est attaché, verrouillé, fixé ou autrement fixé au corps de la personne (1), lorsque le dispositif informatique et l'application (2a, 2-3, 2-4, 2-5) comprennent ou communiquent autrement avec un appareil photo pour la capture d'images, où au moins une donnée biométrique de la personne (1) préenregistrée auprès du dispositif informatique et de l'application (2a, 2- 3, 2-4, 2-5) comprend une image faciale, où à un moment donné pendant ou autrement au cours d'une certaine durée concomitante de la (fonction a) et de la (fonction b1), le dispositif informatique et l'application (2a, 2-3, 2-4, 2-5) (i) reçoivent du dispositif portable (3-1-d4, 3-1-d5, 4-1a, 4-1b.) à engagement soutenu ( ) les informations relatives à l'état sécurisé, où le dispositif informatique et
l'application (2a, 2-3, 2-4, 2-5) (i) reçoivent du dispositif portable (3-1-d4, 3-1-d5, 4-1a, 4-1b, 4-3) les informations relatives à l'état sécurisé, (ii) reçoit de la part du dispositif portable (3-1-d4, 3-1-d5, 4-1a, 4- 1b, 4-3) le schéma corporel temporel d'engagement soutenu, (iii) capture deux images ou plus comprenant essentiellement le visage de la personne (1), (iv) traite les deux images ou plus pour (iv-a) comparer le visage capturé de la personne (1) à l'image faciale préenregistrée, (iv-b) déterminer un schéma corporel temporel du dispositif informatique (2a-d2) comprenant une séquence de mesure des battements de cœur de la personne (1), et (iv-c) comparer le schéma corporel temporel du dispositif informatique (2a-d2) au schéma corporel temporel portable, après quoi des correspondances suffisantes et en combinaison avec la détermination par le dispositif informatique et l'application (2a, 2-3, 2-4, 2-5) que le motif corporel temporel du vêtement à engagement soutenu (3-1-d4, 3-1-d5, 4-1a, 4-1b, 4-3) est fixé, verrouillé, verrouillé ou autrement fixé au corps de la personne (1) à l'aide, au moins en partie, des informations sur l'état sécurisé, le dispositif informatique et l'application (2a, 2-3, 2-4, 2-5) déterminent que le dispositif vestimentaire à engagement soutenu (3-1-d4, 3-1-d5, 4-1a, 4-1b, 4-3) entre dans une durée de temps sécurisé, où la durée du temps sécurisé se poursuit jusqu'à un moment ultérieur où des informations d'état sécurisé ultérieures sont reçues par le dispositif informatique et l'application (2a, 2-3, 2-4, 2-5) du dispositif portable à engagement soutenu par un schéma corporel temporel (3-1-d4, 3-1-d5, 4-1a, 4-1b, 4-3) et utilisées au moins en partie par le dispositif informatique et l'application (2a, 2-3, 2-4, 2-5) pour déterminer que le dispositif portable à engagement soutenu par un schéma corporel temporel (3-1-d4, 3-1-d5, 4-1a, 4-1b, 4-3) n'est plus suffisamment fixé au corps de la personne (1) à la fin de la période sécurisée, et où le dispositif informatique et l'application (2a, 2-3, 2-4, 2-5) exécutent (fonction c1) et reçoivent et acceptent une ou plusieurs mesures de santé (2d) déterminées pendant la période sécurisée à partir du dispositif portable à engagement soutenu dans le temps (3-1-d4, 3-1-d5, 4-1a, 4-1b, 4-3) comme étant authentifiées par la personne (1).

5. Le système de la revendication 1 dans lequel l'au moins un dispositif de mesure de la santé (3-1, 3-1-d1, 3- 1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131)
est un dispositif de collecte d'échantillons biométriques (4-2, 80, 84), et où, lors de l'utilisation d'un dispositif de collecte d'échantillons biométriques (4-2, 80, 84), l'exécution de la (fonction 1) comprenant la combinaison de la (fonction a), de la (fonction b1) et de la (fonction c1) comprend l'un des éléments suivants:
reconnaissance du visage de la personne (1) en même temps que le balayage des marques visuelles (80- bcd, 84-bcd) pour déterminer l'identifiant d'un dispositif de collecte d'échantillons, le dispositif biométrique de collecte d'échantillons (80, 84) collectant, recevant, prélevant ou obtenant d'une autre manière un fluide corporel ou un autre échantillon de la personne (1) à l'aide d'un réceptacle d'échantillons (80-tip, 84-smp), où un fluide corporel ou un autre échantillon comprend du mucus, de la salive ou du sang, où le dispositif biométrique de collecte d'échantillons (80, 84) comprend des marques
visuelles (80-bcd, 84-bcd) à utiliser au moins en partie pour déterminer l'identifiant du dispositif de collecte d'échantillons, où le dispositif informatique et l'application (2a, 2-3, 2-4, 2-5) comprennent ou communiquent d'une autre manière avec un appareil photo pour capturer des images, où au moins un élément biométrique de la personne (1) préenregistrée avec le dispositif informatique et l'application (2a, 2-3, 2-4, 2-5) comprend une image faciale , où à un moment donné pendant ou autrement sur une certaine durée concomitante de la (fonction a) et de la (fonction b1), le dispositif informatique et l'application (2a, 2-3, 2-4, 2-5) capturent deux images ou plus comprenant essentiellement une vue simultanée du visage de la personne (1), du dispositif de collecte d'échantillons biométriques (80, 84) et des marques visuelles (80-bcd, 84-bcd), et la personne (1) utilise le dispositif de collecte d'échantillons biométriques (80, 84) pour collecter, recevoir, prélever ou obtenir de toute autre manière un échantillon de fluide corporel ou autre de la personne (1), lorsque le dispositif informatique et l'application (2a, 2-3,
2-4, 2-5) (i) traitent les deux images ou plus pour (i-a) déterminer l'identifiant du dispositif de collecte d'échantillons sur la base, au moins en partie, des marquages visuels (80-bcd, 84-bcd), (i-b) comparer le visage capturé de la personne (1) avec l'image faciale préenregistrée, et (i-c) confirmer le contact substantiel ou la proximité suffisante entre le réceptacle d'échantillon (80-tip, 84-smp) et le corps de la personne (1) pour s'assurer que chacun des éléments biométriques collectés, prélevés ou obtenus d'une autre manière, prélevés ou obtenus d'une autre manière sont ceux de la personne (1), et après une correspondance et une confirmation suffisantes, le dispositif informatique et l'application (2a, 2-3, 2-4, 2- 5) exécutent (fonction c1) et déterminent une association entre l'identifiant du dispositif de collecte d'échantillons et la personne (1) pour l'utiliser au moins en partie pour l'associer à une évaluation future des échantillons collectés, prélevés ou obtenus d'une autre manière, l'échantillon biométrique collecté, prélevé ou obtenu d'une autre manière, de sorte que l'évaluation future est authentifiée comme étant celle de la personne (1), l'évaluation future comprenant une ou plusieurs des mesures de santé authentifiées (2d);
reconnaissance du visage de la personne (1) en même temps que le balayage d'une étiquette électronique (80-nfc, 84-nfc) pour déterminer l'identifiant d'un dispositif de collecte d'échantillons, lorsque le dispositif biométrique de collecte d'échantillons (80, 84) recueille, reçoit, prélève ou obtient d'une autre manière un fluide corporel ou un autre échantillon de la personne (1) à l'aide d'un réceptacle d'échantillons (80-tip, 84-smp), où un fluide corporel ou un autre échantillon comprend du mucus, de la salive ou du sang, où le dispositif biométrique de collecte d'échantillons (80, 84) comprend une étiquette électronique (80-nfc, 84-nfc) pour fournir l'identifiant du dispositif de collecte d'échantillons, où le dispositif informatique et l'application (2a, 2-3, 2-4, 2-5) comprennent ou communiquent d'une autre manière avec un appareil photo pour capturer des images et comprennent des moyens de lecture d'étiquette électronique pour scanner électroniquement l'étiquette électronique (80-nfc, 84-nfc) afin de déterminer l'identifiant du dispositif de collecte d'échantillons, où au moins un élément biométrique de la personne (1) préenregistrée avec le dispositif informatique et l'application (2a, 2-3, 2-4, 2-5) comprend
une image faciale, où à un moment donné pendant ou autrement sur une certaine durée simultanée de la (fonction a) et de la (fonction b1) le dispositif informatique et l'application (2a, 2-3, 2-4, 2-5) capturent deux images ou plus comprenant essentiellement une vue simultanée du visage de la personne (1), le dispositif de collecte d'échantillons biométriques (80, 84) et la personne (1) à l'aide du dispositif de collecte d'échantillons biométriques (80, 84), et la personne (1) utilisant le dispositif biométrique de collecte d'échantillons (80, 84) pour collecter, recevoir, prélever ou obtenir d'une autre manière un fluide corporel ou un autre échantillon de la personne (1), lorsque le dispositif informatique et l'application (2a, 2-3, 2-4, 2-5) (i) utilisent les moyens de lecture de l'étiquette électronique du dispositif informatique pour déterminer l'identifiant du dispositif de collecte d'échantillons en scannant électroniquement l'étiquette électronique (80-nfc, 84-nfc), (ii) traite les deux images ou plus pour (ii-a) comparer le visage capturé de la personne (1) avec l'image faciale préenregistrée, et (ii-b) confirmer le contact substantiel ou une autre proximité suffisante entre le réceptacle de l'échantillon (80-tip, 84-smp) et le corps de la personne (1) pour s'assurer que chacun des échantillons biométriques collectés, prélevés ou obtenus d'une autre manière est celui de la personne (1), après quoi le dispositif informatique et l'application (2a, 2-3, 2-4, 2-5) exécutent (fonction c1) et déterminent une association entre l'identifiant du dispositif de collecte d'échantillons et la personne (1) en vue d'une utilisation au moins partielle pour associer à une évaluation future de l'échantillon ou des échantillons biométriques collectés, prélevés ou obtenus d'une autre manière, de sorte que l'évaluation future est authentifiée comme étant celle de la personne (1), l'évaluation future comprenant une ou plusieurs des mesures de santé authentifiées (2d);
reconnaissance du visage de la personne (1) en même temps que l'utilisation d'un dispositif compagnon (3-1-d9, 3-1-d10) pour fournir un signal lumineux compagnon avec balayage des marques visuelles (80- bcd, 84-bcd) ou d'une étiquette électronique (80-nfc, 84-nfc) pour déterminer l'identification d'un dispositif de collecte d'échantillons, où le dispositif compagnon (3-1-d9, 3-1-d10) comprend un émetteur de lumière (3-1-d9-led, 3-1-d10-led) pour émettre le signal lumineux compagnon et comprend éventuellement des moyens de lecture d'étiquette électronique (3-1-d10-nfc) pour scanner électroniquement l'étiquette électronique (80-nfc, 84-nfc) pour déterminer l'identifiant du dispositif de collecte d'échantillons, lorsque le dispositif biométrique de collecte d'échantillons (80, 84) collecte, reçoit, prend ou obtient d'une autre manière un fluide corporel ou un autre échantillon de la personne (1) à l'aide d'un réceptacle d'échantillons (80-tip, 84-smp), lorsqu'un fluide corporel ou un autre échantillon comprend du mucus, de la salive ou du sang, lorsque le dispositif biométrique de collecte d'échantillons (80, 84) comprend, ou les deux, des marques visuelles (80-bcd, 84-bcd) à utiliser au moins en partie pour déterminer l'identifiant du dispositif de collecte d'échantillons et comprend une étiquette électronique (80-nfc, 84-nfc) pour fournir l'identifiant du dispositif de collecte d'échantillons, lorsque le dispositif informatique et l'application (2a, 2-3, 2-4, 2-5) comprennent ou communiquent d'une autre manière avec une caméra pour capturer des images et comprennent des moyens de lecture d'étiquette électronique pour scanner électroniquement l'étiquette électronique (80-nfc, 84-nfc) afin de déterminer l'identifiant
du dispositif de collecte d'échantillons, lorsqu'au moins une donnée biométrique de la personne (1) préenregistrée avec le dispositif informatique et l'application (2a, 2-3, 2-4, 2-5) comprend une image faciale, le dispositif informatique et l'application (2a, 2-3, 2-4, 2-5) fournissent un signal de commande au dispositif informatique et à l'application (2a, 2-3, 2-4, 2-5) à un moment donné pendant la durée de la (fonction a) et de la (fonction b1), ou pendant une durée simultanée de ces deux fonctions, 2-5) fournissent un signal de commande au dispositif compagnon (3-1-d9) tout en capturant simultanément deux images ou plus comprenant essentiellement une vue simultanée du visage de la personne (1), du dispositif compagnon (3-1-d9, 3-1-d10), du dispositif de collecte d'échantillons biométriques (80, 84), de l'une quelconque des marques visuelles (80-bcd, 84-bcd) présents sur le dispositif de prélèvement d'échantillons biométriques (80, 84), et la personne (1) utilisant le dispositif de prélèvement d'échantillons biométriques (80, 84) pour collecter, recevoir, prélever ou obtenir de toute autre manière un échantillon de fluide corporel ou autre de la personne (1), le dispositif compagnon (3-1-d9, 3-1-d10), lorsqu'il se trouve dans le champ de vision simultané de la caméra et qu'il utilise au moins en partie le signal de commande, fait en sorte que l'émetteur de lumière (3-1-d9-led, 3-1-d10-led) émette un signal de réponse comprenant de la lumière pouvant être détectée par la caméra, lorsque le dispositif informatique et l'application (2a, 2-3, 2-4, 2-5) déterminent l'identifiant du dispositif de collecte d'échantillons par l'une ou l'autre combinaison de l'utilisation des moyens de lecture de l'étiquette électronique du dispositif informatique pour scanner électroniquement l'étiquette électronique (80-nfc, 84-nfc) et du traitement des deux images ou plus pour déterminer l'identifiant du dispositif de collecte d'échantillons sur la base, au moins en partie, des marques visuelles (80-bcd, 84-bcd), et lorsque le dispositif informatique et l'application (2a, 2-3, 2-4, 2-5) (i) traitent les deux images ou plus pour (i-a) comparer le visage capturé de la personne (1) avec l'image faciale préenregistrée, (i-b) comparer le signal de réponse émis avec le signal de contrôle fourni, et (i-c) confirmer le contact substantiel ou la proximité suffisante entre le réceptacle d'échantillon (80-tip, 84-smp) et le corps de la personne (1) pour s'assurer que chacun des échantillons biométriques collectés, prélevés ou obtenus d'une autre manière est celui de la personne (1), et à la suite de correspondances et de confirmations suffisantes, le dispositif informatique et l'application (2a, 2-3, 2- 4, 2-5) exécutent (fonction c1) et déterminent une association entre l'identifiant du dispositif de collecte d'échantillons et la personne (1) afin de l'utiliser, au moins en partie, pour l'associer à une évaluation future de l'échantillon ou des échantillons biométriques collectés, prélevés ou obtenus d'une autre manière, de sorte que l'évaluation future est authentifiée comme étant celle de la personne (1), l'évaluation future comprenant une ou plusieurs des mesures de santé authentifiées (2d), et
la reconnaissance du visage de la personne (1) et l'utilisation d'un dispositif compagnon (3-1-d10) pour fournir un signal lumineux compagnon et pour scanner une étiquette électronique afin de déterminer l'identifiant du dispositif de collecte d'échantillons, le dispositif compagnon (3-1-d10) comprenant un émetteur de lumière (3-1-d10-led) et un lecteur d'étiquette électronique (3-1-d10-nfc) pour scanner électroniquement une étiquette électronique intégrée ou appliquée (4-tag) afin de déterminer
l'identifiant du dispositif de collecte d'échantillons, le dispositif de collecte d'échantillons biométriques est un dispositif de collecte d'échantillons d'analyseur d'haleine (4-2) comprenant une étiquette électronique intégrée ou appliquée (4-tag) pour fournir l'identifiant du dispositif de collecte d'échantillons et comprenant des moyens pour recevoir et traiter l'haleine expulsée de la personne (1) pour l'utiliser dans une analyse de l'haleine résultant en une ou plusieurs mesures de santé (2d), le dispositif informatique et l'application (2a, 2-3, 2-4, 2-5) comprennent ou communiquent avec un appareil photo pour capturer des images, et au moins un élément biométrique de la personne (1) préenregistré avec le dispositif informatique et l'application (2a, 2-3, 2-4, 2-5) comprend une image faciale, la personne (1) utilise simultanément le dispositif de collecte d'échantillons de l'analyseur d'haleine (4-2) pour recevoir l'haleine de la personne (1) et effectuer l'analyse de l'haleine de la personne (1) et utilise le lecteur d'étiquettes électroniques de l'appareil compagnon (3-1-d10-nfc) pour déterminer l'identifiant du dispositif de collecte d'échantillons à fournir à l'appareil informatique et à l'application (2a, 2-3, 2-4, 2-5), où au moins une donnée biométrique de la personne (1) préenregistrée auprès de l'appareil informatique et de l'application (2a, 2-3, 2-4, 2-5) comprend une image faciale 2-3, 2-4, 2-5) tandis que le dispositif informatique et l'application (2a, 2-3, 2-4, 2-5) fournissent un signal de commande au dispositif compagnon (3-1-d10) et capturent simultanément deux images ou plus comprenant essentiellement une vue simultanée du visage de la personne (1), du dispositif compagnon (3-1-d10), du dispositif de collecte d'échantillons de l'analyseur d'haleine (4-2) et de la personne (1) à l'aide du dispositif de collecte d'échantillons de l'analyseur d'haleine (3-1-d10), et la personne (1) utilisant le dispositif de collecte d'échantillons de l'analyseur d'haleine (4-2) pour recevoir l'haleine de la personne (1), où le dispositif compagnon (3-1-d10), tout en étant dans la vue simultanée de la caméra et en utilisant au moins en partie le signal de commande, fait en sorte que l'émetteur de lumière (3-1-d10-led) émette un signal de réponse comprenant de la lumière détectable par la caméra, où le dispositif informatique et l'application (2a, 2-3, 2-4, 2-5) (i) traitent les deux images ou plus pour (i-a) comparer le visage capturé de la personne
(1) avec l'image faciale préenregistrée, (i-b) comparer le signal de réponse émis avec le signal de commande fourni, et (i-c) confirmer le contact substantiel ou une autre proximité suffisante entre le dispositif d'engagement temporaire de l'analyseur d'haleine (4-2) et la bouche de la personne (1) pour s'assurer que l'analyse de l'haleine comprenant une ou plusieurs mesures de santé (2d) est celle de la personne (1), et , à la suite de correspondances et de confirmations suffisantes, le dispositif informatique et l'application (2a, 2-3, 2-4, 2-5) exécutent (fonction c1) et reçoivent ou acceptent d'une autre manière l'une ou plusieurs des mesures de santé (2d) provenant du dispositif de collecte d'échantillons de l'analyseur d'haleine (4-2) comme étant authentifiées par la personne (1).

6. Le système de la revendication 5 pour déterminer une ou plusieurs mesures de santé authentifiées (2d) basées au moins en partie sur l'évaluation d'un ou plusieurs échantillons biométriques authentifiés, comprend en outre l'un des éléments suivants:
un laboratoire d'essai de services de santé qui fournit à la personne (1) un kit d'essai de santé (90-1) comprenant le dispositif de collecte d'échantillons biométriques (80, 84) et un contenant scellable de dispositif de collecte d'échantillons biométriques (82), où les deux images ou plus capturées par le dispositif informatique et l'application (2a, 2-3, 2-4, 2-5) à un moment donné pendant ou autrement sur une durée concomitante de la (fonction a) et de la (fonction b1) comprenant l'utilisation par la personne
(1) du dispositif de prélèvement d'échantillons biométriques (80, 84) pour collecter, recevoir, prendre ou obtenir d'une autre manière un fluide corporel authentifié ou un autre échantillon de la personne (1) comprennent en outre ou autrement en plus et ultérieurement deux images ou plus de la personne (1) plaçant au moins le réceptacle d'échantillon (80-tip, 84-smp) ou la partie du dispositif de collecte d'échantillons biométriques (80, 84) comprenant l'échantillon biométrique dans le récipient scellable du dispositif de collecte d'échantillons biométriques (82) pour s'assurer que le récipient scellable du dispositif de collecte d'échantillons biométriques (82) est authentifié comme contenant l'échantillon biométrique authentifié de la personne (1), lorsque le dispositif informatique et l'application (2a, 2-3, 2-4, 2-5), tout en traitant les deux images ou plus, confirment en outre qu'au moins la partie du dispositif de collecte d'échantillons biométriques (80, 84) comprenant le réceptacle d'échantillon (80-tip, 84-smp) ou l'échantillon biométrique a été placée dans le conteneur du dispositif de collecte d'échantillons biométriques (82) et que le conteneur (82) a ensuite été scellé, lorsqu'après avoir scellé le récipient (82), la personne (1) utilise l'un des services d'expédition disponibles ou d'autres méthodes de livraison pour fournir le récipient scellé (82) contenant au moins le récipient de l'échantillon biométrique (80-tip, 84- smp) ou l'échantillon biométrique au laboratoire d'essai du service de santé, lorsque la fourniture du récipient de l'échantillon biométrique (80-tip, 84-smp) ou l'échantillon biométrique au laboratoire d'essai des services de santé est anonyme et ne comprend aucune information d'identification personnelle, lorsque le laboratoire d'essai des services de santé détermine l'évaluation comprenant une ou plusieurs mesures de santé (2d) en se fondant au moins en partie sur le récipient d'échantillon authentifié fourni (80-tip, 84-smp) ou de l'échantillon biométrique fourni et stocke les informations numériques indiquant la ou les mesures de santé (2d) en association avec l'identifiant du dispositif de collecte de l'échantillon dans une base de données d'évaluation accessible par le dispositif informatique et l'application (2a, 2-3, 2-4, 2- 5), et où, à un moment donné après la fourniture du récipient scellé (82) au laboratoire d'essais des services de santé, la personne (1) utilise le dispositif informatique et l'application (2a, 2-3, 2-4, 2-5) pour exécuter (la fonction c1) en accédant à la base de données d'évaluation pour récupérer une ou plusieurs mesures de santé (2d) en fournissant au moins en partie l'identifiant du dispositif de collecte d'échantillons à la base de données d'évaluation, puis en recevant et en acceptant une ou plusieurs mesures de santé (2d) déterminées par l'évaluation du laboratoire d'essais sanitaires comme étant authentifiées de la personne (1), et que
un laboratoire de tests de santé qui fournit à la personne (1) un kit de tests de santé comprenant le dispositif de collecte d'échantillons biométriques (80, 84) comprenant en outre un milieu réactif pour
fournir une évaluation immédiate, où le réceptacle d'échantillon (80-tip, 84-smp) comprend en outre ou peut être placé en contact avec le milieu réactif de l'échantillon fourni dans le kit de tests de santé, le milieu réactif de l'échantillon change d'aspect visuel au moins en partie lorsqu'il entre suffisamment en contact avec le fluide corporel ou l'échantillon biométrique de la personne (1), les changements d'aspect visuel indiquant une ou plusieurs mesures de santé (2d), la personne (1) utilise le dispositif informatique et l'application (2a, 2-3, 2-4, 2-5) pour exécuter (la fonction c1) en capturant au moins une image de l'échantillon de milieu réactif après l'apparition de tout changement dans l'apparence visuelle de l'échantillon de milieu réactif et en traitant ensuite l'image au moins pour déterminer l'une ou plusieurs des mesures de santé (2d) basées au moins en partie sur l'apparence visuelle de l'échantillon de milieu réactif, le dispositif informatique et l'application (2a, 2-3, 2-4, 2-5) acceptent la ou les mesures de santé (2d) comme étant authentifiées par la personne (1).

7. Le système de la revendication 1 dans lequel l'au moins un dispositif de mesure de la santé (3-1, 3-1-d1, 3- 1-d2, 3-1-d4, 3-1-d5, 3-2, 3-3, 3-1-d6, 3-1-d7, 3-1-d8, 3-1-d9, 3-1-d10, 4-1a, 4-1b, 4-2, 4-3, 4-4, 80, 84, 131)
est l'un quelconque des dispositifs d'engagement temporaire (3-1-d1, 3-1-d2, 3-2, 3-1-d6, 3-1-d7, 3-1-d8, 3-1-d9, 4-2, 4-4), un dispositif portable à engagement soutenu (3-1-d4, 3-1-d5, 4-1a, 4-1b, 4-3, 131), ou un dispositif de collecte d'échantillons biométriques (4-2, 80, 84), et où:
le dispositif d'engagement temporaire (3-1-d1, 3-1-d2, 3-2, 3-1-d6, 3-1-d7, 3-1-d8, 3-1-d9, 4-2, 4-4) est un dispositif porté au doigt (3-1-d2, 3-1-d7, 3-1-d8, 3-1-d9), un dispositif inséré dans un orifice corporel (3-1- d1), un dispositif de télédétection (3-2, 3-1-d6), un dispositif d'analyse de la respiration de la personne (1) (4-2), ou un dispositif de mesure du poids corporel de la personne (1) ou d'autres forces exercées par le corps et des données connexes (4-4);
le dispositif portable d'engagement soutenu (3-1-d4, 3-1-d5, 4-1a, 4-1b, 4-3, 131) est n'importe quel d'un dispositif porté au poignet, au cou, à la cheville, à la taille, au torse ou au doigt (3-1-d4, 3-1-d5, 4-3), d'un dispositif ou masque porté au visage (131) et d'un dispositif ou patch collé à la peau (4-1a, 4-1b) , et
le dispositif de collecte d'échantillons biométriques (4-2, 80, 84) est un écouvillon (80) comprenant une pointe d'écouvillon (80-tip), une bandelette de test (84) comprenant une zone de collecte d'échantillons (84-smp), ou un éthylotest (4-2).

8. Le système de la revendication 1 dans lequel le prestataire de services de santé (42) fournit au moins un service de santé (3-2), et lors de la fourniture d'au moins un service de santé (3-2), l'exécution de la (fonction 2) comprenant la combinaison de la (fonction a), de la (fonction b2), et de la (fonction c2-2) comprend:
la personne (1) utilise le dispositif informatique et l'application (2a, 2-3, 2-4, 2-5) pour exécuter la (fonction a) et la (fonction b2) tout en étant pratiquement isolée à l'intérieur d'une zone de confirmation (73), où pendant la (fonction b2) le dispositif informatique et l'application (2a, 2-3, 2-4, 2-5) fournissent en outre ou alternativement à un système ou à un agent de service du prestataire de services de santé (42) un certificat d'authentification ou d'autres informations de confirmation comprenant l'un des éléments suivants ou toute combinaison de ceux-ci: un code d'identité ou un certificat numérique unique de l'application (2a), un numéro de transaction unique, des informations sur l'assurance, un code de réponse provoqué au moins en partie par un code initial précédemment fourni par le système ou l'agent de service, une image de la personne (1), ou d'autres données confirmant que le dispositif informatique et l'application (2a, 2-3, 2-4, 2-5) ont déterminé dans la (fonction a) que la personne (1) actuellement isolée dans la zone de confirmation (73) est authentifiée par le dispositif informatique et l'application (2a, 2-3, 2- 4, 2-5) comme étant la personne (1) préenregistrée, le certificat d'authentification ou toute autre information de confirmation est fourni dans un format comprenant des informations transmises par voie électronique ou des informations visuelles présentées sur un écran du dispositif informatique et de l'application (2a, 2-3, 2-4, 2-5) et ne comprend aucune information d'identification personnelle autre qu'une image de la personne (1), de sorte que la personne (1) reste anonyme, après réception du certificat d'authentification ou d'une autre confirmation, l'agent de service ou tout autre agent du prestataire de services de santé (42) fournit au moins un service de santé (3-2) à la personne (1), après avoir fourni au moins un service de santé (3-2), le système ou un agent de service du prestataire de services de santé (42) transmet le reçu de confirmation de service au dispositif informatique et à l'application (2a, 2-3, 2-4, 2-5), 2-3, 2-4, 2-5) authentifiant que l'au moins un service de santé (3-2) a été fourni à la personne (1), le reçu de confirmation de service comprenant l'un des éléments suivants ou une combinaison de ceux-ci une identification du prestataire de services de santé (42), une identification de l'un ou de plusieurs agents de service, une identification d'au moins un service de santé (3-2) fourni, des informations sur l'assurance, un certificat d'authentification ou d'autres informations de confirmation, ou d'autres données de transaction liées à au moins un service de santé (3-2) fourni, lorsque le reçu de confirmation de service est fourni dans un format comprenant des informations transmises par voie électronique ou des informations présentées visuellement, et où le dispositif informatique et l'application (2a, 2-3, 2-4, 2-5) exécutent (fonction c2-2) et reçoivent le reçu de confirmation de service pour l'utiliser au moins en partie dans la confirmation que l'au moins un service de santé (3-2) a été fourni à la personne anonyme associée (1).

9. Le système de la revendication 1 dans lequel le système de contrôle d'accès (73) comprend au moins un point d'accès vérifié par (5a, 5b) séparant et reliant une zone de confirmation (73) et les locaux ou le rassemblement (74), dans lequel la zone de confirmation (73) comprend une zone physiquement, électroniquement ou visuellement restreinte, semi-restreinte ou autrement surveillée (73) pour isoler substantiellement la personne (1) tout en recevant anonymement l'état de santé authentifié (2d) suffisant
pour garantir que l'état reçu (2d) est celui de la personne (1), le système de contrôle d'accès (73) exécute (fonction 3) en communiquant avec le dispositif informatique et l'application (2a, 2-3, 2-4, 2-5) pour négocier la demande d'entrée pendant que la personne (1) se trouve dans la zone de confirmation (73), et le système de contrôle d'accès (73) régit l'accès aux locaux ou au rassemblement (74) par la personne (1) à travers au moins un point d'accès contrôlé et vérifié (5a, 5b) en se basant au moins en partie sur l'état de santé authentifié anonyme reçu (2d).

10. Le système de la revendication 9 dans lequel la personne (1) utilise le dispositif informatique et l'application (2a, 2-3, 2-4, 2-5) pendant qu'elle se trouve dans la zone de confirmation (73) pour fournir en plus l'une des informations suivantes ou une combinaison de celles-ci:
des informations d'identification personnelle, et
des informations électroniques justifiant d'un droit d'entrée payant (2b, 2c) dans les locaux ou le rassemblement (74), le droit d'entrée payant (2b, 2c) étant soit une entrée publique anonyme (1), soit une entrée privée non anonyme (1-w), le système de contrôle d'accès (73) régit l'accès aux locaux ou au rassemblement (74) par la personne publique (1) ou la personne privée (1-w) en se basant au moins en partie sur l'état de santé authentifié reçu (2d) et au moins en partie sur le droit d'entrée authentifié (2b, 2c).

11. Le système de la revendication 1, dans lequel une multiplicité de personnes (1) ont reçu et suivent l'un des régiments publics (2d-1, 2d-2) pour déterminer l'état de santé authentifié privé (2d) de chaque personne (1) faisant partie de la multiplicité de personnes (1), comprend en outre:
une base de données de santé publique partagée (102-db) pour recevoir des données de santé privées anonymes (2a-db) de l'une quelconque des multiples personnes (1), les données de santé privées anonymes (2a-db) comprenant l'une quelconque ou toute combinaison d'informations relatives à l'état de santé (2d) de l'une quelconque des multiples personnes (1), et
un ou plusieurs algorithmes de santé publique (102-ml) pour traiter les données de santé privées anonymisées (2a-db) composant la base de données de santé publique partagée (102-db), où le ou les algorithmes de santé publique (102-ml) déterminent un ou plusieurs messages de diffusion (102-msg) basés au moins en partie sur l'un ou l'autre des traitements, chacun des messages diffusés (102-msg) comprend au moins un détecteur entraîné (102-det), aucune information d'identification personnelle et une ou plusieurs combinaisons de recommandations ou de mises à jour pour l'un ou l'autre des régiments publics (2d-1, 2d-2) , les messages diffusés (102-msg) étant distribués par voie électronique aux dispositifs informatiques et aux applications (2a, 2-3, 2-4, 2-5) utilisés par une partie ou la totalité des personnes (1),
après réception d'un message diffusé (102-msg), le dispositif informatique et l'application de réception (2a, 2-3, 2-4, 2-5) exécutent le détecteur entraîné (102-det) pour traiter l'une des données de santé privées (2a-db) de chaque personne (1) associée au dispositif informatique et à l'application de réception (2a, 2-3, 2-4, 2-5) pour déterminer un ou plusieurs résultats privés , et où le dispositif informatique et l'application de réception (2a, 2-3, 2-4, 2-5) fournissent les recommandations à chaque personne associée
(1) et mettent à jour un ou plusieurs régimes publics (2d-1, 2d-2) actuellement utilisés par chaque personne associée (1) en se basant au moins en partie sur les résultats privés de chaque personne (1).

12. Le système de la revendication 1 dans lequel les locaux ou le rassemblement (74) sont ou se produisent dans une structure fixe ou un véhicule de transport mobile appartenant à une entité (4) ou exploité par elle, dans lequel le système de contrôle d'accès (73) pour régir l'accès aux locaux ou au rassemblement
(74) comprend un ou plusieurs points d'accès publics ou privés (5a, 5b) permettant chacun de régir l'accès public ou privé d'une multiplicité de personnes (1, 1-w) sur la base, au moins en partie, de la réception d'un état de santé suffisamment conforme (2d) de la part des personnes (1, 1-w) qui tentent d'accéder, l'entité (4) fournissant des informations d'assurance mutuelle concernant l'état de santé actuel des locaux ou du rassemblement (74) à l'une quelconque des personnes (1, 1-w) envisageant d'accéder, cherchant actuellement à accéder ou ayant déjà accédé aux locaux ou au rassemblement (74), l'information d'assurance mutuelle étant une indication de l'état de santé (2d) de certaines ou de la quasi-totalité des personnes (1, 1-w) qui se trouvent actuellement dans les locaux ou le rassemblement (74), qui les occupent, ou participant d'une autre manière aux locaux ou au rassemblement (74) au cours d'une période donnée, et où les informations d'assurance mutuelle sont basées au moins en partie sur l'état de santé (2d) fourni par chaque personne (1, 1-w) autorisée par le système de contrôle d'accès (73) à pénétrer dans les locaux ou le rassemblement (74) au cours de la période donnée.

13. Le système de la revendication 1 dans lequel les locaux ou le rassemblement (74) sont ou se produisent dans une structure fixe ou un véhicule de transport mobile appartenant à ou exploité par une entité (4), dans lequel le système de contrôle d'accès (73) pour régir l'accès aux locaux ou au rassemblement (74) comprend un ou plusieurs points d'accès publics ou privés (5a, 5b) chacun pour régir l'accès public ou privé par toute personne (1, 1-w) d'une multiplicité de personnes (1, 1-w) sur la base au moins en partie de la réception d'un état de santé suffisamment conforme (2d) de la personne (1, 1-w) qui tente d'accéder, dans lequel une ou plusieurs des personnes (1, 1-w) admises à accéder aux locaux ou au rassemblement (74) par le système de contrôle d'accès (73) sont des personnes suivies portant un vêtement sécurisé, 1-w) tentant d'accéder, où une ou plusieurs des personnes (1, 1-w) admises dans les locaux ou le rassemblement (74) par le système de contrôle d'accès (73) sont des personnes admises suivies portant un vêtement sécurisé (2-3, 3-1-d4, 3-1-d5) comprenant des moyens de suivi électronique, où les locaux ou le rassemblement (74) comprennent un réseau d'infrastructure de suivi suffisant positionné dans une partie ou la quasi-totalité des locaux ou du rassemblement (74) pour suivre de
manière substantielle les mouvements de tous les vêtement sécurisés (2-3, 3-1-d4, 3-1-d5) portés par une ou plusieurs personnes admises suivies (1, 1-w), suffisant pour déterminer les contacts dans une proximité donnée entre deux ou plusieurs personnes admises suivies (1, 1-w) portant actuellement un vêtement sécurisé (2-3, 3-1-d4, 3-1-d5) et se trouvant actuellement dans les locaux ou le rassemblement (74), lorsque l'entité (4) fournit des informations de suivi de contrat (2a-db-6) à une ou plusieurs personnes admises suivies (1, 1-w) portant actuellement ou ayant porté un vêtement sécurisé (2-3, 3-1- d4, 3-1-d5) et actuellement présentes ou ayant été présentes dans les locaux ou le rassemblement (74), sur la base, au moins en partie, des contacts déterminés impliquant la personne admise suivie (1, 1-w) et, au moins en partie, de l'état de santé (2d) d'une ou plusieurs autres personnes admises suivies (1, 1-w) portant ou ayant porté un vêtement sécurisé (2-3, 3-1-d4, 3-1-d5) et impliquées dans les contacts déterminés.

14. Le système de la revendication 13 dans lequel l'un ou l'autre ou les deux se produisent:
au moins une des personnes admises suivies (1, 1-w) après avoir été admise dans les locaux ou le rassemblement (74) reste dans les locaux ou le rassemblement (74) pendant une durée suffisante pour nécessiter la détermination d'au moins une mesure de santé supplémentaire (2d, 2a-db-4a) ou d'au moins un service de soins de santé supplémentaire (2d, 2a-db-4a) pour déterminer un état de santé actualisé (2d) afin de rester en conformité avec un ou plusieurs régimes publics (2d-1, 2d-2) après être resté dans les locaux ou au rassemblement (74), l'entité (4) exigeant qu'au moins une personne admise et restée sur place (1, 1-w) continue à suivre un ou plusieurs régimes publics (2d-1, 2d-2) et à s'y conformer, lorsque l'entité (4) continue à déterminer et à recevoir l'état de santé actualisé (2d) d'au moins une personne admise et restante (1-w) ayant fait l'objet d'un suivi pendant toute la durée où au moins une personne admise et restante (1, 1-w) ayant fait l'objet d'un suivi se trouve dans les locaux ou le rassemblement (74), les occupe ou y participe d'une autre manière, et lorsqu'un changement suffisant de l'état de santé (2d) d'au moins une personne admise et restante ayant fait l'objet d'un suivi (1, 1-w), l'entité (4) fournit des informations de traçage contractuel à une ou plusieurs autres personnes admises suivies (1, 1-w) dont l'entité (4) a déterminé au préalable qu'elles étaient entrées en contact suffisamment proche avec au moins une personne admise suivie et restante (1, 1-w) alors qu'elles étaient simultanément présentes dans les locaux ou le rassemblement (74), et
au moins une des personnes admises suivies (1, 1-w) après avoir été admise dans les locaux ou le rassemblement (74) quitte les locaux ou le rassemblement (74) pendant une durée suffisante pour nécessiter la détermination d'au moins une mesure de santé supplémentaire (2d, 2a-db-4a) ou au moins un service de santé supplémentaire (2d, 2a-db-4a) à utiliser pour déterminer un état de santé actualisé (2d) afin de rester en conformité avec un ou plusieurs régimes publics (2d-1, 2d-2) après avoir quitté les lieux ou le rassemblement (74), l'entité (4) exigeant qu'au moins une personne admise et quittant les lieux
et faisant l'objet d'un suivi (1, 1-w) continue à suivre un ou plusieurs régimes publics (2d-1, 2d-2) et à s'y conformer après avoir quitté les lieux ou le rassemblement (74) pendant une durée prolongée supérieure ou égale à une durée suffisante, l'entité (4) continue à déterminer et à recevoir l'état de santé actualisé (2d) d'au moins une personne (1, 1-w) admise et quittant les lieux pendant la durée prolongée du temps suffisant après lequel au moins une personne (1, 1-w) admise et quittant les lieux a quitté les locaux ou le rassemblement (74), et après un changement suffisant de l'état de santé (2d) d'au moins une personne (1, 1-w) admise et quittant les lieux après avoir quitté les locaux ou le rassemblement (74), et après un changement suffisant de l'état de santé (2d) d'au moins une personne (1, 1-w) admise et quittant les lieux après avoir quitté les locaux ou le rassemblement (74), 1-w) après avoir quitté les lieux ou le rassemblement (74), l'entité (4) fournit des informations de suivi de contrat à une ou plusieurs autres personnes admises suivies (1, 1-w) dont l'entité (4) a déterminé au préalable qu'elles étaient entrées en contact suffisamment proche avec au moins une personne admise suivie et quittant les lieux (1, 1-w) alors qu'elles étaient simultanément présentes dans les lieux ou sur le site (74).

15. Le système de la revendication 1, dans lequel les locaux ou le rassemblement (74) appartiennent ou sont gérés par une entité (4), dans lequel la personne (1) planifie l'accès aux locaux ou au rassemblement (74), comprend en outre l'un ou l'autre des éléments suivants:
un système de rendez-vous personne-service (104, 52) exploité par ou pour l'entité (4), dans lequel:
la personne (1) interagit avec le système de rendez-vous personne-service (104, 52) pour programmer au moins une date ou une date et une heure d'accès planifié aux locaux ou au rassemblement (74), à la suite ou après avoir interagi avec le système de rendez-vous personne-service (104, 52), le système de rendez- vous personne-service (104, 52) fournit ou met à la disposition du dispositif informatique et de l'application (2a, 2-3, 2-4, 2-5) enregistrés et utilisés par la personne (1) un programme de rendez-vous (2d-3a) comprenant des informations suffisantes pour servir, mettre à jour, modifier ou constituer une partie ou la totalité d'un programme de santé (2d-1, 2d-2) que la personne (1) doit respecter avant de tenter l'accès programmé, lorsqu'avant de tenter l'accès programmé aux locaux ou au rassemblement (74), le dispositif informatique et l'application (2a, 2-3, 2-4, 2-5) fournissent un ou plusieurs jetons de vérification (2d-3b) comprenant des données de vérification fondées au moins en partie sur l'une ou l'autre des informations de santé relatives au régime de rendez-vous (2d-3a) ou à un ou plusieurs régimes publics (2d-1, 2d-2), ou sur une combinaison de ces informations, lorsque les informations de santé se rapportent au régime de rendez-vous (2d-3a) ou à un ou plusieurs régimes publics (2d-1, 2d-2), 2d-2), les informations de santé comprenant les informations relatives à l'état de santé (2d) de la personne (1), le système de prise de rendez-vous personne-service (104, 52) recevant un ou plusieurs jetons de vérification (2d-3b) agissant de manière à réaliser l'une des actions suivantes ou une combinaison de ces actions:
(i) reporter la date ou la date et l'heure de l'accès prévu aux locaux ou au rassemblement (74);
(ii)fournir ou mettre à disposition du dispositif informatique et de l'application (2a, 2-3, 2-4, 2-5) des mises à jour ou un nouveau programme de rendez-vous (2d-3a);
(iii) alerter ou mettre à jour d'une autre manière un ou plusieurs membres du personnel travaillant pour l'entité (4) ou devant interagir d'une autre manière avec la personne (1) lorsque ou après que la personne
(1) a accédé aux locaux ou au rassemblement (74);
(iv) fournir un jeton d'accès (2d-3c) au dispositif informatique et à l'application (2a, 2-3, 2-4, 2-5), le jeton d'accès (2d-3c) étant basé au moins en partie sur le respect suffisant par la personne (1) d'au moins un programme de rendez-vous (2d-3a) fourni par le système de programmation (104, 52), et
lorsqu'en interagissant avec le système de contrôle d'accès (73) à la date ou à la date et à l'heure prévues de l'accès planifié, la personne (1) utilise le dispositif informatique et l'application (2a, 2-3, 2-4, 2-5) pour fournir l'une des informations du jeton d'accès basées au moins en partie sur le jeton d'accès (2d-3c), soit en alternative, soit en complément de la fourniture de l'état de santé anonyme et authentifié (2d), la personne (1) peut fournir un jeton d'accès (2d-3c), authentifié (2d), et où le système de contrôle d'accès
(73) régit l'accès de la personne (1) aux locaux ou au rassemblement (74) en se basant au moins en partie sur les informations du jeton d'accès (2d-3c),
et
un système de rendez-vous de personne à personne (105, 53) exploité par ou pour l'entité (4) ou par une ou plusieurs personnes gérant un rassemblement entre deux ou plusieurs personnes (1), dans lequel:
les deux ou plusieurs personnes (1) interagissent avec le système de rendez-vous de personne à personne (105, 53) pour programmer au moins une date ou une date et une heure d'un rassemblement planifié ou d'une autre réunion (74), où, lors de l'interaction avec le système de rendez-vous de personne à personne (105, 53), le système de rendez-vous de personne à personne (105, 53) fournit ou met à disposition de chacun des dispositifs informatiques et des applications (2a, 2-5-1, 2-5-2) enregistrés et utilisés par chacune des deux personnes ou plus (1) un régime de rendez-vous (2d-3a-1, 2d-3a-2) comprenant toute information suffisante pour servir, mettre à jour, modifier ou constituer autrement une partie ou la totalité d'un régime de santé (2d-1, 2d-2) à respecter par les deux personnes ou plus (1) avant de tenter de participer au rassemblement ou à la réunion planifié(e) (74), où, avant de tenter de participer au rassemblement planifié ou à une autre réunion (74), chaque dispositif informatique et application (2a, 2-5-1, 2-5-2) fournit un ou plusieurs jetons de vérification (2d-3b-1, 2d-3b-2) comprenant des données de vérification fondées au moins en partie sur l'une ou l'autre des informations de santé relatives au régime de rendez-vous (2d-3a-1, 2d-3a-2) ou au régime de santé (2d-3a-1, 2d-3a-2), ou encore sur une combinaison de ces informations, 2d-3a-2) ou du ou des régiments publics (2d-1, 2d-2), les informations de santé comprenant des informations relatives à l'état de santé (2d) de l'une des deux personnes ou plus (1), le système de prise de rendez-vous de personne à personne (105, 53) recevant un ou plusieurs jetons de vérification (2d-3b-1, 2d-3b-2) agissant de manière à réaliser l'une des actions suivantes ou une combinaison de ces actions:
(i) reprogrammer la date ou la date et l'heure du rassemblement ou de la réunion prévus (74);
(ii) fournir ou mettre à la disposition de chacun des appareils informatiques et des applications (2a, 2-5-1, 2-5-2) des mises à jour ou un nouveau programme de rendez-vous (2d-3a-1, 2d-3a-2);
(iii) alerter ou mettre à jour d'une autre manière un ou plusieurs membres du personnel travaillant pour l'entité (4), gérant le rassemblement ou la réunion (74), ou devant interagir d'une autre manière avec l'une des deux personnes ou plus (1) lorsque ou après que l'une des deux personnes ou plus (1) accède au rassemblement ou à la réunion prévu (74), et l'une des deux personnes ou plus (1) prévoyant d'assister au rassemblement ou à la réunion (74);
(iv) fournir un jeton d'accès (2d-3c-1, 2d-3c-2) à chacun des dispositifs informatiques et applications (2a, 2-5-1, 2-5-2), chaque jeton d'accès fourni (2d-3c-1, 2d-3c-2) étant basé au moins en partie sur le respect suffisant par chaque personne (1) des deux personnes ou plus (1) d'au moins un programme de rendez- vous (2d-3a-1, 2d-3a-2) fourni par le système de planification (105, 53), et
où, après avoir reçu un jeton d'accès (2d-3c), chaque personne (1) parmi les deux personnes ou plus (1) est autorisée à assister au rassemblement planifié ou à une autre réunion (74), et où l'assistance comprend éventuellement une interaction avec le système de contrôle d'accès (73) à la date ou à la date et à l'heure prévues du rassemblement planifié ou d'une autre réunion (74) pour fournir des informations sur le jeton d'accès basées au moins en partie sur le jeton d'accès reçu (2d-3c-1, 2d-3c-2), soit en alternative ou en complément de la fourniture de l'état de santé anonyme et authentifié (2d), soit en interagissant avec chacun des dispositifs informatiques et des applications (2a, 2-5-1, 2-5-2) utilisés par l'une des deux autres personnes ou plus (1) pour fournir des informations sur le jeton d'accès basées au moins en partie sur le jeton d'accès reçu (2d-3c), soit en alternative ou en complément de la fourniture de l'état de santé anonyme et authentifié (2d).
